# EUROPEAN PATENT SPECIFICATION

(11) **EP 4 482 583 B1**
(45) Date of publication and mention of the grant of the patent: **01.04.2026**
(21) Application number: 23707330.9
(22) Date of filing: 23.02.2023
(51) Int. Cl.: A61P 31/04, C07D 401/14, C07D 403/14, C07D 409/14, C07D 413/14, C07D 417/14, C07D 471/04, C07D 487/08

(54) **ZWITTERIONIC ANTIBACTERIAL COMPOUNDS**
ZWITTERIONISCHE ANTIBAKTERIELLE VERBINDUNGEN
COMPOSÉS ANTIBACTÉRIENS ZWITTÉRIONIQUES

(30) Priority: 25.02.2022 EP 22158739
(43) Date of publication of application: 01.01.2025
(73) Proprietor: F. Hoffmann-La Roche AG, 4070 Basel (CH)
(72) Inventor: ULSEMER, Sandra Marie Joseph, 4070 Basel (CH); LERNER, Christian, 4070 Basel (CH); MATTEI, Patrizio, 4070 Basel (CH); SCHMITT, Sébastien, 4070 Basel (CH); URNER, Lorenz Michael, 4070 Basel (CH); BLANC, Jean-Baptiste Emmanuel, 4070 Basel (CH); POVAH, Jacob William, 4070 Basel (CH); STOLL, Theodor Walter Jakob, 4070 Basel (CH); WIMMER, Stefan Hermann, 4070 Basel (CH)
(74) Representative: Neuhaus, Christian Michel
(86) International application number: PCT/EP2023/054499
(87) International publication number: WO 2023/161316

(56) References cited:
- WO-A1-2020/182648
- WO-A1-2021/148420

## Description

### Field of the Invention

The present invention relates to novel imidazole-pyrazole derivatives which exhibit antibacterial properties. The invention also relates to methods of using the c15801580ompounds for the treatment or prevention of bacterial infections and resulting diseases, in particular for the treatment or prevention of infections with *Acinetobacter baumannii* and resulting diseases.

### Background of the Invention

*Acinetobacter baumannii* is a Gram-negative, aerobic, nonfermenting bacterium recognized over the last decades as an emergining pathogen with very limited treatment options.

*A. baumannii* is considered to be a serious threat by the US Centers for Disease Control and Prevention and belongs to the so called 'ESKAPE' pathogens (***E**nterococcus faecium, **S**taphylococcus aureus, **K**lebsiella pneumoniae, **A**cinetobacter baumannii, **P**seudomonas aeruginosa* and ***E**nterobacter species & E. coli*) that currently cause the majority of nosocomial infections and effectively "escape" the activity of antimicrobial agents.

*A. baumannii* is most often encountered in intensive care units and surgical wards, where extensive antibiotic use has enabled selection for resistance against all known antimicrobials and where it causes infections that include bacteremia, pneumonia, meningitis, urinary tract infection, and wound infection.

*A. baumannii* has an exceptional ability to upregulate and acquire resistance determinants and shows an environmental persistance that allows its survival and spread in the nosocomial setting, making this organism a frequent cause of outbreaks of infection and an endemic, health care-associated pathogen.

Due to increasing antibiotic resistance to most if not all available therapeutic options, Muti-Drug Resistant (MDR) *A. baumanniii* infections, especially those caused by Carbapenem resistant *A. baumannii,* are extremely difficult or even impossible to treat with high mortality rate as well as increased morbidity and length of stay in intensive care unit.

*Acinetobacter baumannii* has been defined and still remains "a prime example of a mismatch between unmet medical needs and the current antimicrobial research and development pipeline" according to the Antimicrobial Availability Task Force (AATF) of the Infectious Diseases Society of America (IDSA). Thus, there is a high demand and need to identify compounds suitable for the treatment of diseases and infections caused by *Acinetobacter baumannii.* WO 2020/182648 already disclosed compounds which are useful for treating nosocomial infections and infections caused by Gram-negative bacteria.

The present invention provides novel compounds which exhibit activity against drug-susceptible as well as drug-resistant strains of *Acinetobacter baumannii.*

### Summary of the Invention

The scope of the invention is defined by the claims. Any references in the description to methods of treatment refer to the compounds, pharmaceutical compositions and medicaments of the present invention for use in a method for treatment of the human (or animal) body by therapy (or for diagnosis). In a first aspect, the present invention provides a compound of formula (I) 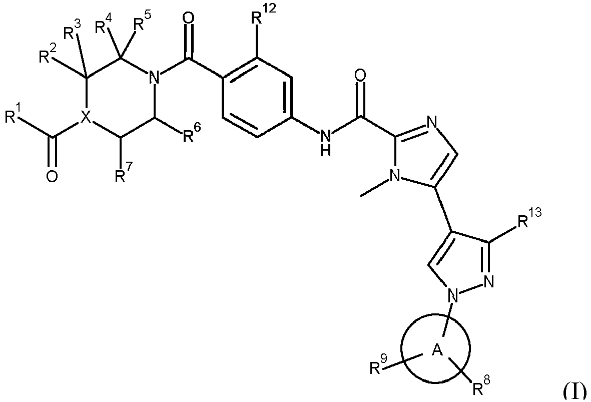 or a pharmaceutically acceptable salt thereof, wherein A, X, R¹-R⁹, R¹² and R¹³ are as defined herein.

In one aspect, the present invention provides a process of manufacturing the compounds of formula (I) described herein, wherein said process is as decribed in Schemes 1-12 below, in particular as described in Scheme 4.

In a further aspect, the present invention provides a compound of formula (I) as described herein, when manufactured according to the processes described herein.

In a further aspect, the present invention provides a compound of formula (I) as described herein, or a pharmaceutically acceptable salt thereof, for use as therapeutically active substance.

In a further aspect, the present invention provides a pharmaceutical composition comprising a compound of formula (I) as described herein, or a pharmaceutically acceptable salt thereof, and a therapeutically inert carrier.

In a further aspect, the present invention provides a compound of formula (I) as described herein, or a pharmaceutically acceptable salt thereof, for use as antibiotic.

### Detailed Description of the Invention

### Definitions

Features, integers, characteristics, compounds, chemical moieties or groups described in conjunction with a particular aspect, embodiment or example of the invention are to be understood to be applicable to any other aspect, embodiment or example described herein, unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The term "alkyl" refers to a mono- or multivalent, e.g., a mono- or bivalent, linear or branched saturated hydrocarbon group of 1 to 6 carbon atoms ("C₁-C₆-alkyl"), e.g., 1, 2, 3, 4, 5, or 6 carbon atoms. In some embodiments, the alkyl group contains 1 to 3 carbon atoms, e.g., 1, 2 or 3 carbon atoms. Some non-limiting examples of alkyl include methyl, ethyl, propyl, 2-propyl (isopropyl), n-butyl, iso-butyl, sec-butyl, tert-butyl, and 2,2-dimethylpropyl. A particularly preferred, yet non-limiting example of alkyl is methyl.

The term "alkoxy" refers to an alkyl group, as previously defined, attached to the parent molecular moiety via an oxygen atom. Unless otherwise specified, the alkoxy group contains 1 to 6 carbon atoms ("C₁-C₆-alkoxy"). In some preferred embodiments, the alkoxy group contains contains 1 to 4 carbon atoms. In still other embodiments, the alkoxy group contains 1 to 3 carbon atoms. Some non-limiting examples of alkoxy groups include methoxy, ethoxy, n-propoxy, isopropoxy, n-butoxy, isobutoxy and tert-butoxy. A particularly preferred, yet non-limiting example of alkoxy is methoxy.

The term "halogen" or "halo" refers to fluoro (F), chloro (Cl), bromo (Br), or iodo (I). Preferably, the term "halogen" or "halo" refers to fluoro (F), chloro (Cl) or bromo (Br). Particularly preferred, yet non-limiting examples of "halogen" or "halo" are fluoro (F) and chloro (Cl).

The term "aminoalkyl" refers to an alkyl group, wherein at least one of the hydrogen atoms of the alkyl group has been replaced by an amino group. Preferably, "aminoalkyl" refers to an alkyl group wherein 1, 2 or 3 hydrogen atoms of the alkyl group have been replaced by an amino group. Preferred, yet non-limiting examples of aminoalkyl are aminomethyl, 2-aminoethyl, 3-aminopropyl, 4-aminobutyl, 5-aminopentyl and 6-aminohexyl. Particularly preferred, yet non-limiting examples of aminoalkyl are 3-aminopropyl, 4-aminobutyl and 6-aminohexyl.

The term "heterocyclylalkyl" refers to an alkyl group, wherein at least one of the hydrogen atoms of the alkyl group has been replaced by a heterocycle. Preferably, "heterocyclylalkyl" refers to an alkyl group wherein 1, 2 or 3 hydrogen atoms of the alkyl group have been replaced by a heterocycle. Most preferably, "heterocyclylalkyl" refers to an alkyl group wherein 1 hydrogen atom of the alkyl group has been replaced by a heterocycle. A preferred, yet non-limiting example of heterocyclylalkyl is azetidinylmethyl, e.g., azetidin-3-ylmethyl.

The term "aminoalkoxy" refers to an alkoxy group, wherein at least one of the hydrogen atoms of the alkoxy group has been replaced by an amino group. Preferably, "aminoalkoxy" refers to an alkoxy group wherein 1, 2 or 3 hydrogen atoms of the alkoxy group have been replaced by an amino group. Most preferably, "aminoalkoxy" refers to an alkoxy group wherein 1 hydrogen atom of the alkoxy group has been replaced by an amino group. Preferred, yet non-limiting examples of aminoalkoxy are aminomethoxy and 2-aminoethoxy.

The term "cycloalkyl" as used herein refers to a saturated or partly unsaturated monocyclic or bicyclic hydrocarbon group of 3 to 10 ring carbon atoms ("C₃₋₁₀-cycloalkyl"). In some preferred embodiments, the cycloalkyl group is a saturated monocyclic hydrocarbon group of 3 to 8 ring carbon atoms. "Bicyclic cycloalkyl" refers to cycloalkyl moieties consisting of two saturated carbocycles having two carbon atoms in common, i.e., the bridge separating the two rings is either a single bond or a chain of one or two ring atoms, and to spirocyclic moieties, i.e., the two rings are connected via one common ring atom. Preferably, the cycloalkyl group is a saturated monocyclic hydrocarbon group of 3 to 6 ring carbon atoms, e.g., of 3, 4, 5 or 6 carbon atoms. Some non-limiting examples of cycloalkyl include cyclopropyl, cyclobutyl, cyclopentyl, cyclohexyl, cycloheptyl, 1-bicyclo[1.1.1]pentanyl, norbornanyl, and 1-bicyclo[2.2.2]octanyl. Particularly preferred, yet non-limiting examples of cycloalkyl are cyclopropyl and cyclopentyl.

The term "heterocyclyl" refers to a saturated or partly unsaturated mono- or bicyclic, preferably monocyclic ring system of 3 to 14 ring atoms, preferably 3 to 10 ring atoms, preferably 3 to 8 ring atoms, wherein 1, 2, or 3 of said ring atoms are heteroatoms selected from N, O and S, the remaining ring atoms being carbon. Preferably, 1 to 2 of said ring atoms are selected from N and O, the remaining ring atoms being carbon. "Bicyclic heterocyclyl" refers to heterocyclic moieties consisting of two cycles having two ring atoms in common, i.e., the bridge separating the two rings is either a single bond or a chain of one or two ring atoms, and to spirocyclic moieties, i.e., the two rings are connected via one common ring atom. Some non-limiting examples of heterocyclyl groups include morpholine, thiomorpholine, piperazine, pyrrolidinyl, piperidyl, pyridyl, cyclopropyl, cyclopentyl, 1,2,3,3a,4,5,6,6a-octahydropyrrolo[3,4-c]pyrrole, 9-oxa-3,7-diazabicyclo[3.3.1]nonane, 7-oxa-2-azaspiro[3.5]nonane, and 2,6-dioxa-9-azaspiro[4.5]decane.

The term "aryl" refers to a monocyclic, bicyclic, or tricyclic carbocyclic ring system having a total of 6 to 14 ring members ("C₆-C₁₄-aryl"), preferably, 6 to 12 ring members, and more preferably 6 to 10 ring members, and wherein at least one ring in the system is aromatic. Some non-limiting examples of aryl include phenyl and 9H-fluorenyl (e.g. 9H-fluoren-9-yl). A particularly preferred, yet non-limiting example of aryl is phenyl.

The term "heteroaryl" refers to a mono- or multivalent, monocyclic or bicyclic, preferably bicyclic ring system having a total of 5 to 14 ring members, preferably, 5 to 12 ring members, and more preferably 5 to 10 ring members, wherein at least one ring in the system is aromatic, and at least one ring in the system contains one or more heteroatoms. Preferably, "heteroaryl" refers to a 5-10 membered heteroaryl comprising 1, 2, 3 or 4 heteroatoms independently selected from O, S and N. Preferably, "heteroaryl" refers to a 5-10 membered heteroaryl comprising 1 to 2 heteroatoms independently selected from O and N. Most preferably, "heteroaryl" refers to a 5-6 membered heteroaryl comprising 1 to 2 heteroatoms independently selected from O and N. Some preferred, yet non-limiting examples of heteroaryl include thiazolyl; isothiazolyl; pyridyl; 1H-pyrrolo[3,2-b]pyridine; 1H-imidazo[4,5-b]pyridine; 1,3-benzothiazole; benzothiophene; 1H-pyrazolo[4,3-b]pyridine; 1H-pyrrolo[3,2-c]pyridine; 1H-indazolyl; furo[3,2-b]pyridine; 2,3-dihydro-1H-pyrrolo[3,2-b]pyridine. A particularly preferred, yet non-limiting example of heteroaryl is pyridyl.

The term "hydroxy" refers to an -OH group.

The term "amino" refers to an -NH₂ group.

The term "carboxy" refers to a group -COOH.

The term "cyano" refers to a -CN (nitrile) group.

The term "oxo" refers to an =O group.

The term "imino" refers to an =NH group.

The term "carbamoyl" refers to a group -C(O)-NH₂.

The term "hydroxycarbamoyl" refers to a group -C(O)-NH-OH.

The term "carboxyalkyl" refers to an alkyl group, wherein at least one of the hydrogen atoms of the alkyl group has been replaced by a carboxy group. Preferably, "carboxyalkyl" refers to an alkyl group wherein 1, 2 or 3 hydrogen atoms of the alkyl group have been replaced by a carboxy group. A preferred, yet non-limiting example of carboxyalkyl is carboxymethyl.

The term "alkoxyalkyl" refers to an alkyl group, wherein at least one of the hydrogen atoms of the alkyl group has been replaced by an alkoxy group. Preferably, "alkoxyalkyl" refers to an alkyl group wherein 1, 2 or 3 hydrogen atoms of the alkyl group have been replaced by an alkoxy group. Most preferably, "alkoxyalkyl" refers to an alkyl group wherein 1 hydrogen atom of the alkyl group has been replaced by an alkoxy group. Particular, yet non-limiting examples of alkoxyalkyl groups are methoxymethyl and 2-methoxyethyl, in particular 2-methoxyethyl.

The term "hydroxyalkyl" refers to an alkyl group, wherein at least one of the hydrogen atoms of the alkyl group has been replaced by a hydroxy group. Preferably, "hydroxyalkyl" refers to an alkyl group wherein 1, 2 or 3 hydrogen atoms, most preferably 1 hydrogen atom of the alkyl group have been replaced by a hydroxy group. Preferred, yet non-limiting examples of hydroxyalkyl are hydroxymethyl, hydroxyethyl (e.g. 2-hydroxyethyl), hydroxypropyl (e.g. 3-hydroxypropyl), 2-hydroxy-2-methyl-propyl, 2,3-dihydroxypropyl, 2-hydroxy-1-(hydroxymethyl)-ethyl 3-hydroxy-3-methyl-butyl, 1,2-dihydroxyethyl.

The term "halohydroxyalkyl" refers to a hydroxyalkyl group, wherein at least one of the hydrogen atoms of the hydroxyalkyl group has been replaced by a halogen atom. Preferably, "halohydroxyalkyl" refers to a hydroxyalkyl group wherein 1, 2 or 3 hydrogen atoms of the hydroxyalkyl group have been replaced by a halogen atom. A preferred, yet non-limiting example of halohydroxyalkyl is 3,3,3-trifluoro-2-hydroxypropyl.

The term "pharmaceutically acceptable salt" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, trifluoroacetic acid, propionic acid, glycolic acid, pyruvic acid, oxalic acid, maleic acid, malonic acid, succinic acid, fumaric acid, tartaric acid, lactic acid, citric acid, benzoic acid, cinnamic acid, mandelic acid, methanesulfonic acid, ethanesulfonic acid, p-toluenesulfonic acid, salicylic acid, N-acetylcystein and the like. In addition these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of formula (I) are hydrochlorides, fumarates, lactates (in particular derived from L-(+)-lactic acid), tartrates (in particular derived from L-(+)-tartaric acid) and trifluoroacetates.

The compounds of formula (I) can contain several asymmetric centers and can be present in the form of optically pure enantiomers, mixtures of enantiomers such as, for example, racemates, optically pure diastereioisomers, mixtures of diastereoisomers, diastereoisomeric racemates or mixtures of diastereoisomeric racemates.

According to the Cahn-Ingold-Prelog Convention, the asymmetric carbon atom can be of the "R" or "S" configuration.

The term "treatment" as used herein includes: (1) inhibiting the state, disorder or condition (e.g. arresting, reducing or delaying the development of the disease, or a relapse thereof in case of maintenance treatment, of at least one clinical or subclinical symptom thereof); and/or (2) relieving the condition (i.e., causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

The term "prophylaxis" or "prevention" as used herein includes: preventing or delaying the appearance of clinical symptoms of the state, disorder or condition developing in a mammal and especially a human that may be afflicted with or predisposed to the state, disorder or condition but does not yet experience or display clinical or subclinical symptoms of the state, disorder or condition.

The term "mammal" as used herein includes both humans and non-humans and includes but is not limited to humans, non-human primates, canines, felines, murines, bovines, equines, and porcines. In a particularly preferred embodiment, the term "mammal" refers to humans.

The term "nosocomial infection" refers to a hospital-acquired infection (HAI), which is an infection that is acquired in a hospital or other health care facility. To emphasize both hospital and nonhospital settings, it is sometimes instead called a health care-associated infection (HAI or HCAI). Such an infection can be acquired in hospitals, nursing homes, rehabilitation facilities, outpatient clinics, or other clinical settings.

### Compounds of the Invention

In a first aspect, the present invention provides a compound of formula (I) 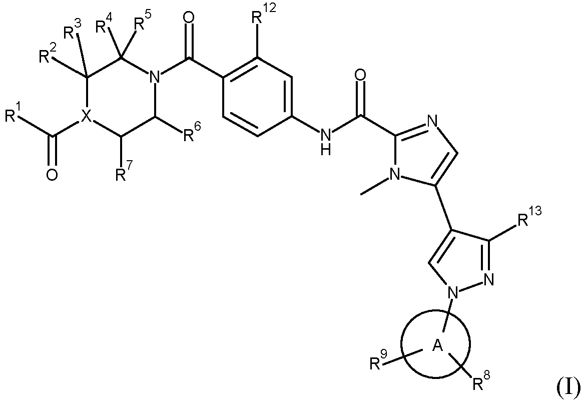 or a pharmaceutically acceptable salt thereof, wherein:
- X and Y: are each independently selected from N and CH;
- n: is selected from 0 to 6;
- A: is selected from 5- to 14-membered heteroaryl and C₆-C₁₄-aryl;
- B: is selected from 3- to 14-membered heterocyclyl, 5- to 14-membered heteroaryl and C₃-C₁₀-cycloalkyl;
- L: is selected from a covalent bond, carbonyl, NHC(O), C(O)NH, CH₂NH, NHCH₂ and NH;
- L¹: is -O- or a covalent bond;
- R¹: is selected from
(i) C₁-C₆-alkyl substituted with one amino and one carboxy substituent;
(ii) C₁-C₆-alkyl substituted with one amino and one carboxy-C₁-C₆-alkyl-NH-substituent;
(iii) C₁-C₆-alkyl-N(R^{1a}R^{1b});
(iv) -L¹-C₁-C₆-alkyl-N⁺(R^{1c}R^{1d}R^{1e});
(v) a group 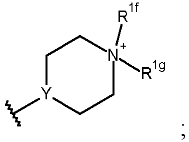 and
(vi) a group 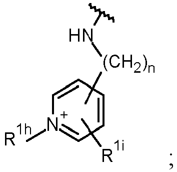
- R^{1a}: is amino-C₁-C₆-alkyl;
- R^{1b}: is carboxy-C₁-C₆-alkyl;
- R^{1c}: is selected from amino-C₁-C₆-alkyl and (3- to 14-membered heterocyclyl)-C₁-C₆-alkyl-;
- R^{1d}: is carboxy-C₁-C₆-alkyl;
- R^{1e}: is selected from C₁-C₆-alkyl, amino-C₁-C₆-alkyl and (3- to 14-membered heterocyclyl)-C₁-C₆-alkyl-;
- R^{1f}: is selected from C₁-C₆-alkyl, hydroxy-C₁-C₆-alkyl, amino-C₁-C₆-alkyl, C₁-C₆-alkyl-NH-C₁-C₆-alkyl, (C₁-C₆-alkyl)₂N-C₁-C₆-alkyl, and (3- to 14-membered heterocyclyl)-C₁-C₆-alkyl-;
- R^{1g}: is carboxy-C₁-C₆-alkyl;
- R^{1h}: is carboxy-C₁-C₆-alkyl;
- R¹ⁱ: is amino-C₁-C₆-alkyl;
- R²: is selected from hydrogen, C₁-C₆-alkyl and C₁-C₆-alkoxy; and R³ and R⁷ are both hydrogen; or
- R² and R⁷,: taken together with the atoms to which they are attached, form a 4- to 14-membered heterocycle or a C₄-C₁₀-cycloalkyl; and R³ is hydrogen; or
- R² and R³,: taken together with the carbon atom to which they are attached, form a C₃-C₁₀-cycloalkyl; and R⁷ is hydrogen;
- R⁴: is selected from hydrogen and C₁-C₆-alkyl; and R⁵ and R⁶ are both hydrogen; or
- R⁴ and R⁶,: taken together with the atoms to which they are attached, form a 4- to 14-membered heterocycle; and R⁵ is hydrogen; or
- R⁴ and R⁵,: taken together with the carbon atom to which they are attached, form a C₃-C₁₀-cycloalkyl; and R⁶ is hydrogen;
- R⁸: is selected from hydrogen, cyano, hydroxy, amino, C₁-C₆-alkyl-NH-, (C₁-C₆-alkyl)₂N-, (C₁-C₆-alkyl)₂N-SO₂-, (C₁-C₆-alkyl)₂N-C₁-C₆-alkyl-, amino-C₁-C₆-alkyl, amino-C₁-C₆-alkoxy, amino-C₁-C₆-alkyl-NH-, (amino-C₁-C₆-alkyl)₂N-, (amino-C₁-C₆-alkyl)₂N-C₁-C₆-alkyl-NH-, C₁-C₆-alkyl-NH-C₁-C₆-alkyl-NH-, C₁-C₆-alkoxy-C₁-C₆-alkyl-NH-, halo-C₁-C₆-alkoxy-C₁-C₆-alkyl-NH-, C₃-C₁₀-cycloalkyl-NH-, C₁-C₆-alkoxy, C₁-C₆-alkyl, cyano-C₁-C₆-alkyl-, hydroxy-C₁-C₆-alkyl-, hydroxy-C₁-C₆-alkyl-NH-, hydroxy-C₁-C₆-alkyl-NHC(O)-, halo-hydroxy-C₁-C₆-alkyl-NHC(O)-, carbamoyl, hydroxycarbamoyl, C₁-C₆-alkyl-NHC(O)-, (C₁-C₆-alkyl)₂NC(O)-, carbamoyl-C₁-C₆-alkyl-, and a group 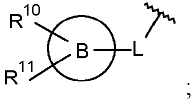
- R⁹: is selected from hydrogen, halogen, amino, C₁-C₆-alkyl, and hydroxy-C₁-C₆-alkyl-;
- R¹⁰: is selected from hydrogen, C₁-C₆-alkyl, halogen, hydroxy, oxo, imino, and amino;
- R¹¹: is selected from hydrogen and halogen;
- R¹²: is selected from halogen and C₁-C₆-alkyl;
- R¹³: is halo-C₁-C₆-alkyl.

In one embodiment, the present invention provides a compound of formula (I) as described herein, or a pharmaceutically acceptable salt thereof, wherein L is selected from a covalent bond, carbonyl, NHC(O), CH₂NH and NH.

In one embodiment, the present invention provides a compound of formula (I) as described herein, or a pharmaceutically acceptable salt thereof, wherein R^{1f} is selected from C₁-C₆-alkyl, hydroxy-C₁-C₆-alkyl, amino-C₁-C₆-alkyl, (C₁-C₆-alkyl)₂N-C₁-C₆-alkyl, and (3- to 14-membered heterocyclyl)-C₁-C₆-alkyl-.

In a preferred embodiment, the present invention provides a compound of formula (I) as described herein, or a pharmaceutically acceptable salt thereof, wherein:
- R¹: is selected from
(i) C₁-C₆-alkyl-N⁺(R^{1c}R^{1d}R^{1e}); and
(ii) a group 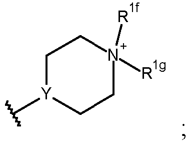 wherein
- Y: is CH;
- R^{1c} and R^{1e}: are both amino-C₁-C₆-alkyl;
- R^{1d} and R^{1g}: are both carboxy-C₁-C₆-alkyl; and
- R^{1f}: is selected from amino-C₁-C₆-alkyl and (3- to 14-membered heterocyclyl)-C₁-C₆-alkyl-.

In a preferred embodiment, the present invention provides a compound of formula (I) as described herein, or a pharmaceutically acceptable salt thereof, wherein:
- R¹: is a group 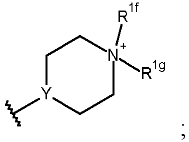 wherein
- Y: is CH;
- R^{1g}: is carboxy-C₁-C₆-alkyl; and
- R^{1f}: is selected from amino-C₁-C₆-alkyl and (3- to 14-membered heterocyclyl)-C₁-C₆-alkyl-.

In a preferred embodiment, the present invention provides a compound of formula (I) as described herein, or a pharmaceutically acceptable salt thereof, wherein:
- R¹: is C₁-C₆-alkyl-N⁺(R^{1c}R^{1d}R^{1e}); wherein
- R^{1c} and R^{1e}: are both amino-C₁-C₆-alkyl; and
- R^{1d}: is carboxy-C₁-C₆-alkyl.

In a particularly preferred embodiment, the present invention provides a compound of formula (I) as described herein, or a pharmaceutically acceptable salt thereof, wherein:
- R¹: is selected from
(i) -(CH₂)₃-N⁺(R^{1c}R^{1d}R^{1e}); and
(ii) a group 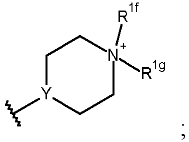 wherein
- Y: is CH;
- R^{1c} and R^{1e}: are both 4-aminobutyl;
- R^{1d} and R^{1g}: are both carboxymethyl; and
- R^{1f}: is selected from 3-aminopropyl and azetidin-3-ylmethyl.

In a particularly preferred embodiment, the present invention provides a compound of formula (I) as described herein, or a pharmaceutically acceptable salt thereof, wherein:
- R¹: is a group 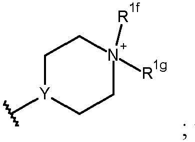 wherein
- Y: is CH;
- R^{1g}: is carboxymethyl; and
- R^{1f}: is selected from 3-aminopropyl and azetidin-3-ylmethyl.

In a particularly preferred embodiment, the present invention provides a compound of formula (I) as described herein, or a pharmaceutically acceptable salt thereof, wherein:
- R¹: is -(CH₂)₃-N⁺(R^{1c}R^{1d}R^{1e}); wherein
- R^{1c} and R^{1e}: are both 4-aminobutyl; and
- R^{1d}: is carboxymethyl.

In a preferred embodiment, the present invention provides a compound of formula (I) as described herein, or a pharmaceutically acceptable salt thereof, wherein R², R³, R⁴, R⁵, R⁶ and R⁷ are all hydrogen.

In one embodiment, the present invention provides a compound of formula (I) as described herein, or a pharmaceutically acceptable salt thereof, wherein A is selected from thiazolyl; isothiazolyl; pyridyl; 1H-pyrrolo[3,2-b]pyridine; 1H-imidazo[4,5-b]pyridine; 1,3-benzothiazole; benzothiophene; 1H-pyrazolo[4,3-b]pyridine; 1H-pyrrolo[3,2-c]pyridine; 1H-pyrrolo[2,3-b]pyridine, 1H-pyrrolo[2,3-c]pyridine, 2H-pyrazolo[4,3-c]pyridine, 1H-pyrrolo[3,2-d]pyrimidine, 1H-indazolyl; furo[3,2-b]pyridine; 2,3-dihydro-1H-pyrrolo[3,2-b]pyridine; and phenyl.

In one embodiment, the present invention provides a compound of formula (I) as described herein, or a pharmaceutically acceptable salt thereof, wherein B is selected from morpholine, thiomorpholine, piperazine, pyrrolidinyl, piperidyl, pyridyl, cyclopropyl, cyclopentyl, 1,2,3,3a,4,5,6,6a-octahydropyrrolo[3,4-c]pyrrole, 9-oxa-3,7-diazabicyclo[3.3.1]nonane, 7-oxa-2-azaspiro[3.5]nonane, and 2,6-dioxa-9-azaspiro[4.5]decane.

In a preferred embodiment, the present invention provides a compound of formula (I) as described herein, or a pharmaceutically acceptable salt thereof, wherein:
- A: is selected from 5- to 14-membered heteroaryl and C₆-C₁₄-aryl;
- B: is 3- to 14-membered heterocyclyl;
- L: is a covalent bond;
- R⁸: is selected from hydrogen, amino, hydroxy, C₁-C₆-alkyl-NH-, amino-C₁-C₆-alkyl-NH-, hydroxy-C₁-C₆-alkyl-, hydroxy-C₁-C₆-alkyl-NH-, C₁-C₆-alkoxy-C₁-C₆-alkyl-NH-, carbamoyl-C₁-C₆-alkyl-, (C₁-C₆-alkyl)₂NC(O)-, and a group 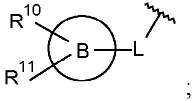
- R⁹: is selected from hydrogen and halogen;
- R¹⁰: is amino; and
- R¹¹: is hydrogen.

In a particularly preferred embodiment, the present invention provides a compound of formula (I) as described herein, or a pharmaceutically acceptable salt thereof, wherein:
- A: is selected from pyridyl, 1H-pyrrolo[3,2-b]pyridine, 1H-pyrrolo[3,2-c]pyridine, 2H-pyrazolo[4,3-c]pyridine, and phenyl;
- B: is pyrrolidine;
- L: is a covalent bond;
- R⁸: is selected from hydrogen, hydroxy, amino, CH₃-NH-, amino-(CH₂)₃-NH-, hydroxymethyl, 2-hydroxyethyl, hydroxy-(CH₂)₂-NH-, methoxy-(CH₂)₂-NH-, H₂N-C(O)-CH₂-, (CH₃)₂N-C(O)-, and a group 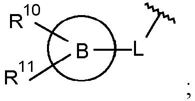
- R⁹: is selected from hydrogen and fluoro;
- R¹⁰: is amino; and
- R¹¹: is hydrogen.

In a preferred embodiment, the present invention provides a compound of formula (I) as described herein, or a pharmaceutically acceptable salt thereof, wherein X is N.

In a preferred embodiment, the present invention provides a compound of formula (I) as described herein, or a pharmaceutically acceptable salt thereof, wherein:
- R¹²: is selected from chloro and methyl; and
- R¹³: is selected from CHF₂ and CF₃.

In a particularly preferred embodiment, the present invention provides a compound of formula (I) as described herein, or a pharmaceutically acceptable salt thereof, wherein:
- R¹²: is selected from chloro and methyl; and
- R¹³: is CF₃.

In a particularly preferred embodiment, the present invention provides a compound of formula (I) as described herein, or a pharmaceutically acceptable salt thereof, wherein R¹² is selected from chloro and methyl.

In a particularly preferred embodiment, the present invention provides a compound of formula (I) as described herein, or a pharmaceutically acceptable salt thereof, wherein R¹² is chloro.

In a particularly preferred embodiment, the present invention provides a compound of formula (I) as described herein, or a pharmaceutically acceptable salt thereof, wherein R¹² is methyl.

In a particularly preferred embodiment, the present invention provides a compound of formula (I) as described herein, or a pharmaceutically acceptable salt thereof, wherein R¹³ is CF₃.

In one embodiment, the present invention provides a compound of formula (I) as described herein, or a pharmaceutically acceptable salt thereof, wherein:
- X and Y: are each independently selected from N and CH;
- n: is selected from 0 to 6;
- A: is selected from 5- to 14-membered heteroaryl and C₆-C₁₄-aryl;
- B: is selected from 3- to 14-membered heterocyclyl, 5- to 14-membered heteroaryl and C₃-C₁₀-cycloalkyl;
- L: is selected from a covalent bond, carbonyl, NHC(O), CH₂NH and NH;
- L¹: is -O- or a covalent bond;
- R¹: is selected from
(i) C₁-C₆-alkyl substituted with one amino and one carboxy substituent;
(ii) C₁-C₆-alkyl substituted with one amino and one carboxy-C₁-C₆-alkyl-NH-substituent;
(iii) C₁-C₆-alkyl-N(R^{1a}R^{1b});
(iv) -L¹-C₁-C₆-alkyl-N⁺(R^{1c}R^{1d}R^{1e});
(v) a group 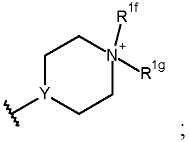 and
(vi) a group 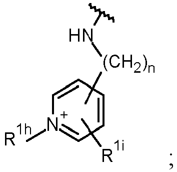
- R^{1a}: is amino-C₁-C₆-alkyl;
- R^{1b}: is carboxy-C₁-C₆-alkyl;
- R^{1c}: is selected from amino-C₁-C₆-alkyl and (3- to 14-membered heterocyclyl)-C₁-C₆-alkyl-;
- R^{1d}: is carboxy-C₁-C₆-alkyl;
- R^{1e}: is selected from C₁-C₆-alkyl, amino-C₁-C₆-alkyl and (3- to 14-membered heterocyclyl)-C₁-C₆-alkyl-;
- R^{1f}: is selected from C₁-C₆-alkyl, hydroxy-C₁-C₆-alkyl, amino-C₁-C₆-alkyl, (C₁-C₆-alkyl)₂N-C₁-C₆-alkyl, and (3- to 14-membered heterocyclyl)-C₁-C₆-alkyl-;
- R^{1g}: is carboxy-C₁-C₆-alkyl;
- R^{1h}: is carboxy-C₁-C₆-alkyl;
- R¹ⁱ: is amino-C₁-C₆-alkyl;
- R²: is selected from hydrogen, C₁-C₆-alkyl and C₁-C₆-alkoxy; and R³ and R⁷ are both hydrogen; or
- R² and R⁷,: taken together with the atoms to which they are attached, form a 4- to 14-membered heterocycle or a C₄-C₁₀-cycloalkyl; and R³ is hydrogen; or
- R² and R³,: taken together with the carbon atom to which they are attached, form a C₃-C₁₀-cycloalkyl; and R⁷ is hydrogen;
- R⁴: is selected from hydrogen and C₁-C₆-alkyl; and R⁵ and R⁶ are both hydrogen; or
- R⁴ and R⁶,: taken together with the atoms to which they are attached, form a 4- to 14-membered heterocycle; and R⁵ is hydrogen; or
- R⁴ and R⁵,: taken together with the carbon atom to which they are attached, form a C₃-C₁₀-cycloalkyl; and R⁶ is hydrogen;
- R⁸: is selected from hydrogen, cyano, amino, hydroxy, C₁-C₆-alkyl-NH-, (C₁-C₆-alkyl)₂N-, (C₁-C₆-alkyl)₂N-SO₂-, (C₁-C₆-alkyl)₂N-C₁-C₆-alkyl-, amino-C₁-C₆-alkyl, amino-C₁-C₆-alkoxy, amino-C₁-C₆-alkyl-NH-, (amino-C₁-C₆-alkyl)₂N-, (amino-C₁-C₆-alkyl)₂N-C₁-C₆-alkyl-NH-, C₁-C₆-alkyl-NH-C₁-C₆-alkyl-NH-, C₁-C₆-alkoxy-C₁-C₆-alkyl-NH-, halo-C₁-C₆-alkoxy-C₁-C₆-alkyl-NH-, C₃-C₁₀-cycloalkyl-NH-, C₁-C₆-alkoxy, C₁-C₆-alkyl, cyano-C₁-C₆-alkyl-, hydroxy-C₁-C₆-alkyl-, hydroxy-C₁-C₆-alkyl-NH-, hydroxy-C₁-C₆-alkyl-NHC(O)-, halo-hydroxy-C₁-C₆-alkyl-NHC(O)-, carbamoyl, hydroxycarbamoyl, C₁-C₆-alkyl-NHC(O)-, (C₁-C₆-alkyl)₂NC(O)-, carbamoyl-C₁-C₆-alkyl-, and a group 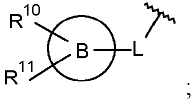
- R⁹: is selected from hydrogen, halogen amino, C₁-C₆-alkyl, and hydroxy-C₁-C₆-alkyl-;
- R¹⁰: is selected from hydrogen, C₁-C₆-alkyl, halogen, hydroxy, oxo, imino, and amino;
- R¹¹: is selected from hydrogen and halogen;
- R¹²: is selected from halogen and C₁-C₆-alkyl;
- R¹³: is halo-C₁-C₆-alkyl.

In a preferred embodiment, the present invention provides a compound of formula (I) as described herein, or a pharmaceutically acceptable salt thereof, wherein:
- A: is selected from 5- to 14-membered heteroaryl and C₆-C₁₄-aryl;
- B: is 3- to 14-membered heterocyclyl;
- L: is a covalent bond;
- X: is N;
- Y: is CH;
- R¹: is selected from
(i) C₁-C₆-alkyl-N⁺(R^{1c}R^{1d}R^{1e}); and
(ii) a group 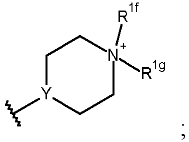
- R^{1c} and R^{1e}: are both amino-C₁-C₆-alkyl;
- R^{1d} and R^{1g}: are both carboxy-C₁-C₆-alkyl;
- R^{1f}: is selected from amino-C₁-C₆-alkyl and (3- to 14-membered heterocyclyl)-C₁-C₆-alkyl-;
- R², R³, R⁴, R⁵, R⁶ and R⁷: are all hydrogen;
- R⁸: is selected from hydrogen, amino, hydroxy, C₁-C₆-alkyl-NH-, amino-C₁-C₆-alkyl-NH-, hydroxy-C₁-C₆-alkyl-, hydroxy-C₁-C₆-alkyl-NH-, C₁-C₆-alkoxy-C₁-C₆-alkyl-NH-, carbamoyl-C₁-C₆-alkyl-, (C₁-C₆-alkyl)₂NC(O)-, and a group 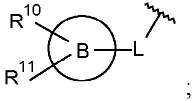
- R⁹: is selected from hydrogen and halogen;
- R¹⁰: is amino;
- R¹¹: is hydrogen;
- R¹²: is selected from halogen and C₁-C₆-alkyl; and
- R¹³: is halo-C₁-C₆-alkyl.

In a particularly preferred embodiment, the present invention provides a compound of formula (I) as described herein, or a pharmaceutically acceptable salt thereof, wherein:
- A: is selected from pyridyl, 1H-pyrrolo[3,2-b]pyridine, 1H-pyrrolo[3,2-c]pyridine, 2H-pyrazolo[4,3-c]pyridine, and phenyl;
- B: is pyrrolidine;
- L: is a covalent bond;
- R¹: is selected from
(i) -(CH₂)₃-N⁺(R^{1c}R^{1d}R^{1e}); and
(ii) a group 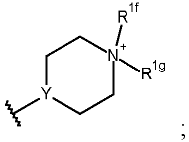
- R^{1c} and R^{1e}: are both 4-aminobutyl;
- R^{1d} and R^{1g}: are both carboxymethyl;
- R^{1f}: is selected from 3-aminopropyl and azetidin-3-ylmethyl;
- R⁸: is selected from hydrogen, hydroxy, amino, CH₃-NH-, amino-(CH₂)₃-NH-, hydroxymethyl, 2-hydroxyethyl, hydroxy-(CH₂)₂-NH-, methoxy-(CH₂)₂-NH-, H₂N-C(O)-CH₂-, (CH₃)₂N-C(O)-, and a group 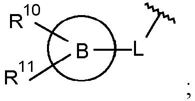
- R⁹: is selected from hydrogen and fluoro;
- R¹⁰: is amino;
- R¹¹: is hydrogen;
- R¹²: is selected from chloro and methyl; and
- R¹³: is CF₃.

In a preferred embodiment, the present invention provides a compound of formula (I) as described herein, or a pharmaceutically acceptable salt thereof, wherein A is selected from 5- to 14-membered heteroaryl and C₆-C₁₄-aryl.

In a preferred embodiment, the present invention provides a compound of formula (I) as described herein, or a pharmaceutically acceptable salt thereof, wherein B is 3- to 14-membered heterocyclyl.

In a preferred embodiment, the present invention provides a compound of formula (I) as described herein, or a pharmaceutically acceptable salt thereof, wherein L is a covalent bond.

In a preferred embodiment, the present invention provides a compound of formula (I) as described herein, or a pharmaceutically acceptable salt thereof, wherein X is N.

In a preferred embodiment, the present invention provides a compound of formula (I) as described herein, or a pharmaceutically acceptable salt thereof, wherein Y is CH.

In a preferred embodiment, the present invention provides a compound of formula (I) as described herein, or a pharmaceutically acceptable salt thereof, wherein:
- R¹: is selected from
(i) C₁-C₆-alkyl-N⁺(R^{1c}R^{1d}R^{1e}); and
(ii) a group

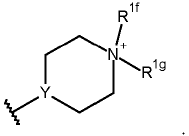

In a preferred embodiment, the present invention provides a compound of formula (I) as described herein, or a pharmaceutically acceptable salt thereof, wherein R^{1c} is amino-C₁-C₆-alkyl.

In a preferred embodiment, the present invention provides a compound of formula (I) as described herein, or a pharmaceutically acceptable salt thereof, wherein R^{1e} is amino-C₁-C₆-alkyl.

In a preferred embodiment, the present invention provides a compound of formula (I) as described herein, or a pharmaceutically acceptable salt thereof, wherein R^{1f} is selected from amino-C₁-C₆-alkyl and (3- to 14-membered heterocyclyl)-C₁-C₆-alkyl-.

In a preferred embodiment, the present invention provides a compound of formula (I) as described herein, or a pharmaceutically acceptable salt thereof, wherein R² is hydrogen.

In a preferred embodiment, the present invention provides a compound of formula (I) as described herein, or a pharmaceutically acceptable salt thereof, wherein R³ is hydrogen.

In a preferred embodiment, the present invention provides a compound of formula (I) as described herein, or a pharmaceutically acceptable salt thereof, wherein R⁴ is hydrogen.

In a preferred embodiment, the present invention provides a compound of formula (I) as described herein, or a pharmaceutically acceptable salt thereof, wherein R⁵ is hydrogen.

In a preferred embodiment, the present invention provides a compound of formula (I) as described herein, or a pharmaceutically acceptable salt thereof, wherein R⁶ is hydrogen.

In a preferred embodiment, the present invention provides a compound of formula (I) as described herein, or a pharmaceutically acceptable salt thereof, wherein R⁷ is hydrogen.

In a preferred embodiment, the present invention provides a compound of formula (I) as described herein, or a pharmaceutically acceptable salt thereof, wherein R⁸ is selected from hydrogen, amino, hydroxy, C₁-C₆-alkyl-NH-, amino-C₁-C₆-alkyl-NH-, hydroxy-C₁-C₆-alkyl-, hydroxy-C₁-C₆-alkyl-NH-, C₁-C₆-alkoxy-C₁-C₆-alkyl-NH-, carbamoyl-C₁-C₆-alkyl-, (C₁-C₆-alkyl)₂NC(O)-, and a group

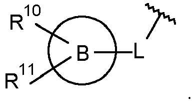

In a preferred embodiment, the present invention provides a compound of formula (I) as described herein, or a pharmaceutically acceptable salt thereof, wherein R⁹ is selected from hydrogen and halogen.

In a preferred embodiment, the present invention provides a compound of formula (I) as described herein, or a pharmaceutically acceptable salt thereof, wherein R¹⁰ is amino.

In a preferred embodiment, the present invention provides a compound of formula (I) as described herein, or a pharmaceutically acceptable salt thereof, wherein R¹¹ is hydrogen.

In a particularly preferred embodiment, the present invention provides a compound of formula (I) as described herein, or a pharmaceutically acceptable salt thereof, wherein A is selected from pyridyl, 1H-pyrrolo[3,2-b]pyridine, 1H-pyrrolo[3,2-c]pyridine, 2H-pyrazolo[4,3-c]pyridine, and phenyl.

In a particularly preferred embodiment, the present invention provides a compound of formula (I) as described herein, or a pharmaceutically acceptable salt thereof, wherein A is pyridyl.

In a particularly preferred embodiment, the present invention provides a compound of formula (I) as described herein, or a pharmaceutically acceptable salt thereof, wherein A is 1H-pyrrolo[3,2-b]pyridine.

In a particularly preferred embodiment, the present invention provides a compound of formula (I) as described herein, or a pharmaceutically acceptable salt thereof, wherein A is 1H-pyrrolo[3,2-c]pyridine.

In a particularly preferred embodiment, the present invention provides a compound of formula (I) as described herein, or a pharmaceutically acceptable salt thereof, wherein A is 2H-pyrazolo[4,3-c]pyridine.

In a particularly preferred embodiment, the present invention provides a compound of formula (I) as described herein, or a pharmaceutically acceptable salt thereof, wherein A is phenyl.

In a particularly preferred embodiment, the present invention provides a compound of formula (I) as described herein, or a pharmaceutically acceptable salt thereof, wherein B is pyrrolidine.

In a particularly preferred embodiment, the present invention provides a compound of formula (I) as described herein, or a pharmaceutically acceptable salt thereof, wherein
- R¹: is selected from
(i) -(CH₂)₃-N⁺(R^{1c}R^{1d}R^{1e}); and
(ii) a group

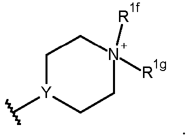

In a particularly preferred embodiment, the present invention provides a compound of formula (I) as described herein, or a pharmaceutically acceptable salt thereof, wherein R^{1c} and R^{1e} are both 4-aminobutyl.

In a particularly preferred embodiment, the present invention provides a compound of formula (I) as described herein, or a pharmaceutically acceptable salt thereof, wherein R^{1d} is carboxymethyl.

In a particularly preferred embodiment, the present invention provides a compound of formula (I) as described herein, or a pharmaceutically acceptable salt thereof, wherein R^{1g} is carboxymethyl.

In a particularly preferred embodiment, the present invention provides a compound of formula (I) as described herein, or a pharmaceutically acceptable salt thereof, wherein R^{1f} is selected from 3-aminopropyl and azetidin-3-ylmethyl.

In a particularly preferred embodiment, the present invention provides a compound of formula (I) as described herein, or a pharmaceutically acceptable salt thereof, wherein R⁸ is selected from hydrogen, hydroxy, amino, CH₃-NH-, amino-(CH₂)₃-NH-, hydroxymethyl, 2-hydroxyethyl, hydroxy-(CH₂)₂-NH-, methoxy-(CH₂)₂-NH-, H₂N-C(O)-CH₂-, (CH₃)₂N-C(O)-, and a group

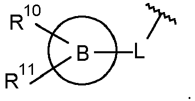

In a particularly preferred embodiment, the present invention provides a compound of formula (I) as described herein, or a pharmaceutically acceptable salt thereof, wherein R⁹ is selected from hydrogen and fluoro.

In one embodiment, the present invention provides a compound of formula (I), wherein the compound of formula (I) is a compound of formula (Ia) 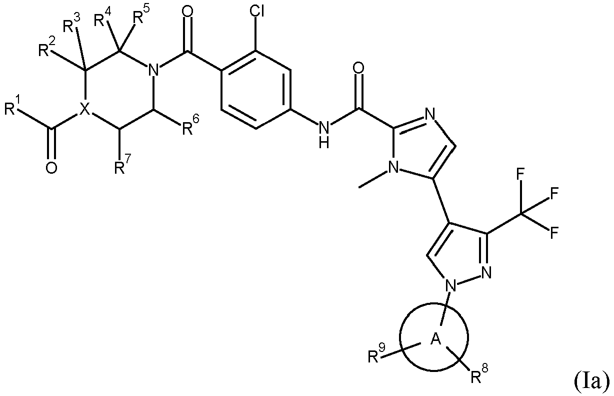 or a pharmaceutically acceptable salt thereof, wherein:
- X and Y: are each independently selected from N and CH;
- n: is selected from 0 to 6;
- A: is selected from 5- to 14-membered heteroaryl and C₆-C₁₄-aryl;
- B: is selected from 3- to 14-membered heterocyclyl, 5- to 14-membered heteroaryl and C₃-C₁₀-cycloalkyl;
- L: is selected from a covalent bond, CH₂NH, NHCH₂ and NH;
- R¹: is selected from
(i) C₁-C₆-alkyl substituted with one amino and one carboxy substituent;
(ii) C₁-C₆-alkyl substituted with one amino and one carboxy-C₁-C₆-alkyl-NH-substituent;
(iii) C₁-C₆-alkyl-N(R^{1a}R^{1b});
(iv) C₁-C₆-alkyl-N⁺(R^{1c}R^{1d}R^{1e});
(v) a group 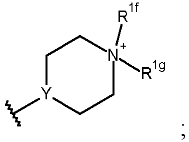 and
(vi) a group 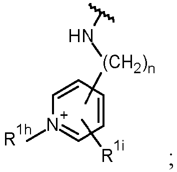
- R^{1a}: is amino-C₁-C₆-alkyl;
- R^{1b}: is carboxy-C₁-C₆-alkyl;
- R^{1c}: is selected from amino-C₁-C₆-alkyl and (3- to 14-membered heterocyclyl)-C₁-C₆-alkyl-;
- R^{1d}: is carboxy-C₁-C₆-alkyl;
- R^{1e}: is selected from C₁-C₆-alkyl, amino-C₁-C₆-alkyl and (3- to 14-membered heterocyclyl)-C₁-C₆-alkyl-;
- R^{1f}: is selected from amino-C₁-C₆-alkyl and (3- to 14-membered heterocyclyl)-C₁-C₆-alkyl-;
- R^{1g}: is carboxy-C₁-C₆-alkyl;
- R^{1h}: is carboxy-C₁-C₆-alkyl;
- R¹ⁱ: is amino-C₁-C₆-alkyl;
- R²: is selected from hydrogen, C₁-C₆-alkyl and C₁-C₆-alkoxy; and R³ and R⁷ are both hydrogen; or
- R² and R⁷,: taken together with the atoms to which they are attached, form a 4- to 14-membered heterocycle or a C₄-C₁₀-cycloalkyl; and R³ is hydrogen; or
- R² and R³,: taken together with the carbon atom to which they are attached, form a C₃-C₁₀-cycloalkyl; and R⁷ is hydrogen;
- R⁴: is selected from hydrogen and C₁-C₆-alkyl; and R⁵ and R⁶ are both hydrogen; or
- R⁴ and R⁶,: taken together with the atoms to which they are attached, form a 4- to 14-membered heterocycle; and R⁵ is hydrogen; or
- R⁴ and R⁵,: taken together with the carbon atom to which they are attached, form a C₃-C₁₀-cycloalkyl; and R⁶ is hydrogen;
- R⁸: is selected from hydrogen, cyano, amino, C₁-C₆-alkyl-NH-, (C₁-C₆-alkyl)₂N-, (C₁-C₆-alkyl)₂N-C₁-C₆-alkyl-, amino-C₁-C₆-alkyl, amino-C₁-C₆-alkoxy, amino-C₁-C₆-alkyl-NH-, C₁-C₆-alkyl-NH-C₁-C₆-alkyl-NH-, C₁-C₆-alkoxy-C₁-C₆-alkyl-NH-, C₃-C₁₀-cycloalkyl-NH-, C₁-C₆-alkoxy, C₁-C₆-alkyl, hydroxy-C₁-C₆-alkyl-, hydroxy-C₁-C₆-alkyl-NH- and a group 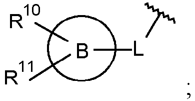
- R⁹: is selected from hydrogen, halogen and amino;
- R¹⁰: is selected from hydrogen, halogen and amino; and
- R¹¹: is selected from hydrogen and halogen.

In a preferred embodiment, the present invention provides a compound of formula (Ia) as described herein, or a pharmaceutically acceptable salt thereof, wherein said compound of formula (I) is a compound of formula (II) 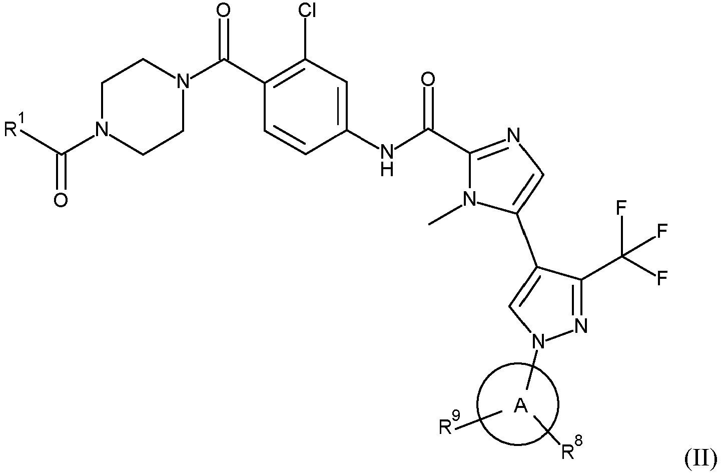 wherein R¹, R⁸, R⁹ and A are as described herein.

In one embodiment, the present invention provides a compound of formula (Ia) as described herein, or a pharmaceutically acceptable salt thereof, wherein L is selected from a covalent bond, CH₂NH and NH.

In one embodiment, the present invention provides a compound of formula (Ia) as described herein, wherein B is selected from morpholine, piperazine, pyrrolidinyl, piperidyl, pyridyl, cyclopropyl and cyclopentyl.

In one embodiment, the present invention provides a compound of formula (Ia) as described herein, or a pharmaceutically acceptable salt thereof, wherein:
- L: is selected from a covalent bond, CH₂NH and NH; and
- B: is selected from morpholinyl, piperazinyl, pyrrolidinyl, piperidyl, pyridyl, cyclopropyl and cyclopentyl.

In a preferred embodiment, the present invention provides a compound of formula (Ia) as described herein, or a pharmaceutically acceptable salt thereof, wherein:
- R¹: is selected from
(i) C₁-C₆-alkyl-N⁺(R^{1c}R^{1d}R^{1e}); and
(ii) a group 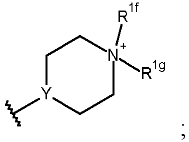 wherein
- Y: is CH;
- R^{1c} and R^{1e}: are both amino-C₁-C₆-alkyl;
- R^{1d} and R^{1g}: are both carboxy-C₁-C₆-alkyl; and
- R^{1f}: is selected from amino-C₁-C₆-alkyl and (3- to 14-membered heterocyclyl)-C₁-C₆-alkyl-.

In a particularly preferred embodiment, the present invention provides a compound of formula (Ia) as described herein, or a pharmaceutically acceptable salt thereof, wherein:
- R¹: is selected from
(i) -(CH₂)₃-N⁺(R^{1c}R^{1d}R^{1e}); and
(ii) a group 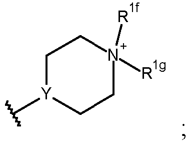 wherein
- Y: is CH;
- R^{1c} and R^{1e}: are both 4-aminobutyl;
- R^{1d} and R^{1g}: are both carboxymethyl; and
- R^{1f}: is selected from 3-aminopropyl and azetidin-3-ylmethyl.

In a particularly preferred embodiment, the present invention provides a compound of formula (Ia) as described herein, or a pharmaceutically acceptable salt thereof, wherein R², R³, R⁴, R⁵, R⁶ and R⁷ are all hydrogen.

In a preferred embodiment, the present invention provides a compound of formula (Ia) as described herein, or a pharmaceutically acceptable salt thereof, wherein A is selected from thiazolyl; isothiazolyl; pyridyl; 1H-pyrrolo[3,2-b]pyridine; 1H-imidazo[4,5-b]pyridine; 1,3-benzothiazole; benzothiophene; 1H-pyrazolo[4,3-b]pyridine; 1H-pyrrolo[3,2-c]pyridine; 1H-indazolyl; furo[3,2-b]pyridine; 2,3-dihydro-1H-pyrrolo[3,2-b]pyridine; and phenyl.

In a particularly preferred embodiment, the present invention provides a compound of formula (Ia) as described herein, or a pharmaceutically acceptable salt thereof, wherein A is selected from pyridyl, 1H-pyrrolo[3,2-b]pyridine, 1H-pyrrolo[3,2-c]pyridine and phenyl.

In a preferred embodiment, the present invention provides a compound of formula (Ia) as described herein, or a pharmaceutically acceptable salt thereof, wherein:
- R⁸: is selected from hydrogen, amino, C₁-C₆-alkyl-NH-, amino-C₁-C₆-alkyl-NH-, hydroxy-C₁-C₆-alkyl- and hydroxy-C₁-C₆-alkyl-NH-; and
- R⁹: is selected from hydrogen and halogen.

In a particularly preferred embodiment, the present invention provides a compound of formula (Ia) as described herein, or a pharmaceutically acceptable salt thereof, wherein:
- R⁸: is selected from hydrogen, amino, CH₃-NH-, amino-(CH₂)₃-NH-, hydroxymethyl and hydroxy-(CH₂)₂-NH-; and
- R⁹: is selected from hydrogen and fluoro.

In a preferred embodiment, the present invention provides a compound of formula (Ia) as described herein, or a pharmaceutically acceptable salt thereof, wherein:
- A: is selected from 5- to 14-membered heteroaryl and C₆-C₁₄-aryl;
- R⁸: is selected from hydrogen, amino, C₁-C₆-alkyl-NH-, amino-C₁-C₆-alkyl-NH-, hydroxy-C₁-C₆-alkyl- and hydroxy-C₁-C₆-alkyl-NH-; and
- R⁹: is selected from hydrogen and halogen.

In a particularly preferred embodiment, the present invention provides a compound of formula (Ia) as described herein, or a pharmaceutically acceptable salt thereof, wherein:
- A: is selected from pyridyl, 1H-pyrrolo[3,2-b]pyridine, 1H-pyrrolo[3,2-c]pyridine and phenyl;
- R⁸: is selected from hydrogen, amino, CH₃-NH-, amino-(CH₂)₃-NH-, hydroxymethyl and hydroxy-(CH₂)₂-NH-; and
- R⁹: is selected from hydrogen and fluoro.

In a preferred embodiment, the present invention provides a compound of formula (Ia) as described herein, or a pharmaceutically acceptable salt thereof, wherein X is N.

In one embodiment, the present invention provides a compound of formula (Ia) as described herein, or a pharmaceutically acceptable salt thereof, wherein:
- X and Y: are each independently selected from N and CH;
- n: is selected from 0 to 6;
- A: is selected from 5- to 14-membered heteroaryl and C₆-C₁₄-aryl;
- B: is selected from 3- to 14-membered heterocyclyl, 5- to 14-membered heteroaryl and C₃-C₁₀-cycloalkyl;
- L: is selected from a covalent bond, CH₂NH and NH;
- R¹: is selected from
(i) C₁-C₆-alkyl substituted with one amino and one carboxy substituent;
(ii) C₁-C₆-alkyl substituted with one amino and one carboxy-C₁-C₆-alkyl-NH-substituent;
(iii) C₁-C₆-alkyl-N(R^{1a}R^{1b});
(iv) C₁-C₆-alkyl-N⁺(R^{1c}R^{1d}R^{1e});
(v) **a group** 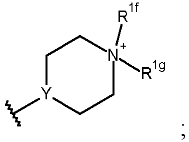 and
(vi) a group 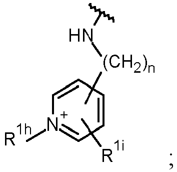
- R^{1a}: is amino-C₁-C₆-alkyl;
- R^{1b}: is carboxy-C₁-C₆-alkyl;
- R^{1c}: is selected from amino-C₁-C₆-alkyl and (3- to 14-membered heterocyclyl)-C₁-C₆-alkyl-;
- R^{1d}: is carboxy-C₁-C₆-alkyl;
- R^{1e}: is selected from C₁-C₆-alkyl, amino-C₁-C₆-alkyl and (3- to 14-membered heterocyclyl)-C₁-C₆-alkyl-;
- R^{1f}: is selected from amino-C₁-C₆-alkyl and (3- to 14-membered heterocyclyl)-C₁-C₆-alkyl-;
- R^{1g}: is carboxy-C₁-C₆-alkyl;
- R^{1h}: is carboxy-C₁-C₆-alkyl;
- R¹ⁱ: is amino-C₁-C₆-alkyl;
- R²: is selected from hydrogen, C₁-C₆-alkyl and C₁-C₆-alkoxy; and R³ and R⁷ are both hydrogen; or
- R² and R⁷,: taken together with the atoms to which they are attached, form a 4- to 14-membered heterocycle or a C₄-C₁₀-cycloalkyl; and R³ is hydrogen; or
- R² and R³,: taken together with the carbon atom to which they are attached, form a C₃-C₁₀-cycloalkyl; and R⁷ is hydrogen;
- R⁴: is selected from hydrogen and C₁-C₆-alkyl; and R⁵ and R⁶ are both hydrogen; or
- R⁴ and R⁶,: taken together with the atoms to which they are attached, form a 4- to 14-membered heterocycle; and R⁵ is hydrogen; or
- R⁴ and R⁵,: taken together with the carbon atom to which they are attached, form a C₃-C₁₀-cycloalkyl; and R⁶ is hydrogen;
- R⁸: is selected from hydrogen, cyano, amino, C₁-C₆-alkyl-NH-, (C₁-C₆-alkyl)₂N-, (C₁-C₆-alkyl)₂N-C₁-C₆-alkyl-, amino-C₁-C₆-alkyl, amino-C₁-C₆-alkoxy, amino-C₁-C₆-alkyl-NH-, C₁-C₆-alkyl-NH-C₁-C₆-alkyl-NH-, C₁-C₆-alkoxy-C₁-C₆-alkyl-NH-, C₃-C₁₀-cycloalkyl-NH-, C₁-C₆-alkoxy, C₁-C₆-alkyl, hydroxy-C₁-C₆-alkyl-, hydroxy-C₁-C₆-alkyl-NH- and a group 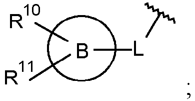
- R⁹: is selected from hydrogen, halogen and amino;
- R¹⁰: is selected from hydrogen, halogen and amino; and
- R¹¹: is selected from hydrogen and halogen.

In a preferred embodiment, the present invention provides a compound of formula (Ia) as described herein, or a pharmaceutically acceptable salt thereof, wherein:
- X: is N;
- Y: is CH;
- A: is selected from 5- to 14-membered heteroaryl and C₆-C₁₄-aryl;
- R¹: is selected from
(i) C₁-C₆-alkyl-N⁺(R^{1c}R^{1d}R^{1e}); and
(ii) a group 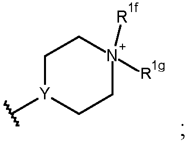
- R^{1c} and R^{1e}: are both amino-C₁-C₆-alkyl;
- R^{1d} and R^{1g}: are both carboxy-C₁-C₆-alkyl;
- R^{1f}: is selected from amino-C₁-C₆-alkyl and (3- to 14-membered heterocyclyl)-C₁-C₆-alkyl-;
- R², R³, R⁴, R⁵, R⁶ and R⁷: are all hydrogen;
- R⁸: is selected from hydrogen, amino, C₁-C₆-alkyl-NH-, amino-C₁-C₆-alkyl-NH-, hydroxy-C₁-C₆-alkyl- and hydroxy-C₁-C₆-alkyl-NH-; and
- R⁹: is selected from hydrogen and halogen.

In a preferred embodiment, the present invention provides a compound of formula (Ia) as described herein, or a pharmaceutically acceptable salt thereof, wherein:
- X: is N;
- Y: is CH;
- A: is selected from pyridyl, 1H-pyrrolo[3,2-b]pyridine, 1H-pyrrolo[3,2-c]pyridine and phenyl;
- R¹: is selected from
(i) -(CH₂)₃-N⁺(R^{1c}R^{1d}R^{1e}); and
(ii) a group 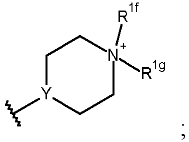
- R^{1c} and R^{1e}: are both 4-aminobutyl;
- R^{1d} and R^{1g}: are both carboxymethyl;
- R^{1f}: is selected from 3-aminopropyl and azetidin-3-ylmethyl;
- R⁸: is selected from hydrogen, amino, CH₃-NH-, amino-(CH₂)₃-NH-, hydroxymethyl and hydroxy-(CH₂)₂-NH-; and
- R⁹: is selected from hydrogen and fluoro.

In one embodiment, the present invention provides a compound of formula (I) as described herein, or a pharmaceutically acceptable salt thereof, wherein the compound of formula (I) is selected from:
bis(4-aminobutyl)-(carboxymethyl)-[4-[4-[2-chloro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazino]-4-keto-butyl]ammonium;
cis-2-[4-[4-[4-[[5-[1-[5-(2-aminoethylamino)-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
bis(4-aminobutyl)-[4-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl]piperazino]-4-keto-butyl]-(carboxymethyl)ammonium;
2-[4-[4-[4-[[5-[1-[5-(2-aminoethylamino)-2-pyridyl]-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
bis(3-aminopropyl)-(carboxymethyl)-[4-[4-[2-chloro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazino]-4-keto-butyl]ammonium;
cis-2-[1-(azetidin-3-ylmethyl)-4-[4-[2-chloro-4-[[1-methyl-5-[1-(1H-pyrrolo[3,2-c]pyridin-6-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[4-[4-[4-[[5-[1-[5-(3-aminopropylamino)-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
cis-2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[1-(azetidin-3-ylmethyl)-4-[4-[2-chloro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
2-[4-[4-[4-[[5-[1-[5-[[(2S)-2-aminopropyl]amino]-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
bis(4-aminobutyl)-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyl]-(carboxymethyl)ammonium;
2-[4-[4-[4-[[5-[1-[5-(aminomethyl)-2-pyridyl]-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[1-(azetidin-3-ylmethyl)-4-[4-[2-chloro-4-[[5-[1-(5-methoxy-2-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[4-[4-[4-[[5-[1-[5-(2-aminoethyl)-2-pyridyl]-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[4-[4-[4-[[5-[1-[5-(2-aminoethoxy)-2-pyridyl]-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[1-(azetidin-3-ylmethyl)-4-[4-[2-chloro-4-[[1-methyl-5-[1-[5-(2-pyridylmethylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[4-[4-[4-[[5-[1-[5-[(1-aminocyclopentyl)methylamino]-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
2-[1-(azetidin-3-ylmethyl)-4-[4-[2-chloro-4-[[1-methyl-5-[1-[5-(methylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-(5-morpholino-2-pyridyl)-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-[5-[[(2S)-pyrrolidin-2-yl]methylamino]-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[5-(methylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid;
cis-2-[1-(azetidin-3-ylmethyl)-4-[4-[2-chloro-4-[[5-[1-[5-(2-methoxyethylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
trans-2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-(5-piperazino-2-pyridyl)-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-[5-[2-(methylamino)ethylamino]-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
cis-2-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
bis(3-aminopropyl)-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyl]-(carboxymethyl)ammonium;
bis(4-aminobutyl)-[4-[4-[4-[[5-[1-[5-(2-aminoethylamino)-2-pyridyl]-3-(trifluoromethyl)pyrazol-4-yl]-1-methyl-imidazole-2-carbonyl]amino]-2-chlorobenzoyl]piperazino]-4-keto-butyl]-(carboxymethyl)ammonium;
2-[1-(azetidin-3-ylmethyl)-4-[4-[2-chloro-4-[[5-[1-(4-methoxyphenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[5-(2-hydroxyethylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(1-methylpyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-[5-(isopropylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-[5-(2-pyridylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(1H-pyrazolo[4,3-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-thiazol-2-yl-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-[5-(cyclopropylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-[5-(dimethylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-[4-(dimethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-[5-[[(3S)-morpholin-3-yl]methylamino]-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid;
2-[1-(azetidin-3-ylmethyl)-4-[4-[2-chloro-4-[[5-[1-[6-(dimethylamino)-3-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
[4-[4-[4-[[5-[1-(5-aminopyridin-2-yl)-3-(trifluoromethyl)pyrazol-4-yl]-1-methylimidazole-2-carbonyl]amino]-2-chlorobenzoyl]piperazin-1-yl]-4-oxobutyl]-bis(azetidin-3-ylmethyl)-(carboxymethyl)azanium;
2-[4-[4-[4-[[5-[1-[5-(6-aminohexylamino)-2-pyridyl]-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[1-(azetidin-3-ylmethyl)-4-[4-[2-chloro-4-[[1-methyl-5-[1-[4-(methylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-[5-(4-methylpiperazino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-(5-hydroxy-2-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-(1H-imidazo[4,5-b]pyridin-5-yl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[4-[4-[4-[[5-[1-(6-amino-2-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid;
2-[1-(azetidin-3-ylmethyl)-4-[4-[2-chloro-4-[[1-methyl-5-[1-[5-(4-piperidylmethylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-[5-[[(2S,4S)-4-fluoropyrrolidin-2-yl]methylamino]-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[4-[4-[4-[[5-[1-(5-amino-3-fluoro-2-pyridyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
4-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]butyric acid;
2-[1-(azetidin-3-ylmethyl)-4-[4-[2-chloro-4-[[5-[1-(2,3-dihydro-1H-pyrrolo[3,2-b]pyridin-5-yl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-(1H-indazol-3-yl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-furo[3,2-b]pyridin-5-yl-3-(trifluoromethyl)pyrazol-4-yl]-1-methyl-imidazole-2-carbonyl]amino]benzoyl]piperazine-1-carbonyl]piperidin-1-ium-1-yl]acetic acid;
trans-2-[4-[4-[4-[[5-[1-(5-amino-2-pyridyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[1-(azetidin-3-ylmethyl)-4-[4-[2-chloro-4-[[5-[1-[5-[(2,2-difluorocyclopropyl)methylamino]-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
3-aminopropyl-[4-[4-[4-[[5-[1-(5-amino-2-pyridyl)-3-(trifluoromethyl)pyrazol-4-yl]-1-methylimidazole-2-carbonyl]amino]-2-chloro-benzoyl]piperazino]-4-keto-butyl]-(carboxymethyl)-methyl-ammonium;
bis(3-aminopropyl)-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyl]-(carboxymethyl)ammonium;
2-[1-(azetidin-3-ylmethyl)-4-[4-[2-chloro-4-[[5-[1-(5,6-diamino-2-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-[6-(methylamino)-3-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-[5-(cyclobutylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[2-(3-aminopropyl)-4-[3-[[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]amino]propyl]pyridin-1-ium-1-yl]acetic acid;
2-[4-[4-[4-[[5-[1-(4-amino-2-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
2-[3-aminopropyl-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyl]amino]acetic acid;
cis-2-[4-[4-[4-[[5-[1-[5-(2-aminoethylamino)-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
2-[4-[4-[4-[[5-[1-(2-amino-4-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
2-[1-(3-aminopropyl)-4-[1-[4-[[5-[1-(5-amino-2-pyridyl)-3-(trifluoromethyl)pyrazol-4-yl]-1-methyl-imidazole-2-carbonyl]amino]-2-chloro-benzoyl]piperidine-4-carbonyl]piperazin-1-ium-1-yl]acetic acid;
2-[1-(azetidin-3-ylmethyl)-4-[4-[2-chloro-4-[[5-[1-(5-cyano-2-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[4-[(2S)-4-[4-[[5-[1-(5-amino-2-pyridyl)-3-(trifluoromethyl)pyrazol-4-yl]-1-methyl-imidazole-2-carbonyl]amino]-2-chloro-benzoyl]-2-methyl-piperazine-1-carbonyl]-1-(azetidin-3-ylmethyl)piperidin-1-ium-1-yl]acetic acid;
2-[4-[(3R)-4-[4-[[5-[1-(5-amino-2-pyridyl)-3-(trifluoromethyl)pyrazol-4-yl]-1-methylimidazole-2-carbonyl]amino]-2-chloro-benzoyl]-3-ethyl-piperazine-1-carbonyl]-1-(azetidin-3-ylmethyl)piperidin-1-ium-1-yl]acetic acid;
2-[4-[(3S)-4-[4-[[5-[1-(5-amino-2-pyridyl)-3-(trifluoromethyl)pyrazol-4-yl]-1-methyl-imidazole-2-carbonyl]amino]-2-chloro-benzoyl]-3-methyl-piperazine-1-carbonyl]-1-(azetidin-3-ylmethyl)piperidin-1-ium-1-yl]acetic acid;
2-[1-(azetidin-3-ylmethyl)-4-[4-[4-[[5-[1-(1,3-benzothiazol-2-yl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid;
2-[[(5S)-5-amino-6-[4-[4-[[5-[1-(5-amino-2-pyridyl)-3-(trifluoromethyl)pyrazol-4-yl]-1-methylimidazole-2-carbonyl]amino]-2-chloro-benzoyl]piperazino]-6-keto-hexyl]amino]acetic acid;
2-[1-(azetidin-3-ylmethyl)-4-[4-[2-chloro-4-[[5-[1-(6-methoxy-3-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
(2S)-2-amino-4-[4-[4-[[5-[1-(5-amino-2-pyridyl)-3-(trifluoromethyl)pyrazol-4-yl]-1-methylimidazole-2-carbonyl]amino]-2-chloro-benzoyl]piperazino]-4-keto-butyric acid;
2-[1-(azetidin-3-ylmethyl)-4-[4-[2-chloro-4-[[5-[1-[5-[(dimethylamino)methyl]-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
(3R)-3-amino-4-[4-[4-[[5-[1-(5-amino-2-pyridyl)-3-(trifluoromethyl)pyrazol-4-yl]-1-methylimidazole-2-carbonyl]amino]-2-chloro-benzoyl]piperazino]-4-keto-butyric acid;
2-[4-[7-[4-[[5-[1-(5-amino-2-pyridyl)-3-(trifluoromethyl)pyrazol-4-yl]-1-methyl-imidazole-2-carbonyl]amino]-2-chloro-benzoyl]-4,7-diazaspiro[2.5]octane-4-carbonyl]-1-(azetidin-3-ylmethyl)piperidin-1-ium-1-yl]acetic acid;
2-[4-[1-[4-[[5-[1-(5-amino-2-pyridyl)-3-(trifluoromethyl)pyrazol-4-yl]-1-methyl-imidazole-2-carbonyl]amino]-2-chloro-benzoyl]piperidine-4-carbonyl]-1-(azetidin-3-ylmethyl)piperazin-1-ium-1-yl]acetic acid;
2-[1-(azetidin-3-ylmethyl)-4-[4-[2-chloro-4-[[1-methyl-5-[1-[2-(methylamino)-4-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[4-[4-[4-[[5-[1-(6-amino-3-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
2-[4-[8-[4-[[5-[1-(5-amino-2-pyridyl)-3-(trifluoromethyl)pyrazol-4-yl]-1-methyl-imidazole-2-carbonyl]amino]-2-chloro-benzoyl]-3,8-diazabicyclo[3.2.1]octane-3-carbonyl]-1-(azetidin-3-ylmethyl)piperidin-1-ium-1-yl]acetic acid;
(2S)-2-amino-5-[4-[4-[[5-[1-(5-amino-2-pyridyl)-3-(trifluoromethyl)pyrazol-4-yl]-1-methylimidazole-2-carbonyl]amino]-2-chloro-benzoyl]piperazino]-5-keto-valeric acid;
(3S)-3-amino-4-[4-[4-[[5-[1-(5-amino-2-pyridyl)-3-(trifluoromethyl)pyrazol-4-yl]-1-methylimidazole-2-carbonyl]amino]-2-chloro-benzoyl]piperazino]-4-keto-butyric acid;
2-[4-[4-[4-[[5-[1-[6-(2-aminoethylamino)-3-pyridyl]-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[4-[3-[4-[[5-[1-(5-amino-2-pyridyl)-3-(trifluoromethyl)pyrazol-4-yl]-1-methyl-imidazole-2-carbonyl]amino]-2-chloro-benzoyl]-3,9-diazabicyclo[3.3.1]nonane-9-carbonyl]-1-(azetidin-3-ylmethyl)piperidin-1-ium-1-yl]acetic acid;
2-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]-4,7-diazaspiro[2.5]octane-7-carbonyl]-1-(azetidin-3-ylmethyl)piperidin-1-ium-1-yl]acetic acid;
2-[4-[4-[4-[[5-[1-(4-aminoisothiazol-5-yl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
2-[1-(azetidin-3-ylmethyl)-4-[4-[4-[[5-[1-(benzothiophen-2-yl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid;
2-[2-(3-aminopropyl)-4-[3-[[1-[4-[[5-[1-(5-amino-2-pyridyl)-3-(trifluoromethyl)pyrazol-4-yl]-1-methyl-imidazole-2-carbonyl]amino]-2-chloro-benzoyl]piperidine-4-carbonyl]amino]propyl]pyridin-1-ium-1-yl]acetic acid;
2-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]-2-(methoxymethyl)piperazine-1-carbonyl]-1-(azetidin-3-ylmethyl)piperidin-1-ium-1-yl]acetic acid;
cis-2-[1-(3-aminopropyl)-4-[4-[2-chloro-4-[[5-[1-[2-(3-hydroxypropyl)-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[4-[4-[4-[[5-[1-[2-(3-amino-3-oxo-propyl)-1H-pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(3-aminopropyl)piperidin-1-ium-1-yl]acetic acid;
cis-2-[1-(3-aminopropyl)-4-[4-[2-chloro-4-[[5-[1-(2-fluoro-4-morpholino-phenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(3-aminopropyl)-4-[4-[4-[[5-[1-[2-[(1S)-1-hydroxyethyl]-1H-pyrrolo[3,2-b]pyridin-5-yl]-3-(trifluoromethyl)pyrazol-4-yl]-1-methyl-imidazole-2-carbonyl]amino]-2-methylbenzoyl]piperazine-1-carbonyl]piperidin-1-ium-1-yl]acetic acid;
cis-2-[1-(3-aminopropyl)-4-[4-[2-chloro-4-[[5-[1-[2-[2-hydroxy-1-(hydroxymethyl)ethyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(3-aminopropyl)-4-[4-[2-chloro-4-[[1-methyl-5-[1-[1-methyl-2-(morpholine-4-carbonyl)pyrrolo[3,2-b]pyridin-5-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(3-aminopropyl)-4-[4-[2-chloro-4-[[5-[1-[2-(2-hydroxyethylcarbamoyl)-1-methyl-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-[(1S)-1,2-dihydroxyethyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-[(1R)-1,2-dihydroxyethyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[4-[[5-[1-[2-fluoro-4-(methylamino)phenyl]-3-(trifluoromethyl)pyrazol-4-yl]-1-methyl-imidazole-2-carbonyl]amino]-2-methylbenzoyl]piperazine-1-carbonyl]piperidin-1-ium-1-yl]acetic acid;
cis-2-[1-(azetidin-3-ylmethyl)-4-[4-[2-fluoro-4-[[5-[1-[2-fluoro-4-(methylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[4-[[5-[1-[4-(dimethylamino)-2-fluoro-phenyl]-3-(trifluoromethyl)pyrazol-4-yl]-1-methyl-imidazole-2-carbonyl]amino]-2-fluorobenzoyl]piperazine-1-carbonyl]piperidin-1-ium-1-yl]acetic acid;
cis-2-[1-(3-aminopropyl)-4-[4-[4-[[5-[1-(2-fluoro-4-hydroxy-phenyl)-3-(trifluoromethyl)pyrazol-4-yl]-1-methyl-imidazole-2-carbonyl]amino]-2-methylbenzoyl]piperazine-1-carbonyl]piperidin-1-ium-1-yl]acetic acid;
cis-2-[1-(azetidin-3-ylmethyl)-4-[4-[4-[[5-[1-[2-fluoro-4-(2-methoxyethylamino)phenyl]-3-(trifluoromethyl)pyrazol-4-yl]-1-methyl-imidazole-2-carbonyl]amino]-2-methylbenzoyl]piperazine-1-carbonyl]piperidin-1-ium-1-yl]acetic acid;
cis-2-[4-[4-[4-[[5-[1-[2-[(3aS,6aR)-2,3,3a,4,6,6a-hexahydro-1H-pyrrolo[3,4-c]pyrrole-5-carbonyl]-1H-pyrrolo[3,2-c]pyridin-6-yl]-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[1-methyl-5-[1-[2-(7-oxa-2-azaspiro[3.5]nonane-2-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-(1-imino-1-keto-1,4-thiazinane-4-carbonyl)-1H-pyrrolo[3,2-c]pyridin-6-yl]-3-(trifluoromethyl)pyrazol-4-yl]-1-methylimidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-(2-hydroxyethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[2-(9-oxa-3,7-diazabicyclo[3.3.1]nonane-3-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[2-[(3,3,3-trifluoro-2-hydroxypropyl)carbamoyl]-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[2-(piperazine-1-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-(2,2-difluoromorpholine-4-carbonyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-[(2-ketopyrrolidin-3-yl)carbamoyl]-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-[(5R)-2,6-dioxa-9-azaspiro[4.5]decane-9-carbonyl]-1H-pyrrolo[3,2-c]pyridin-6-yl]-3-(trifluoromethyl)pyrazol-4-yl]-1-methylimidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[2-(thiomorpholine-4-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-(dimethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-fluoro-4-[[5-[1-[2-fluoro-4-(2-methoxyethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-(hydroxycarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[4-[2-(difluoromethoxy)ethylamino]-2-fluoro-phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(3-aminopropyl)-4-[4-[4-[[5-[1-[2-(2-hydroxyethyl)-1H-pyrrolo[3,2-b]pyridin-5-yl]-3-(trifluoromethyl)pyrazol-4-yl]-1-methyl-imidazole-2-carbonyl]amino]-2-methylbenzoyl]piperazine-1-carbonyl]piperidin-1-ium-1-yl]acetic acid;
cis-2-[4-[4-[4-[[5-[1-(4-Amino-2-fluoro-phenyl)-3-(difluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
cis-2-[4-[4-[4-[[5-[1-[2-(2-aminoethyl)-1H-pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
2-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl]piperazine-1-carbonyl]oxyethyl-bis(3-aminopropyl)-(carboxymethyl)ammonium;
cis-2-[1-(3-aminopropyl)-4-[4-[2-fluoro-4-[[5-[1-(2-fluoro-4-hydroxy-phenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-methyl-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-fluoro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
2-[1-(3-aminopropyl)-4-[1-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]isonipecotoyl]piperazin-1-ium-1-yl]acetic acid;
2-[4-[1-[4-[[5-[1-(4-amino-2-fluoro-phenyl)-3-(trifluoromethyl)pyrazol-4-yl]-1-methylimidazole-2-carbonyl]amino]-2-chloro-benzoyl]isonipecotoyl]-1-(3-aminopropyl)piperazin-1-ium-1-yl]acetic acid;
cis-2-[1-(azetidin-3-ylmethyl)-4-[4-[2-fluoro-4-[[5-[1-(2-fluoro-4-hydroxy-phenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[4-[[(2S)-2,3-dihydroxypropyl]amino]-2-fluoro-phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[4-[4-[4-[[5-[1-[1-(2-amino-1,1-dimethyl-2-oxo-ethyl)pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[1-methyl-5-[1-[2-(morpholine-4-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperazin-1-ium-1-yl]acetic acid;
2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-(3-aminopropyl)piperazin-1-ium-1-yl]acetic acid;
cis-2-[4-[4-[4-[[5-[1-[2-(2-aminoethyl)-1H-pyrrolo[3,2-c]pyridin-6-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[1-methyl-5-[1-[1-(methylcarbamoyl)pyrrolo[3,2-b]pyridin-5-yl]-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperazin-1-ium-1-yl]acetic acid;
2-[1-(3-aminopropyl)-4-[4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperazin-1-ium-1-yl]acetic acid;
cis-2-[1-(azetidin-3-ylmethyl)-4-[4-[2-chloro-4-[[5-[1-[3-(2-hydroxyethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[3-(piperazine-1-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[3-(4-methylpiperazine-1-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[4-[4-[4-[[5-[1-[2-(2-amino-2-keto-ethyl)pyrazolo[4,3-c]pyridin-6-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[5-[1-[2-[(1R)-1-hydroxyethyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-fluoro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[4-[4-[4-[[5-[1-(4-amino-2-fluoro-phenyl)-3-(trifluoromethyl)pyrazol-4-yl]-1-methylimidazole-2-carbonyl]amino]-2-methyl-benzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-methyl-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-(2-hydroxyethyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[3-[(3S)-3-hydroxypyrrolidine-1-carbonyl]-1H-pyrrolo[3,2-c]pyridin-6-yl]-3-(trifluoromethyl)pyrazol-4-yl]-1-methylimidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[3-(methylcarbamoyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[4-[[5-[1-(3-carbamoyl-1H-pyrrolo[3,2-c]pyridin-6-yl)-3-(trifluoromethyl)pyrazol-4-yl]-1-methyl-imidazole-2-carbonyl]amino]-2-chlorobenzoyl]piperazine-1-carbonyl]piperidin-1-ium-1-yl]acetic acid;
cis-2-[1-(azetidin-3-ylmethyl)-4-[4-[2-chloro-4-[[5-[1-[1-(dimethylsulfamoyl)-2-(2-hydroxyethyl)pyrrolo[3,2-c]pyridin-6-yl]-3-(trifluoromethyl)pyrazol-4-yl]-1-methylimidazole-2-carbonyl]amino]benzoyl]piperazine-1-carbonyl]piperidin-1-ium-1-yl]acetic acid;
2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-(3-hydroxypropyl)piperidin-1-ium-1-yl]acetic acid;
cis-2-[1-(azetidin-3-ylmethyl)-4-[4-[2-chloro-4-[[5-[1-[2-[(2S)-2,3-dihydroxypropyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[4-[4-[4-[[5-[1-[1-(3-amino-2-methyl-3-oxo-propyl)pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[5-[1-[2-(2-hydroxyethyl)-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-(2-fluoro-4-hydroxy-phenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-fluoro-4-[(2-hydroxy-2-methylpropyl)amino]phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(5H-pyrrolo[3,2-d]pyrimidin-2-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[4-[[5-[1-[5-[2-[bis(2-aminoethyl)amino]ethylamino]-2-pyridyl]-3-(trifluoromethyl)pyrazol-4-yl]-1-methyl-imidazole-2-carbonyl]amino]-2-chlorobenzoyl]piperazine-1-carbonyl]piperidin-1-ium-1-yl]acetic acid;
cis-2-[4-[4-[4-[[5-[1-[2-(2-amino-2-oxo-ethyl)-1H-pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[4-[4-[4-[[5-[1-[1-(2-amino-2-keto-ethyl)pyrrolo[3,2-c]pyridin-6-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[5-[1-[1-(cyanomethyl)-2-methylol-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-(3-aminopropyl)piperidin-1-ium-1-yl]acetic acid;
cis-2-[1-(3-aminopropyl)-4-[4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[3-(dimethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(1H-pyrazolo[4,3-c]pyridin-6-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[1-(2-hydroxyethyl)pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[1-(2-hydroxyethyl)-2-methylol-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-fluoro-4-(methylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[3-(morpholine-4-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-fluoro-4-(2-hydroxyethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[3-(4,4-difluoropiperidine-1-carbonyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(2-methylol-1H-pyrrolo[3,2-c]pyridin-6-yl)-3-(trifluoromethyl)pyrazol-4-yl]imidazole-2-carbonyl]amino]benzoyl]piperazine-1-carbonyl]piperidin-1-ium-1-yl]acetic acid;
4-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]butanoic acid;
[4-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl]piperazin-1-yl]-4-oxo-butyl]-bis(3-aminopropyl)-(3-carboxypropyl)ammonium;
[4-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl]piperazin-1-yl]-4-oxo-butyl]-bis(3-aminopropyl)-(carboxymethyl)ammonium;
cis-2-[4-[4-[4-[[5-[1-[2-(aminomethyl)-1H-pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[5-[1-[2-fluoro-4-(2-methoxyethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[4-[4-[4-[[5-[1-[4-(3-aminopropylamino)-2-fluoro-phenyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
trans-2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-[2-(dimethylamino)ethyl]piperidin-1-ium-1-yl]acetic acid;
cis-2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-[2-(dimethylamino)ethyl]piperidin-1-ium-1-yl]acetic acid;
cis-2-[4-[4-[4-[[5-[1-[2-(aminomethyl)-1H-pyrrolo[3,2-c]pyridin-6-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[4-[4-[4-[[5-[1-[1-(2-aminoethyl)pyrrolo[3,2-b]pyridin-5-yl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[1-methyl-5-[1-(2-methylol-1H-pyrrolo[2,3-c]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[4-[4-[4-[[5-[1-(6-amino-2-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-methyl-piperidin-1-ium-1-yl]acetic acid;
cis-2-[1-(azetidin-3-ylmethyl)-4-[4-[2-chloro-4-[[5-[1-[3-(2-hydroxyethyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazin-1-yl]-4-oxo-butyl]-(azetidin-3-ylmethyl)-methyl-ammonio]acetate;
cis-2-[1-(azetidin-3-ylmethyl)-4-[4-[4-[[5-[1-[5-[bis(2-aminoethyl)amino]-2-pyridyl]-3-(trifluoromethyl)pyrazol-4-yl]-1-methyl-imidazole-2-carbonyl]amino]-2-chlorobenzoyl]piperazine-1-carbonyl]piperidin-1-ium-1-yl]acetic acid;
cis-2-[1-(azetidin-3-ylmethyl)-4-[4-[2-chloro-4-[[5-[1-[1-(cyanomethyl)pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[1-(2-hydroxyethyl)pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[4-[4-[4-[[5-[1-[1-(2-amino-2-keto-ethyl)pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[1-methyl-5-[1-(1H-pyrrolo[2,3-c]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
2-[4-[4-[4-[[5-[1-[5-(2-aminoethylamino)-2-pyridyl]-3-(trifluoromethyl)pyrazol-4-yl]-1-methylimidazole-2-carbonyl]amino]-2-chloro-benzoyl]piperazine-1-carbonyl]-1-methyl-piperidin-1-ium-1-yl]acetate; and
cis-2-[4-[4-[4-[[5-[1-[5-[(3S)-3-aminopyrrolidin-1-yl]-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid.

In a preferred embodiment, the present invention provides a compound of formula (I) as described herein, or a pharmaceutically acceptable salt thereof, wherein the compound of formula (I) is selected from:
bis(4-aminobutyl)-[4-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl]piperazino]-4-keto-butyl]-(carboxymethyl)ammonium;
cis-2-[1-(azetidin-3-ylmethyl)-4-[4-[2-chloro-4-[[1-methyl-5-[1-(1H-pyrrolo[3,2-c]pyridin-6-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[4-[4-[4-[[5-[1-[5-(3-aminopropylamino)-2-pyridyl]-3-(trifluoromethyl)pyrazol-4-yl1]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
cis-2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[1-methyl-5-[1-[5-(methylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
trans-2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[5-[1-[5-(2-hydroxyethylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid;
cis-2-[1-(3-aminopropyl)-4-[4-[4-[[5-[1-(2-fluoro-4-hydroxy-phenyl)-3-(trifluoromethyl)pyrazol-4-yl]-1-methyl-imidazole-2-carbonyl]amino]-2-methylbenzoyl]piperazine-1-carbonyl]piperidin-1-ium-1-yl]acetic acid;
cis-2-[1-(azetidin-3-ylmethyl)-4-[4-[2-methyl-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[4-[4-[4-[[5-[1-[2-(2-amino-2-keto-ethyl)pyrazolo[4,3-c]pyridin-6-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[4-[4-[4-[[5-[1-(4-amino-2-fluoro-phenyl)-3-(trifluoromethyl)pyrazol-4-yl]-1-methylimidazole-2-carbonyl]amino]-2-methyl-benzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-methyl-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-(2-hydroxyethyl)-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[3-(dimethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[1-(2-hydroxyethyl)pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-fluoro-4-(2-hydroxyethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-fluoro-4-(2-methoxyethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[4-[4-[4-[[5-[1-[1-(2-amino-2-keto-ethyl)pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid; and
cis-2-[4-[4-[4-[[5-[1-[5-[(3S)-3-aminopyrrolidin-1-yl]-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid.

In a particularly preferred embodiment, the present invention provides a compound of formula (I) as described herein, or a pharmaceutically acceptable salt thereof, wherein the compound of formula (I) is bis(4-aminobutyl)-[4-[4-[4-[[5-[1-(4-amino-2-fluoro-phenyl)-3-(trifluoromethyl)pyrazol-4-yl]-1-methyl-imidazole-2-carbonyl]amino]-2-chlorobenzoyl]piperazino]-4-keto-butyl]-(carboxymethyl)ammonium.

In a particularly preferred embodiment, the present invention provides a compound of formula (I) as described herein, or a pharmaceutically acceptable salt thereof, wherein the compound of formula (I) is cis-2-[1-(azetidin-3-ylmethyl)-4-[4-[2-chloro-4-[[1-methyl-5-[1-(1H-pyrrolo[3,2-c]pyridin-6-yl)-3-(trifluoromethyl)pyrazol-4-yl]imidazole-2-carbonyl]amino]benzoyl]piperazine-1-carbonyl]piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[4-[4-[4-[[5-[1-[5-(3-aminopropylamino)-2-pyridyl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[4-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[1-(azetidin-3-ylmethyl)-4-[4-[2-chloro-4-[[1-methyl-5-[1-[5-(methylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid.

In a particularly preferred embodiment, the present invention provides a compound of formula (I) as described herein, or a pharmaceutically acceptable salt thereof, wherein the compound of formula (I) is trans-2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chlorobenzoyl]piperazine-1-carbonyl]piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[1-(azetidin-3-ylmethyl)-4-[4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[1-(azetidin-3-ylmethyl)-4-[4-[2-chloro-4-[[5-[1-[5-(2-hydroxyethylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chlorobenzoyl]piperazine-1-carbonyl]piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[1-(3-aminopropyl)-4-[4-[4-[[5-[1-(2-fluoro-4-hydroxy-phenyl)-3-(trifluoromethyl)pyrazol-4-yl]-1-methyl-imidazole-2-carbonyl]amino]-2-methylbenzoyl]piperazine-1-carbonyl]piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[1-(azetidin-3-ylmethyl)-4-[4-[2-methyl-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[4-[4-[4-[[5-[1-[2-(2-amino-2-keto-ethyl)pyrazolo[4,3-c]pyridin-6-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[4-[4-[4-[[5-[1-(4-amino-2-fluoro-phenyl)-3-(trifluoromethyl)pyrazol-4-yl]-1-methyl-imidazole-2-carbonyl]amino]-2-methyl-benzoyl]piperazine-1-carbonyl]-1-(azetidin-3-ylmethyl)piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[1-(azetidin-3-ylmethyl)-4-[4-[2-methyl-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[1-(azetidin-3-ylmethyl)-4-[4-[2-chloro-4-[[5-[1-[2-(2-hydroxyethyl)-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[1-(azetidin-3-ylmethyl)-4-[4-[2-chloro-4-[[5-[1-[3-(dimethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[1-(azetidin-3-ylmethyl)-4-[4-[2-chloro-4-[[5-[1-[1-(2-hydroxyethyl)pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[1-(azetidin-3-ylmethyl)-4-[4-[2-chloro-4-[[5-[1-[2-fluoro-4-(2-hydroxyethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[1-(azetidin-3-ylmethyl)-4-[4-[2-chloro-4-[[5-[1-[2-fluoro-4-(2-methoxyethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[4-[4-[4-[[5-[1-[1-(2-amino-2-keto-ethyl)pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid.

In a particularly preferred embodiment, the present invention provides a compound of formula (I) as described herein, or a pharmaceutically acceptable salt thereof, wherein the compound of formula (I) is cis-2-[4-[4-[4-[[5-[1-[5-[(3S)-3-aminopyrrolidin-1-yl]-2-pyridyl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid.

In some instances, the compound of formula (I) according to the invention comprises a quaternary ammonium cation, and is therefore permanently positively charged. In one embodiment, the associated negatively charged counterion is selected from the group consisting of trifluoroacetate, formate, chloride, and bromide.

In one embodiment, the present invention provides a compound of formula (I) as described herein, wherein when said compound of formula (I) comprises a quaternary ammonium cation, the associated negatively charged counterion is selected from the group consisting of trifluoroacetate, formate, chloride, and bromide.

In one embodiment, the present invention provides pharmaceutically acceptable salts of the compounds of formula (I) as described herein, especially pharmaceutically acceptable salts selected from hydrochlorides, fumarates, lactates (in particular derived from L-(+)-lactic acid), tartrates (in particular derived from L-(+)-tartaric acid) and trifluoroacetates. In yet a further particular embodiment, the present invention provides compounds according to formula (I) as described herein (i.e., as "free bases" or "free acids", respectively).

In some embodiments, the compounds of formula (I) are isotopically-labeled by having one or more atoms therein replaced by an atom having a different atomic mass or mass number. Such isotopically-labeled (i.e., radiolabeled) compounds of formula (I) are considered to be within the scope of this disclosure. Examples of isotopes that can be incorporated into the compounds of formula (I) include isotopes of hydrogen, carbon, nitrogen, oxygen, phosphorous, sulfur, fluorine, chlorine, and iodine, such as, but not limited to, ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Certain isotopically-labeled compounds of formula (I), for example, those incorporating a radioactive isotope, are useful in drug and/or substrate tissue distribution studies. The radioactive isotopes tritium, i.e. ³H, and carbon-14, i.e., ¹⁴C, are particularly useful for this purpose in view of their ease of incorporation and ready means of detection. For example, a compound of formula (I) can be enriched with 1, 2, 5, 10, 25, 50, 75, 90, 95, or 99 percent of a given isotope.

Substitution with heavier isotopes such as deuterium, i.e. ²H, may afford certain therapeutic advantages resulting from greater metabolic stability, for example, increased *in vivo* half-life or reduced dosage requirements.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of formula (I) can generally be prepared by conventional techniques known to those skilled in the art or by processes analogous to those described in the Examples as set out below using an appropriate isotopically-labeled reagent in place of the non-labeled reagent previously employed.

### Processes of Manufacturing

In one aspect, the present invention provides a process of manufacturing the compounds of formula (I) described herein.

In a further aspect, the present invention provides a compound of formula (I) as described herein, or a pharmaceutically acceptable salt thereof, when manufactured according to the processes described herein.

The preparation of compounds of formula (I) of the present invention may be carried out in sequential or convergent synthetic routes. Syntheses of the compounds of the invention are shown in the following schemes. The skills required for carrying out the reactions and purifications of the resulting products are known to those skilled in the art. The substituents and indices used in the following description of the processes have the significance given herein before unless indicated to the contrary. In more detail, the compounds of formula (I) can be manufactured by the methods given below, by the methods given in the examples or by analogous methods. Appropriate reaction conditions for the individual reaction steps are known to a person skilled in the art. Also, for reaction conditions described in literature affecting the described reactions see for example: *Comprehensive Organic Transformations: A Guide to Functional Group Preparations, 3rd Edition, Richard C. Larock. John Wiley & Sons, New York, NY. 2018).* We find it convenient to carry out the reactions in the presence or absence of a solvent. There is no particular restriction on the nature of the solvent to be employed, provided that it has no adverse effect on the reaction or the reagents involved and that it can dissolve the reagents, at least to some extent. The described reactions can take place over a wide range of temperatures, and the precise reaction temperature is not critical to the invention. It is convenient to carry out the described reactions in a temperature range between -78 °C to reflux temperature. The time required for the reaction may also vary widely, depending on many factors, notably the reaction temperature and the nature of the reagents. However, a period of from 0.5 h to several days will usually suffice to yield the described intermediates and compounds. The reaction sequence is not limited to the one displayed in the schemes, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

The following abbreviations are used in the present text:
- Å: Angstrom (10⁻¹⁰ m)
- ACN or MeCN: Acetonitrile
- aq: aqueous
- BINAP: 2,2'-bis(diphenylphosphino)-1,1'-binaphthyl
- BOC or Boc: tert-butyloxycarbonyl
- CDI: carbonyldiimidazole
- CFU: colony-forming unit
- d: day(s)
- DCM: dichloromethane
- DEA: Diethylamine
- DIPEA: N,N-diisopropylethylamine
- DMF: N,N-dimethylformamide
- EDTA: ethylenediaminetetraacetic acid
- EtOAc or EA: ethyl acetate
- EtOH: ethanol
- eq: equivalent(s)
- FA: Formic acid
- FMOC or Fmoc: fluorenylmethoxycarbonyl
- h(s) or hr(s): hour
- HATU:: 1-[bis(dimethylamino)methylene]-1H-1,2,3-triazolo[4,5-b]pyridinium 3-oxid hexafluorophosphate
- HPLC:: high performance liquid chromatography
- HPLC-UV:: high performance liquid chromatography with ultraviolet detector
- HV: high vacuum
- IC50: half maximal inhibitory concentration
- IC90: 90% inhibitory concentration
- LED: light-emitting diode
- [M]: molecular mass
- M: molar
- MeOH: Methanol
- min: minute(s)
- mL: milliliter(s)
- MS: mass spectrometry
- NaBH₃CN: Sodium cyanoborohydride
- O/N: overnight, approximately 16 hours
- PE: petroleum ether
- PdCl₂(DPPF): [1,1'-bis(diphenylphosphino)ferrocene]dichloropalladium(II)
- Pd₂(dba)₃: tris(dibenzylideneacetone)dipalladium(0)
- PG: protective group
- Precat: precatalyst
- PyAOP: (7-azabenzotriazol-1-yloxy)tripyrrolidinophosphonium hexafluorophosphate
- quant.: quantitative
- rac: racemic
- RP: reversed phase
- RPHPLC: reversed-phase high-performance liquid chromatography
- Rt: retention time
- RT: room temperature, approximately 23 °C
- sat: saturated
- SEM: 2-methoxyethyl(trimethyl)silane
- SFC: supercritical fluid chromatography
- T3P: propylphosphonic anhydride solution
- TEMPO: (2,2,6,6-tetramethylpiperidin-1-yl)oxyl
- TFA: trifluoroacetic acid
- THF: tetrahydrofuran
- wt: weight
- XANTPHOS: 4,5-bis(diphenylphosphino)-9,9-dimethylxanthene

In the following Schemes, the variables are as defined herein and in the claims, unless defined otherwise. R^{8'} may equal R⁸ as defined herein. In some instances, R^{8'} is a protected version of R⁸ as defined herein, for example when R⁸ is amino, R^{8'} is a Boc protected amine. In some instances, R^{8'} is a synthetic precursor to a group R⁸ as defined herein. For example, when R⁸ is amino, R^{8'} might represent a nitro group, which may then be reduced to said amino group. In a further example, R^{8'} might be fluoro, which can be substituted by a group R⁸ as defined herein, or by a precursor to R⁸, etc. Similar considerations apply to the definition of R^{9'}.

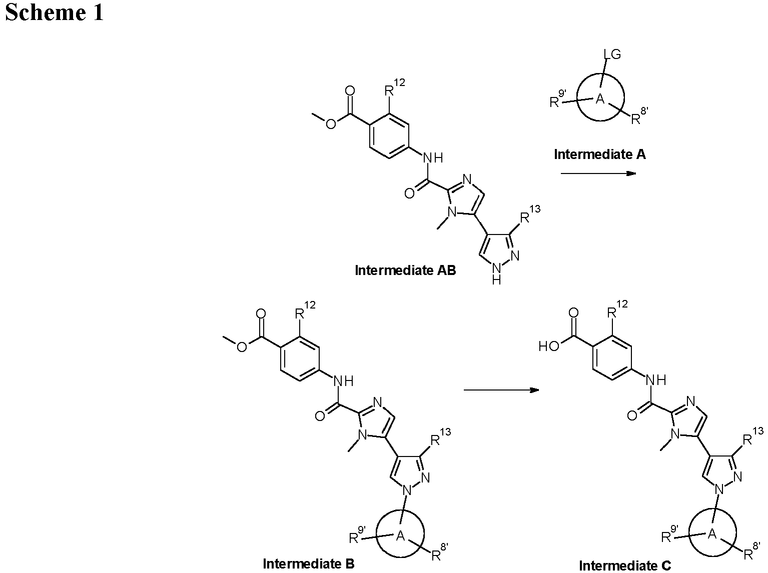

In Scheme 1, LG denotes a suitable leaving group for substitution or coupling reactions, for example, a halide or a boronic acid.

Scheme 1 summarizes the synthetic route for the preparation of Intermediates B and **C,** which can be made from **Intermediate AB** (for example, methyl 2-chloro-4-[[1-methyl-5-[3-(trifluoromethyl)-1H-pyrazol-4-yl]imidazole-2-carbonyl]amino]benzoate, CAS 2489205-78-7, or as described in WO 2020/182648) and **Intermediate A. Intermediate** A can be a commercially available compound, or can be made from a commercially available compound, respectively, by modifications of the substituents R^{8'} and/or R^{9'}, such as for example by an alkylation reaction or by the attachment of a protecting group, for example Boc. Additional routes for the preparation of Intermediate A are summarized in Schemes 5 and 6. **Intermediate** A is coupled to **Intermediate AB,** for example by an S_{N}Ar reaction or a coupling reaction, such as for example an Ullmann or Chan-Lam reaction to afford **Intermediate** B. Hydrolysis of the methyl ester group of **Intermedate** B yields carboxylic acid **Intermediate** C. In some cases, additional modificiations of the substituents R^{8'} and/or R^{9'} can be carried out before performing the hydrolysis of the methyl ester, such as for example a nitro group reduction, which can be followed by a reductive amination with diverse aldehydes, or a an S_{N}Ar reaction, or a Buchwald-Hartwig reaction.

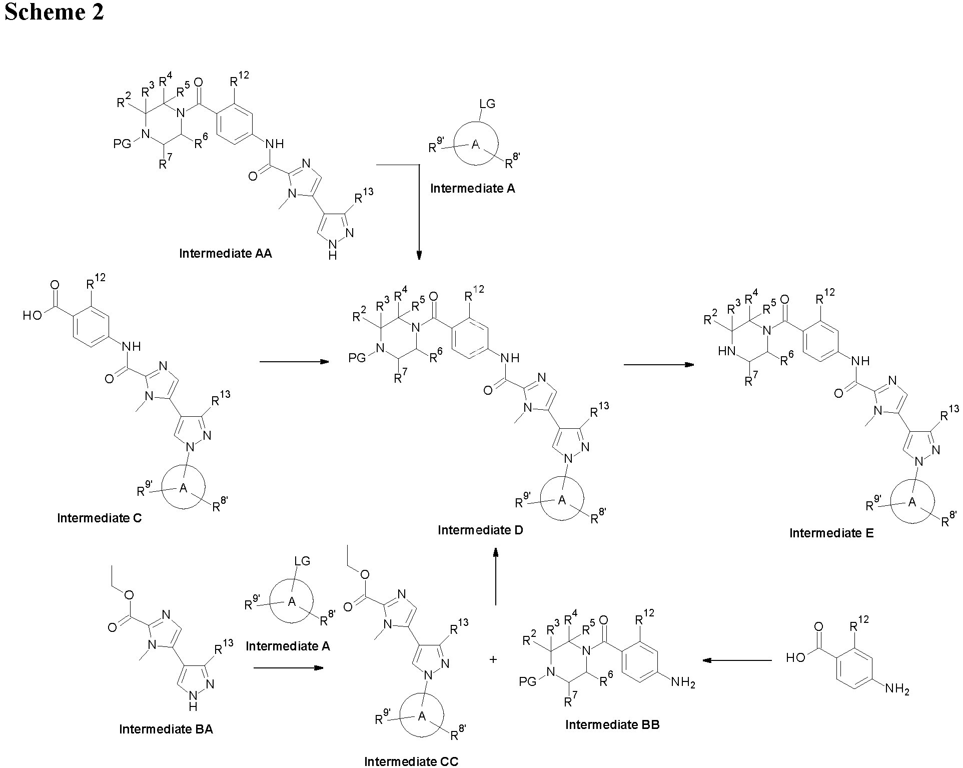

In Scheme 2, PG denotes a suitable protecting group, such as Boc or Fmoc.

**Intermediates D,** and E can be prepared according to Scheme 2. In analogy to the route outlined in Scheme 1, **Intermediate** A is coupled to **Intermediate** AA (for example, tert-butyl 4-[2-chloro-4-[[1-methyl-5-[3-(trifluoromethyl)-1H-pyrazol-4-yl]imidazole-2-carbonyl]amino]benzoyl]piperazine-1-carboxylate, CAS 2489206-18-8), for example by an S_{N}Ar reaction or a coupling reaction, such as for example an Ullmann or Chan-Lam reaction, to give **Intermediate D. Intermediate AA,** in turn, can be prepared according to Scheme 7. Alternatively, **Intermediate C** can be coupled with diverse amines in the presence of a condensing agent, such as HATU/DIPEA in a solvent, such as DMF, to afford **Intermediate D.** In another route, **Intermediate D** can be prepared by an aminolysis reaction of **Intermediate CC** and **Intermediate BB,** using for example a base, such as NaHMDS in a solvent, such as THF. **Intermediate CC,** in turn, can be prepared from **Intermediate BA** (for example, ethyl 1-methyl-5-[3-(trifluoromethyl)-1H-pyrazol-4-yl]imidazole-2-carboxylate, CAS 2489206-33-7) and **Intermediate A** by an S_{N}Ar or coupling reaction, such as for example an Ullmann or Chan-Lam reaction. **Intermediate BB,** in turn, can be prepared from a 4-aminobenzoic acid derivative, that is coupled with diverse amines in the presence of a condensing agent, such as HATU/DIPEA in a solvent, such as DMF. Subsequent removal of the protecting group PG of **Intermediate D** yields **Intermediate E.** In some cases, additional modifications of the substituents R^{8'} and/or R^{9'} can be carried out before performing the removal of the protecting group PG, such as for example a nitro group reduction, which can be followed by a reductive amination with diverse aldehydes, or an S_{N}Ar reaction, or a Buchwald-Hartwig reaction.

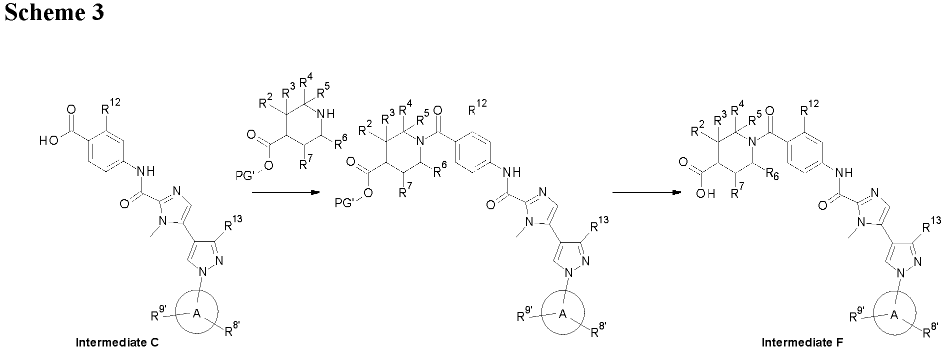

In Scheme 3, PG' denotes a suitable protecting group, such as tert-Bu.

**Intermediate F** can be prepared according to Scheme 3. **Intermediate C** is coupled with an amine comprising a carboxylic ester group in the presence of a condensing agent, such as HATU/DIPEA in a solvent, such as DMF. Subsequent removal of the protecting group PG' yields **Intermediate F.**

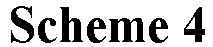

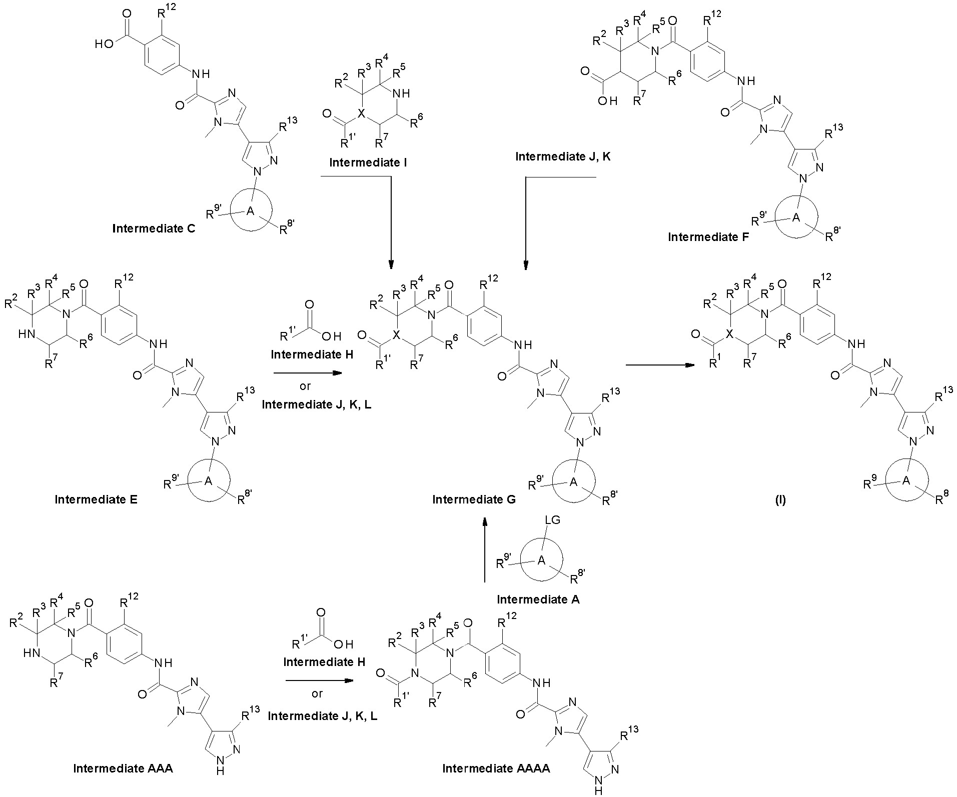

In Scheme 4, R^{1'} may equal R¹ as defined herein. In some instances, R^{1'} is a synthetic precursor of R¹ as defined herein, for example, a protected version thereof. Thus, for example, when R¹ comprises an amino group, R^{1'} may be the Boc protected analogue thereof.

The compounds of formula (I) according to the invention can be prepared according to the different synthetic routes summarized in Scheme 4. Thus, **Intermediate C** is coupled with amine **Intermediate I** in the presence of a condensing agent, such as HATU/DIPEA in a solvent, such as DMF, to afford **Intermediate G.** Alternatively, **Intermediate E** can be coupled with carboxylic acid **Intermediate H** in the presence of a condensing agent, such as HATU/DIPEA in a solvent, such as DMF, to afford **Intermediate G, or Intermediate E** can also be coupled with amine **Intermediate J, or K,** or with alcohol **Intermediate L** in the presence of a coupling reagent, such as CDI in a solvent, such as DMF, to afford **Intermediate G.** Alternatively, **Intermediate F** is coupled with amine **Intermediate J,** or **K** in the presence of a condensing agent, such as HATU/DIPEA in a solvent, such as DMF, to afford **Intermediate G.** In another route, **Intermediate AAA** (for example, N-[3-chloro-4-(piperazine-1-carbonyl)phenyl]-1-methyl-5-[3-(trifluoromethyl)-1H-pyrazol-4-yl]imidazole-2-carboxamide;hydrochloride, CAS 2489198-91-4) can be coupled with carboxylic acid **Intermediate H** in the presence of a condensing agent, such as HATU/DIPEA in a solvent, such as DMF, to afford **Intermediate AAAA. Intermediate AAA** can also be coupled with amine **Intermediate J, or K,** or alcohol **Intermediate L** in the presence of a coupling reagent, such as CDI in a solvent, such as DMF, to afford **Intermediate AAAA.** Subsequently, coupling with **Intermediate A ,** for example by an S_{N}Ar or coupling reaction, such as for example an Ullmann or Chan-Lam reaction can afford **Intermediate G.** Subsequent removal of the protecting groups of substituents R^{1'}, R^{8'}, and R^{9'} finally affords the compounds of formula (I) of the invention. **Intermediate AAA,** in turn, can be prepared according to the route summarized in Scheme 7.

If needed, the routes summarized in Scheme 4 can be conducted as one-pot procedures, which means that **Intermediate G** does not have to be isolated, and the subsequent reaction (e.g., removal of protecting groups of R^{1'}, R^{8'}, and R^{9'}) leading to compounds of formula (I) can directly be carried out. In some cases, additional modifications of **Intermediate G** can be carried out before or after the removal of the protecting groups of R^{1'}, R^{8'}, and R^{9'}, based on the required substitution pattern, such as for example an alkylation reaction or a reductive amination.

In case the compound of formula (I) comprises a quaternary ammonium cation, and is therefore permanently positively charged, the associated negatively charged counterion (not shown in Scheme) can be for example trifluoroacetate, formate, chloride, or bromide, as needed.

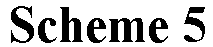

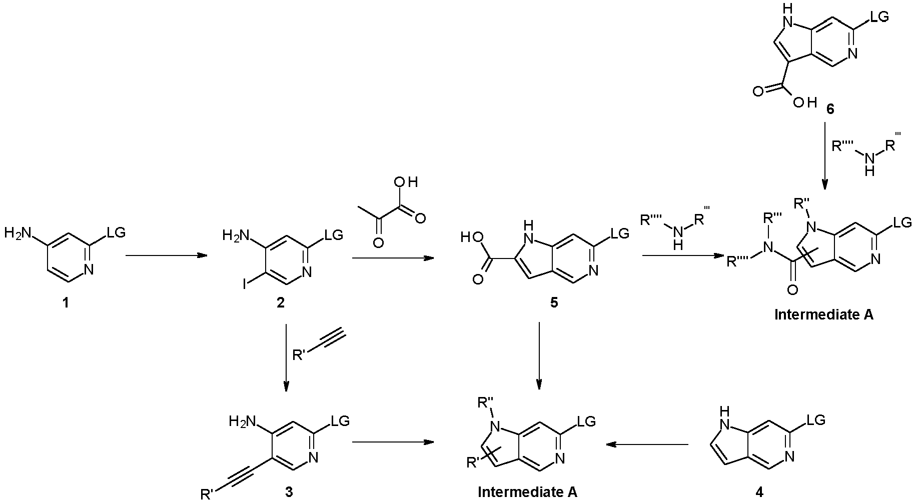

In Scheme 5:
- R': is a substituent corresponding to R^{8'} or R^{9'}, respectively, or H;
- R": is a suitable protecting group, such as Boc or SEM, or a substituent corresponding to R^{8'} or R^{9'}, respectively, or H;
- R‴ and Rʺʺ: are substituents that correspond as CONR‴Rʺʺ to R^{8'} or R^{9'}, respectively.

**Intermediate A,** that consists of a fused bicyclic heteroaromatic scaffold, can be prepared according to the routes shown in Scheme 5. The depicted routes also apply for the preparation of related bicyclic heteroaromatic rings, such as regioisomers.

As shown in Scheme 5, an aminopyridine derivative 1 is transformed into iodo-substituted aminopyridine derivative 2 in the presence of a reagent such as N-iodosuccinimide in a solvent such as DMF. Compound **2** is then reacted in a Sonogashira reaction with diverse alkynes in the presence of a catalyst, such as CuI/[(dppf)PdCl₂], a base such as Et₃N and in a solvent, such as THF, to afford alkynyl-substituted aminopyridine derivative **3.** Cyclization of compound **3** in the presence of a base, such as KOtBu in a solvent, such as NMP, affords bicyclic heteroaromatic **Intermediate A.** In another route, commercially available azaindole derivative **4** can be substituted on the NH of the fused pyrrole ring with R" in the presence of, for example a base, such as Cs₂CO₃ in a solvent, such as DMF, or for example in the presence of a condensing agent, such as CDI in a solvent, such as ACN. In some cases, subsequent introduction of an additional substituent R^{'} on the 2-position or 3-position of the azaindole scaffold can be carried out. For example, substituents in the 2-position can be introduced in the presence of a catalyst, such as Pd(PhCN)₂Cl₂/norbornene in a solvent, such as DMF, or for example in the presence of a strong base, such as LDA, in a solvent, such as THF. For example, substituents in the 3-position can be introduced in the presence of a Lewis acid, such as AlCl₃, and an acyl chloride or similar reagent, such as ethyl oxalyl chloride. The order of introduction of substituents R" and R' can also be interchanged, as needed. In some cases, additional modifications of the substituents R" and R' can be carried out, such as for example addition or removal of a protecting group, substitution reaction, Wittig reaction, or a reduction reaction, as needed.

In another route, iodo-substituted aminopyridine derivative **2** is transformed into carboxylic acid **5** via palladium-catalyzed annulation with a reagent, such as pyruvic acid, and a catalyst/base, such as Pd(OAc)₂/DABCO in a solvent, such as DMF. Carboxylic acid derivative **5** can be further transformed into **Intermediate** A via different routes. For example, carboxylic acid derivative **5** can be reacted with a reducing agent, such as DIBAL-H in a solvent such as THF, to obtain **Intermediate A,** in which R^{'} is hydroxymethyl. Alternatively, carboxylic acid derivative **5** can be transformed via an esterification reaction into **Intermediate** A. In another route, carboxylic acid derivative **5,** or **6** (for example, 6-chloro-1H-pyrrolo[3,2-c]pyridine-3-carboxylic acid, CAS 1000341-67-2), respectively, can be coupled with diverse amines in the presence of a condensing agent, such as HATU/DIPEA in a solvent, such as DMF, to afford **Intermediate A,** in which R' is an amide group. In some cases, additional modifications of the substituents R" and R' can be carried out, such as for example attachment of a protecting group, or for example transformation of an amide substituent into a ketone, which is subsequently reduced to an alcohol.

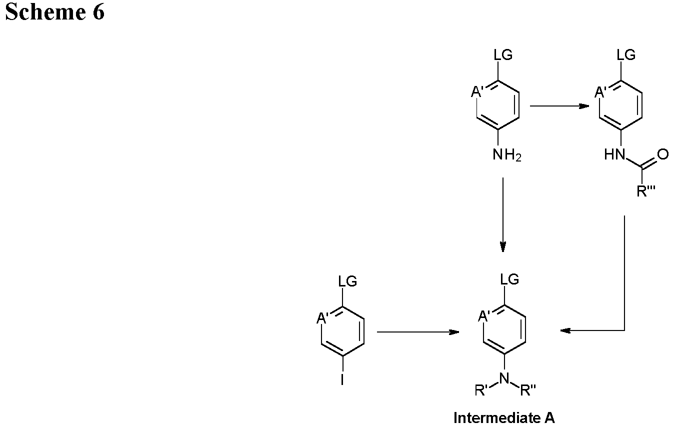

In Scheme 6:
- A^{'}: is nitrogen, CH, or any other C, that is substituted with R^{8'} or R^{9'}, respectively;
- R^{'} and R": are substituents that correspond as NR'R" to R^{8'} or R^{9'}, respectively;
- R^{‴} is R^{'}: but without a terminal methylene (CH₂) group.

**Intermediate A,** that consists of a monocyclic (hetero)aromatic scaffold, can be prepared according to the routes shown in Scheme 6. The depicted routes also apply for the preparation of related monocylic (hetero)aromatic rings, such as regioisomers.

In one of the routes, depicted in Scheme 6, a monocyclic (hetero)aromatic amine can be coupled with diverse carboxylic acids in the presence of a condensing agent, such as HATU/Et₃N in a solvent, such as DMF. Subsequent reduction of the amide group in the presence of a reducing agent, such as BH₃-THF in a solvent, such as THF, affords **Intermediate A.** Alternatively, a monocyclic (hetero)aromatic amine can be transformed into **Intermediate A** by the attachment of substituents R^{'} and/or R", respectively, for example via an alkylation reaction that is conducted in the presence of a base, such as KOtBu, and in a solvent, such as tBuOH. In another route, a monocyclic (hetero)aromatic iodide is coupled with diverse amines, for example by an Ullmann reaction using a catalyst/ligand, such as CuI/L-proline, in a solvent, such as DMSO, to afford **Intermediate A.** In some cases, additional modifications of **Intermediate A** can be carried out, based on the required substitution pattern, such as for example modifications of protecting groups, or a reductive amination.

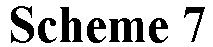

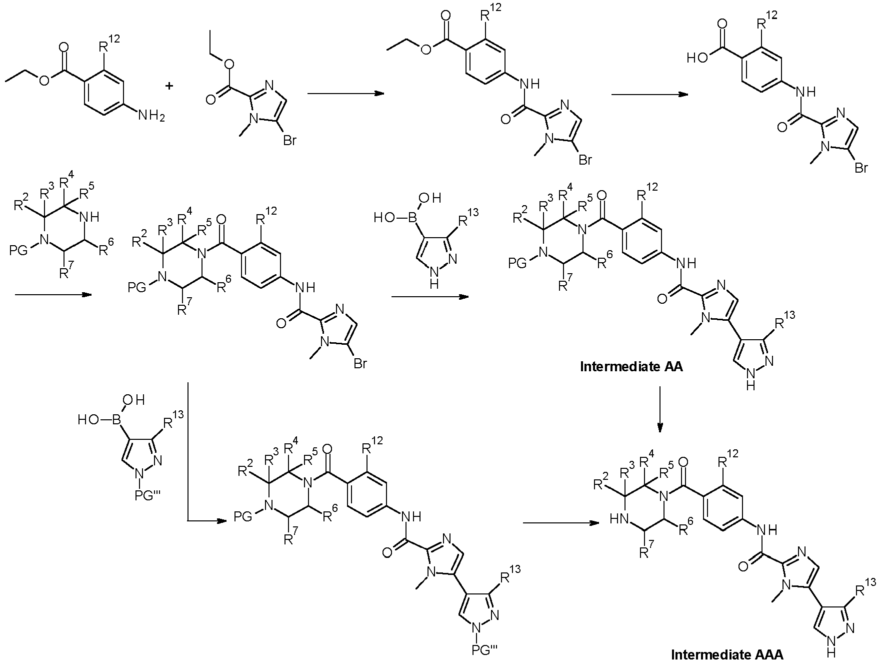

In Scheme 7, PG is as defined above. PG^{‴} is a suitable protecting group, such as trityl.

**Intermediate AA and AAA** can be prepared according to the routes shown in Scheme 7. A general route to these Intermediates is also described in WO 2020/182648. A commercially available substituted aniline carboxylate derivative is coupled with a 1-methyl-imidazole-2-carboxylate derivative via an aminolysis reaction in the presence of a base, such as LiHMDS, in a solvent, such as THF. The ester group is then hydrolysed to afford the carboxylic acid intermediate, which is then coupled with a mono-protected piperazine derivative in the presence of a condensing agent, such as HATU/DIPEA in a solvent, such as DMF. Subsequently, Suzuki reaction with a pyrazole boronic acid derivative in the presence of a catalyst, such as cataCXium A Pd G2, a base such as Na₂CO₃, and in a solvent, such as 1,4-dioxane/water 10:1, affords **Intermediate AA.**

**Intermediate AAA** can be accessed from **Intermediate AA** by removal of the protecting group PG. Alternatively, **Intermediate AAA** can also be prepared via a similar route to **Intermediate AA,** in which the Suzuki reaction is carried out with a pyrazole boronic acid derivative protected with PG‴. This protected pyrazole boronic acid derivative, in turn, is either commercially available or can be prepared via protection of a boronic acid derivative or a synthetic precursor thereof, for example from 1H-pyrazole-3-carbaldehyde. Lastly, removal of both protecting groups PG and PG‴ afford **Intermediate AAA.**

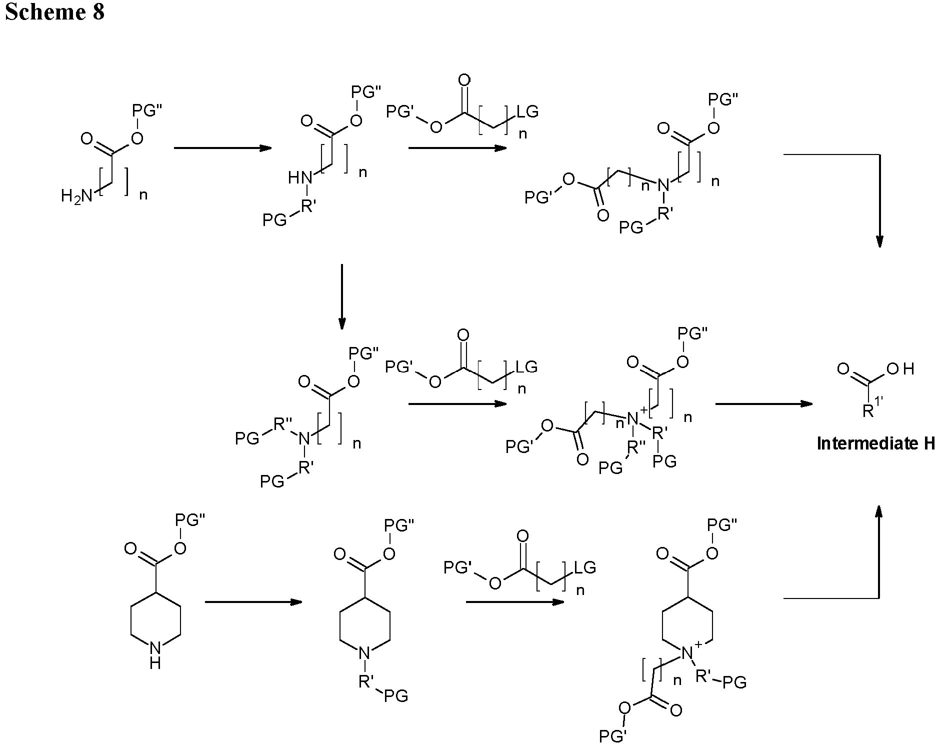

In Scheme 8:
- PG: is a suitable protecting group, such as Boc or Bn, or H;
- PG': is as defined above;
- PG^{"}: is a suitable protecting group, such as Bn, which can be selectively removed in the presence of other protecting groups on R^{1'}, such as PG or PG', if needed;
- LG: is a suitable leaving group, such as for example a halide or tosylate;
- R^{'} and R": are substituents that correspond to a substructure of R^{1'}, for example an alkyl or aminoalkyl or hydroxyalkyl group.

**Intermediate H** can be prepared according to the routes shown in Scheme 5. A commercially available compound comprising an amine as well as a carboxylic acid moiety, in which the amine is not protected and can be either a primary or secondary amine, and in which the carboxyl group is protected with a protecting group PG^{"}, is substituted on the amino group once or twice, respectively, with substituents R^{'} and R", respectively, bearing a protecting group PG. The introduction of these substituents R' and R^{"}, respectively, can be performed via an alkylation reaction using a base such as K₂CO₃, in a solvent such as DMF, or via a reductive amination reaction in the presence of a reducing agent, such as sodium cyanoborohydride, in a solvent, such as MeOH. Subsequently, an alkylation is carried out with an alkylating reagent comprising a carboxy group protected with PG^{'} in the presence of a base, such as DIPEA, in a solvent such as DMF, followed by removal of the protecting group PG^{"} to afford carboxylic acid **Intermediate H.** In some cases, additional modifications of **Intermediate H** can be carried out before or after the removal of the protecting groups of R^{1'}, based on the required substitution pattern, such as for example a reductive amination.

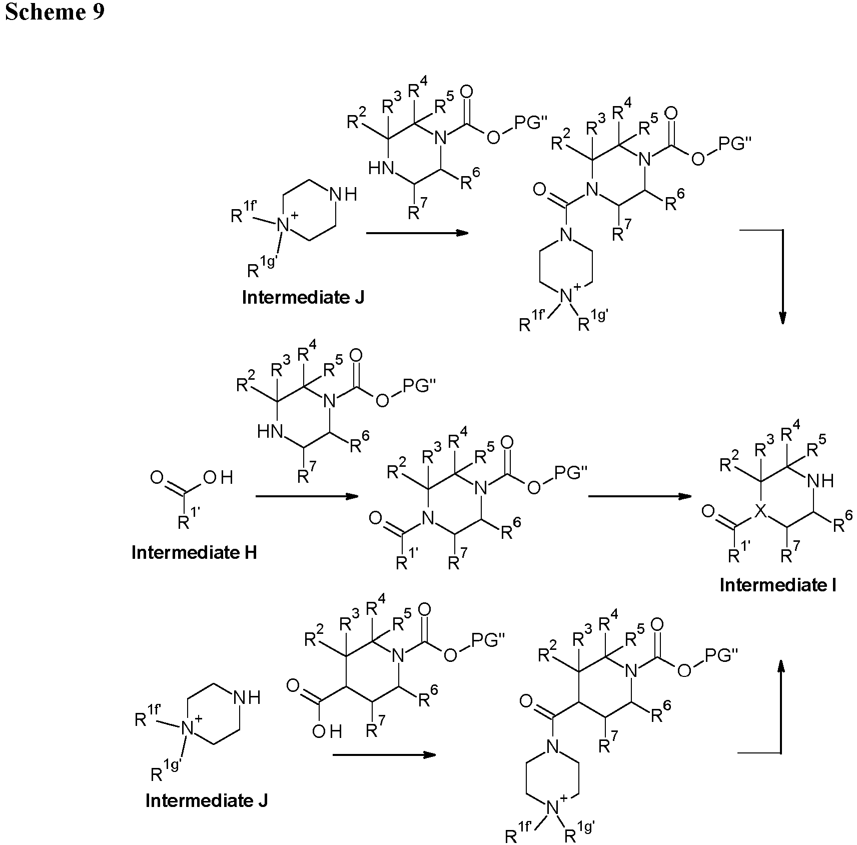

In Scheme 9, R^{1'} and PG" are as defined above; R^{1f'} and R^{1g'} are as defined in Scheme 10.

**Intermediate** I can be prepared according to Scheme 9. Carboxylic acid **Intermediate H is** coupled with diverse amines in the presence of a condensing agent, such as HATU/DIPEA in a solvent, such as DMF. Subsequent removal of the protecting group PG^{"} yields **Intermediate I.** In another route, **Intermediate J** is coupled with diverse amines in the presence of a coupling reagent, such as triphosgene/DIPEA, in a solvent such as DCM, to form a urea linked structure. Subsequent removal of the protecting group PG^{"} yields **Intermediate** I. In another route, **Intermediate** J is coupled with a carboxylic acid derivative in the presence of a condensing agent, such as HATU/Et₃N in a solvent, such as DCM. Subsequent removal of the of the protecting group PG^{"} yields **Intermediate I.**

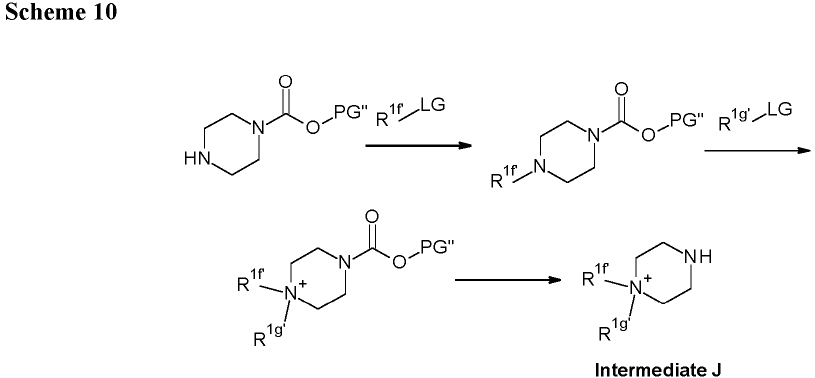

In Scheme 10:
- LG: is a suitable leaving group such as a halide or tosylate;
- R^{1f'}: is R^{1f}, bearing a protecting group, such as Boc;
- R^{1g'}: is R^{1g}, bearing a protecting group, such as tBu.

**Intermediate J** can be prepared according to Scheme 10. Monoprotected piperazine is alkylated with suitably protected alkylating reagents R^{1f'} and R^{1g'}, respectively, in the presence of a base, such as triethylamine, in a solvent such as MeCN. Subsequently, removal of the protecting group PG^{"} yields **Intermediate J.**

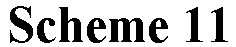

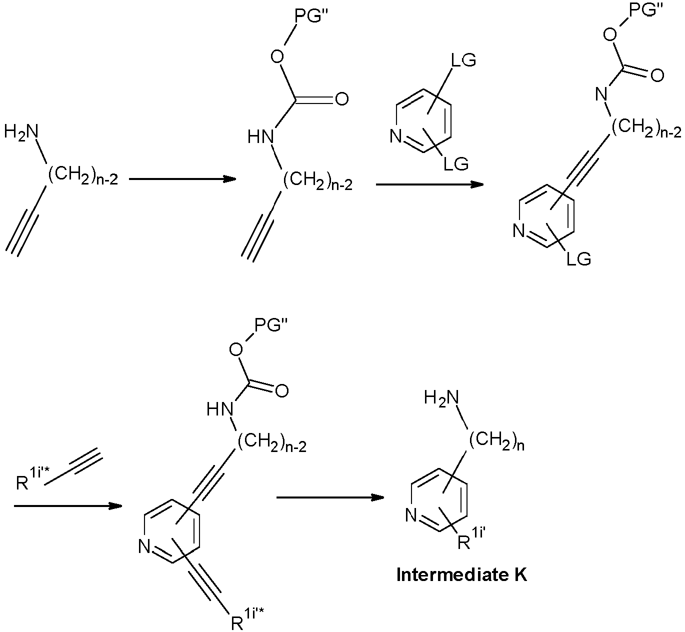

In Scheme 11:
- PG^{"}: is a protecting group, which can be removed in the presence of other protecting groups, such as PG or PG'. For example PG" is a Bn group;
- LG: is a group, which can be used in the coupling with an alkyne, such as bromide or iodide;
- R^{1i'}: is R¹ⁱ, bearing a protecting group, such as Boc;
- R^{1i'*}: is R^{1i'} but without the terminal ethylene (CH₂)₂ group.

**Intermediate K** can be prepared according to Scheme 11. The amino group of the aminoalkyne starting material is protected as a carbamate with protecting group PG^{"}. This protected aminoalkyne is coupled with pyridine, which is disubstituted for example with an iodide and a bromide, by for example a Sonogashira reaction, that is conducted in the presence of a catalyst, such as CuI/[Pd(PPh₃)₄], a base such as Et₃N, and a solvent, such as toluene. Subsequently, a second alkynyl substituent is attached to the pyridine intermediate, for example via another Sonogashira reaction. Finally, a hydrogenation reaction removes the protecting group PG^{"} as well as reduces the two alkyne groups to afford **Intermediate K.**

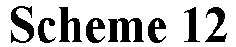

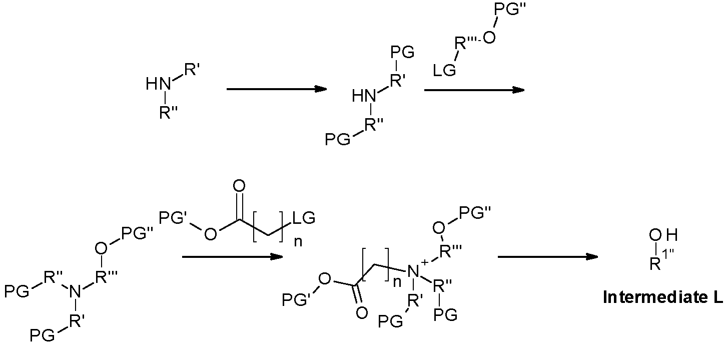

In Scheme 12:
- PG^{"}: is a suitable protecting group, which can be selectively removed in the presence of other protecting groups on R^{1"}, such as PG or PG^{'}. For example, PG^{"} is a Bn group;
- PG^{'}: is as defined above;
- PG: is a suitable protecting group, such as Boc, or H;
- LG: is a leaving group, such as for example a halide or tosylate;
- R^{'} and R": are substituents that correspond to a substructure of R^{1"}, for example an alkyl or aminoalkyl group;
- R^{‴}: is a substituent comprising an alkyl group;
- R^{1"}: is R^{1'} but without the terminal hydroxyl group.

**Intermediate L** can be prepared according to the route shown in Scheme 12. A commercially available compound comprising a secondary amine, which is substituted with (amino)alkyl substituents R' and R", respectively, is protected with a protecting group PG. Alternatively, this intermediate can also be prepared by methods such as reductive amination or alkylation reactions from suitable, commercially available starting materials. Subsequently, an alkylation is carried out with an alkylating reagent comprising a hydroxyl group, which is protected with PG^{"} in the presence of a base, such as triethylamine, in a solvent, such as ACN, to give a tertiary amine.

Subsequently, an alkylation is performed with an alkylating reagent comprising a carboxy group protected with PG^{'}. Lastly, the protecting group PG^{"} is removed to afford the alcohol **Intermediate L.**

In case **Intermediates H, I, J, K,** or **L** comprise a quaternary ammonium cation, and comprise therefore a permanently positively charged group, the associated negatively charged counterion (not shown in Schemes) can be for example trifluoroacetate, formate, chloride, or bromide, as needed. Reactions with these **Intermediates H, I, J, K,** or **L** as shown in Scheme 4, can be carried out in the presence of different counterions, such as trifluoroacetate, formate, chloride, or bromide. The skilled person will acknowledge that, for some of the reactions represented in Scheme 4, the outcome may depend on the specific counterion present in the intermediates used. For example, for amide coupling reactions, intermediates with a formate counterion have been found to provide lower conversion than the analogous intermediates with a trifluoroacetate counterion. The examples given below for the preparation of the various Intermediates and Examples usually refer to the **Intermediates H, I, J, K,** or **L** without specifying the exact couterion or salt form, respectively, that was used, as the different salt forms can be typically employed interchangeably in these reactions.

In one aspect, the present invention provides a process of manufacturing a compound of formula (I) as described herein, or a pharmaceutically acceptable salt thereof, wherein said process is as described in any one of schemes 1 to 12 above.

### Using the Compounds of the Invention

As illustrated in the experimental section, the compounds of formula (I) and their pharmaceutically acceptable salts possess valuable pharmacological properties for the treatment or prevention of infections and resulting diseases, particularly bacteremia, pneumonia, meningitis, urinary tract infection, and wound infection, caused by pathogens, particularly by bacteria, more particularly by *Acinetobacter* species, most particularly by *Acinetobacter baumannii.*

The compounds of formula (I) and their pharmaceutically acceptable salts exhibit activity as antibiotics, particularly as antibiotics against *Acinetobacter* species, more particularly as antibiotics against *Acinetobacter baumannii,* most particularly as pathogen-specific antibiotics against *Acinetobacter baumannii.*

The compounds of formula (I) and their pharmaceutically acceptable salts can be used as antibiotics, i.e. as antibacterial pharmaceutical ingredients suitable in the treatment and prevention of bacterial infections, particularly in the treatment and prevention of bacterial infections caused by *Acinetobacter* species, more particularly in the treatment and prevention of bacterial infections caused by *Acinetobacter baumannii.*

The compounds of the present invention can be used, either alone or in combination with other drugs, for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.*

In one aspect, the present invention provides compounds of formula (I) or their pharmaceutically acceptable salts as described herein for use as therapeutically active substances.

In a further aspect, the present invention provides a compound of formula (I) as described herein, or a pharmaceutically acceptable salt thereof, for use as antibiotic.

In a further aspect, the present invention provides a compound of formula (I) as described herein, or a pharmaceutically acceptable salt thereof, for use in the treatment or prevention of nosocomial infections and resulting diseases.

In a particular embodiment, said nosocomial infections and resulting diseases are selected from bacteremia, pneumonia, meningitis, urinary tract infection and wound infection, or a combination thereof.

In a further aspect, the present invention provides a compound of formula (I) as described herein, or a pharmaceutically acceptable salt thereof, for use in the treatment or prevention of infections and resulting diseases caused by Gram-negative bacteria.

In a particular embodiment, said infections and resulting diseases caused by Gram-negative bacteria are selected from bacteremia, pneumonia, meningitis, urinary tract infection and wound infection, or a combination thereof.

In a further aspect, the present invention provides a compound of formula (I) as described herein, or a pharmaceutically acceptable salt thereof, for use in the treatment or prevention of infections and resulting diseases caused by *Enterococcus faecium, Staphylococcus aureus, Klebsiella pneumoniae, Acinetobacter baumannii, Pseudomonas aeruginosa, Enterobacter species* or E. *coli,* or a combination therof.

In a further aspect, the present invention provides a method for the treatment or prevention of infections and resulting diseases caused by *Enterococcus faecium, Staphylococcus aureus, Klebsiella pneumoniae, Acinetobacter baumannii, Pseudomonas aeruginosa, Enterobacter species* or *E. coli,* or a combination therof, which method comprises administering a compound of formula (I) as described herein, or a pharmaceutically acceptable salt thereof, to a mammal.

In a further aspect, the present invention provides the use of a compound of formula (I) as described herein, or a pharmaceutically acceptable salt thereof, as an antibiotic.

In a further aspect, the present invention provides the use of a compound of formula (I) as described herein, or a pharmaceutically acceptable salt thereof, for the treatment or prevention of infections and resulting diseases caused by *Enterococcus faecium, Staphylococcus aureus, Klebsiella pneumoniae, Acinetobacter baumannii, Pseudomonas aeruginosa, Enterobacter species* or *E. coli,* or a combination therof.

In a further aspect, the present invention provides the use of a compound of formula (I) as described herein, or a pharmaceutically acceptable salt thereof, for the preparation of medicaments useful for the treatment or prevention of infections and resulting diseases caused by *Enterococcus faecium, Staphylococcus aureus, Klebsiella pneumoniae, Acinetobacter baumannii, Pseudomonas aeruginosa, Enterobacter species* or *E. coli,* or a combination therof.

In a particular embodiment, said infections and resulting diseases caused by *Enterococcus faecium, Staphylococcus aureus, Klebsiella pneumoniae, Acinetobacter baumannii, Pseudomonas aeruginosa, Enterobacter species* or *E. coli,* or a combination therof, are selected from bacteremia, pneumonia, meningitis, urinary tract infection and wound infection, or a combination thereof.

In a further aspect, the present invention provides compounds of formula (I) or their pharmaceutically acceptable salts as defined above for use in the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.*

In a further aspect, the present invention provides a method for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii,* which method comprises administering a compound of formula (I) or a pharmaceutically acceptable salt thereof as defined above to a mammal.

In a further aspect, the present invention provides the use of compounds of formula (I) or their pharmaceutically acceptable salts as defined above for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.*

In a further aspect, the present invention provides the use of compounds of formula (I) or their pharmaceutically acceptable salts as defined above for the preparation of medicaments for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.* Such medicaments comprise compounds of formula (I) or their pharmaceutically acceptable salts as defined above.

### Pharmaceutical Compositions and Administration

In one aspect, the present invention provides pharmaceutical compositions comprising compounds of formula (I) or their pharmaceutically acceptable salts as defined above and one or more pharmaceutically acceptable excipients. Exemplary pharmaceutical compositions are described in Examples 203 to 206.

In a further aspect, the present invention relates to pharmaceutical compositions comprising compounds of formula (I) or their pharmaceutically acceptable salts as defined above and one or more pharmaceutically acceptable excipients for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.*

The compounds of formula (I) and their pharmaceutically acceptable salts can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays) or rectally (e.g. in the form of suppositories). However, the administration can also be effected parentally, such as intramuscularly or intravenously (e.g. in the form of injection solutions or infusion solutions).

The compounds of formula (I) and their pharmaceutically acceptable salts can be processed with pharmaceutically inert, inorganic or organic excipients for the production of tablets, coated tablets, dragées and hard gelatin capsules. Lactose, corn starch or derivatives thereof, talc, stearic acid or its salts etc. can be used, for example, as such excipients for tablets, dragées and hard gelatin capsules.

Suitable excipients for soft gelatin capsules are, for example, vegetable oils, waxes, fats, semisolid substances and liquid polyols, etc.

Suitable excipients for the production of solutions and syrups are, for example, water, polyols, saccharose, invert sugar, glucose, etc.

Suitable excipients for injection solutions are, for example, water, alcohols, polyols, glycerol, vegetable oils, etc.

Suitable excipients for suppositories are, for example, natural or hardened oils, waxes, fats, semisolid or liquid polyols, etc.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should be appropriate. It will, however, be clear that the upper limit given herein can be exceeded when this is shown to be indicated.

### Co-Administration of Compounds of Formula (I) and Other Agents

The compounds of formula (I) or salts thereof or a compound disclosed herein or a pharmaceutically acceptable salt thereof may be employed alone or in combination with other agents for treatment. For example, the second agent of the pharmaceutical combination formulation or dosing regimen may have complementary activities to the compound of formula (I) such that they do not adversely affect each other. The compounds may be administered together in a unitary pharmaceutical composition or separately. In one embodiment a compound or a pharmaceutically acceptable salt can be co-administered with an antibiotic, in particular with an antibiotic for the treatment or prevention of infections and resulting diseases caused by *Enterococcus faecium, Staphylococcus aureus, Klebsiella pneumoniae, Acinetobacter baumannii, Pseudomonas aeruginosa, Enterobacter species* or *E. coli,* or a combination therof.

The term "co-administering" refers to either simultaneous administration, or any manner of separate sequential administration, of a compound of formula (I) or a salt thereof or a compound disclosed herein or a pharmaceutically acceptable salt thereof and a further active pharmaceutical ingredient or ingredients, including antibiotic agents. If the administration is not simultaneous, the compounds are administered in a close time proximity to each other. Furthermore, it does not matter if the compounds are administered in the same dosage form, e.g. one compound may be administered intravenously and another compound may be administered orally.

Typically, any agent that has antimicrobial activity may be co-administered. Particular examples of such agents are Carbapenems (meropenem), Fluoroquinolone (Ciprofloxacin), Aminoglycoside (amikacin), Tetracyclines (tigecycline), Colistin, Sulbactam, Sulbactam+Durlobactam, Cefiderocol (Fetroja), macrocyclic peptides as exemplified e.g. in WO 2017072062 A1, WO 2019185572 A1 and WO 2019206853 A1, and Macrolides (erythromycin).

In one aspect, the present invention provides a pharmaceutical composition described herein, further comprising an additional therapeutic agent.

In one aspect, the present invention provides a pharmaceutical combination comprising a compound of formula (I) described herein and an additional therapeutic agent.

In one embodiment, said additional therapeutic agent is an antibiotic agent.

In one embodiment, said additional therapeutic agent is an antibiotic agent that is useful for the treatment or prevention of infections and resulting diseases caused by *Enterococcus faecium, Staphylococcus aureus, Klebsiella pneumoniae, Acinetobacter baumannii, Pseudomonas aeruginosa, Enterobacter species* or *E. coli,* or a combination therof.

In one embodiment, said additional therapeutic agent is an antibiotic agent selected from Carbapenems (meropenem), Fluoroquinolone (Ciprofloxacin), Aminoglycoside (amikacin), Tetracyclines (tigecycline), Colistin, Sulbactam, Sulbactam+Durlobactam, Cefiderocol (Fetroja), macrocyclic peptides as exemplified in WO 2017072062 A1, WO 2019185572 A1 and WO 2019206853 A1, and Macrolides (erythromycin).

### Examples

The invention will be more fully understood by reference to the following examples. The claims should not, however, be construed as limited to the scope of the examples.

In case the preparative examples are obtained as a mixture of enantiomers, the pure enantiomers can be separated by methods described herein or by methods known to the man skilled in the art, such as e.g., chiral chromatography (e.g., chiral SFC) or crystallization.

All reaction examples and intermediates were prepared under an argon atmosphere if not specified otherwise.

### Intermediate A1

### 5-Bromopyrazolo[4,3-b]pyridine-1-carboxylic acid tert-butyl ester

A mixture of 5-bromo-1H-pyrazolo[4,3-b]pyridine (150 mg, 0.758 mmol, 1 eq) in dichloromethane, extra dry (3.5 mL) / tetrahydrofuran, extra dry (3.5 mL) was treated with triethylamine (114.98 mg, 158.15 µL, 1.14 mmol, 1.5 eq), di-tert-butyl dicarbonate (214.92 mg, 226.23 µL, 0.985 mmol, 1.3 eq), and 4-dimethylaminopyridine (12 mg, 0.098 mmol, 0.130 eq). The mixture was stirred at RT for 2 h, and then quenched by the addition of water (50 mL). The phases were separated, and the aq. phase was extracted with DCM (3 x 20 mL). The combined organics were washed with brine (20 mL), dried (Na₂SO₄), filtered, and evaporated. Purification by column chromatography (0-30% DCM/MeOH in DCM) afforded the title compound **Intermediate A1** (151 mg, 64%) as white solid. MS [M+H]⁺ 298.1.

### Intermediate A2

### 2-[(3-Iodoindazol-1-yl)methoxy]ethyl-trimethyl-silane

A solution of 3-iodo-1H-indazole (2.0 g, 8.2 mmol, 1 eq) in DMF (20 mL) was treated with sodium hydride, 60% in oil (0.36 g, 9.02 mmol, 1.1 eq) at 0 °C, stirred for 1 h, and treated with 2-(trimethylsilyl)ethoxymethyl chloride (1.6 mL, 9.02 mmol, 1.1 eq). The reaction mixture was stirred at 0°C for 1 h, poured into NH₄Cl solution (100 mL), and extracted with EtOAc (2 x 50 mL). The combined organics were washed with brine (100 mL), dried over Na₂SO₄, filtered, and concentrated. Purification by column chromatography (petroleum ether/EtOAc 3:1) to afford the title compound Intermediate A2 (2.2 g, 5.88 mmol, 72%) as colorless oil. MS [M+H]⁺ 375.0.

### Intermediate A7

### 2-[[5-bromo-2-(2,2-dimethyl-1,3-dioxan-5-yl)pyrrolo[3,2-b]pyridin-1-yl]methoxy]ethyl-trimethyl-silane

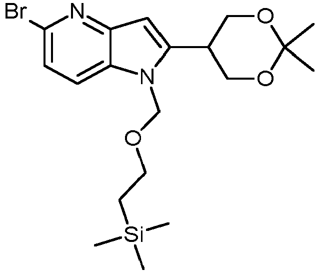

### Step 1: 2,2-dimethyl-1,3-dioxane-5-carbaldehyde

To a solution of oxalyl chloride (10.42 g, 82.09 mmol, 1.2 eq) in DCM (100 mL) was added dimethylsulfoxide (14.56 mL, 205.23 mmol, 3.0 eq) at -78 °C under N₂ and stirred for 15 min. To the mixture was added (2,2-dimethyl-1,3-dioxan-5-yl)methanol (10.0 g, 68.41 mmol, 1.0 eq) in DCM (50 mL) at -78 °C under N₂ and stirred for 1.5 h. Then the mixture was added triethylamine (47.67 mL, 342.04 mmol, 5.0 eq) and stirred for 1 h. Water (200 mL) was added and layers were separated, the mixture was then extracted with DCM (2 x 100 mL). Combined extracts were washed with brine (300 mL), dried over Na₂SO₄, filtered, and concentrated under vacuum to afford the title compound 2,2-dimethyl-1,3-dioxane-5-carbaldehyde (8.1 g, 56.18 mmol, 82.1%) as a yellow oil. 1H NMR (400 MHz, CHLOROFORM-d) δ = 9.85 (s, 1H), 4.22 - 4.13 (m, 4H), 2.36 - 2.32 (m, 1H), 1.45 (s, 3H), 1.35 (s, 3H).

### Step 2: 5-ethynyl-2,2-dimethyl-1,3-dioxane

To a solution of 2,2-dimethyl-1,3-dioxane-5-carbaldehyde (7.0 g, 48.55 mmol, 1.0 eq) in methanol (200 mL) and THF (100 mL) was added 1-diazonio-1-dimethoxyphosphorylprop-1-en-2-olate (18.66 g, 97.11 mmol, 2.0 eq) and potassium carbonate (26.84 g, 194.22 mmol, 4.0 eq). The mixture was stirred at 25 °C for 17 h. Water (500 mL) and PE (300 ml) were added and layers were separated, the mixture was then extracted with PE (2 x 30 mL). Combined extracts were washed with brine (1 L), dried over Na₂SO₄, filtered, and concentrated under vacuum to afford the title compound 5-ethynyl-2,2-dimethyl-1,3-dioxane (4.3 g, 30.67 mmol, 63.2%) as yellow oil, which was used without further purificaiton. 1H NMR (400 MHz, CHLOROFORM-d) δ = 3.97 - 3.91 (m, 2H), 3.88 - 3.79 (m, 2H), 2.85 - 2.76 (m, 1H), 2.10 (d, J = 2.4 Hz, 1H), 1.46 (s, 3H), 1.38 (s, 3H).

### Step 3: 6-bromo-2-iodo-pyridin-3-amine

To a solution of 5-amino-2-bromopyridine (20.0 g, 115.6 mmol, 1.0 eq) in DMF (200 mL) was N-iodosuccinimide (31.21 g, 138.72 mmol, 1.2 eq) at 20°C in one portion and the reaction stirred at 20 °C for 16 h. The mixture was diluted with EtOAc (500 mL), washed with sat.Na2SO3 solution (500 mL) and brine (500 mL), dried over Na₂SO₄, concentrated in vacuo. The residue was purified by silica column chromatography (70% PE in EtOAc ) to afford the title compound 6-bromo-2-iodo-pyridin-3-amine (31.0 g, 103.71 mmol, 89.7%) as red solid. MS [M+H]⁺ 298.9.

### Step 4: 6-bromo-2-[2-(2,2-dimethyl-1,3-dioxan-5-yl)ethynyl]pyridin-3-amine

A mixture of 6-bromo-2-iodo-pyridin-3-amine (5.0 g, 16.73 mmol, 1.0 eq), 5-ethynyl-2,2-dimethyl-1,3-dioxane (2.46 g, 17.54 mmol, 1.05 eq), triethylamine (6.99 mL, 50.18 mmol, 3.0 eq), copper(I) iodide (0.06 mL, 1.67 mmol, 0.1 eq) and [1,1'bis(diphenylphosphino)ferrocene]dichloropalladium(II) (1223.97 mg, 1.67 mmol, 0.1 eq) in THF (50 mL) was evacuated and backfilled with N₂ (3×). The yellow mixture was stirred under N₂ at 60 °C for 16 h. The reaction mixture was poured into water (100 mL), extracted with EA (3 x 100 mL), the combined organic layer was washed with brine and dried over Na₂SO₄, and concentrated in vacuum. The residue was purified by column chromatography (30% of EA in PE) to afford the title compound 6-bromo-2-[2-(2,2-dimethyl-1,3-dioxan-5-yl)ethynyl]pyridin-3-amine (2.8 g, 9.0 mmol, 44.4%) as yellow solid. MS [M+H]⁺ 311.2.

### Step 5: 5-bromo-2-(2,2-dimethyl-1,3-dioxan-5-yl)-1H-pyrrolo[3,2-b]pyridine

To a yellow solution of 6-bromo-2-[2-(2,2-dimethyl-1,3-dioxan-5-yl)ethynyl]pyridin-3-amine (2.8 g, 9.0 mmol, 1.0 eq) in 1-methyl-2-pyrrolidinone (50 mL) was added potassium tert-butoxide (2.52 g, 22.5 mmol, 2.5 eq) in one portion at 20°C. The mixture was stirred at 50 °C for 2 h. The mixture was slowly poured into cooled HCl solution (0.05 M, 5 mL) and water (100 ml), extracted with EtOAc (3 x 60 mL), washed with brine (100 mL×2), dried over Na₂SO₄, and concentrated in vacuum. The crude was purified by silica column (50-100% EA in PE) and concentrated to afford the title compound 5-bromo-2-(2,2-dimethyl-1,3-dioxan-5-yl)-1H-pyrrolo[3,2-b]pyridine (2.5 g, 8.03 mmol, 78.8%) as yellow solid. MS [M+H]⁺ 311.2.

### Step 6: 2-[[5-bromo-2-(2,2-dimethyl-1,3-dioxan-5-yl)pyrrolo[3,2-b]pyridin-1-yl]methoxy]ethyl-trimethyl-silane

To a solution of 5-bromo-2-(2,2-dimethyl-1,3-dioxan-5-yl)-1H-pyrrolo[3,2-b]pyridine (2.15 g, 6.91 mmol, 1.0 eq) in ACN (15 mL) and DMF (15 mL) was added cesium carbonate (4.5 g, 13.82 mmol, 2.0 eq). The reaction mixture was stirred at 0 °C for 1 h under N₂. Then 2-(trimethylsilyl)ethoxymethyl chloride (1.47 mL, 8.29 mmol, 1.2 eq) was added. The mixture was stirred for 1 hour at 0°C under N₂. EtOAc (50 mL) and water (50 mL) were added and layers were separated, the mixture was then extracted with EtOAc (2 x 50 mL). Combined extracts were washed with brine (100 mL), dried over Na₂SO₄, filtered, and concentrated under vacuum. The crude was purified by silica gel chromatography eluting with 25% EA in PE to afford the title compound 2-[[5-bromo-2-(2,2-dimethyl-1,3-dioxan-5-yl)pyrrolo[3,2-b]pyridin-1-yl]methoxy]ethyl-trimethyl-silane (2.5 g, 5.66 mmol, 76.6%) as yellow oil. MS [M+H]⁺ 443.2.

### Intermediate A8

### (5-chloro-1-methyl-pyrrolo[3,2-b]pyridin-2-yl)-morpholino-methanone

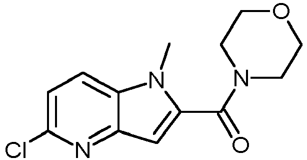

### Step 1: (5-chloro-1H-pyrrolo[3,2-b]pyridin-2-yl)-morpholino-methanone

To a solution of 5-chloro-1H-pyrrolo[3,2-b]pyridine-2-carboxylic acid **(Intermediate** A6 Step 1; 1.0 g, 5.09 mmol, 1.0 eq), morpholine (0.47 g, 5.34 mmol, 1.05 eq) and N,N-diisopropylethylamine (1.06 mL, 6.1 mmol, 1.2 eq) in DMF (2 mL) was added O-(7-azabenzotriazol-1-yl)-N,N,N',N'-tetramethyluronium hexafluorophosphate (2.13 g, 5.6 mmol, 1.1 eq) at 0 °C. The mixture was stirred at 0 °C for 2 h. The mixture was poured into water (30 mL) and then extracted with EA (3 x 40 mL). The combined organic layers were washed with brine (3 x 60 mL), dried over sodium sulfate, filtered, and concentrated under vacuum. The residue was purified by column chromatography (0-100% ACN in water(FA)) and then concentrated under vacuum to afford the title compound (5-chloro-1H-pyrrolo[3,2-b]pyridin-2-yl)-morpholino-methanone (1.2 g, 4.52 mmol, 88.8%) as a light yellow solid. MS [M+H]⁺ 266.1.

### Step 2: (5-chloro-1-methyl-pyrrolo[3,2-b]pyridin-2-yl)-morpholino-methanone

To a solution of (5-chloro-1H-pyrrolo[3,2-b]pyridin-2-yl)-morpholino-methanone (1.2 g, 4.52 mmol, 1.0 eq) in DMF (15 mL) was added sodium hydride, 60% in oil (0.27 g, 6.77 mmol, 1.5 eq) .The mixture was stirred at 25 °C for 0.5 h.To the solution was added iodomethane (0.42 mL, 6.77 mmol, 1.5 eq) and stirred at 25 °C for another 16 h. The mixture was poured into water (80 mL) and then extracted with EA (3 x 40 mL). The combined organic layers were washed with brine (3 x 60mL), dried over sodium sulfate, filtered, and concentrated under vacuum to afford the title compound (5-chloro-1-methyl-pyrrolo[3,2-b]pyridin-2-yl)-morpholino-methanone (1.2 g, 4.29 mmol, 84.5%) as a light yellow solid, whichh was used without further purification. MS [M+H]⁺ 280.0.

The following examples were prepared in analogy of **Intermediate A8**

| **Ex#** | **Name** | **MS ESI** | **Starting Material** |
|---|---|---|---|
| **Interme diate A9** | 5-chloro-N-(2-hydroxyethyl)-1-methyl-pyrrolo[3,2-b]pyridine-2-carboxamide | 254.1 [M+H]⁺ | 2-hydroxyethylamine |

### Intermediate A12

### N-[2-(difluoromethoxy)ethyl]-3-fluoro-4-iodo-aniline

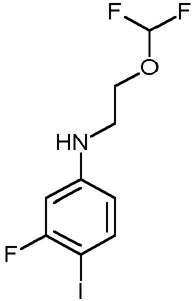

### Step 1: benzyl 2-(difluoromethoxy)acetate

To a mixture of benzyl glycolate (20.0 g, 120.36 mmol, 1.0 eq) in MeCN (50 mL) was added copper (l) iodide (5.73 g, 30.09 mmol, 0.25 eq). The reaction mixture was stirred at 50 °C. Then, 2,2-difluoro-2-(fluorosulfonyl)acetic acid (32.15 g, 180.54 mmol, 1.5 eq) in MeCN (250 mL) was dropwise added into the above solution over 20 min. After addition, the reaction mixtrue was stirred at 50 °C for 1 h under N₂. The mixture was concentrated in vacuum and the crude product purified by silica column (5% EA in PE) to afford the title compound benzyl 2-(difluoromethoxy)acetate (7.8 g, 36.08 mmol, 30%) as colorless oil. 1H NMR (400 MHz, CHLOROFORM-d) δ = 7.41 - 7.38 (m, 5H), 6.38 (t, J = 73.6, 1H), 5.25 (s, 2H), 4.49 (s, 2H).

### Step 2: 2-(difluoromethoxy)acetic acid

To a mixture of benzyl 2-(difluoromethoxy)acetate (7.8 g, 36.08 mmol, 1.0 eq) in THF (50 mL) was added lithium hydroxide (1.7 mL, 180.41 mmol, 5.0 eq) .The reaction mixture was stirred at 25 °C for 16 h. EtOAc (100 mL) and water (50 mL) were added and layers were separated. The water was washed with EtOAc (2 x 100 mL). The water was adjusted to pH=6 with 1 M HCl, and the mixture was then extracted with EtOAc (3 x 50 mL). Combined extracts were washed with brine (100 mL), dried over Na₂SO₄, filtered, and concentrated under vacuum to afford the title compound 2-(difluoromethoxy)acetic acid (3.3 g, 26.18 mmol, 72.6%) as yellow oil. 1H NMR (400 MHz, CHLOROFORM-d) δ = 6.35 (t, J = 73.2, 1H), 4.47 (s, 2H).

### Step 3: 2-(difluoromethoxy)-N-(3-fluoro-4-iodo-phenyl)acetamide

A mixture of 3-fluoro-4-iodoaniline (4.0 g, 16.88 mmol, 1.0 eq), 2-(difluoromethoxy)acetic acid (3.19 g, 25.32 mmol, 1.5 eq), triethylamine (7.06 mL, 50.63 mmol, 3.0 eq) in DMF (50 mL) was added O-(7-azabenzotriazol-1-yl)-N,N,N',N'-tetramethyluronium hexafluorophosphate (10.91 g, 28.69 mmol, 1.7 eq) at 25 °C and stirred at 25 °C for 16 h. The solution was poured into water (80 mL), extracted with EtOAc (50 mL*2), washed with brine (100 mL), concentrated to afford a residue, which was purified by silica column (20% EA in PE) and concentrated. The obtained material was again purified by RPHPLC (50-80% ACN in water, 0.1% FA) and lyophilized to afford the title compound 2-(difluoromethoxy)-N-(3-fluoro-4-iodo-phenyl)acetamide (3.5 g, 10.14 mmol, 60.1%) as light yellow solid. 1H NMR (400 MHz, CHLOROFORM-d) δ = 8.02 (br s, 1H), 7.69 (dd, J = 7.2, 8.4 Hz, 1H), 7.59 (dd, J = 2.4, 9.6 Hz, 1H), 7.04 (dd, J = 2.0, 8.4 Hz, 1H), 6.41 (t, J = 72.4, 1H), 4.48 (s, 2H).

### Step 4: N-[2-(difluoromethoxy)ethyl]-3-fluoro-4-iodo-aniline

A mixture of 2-(difluoromethoxy)-N-(3-fluoro-4-iodo-phenyl)acetamide (2.0 g, 5.8 mmol, 1.0 eq) in THF (30 mL) was cooled to 0 °C, then borane-tetrahydrofuran complex (16.0 mL, 16.0 mmol, 2.76 eq) was added dropwise. The reaction was stirred 14 hours at 50 °C. The resulting mixture was cooled to 0 °C and methanol (30 mL) was added. The reaction mixture was then stirred at 80°C for 2 h. The reaction mixture was poured into water (60 mL), extracted with EtOAc (2 x 40 mL), washed with brine (80 mL), and concentrated. The crude material was purified by silica column (20% EA in PE) and concentrated to afford the title compound **Intermediate A12** (800.0 mg, 2.42 mmol, 36%) as yellow oil. MS [M+H]⁺ 332.2.

### Intermediate A4

### methyl 3-[5-bromo-1-(2-trimethylsilylethoxymethyl)pyrrolo[3,2-b]pyridin-2-yl]propanoate

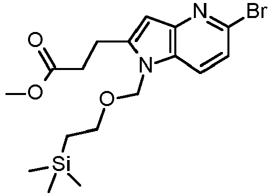

### Step 1: methyl 3-(5-bromo-1H-pyrrolo[3,2-b]pyridin-2-yl)propanoate

A mixture of 5-bromo-4-azaindole (2.0 g, 10.15 mmol, 1.0 eq), methyl 3-bromopropionate (2.03 g, 12.18 mmol, 1.2 eq), Pd(PhCN)₂Cl₂ (0.39 g, 1.02 mmol, 0.1 eq), norbornene (1.91 g, 20.3 mmol, 2.0 eq), NaHCO₃ (3.41 g, 40.6 mmol, 4.0 eq) and water (0.18 mL, 10.15 mmol, 1.0 eq) in DMF (20 mL) was stirred under N₂ at 80 °C for 15 h. The reaction mixture was cooled to RT. EtOAc (30 mL) and water (80 mL) were added, and the layers were separated. The aq. phase was extracted with EtOAc (2 x 30 mL). The combined organic layers were washed with brine (40 mL), dried over MgSO4, filtered, and concentrated under vacuum to give a residue. The residue was purified by silica gel chromatography eluting with PE:EA=1:1 (TLC, PE:EA=1:1, Rf=0.2, UV) and concentrated under vacuum to afford compound the title compound (600.0 mg, 2.12 mmol, 20.88%) as yellow oil. MS [M+H]⁻ 283.0.

### Step 2: methyl 3-[5-bromo-1-(2-trimethylsilylethoxymethyl)pyrrolo[3,2-b]pyridin-2-yl]propanoate

To a solution of methyl 3-(5-bromo-1H-pyrrolo[3,2-b]pyridin-2-yl)propanoate (600.0 mg, 2.12 mmol, 1.0 eq) and cesium carbonate (1.38 g, 4.24 mmol, 2.0 eq) in ACN (8 mL) was added was added 2-(trimethylsilyl)ethoxymethyl chloride (0.45 mL, 2.54 mmol, 1.2 eq) at 0°C. The mixture was stirred for 1 h at 0 °C under N₂. The reaction mixture was filtered and concentrated under vacuum to give a residue. The residue was purified by silica gel chromatography (PE/EA 5:1) to afford the title compound **Intermediate A4** (400.0 mg, 0.97 mmol, 45.7%) as yellow oil. MS [M+H]⁺ 414.8.

### Intermediate A3

### 3-(5-bromo-1H-pyrrolo[3,2-b]pyridin-2-yl)propoxy-tert-butyl-dimethyl-silane

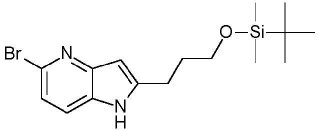

A vial was charged with 5-bromo-4-azaindole (600.0 mg, 3.05 mmol, 1.0 eq), (3-bromopropoxy)-tert-butyldimethylsilane (1156.8 mg, 4.57 mmol, 1.5 eq), Pd(PhCN)₂Cl₂ (587.07 mg, 1.52 mmol, 0.5 eq), norbornene (573.44 mg, 6.09 mmol, 2.0 eq), potassium carbonate (420.87 mg, 3.05 mmol, 1.0 eq), water (0.01 mL, 0.76 mmol, 0.25 eq) and DMF (10 mL). The mixture was stirred under N₂ at 80 °C for 15 h. The reaction mixture was cooled to RT. EtOAc (20 mL) and water (30 mL) were added, and the layers were separated. The aq. phase was extracted with EtOAc (20 mL x 2), and the combined organic layers were washed with brine (40 mL), dried over MgSO4, filtered, and concentrated under vacuum to give a residue. The residue was purified by silica gel chromatography (PE/EA 1:1) to afford the title compound **Intermediate A3** (200.0 mg, 0.54 mmol, 17.8%) as yellow solid. MS [M+H]⁺ 369.2.

### Intermediate A5

### 4-(4-bromo-3-fluoro-phenyl)morpholine

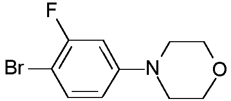

A mixture of 1-bromo-2-fluoro-4-iodobenzene (2.0 g, 6.65 mmol, 1.0 eq), morpholine (0.58 g, 6.65 mmol, 1.0 eq), (2S)-pyrrolidine-2-carboxylic acid (0.15 g, 1.33 mmol, 0.2 eq) and copper(I) iodide (0.05 mL, 1.33 mmol, 0.2 eq) in DMSO (20 mL) was evacuated and backfilled with N₂ (3×). Then the mixture was stirred under N₂ at 40 °C for 16 h. EtOAc (40 mL) and water (40 mL) were added, and the mixture was filtered. Then the filtrate were separated. The aqueous phase was extracted with EtOAc (2 x 30 mL). The combined extracts were washed with brine (100 mL), dried over Na₂SO₄, filtered, and concentrated under vacuum. The residue was purified by column chromatography (45% EA in PE) to afford the title compound **Intermediate A5** (330.0 mg, 1.27 mmol, 19.1%) as yellow soild. MS [M+H]⁺ 261.9.

### Intermediate A14

### 2-[[2-(2-azidoethyl)-5-bromo-pyrrolo[3,2-b]pyridin-1-yl]methoxy]ethyl-trimethyl-silane

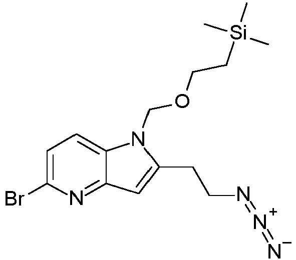

### Step 1: 2-[(5-bromopyrrolo[3,2-b]pyridin-1-yl)methoxy]ethyl-trimethyl-silane

A solution of 5-bromo-4-azaindole (3.5 g, 17.76 mmol, 1.0 eq) in THF (30 mL) was cooled to 0 °C, and then sodium hydride 60% in oil (1.07 g, 26.65 mmol, 1.5 eq) was added in several portions. The mixture was stirred for 0.5 hours at 0 °C under N₂.The reaction mixture was treated with 2-(trimethylsilyl)ethoxymethyl chloride (3.77 mL, 21.32 mmol, 1.2 eq) in several portions, stirred at 0 °C for 2 h, and warmed to 20 °C for 4 h. To the reaction mixture was added water (100 mL), and EtOAc (200 mL) and additional water (200 mL) were added, and the layers were separated. The aqueous layer was extracted with EtOAc (4 x 80 mL). The combined extracts were concentrated under vacuum. The residue was purified by silica gel chromatography (PE/EA 4:1). The obtained material was further purified by RP column chromatography (80-90% ACN in water, 0.1% TFA) to afford the title compound 2-[(5-bromopyrrolo[3,2-b]pyridin-1-yl)methoxy]ethyl-trimethyl-silane (5.8 g, 17.72 mmol, 99.8%) as light brown oil. MS [M+H]⁺ 329.1.

### Step 2: 2-[5-bromo-1-(2-trimethylsilylethoxymethyl)pyrrolo[3,2-b]pyridin-2-yl]ethanol

A solution of 2-[(5-bromopyrrolo[3,2-b]pyridin-1-yl)methoxy]ethyl-trimethyl-silane (3.8 g, 11.61 mmol, 1.0 eq) in THF (80 mL) was stirred under N₂ at -78 °C for 30 min. To the mixture was added LDA (7.55 mL, 15.09 mmol, 1.3 eq) dropwise, and the mixture was stirred at -78 °C for 1 h. Then, the mixture was treated with 1,3,2-dioxathiolane 2,2-dioxide (1.87 g, 15.09 mmol, 1.3 eq) in THF (10 mL), and stirred at -78 °C for 1 h. Finally, the mixture was treated with 12 M HCl (4.07 mL, 48.86 mmol, 4.21 eq) at 0 °C, and subsequently stirred at 25 °C for 16 h. The reaction mixture was diluted with EtOAc (200 mL), and poured into aq. NaHCO₃ (100 mL). The aq. phase was extracted with EtOAc (3 x 200 mL). The combined organic layers were washed with water (2 x 300 mL), brine (300 mL), dried over Na₂SO₄, filtered, and concentrated under vacuum. The residue was purified by column chromatography (20-80% EtOAc in PE) to afford 2-[5-bromo-1-(2-trimethylsilylethoxymethyl)pyrrolo[3,2-b]pyridin-2-yl]ethanol (600.0 mg, 1.62 mmol, 13.9%) as light yellow solid. MS [M+H]⁺ 373.1.

### Step 3: 2-[5-bromo-1-(2-trimethylsilylethoxymethyl)pyrrolo[3,2-b]pyridin-2-yl]ethyl 4-methylbenzenesulfonate

To a solution of 2-[5-bromo-1-(2-trimethylsilylethoxymethyl)pyrrolo[3,2-b]pyridin-2-yl]ethanol (1.2 g, 3.23 mmol, 1.0 eq) in DCM (10 mL) was s added triethylamine (0.9 mL, 6.46 mmol, 2.0 eq), and the mixture was stirred at 0 °C. Then, p-toluenesulfonyl chloride was added (739.31 mg, 3.88 mmol, 1.2 eq), and the mixture stirred at 0 °C for 1 h, and warmed to 25 °C for 16 h. Then the reaction mixture was diluted with EtOAc (20 mL), and poured into aq. NaHCO₃ (20 mL). The water layer was extracted with EtOAc (3 x 20 mL). The combined organic layers were washed with water (2 x 30 mL), brine (30 mL), dried over Na₂SO₄, filtered, and concentrated under vacuum. The residue was purified by column chromatography (17-33% EA in PE) to afford the title compound 2-[5-bromo-1-(2-trimethylsilylethoxymethyl)pyrrolo[3,2-b]pyridin-2-yl]ethyl 4-methylbenzenesulfonate (1.2 g, 2.28 mmol, 70.7%) as light yellow solid. MS [M+H]⁺ 527.0.

### Step 4: 2-[[2-(2-azidoethyl)-5-bromo-pyrrolo[3,2-b]pyridin-1-yl]methoxy]ethyl-trimethylsilane

To a solution of 2-[5-bromo-1-(2-trimethylsilylethoxymethyl)pyrrolo[3,2-b]pyridin-2-yl]ethyl 4-methylbenzenesulfonate (1.2 g, 2.28 mmol, 1.0 eq) in DMF (20 mL) was added sodium azide (0.19 g, 2.92 mmol, 1.28 eq), and the mixture stirred at 25 °C for 16 h. The reaction mixture was diluted with aq. NaHCO₃ (50 mL), and then extracted with EA (3 x 30 mL). The combined extracts were washed with brine (50 mL), dried over Na₂SO₄, filtered, and concentrated under vacuum. The residue was purified by RP column chromatography (90-100% ACN in water, 0.1%FA) to afford the title compound **Intermediate A14** (740.0 mg, 1.87 mmol, 81.8%) as light yellow oil. MS [M+H]⁺ 398.0.

### Intermediate A44

### (5-bromo-1H-pyrrolo[3,2-b]pyridin-2-yl)methanol

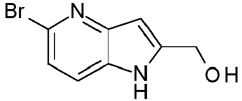

5-bromo-1H-pyrrolo[3,2-b]pyridine-2-carboxylic acid ethyl ester (CAS 1255098-82-8; 1790 mg, 6.65 mmol, 1 eq) in THF (40 mL) was treated dropwise with 1 M DIBAL-H in THF (25.28 mL, 25.28 mmol, 3.8 eq) and stirred at -78 °C for 1 h. The reaction mixture was then warmed to RT and stirred for a further 2 h. The reaction mixture was then cooled to 0 °C, and quenched with EtOAc (5 mL), and subsequently Rochelle's salt (25 mL) was added. The mixture was stirred overnight, and the organic solvent was removed in vacuo. The reaction mixture was diluted with water (60 mL) and brine (20 mL), and extracted with EtOAc (4 x100 mL). The combined organcis were washed with brine, dried (Na₂SO₄), filtered, and concentrated to give the title compound **Intermediate A44** (917 mg, 83.3%) as a light brown solid. MS [M+H]⁺ 227.04.

### Intermediate A15

### (2S)-3-(3-fluoro-4-iodo-anilino)propane-1,2-diol

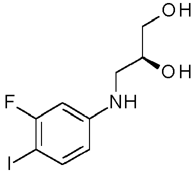

To a solution of 3-fluoro-4-iodoaniline (CAS 656-66-6; 2.0 g, 8.44 mmol, 1.0 eq) in methanol (30 mL) was added (R)-(+)-glycidol (1.06 g, 14.35 mmol, 1.7 eq) and stirred at 90 °C for 16 h. Then, the mixture was treated with triethylamine (1.76 mL, 12.66 mmol, 1.5 eq) and stirred at 90 °C for 2 h. The mixture was concentrated in vacuum, and the residue was purified by column chromatography (50% EA in PE) to afford the title compound **Intermediate A15** (1.8 g, 5.79 mmol, 68.6%) as a yellow solid. MS [M+H]⁺ 312.2.

### Intermediate A31

### 1-(3-Fluoro-4-iodo-anilino)-2-methyl-propan-2-ol

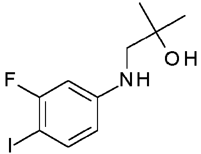

To a light yellow solution of 3-fluoro-4-iodoaniline (2.0 g, 8.44 mmol, 1.0 eq) in tert-butanol (10 mL) was added potassium tert-butoxide (2.84 g, 25.32 mmol, 3.0 eq) and isobutylene oxide (3.04 g, 42.19 mmol, 5.0 eq) at 10°C, and the solution was heated to 80 °C and stirred for 16 h. The mixture was concentrated in vacuum. The crude material was purified by column chromatography (30% EA in PE) and concentrated in vacuum to afford the title compound **Intermediate A31** (650.0 mg, 2.1 mmol, 24.9%) as a yellow solid. MS [M+H]⁺ 310.0.

### Intermediate A17

### (6-bromo-1H-pyrrolo[3,2-c]pyridin-2-yl)-morpholino-methanone

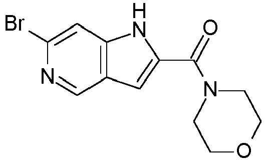

A mixture of 6-bromo-1H-pyrrolo[3,2-c]pyridine-2-carboxylic acid **(Intermediate A11** Step 2; 250 mg, 622.3 µmol, 1 eq) in N,N-dimethylformamide (1.75 mL) and DIPEA (402.16 mg, 543.46 µL, 3.11 mmol, 5 eq) was treated with morpholine (70.48 mg, 70.48 µL, 808.99 umol, 1.3 eq) and HATU (283.94 mg, 746.76 µmol, 1.2 eq), and the reaction mixture was stirred at RT for 60 min. Water was added, and the mixture was extracted with ethyl acetate (3x). The combined organics were washed twice with 5 % lithium chloride, and brine, dried over sodium sulfate, filtered, and evaporated to afford the title compound **Intermediate A17** (100.3 mg, 46.8%) as a yellow solid, which was directly used in the next step without any further purification. MS [M+H]⁺ 310.06.

### Intermediate A19

### 5-bromo-N-methyl-pyrrolo[3,2-b]pyridine-1-carboxamide

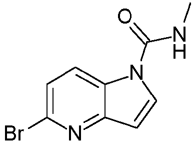

5-bromo-1H-pyrrolo[3,2-b]pyridine (120 mg, 609.04 µmol, 1 eq) was dissolved in acetonitrile, extra dry (1 mL). DMAP (7.44 mg, 60.9 µmol, 0.1 eq) and CDI (148.13 mg, 913.57 µmol, 1.5 eq) were added to the mixture, and it was stirred at 90 °C overnight. 2 M methylamine in THF (609.04 µL, 1.22 mmol, 2 eq) was added, and stirring was continued at 90 °C overnight. The mixture was concentrated in vacuo, and the residue was purified by column chromatography (0-50% DCM/MeOH 9:1 in DCM) to yield the title compound **Intermediate A19** (45 mg, 29.1%) as a yellow solid. MS [M+H]⁺ 254.04.

### Intermediate A24

### 2-[2-[tert-butyl(dimethyl)silyl]oxyethyl]-6-chloro-N,N-dimethyl-pyrrolo[3,2-c]pyridine-1-sulfonamide

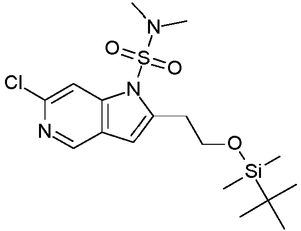

### Step 1: 6-chloro-N,N-dimethyl-pyrrolo[3,2-c]pyridine-1-sulfonamide

To a solution of 6-chloro-5-azaindole (9.0 g, 58.99 mmol, 1.0 eq) in DCM (300 mL) was added tetrabutylammonium hydrogen sulfate (2.0 g, 5.9 mmol, 0.1 eq), sodium hydroxide (5.9 g, 147.46 mmol, 2.5 eq) and dimethylsulfamoyl chloride (12.7 g, 88.48 mmol, 1.5 eq). The reaction mixture was stirred at 20 °C for 4 h. The mixture was concentrated in vacuum. The residue was poured into water (500 mL), extracted with EtOAc (3 x 200 mL). The combined organic layers were washed with brine, dried over Na₂SO₄, filtered, and concentrated in vacuum. The crude material was purified by column chromatography (9-25% EA in PE) to afford the title compound 6-chloro-N,N-dimethyl-pyrrolo[3,2-c]pyridine-1-sulfonamide (13.4 g, 51.6 mmol, 80.2%) as a light yellow solid. MS [M+H]⁺ 260.0.

### Step 2: 6-chloro-2-(2-hydroxyethyl)-N,N-dimethyl-pyrrolo[3,2-c]pyridine-1-sulfonamide

A solution of 6-chloro-N,N-dimethyl-pyrrolo[3,2-c]pyridine-1-sulfonamide (13.6 g, 52.37 mmol, 1.0 eq) in THF (200 mL) was stirred under N₂ at -78 °C for 30 min. Then, to the mixture was added dropwise LDA (34.04 mL, 68.08 mmol, 1.3 eq), and stirring at -78 °C continued for 1 h. Then, 1,3,2-dioxathiolane 2,2-dioxide (8.45 g, 68.08 mmol, 1.3 eq) in THF (50 mL) was added at -78 °C, and the mixture was stirred at 25 °C for 1 h. Finally HCl (40.0 mL, 480.0 mmol, 9.17 eq) was added, and stirring continued at 25 °C for 16 h. EtOAc (200 mL) and aq. NaHCO₃ (300 mL) were added, and the layers were separated. The aq. phase was extracted with EtOAc (2 x 100 mL). The combined extracts were washed with brine (300 mL), dried over Na₂SO₄, filtered, and concentrated under vacuum. The residue was purified by column chromatography (80% EA in PE) to afford the title compound 6-chloro-2-(2-hydroxyethyl)-N,N-dimethyl-pyrrolo[3,2-c]pyridine-1-sulfonamide (1.1 g, 3.62 mmol, 6.8%) as a light yellow solid. MS [M+H]⁺ 304.0.

### Step 3: 2-[2-[tert-butyl(dimethyl)silyl]oxyethyl]-6-chloro-N,N-dimethyl-pyrrolo[3,2-c]pyridine-1-sulfonamide

A solution of 6-chloro-2-(2-hydroxyethyl)-N,N-dimethyl-pyrrolo[3,2-c]pyridine-1-sulfonamide (100.0 mg, 0.33 mmol, 1.0 eq) and imidazole (24.65 mg, 0.36 mmol, 1.1 eq) in DCM (5 mL) was added tert-butyldimethylchlorosilane (54.58 mg, 0.36 mmol, 1.1 eq).The mixture was stirred at 25 °C for 16 h. The reaction mixture was filtered and concentrated in vacuum. The crude material was purified by column chromatography (20% EA in PE) and concentrated to afford the title compound **Intermediate A24** (108.0 mg, 0.26 mmol, 73.8%) as white solid. MS [M+H]⁺ 418.1.

The following examples were prepared in analogy of **Intermediate A24**

| **Ex#** | **Name** | **MS ESI** | **Starting Material** |
|---|---|---|---|
| **Interme diate A30** | 5-bromo-2-[2-[tert-butyl(dimethyl)silyl]oxyethyl]-N,N-dimethyl-pyrrolo[3,2-b]pyridine-1-sulfonamide | 464.1 [M+H]⁺ | 5-bromo-4-azaindole (CAS 1000341-51-4) |

### Intermediate A28

### 5-Bromo-2-[(2S)-3-[tert-butyl(dimethyl)silyl]oxy-2-hydroxy-propyl]-N,N-dimethyl-pyrrolo[3,2-b]pyridine-1-sulfonamide

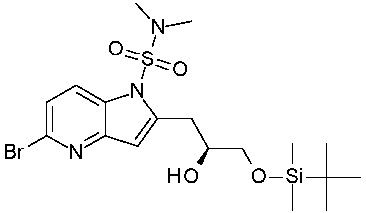

### Step 1: 5-bromo-N,N-dimethyl-pyrrolo[3,2-b]pyridine-1-sulfonamide

To a solution of 5-bromo-4-azaindole (CAS 1000341-51-4; 1.0 g, 5.08 mmol, 1.0 eq) in DCM (10 mL) was added tetrabutylammonium hydrogensulfate (0.17 g, 0.51 mmol, 0.1 eq), sodium hydroxide (0.51 g, 12.69 mmol, 2.5 eq) and dimethylsulfamoyl chloride (1.09 g, 7.61 mmol, 1.5 eq). The reaction mixture was stirred at 20 °C for 16 h. The reaction mixture was poured into water (50 mL), and extracted with EA (2 x 50 mL). The combined organic layers were washed with brine, dried over Na₂SO₄, filtered, and concentrated in vacuum. The residue was purified by column chromatography (PE/EA 5:1) to afford the title compound 5-bromo-N,N-dimethyl-pyrrolo[3,2-b]pyridine-1-sulfonamide (1.36 g, 4.47 mmol, 88.1%) as a white solid. MS [M+H]⁺ 304.0.

### Step 2: 5-bromo-2-[(2S)-3-[tert-butyl(dimethyl)silyl]oxy-2-hydroxy-propyl]-N,N-dimethyl-pyrrolo[3,2-b]pyridine-1-sulfonamide

A solution of 5-bromo-N,N-dimethyl-pyrrolo[3,2-b]pyridine-1-sulfonamide (500.0 mg, 1.64 mmol, 1.0 eq) in THF (10 mL) was cooled to -40 °C, and treated dropwise with LDA solution (1.64 mL, 3.29 mmol, 2.0 eq). The mixture was stirred at -40 °C for 0.5 h. Then, tert-butyldimethyl-[[(2S)-oxiran-2-yl]methoxy]silane (464.41 mg, 2.47 mmol, 1.5 eq) was added to the mixture, and stirring was continued at 0 °C for 1 h. The solution was poured into NH₄Cl solution (100 mL), extracted with EtOAc (2 x 50 mL), and the combined organic layers were washed with brine (100 mL), dried over Na₂SO₄, filtered, and concentrated. The residue was purified by RPHPLC (95% ACN in 0.1% FA in water) to afford the title compound **Intermediate A28** (220.0 mg, 0.45 mmol, 27.2%) as a yellow oil. MS [M+H]⁺ 494.1.

### Intermediate A20

### 6-chloro-N-(2-hydroxyethyl)-1H-pyrrolo[3,2-c]pyridine-3-carboxamide

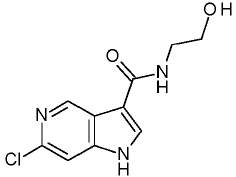

To a solution of 6-chloro-1H-pyrrolo[3,2-c]pyridine-3-carboxylic acid (500.0 mg, 2.54 mmol, 1.0 eq) and N,N-diisopropylethylamine (0.89 mL, 5.09 mmol, 2.0 eq) in DMF (5 mL) was added O-(7-azabenzotriazol-1-yl)-N,N,N',N'-tetramethyluronium hexafluorophosphate (1.26 g, 3.31 mmol, 1.3 eq). The mixture was stirred at 25 °C for 15 min. 2-hydroxyethylamine (0.31 mL, 5.09 mmol, 2.0 eq) was added into the mixture. The mixture was stirred at 25 °C for 1 h. The mixture was quenched with water (0.5 mL), and then concentrated under vacuum. The residue was purified by RP column chromatography (0-60% ACN in water(FA)) to give the title compound **Intermediate A20** (600.0 mg, 2.5 mmol, 98.4%) as a light yellow gum. MS [M+H]⁺ 240.1.

The following examples were prepared in analogy of Intermediate A20

| **Ex#** | **Name** | **MS ESI** | **Starting Material** |
|---|---|---|---|
| **Intermediate A22** | (6-bromo-1H-pyrrolo[3,2-c]pyridin-3-yl)-(4-methylpiperazino)methanone | 323.18 [M+H]⁺ | 6-bromo-1H-pyrrolo[3,2-c]pyridine-3-carboxylic acid and 1-methylpiperazine |
| **Intermediate A25** | (6-bromo-1H-pyrrolo[3,2-c]pyridin-3-yl)-[(3S)-3-hydroxypyrrolidino]methanone | 310.12 [M+H]⁺ | 6-bromo-1H-pyrrolo[3,2-c]pyridine-3-carboxylic acid and (3S)-pyrrolidin-3-ol |
| **Intermediate A21** | 4-(6-bromo-1H-pyrrolo[3,2-c]pyridine-3-carbonyl)piperazine-1-carboxylic acid tert-butyl ester | 409.2 [M+H]⁺ | 6-bromo-1H-pyrrolo[3,2-c]pyridine-3-carboxylic acid and 1-Boc-piperazine |

### Intermediate A27

### 6-bromo-1H-pyrrolo[3,2-c]pyridine-3-carboxamide

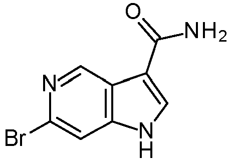

6-bromo-1H-pyrrolo[3,2-c]pyridine-3-carbaldehyde (65 mg, 288.84 µmol, 1 eq) was dissolved in dimethyl sulfoxide, extra dry (1 mL). Hydroxylamine hydrochloride (40.14 mg, 577.68 µmol, 2 eq) was added, and the mixture was stirred at 100 °C for 1 h. While still hot, 2 M sodium hydroxide (433.26 µL, 866.51 µmol, 3 eq) was added dropwise, and the mixture was stirred for 5 min. Then, hydrogen peroxide 30% (327.45 mg, 295 µL, 2.89 mmol, 10 eq) was added dropwise, and the mixture was again stirred for 5 min. Water (10 mL) was added, and the mixture was extracted with DCM (3 x 10 mL). The organic phase was discarded, and the aq. phase was cooled to 0 °C, and the formed precipitate was filtered, and dried in vacuo to give the title compound Intermediate A27 (46 mg, 62.4%) as a yellow solid. MS [M+H]⁺ 240.01.

### Intermediate A32

### 2-[(2-chloropyrrolo[3,2-d]pyrimidin-5-yl)methoxy]ethyl-trimethyl-silane

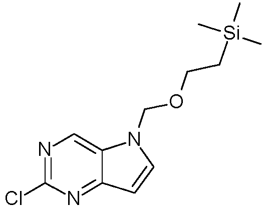

To a stirred light yellow solution of 2-chloro-5H-pyrrolo[3,2-d]pyrimidine (500.0 mg, 3.26 mmol, 1.0 eq) in THF (10 mL) was added sodium hydride, 60% in oil (182.31 mg, 4.56 mmol, 1.4 eq) in three portions at 0 °C. After stirring for 0.5 h at 0 °C, 2-(trimethylsilyl)ethoxymethyl chloride (0.69 mL, 3.91 mmol, 1.2 eq) was added to the solution and stirred for 2 h at 0 °C. The solution was slowly poured into 0.5 N HCl solution (30 mL), and extracted with EtOAc (2 x 20 mL). The combined organic layers were washed with brine (50 mL), dried over Na₂SO₄, and concentrated. The residue was purified by column chromatography (27% EA in PE) to afford the title compund **Intermediate A32** (590.0 mg, 2.08 mmol, 58.1%) as a light yellow oil. MS [M+H]⁺ 284.0.

### Intermediate A33

### benzyl N-[2-[2-(benzyloxycarbonylamino)ethyl-[2-[(6-bromo-3-pyridyl)amino]ethyl]amino]ethyl]carbamate

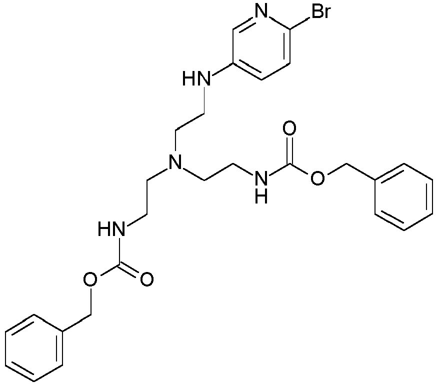

### Step 1: tert-butyl N-[2-[(6-bromo-3-pyridyl)amino]ethyl]carbamate

To a solution of 2-bromo-5-iodopyridine (5.0 g, 17.61 mmol, 1.0 eq), copper(I) iodide (0.72 mL, 21.13 mmol, 1.2 eq), 2-(2,6-dimethylanilino)-2-oxo-acetic acid (1360.17 mg, 7.04 mmol, 0.4 eq) and phosphoric acid, potassium salt (4.37 mL, 52.84 mmol, 3.0 eq) in DMSO (100 mL) was added N-BOC-ethylenediamine (3.1 g, 19.37 mmol, 1.1 eq). The mixture was degassed and then stirred at 35 °C for 16 h under N₂. The mixture was poured into water (80 mL) and EA (80 mL). The mixture was filtered through a celite pad (washings with EA, 3 x 20 mL). The organic layer was separated from the combined filtrate. The aqueous layer was extracted with EA (2 x 80 mL). The combined organic layers were washed with brine (3 x 50 mL), dried over sodium sulfate, filtered, and concentrated. The residue was purified by column chromatography (17-50% EA in PE) to give the title compound tert-butyl N-[2-[(6-bromo-3-pyridyl)amino]ethyl]carbamate (1.3 g, 4.11 mmol, 23.3%) as a light yellow solid. MS [M+H]⁺ 316.0.

### Step 2: N'-(6-bromo-3-pyridyl)ethane-1,2-diamine dihydrochloride

To a solution of tert-butyl N-[2-[(6-bromo-3-pyridyl)amino]ethyl]carbamate (1.0 g, 3.16 mmol, 1.0 eq) in methanol (10 mL) was added hydrochloric acid in methanol (20.0 mL, 80.0 mmol, 25.3 eq). The mixture was stirred at 15 °C for 2 h, and then concentrated under vacuum to afford the title compound N'-(6-bromo-3-pyridyl)ethane-1,2-diamine dihydrochloride (1.0 g, 3.46 mmol, 98.5%) as a white solid. MS [M+H]⁺ 218.

### Step 3: benzyl N-(2,2-dimethoxyethyl)carbamate

To a solution of aminoacetaldehyde dimethyl acetal (10.0 g, 95.11 mmol, 1.0 eq) and sodium hydrogen carbonate (10.39 g, 123.64 mmol, 1.3 eq) in THF (100 mL) and water (50 mL) was drop-wise added benzyl chloroformate (17.85 g, 104.62 mmol, 1.1 eq) at 0 °C. The mixture was stirred at 0 °C for 1 h, and then at 15 °C for 16 h to give a white suspension. The mixture was diluted with water (50 mL), and extracted with EA (3 x 80 mL). The combined organic layers were washed with brine (80 mL), dried over sodium sulfate, filtered, and concentrated to give the title compound benzyl N-(2,2-dimethoxyethyl)carbamate (23.0 g, 96.13 mmol, 99.1%) as a light yellow liquid. 1H NMR (CDCl3, 400MHz): δ = 7.39 - 7.35 (m, 5H), 5.17 (s, 2H), 4.95 (br. s, 1H), 4.14 (t, J = 7.2 Hz, 1H), 3.40 (s, 6H), 3.35 (t, J = 5.6 Hz, 2H).

### Step 4: benzyl N-(2-oxoethyl)carbamate

To a solution of benzyl N-(2,2-dimethoxyethyl)carbamate (5.0 g, 20.9 mmol, 1.0 eq) in THF (30 mL) was added hydrochloric acid (20.9 mL, 41.79 mmol, 2.0 eq). The mixture was stirred at 15 °C for 4 h to give a colorless solution. The mixture was diluted with water (50 mL) and then extracted with EA (3 x 50 mL). The combined organic layers were washed with brine (30 mL), dried over sodium suflate, filtered, and concentrated to afford the title compound benzyl N-(2-oxoethyl)carbamate (4.6 g, 23.81 mmol, 96.9%) as a light yellow oil, which was directly used in the next step without any further purification.

### Step 5: benzyl N-[2-[2-(benzyloxycarbonylamino)ethyl-[2-[(6-bromo-3-pyridyl)amino]ethyl]amino]ethyl]carbamate

To a mixture of N'-(6-bromo-3-pyridyl)ethane-1,2-diamine dihydrochloride (1.0 g, 3.11 mmol, 1.0 eq), sodium acetate (0.94 mL, 12.46 mmol, 4.0 eq), and 4Å molecular sieve in methanol (20 mL) was added benzyl N-(2-oxoethyl)carbamate (3.15 g, 13.84 mmol, 4.44 eq). The mixture was stirred at 15 °C for 0.5 h. Then, sodium cyanoborohydride (1.17 g, 18.69 mmol, 6.0 eq) was added into the mixture in four portions. The mixture was stirred at 15 °C for another 16 h. Then, additional benzyl N-(2-oxoethyl)carbamate (2.03 g, 9.34 mmol, 3.0 eq) was added into the mixture, followed by sodium cyanoborohydride (0.59 g, 9.34 mmol, 3.0 eq) in three portions. The mixture was stirred at 15 °C for another 16 h. The mixture was filtered, the solid was washed with MeOH (3 x 2 mL). The combined filtrate was concentrated under vacuum, and the residue was diluted with EA (200 mL). The mixture was washed with brine (50 mL), dried over sodium sulfate, filtered, and concentrated. The residue was purified by RP column chromatography (0-80% ACN in water(HCl)) to afford the title compound (2.0 g, 3.51 mmol, 88.2%) as a colorless gum. MS [M+H]⁺ 572.1.

### Intermediate A13

### 2-(5-bromo-1H-pyrrolo [3,2-b] pyridin-2-yl)ethanol

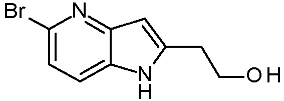

### Step 1: 4-(3-amino-6-bromo-2-pyridyl)but-3-yn-1-ol

(6-bromo-2-iodo-3-pyridyl)amine **(Intermediate A7** Step 3; 683 mg, 2.28 mmol, 1 eq) was dissolved in acetonitrile, extra dry (15 mL), and copper (I) iodide (87.03 mg, 456.99 µmol, 0.2 eq), Pd(PPh₃)₂Cl₂ (160.38 mg, 228.5 µmol, 0.1 eq) and triethylamine (693.65 mg, 955.44 µL, 6.85 mmol, 3 eq) were added to the mixture. Subsequently, 3-butyn-1-ol (288.28 mg, 313.34 µL, 4.11 mmol, 1.8 eq) was added dropwise, and the mixture was stirred at 50 °C overnight. The mixture was concentrated in vacuo, and purified by column chromatography (0-50% DCM/MeOH 9:1 in DCM ) to give the title compound 4-(3-amino-6-bromo-2-pyridyl)but-3-yn-1-ol (503 mg, 91.3%) as a brown viscous oil. MS [M+H]⁺ 241.11.

### Step 2: 2-(5-bromo-1H-pyrrolo[3,2-b]pyridin-2-yl)ethanol

4-(3-amino-6-bromo-2-pyridyl)but-3-yn-1-ol (307 mg, 1.24 mmol, 1 eq) was dissolved in N-methyl-2-pyrrolidinone, extra dry (6 mL), treated with potassium tert-butoxide (304.93 mg, 2.72 mmol, 2.2 eq), and the mixture was stirred at ambient temperature for 4 d. Water (2 mL) was added, and the mixture was directly purified by RP column chromatography (10-50% ACN in water) to give the title compound **Intermediate A13** (302 mg, 99.4%) as a yellow solid. MS [M+H]⁺ 241.08.

### Intermediate A34

### tert-butyl 5-bromo-2-(2-ethoxy-2-oxo-ethyl)pyrrolo[3,2-b]pyridine-1-carboxylate

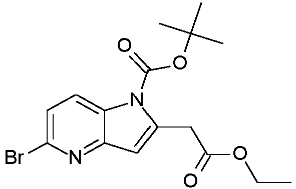

### Step 1: ethyl 2-(5-bromo-1H-pyrrolo[3,2-b]pyridin-2-yl)acetate

To a solution of 5-bromo-4-azaindole (1100.0 mg, 5.58 mmol, 1.0 eq) in DMF (5 mL) was added 2-norbornene (1051.31 mg, 11.17 mmol, 2.0 eq), bis(benzonitrile)palladium(II) chloride (214.14 mg, 0.56 mmol, 0.1 eq), ethyl bromoacetate (0.74 mL, 6.7 mmol, 1.2 eq), sodium hydrogen carbonate (1876.0 mg, 22.33 mmol, 4.0 eq) and water (0.1 mL, 5.58 mmol, 1.0 eq). The reaction was stirred under N₂ at 70 °C for 48 h. The mixture was diluted with EtOAc (20 mL) and poured into water (20 mL). The water layer was extracted with EtOAc (3 x 20 mL). The combined organic layers were washed with water (2 x 50 mL) and brine (50 mL), dried over Na₂SO₄ and concentrated under vacuum. The residue was purified by column chromatography (30% EA in PE) and concentrated. The obtained material was again purified by RPHPLC (50-60% ACN in 0.1% FA in water) to afford the title compound ethyl 2-(5-bromo-1H-pyrrolo[3,2-b]pyridin-2-yl)acetate (190.0 mg, 0.67 mmol, 11.8%) as a pink solid. MS [M+H]⁺ 282.9.

### Step 2: tert-butyl 5-bromo-2-(2-ethoxy-2-oxo-ethyl)pyrrolo[3,2-b]pyridine-1-carboxylate

To a solution of ethyl 2-(5-bromo-1H-pyrrolo[3,2-b]pyridin-2-yl)acetate (190.0 mg, 0.67 mmol, 1.0 eq) in THF (5 mL) was added triethylamine (0.14 mL, 1.01 mmol, 1.5 eq), di-t-butyldicarbonate (0.19 mL, 0.81 mmol, 1.2 eq), and 4-dimethylaminopyridine (16.4 mg, 0.13 mmol, 0.2 eq). The reaction mixture was stirred at 10 °C for 3 h. The mixture was concentrated in vacuum, and the residue was purified using column chromatography (PE/EtOAc 10:1) to give the title compound **Intermediate A34** (280.0 mg, 0.73 mmol, quant.) as yellow solid. MS [M+H]⁺ 385.1.

### Intermediate A6

### (1R)-1-[5-chloro-1-(2-trimethylsilylethoxymethyl)pyrrolo[3,2-b]pyridin-2-yl]ethanol

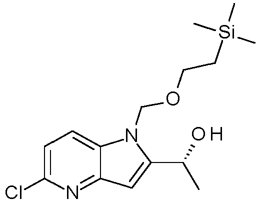

### Step 1: 5-chloro-1H-pyrrolo[3,2-b]pyridine-2-carboxylic acid

A mixture of 2-bromo-6-chloro-pyridin-3-amine (23.0 g, 110.86 mmol, 1.0 eq), pyruvic acid (26.39 g, 299.63 mmol, 2.7 eq), palladium(II)acetate (2.49 g, 11.09 mmol, 0.1 eq), triethylamine (48.51 g, 479.42 mmol, 4.32 eq), and triphenylphosphine (25.93 g, 98.88 mmol, 0.89 eq) in DMF (30 mL) was stirred at 100 °C for 16 h. The reaction mixture was cooled to room temperature. EtOAc (500 mL) and water (400 mL) were added, and the layers were separated. 1 M HCl (300 mL) was added, and the mixture was extracted with EtOAc (4 x 300 mL). The combined extracts were concentrated under vacuum to afford the title compound 5-chloro-1H-pyrrolo[3,2-b]pyridine-2-carboxylic acid (14.0 g, 71.21 mmol, 64.2%) as a light brown solid, which was used without further purification in the next step. MS [M+H]⁺ 197.1.

### Step 2: 5-chloro-N-methoxy-N-methyl-1H-pyrrolo[3,2-b]pyridine-2-carboxamide

To a light yellow stirred solution of 5-chloro-1H-pyrrolo[3,2-b]pyridine-2-carboxylic acid (11.0 g, 55.95 mmol, 1.0 eq), O,N-dimethylhydroxylamine hydrochloride (10.92 g, 111.91 mmol, 2.0 eq) and N,N-diisopropylethylamine (38.98 mL, 223.82 mmol, 4.0 eq) in DMF (100 mL) was added HATU (23.4 g, 61.55 mmol, 1.1 eq) in portions at 10 °C. After addition, the solution was stirred at 10 °C for 1 h. The reaction mixture was diluted with sat. aq NaCl (200 mL), extracted with EtOAc (3 x 200 mL). The combined organic layers were washed with brine (500 mL), dried over MgSO4, filtered, and concentrated under vacuum to give a residue. The residue was diluted with EtOAc (50 mL) and stirred for 10 min, filtered, and the filter cake was washed with MeOH (50 mL), and dried under vacuum to give the title compound 5-chloro-N-methoxy-N-methyl-1H-pyrrolo[3,2-b]pyridine-2-carboxamide (10.0 g, 41.73 mmol, 74.6%) as a light yellow solid. MS [M+H]⁺ 240.1.

### Step 3: 1-(5-chloro-1H-pyrrolo[3,2-b]pyridin-2-yl)ethanone

To a solution of 5-chloro-N-methoxy-N-methyl-1H-pyrrolo[3,2-b]pyridine-2-carboxamide (10.0 g, 41.73 mmol, 1.0 eq) in THF (120 mL) was added dropwise methyllithium solution (83.45 mL, 83.45 mmol, 2.0 eq, 1 M) at -78 °C under N₂. After addition, the mixture was stirred at -78 °C for 2 h. The reaction mixture was poured into sat. NH₄Cl solution (100 mL), extracted with EtOAc (4 x 100 mL). The combined organic layers were concentrated under vacuum, and purified by column chromatography (50-75% EA in PE) to afford the title compound 1-(5-chloro-1H-pyrrolo[3,2-b]pyridin-2-yl)ethanone (5.5 g, 28.26 mmol, 67.7%) as a yellow solid. MS [M+H]⁺ 195.1.

### Step 4: 1-[5-chloro-1-(2-trimethylsilylethoxymethyl)pyrrolo[3,2-b]pyridin-2-yl]ethanone

To a solution of 1-(5-chloro-1H-pyrrolo[3,2-b]pyridin-2-yl)ethanone (5.5 g, 28.26 mmol, 1.0 eq) in THF (30 mL) was added sodium hydride (1.02 g, 42.39 mmol, 1.5 eq) at 0 °C, and the mixture was stirred for 0.5 h. Then, 2-(trimethylsilyl)ethoxymethyl chloride (6.0 mL, 33.91 mmol, 1.2 eq) was added, and the reaction mixture was stirred at 0 °C for another 4 h, before being poured into water (20 mL), and extracted with EtOAc (3 x 50 mL). The combined organic layer were washed with brine, dried over Na₂SO₄, filtered, and concentrated in vacuum. Purification by RP column chromatography (70-90% ACN in water(0.1%FA)) afforded the title compound 1-[5-chloro-1-(2-trimethylsilylethoxymethyl)pyrrolo[3,2-b]pyridin-2-yl]ethanone (4.0 g, 12.31 mmol, 43.6%) as a brown oil. MS [M+H]⁺ 325.1.

### Step 5: (1R)-1-[5-chloro-1-(2-trimethylsilylethoxymethyl)pyrrolo[3,2-b]pyridin-2-yl]ethanol

To a solution of dichloro (p-cymene) ruthenium(ll) dimer (188.5 mg, 0.31 mmol, 0.1 eq) in water (10 mL) was added (1R,2R)-(-)-N-p-tosyl-1,2-diphenylethylenediamine (135.37 mg, 0.37 mmol, 0.12 eq), and the mixture was stirred at 70 °C for 1.5 h, and degassed with N₂. Then the mixture was allowed to cool to room temperature (solution A). To a solution of 1-[5-chloro-1-(2-trimethylsilylethoxymethyl)pyrrolo[3,2-b]pyridin-2-yl]ethanone (1.0 g, 3.08 mmol, 1.0 eq) in THF (10 mL) was added sodium formate (1.05 g, 15.39 mmol, 5.0 eq) (solution B). Solution B was added to solution A under N₂, and the reaction mixture was stirred at 40 °C for another 2 h. The mixture was cooled to room temperature. EtOAc (20 mL) and water (20 mL) were added, and the layers were separated. The aq. phase was extracted with EtOAc (2 x 30 mL). The combined extracts were washed with brine (30 mL), dried over Na₂SO₄, filtered, and concentrated under vacuum. Purification by RP column chromatography (70-90% ACN in water(0.1%FA)) afforded the title compound **Intermediate A6** (960.0 mg, 2.94 mmol, 95.4%) as a brown oil. MS [M+H]⁺ 327.2.

### Intermediate A48

### (1S)-1-[5-chloro-1-(2-trimethylsilylethoxymethyl)pyrrolo[3,2-b]pyridin-2-yl]ethanol

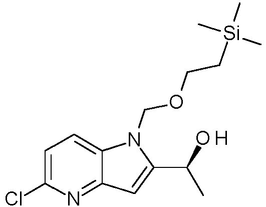

To a solution of 1-[5-chloro-1-(2-trimethylsilylethoxymethyl)pyrrolo[3,2-b]pyridin-2-yl]ethanone **(Intermediate A6** Step 4; 1 g, 3.1 mmol, 1 eq) in methanol (10 mL) was added sodium borohydride (58.23 mg, 1.54 mmol, 0.5 eq). The reaction mixture was stirred at -10 °C for 2 h, and concentrated. The crude was purified by RP column chromatography (70-90% ACN in water(0.1%FA). Then, the residue was further purified by SFC (column: DAICEL CHIRALCEL OD (250mm*30mm, 10µm); mobile phase: A1=CO₂-MeOH (0.05% DEA); B1=EtOH (0.1% NH₃H₂O); gradient elution:CO₂-MeOH(0.1%NH₃H₂O) 20; flow rate: 150 mL/min; column temp:35 °C) to obtain the title compound **Intermediate A48** (490.0 mg, 1.5 mmol, 48.7%) (peak1: 1.065 min) as light yellow oil and confirmed by comparison with **Intermediate A6** (peak2: 1.238 min).

### Intermediate A16

### Ethyl 2-(5-chloropyrrolo[3,2-b]pyridin-1-yl)-2-methyl-propanoate

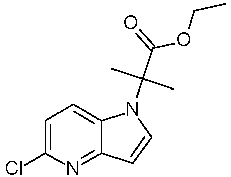

A solution of 5-chloro-1H-pyrrolo[3,2-b]pyridine (3.0 g, 19.66 mmol, 1.0 eq) in THF (50 mL) was cooled to 0 °C, then sodium hydride, 60% in oil (1.18 g, 29.49 mmol, 1.5 eq) was added in portions. The mixture was stirred for 0.5 h at 0 °C under N₂.To the solution was added ethyl 2-bromoisobutyrate (3.84 g, 19.66 mmol, 1.0 eq) in portions, and the mixture was stirred at 0 °C for 2 h, and warmed to 20 °C over 14 h. To the reaction mixture was added 1 M HCl (10 mL) and water (20 mL), and the mixture was extracted with EtOAc (4 x 30 mL). The combined organic layers were dried over Na₂SO₄, filtered, and concentrated under vacuum. The residue was purified by column chromatography (PE/EA 2:1) to afford the title compund **Intermediate A16** (1.9 g, 7.12 mmol, 36.2%) as a light yellow oil. MS [M+H]⁺ 267.1.

### Intermediate A11

### 6-Bromo-1H-pyrrolo[3,2-c]pyridine-2-carboxylic acid methyl ester

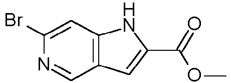

### Step 1: (2-bromo-5-iodo-4-pyridyl)amine

A mixture of (2-bromo-4-pyridyl)amine (9.75 g, 56.36 mmol, 1 eq) in acetonitrile (200 mL) was treated with N-iodosuccinimide (13.95 g, 61.99 mmol, 1.1 eq) at 80 °C, and the mixture was stirred at 80 °C overnight. The reaction mixture was cooled to RT, and concentrated in vacuo. 10% aq. Na₂S₂O₃ (300 mL) was added, and the mixture was extracted with ethyl acetate (3 x 250 mL). The combined organic phases were dried (Na₂SO₄), filtered, and concentrated in vacuo. The residue was purified by column chromatography (10-35% ethyl acetate in heptane) to give (2-bromo-3-iodo-4-pyridyl)amine (6.52 g, 38.7%) as off-white solid and the title compound (2-bromo-5-iodo-4-pyridyl)amine (5.12 g, 30.4%) as a yellow solid. MS [M+H]⁺ 298.93.

### Step 2: 6-bromo-1H-pyrrolo[3,2-c]pyridine-2-carboxylic acid

In a MW vial, a mixture of (2-bromo-5-iodo-4-pyridyl)amine (5100 mg, 17.06 mmol, 1.000 eq) in N,N-dimethylformamide, extra dry (35 mL) was treated with pyruvic acid (4.51 g, 3.58 mL, 51.19 mmol, 3 eq), palladium diacetate (162.47 mg, 853.1 µmol, 0.05 eq) and DABCO (5.74 g, 51.19 mmol, 3 eq), and the mixture was stirred at 110 °C for 90 min under MW irridation. Methanol (100 mL) was added, and the mixture was filtered over Celite. The solvent was removed in vacuo. The residue was purified by column chromatography (5-30% MeOH+2% AcOH in DCM) to give the title compound 6-bromo-1H-pyrrolo[3,2-c]pyridine-2-carboxylic acid (5.55 g, 89.1%) as a brown solid. MS [M+H]⁺ 241.02.

### Step 3: 6-bromo-1H-pyrrolo[3,2-c]pyridine-2-carboxylic acid methyl ester

A mixture of 6-bromo-1H-pyrrolo[3,2-c]pyridine-2-carboxylic acid (4.85 g, 13.28 mmol, 1 eq) in methanol (150 mL) was treated with H₂SO₄ (6.51 g, 3.54 mL, 66.4 mmol, 5 eq), and the mixture was stirred at 70 °C for 24 h. The mixture was concentrated in vacuo. Sat. aq. NaHCO₃ (100 mL) was slowly added, and the mixture was extracted with ethyl acetate (3 x150 mL). The combined organic phases were dried (Na₂SO₄), filtered, and concentrated in vacuo. The residue was purified by column chromatography (0-30% DCM/MeOH 9:1 in DCM) to give the title compound **Intermediate A11** (1.094 mg, 32.3%) as a light yellow solid. MS [M+H]⁺ 255.08.

### Intermediate A39

### (6-Bromo-1H-pyrrolo[3,2-c]pyridin-2-yl)methanol

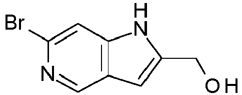

A mixture of 6-bromo-1H-pyrrolo[3,2-c]pyridine-2-carboxylic acid **(Intermediate A11** Step 2; 195 mg, 808.99 µmol, 1 eq) in tetrahydrofuran, extra dry (5 mL) was treated dropwise with 2 M borane dimethyl sulfide complex (889.89 µL, 1.78 mmol, 2.200 eq) at 0 °C. The reaction mixture was then warmed to RT, and stirred for 3 d. Water (10 mL) and sat. aq. NaHCO₃ (5 mL) were added, and the mixture was extracted with ethyl acetate (3 x 20 mL). The combined organic phases were dried (Na₂SO₄), filtered, and concentrated in vacuo to afford the title compound **Intermediate A39** (87 mg, 47.4%) as a yellow oil. MS [M+H]⁺ 229.0.

### Intermediate A18

### tert-butyl N-[2-[6-bromo-1-(dimethylsulfamoyl)pyrrolo[3,2-c]pyridin-2-yl]ethyl]carbamate

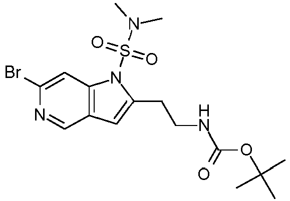

### Step 1: 2-bromo-5-(3,3-diethoxyprop-1-ynyl)pyridin-4-amine

A mixture of (2-bromo-5-iodo-4-pyridyl)amine **(Intermediate A11** Step 1; 17.2 g, 57.54 mmol, 1.0 eq), propargylaldehyde diethyl acetal (7.74 g, 60.42 mmol, 1.05 eq), triethylamine (24.06 mL, 172.63 mmol, 3.0 eq), copper(I) iodide (0.19 mL, 5.75 mmol, 0.1 eq) and [1,1'-bis(diphenylphosphino)ferrocene]dichloropalladium(II) (4.21 g, 5.75 mmol, 0.1 eq) in THF (250 mL) was evacuated and backfilled with N₂ (3x). The yellow mixture was stirred under N₂ at 60 °C for 16 h. The reaction mixture was poured into water (300 mL), and extracted with EA (3 x 150 mL). The combined organic layers were washed with brine, dried over Na₂SO₄, filtered, and concentrated in vacuum. The residue was purified by column chromatography (30% EA in PE) to afford the title compound 2-bromo-5-(3,3-diethoxyprop-1-ynyl)pyridin-4-amine (10.0 g, 33.43 mmol, 50.3%) as a yellow solid. MS [M+H]⁺ 299.0.

### Step 2: 6-bromo-2-(diethoxymethyl)-1H-pyrrolo[3,2-c]pyridine

To a yellow solution of 2-bromo-5-(3,3-diethoxyprop-1-ynyl)pyridin-4-amine (10.0 g, 33.43 mmol, 1.0 eq) in 1-methyl-2-pyrrolidinone (200 mL) was added potassium tert-butoxide (9.38 g, 83.57 mmol, 2.5 eq) in one portion at 20 °C. The mixture was stirred at 50 °C for 2 h. The mixture was slowly poured into cooled 0.05 N HCl solution (50 mL, pH=6) and water (300 mL), and extracted with EtOAc (3 x 150 mL). The combined organics were washed with brine (2 x 400 mL), dried over Na₂SO₄, filtered, and concentrated in vacuum. The crude material was purified by column chromatography (30% EA in PE) to afford the title compound 6-bromo-2-(diethoxymethyl)-1H-pyrrolo[3,2-c]pyridine (9.8 g, 32.76 mmol, 65.7%) as a yellow solid. MS [M+H]⁺ 301.0.

### Step 3: 6-bromo-2-(diethoxymethyl)-N,N-dimethyl-pyrrolo[3,2-c]pyridine-1-sulfonamide

To a solution of 6-bromo-2-(diethoxymethyl)-1H-pyrrolo[3,2-c]pyridine (9.7 g, 32.42 mmol, 1.0 eq) in DCM (150 mL) was added tetrabutylammonium hydrogen sulfate (1.1 g, 3.24 mmol, 0.1 eq), sodium hydroxide (3.24 g, 81.06 mmol, 2.5 eq), and dimethylsulfamoyl chloride (6.98 g, 48.64 mmol, 1.5 eq). The reaction mixture was stirred at 20 °C for 16 h, and concentrated in vacuum. The residue was poured into water (200 mL), and extracted with EtOAc (2 x 100 mL). The combined organic layers were washed with brine, dried over Na₂SO₄, filtered, and concentrated in vacuum. Purification by column chromatography (9-25% EA in PE) afforded the title compound 6-bromo-2-(diethoxymethyl)-N,N-dimethyl-pyrrolo[3,2-c]pyridine-1-sulfonamide (10.5 g, 25.84 mmol, 79.7%) as a yellow solid. MS [M+H]⁺ 408.0.

### Step 4: 6-bromo-2-formyl-N,N-dimethyl-pyrrolo[3,2-c]pyridine-1-sulfonamide

To a solution of 6-bromo-2-(diethoxymethyl)-N,N-dimethyl-pyrrolo[3,2-c]pyridine-1-sulfonamide (10.5 g, 25.84 mmol, 1.0 eq) in THF (100 mL)/water (20 mL) was added p-toluenesulfonic acid monohydrate (7.37 g, 38.76 mmol, 1.5 eq) at 20°C. The mixture was stirred at 20 °C for 16 h. The solution was poured into sat. aq. NaHCO₃ solution (200 mL), and extracted with EtOAc (2 x 100 mL). The combined organics were washed with brine (200 mL), dried over Na₂SO₄, filtered, andd concentrated to afford a yellow residue. The residue was purified by column chromatography (35% EA in PE) to afford the title compound 6-bromo-2-formyl-N,N-dimethyl-pyrrolo[3,2-c]pyridine-1-sulfonamide (5.0 g, 15.05 mmol, 58.3%) as a yellow solid. MS [M+H]⁺ 334.0.

### Step 5: 6-bromo-2-[(E)-2-methoxyvinyl]-N,N-dimethyl-pyrrolo[3,2-c]pyridine-1-sulfonamide

To a solution of (methoxymethyl)triphenylphosphonium chloride (9.29 g, 27.09 mmol, 2.0 eq) in THF (100 mL) was added sodium bis(trimethylsilyl)amide (27.09 mL, 27.09 mmol, 2.0 eq) at 0 °C, and the mixture was stirred for 1 h. A solution of 6-bromo-2-formyl-N,N-dimethyl-pyrrolo[3,2-c]pyridine-1-sulfonamide (4.5 g, 13.55 mmol, 1.0 eq) in THF (10 mL) was added dropwise into the reaction mixture at 0 °C, and the mixture was stirred at that temperature for 1 h, and subsequently at 20 °C for 16 h. The reaction mixture was cooled to room temperature. EtOAc (100 mL) and sat. aq. NH₄Cl (150 mL) were added, and the layers were separated. The aqueous phase was extracted with EtOAc (2 x 100 mL). The combined extracts were washed with brine (300 mL), dried over Na₂SO₄, filtered, and concentrated under vacuum. The residue was purified by RPHPLC (50-80% ACN in 0.1% FA in water) to afford the title compound 6-bromo-2-[(E)-2-methoxyvinyl]-N,N-dimethyl-pyrrolo[3,2-c]pyridine-1-sulfonamide (2.6 g, 7.22 mmol, 51.7%) as a yellow oil. MS [M+H]⁺ 361.9.

### Step 6: 6-bromo-N,N-dimethyl-2-(2-oxoethyl)pyrrolo[3,2-c]pyridine-1-sulfonamide

To a solution of 6-bromo-2-[(E)-2-methoxyvinyl]-N,N-dimethyl-pyrrolo[3,2-c]pyridine-1-sulfonamide (2.6 g, 7.22 mmol, 1.0 eq) in THF (16 mL) was added 3 M HCl (16.0 mL, 48.0 mmol, 6.65 eq) at 20 °C, and the mixture was stirred at 20 °C for 16 h. The mixture was concentrated in vacuum, and the crude material was purified by RPHPLC (50-80% ACN in water, 0.1% HCl) to afford the title compound 6-bromo-N,N-dimethyl-2-(2-oxoethyl)pyrrolo[3,2-c]pyridine-1-sulfonamide (1.8 g, 5.2 mmol, 61.1%) as a yellow solid. MS [M+H]⁺ 347.8.

### Step 7: tert-butyl N-[2-[6-bromo-1-(dimethylsulfamoyl)pyrrolo[3,2-c]pyridin-2-yl]ethyl]carbamate

A solution of 6-bromo-N,N-dimethyl-2-(2-oxoethyl)pyrrolo[3,2-c]pyridine-1-sulfonamide (0.7 g, 2.02 mmol, 1.0 eq), ammonium chloride (0.54 g, 10.11 mmol, 5.0 eq), 4Å molecular sieves, (817 mg), and di-t-butyldicarbonate (882.57 mg, 4.04 mmol, 2.0 eq) in methanol (10 mL) was stirred at 20 °C for 4 h.Then, the mixture was treated with sodium cyanoborohydride (381.18 mg, 6.07 mmol, 3.0 eq) and stirred at 20 °C for 12 h. The mixture was filtered, concentrated in vacuum, and purified by RPHPLC (50-80% ACN in 0.1% FA in water) to afford the title compound **Intermediate A18** (200.0 mg, 0.45 mmol, 20.9%) as a yellow solid. MS [M+H]⁺ 449.1.

### Intermediate A41

### tert-butyl 2-[[bis(tert-butoxycarbonyl)amino]methyl]-6-bromo-pyrrolo[3,2-c]pyridine-1-carboxylate

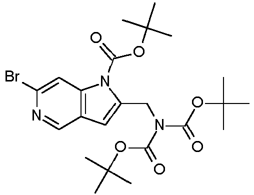

### Step 1: 6-bromo-1H-pyrrolo[3,2-c]pyridine-2-carbaldehyde

To a solution of 6-bromo-2-(diethoxymethyl)-1H-pyrrolo[3,2-c]pyridine **(Intermediate A18** Step 2; 1.2 g, 4.01 mmol, 1.0 eq) in THF (20 mL)/water (4 mL) was added p-toluenesulfonic acid monohydrate (1144.51 mg, 6.02 mmol, 1.5 eq) at 10 °C. The mixture was stirred at 10 °C for 1 h. The solution was poured into sat. aq. NaHCO₃ solution (20 mL), and extracted with EtOAc (50 mL). The combined extracts were washed with brine (50 mL), dried over Na₂SO₄, filtered, and concentrated. The residue was purified by column chromatography (35% EA in PE) to afford the title compound 6-bromo-1H-pyrrolo[3,2-c]pyridine-2-carbaldehyde (0.8 g, 3.55 mmol, 88.6%) as a light yellow solid. MS [M+H]⁺ 227.0.

### Step 2: tert-butyl N-[(6-bromo-1H-pyrrolo[3,2-c]pyridin-2-yl)methyl]carbamate

To a mixture of 6-bromo-1H-pyrrolo[3,2-c]pyridine-2-carbaldehyde (700.0 mg, 3.11 mmol, 1.0 eq), tert-butyl carbamate (728.8 mg, 6.22 mmol, 2.0 eq), and trifluoroacetic acid (0.72 mL, 9.33 mmol, 3.0 eq) in ACN (30 mL) was added triethylsilane (3616.96 mg, 31.11 mmol, 10.0 eq) at 10 °C. The mixture was stirred at 10 °C for 16 h. The solution was poured into sat. aq. NaHCO₃ solution (100 mL) and extracted with EtOAc (2 x 75 mL). The combined extracts were washed with brine (150 mL), dried over Na₂SO₄, filtered, and concentrated. The residue was purified by RPHPLC (50% ACN in 0.1% FA in water). Acetontrile was removed in vacuo (below 30 °C), and the remaining aq. phase was extracted with EtAOc (100 mL), washed with brine (150 mL), dried over Na₂SO₄, filtered, and concentrated to afford the title compound tert-butyl N-[(6-bromo-1H-pyrrolo[3,2-c]pyridin-2-yl)methyl]carbamate (590.0 mg, 1.81 mmol, 58.2%) as a light yellow solid. MS [M+H]⁺ 326.0.

### Step 3: tert-butyl 2-[[bis(tert-butoxycarbonyl)amino]methyl]-6-bromo-pyrrolo[3,2-c]pyridine-1-carboxylate

To a yellow solution of tert-butyl N-[(6-bromo-1H-pyrrolo[3,2-c]pyridin-2-yl)methyl]carbamate (590.0 mg, 1.81 mmol, 1.0 eq), triethylamine (0.38 mL, 2.71 mmol, 1.5 eq), and 4-dimethylaminopyridine (44.19 mg, 0.36 mmol, 0.2 eq) in THF (10 mL) was added di-t-butyldicarbonate (0.83 mL, 3.62 mmol, 2.0 eq) at 10 °C. The mixture was stirred at 10 °C for 3 h. The reaction mixture was directly purified by column chromatography (20% EA in PE) to afford the title compound **Intermediate A41** (700.0 mg, 1.33 mmol, 71.3%) as light yellow solid. MS [M+H]⁺ 528.1.

### Intermediate A36

### 2-(6-bromopyrrolo[3,2-c]pyridin-1-yl)ethoxy-tert-butyl-dimethyl-silane

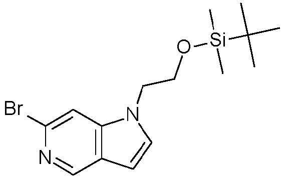

A mixture of 6-bromo-1H-pyrrolo[3,2-c]pyridine (83 mg, 421.26 umol, 1 eq) in N,N-dimethylacetamide (500 mL) was treated with (2-bromoethoxy)-tert-butyldimethylsilane (503.88 mg, 453.95 µL, 2.11 mmol, 5 eq), PdCl₂(MeCN)₂ (10.93 mg, 42.13 µmol, 0.1 eq), norbornene (79.33 mg, 842.51 µmol, 2 eq), water (18.98 mg, 18.98 µL, 1.05 mmol, 2.5 eq) and K₂CO₃ (116.44 mg, 842.51 µmol, 2 eq). The mixture was stirred at 70 °C overnight, and diluted with MTBE (20 mL), filtered over Celite, and concentrated in vacuo. The residue was purified by column chromatography (10-30% ethyl acetate in heptane) to give the title compound **Intermediate A36** (82 mg, 52%) as a yellow oil. MS [M+H]⁺ 355.10.

### Intermediate A26

### 6-Bromo-N-methyl-1H-pyrrolo[3,2-c]pyridine-3-carboxamide

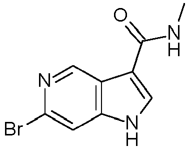

A mixture of 6-bromo-1H-pyrrolo[3,2-c]pyridine-3-carboxylic acid (210 mg, 871.22 µmol, 1 eq) in acetonitrile, extra dry (4 mL) was treated with 2 M methylamine in THF (871.22 µL, 1.74 mmol, 2 eq), 1-methylimidazole (214.58 mg, 208.33 µL, 2.61 mmol, 3 eq) and chloro-N,N,N',N'-tetramethylformamidinium hexafluorophosphate (366.67 mg, 1.31 mmol, 1.5 eq), and the mixture was stirred at ambient temperature overnight. The mixture was concentrated in vacuo, and purified by RP column chromatography (0-50% ACN in water) to give the title compound **Intermediate A26** (148 mg, 66.9%) as a colorless solid. MS [M+H]⁺ 254.06.

### Intermediate A45

**6-Bromopyrrolo[3,2-c]pyridine-1,3-dicarboxylic acid O1-tert-butyl ester O3-methyl ester**

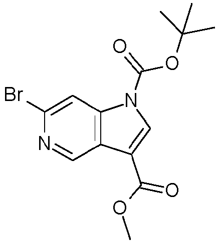

6-bromo-1H-pyrrolo[3,2-c]pyridine-3-carboxylic acid methyl ester (CAS 1427503-50-1; 721 mg, 2.83 mmol, 1 eq) was dissolved in dichloromethane, extra dry (6 mL). Triethylamine (429.05 mg, 590.97 µL, 4.24 mmol, 1.5 eq) and (Boc)₂O (740.31 mg, 787.56 µL, 3.39 mmol, 1.2 eq) were added, and the mixture was stirred for 90 min. Sat. aq. NaHCO₃ (5 mL) and water (30 mL) were added, and the mixture was extracted with DCM (3 x 30 mL). The combined organic layers were dried (Na₂SO₄), filtered, and concentrated in vacuo to afford the title compound **Intermediate A45** (1.026 g, quant.) as an off-white solid. MS [M+H]⁺ 355.15.

### Intermediate A37

### (6-bromo-1H-pyrrolo[3,2-c]pyridin-3-yl)-morpholino-methanone

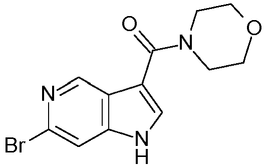

### Step 1: 6-bromo-1H-pyrrolo[3,2-c]pyridine-3-carboxylic acid hydrochloride

**Intermediate A45** (108 mg, 0.304 mmol, 1 eq) was dissolved in isopropanol (0.313 mL). After adding lithium hydroxide (36.41 mg, 1.52 mmol, 5 eq), the mixture was stirred at 100 °C for 1 h. After removal of iPrOH, 1 M HCl (1 mL) was added to the aqueous mixture, whereby a white precipitate formed. The mixture was extracted with EtOAc (3x), and the combined organics were washed with brine, dried over sodium sulfate, filtered and evaporated to afford the title compound 6-bromo-1H-pyrrolo[3,2-c]pyridine-3-carboxylic acid hydrochloride (80 mg, 94.8%) as a white solid. MS [M+HCOO]⁻ 285.19.

### Step 2: (6-bromo-1H-pyrrolo[3,2-c]pyridin-3-yl)-morpholino-methanone

6-bromo-1H-pyrrolo[3,2-c]pyridine-3-carboxylic acid hydrochloride (100 mg, 360.36 µmol, 1 eq) was dissolved in N,N-dimethylformamide (1 mL) and DIPEA (232.88 mg, 314.71 µL, 1.8 mmol, 5 eq) was added. Then, morpholine (156.97 mg, 156.97 µL, 1.8 mmol, 5 eq) and HATU (164.42 mg, 432.43 µmol, 1.2 eq) were added, and the reaction mixture was stirred at RT for 7.5 h. Again, morpholine (156.97 mg, 156.97 µL, 1.8 mmol, 5 eq) was added, and the reaction was stirred overnight. Again, DIPEA (93.15 mg, 125.88 µL, 720.72 µmol, 2 eq) and morpholine (156.97 mg, 156.97 µL, 1.8 mmol, 5 eq) were added, and stirring continued for 4 d. Water was added, and the mixture was extracted with ethyl acetate. The combined organics were washed twice with 5 % LiCl solution and brine. The solvents were evaporated, and the crude material was purified by column chromatography (DCM:MeOH 9:1 in DCM) to yield the title compound **Intermediate A37** (35.8 mg, 32%) as a white solid. MS [M+H]⁺ 309.98.

### Intermediate A38

### (6-bromo-1H-pyrrolo[3,2-c]pyridin-3-yl)-(4,4-difluoropiperidino)methanone

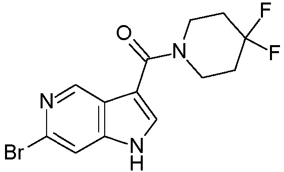

6-bromo-1H-pyrrolo[3,2-c]pyridine-3-carboxylic acid **(Intermediate A37** Step 1; 100 mg, 414.87 µmol, 1 eq) was dissolved in N,N-dimethylformamide (1 mL). DIPEA (268.11 mg, 362.31 µL, 2.07 mmol, 5 eq), 4,4-difluoropiperidine (100.51 mg, 829.74 µmol, 2 eq), and HATU (236.62 mg, 622.3 µmol, 1.5 eq) were added, and the reaction mixture was stirred at RT for 3 d. 5% LiCl (10 mL) was added, and the mixture was extracted with ethyl acetate (3 x 20 mL). The organic phases were dried (Na₂SO₄), filtered, and concentrated in vacuo to afford the title compound **Intermediate A38** (336 mg, quant.) as yellow oil, which was used without further purification. MS [M+H]⁺ 346.0.

The following examples were prepared in analogy of **Intermediate A38**

| **Ex#** | **Name** | **MS ESI** | **Starting Material** |
|---|---|---|---|
| **Intermediate A46** | 6-bromo-N,N-dimethyl-1H-pyrrolo[3,2-c]pyridine-3-carboxamide | 268.13 [M+H]⁺ | 6-bromo-1H-pyrrolo[3,2-c]pyridine-3-carboxylic acid and dimethylamine in THF |

### Intermediate A35

### 6-Bromopyrazolo[4,3-c]pyridine-1-carboxylic acid tert-butyl ester

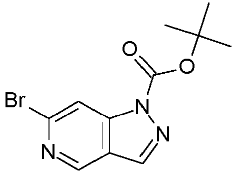

6-bromo-1H-pyrazolo[4,3-c]pyridine (191 mg, 0.965 mmol, 1 eq) was dissolved in dichloromethane, extra dry (2 mL). Triethylamine (146.4 mg, 201.66 µL, 1.45 mmol, 1.5 eq) and (Boc)₂O (252.62 mg, 1.16 mmol, 1.2 eq) were added, and the mixture was stirred for 45 min. NaHCO₃ (5 mL) and water (30 mL) were added, and the mixture was extracted with ethyl acetate (3 x 30 mL). The organic layers were dried (Na₂SO₄), filtered, and concentrated in vacuo. The residue was purified by column chromatography (0-20% DCM/MeOH 9:1 in DCM) to give the title compound **Intermediate A35** (206 mg, 71.6%) as a colorless solid. MS [M+H]⁺ 298.00.

The following examples were prepared in analogy of **Intermediate A35**

| **Ex#** | **Name** | **MS ESI** | **Starting Material** |
|---|---|---|---|
| **Intermediate A42** | 5-bromopyrrolo[2,3-c]pyridine-1,2-dicarboxylic acid O1-tert-butyl ester O2-ethyl ester | 369.09 [M+H]⁺ | 5-bromo-1H-pyrrolo[2,3-c]pyridine-2-carboxylic acid ethyl ester |

### Intermediate A47

### 2-Bromo-4H-thieno[3,2-b]pyrrole-5-carboxylic acid methyl ester

4H-Thieno[3,2-b]pyrrole-5-carboxylic acid methyl ester (CAS 82782-85-2; 863 mg, 4.76 mmol, 1 eq) was dissolved in ethyl acetate (10.01 mL). At 0 °C, NBS (873.09 mg, 4.91 mmol, 1.03 eq) in ethyl acetate (10.01 mL) was added dropwise. The yellow mixture was warmed to RT, and stirred for 1 h. The mixture was filtered through a pad of silica, and washed with chloroform (60 mL). 10% aq. Na2S2O3 (30 mL) was added to the filtrate, and the aq. phase was extracted with DCM (3 x 30 mL). The combined organic phases were dried (Na₂SO₄), filtered, and concentrated in vacuo to give the title compound **Intermediate A47** (1.269 g, quant.) as a yellow solid. MS [M+H]⁺ 260.01.

### Intermediate A49

### 1-[5-Bromo-1-(2-trimethylsilylethoxymethyl)pyrrolo[3,2-b]pyridin-2-yl]ethane-1,2-diol

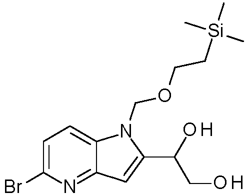

### Step 1: 2-[(5-bromo-2-vinyl-pyrrolo[3,2-b]pyridin-1-yl)methoxy]ethyl-trimethyl-silane

To a mixture of 2-[5-bromo-1-(2-trimethylsilylethoxymethyl)pyrrolo[3,2-b]pyridin-2-yl]ethanol **(Intermediate A14** Step 2; 550.0 mg, 1.48 mmol, 1.0 eq) in DCM (5 mL) was added 1,8-diazabicyclo[5.4.0]undec-7-ene (1.33 mL, 8.89 mmol, 6.0 eq) and methanesulfonic anhydride (1.21 g, 6.96 mmol, 4.7 eq) at 0 °C. The reaction mixture was stirred at 25 °C for 16 h. The mixture was treated with additional 1,8-diazabicyclo[5.4.0]undec-7-ene (1.3 mL, 8.69 mmol, 5.87 eq), and stirred at 40 °C for 2 h. The mixture was poured into water (20 mL). EtOAc (40 mL) and water (40 mL) were added, and the layers were separated. The aqueous phase was extracted with EtOAc (2 x 30 mL). The combined extracts were washed with brine (60 mL), dried over MgSO4, filtered, and concentrated under vacuum to give a residue. The residue was purified by RP column chromatography (80-90% ACN in water(0.1%FA)) to give the title compound 2-[(5-bromo-2-vinyl-pyrrolo[3,2-b]pyridin-1-yl)methoxy]ethyl-trimethyl-silane (416.0 mg, 1.18 mmol, 79.5%) as a light yellow solid. MS [M+H]⁺ 353.0.

### Step 2: 1-[5-bromo-1-(2-trimethylsilylethoxymethyl)pyrrolo[3,2-b]pyridin-2-yl]ethane-1,2-diol

To a solution of 2-[(5-bromo-2-vinyl-pyrrolo[3,2-b]pyridin-1-yl)methoxy]ethyl-trimethyl-silane (380.0 mg, 1.08 mmol, 1.0 eq) in tert-butanol (2 mL) and water (2 mL) was added K₂OsO₄·2H₂O (3.96 mg, 0.01 mmol, 0.01 eq), and then AD-mix-β (1.52 g, 1.95 mmol, 1.81 eq) at 0 °C, and the mixture was stirred for 1 h, and subsequently warmed to 25 °C over 16 h. The mixture was directly purified by RP column chromatography (70-80% ACN in water(0.1%FA)) to obtain the title compound **Intermediate A49** (60.0 mg, 0.15 mmol, 14.4%) as a light brown oil as a mixture of enantiomers (of unknown ratio). MS [M+H]⁺ 389.0.

### Intermediate A40

### tert-butyl 5-bromo-2-[(tert-butoxycarbonylamino)methyl]pyrrolo [3,2-b] pyridine-1-carboxylate

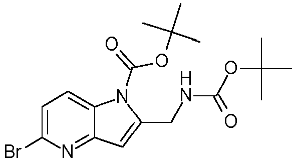

### Step 1: 6-bromo-2-(3,3-diethoxyprop-1-ynyl)pyridin-3-amine

A mixture of triethylamine (20.98 mL, 150.55 mmol, 3.0 eq), copper(I) iodide (0.17 mL, 5.02 mmol, 0.1 eq), [1,1'-bis(diphenylphosphino)ferrocene]dichloropalladium(II) (3.67 g, 5.02 mmol, 0.1 eq), propargylaldehyde diethyl acetal (7.08 g, 55.2 mmol, 1.1 eq) and 6-bromo-2-iodo-pyridin-3-amine **(Intermediate A7** Step 3; 15.0 g, 50.18 mmol, 1.0 eq) in THF (300 mL) was evacuated and backfilled with N₂ (3x). The yellow mixture was stirred at 50 °C for 16 h, and concentrated. The residue was purified by column chromatography (20% EA in PE) and concentrated to afford the title compound 6-bromo-2-(3,3-diethoxyprop-1-ynyl)pyridin-3-amine (9.4 g, 31.42 mmol, 56.6%) as a yellow solid. MS [M+H]⁺ 301.0.

### Step 2: 5-bromo-2-(diethoxymethyl)-1H-pyrrolo[3,2-b]pyridine

To a solution of 6-bromo-2-(3,3-diethoxyprop-1-ynyl)pyridin-3-amine (9.4 g, 31.42 mmol, 1.0 eq) in NMP (120 mL) was added potassium tert-butoxide (7.05 g, 62.84 mmol, 2.0 eq) at 10 °C. The mixture was stirred at 50 °C for 12 h. The mixture slowly poured into cooled 0.05 N HCl solution (80 mL), and extracted with EtOAc (3 x 100 mL). The combined organics were washed with brine (2 x 100 mL), dried over Na₂SO₄, filtered, and concentrated to afford a yellow residue. The residue was purified by column chromatography (25% EA in PE) to afford the title compound 5-bromo-2-(diethoxymethyl)-1H-pyrrolo[3,2-b]pyridine (7.0 g, 23.4 mmol, 74.5%) as a yellow solid. MS [M+H]⁺ 299.3.

### Step 3: 5-bromo-1H-pyrrolo[3,2-b]pyridine-2-carbaldehyde

To a solution of 5-bromo-2-(diethoxymethyl)-1H-pyrrolo[3,2-b]pyridine (3.0 g, 10.03 mmol, 1.0 eq) in THF (50 mL) and water (10 mL) was added p-toluenesulfonic acid monohydrate (2.86 g, 15.04 mmol, 1.5 eq) at 10 °C. The mixture was stirred at 10 °C for 2 h. EtOAc (60 mL) and sat. aq. NaHCO₃ (60 mL) were added and layers were separated. The aqueous phase was extracted with EtOAc (2 x 50 mL). The combined extracts were washed with brine (100 mL), dried over Na₂SO₄, filtered, and concentrated under vacuum to give a residue. The residue was purified by column chromatography (30% EA in PE) to afford the title compound 5-bromo-1H-pyrrolo[3,2-b]pyridine-2-carbaldehyde (1.5 g, 6.67 mmol, 47.1%) as a yellow solid. MS [M+H]⁺ 225.0.

### Step 4: tert-butyl N-[(5-bromo-1H-pyrrolo[3,2-b]pyridin-2-yl)methyl]carbamate

To a mixture of 5-bromo-1H-pyrrolo[3,2-b]pyridine-2-carbaldehyde (1.5 g, 6.67 mmol, 1.0 eq), tert-butyl carbamate (1.56 g, 13.33 mmol, 2.0 eq), and trifluoroacetic acid (1.54 mL, 20.0 mmol, 3.0 eq) in ACN (30 mL) was added triethylsilane (7.75 g, 66.65 mmol, 10.0 eq) at 10 °C. The mixture was stirred at 10 °C for 16 h. The mixture was concentrated in vacuum. The residue was purified by RPHPLC (50-80% ACN in 0.1% FA in water) to afford the title compound tert-butyl N-[(5-bromo-1H-pyrrolo[3,2-b]pyridin-2-yl)methyl]carbamate (400 mg, 1.23 mmol, 17.9%) as a yellow solid. MS [M+H]⁺ 328.0.

### Step 5: tert-butyl 5-bromo-2-[(tert-butoxycarbonylamino)methyl]pyrrolo[3,2-b]pyridine-1-carboxylate

To a solution of tert-butyl N-[(5-bromo-1H-pyrrolo[3,2-b]pyridin-2-yl)methyl]carbamate (400.0 mg, 1.23 mmol, 1.0 eq) in THF (10 mL) was added triethylamine (0.26 mL, 1.84 mmol, 1.5 eq), and di-t-butyldicarbonate (0.56 mL, 2.45 mmol, 2.0 eq). The reaction mixture was stirred at 10 °C for 3 h. The mixture was concentrated in vacuum to afford the crude. The crude was purified by column chromatography (20% EA in PE) to afford the title compound **Intermediate A40** (180.0 mg, 0.58 mmol, 47.5%) as a yellow solid. MS [M+H]⁺ 428.3.

### Intermediate A43

### 2-(6-bromo-1H-pyrrolo[3,2-c]pyridin-3-yl)ethanol

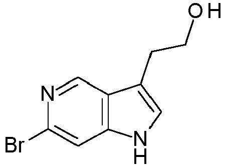

### Step 1: 2-(6-bromo-1H-pyrrolo[3,2-c]pyridin-3-yl)-2-keto-acetic acid

A stirred suspension of 6-bromo-1H-pyrrolo[3,2-c]pyridine (600 mg, 3.05 mmol, 1 eq) in dichloromethane (10 mL) at ambient temperature was treated with aluminum chloride (2.02 g, 15.15 mmol, 4.975 eq). After stirring for 1 h, the mixture was treated with ethyl oxalyl chloride (2.07 g, 1.7 mL, 15.19 mmol, 4.988 eq), and the resulting mixture was stirred overnight. The mixture was treated dropwise with MeOH (792 mg, 1 mL, 24.72 mmol, 8.117 eq), and stirred at ambient temperature for 30 min. The mixture was then filtered through Celite, and the filtrate was concentrated in vacuo. The residue was purified by column chromatography (0-2% DCM/MeOH 9:1 in DCM) to afford the title compound 2-(6-bromo-1H-pyrrolo[3,2-c]pyridin-3-yl)-2-keto-acetic acid (0.27 g, 32%) as a colourless oil. MS [M+H]⁺ 268.9.

### Step 2: 2-(6-bromo-1H-pyrrolo[3,2-c]pyridin-3-yl)ethanol

A stirred suspension of 2-(6-bromo-1H-pyrrolo[3,2-c]pyridin-3-yl)-2-keto-acetic acid (220 mg, 0.818 mmol, 1 eq) in tetrahydrofuran (5 mL) at RT was treated with 2 M borane dimethyl sulfide complex (1.7 mL, 3.4 mmol, 4.158 eq), and the resulting mixture was heated at 65 °C for 2 h. The mixture was cooled to rt and partitioned between aqueous saturated sodium bicarbonat and EtOAc three times. The organic phase was washed with brine, dried over MgSO4, filtered, and concentrated in vacuo. The residue was purified by column chromatography (0-80% DCM/MeOH 9:1 in DCM) to give the title compound **Intermediate A43** (197.13 mg, 93.8%) as a white solid. MS [M+H]⁺ 242.98.

### Intermediate AA1

### 4-[2-methyl-4-[[1-methyl-5-[3-(trifluoromethyl)-1H-pyrazol-4-yl]imidazole-2-carbonyl]amino]benzoyl]piperazine-1-carboxylic acid tert-butyl ester

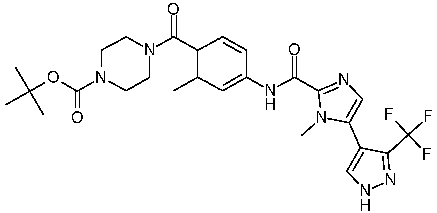

A mixture of 4-[4-[(5-bromo-1-methyl-imidazole-2-carbonyl)amino]-2-methylbenzoyl]piperazine-1-carboxylic acid tert-butyl ester (CAS 2673221-29-7; 6.12 g, 9.61 mmol, 1 eq), chloro[(di(1-adamantyl)-n-butylphosphine)-2-(2-aminobiphenyl)]palladium(II) (642.42 mg, 0.961 mmol, 0.1 eq), Na₂CO₃ (2.04 g, 19.22 mmol, 2 eq) and [3-(trifluoromethyl)-1H-pyrazol-4-yl]boronic acid (2.59 g, 14.41 mmol, 1.5 eq) in 1,4-dioxane (50 mL) and water (5 mL) was evacuated and backfilled with argon (3x). The reaction mixture was then stirred at 100 °C overnight. The mixture was allowed to cool to RT before filtering through a Dicalite plug (washing with AcOEt). The combined organics were evaporated and the crude purified by column chromatography (0-30% DCM/MeOH 9:1 in DCM) to afford the title compound **Intermediate AA1** (2.07 g, 38.4%) as a light brown solid. MS [M+H]⁺ 562.69.

### Intermediate AA2

### 4-[2-fluoro-4-[[1-methyl-5-[3-(trifluoromethyl)-1H-pyrazol-4-yl]imidazole-2-carbonyl]amino]benzoyl]piperazine-1-carboxylic acid tert-butyl ester

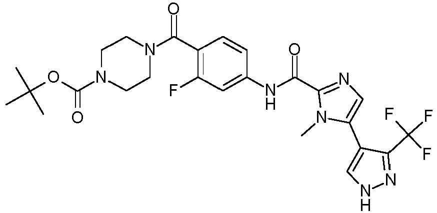

### Step 1: 4-[(5-bromo-1-methyl-imidazole-2-carbonyl)amino]-2-fluoro-benzoic acid ethyl ester

4-Amino-2-fluoro-benzoic acid methyl ester (500 mg, 2.96 mmol, 1 eq) and 5-bromo-1-methylimidazole-2-carboxylic acid ethyl ester (826.7 mg, 3.55 mmol, 1.2 eq) were dissolved in tetrahydrofuran, extra dry (15 mL) and the mixture cooled to -78 °C. The reaction mixture was then treated dropwise with 1 M LiHMDS in THF (4.43 mL, 4.43 mmol, 1.5 eq). The reaction mixture was stirred for a further 2 h at -78 °C before warming to RT, and stirring for an additional 18 h. The reaction mixture was subsequently concentrated in vacuo, and the residue redissolved in EtOAc (150 mL), which was washed with 10% citric acid (50 mL), water (50 mL), and brine (50 mL). The organic phase was dried (Na₂SO₄), filtered, and evaporated. The crude was purified by a column chromatography (0-35% EtOAc/EtOH 3:1 in heptane) to afford a 780 mg mixture of the title compound 4-[(5-bromo-1-methyl-imidazole-2-carbonyl)amino]-2-fluoro-benzoic acid ethyl ester (780 mg) along with the corresponding methyl ester (ratio ~2:1) as light yellow powder, which was used without further purification in the next step. MS [M+H]⁺ 370.0.

### Step 2: 4-[(5-bromo-1-methyl-imidazole-2-carbonyl)amino]-2-fluoro-benzoic acid

4-[(5-bromo-1-methyl-imidazole-2-carbonyl)amino]-2-fluoro-benzoic acid ethyl ester (780 mg, 2.11 mmol, 1 eq) was dissolved in tetrahydrofuran (6 mL), methanol (2 mL) and water (2 mL), and treated with LiOH (504.66 mg, 21.07 mmol, 10 eq). The reaction mixture was stirred at RT for 16 h. Additional LiOH (252.33 mg, 10.54 mmol, 5 eq) was added, and the reaction mixture stirred for a further 6 h at RT. The reaction mixture was concentrated in vacuo, and the residue diluted with 0.5 M HCl (30 mL), and extracted with EtOAc (3 x 20 mL). The combined organics were washed with brine, dried (Na₂SO₄), filtered and evaporated to yield the title compound 4-[(5-bromo-1-methyl-imidazole-2-carbonyl)amino]-2-fluoro-benzoic acid (721 mg, quant.) as a yellow solid, which was used in subsequent steps without further purification. MS [M+H]⁺ 341.9.

### Step 3: 4-[4-[(5-bromo-1-methyl-imidazole-2-carbonyl)amino]-2-fluoro-benzoyl]piperazine-1-carboxylic acid tert-butyl ester

4-[(5-bromo-1-methyl-imidazole-2-carbonyl)amino]-2-fluoro-benzoic acid (720 mg, 2.1 mmol, 1 eq) was dissolved in N,N-dimethylformamide, extra dry (30 mL), and treated with DIPEA (1.36 g, 1.84 mL, 10.52 mmol, 5 eq), and subsequently with 1-Boc-piperazine (470.36 mg, 2.53 mmol, 1.2 eq) and HATU (960.24 mg, 2.53 mmol, 1.2 eq). The resultant mixture was stirred at RT for 2.5 d. An additional portion of 1-Boc-piperazine (117.59 mg, 631.36 umol, 0.3 eq) and HATU (240.06 mg, 631.36 umol, 0.3 eq) were added. After stirring at RT for an additional 2 h the reaction mixture was concentrated in vacuo, and the residue suspended in half-sat. NaHCO₃ (40 mL), and extracted with EtOAc (3 x 25 mL). The combined organics were then washed with brine, dried (Na₂SO₄), filtered, and evaporated. The crude was purified by column chromatography (0-55% EtOAc/EtOH 3:1 in heptane) to afford the title compound 4-[4-[(5-bromo-1-methyl-imidazole-2-carbonyl)amino]-2-fluoro-benzoyl]piperazine-1-carboxylic acid tert-butyl ester (425 mg, 39.6%) as a white solid. MS [M-H]⁻ 510.1.

### Step 4: 4-[2-fluoro-4-[[1-methyl-5-[3-(trifluoromethyl)-1H-pyrazol-4-yl]imidazole-2-carbonyl]amino]benzoyl]piperazine-1-carboxylic acid tert-butyl ester

4-[4-[(5-bromo-1-methyl-imidazole-2-carbonyl)amino]-2-fluoro-benzoyl]piperazine-1-carboxylic acid tert-butyl ester (425 mg, 0.833 mmol, 1 eq), chloro[(di(1-adamantyl)-n-butylphosphine)-2-(2-aminobiphenyl)]palladium(II) (55.68 mg, 83.27 µmol, 0.1 eq), Na₂CO₃ (176.53 mg, 1.67 mmol, 2 eq) and [3-(trifluoromethyl)-1H-pyrazol-4-yl]boronic acid (224.7 mg, 1.25 mmol, 1.5 eq) were dissolved in 1,4-dioxane (5 mL) and water (0.5 mL). The flask was then evacuated and backfilled with argon (3x). The reaction mixture was then heated to 100 °C and stirred under reflux for 2 d. The reaction mixture was allowed to cool to RT, and filtered through a Dicalite plug (washing with EtOAc (4x) and DCM/MeOH (1x)). The combined organics were evaporated, and the crude purified by column chromatography (0-55% EtOAc/EtOH 3:1 in heptane) to afford the title compound **Intermediate AA2** (269 mg, 56%) as a white solid. MS [M-H]⁻ 564.3.

### Intermediate AAA1

### N-[3-chloro-4-(piperazine-1-carbonyl)phenyl]-5-[3-(difluoromethyl)-1H-pyrazol-4-yl]-1-methyl-imidazole-2-carboxamide hydrochloride

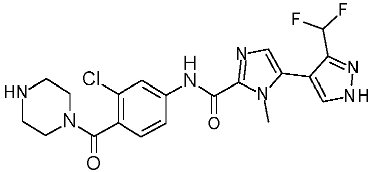

### Step 1: 4-iodo-1H-pyrazole-3-carbaldehyde

A solution of 1H-pyrazole-3-carbaldehyde (10.0 g, 104.07 mmol, 1.0 eq) in water (100 mL) / sulfuric acid (10.0 mL, 104.07 mmol, 1.0 eq) was added N-iodosuccinimide (24.58 g, 109.27 mmol, 1.05 eq) at 0 °C, then the solution was stirred at 30 °C for 16 h. The mixture was filtered, the residue was washed with water (500 mL) and MTBE/MeOH 10:1 (1 L) to afford the title compound 4-iodo-1H-pyrazole-3-carbaldehyde (16.6 g, 74.78 mmol, 71.9%) as a white solid. 1H NMR (400 MHz, CD3 SOCD3, 298 K): δ (ppm) = 14.13 (br s, 1H), 9.86 (s, 1H), 8.11 (br s, 1H).

### Step 2: 4-iodo-1-trityl-pyrazole-3-carbaldehyde

To a solution of 4-iodo-1H-pyrazole-3-carbaldehyde (16.6 g, 74.78 mmol, 1.0 eq) in THF (200 mL) was added sodium hydride, 60% in oil (3.29 g, 82.26 mmol, 1.1 eq) at 0 °C. The mixture was stirred for 0.5 h at 0 °C, then triphenylmethyl chloride (22.93 g, 82.26 mmol, 1.1 eq) was added to the solution in portions, and subsequently the mixture was stirred for 16 h at 10 °C. The mixture was quenched with sat. NH₄Cl (300 mL), and extracted with EtOAc (2 x 100 mL). The combined organics were washed with brine (200 mL), dried over sodium sulfate, filtered, and concentrated in vacuum. The crude product was washed with EtOAc/MTBE 10:1 to afford the title compound 4-iodo-1-trityl-pyrazole-3-carbaldehyde (16.6 g, 35.75 mmol, 47.8%) as a white solid. MS [M+Na]⁺ 487.1.

### Step 3: 3-(difluoromethyl)-4-iodo-1-trityl-pyrazole

To a solution of 4-iodo-1-trityl-pyrazole-3-carbaldehyde (16.6 g, 35.75 mmol, 1.0 eq) in DCM (200 mL) cooled to -15°C was added diethylaminosulfur trifluoride (14.06 mL, 107.26 mmol, 3.0 eq). The resulting mixture was stirred at 30 °C for 16 h. The mixture was quenched with sat. NaHCO₃, the organic layer was separated, dried over sodium sulfate, filtered, and concentrated. The residue was purified by column chromatography (PE/EA 100:1) to afford the title compound 3-(difluoromethyl)-4-iodo-1-trityl-pyrazole (17.2 g, 35.37 mmol, 98.9%) as a white solid. MS [M+H]⁺ 487.1.

### Step 4: [3-(difluoromethyl)-1-trityl-pyrazol-4-yl]boronic acid

To a solution of 3-(difluoromethyl)-4-iodo-1-trityl-pyrazole (8.0 g, 16.45 mmol, 1.0 eq) and boron isopropoxide (4.56 mL, 19.74 mmol, 1.2 eq) in THF (100 mL) was added dropwise butyllithium solution (7.9 mL, 19.74 mmol, 1.2 eq) at -70 °C under N₂, and the mixture was stirred for 1 h at -70 °C. The mixture was poured into 300 mL sat. NH₄Cl, and stirred for 0.5 h. The mixture was extracted with EtOAc (2 x 300 mL), washed with brine (500 mL), dried over sodium sulfate, filtered, and concentrated in vacuum. The crude material was purified by column chromatography (PE/EA 4: 1) to afford the title compound [3-(difluoromethyl)-1-trityl-pyrazol-4-yl]boronic acid (1.1 g, 2.72 mmol, 16.5%) as a colorless oil, which was not further characterized, and directly used in the next step.

### Step 5: tert-butyl 4-[2-chloro-4-[[5-[3-(difluoromethyl)-1-trityl-pyrazol-4-yl]-1-methylimidazole-2-carbonyl]amino]benzoyl]piperazine-1-carboxylate

A mixture of [1,1'-bis(diphenylphosphino)ferrocene]dichloropalladium(II) (152.78 mg, 0.21 mmol, 0.1 eq), tert-butyl 4-[4-[(5-bromo-1-methyl-imidazole-2-carbonyl)amino]-2-chlorobenzoyl]piperazine-1-carboxylate (CAS 2489205-91-4; 1.1 g, 2.09 mmol, 1.0 eq), sodium carbonate (442.61 mg, 4.18 mmol, 2.0 eq), and [3-(difluoromethyl)-1-trityl-pyrazol-4-yl]boronic acid (1.01 g, 2.51 mmol, 1.2 eq) in 1,4-dioxane (15 mL) / water (1 mL) was stirred under N₂ at 85 °C for 16 h. The mixture was filtered, concentrated, and purified by column chromatography (35% EA in PE) and RPHPLC (100% ACN in 0.1% FA in water) to afford the title compound tert-butyl 4-[2-chloro-4-[[5-[3-(difluoromethyl)-1-trityl-pyrazol-4-yl]-1-methyl-imidazole-2-carbonyl]amino]benzoyl]piperazine-1-carboxylate (330.0 mg, 0.41 mmol, 19.6%) as a white solid. MS [M+H]⁺ 806.3.

### Step 6: N-[3-chloro-4-(piperazine-1-carbonyl)phenyl]-5-[3-(difluoromethyl)-1H-pyrazol-4-yl]-1-methyl-imidazole-2-carboxamide hydrochloride

A solution of tert-butyl 4-[2-chloro-4-[[5-[3-(difluoromethyl)-1-trityl-pyrazol-4-yl]-1-methylimidazole-2-carbonyl]amino]benzoyl]piperazine-1-carboxylate (330.0 mg, 0.41 mmol, 1.0 eq) in hydrochloric acid in MeOH (10.0 mL, 40.0 mmol, 97.73 eq) was stirred at 30 °C for 16 h. The mixture was concentrated, and the residue was diluted with MTBE/MeOH 10:1 (100 mL). The mixture was filtered, and the solid was dried to afford the title compound **Intermediate AAA1** (160.0 mg, 0.32 mmol, 84.3%) as a white solid. MS [M+H]⁺ 464.2.

### Intermediate AAA2

### N-[3-fluoro-4-(piperazine-1-carbonyl)phenyl]-1-methyl-5-[3-(trifluoromethyl)-1H-pyrazol-4-yl]imidazole-2-carboxamide;dihydrochloride

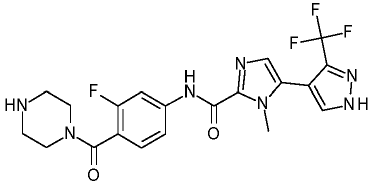

### Step 1: tert-butyl 4-[2-fluoro-4-[[1-methyl-5-[3-(trifluoromethyl)-1-trityl-pyrazol-4-yl]imidazole-2-carbonyl]amino]benzoyl]piperazine-1-carboxylate

A mixture of **Intermediate BB1** (1.46 g, 4.52 mmol, 1.2 eq) and ethyl 1-methyl-5-[3-(trifluoromethyl)-1-trityl-pyrazol-4-yl]imidazole-2-carboxylate (CAS 2489206-32-6; 2.0 g, 3.77 mmol, 1.0 eq) in THF (40 mL) was cooled to -40 °C, and treated dropwise with sodium bis(trimethylsilyl)amide (4.9 mL, 4.9 mmol, 1.3 eq) at -40 °C. The mixture was stirred at -40 °C for 1 h. The mixture was quenched with saturated NH₄Cl (100 mL), and then extracted with EA (3 x 80 mL). The combined organic layers were washed with brine (50 mL), dried over sodium suflate, filtered, and concentrated under vacuum. The residue was purified by RP column chromatography (13-75% ACN in water(FA)) to give the title compound tert-butyl 4-[2-fluoro-4-[[1-methyl-5-[3-(trifluoromethyl)-1-trityl-pyrazol-4-yl]imidazole-2-carbonyl]amino]benzoyl]piperazine-1-carboxylate (2.1 g, 2.6 mmol, 67.2%) as a light yellow solid. MS [M+H]⁺ 808.2.

### Step 2: N-[3-fluoro-4-(piperazine-1-carbonyl)phenyl]-1-methyl-5-[3-(trifluoromethyl)-1H-pyrazol-4-yl]imidazole-2-carboxamide;dihydrochloride

A mixture of tert-butyl 4-[2-fluoro-4-[[1-methyl-5-[3-(trifluoromethyl)-1-trityl-pyrazol-4-yl]imidazole-2-carbonyl]amino]benzoyl]piperazine-1-carboxylate (1.0 g, 1.24 mmol, 1.0 eq) in 4 M HCl in dioxane (20.0 mL, 80.0 mmol, 64.63 eq) was stirred at 30 °C for 16 h to give a white suspension. The mixture was concentrated under vacuum, and the residue was triturated with EA/MTBE 1:3 (30 mL) to give the title compound **Intermediate AAA2** (690.0 mg, 1.28 mmol, 89.6%) as a white solid. MS [M+H]⁺ 466.2.

### Intermediate BB1

### tert-butyl 4-(4-amino-2-fluoro-benzoyl)piperazine-1-carboxylate

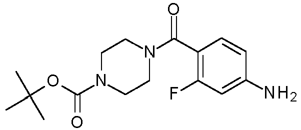

To a solution of 1-BOC-piperazine (12.01 g, 64.46 mmol, 1.0 eq), 4-amino-2-fluorobenzoic acid (10.0 g, 64.46 mmol, 1.0 eq) and N,N-diisopropylethylamine (13.47 mL, 77.35 mmol, 1.2 eq) in DMF (120 mL) was added O-(7-azabenzotriazol-1-yl)-N,N,N',N'-tetramethyluronium hexafluorophosphate (26.96 g, 70.91 mmol, 1.1 eq) at 0 °C. The mixture was stirred at 0 °C for 0.5 h to give a brown solution. The mixture was poured into water (300 mL), and then extracted with EA (3 x 200 mL). The combined organic layers were washed with brine (200 mL), dried over sodium sulfate, filtered, and concentrated under vacuum. The residue was purified by column chromatography (0-100% EA in PE) to yield the title compound **Intermediate BB1** (20.0 g, 61.85 mmol, 92.2%) as a light brown gum. MS [M-tBu+H]⁺ 268.1.

### Intermediate CC1

### Ethyl 5-[1-(2-fluoro-4-methoxy-phenyl)-3-(trifluoromethyl)pyrazol-4-yl]-1-methylimidazole-2-carboxylate

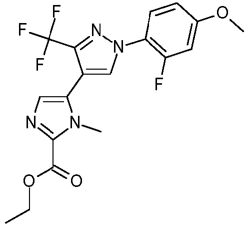

To a solution of 2-fluoro-4-methoxyphenylboronic acid (2.359 g, 13.88 mmol, 2.0 eq), ethyl 1-methyl-5-[3-(trifluoromethyl)-1H-pyrazol-4-yl]imidazole-2-carboxylate (CAS 2489206-33-7; 2.0 g, 6.94 mmol, 1.0 eq), pyridine (1.68 mL, 20.82 mmol, 3.0 eq) and 4Å molecular sieve (2.0 g) in DMF (40 mL) was added copper(II) acetate (2.521 g, 13.88 mmol, 2.0 eq). The mixture was stirred at 20 °C for 16 h under air. Again, 2-fluoro-4-methoxyphenylboronic acid (1.179 g, 6.94 mmol, 1.0 eq) and copper(II) acetate (1.260 g, 6.94 mmol, 1.0 eq) were added into the mixture. The mixture was stirred at 20 °C for another 20 h under air. Then, additional 2-fluoro-4-methoxyphenylboronic acid (1.179 g, 6.94 mmol, 1.0 eq), diacetoxycopper (1.260 mg, 6.94 mmol, 1.0 eq) and pyridine (1.12 mL, 13.88 mmol, 2.0 eq) were added into the mixture. The mixture was stirred at 20 °C for another 20 h under air. The mixture was poured into water (200 mL), and stirred for 24 h. The mixture was filtered through a pad of Celite (washings with EA, 4 x 30 mL). The combined filtrate was extracted with EA (2 x 80 mL). The combined organic layers were washed with brine (3 x 50 mL), dried over sodium sulfate, filtered, and concentrated under vacuum. The residue was purified by column chromatography (30-100% EA in PE) to afford the title compound **Intermediate CC1** (280.0 mg, 0.68 mmol, 9.8%) as a light yellow oil. MS [M+H]⁺ 413.1.

### Intermediate I1

### 3-[[1-(2-tert-Butoxy-2-keto-ethyl)-4-(piperazine-1-carbonyl)piperidin-1-ium-1-yl]methyl]azetidine-1-carboxylic acid tert-butyl ester .1:1 2,2,2-trifluoroacetate

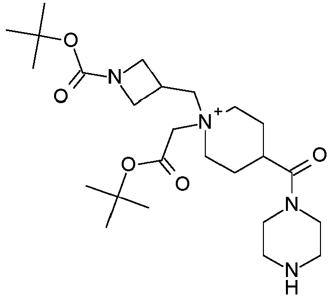

### Step 1: 4-[1-[(1-tert-Butoxycarbonylazetidin-3-yl)methyl]-1-(2-tert-butoxy-2-keto-ethyl)piperidin-1-ium-4-carbonyl]piperazine-1-carboxylic acid benzyl ester .1:1 2,2,2-trifluoroacetate

A mixture of **Intermediate H2** (155 mg, 0.338 mmol, 1 eq) in N,N-dimethylformamide (1.5 mL) was treated with DIPEA (218.44 mg, 295.19 µL, 1.69 mmol, 5 eq), benzyl 1-piperazinecarboxylate (111.68 mg, 97.97 µL, 0.507 mmol, 1.5 eq), and subsequently with HATU (192.79 mg, 0.507 mmol, 1.5 eq). The reaction mixture was stirred at RT O/N. Water was added to the reaction mixture, and the mixture was extracted with EtOAc. After removal of the solvent, the crude residue was purified by RPHPLC to give 4-[1-[(1-tert-butoxycarbonylazetidin-3-yl)methyl]-1-(2-tert-butoxy-2-keto-ethyl)piperidin-1-ium-4-carbonyl]piperazine-1-carboxylic acid benzyl ester .1:1 2,2,2-trifluoroacetate (146.3 mg, 57%) as off-white lyophilized solid. MS [M]+ 615.4.

### Step 2: 3-[[1-(2-tert-Butoxy-2-keto-ethyl)-4-(piperazine-1-carbonyl)piperidin-1-ium-1-yl]methyl]azetidine-1-carboxylic acid tert-butyl ester .1:1 2,2,2-trifluoroacetate

A mixture of 4-[1-[(1-tert-butoxycarbonylazetidin-3-yl)methyl]-1-(2-tert-butoxy-2-keto-ethyl)piperidin-1-ium-4-carbonyl]piperazine-1-carboxylic acid benzyl ester .1:1 2,2,2-trifluoroacetate (146.3 mg, 0.193 mmol, 1 eq) in methanol (5 mL) was treated with palladium hydroxide on carbon (1.35 mg, 0.010 mmol, 0.05 eq), and the reaction mixture was stirred under a H₂ atmosphere. After 3.5 h, the reaction mixture was filtered under Ar, and the solvent evaporated to dryness to yield the title compound **Intermediate I1** as white solid. MS [M]+ 481.3.

The following examples were prepared in analogy of **Intermediate I1**

| **Ex#** | **Name** | **MS ESI** | **Starting Material** |
|---|---|---|---|
| **Intermediate I5** | cis-tert-butyl 3-[[1-(2-tert-butoxy-2-oxo-ethyl)-4-(piperazine-1-carbonyl)piperidin-1-ium-1-yl]methyl]azetidine-1-carboxylate;2,2,2-trifluoroacetate | 481.3 [M]+ | Intermediate H4 and benzyl 1-piperazinecarboxylate, then wet 10% Pd/C |
| **Intermediate I2** | tert-butyl 2-[1-[3-(tert-butoxycarbonylamino)propyl]-4-(piperidine-4-carbonyl)piperazin-1-ium-1-yl]acetate;bromide | 469.2 [M]+ | Intermediate J1 and 1-[(benzyloxy)carbonyl]p iperidine-4-carboxylic acid |
| | | | HATU, in DCM, then Pd/C |

### Intermediate I3

### 3-[[1-(2-tert-butoxy-2-keto-ethyl)-4-(piperazine-1-carbonyl)piperazin-1-ium-1-yl]methyl]azetidine-1-carboxylic acid tert-butyl ester .1:1 2,2,2-trifluoroacetate

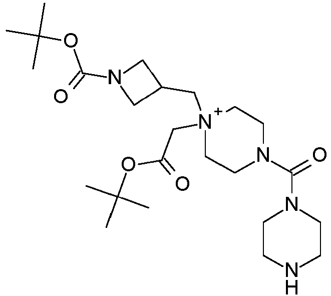

### Step 1: 4-[4-[(1-tert-butoxycarbonylazetidin-3-yl)methyl]-4-(2-tert-butoxy-2-keto-ethyl)piperazin-4-ium-1-carbonyl]piperazine-1-carboxylic acid benzyl ester 2,2,2-trifluoroacetate

To a light brown suspension of **Intermediate J2** (500 mg, 1.11 mmol, 1 eq) in dichloromethane (12.34 mL) was added DIPEA (717.39 mg, 0.969 mL, 5.55 mmol, 5 eq) followed by triphosgene (131.77 mg, 444.04 µmol, 0.4 eq) (careful addition, slightly exothermic). After stirring for 30 min at RT, benzyl 1-piperazinecarboxylate (733.56 mg, 643.48 µL, 3.33 mmol, 3 eq) was added, and stirring continued for 1 h. The reaction mixture was concentrated, and directly purified by RP column chromatography (acetonitrile in water(TFA)) to afford the title compound 4-[4-[(1-tert-butoxycarbonylazetidin-3-yl)methyl]-4-(2-tert-butoxy-2-keto-ethyl)piperazin-4-ium-1-carbonyl]piperazine-1-carboxylic acid benzyl ester 2,2,2-trifluoroacetate (449.5 mg, 43.83%) as a light brown lyophilized solid. MS [M]+ 617.48.

### Step 2: 3-[[1-(2-tert-butoxy-2-keto-ethyl)-4-(piperazine-1-carbonyl)piperazin-1-ium-1-yl]methyl]azetidine-1-carboxylic acid tert-butyl ester 2,2,2-trifluoroacetate

4-[4-[(1-tert-butoxycarbonylazetidin-3-yl)methyl]-4-(2-tert-butoxy-2-keto-ethyl)piperazin-4-ium-1-carbonyl]piperazine-1-carboxylic acid benzyl ester 2,2,2-trifluoroacetate (449.5 mg, 0.487 mmol, 1 eq) was dissolved in methanol to give a light brown solution. Palladium on carbon 10% (2.59 mg, 24.33 umol, 0.05 eq) was added, and the reaction mixture was stirred under H₂ atmosphere overnight. The reaction mixture was filtered. To the obtained solution (filtrate) palladium hydroxide on carbon (3.42 mg, 24.33 umol, 0.05 eq) was added, and the reaction mixture stirred under H₂ atmosphere for 3 h. The suspension was filtered, and concentrated in vacuo to afford the title compound **Intermediate I3** (234.9 mg, 79.18%) as a light brown foam, which was used without further purification in the next step. MS [M]+ 482.18.

The following examples were prepared in analogy of **Intermediate I3**

| **Ex#** | **Name** | **MS ESI** | **Starting Material** |
|---|---|---|---|
| **Intermediate I4** | 2-[1-[3-(tert-butoxycarbonylamino)propyl]-4-(piperazine-1-carbonyl)piperazin-1-ium-1-yl]acetic acid tert-butyl ester .1:1 2,2,2-trifluoroacetate | 470.4 [M]+ | Intermediate J1 and benzyl 1-piperazinecarboxylate |

### Intermediate H3

### bis[3-(tert-Butoxycarbonylamino)propyl]-(2-tert-butoxy-2-keto-ethyl)-(3-carboxypropyl)ammonium;bromide

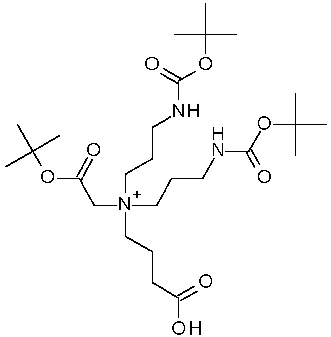

### Step 1: benzyl 4-[bis[3-(tert-butoxycarbonylamino)propyl]amino]butanoate

To a solution of 3-(BOC-amino)propyl bromide (4.56 g, 19.16 mmol) in ACN (30 mL) was added benzyl 4-aminobutanoate;hydrochloride (2.0 g, 8.71 mmol) at 10 °C, and then the mixture was stirred at 70 °C for 16 h.The solution was concentrated, and the residue was purified by RPHPLC to afford benzyl 4-[bis[3-(tert-butoxycarbonylamino) propyl]amino]butanoate (2.2 g). MS [M+H]⁺ 508.4.

### Step 2: (4-benzyloxy-4-oxo-butyl)-bis[3-(tert-butoxycarbonylamino)propyl]-(2-tert-butoxy-2-oxo-ethyl)ammonium; bromide

To a solution of tert-butyl bromoacetate (1.27 mL, 7.88 mmol) in ACN (20 mL) was added benzyl 4-[bis[3-(tert-butoxycarbonylamino)propyl]amino]butanoate (2.0 g, 3.94 mmol) at 10 °C, and then the mixture was stirred at 80 °C for 16 h. The solution was concentrated, and the residue was purified by RPHPLC to afford (4-benzyloxy-4-oxo-butyl)-bis[3-(tert-butoxycarbonylamino)propyl]-(2-tert-butoxy-2-oxo-ethyl)ammonium;bromide (1 g) as colorless oil. MS [M]⁺ 622.5.

### Step 3: bis[3-(tert-butoxycarbonylamino)propyl]-(2-tert-butoxy-2-oxo-ethyl)-(3-carboxypropyl)ammonium; bromide

To a solution of palladium on actived carbon (151.44 mg, 0.140 mmol) in methanol (10 mL) was added (4-benzyloxy-4-oxo-butyl)-bis[3-(tert-butoxycarbonylamino)propyl]-(2-tert-butoxy-2-oxo-ethyl)ammonium;bromide (1.0 g, 1.42 mmol) under N₂, and then the mixture was stirred under H₂ at 10 °C for 16 h. The mixture was filtered, and concentrated to afford the title compound **Intermediate H3** (700 mg). MS [M]⁺ 532.4.

The following examples were prepared in analogy of **Intermediate H3**

| **Ex#** | **Name** | **MS ESI** | **Starting Material** |
|---|---|---|---|
| **Intermediate H1** | bis[4-(tert-butoxycarbonylamino)butyl]-(2-tert-butoxy-2-oxo-ethyl)-(3-carboxypropyl)ammonium;bromide | 560.8 [M]+ | benzyl 4-aminobutanoate;hydroc hloride and 4-(tert-butoxycarbonylamino)b utyl 4-methylbenzenesulfonate and tert-butyl bromoacetate |
| **Internediate H8** | bis[(1-tert-butoxycarbonylazetidin-3-yl)methyl]-(2-tert-butoxy-2-oxo-ethyl)-(3-carboxypropyl)ammonium;bromide | 556.4 [M]+ | benzyl 4-aminobutanoate;hydroc hloride and 1-BOC-3-(bromomethyl)azetidine and tert-butyl bromoacetate |
| **Intermediate H12** | 4-[3-(tert-butoxycarbonylamino)propyl-(2-tert-butoxy-2-oxo-ethyl)amino]butanoic acid | 375.2 [M+H]⁺ | benzyl 4-aminobutanoate;hydroc hloride and 3-(BOC-amino)propyl bromide and tert-butyl bromoacetate |
| **Intermediate H14** | bis[3-(tert-butoxycarbonylamino)propyl]-(3-carboxypropyl)-(4-methoxy-4-oxo-butyl)ammonium bromide | 518.5 [M]⁺ | benzyl 4-aminobutanoate hydrochloride and 3-(BOC-amino)propyl bromide and methyl 4-bromocrotonate |

### Intermediate H2

### 1-[(1-tert-Butoxycarbonylazetidin-3-yl)methyl]-1-(2-tert-butoxy-2-oxo-ethyl)piperidin-1-ium-4-carboxylic acid;2,2,2-trifluoroacetate

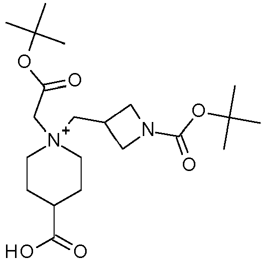

### Step 1: benzyl 1-[(1-tert-butoxycarbonylazetidin-3-yl)methyl]piperidine-4-carboxylate

To a solution of benzyl piperidine-4-carboxylate;hydrochloride (16.0 g, 62.56 mmol, 1 eq) and potassium carbonate (25.94 g, 187.69 mmol, 3 eq) in DMF (150 mL) was added 1-BOC-3-(bromomethyl)azetidine (18.78 g, 75.08 mmol, 1.2 eq). The mixture was stirred at 50 °C for 16 h, diluted with EA (800 mL), and then washed with brine (3 x 300 mL). The organic layer was dried over sodium sulfate, filtered, and concentrated under vacuum. The residue was purified by column chromatography (33-100% EtOAc in PE) to give benzyl 1-[(1-tert-butoxycarbonylazetidin-3-yl)methyl]piperidine-4-carboxylate as light yellow oil. MS [M+H]⁺ 389.1.

### Step 2: benzyl 1-[(1-tert-butoxycarbonylazetidin-3-yl)methyl]-1-(2-tert-butoxy-2-oxoethyl)piperidin-1-ium-4-carboxylate;2,2,2-trifluoroacetate

To a solution of benzyl 1-[(1-tert-butoxycarbonylazetidin-3-yl)methyl]piperidine-4-carboxylate (25.0 g, 64.35 mmol, 1 eq) and sodium iodide (964.57 mg, 6.44 mmol, 0.100 eq) in DMF (250 mL) was added tert-butyl bromoacetate (25.1 g, 128.7 mmol, 2 eq) and N,N-diisopropylethylamine (33.63 mL, 193.05 mmol, 3 eq). The mixture was stirred at 60 °C for 16 h. The mixture was concentrated under vacuum, and the residue was purified twice by RPHPLC to give benzyl 1-[(1-tert-butoxycarbonylazetidin-3-yl)methyl]-1-(2-tert-butoxy-2-oxoethyl)piperidin-1-ium-4-carboxylate;2,2,2-trifluoroacetate (20.2 g, 47.8%) as a yellow solid. MS [M]⁺ 503.2.

### Step 3: 1-[(1-tert-butoxycarbonylazetidin-3-yl)methyl]-1-(2-tert-butoxy-2-oxoethyl)piperidin-1-ium-4-carboxylic acid;2,2,2-trifluoroacetate

To a solution of benzyl 1-[(1-tert-butoxycarbonylazetidin-3-yl)methyl]-1-(2-tert-butoxy-2-oxoethyl)piperidin-1-ium-4-carboxylate;2,2,2-trifluoroacetate (19.0 g, 30.81 mmol, 1 eq) in methanol (300 mL) was added palladium on charcoal (1.4 mL, 1.35 mmol, 0.040 eq) and palladium hydroxide on charcoal (1438.71 mg, 1.02 mmol, 0.030 eq) under N₂. The mixture was degassed, and then stirred at 15 °C for 4 h under H₂. The mixture was filtered through a pad of Celite, and the residue was washed with MeOH (4 x 20 mL). The combined filtrate was concentrated under vacuum. The residue was dissolved in water (100 mL), and then lyophilized to give the title compound **Intermediate H2** (15.07 g, 92.9%) as a light yellow solid. MS [M]⁺ 413.2.

The following examples were prepared in analogy of **Intermediate H2**

| **Ex#** | **Name** | **MS ESI** | **Starting Material** |
|---|---|---|---|
| **Intermediate H13** | 1-(2-tert-butoxy-2-keto-ethyl)-1-(3-hydroxypropyl)piperidin-1-ium-4-carboxylic acid .1:1 2,2,2-trifluoroacetate | 302.1 [M]+ | benzyl piperidine-4-carboxylate; hydrochloride and 3-bromopropoxymethylbenzene and tert-butyl bromoacetate |
| **Intermediate H17** | 1-(2-tert-butoxy-2-oxo-ethyl)-1-methyl-piperidin-1-ium-4-carboxylic acid;iodide | 258.1 [M]+ | benzyl piperidine-4-carboxylate hydrochloride and tert-butyl bromoacetate and iodomethane |
| **Intermediate H9** | 1-[(1-tert-butoxycarbonylazetidin-3-yl)methyl]-1-(4-tert-butoxy-4-oxo-butyl)piperidin-1-ium-4-carboxylic acid;formate | 441.4 [M]+ | benzyl piperidine-4-carboxylate and 1-BOC-3-(bromomethyl)azetidine and t-butyl 4-bromobutanoate |

### Preparation of cis- and trans-isomers of Intermediate H2:

### Intermediate H10

### trans-1-[(1-tert-butoxycarbonylazetidin-3-yl)methyl]-1-(2-tert-butoxy-2-keto-ethyl)piperidin-1-ium-4-carboxylic acid .1:1 2,2,2-trifluoroacetate and

### Intermediate H4

### cis-1-[(1-tert-butoxycarbonylazetidin-3-yl)methyl]-1-(2-tert-butoxy-2-keto-ethyl)piperidin-1-ium-4-carboxylic acid .1:1 2,2,2-trifluoroacetate

The pure cis- and trans-isomers of **Intermediate H2** were prepared in analogy to the preparation of the cis/trans-mixture **Intermediate H2.** The separation of the diastereoisomers was performed after Step 2 by SFC with the conditions: column achiral 100PEI, 5µm, 250x30 mm, 15% MeOH + 0.2% TFA, with the following retention times: trans-isomer Rt = 4.580 min (MS 503.3), cis-isomer Rt = 4.986 min (MS 503.4).

| **Ex#** | **Name** | **MS ESI** | **Starting Material** |
|---|---|---|---|
| **Intermediate H10** | trans-1-[(1-tert-butoxycarbonylazetidin-3-yl)methyl]-1-(2-tert-butoxy-2-keto-ethyl)piperidin-1-ium-4-carboxylic acid .1:1 2,2,2-trifluoroacetate | 413.4 [M]⁺ | benzyl piperidine-4-carboxylate;hydrochloride and 1-BOC-3-(bromomethyl)azetidine and tert-butyl bromoacetate |
| **Intermediate H4** | cis-1-[(1-tert-butoxycarbonylazetidin-3-yl)methyl]-1-(2-tert-butoxy-2-keto-ethyl)piperidin-1-ium-4-carboxylic acid .1:1 2,2,2-trifluoroacetate | 413.4 [M]⁺ | benzyl piperidine-4-carboxylate;hydrochlor ide and 1-BOC-3-(bromomethyl)azetidine and tert-butyl bromoacetate |

### Intermediate H5

### 1-[3-(tert-Butoxycarbonylamino)propyl]-1-(2-tert-butoxy-2-keto-ethyl)piperidin-1-ium-4-carboxylic acid .1:1 formate

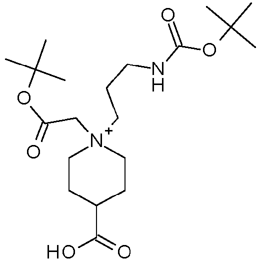

### Step 1: 1-[3-(tert-butoxycarbonylamino)propyl]isonipecotic acid benzyl ester

A mixture of isonipecotic acid benzyl ester .1:1 hydrogen chloride (1 g, 3.79 mmol, 1 eq) in N,N-dimethylformamide, extra dry (18 mL) was treated with N-(3-bromopropyl)carbamic acid tert-butyl ester (1.35 g, 5.69 mmol, 1.5 eq) and K₂CO₃ (1.05 g, 7.59 mmol, 2 eq), and the suspension was stirred at 50°C O/N. The reaction mixture (suspension) was filtered, and extracted with DCM (3 x 15mL). The combined organics were washed with 5% LiCl solution, brine, dried (sodium sulfate), filtered, and evaporated to give crude 1-[3-(tert-butoxycarbonylamino)propyl]isonipecotic acid benzyl ester (3 g, quant.) as light yellow oil, which was directly used in the next step. MS [M+H]⁺ 377.

### Step 2: 1-[3-(tert-butoxycarbonylamino)propyl]-1-(2-tert-butoxy-2-keto-ethyl)piperidin-1-ium-4-carboxylic acid benzyl ester .1:1 formate

A mixture of 1-[3-(tert-butoxycarbonylamino)propyl]isonipecotic acid benzyl ester (1.4 g, 3.72 mmol, 1 eq) and tert-butyl bromoacetate (1.09 g, 824.21 µL, 5.58 mmol, 1.5 eq) in N,N-dimethylformamide was treated with DIPEA (1.92 g, 2.59 mL, 14.87 mmol, 4 eq), and the mixture was stirred at 80 °C for 3 days. The reaction mixture was extracted with DCM (3 x 20 mL). The combined organic layers were washed with 5% LiCl solution (20 mL), brine, dried (Na₂SO₄), filtered, and evaporated. The crude was purified by RPHPLC to give 1-[3-(tert-butoxycarbonylamino)propyl]-1-(2-tert-butoxy-2-keto-ethyl)piperidin-1-ium-4-carboxylic acid benzyl ester .1:1 formate as orange oil. MS [M]⁺ 491.

### Step 3: 1-[3-(tert-butoxycarbonylamino)propyl]-1-(2-tert-butoxy-2-keto-ethyl)piperidin-1-ium-4-carboxylic acid .1:1 formate

To a solution of 1-[3-(tert-butoxycarbonylamino)propyl]-1-(2-tert-butoxy-2-keto-ethyl)piperidin-1-ium-4-carboxylic acid benzyl ester;formate (500 mg, 0.932 mmol, 1 eq) in methanol was added palladium hydroxide on charcoal (39.25 mg, 0.028 mmol, 0.030 eq) under argon. The reaction mixture was degassed, and then stirred under a H₂ atmosphere for 2 h. The reaction mixture was filtered over Celite, and the residue was washed with MeOH (3 x 5 mL). The solvents were evaporated to afford the title compound Intermediate H5 (410 mg, 96.6%) as orange waxy solid, which was lyophilized to give an orange lyophilized solid. MS [M]⁺ 401.

### Preparation of cis- and trans-isomers of Intermediate H5:

### Intermediate H6

### trans-1-[3-(tert-butoxycarbonylamino)propyl]-1-(2-tert-butoxy-2-keto-ethyl)piperidin-1-ium-4-carboxylate and

### Intermediate H7

### cis-1-[3-(tert-butoxycarbonylamino)propyl]-1-(2-tert-butoxy-2-keto-ethyl)piperidin-1-ium-4-carboxylate

The pure cis- and trans-isomers of **Intermediate H5** were prepared in analogy to the preparation of the cis/trans-mixture **Intermediate H5.** The separation of the diastereoisomers was performed after Step 3 by SFC with the conditions: column chiral 4-cellulose, 5µm, 250x20 mm, 30% MeOH + 0.2% DEA, with the following retention times: trans-isomer Rt = 1.252 min (MS 401.5), cis-isomer Rt = 1.291 min (MS 401.5).

| **Ex#** | **Name** | **MS ESI** | **Starting Material** |
|---|---|---|---|
| **Interme diate H6** | trans-1-[3-(tert-butoxycarbonylamino)propyl]-1-(2-tert-butoxy-2-keto-ethyl)piperidin-1-ium-4-carboxylate | 401.3 [M]+ | isonipecotic acid benzyl ester .1:1 hydrogen chloride and N-(3-bromopropyl)carbamic acid tert-butyl ester and tert-butyl bromoacetate |
| **Interme diate H7** | cis-1-[3-(tert-butoxycarbonylamino)propyl]-1-(2-tert-butoxy-2-keto-ethyl)piperidin-1-ium-4-carboxylate | 401.3 [M]+ | isonipecotic acid benzyl ester .1:1 hydrogen chloride and N-(3-bromopropyl)carbamic acid tert-butyl ester and tert-butyl bromoacetate |

### Intermediate H11

### rac-3-(tert-Butoxycarbonylamino)propyl-(2-tert-butoxy-2-keto-ethyl)-(3-carboxypropyl)-methyl-ammonium; bromide

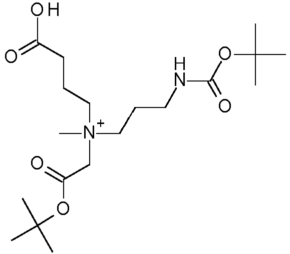

### Step 1: benzyl 4-[3-(tert-butoxycarbonylamino)propylamino]butanoate

To a stirred solution of 3-(BOC-amino)propyl bromide (5.18 g, 21.77 mmol, 1 eq) and triethylamine (6.37 mL, 45.71 mmol, 2.1 eq) in ACN (50 mL) was added benzyl 4-aminobutanoate;hydrochloride (5.0 g, 21.77 mmol, 1 eq) at 10°C , then the mixture was stirred at 30 °C for 16 h. The solution was filtered, and concentrated, and the residue was purified by RPHPLC to afford benzyl 4-[3-(tert-butoxycarbonylamino)propylamino]butanoate (1.8 g, 23.6%) as light yellow oil. MS [M+H]⁺ 351.2.

### Step 2: benzyl 4-[3-(tert-butoxycarbonylamino)propyl-methyl-amino]butanoate

To a stirred solution of benzyl 4-[3-(tert-butoxycarbonylamino)propylamino]butanoate (1.8 g, 5.14 mmol, 1 eq) and formaldehyde in water (0.83 g, 10.27 mmol, 2 eq) in methanol (20 mL) was added sodium cyanoborohydride (645.52 mg, 10.27 mmol, 2 eq) at 10 °C and stirred for 16 h. The solution was purified by RPHPLC to afford benzyl 4-[3-(tert-butoxycarbonylamino)propyl-methyl-amino]butanoate (1.4 g, 74.8%) as colorless oil. MS [M+H]⁺ 365.2.

### Step 3: rac-(4-benzyloxy-4-oxo-butyl)-[3-(tert-butoxycarbonylamino)propyl]-(2-tert-butoxy-2-oxo-ethyl)-methyl-ammonium ; bromide

To a solution of tert-butyl bromoacetate (1.24 mL, 7.68 mmol, 2 eq) in ACN (20 mL) was added benzyl 4-[3-(tert-butoxycarbonylamino)propyl-methyl-amino]butanoate (1.4 g, 3.84 mmol, 1 eq) at 10°C , and then the mixture was stirred at 50 °C for 16 h. The solution was concentrated, and the residue was purified by RPHPLC to afford rac-(4-benzyloxy-4-oxo-butyl)-[3-(tert-butoxycarbonylamino)propyl]-(2-tert-butoxy-2-oxo-ethyl)-methyl-ammonium; bromide (1.1 g, 59.7%) as colorless oil. MS [M]+ 479.3.

### Step 4: rac-3-(tert-butoxycarbonylamino)propyl-(2-tert-butoxy-2-oxo-ethyl)-(3-carboxypropyl)-methyl-ammonium; bromide

To a solution of rac-(4-benzyloxy-4-oxo-butyl)-[3-(tert-butoxycarbonylamino)propyl]-(2-tert-butoxy-2-oxo-ethyl)-methyl-ammonium;bromide (0.63 g, 1.12 mmol, 1 eq) in methanol (20 mL) was added palladium on actived carbon (0.12 g, 0.110 mmol, 0.1 eq) under N₂ at 10 °C, and then the mixture was stirred under H₂ (1460 mmHg) at 35 °C for 16 h. The solution was filtered, and concentrated to afford the title compound **Intermediate H11** (415 mg, 74.7%) as light yellow solid. MS [M]+ 389.1.

The following examples were prepared in analogy of **Intermediate H11**

| **Ex#** | **Name** | **MS ESI** | **Starting Material** |
|---|---|---|---|
| **Interme diate H18** | (1-tert-butoxycarbonylazetidin-3-yl)methyl-(2-tert-butoxy-2-oxo-ethyl)-(3-carboxypropyl)-methylammonium;2,2,2-trifluoroacetate | 401.1 [M]+ | 1-Boc-3-(bromomethyl)azetidine and benzyl 4-aminobutanoate hydrochloride and formaldehyde in water and tert-butyl bromoacetate |

### Intermediate K1

### tert-Butyl N-[3-[4-(3-aminopropyl)-2-pyridyl]propyl]carbamate;hydrochloride

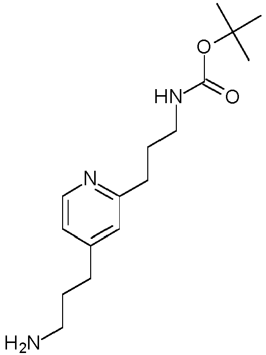

### Step 1: benzyl N-prop-2-ynylcarbamate

To a mixture of propargylamine (5.0 g, 90.78 mmol, 1 eq) and sodium hydrogen carbonate (38.13 g, 453.89 mmol, 5 eq) in EtOAc (100 mL)/water (100 mL) was added dropwise benzyl chloroformate (14.25 mL, 99.85 mmol, 1.1 eq) at 0 °C, then the mixture was stirred for at 0 °C for 1 h. The solution was poured into water (50 mL), and extracted with EtOAc (2 x 30 mL), washed with brine, dried over Na₂SO₄, concentrated in vacuo, and purified by column chromatography (50% EtOAc in PE) to afford benzyl N-prop-2-ynylcarbamate (15.4 g, 89.7%) as light yellow oil. MS [M+H]⁺ 189.9.

### Step 2: benzyl N-[3-(2-bromo-4-pyridyl)prop-2-ynyl]carbamate

A mixture of benzyl N-prop-2-ynylcarbamate (6.0 g, 31.7 mmol, 1 eq), copper(I) iodide (0.32 mL, 9.51 mmol, 0.3 eq) and tetrakis(triphenylphosphine)palladium(0) (1.83 g, 1.59 mmol, 0.05 eq) in toluene (20 mL) was treated with 2-bromo-4-iodopyridine (9.0 g, 31.7 mmol, 1 eq), tetrabutylammonium fluoride in THF (31.7 mL, 31.7 mmol, 1 eq), and triethylamine (13.26 mL, 95.11 mmol, 3 eq) under N₂, and then the mixture was stirred at 25 °C for 16 h. The reaction was directly purified by column chromatography (EtOAc 20-33% in PE) to obtain benzyl N-[3-(2-bromo-4-pyridyl)prop-2-ynyl]carbamate (9.1 g, 83.2%) as brown oil. MS [M+H]⁺ 345.0.

### Step 3: tert-butyl N-[3-[4-[3-(benzyloxycarbonylamino)prop-1-ynyl]-2-pyridyl]prop-2-ynyl]carbamate

A mixture ofN-BOC-propargylamine (4.09 g, 26.36 mmol, 1 eq), copper(I) iodide (0.16 mL, 4.78 mmol, 0.18 eq) and bis(triphenylphosphine)palladium(II) chloride (0.93 g, 1.32 mmol, 0.050 eq) in DMF (10 mL) was treated with benzyl N-[3-(2-bromo-4-pyridyl)prop-2-ynyl]carbamate (9.1 g, 26.36 mmol, 1 eq) and triethylamine (91.0 mL, 652.89 mmol, 24.77 eq) under N₂, and then the mixture was stirred at 50 °C for 16 h. The reaction was directly purified by column chromatography (EtOAc 17-50% in PE) to obtain tert-butyl N-[3-[4-[3-(benzyloxycarbonylamino)prop-1-ynyl]-2-pyridyl]prop-2-ynyl]carbamate (9.4 g, 85%) as a brown oil. [M+H]⁺ 420.3.

### Step 4: tert-butyl N-[3-[4-(3-aminopropyl)-2-pyridyl]propyl]carbamate; hydrochloride

To a mixture of tert-butyl N-[3-[4-[3-(benzyloxycarbonylamino)prop-1-ynyl]-2-pyridyl]prop-2-ynyl]carbamate (4.0 g, 9.54 mmol, 1 eq) and ammonium hydroxide (1 mL, 9.54 mmol, 1 eq) in methanol (50 mL) was added wet palladium 10% on activated carbon (400 mg, 9.54 mmol, 1.0 eq) at 25 °C. The mixture was degassed and purged with H₂ (3x). Then the mixture was stirred under hydrogen (50 psi) at 25 °C for 48 h. The reaction was filtered, and concentrated in vacuo. The residue was purified by RPHPLC, and the obtained purified residue was dissolved in 5% aq. HCl solution (100mL), and lyophilized to obtain the title compound **Intermediate K1** (2.091 g, 64.5%) as light yellow oil. [M+H]⁺ 293.9.

### Intermediate J1

### tert-Butyl 2-[1-[3-(tert-butoxycarbonylamino)propyl]piperazin-1-ium-1-yl]acetate;formate

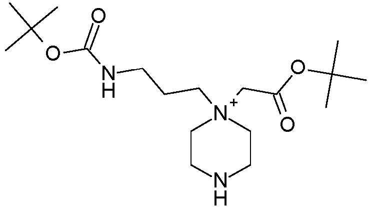

### Step 1: benzyl 4-[3-(tert-butoxycarbonylamino)propyl]piperazine-1-carboxylate

To a solution of 1-CBZ-piperazine (5.0 g, 22.7 mmol) in MeCN (100 mL) was added triethylamine (3.16 mL, 22.7 mmol) and 3-(BOC-amino)propyl bromide (5.68 g, 23.83 mmol), and then the mixture was stirred at 25 °C for 16 h. The mixture was concentrated in vacuum, and purified by column chromatography (EtOAc 1-67% in PE) to obtain benzyl 4-[3-(tert-butoxycarbonylamino)propyl]piperazine-1-carboxylate (5.2 g, 60.7%) as light brown solid. MS [M+H]⁺ 378.3.

### Step 2: benzyl 4-[3-(tert-butoxycarbonylamino)propyl]-4-(2-tert-butoxy-2-oxoethyl)piperazin-4-ium-1-carboxylate;formate

To a solution of benzyl 4-[3-(tert-butoxycarbonylamino)propyl]piperazine-1-carboxylate (5.2 g, 13.78 mmol) in MeCN (100 mL) was added triethylamine (1.92 mL, 13.78 mmol) and tert-butyl bromoacetate (5.37 g, 27.55 mmol), and then the mixture was stirred at 50 °C for 16 h. The mixture was concentrated in vacuum, and purified by RPHPLC obtain benzyl 4-[3-(tert-butoxycarbonylamino)propyl]-4-(2-tert-butoxy-2-oxo-ethyl)piperazin-4-ium-1-carboxylate;formate (4 g, 59%) as light yellow solid. MS [M+H]⁺ 492.4.

### Step 3: tert-butyl 2-[1-[3-(tert-butoxycarbonylamino)propyl]piperazin-1-ium-1-yl]acetate;formate

To a solution of benzyl 4-[3-(tert-butoxycarbonylamino)propyl]-4-(2-tert-butoxy-2-oxoethyl)piperazin-4-ium-1-carboxylate;formate (4.0 g, 8.12 mmol) in THF (40 mL) was added 10% palladium on charcoal (400 mg), and the reaction was stirred under hydrogen atmosphere at 25 °C for 16 h. The mixture was concentrated in vacuum, and purified by RPHPLC to obtain the title compound **Intermediate J1** (1.5 g, 51.5%) as white solid. MS [M]+ 358.3.

The following intermediates were prepared in analogy of **Intermediate J1**

| **Ex#** | **Name** | **MS ESI** | **Starting Material** |
|---|---|---|---|
| **Interme diate J2** | tert-butyl 3-[[1-(2-tert-butoxy-2-oxoethyl)piperazin-1-ium-1-yl]methyl]azetidine-1-carboxylate;formate | 370.6 [M]+ | 1-CBZ-piperazine and 1-BOC-3-(bromomethyl)azetidine and tert-butyl bromoacetate |

### Intermediate L1

### Bis[3-(tert-butoxycarbonylamino)propyl]-(2-tert-butoxy-2-oxo-ethyl)-(2-hydroxyethyl)ammonium formate

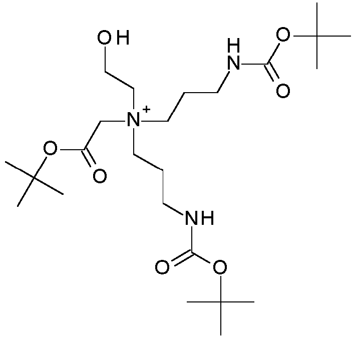

### Step 1: tert-butyl N-[3-[3-(tert-butoxycarbonylamino)propylamino]propyl]carbamate

A solution of dipropylenetriamine (23.0 g, 175.28 mmol, 1.0 eq) and triethylamine (73.29 mL, 525.83 mmol, 3.0 eq) in THF (400 mL) was stirred at 0 °C. To the mixture was added tert-butyl [(E)-[cyano(phenyl)methylene]amino] carbonate (86.33 g, 350.56 mmol, 2.0 eq) in THF (300 mL) and stirred at 0 °C for 2 h. Then, the reaction mixture was stirred at 25 °C for 16 h. The mixture was concentrated in vacuum, and the residue was purified by RP column chromatography (40-60% ACN in water(0.1%FA)) to afford the title compound tert-butyl N-[3-[3-(tert-butoxycarbonylamino)propylamino]propyl]carbamate (58.0 g, 174.99 mmol, 99.8%) as a light brown solid. MS [M]+ 332.2.

### Step 2: tert-butyl N-[3-[2-benzyloxyethyl-[3-(tert-butoxycarbonylamino)propyl]amino]propyl] carbamate

A mixture of tert-butyl N-[3-[3-(tert-butoxycarbonylamino)propylamino]propyl]carbamate (20.0 g, 60.34 mmol, 1.0 eq), benzyl 2-bromoethyl ether (14.28 g, 66.38 mmol, 1.1 eq), and triethylamine (10.93 mL, 78.44 mmol, 1.3 eq) in ACN (200 mL) was heated and stirred at 50 °C for 16 h. The mixture was concentrated under vacuum, and the residue was purified by RPHPLC (50-80% ACN in 0.1% FA in water) to afford the title compound tert-butyl N-[3-[2-benzyloxyethyl-[3-(tert-butoxycarbonylamino)propyl]amino]propyl]carbamate (5.0 g, 10.74 mmol, 16.9%) as a yellow oil. MS [M]+ 466.4.

### Step 3: 2-benzyloxyethyl-bis[3-(tert-butoxycarbonylamino)propyl]-(2-tert-butoxy-2-oxoethyl)ammonium formate

To a solution of tert-butyl N-[3-[2-benzyloxyethyl-[3-(tert-butoxycarbonylamino)propyl]amino]propyl]carbamate (900.0 mg, 1.93 mmol, 1.0 eq) in MeCN (20 mL) was added tert-butyl bromoacetate (2.26 g, 11.6 mmol, 6.0 eq) and N,N-diisopropylethylamine (1.01 mL, 5.8 mmol, 3.0 eq), then the solution was stirred at 55 °C for 16 h. The mixture was concentrated under vacuum, and the residue was purified by RPHPLC (28-48% ACN in water(0.225%FA)) to afford the title compound 2-benzyloxyethyl-bis[3-(tert-butoxycarbonylamino)propyl]-(2-tert-butoxy-2-oxo-ethyl)ammonium formate (900.0 mg, 1.44 mmol, 74.4%) as a brown solid. MS [M]+ 580.6.

### Step 4: bis[3-(tert-butoxycarbonylamino)propyl]-(2-tert-butoxy-2-oxo-ethyl)-(2-hydroxyethyl)ammonium formate

To a solution of 2-benzyloxyethyl-bis[3-(tert-butoxycarbonylamino)propyl]-(2-tert-butoxy-2-oxo-ethyl)ammonium formate (900.0 mg, 1.44 mmol, 1.0 eq) in methanol (20 mL) was added Pd(OH)2/C (180.0 mg, 2.88 mmol, 2.0 eq) and wet palladium 10% on actived carbon (90.0 mg, 2.88 mmol, 2.0 eq). The mixture was degassed and purged with H₂ for 3 times. Then, the mixture was stirred under hydrogen (50 psi) at 25 °C for 48 h. The mixture was filtered, and the filtrate was concentrated under vacuum to afford the title compound **Intermediate L1** (750.0 mg, 1.4 mmol, 97.4%) as a light brown oil, which was used without further purification in the next step. MS [M]+ 490.3.

### Intermediate H15

### trans-1-(2-tert-butoxy-2-oxo-ethyl)-1-[2-(dimethylamino)ethyl]piperidin-1-ium-4-carboxylic acid 2,2,2-trifluoroacetate

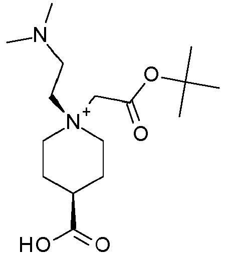

### Step 1: benzyl 1-[2-(tert-butoxycarbonylamino)ethyl]piperidine-4-carboxylate

To a solution of benzyl piperidine-4-carboxylate hydrochloride (500.0 mg, 1.96 mmol, 1.0 eq) and potassium carbonate (540.42 mg, 3.91 mmol, 2.0 eq) in DMF (10 mL) was added tert-butyl N-(2-bromoethyl)carbamate (525.77 mg, 2.35 mmol, 1.2 eq). The mixture was stirred at 20 °C for 16 h. The mixture was diluted with EA (60 mL), and then washed with brine (30 mL). The organic layer was dried over sodium sulfate, filtered, the filtrate was concentrated under vacuum to afford the title compound benzyl 1-[2-(tert-butoxycarbonylamino)ethyl]piperidine-4-carboxylate (900.0 mg, 2.48 mmol, 99.1%) as a light yellow oil, which was used without further purification. MS [M]+ 363.2.

### Step 2: benzyl 1-[2-(tert-butoxycarbonylamino)ethyl]-1-(2-tert-butoxy-2-oxoethyl)piperidin-1-ium-4-carboxylate 2,2,2-trifluoroacetate

To a solution of benzyl 1-[2-(tert-butoxycarbonylamino)ethyl]piperidine-4-carboxylate (9.23 g, 25.46 mmol, 1.0 eq), sodium iodide (381.7 mg, 2.55 mmol, 0.1 eq) and N,N-diisopropylethylamine (6.65 mL, 38.2 mmol, 1.5 eq) in DMF (90 mL) was added tert-butyl bromoacetate (5.96 g, 30.56 mmol, 1.2 eq). The mixture was stirred at 20 °C for 16 h. The mixture was concentrated under vacuum, the residue was purified twice by RPHPLC (TFA condition) to afford the title compound benzyl 1-[2-(tert-butoxycarbonylamino)ethyl]-1-(2-tert-butoxy-2-oxo-ethyl)piperidin-1-ium-4-carboxylate 2,2,2-trifluoroacetate (5.7 g, 9.65 mmol, 46.9%) as a light yellow gum. MS [M]+ 477.2.

### Step 3: cis-benzyl 1-(2-tert-butoxy-2-oxo-ethyl)-1-[2-(dimethylamino)ethyl]piperidin-1-ium-4-carboxylate 2,2,2-trifluoroacetate and trans-benzyl 1-(2-tert-butoxy-2-oxo-ethyl)-1-[2-(dimethylamino)ethyl]piperidin-1-ium-4-carboxylate 2,2,2-trifluoroacetate

To a stirred solution of benzyl 1-[2-(tert-butoxycarbonylamino)ethyl]-1-(2-tert-butoxy-2-oxoethyl)piperidin-1-ium-4-carboxylate 2,2,2-trifluoroacetate (2.0 g, 3.39 mmol, 1.0 eq) and 37% aq formaldehyde (1.65 g, 20.32 mmol, 6.0 eq) in DCM (20 mL) in ACN (10 mL) was added sodium cyanoborohydride (1276.78 mg, 20.32 mmol, 6.0 eq) at 10 °C in one portion. After stirring for 2 h at 10 °C, trifluoroacetic acid (10.0 mL, 129.8 mmol, 38.33 eq) was added into the solution at 10 °C, and stirring was continued for 14 h. The solution was diluted with ACN (100 mL), concentrated (at 10 °C), and purified by RP column chromatography (40% ACN in 0.1% FA in water) to afford a colorless oil. The obtained material was further purified by RPHPLC (water-ACN, 2% ACN, Phenomenex Luna C18 150*40mm*15µm) to afford the title compounds:
trans-benzyl 1-(2-tert-butoxy-2-oxo-ethyl)-1-[2-(dimethylamino)ethyl]piperidin-1-ium-4-carboxylate 2,2,2-trifluoroacetate (360.0 mg, 0.69 mmol, 20.5%) as a light yellow oil; MS [M]⁺ 405.3; Rt = 1.259 min;
   and
cis-benzyl 1-(2-tert-butoxy-2-oxo-ethyl)-1-[2-(dimethylamino)ethyl]piperidin-1-ium-4-carboxylate 2,2,2-trifluoroacetate (320.0 mg, 0.62 mmol, 18.2%) as a light yellow oil; MS [M]⁺ 405.3; Rt = 1.196 min.

### Step 4: trans-1-(2-tert-butoxy-2-oxo-ethyl)-1-[2-(dimethylamino)ethyl]piperidin-1-ium-4-carboxylic acid 2,2,2-trifluoroacetate

A stirred solution of trans-benzyl 1-(2-tert-butoxy-2-oxo-ethyl)-1-[2-(dimethylamino)ethyl]piperidin-1-ium-4-carboxylate 2,2,2-trifluoroacetate (0.36 g, 0.69 mmol, 1.0 eq) in methanol (20 mL) was evacuated and backfilled with N₂ (3×). Palladium on activated carbon (0.15 g, 0.14 mmol, 0.2 eq) was added to the solution in one portion, then the black mixture was evacuated and backfilled with H₂ (3x). The mixture was stirred under H₂ (760 mmHg) at 10 °C for 16 h. The mixture was filtered through diomiate and concentrated to afford the title compound **Intermediate H15** (245.0 mg, 0.57 mmol, 82.4%) as a white oil, which was used without further purification. MS [M]+ 315.1.

### Intermediate H16

### cis-1-(2-tert-butoxy-2-oxo-ethyl)-1-[2-(dimethylamino)ethyl]piperidin-1-ium-4-carboxylic acid;2,2,2-trifluoroacetate

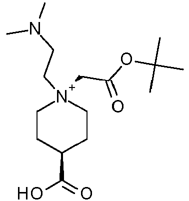

The title compound was prepared in analogy to **Intermediate H15,** using the cis isomer isolated in Step 3 to afford **Intermediate H16** (220.0 mg, 0.51 mmol, 83.2%) as a colorless oil. MS [M]+ 315.1.

### Intermediate B1

### 2-Chloro-4-[[1-methyl-5-[1-(5-nitro-2-pyridyl)-3-(trifluoromethyl)pyrazol-4-yl]imidazole-2-carbonyl]amino]benzoic acid methyl ester

A mixture of 2-chloro-4-[[1-methyl-5-[3-(trifluoromethyl)-1H-pyrazol-4-yl]imidazole-2-carbonyl]amino]benzoic acid methyl ester (600 mg, 1.4 mmol, 1 eq) in acetonitrile (12 mL) was treated with 2-fluoro-5-nitro-pyridine (298.95 mg, 2.1 mmol, 1.5 eq) and potassium carbonate (581.55 mg, 4.21 mmol, 3 eq), and the reaction mixture was stirred at RT for 1 h. The reaction mixture was diluted with DCM, filtered, and evaporated to afford the crude title compound **Intermediate B1** (850 mg, 85%) as orange solid, which was directly used in the next step. MS [M+H]⁺ 550.1.

The following intermediates were prepared in analogy of **Intermediate B1**

| **Ex#** | **Name** | **MS ESI** | **Starting Material** |
|---|---|---|---|
| **Interme diate D1** | 4-[2-chloro-4-[[1-methyl-5-[1-(5-nitro-2-pyridyl)-3-(trifluoromethyl)pyrazol-4-yl]imidazole-2-carbonyl]amino]benzoyl]piperazine-1-carboxylic acid tert-butyl ester | 702.4 [M-H]- | 4-[2-chloro-4-[[1-methyl-5-[3-(trifluoromethyl)-1H-pyrazol-4-yl]imidazole-2-carbonyl]amino]benzoyl]piperazine-1-carboxylic acid tert-butyl ester and 2-fluoro-5-nitro-pyridine |
| **Interme diate D3** | 4-[2-chloro-4-[[5-[1-(2-fluoro-4-nitrophenyl)-3-(trifluoromethyl)pyrazol-4-yl]-1-methyl-imidazole-2-carbonyl]amino]benzoyl]piperazine-1-carboxylic acid tert-butyl ester | 765.4 [M+HCOO]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 1,2-difluoro-4-nitrobenzene in DMF |
| **Interme diate D5** | 4-[4-[[5-[1-(5-bromo-2-pyridyl)-3-(trifluoromethyl)pyrazol-4-yl]-1-methyl-imidazole-2-carbonyl]amino]-2-chloro-benzoyl]piperazine-1-carboxylic acid tert-butyl ester | 681.1, [M+H-Buten]+ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 5-bromo-2-fluoro-pyridine in DMF |
| **Interme diate D17** | 4-[4-[[5-[1-(6-amino-5-nitro-2-pyridyl)-3-(trifluoromethyl)pyrazol-4-yl]-1-methyl-imidazole-2-carbonyl]amino]-2-chlorobenzoyl]piperazine-1-carboxylic acid tert-butyl ester | 719.5 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 2-amino-6-chloro-3-nitropyridine in DMF |
| **Interme diate D19** | 4-[2-chloro-4-[[5-[1-(3-fluoro-5-nitro-2-pyridyl)-3-(trifluoromethyl)pyrazol-4-yl]-1-methyl-imidazole-2-carbonyl]amino]benzoyl]piperazine-1-carboxylic acid tert-butyl ester | 722.3 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 2,3-difluoro-5-nitropyridine |
| **Interme diate D25** | 4-[2-chloro-4-[[5-[1-(5-cyano-2-pyridyl)-3-(trifluoromethyl)pyrazol-4-yl]-1-methyl-imidazole-2-carbonyl]amino]benzoyl]piperazine-1-carboxylic acid tert-butyl ester | 682.5 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 6-fluoronicotinonitrile in DMF |
| **Interme diate D26** | 4-[4-[[5-[1-(1,3-benzothiazol-2-yl)-3-(trifluoromethyl)pyrazol-4-yl]-1-methyl-imidazole-2-carbonyl]amino]-2-chloro-benzoyl]piperazine-1-carboxylic acid tert-butyl ester | 713.4 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 2-chloro-1,3-benzothiazole in DMF |
| **Interme diate D28** | 4-[2-chloro-4-[[5-[1-(5-formyl-2-pyridyl)-3-(trifluoromethyl)pyrazol-4-yl]-1-methyl-imidazole-2-carbonyl]amino]benzoyl]piperazine-1-carboxylic acid tert-butyl ester | 685.3 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 6-fluoronicotinaldehyde in DMF |
| **Interme diate D30** | 4-[2-chloro-4-[[1-methyl-5-[1-(4-nitroisothiazol-5-yl)-3-(trifluoromethyl)pyrazol-4-yl]imidazole-2-carbonyl]amino]benzoyl]piperazine-1-carboxylic acid tert-butyl ester | 708.3 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 5-bromo-4-nitroisothiazole in DMF |
| **Interme diate D58** | tert-butyl 4-[4-[[5-[1-(2-fluoro-4-nitrophenyl)-3-(trifluoromethyl)pyrazol-4-yl]-1-methyl-imidazole-2-carbonyl]amino]-2-methylbenzoyl]piperazine-1-carboxylate | 701.3 [M+H]⁺ | Intermediate AA1 and 3,4-difluoronitrobenzene in DMF |
| **Interme diate G2** | cis-tert-butyl 3-[[1-(2-tert-butoxy-2-oxo-ethyl)-4-[4-[2-fluoro-4-[[5-[1-(2-fluoro-4-nitro-phenyl)-3-(trifluoromethyl)pyrazol-4-yl]-1-methyl-imidazole-2-carbonyl]amino]benzoyl]piperazine-1-carbonyl]piperidin-1-ium-1-yl]methyl]azetidine-1-carboxylate;formate | 999.3 [M]+ | Intermediate AAAA1 and 3,4-difluoronitrobenzene in DMF |
| **Interme diate G3** | cis-tert-butyl 3-[[1-(2-tert-butoxy-2-oxo-ethyl)-4-[4-[2-chloro-4-[[5-[3-(difluoromethyl)-1-(2-fluoro-4-nitrophenyl)pyrazol-4-yl]-1-methylimidazole-2-carbonyl]amino]benzoyl]piperazine-1-carbonyl]piperidin-1-ium-1-yl]methyl]azetidine-1-carboxylate;formate | 997.4 [M]+ | Intermediate AAAA2 and 3,4-difluoronitrobenzene in DMF |
| **Interme diate B12** | 2-chloro-4-[[5-[1-(2-fluoro-4-nitrophenyl)-3-(trifluoromethyl)pyrazol-4-yl]-1-methyl-imidazole-2-carbonyl]amino]benzoic acid methyl ester | 567.15 [M+H]⁺ | 2-chloro-4-[[1-methyl-5-[3-(trifluoromethyl)-1H-pyrazol-4-yl]imidazole-2-carbonyl]amino]benzoi c acid methyl ester (CAS 2489205-78-7) and 1,2-difluoro-4-nitro-benzene in DMF |
| **Interme diate D59** | 4-[4-[[5-[1-(2-fluoro-4-nitro-phenyl)-3-(trifluoromethyl)pyrazol-4-yl]-1-methyl-imidazole-2-carbonyl]amino]-2-methyl-benzoyl]piperazine-1-carboxylic acid tert-butyl ester | 701.3 [M+H]⁺ | Intermediate AA1 and 1,2-difluoro-4-nitrobenzene in DMF |
| **Interme diate B13** | methyl 2-chloro-4-[[5-[1-(5-iodo-2-pyridyl)-3-(trifluoromethyl)pyrazol-4-yl]-1-methyl-imidazole-2-carbonyl]amino]benzoate | 630.9 [M+H]⁺ | 2-chloro-4-[[1-methyl-5-[3-(trifluoromethyl)-1H-pyrazol-4-yl]imidazole-2-carbonyl]amino]benzoi c acid methyl ester (CAS 2489205-78-7) and 2-fluoro-5-iodopyridine (CAS 171197-80-1) in DMF |

### Intermediate B2

### 4-[[5-[1-[5-[2-(tert-Butoxycarbonylamino)ethyl]-2-pyridyl]-3-(trifluoromethyl)pyrazol-4-yl]-1-methyl-imidazole-2-carbonyl]amino]-2-chloro-benzoic acid methyl ester

A microwave vial was charged with a mixture of 2-chloro-4-[[1-methyl-5-[3-(trifluoromethyl)-1H-pyrazol-4-yl]imidazole-2-carbonyl]amino]benzoic acid methyl ester (62 mg, 0.145 mmol, 1 eq), N-[2-(6-bromo-3-pyridyl)ethyl]carbamic acid tert-butyl ester (85 mg, 0.282 mmol, 1.95 eq), cuprous iodide (8.28 mg, 0.043 mmol, 0.3 eq), and K₂CO₃ (42.07 mg, 0.304 mmol, 2.1 eq). The vial was evacuated and backfilled with Ar (3x). DMF, extra dry (0.827 mL) and trans-N,N-dimethylcyclohexane-1,2-diamine (12.37 mg, 13.71 µL, 0.087 mmol, 0.6 eq) were addded, and the vial was sealed and stirred in the microwave at 135 °C for 30 min. The mixture was cooled to RT, and diluted with 5% EDTA (1 mL) and half-sat. aq. NaHCO₃ (30 mL), and extracted with EtOAc (3x12 mL). The combined organics were washed with brine (10 mL), dried (Na₂SO₄), filtered, and evaporated. Purification by column chromatography (0-40% EtOAc/EtOH 3:1 in heptane) afforded the title compound **Intermediate B2** (48 mg, 41.9%) as white solid. MS [M+H]⁺ 648.4.

The following intermediates were prepared in analogy of **Intermediate B2**

| **Ex#** | **Name** | **MS ESI** | **Starting Material** |
|---|---|---|---|
| **Interme diate B3** | 4-[[5-[1-[5-[2-(tert-butoxycarbonylamino)ethoxy]-2-pyridyl]-3-(trifluoromethyl)pyrazol-4-yl]-1-methyl-imidazole-2-carbonyl]amino]-2-chloro-benzoic acid methyl ester | 662.5 [M-H]⁻ | 2-chloro-4-[[1-methyl-5-[3-(trifluoromethyl)-1H-pyrazol-4-yl]imidazole-2-carbonyl]amino]benzoi c acid methyl ester and N-[2-[(6-bromo-3-pyridyl)oxy]ethyl]carba mic acid tert-butyl ester |
| **Interme diate B4** | 4-[6-[4-[2-[(4-carbomethoxy-3-chlorophenyl)carbamoyl]-3-methyl-imidazol-4-yl]-3-(trifluoromethyl)pyrazol-1-yl]-3-pyridyl]piperazine-1-carboxylic acid tert-butyl ester | 689.5 [M+H]⁺ | 2-chloro-4-[[1-methyl-5-[3-(trifluoromethyl)-1H-pyrazol-4-yl]imidazole-2-carbonyl]amino]benzoi c acid methyl ester and 4-(6-bromo-3-pyridyl)piperazine-1-carboxylic acid tert-butyl ester |
| **Interme diate D2** | 4-[2-chloro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carboxylic acid tert-butyl ester | 698.2 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 5-bromo-1H-pyrrolo[3,2-b]pyridine in 1,4-dioxane |
| **Interme diate D4** | 4-[2-chloro-4-[[1-methyl-5-[1-(1H-pyrrolo[3,2-c]pyridin-6-yl)-3-(trifluoromethyl)pyrazol-4-yl]imidazole-2-carbonyl]amino]benzoyl]piperazine-1-carboxylic acid tert-butyl ester | 696.5 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 6-bromo-1H-pyrrolo[3,2-c]pyridine |
| **Interme diate D6** | 4-[2-chloro-4-[[5-[1-(5-methoxy-2-pyridyl)-3-(trifluoromethyl)pyrazol-4-yl]-1-methyl-imidazole-2-carbonyl]amino]benzoyl]piperazine-1-carboxylic acid tert-butyl ester | 687.4 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 2-bromo-5-methoxy-pyridine in 1,4-dioxane |
| **Interme diate D7** | 4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carboxylic acid tert-butyl ester | 726.6 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A44 |
| **Interme diate D8** | 4-[2-chloro-4-[[5-[1-(4-methoxyphenyl)-3-(trifluoromethyl)pyrazol-4-yl]-1-methyl-imidazole-2-carbonyl]amino]benzoyl]piperazine-1-carboxylic acid tert-butyl ester | 686.3 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 1-bromo-4-methoxybenzene in 1,4-dioxane |
| **Interme diate D10** | 4-[2-chloro-4-[[1-methyl-5-[1-(1H-pyrazolo[4,3-b]pyridin-5-yl)-3-(trifluoromethyl)pyrazol-4-yl]imidazole-2-carbonyl]amino]benzoyl]piperazine-1-carboxylic acid tert-butyl ester | 699.6 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A1 |
| **Interme diate D11** | 4-[2-chloro-4-[[1-methyl-5-[1-thiazol-2-yl-3-(trifluoromethyl)pyrazol-4-yl]imidazole-2-carbonyl]amino]benzoyl]piperazine-1-carboxylic acid tert-butyl ester | 663.3 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 2-bromothiazole |
| **Interme diate D13** | 4-[2-chloro-4-[[5-[1-[4-(dimethylamino)phenyl]-3-(trifluoromethyl)pyrazol-4-yl]-1-methyl-imidazole-2-carbonyl]amino]benzoyl]piperazine-1-carboxylic acid tert-butyl ester | 745.4 [M+HCOO]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 4-bromo-N,N-dimethylaniline in 1,4-dioxane |
| **Interme diate D15** | 4-[2-chloro-4-[[1-methyl-5-[1-[4-(methylamino)-2-pyridyl]-3-(trifluoromethyl)pyrazol-4-yl]imidazole-2-carbonyl]amino]benzoyl]piperazine-1-carboxylic acid tert-butyl ester | 316.69 [M-isobutylene+ 2H]2+ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 2-bromo-N-methyl-pyridin-4-amine in 1,4-dioxane |
| **Interme diate D16** | 4-[2-chloro-4-[[1-methyl-5-[1-(5-p-anisyloxy-2-pyridyl)-3-(trifluoromethyl)pyrazol-4-yl]imidazole-2-carbonyl]amino]benzoyl]piperazine-1-carboxylic acid tert-butyl ester | 795.6 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 2-bromo-5-[(4-methoxyphenyl)methox y]pyridine in 1,4-dioxane |
| **Interme diate D18** | 4-[4-[[5-[1-[6-(tert-butoxycarbonylamino)-2-pyridyl]-3-(trifluoromethyl)pyrazol-4-yl]-1-methyl-imidazole-2-carbonyl]amino]-2-chloro-benzoyl]piperazine-1-carboxylic acid tert-butyl ester | 774.6 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and tert-butyl N-(6-bromo-2-pyridyl)carbamate in 1,4-dioxane |
| **Interme diate D20** | 4-[2-chloro-4-[[5-[1-(2,3-dihydro-1H-pyrrolo[3,2-b]pyridin-5-yl)-3-(trifluoromethyl)pyrazol-4-yl]-1-methyl-imidazole-2-carbonyl]amino]benzoyl]piperazine-1-carboxylic acid tert-butyl ester | 698.5 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 5-bromo-2,3-dihydro-1H-pyrrolo[3,2-b]pyridine in DMF |
| **Interme diate D22** | 4-[2-chloro-4-[[5-[1-furo[3,2-b]pyridin-5-yl-3-(trifluoromethyl)pyrazol-4-yl]-1-methyl-imidazole-2-carbonyl]amino]benzoyl]piperazine-1-carboxylic acid tert-butyl ester | 697.6 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 5-chlorofuro[3,2-b]pyridine in 1,4-dioxane |
| **Interme diate D23** | 4-[4-[[5-[1-[4-(tert-butoxycarbonylamino)-2-pyridyl]-3-(trifluoromethyl)pyrazol-4-yl]-1-methyl-imidazole-2-carbonyl]amino]-2-chloro-benzoyl]piperazine-1-carboxylic acid tert-butyl ester | 774.4 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and tert-butyl N-(2-chloro-4-pyridyl)carbamate in 1,4-dioxane |
| **Interme diate D24** | 4-[4-[[5-[1-[2-(tert-butoxycarbonylamino)-4-pyridyl]-3-(trifluoromethyl)pyrazol-4-yl]-1-methyl-imidazole-2-carbonyl]amino]-2-chloro-benzoyl]piperazine-1-carboxylic acid tert-butyl ester | 772.6 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 1,1-dimethylethyl N-(4-bromo-2-pyridinyl)carbamate in 1,4-dioxane |
| **Interme diate D29** | 4-[2-chloro-4-[[1-methyl-5-[1-[2-(methylamino)-4-pyridyl]-3-(trifluoromethyl)pyrazol-4-yl]imidazole-2-carbonyl]amino]benzoyl]piperazine-1-carboxylic acid tert-butyl ester | 688.5 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 4-bromo-N-methyl-2-pyridinamine in 1,4-dioxane |
| **Interme diate D62** | tert-butyl 4-[4-[[5-[1-[2-[3-[tert-butyl(dimethyl)silyl]oxypropyl]-1H-pyrrolo[3,2-b]pyridin-5-yl]-3-(trifluoromethyl)pyrazol-4-yl]-1-methyl-imidazole-2-carbonyl]amino]-2-chloro-benzoyl]piperazine-1-carboxylate | 870.5 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A3 in t-amyl alcohol |
| **Interme diate D63** | tert-butyl 4-[2-chloro-4-[[5-[1-[2-(3-methoxy-3-oxo-propyl)-1-(2-trimethylsilylethoxymethyl)pyrrolo[3, 2-b]pyridin-5-yl]-3-(trifluoromethyl)pyrazol-4-yl]-1-methyl-imidazole-2-carbonyl]amino]benzoyl]piperazine-1-carboxylate | 914.4 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A4 in toluene |
| **Interme diate D64** | tert-butyl 4-[2-chloro-4-[[5-[1-(2-fluoro-4-morpholino-phenyl)-3-(trifluoromethyl)pyrazol-4-yl]-1-methyl-imidazole-2-carbonyl]amino]benzoyl]piperazine-1-carboxylate | 761.1 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A5 in DMSO |
| **Interme diate D65** | tert-butyl 4-[4-[[5-[1-[2-[(1S)-1-hydroxyethyl]-1-(2-trimethylsilylethoxymethyl)pyrrolo[3, 2-b]pyridin-5-yl]-3-(trifluoromethyl)pyrazol-4-yl]-1-methyl-imidazole-2-carbonyl]amino]-2-methyl-benzoyl]piperazine-1-carboxylate | 852.5 [M+H]⁺ | Intermediate AA1 and Intermediate A48 in toluene |
| **Interme diate D66** | tert-butyl 4-[2-chloro-4-[[5-[1-[2-(2,2-dimethyl-1,3-dioxan-5-yl)-1-(2-trimethylsilylethoxymethyl)pyrrolo[3, 2-b]pyridin-5-yl]-3-(trifluoromethyl)pyrazol-4-yl]-1-methyl-imidazole-2-carbonyl]amino]benzoyl]piperazine-1-carboxylate | 942.9 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A7 in toluene |
| **Interme diate D67** | tert-butyl 4-[2-chloro-4-[[1-methyl-5-[1-[1-methyl-2-(morpholine-4-carbonyl)pyrrolo[3,2-b]pyridin-5-yl]-3-(trifluoromethyl)pyrazol-4-yl]imidazole-2-carbonyl]amino]benzoyl]piperazine-1-carboxylate | 825.6 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A8 in DMF |
| **Interme diate D68** | tert-butyl 4-[2-chloro-4-[[5-[1-[2-(2-hydroxyethylcarbamoyl)-1-methyl-pyrrolo[3,2-b]pyridin-5-yl]-3-(trifluoromethyl)pyrazol-4-yl]-1-methyl-imidazole-2-carbonyl]amino]benzoyl]piperazine-1-carboxylate | 799.3 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A9 in DMF |
| **Interme diate D69** | tert-butyl 4-[2-chloro-4-[[5-[1-[2-(1,2-dihydroxyethyl)-1-(2-trimethylsilylethoxymethyl)pyrrolo[3, 2-b]pyridin-5-yl]-3-(trifluoromethyl)pyrazol-4-yl]-1-methyl-imidazole-2-carbonyl]amino]benzoyl]piperazine-1-carboxylate | 888.4 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A49 in toluene |
| **Interme diate D60** | 4-[4-[[5-[1-(4-benzoxy-2-fluorophenyl)-3-(trifluoromethyl)pyrazol-4-yl]-1-methyl-imidazole-2-carbonyl]amino]-2-methylbenzoyl]piperazine-1-carboxylic acid tert-butyl ester | 762.5 [M+H]⁺ | Intermediate AA1 and 4-benzyloxy-1-bromo-2-fluorobenzene (CAS 185346-79-6) in t-amyl alcohol |
| **Interme diate D71** | 6-[4-[2-[[4-(4-tert-butoxycarbonylpiperazine-1-carbonyl)-3-chlorophenyl]carbamoyl]-3-methyl-imidazol-4-yl]-3-(trifluoromethyl)pyrazol-1-yl]-1H-pyrrolo[3,2-c]pyridine-2-carboxylic acid methyl ester | 756.5 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A11 in t-amyl alcohol |
| **Interme diate D70** | tert-butyl 4-[2-chloro-4-[[5-[1-[4-[2-(difluoromethoxy)ethylamino]-2-fluoro-phenyl]-3-(trifluoromethyl)pyrazol-4-yl]-1-methyl-imidazole-2-carbonyl]amino]benzoyl]piperazine-1-carboxylate | 785.4 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A12 in DMSO |
| **Interme diate D61** | 4-[4-[[5-[1-[2-(2-hydroxyethyl)-1H-pyrrolo[3,2-b]pyridin-5-yl]-3-(trifluoromethyl)pyrazol-4-yl]-1-methyl-imidazole-2-carbonyl]amino]-2-methyl-benzoyl]piperazine-1-carboxylic acid tert-butyl ester | 722.5 [M+H]⁺ | Intermediate AA1 and Intermediate A13 in t-amyl alcohol |
| **Interme diate D72** | tert-butyl 4-[4-[[5-[1-[2-(2-azidoethyl)-1-(2-trimethylsilylethoxymethyl)pyrrolo[3, 2-b]pyridin-5-yl]-3-(trifluoromethyl)pyrazol-4-yl]-1-methyl-imidazole-2-carbonyl]amino]-2-chloro-benzoyl]piperazine-1-carboxylate | 897.3 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A14 in toluene |
| **Interme diate D76** | 4-[2-methyl-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carboxylic acid tert-butyl ester | 678.39 [M+H]⁺ | Intermediate AA1 and 5-bromo-1H-pyrrolo[3,2-b]pyridine (CAS 1000341-51-4) in 1,4-dioxane |
| **Interme diate D77** | 4-[2-fluoro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carboxylic acid tert-butyl ester | 682.37 [M+H]⁺ | Intermediate AA2 and 5-bromo-1H-pyrrolo[3,2-b]pyridine (CAS 1000341-51-4) in t-amyl alcohol |
| **Interme diate B15** | 2-chloro-4-[[1-methyl-5-[1-(2-methylol-1H-pyrrolo[3,2-c]pyridin-6-yl)-3-(trifluoromethyl)pyrazol-4-yl]imidazole-2-carbonyl]amino]benzoic acid methyl ester | 574.22 [M+H]⁺ | 2-chloro-4-[[1-methyl-5-[3-(trifluoromethyl)-1H-pyrazol-4-yl]imidazole-2-carbonyl]amino]benzoi c acid methyl ester (CAS 2489205-78-7) and Intermediate A44 in t-amyl alcohol |
| **Interme diate D73** | tert-butyl 4-[2-chloro-4-[[5-[1-[2-fluoro-4-[[(2S)-2,3-dihydroxypropyl]amino]phenyl]-3-(trifluoromethyl)pyrazol-4-yl]-1-methyl-imidazole-2-carbonyl]amino]benzoyl]piperazine-1-carboxylate | 765.2 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A15 in DMSO |
| **Interme diate D74** | tert-butyl 4-[2-chloro-4-[[5-[1-[1-(2-ethoxy-1,1-dimethyl-2-oxoethyl)pyrrolo[3,2-b]pyridin-5-yl]-3-(trifluoromethyl)pyrazol-4-yl]-1-methyl-imidazole-2-carbonyl]amino]benzoyl]piperazine-1-carboxylate | 812.5 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A16 in toluene |
| **Interme diate D78** | 4-[2-chloro-4-[[1-methyl-5-[1-[2-(morpholine-4-carbonyl)-1H-pyrrolo[3,2-c]pyridin-6-yl]-3-(trifluoromethyl)pyrazol-4-yl]imidazole-2-carbonyl]amino]benzoyl]piperazine-1-carboxylic acid tert-butyl ester | 809.33 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A17 in t-amyl alcohol |
| **Interme diate B14** | methyl 4-[[5-[1-[2-[2-(tert-butoxycarbonylamino)ethyl]-1-(dimethylsulfamoyl)pyrrolo[3,2-c]pyridin-6-yl]-3-(trifluoromethyl)pyrazol-4-yl]-1-methyl-imidazole-2-carbonyl]amino]-2-chloro-benzoate | 794.4 [M+H]⁺ | 2-chloro-4-[[1-methyl-5-[3-(trifluoromethyl)-1H-pyrazol-4-yl]imidazole-2-carbonyl]amino]benzoi c acid methyl ester (CAS 2489205-78-7) and Intermediate A18 in DMSO |
| **Interme diate D79** | 4-[2-chloro-4-[[1-methyl-5-[1-[1-(methylcarbamoyl)pyrrolo[3,2-b]pyridin-5-yl]-3-(trifluoromethyl)pyrazol-4-yl]imidazole-2-carbonyl]amino]benzoyl]piperazine-1-carboxylic acid tert-butyl ester | 755.30 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A19 in t-amyl alcohol |
| **Interme diate D75** | 4-[2-chloro-4-[[5-[1-[3-(2-hydroxyethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carboxylic acid tert-butyl ester | 785.5 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A20 in t-amyl alcohol |
| **Interme diate B16** | 4-[6-[4-[2-[(4-carbomethoxy-3-chlorophenyl)carbamoyl]-3-methyl-imidazol-4-yl]-3-(trifluoromethyl)pyrazol-1-yl]-1H-pyrrolo[3,2-c]pyridine-3-carbonyl]piperazine-1-carboxylic acid tert-butyl ester | 756.36 [M+H]⁺ | 2-chloro-4-[[1-methyl-5-[3-(trifluoromethyl)-1H-pyrazol-4-yl]imidazole-2-carbonyl]amino]benzoi c acid methyl ester (CAS 2489205-78-7) and Intermediate A21 in t-amyl alcohol |
| **Interme diate D80** | 4-[2-chloro-4-[[1-methyl-5-[1-[3-(4-methylpiperazine-1-carbonyl)-1H-pyrrolo[3,2-c]pyridin-6-yl]-3-(trifluoromethyl)pyrazol-4-yl]imidazole-2-carbonyl]amino]benzoyl]piperazine-1-carboxylic acid tert-butyl ester | 824.76 [M+H]⁺ | 4-[2-chloro-4-[[1-methyl-5-[3-(trifluoromethyl)-1H-pyrazol-4-yl]imidazole-2-carbonyl]amino]benzoy l]piperazine-1- carboxylic acid tert-butyl ester and Intermediate A22 in t-amyl alcohol |
| **Interme diate D81** | 4-[2-fluoro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carboxylic acid tert-butyl ester | 710.3 [M-H]- | Intermediate AA2 and Intermediate A44 in t-amyl alcohol |
| **Interme diate D82** | tert-butyl 4-[2-chloro-4-[[5-[1-[2-[(1R)-1-hydroxyethyl]-1-(2-trimethylsilylethoxymethyl)pyrrolo[3, 2-b]pyridin-5-yl]-3-(trifluoromethyl)pyrazol-4-yl]-1-methyl-imidazole-2-carbonyl]amino]benzoyl]piperazine-1-carboxylate | 872.3 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A6 in toluene |
| **Interme diate D83** | 4-[2-methyl-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carboxylic acid tert-butyl ester | 708.3 [M+H]⁺ | Intermediate AA1 and Intermediate A44 in t-amyl alcohol |
| **Interme diate D84** | tert-butyl 4-[4-[[5-[1-[2-[2-[tert-butyl(dimethyl)silyl]oxyethyl]-1-(dimethylsulfamoyl)pyrrolo[3,2-c]pyridin-6-yl]-3-(trifluoromethyl)pyrazol-4-yl]-1-methyl-imidazole-2-carbonyl]amino]-2-chloro-benzoyl]piperazine-1-carboxylate | 963.3 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A24 in toluene |
| **Interme diate D85** | 4-[2-chloro-4-[[5-[1-[3-[(3 S)-3-hydroxypyrrolidine-1-carbonyl]-1H-pyrrolo[3,2-c]pyridin-6-yl]-3-(trifluoromethyl)pyrazol-4-yl]-1-methyl-imidazole-2-carbonyl]amino]benzoyl]piperazine-1-carboxylic acid tert-butyl ester | 809.28 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A25 in t-amyl alcohol |
| **Interme diate D86** | 4-[2-chloro-4-[[1-methyl-5-[1-[3-(methylcarbamoyl)-1H-pyrrolo[3,2-c]pyridin-6-yl]-3-(trifluoromethyl)pyrazol-4-yl]imidazole-2-carbonyl]amino]benzoyl]piperazine-1-carboxylic acid tert-butyl ester | 755.90 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A26 in t-amyl alcohol |
| **Interme diate D87** | 4-[4-[[5-[1-(3-carbamoyl-1H-pyrrolo[3,2-c]pyridin-6-yl)-3-(trifluoromethyl)pyrazol-4-yl]-1-methyl-imidazole-2-carbonyl]amino]-2-chloro-benzoyl]piperazine-1-carboxylic acid tert-butyl ester | 741.69 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A27 in t-amyl alcohol |
| **Interme diate D88** | tert-butyl 4-[4-[[5-[1-[2-[(2S)-3-[tert-butyl(dimethyl)silyl]oxy-2-hydroxypropyl]-1-(dimethylsulfamoyl)pyrrolo[3,2-b]pyridin-5-yl]-3-(trifluoromethyl)pyrazol-4-yl]-1-methyl-imidazole-2-carbonyl]amino]-2-chloro-benzoyl]piperazine-1-carboxylate | 993.5 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A28 in DMSO |
| **Interme diate D89** | tert-butyl 4-[4-[[5-[1-[2-[2-[tert-butyl(dimethyl)silyl]oxyethyl]-1-(dimethylsulfamoyl)pyrrolo[3,2-b]pyridin-5-yl]-3-(trifluoromethyl)pyrazol-4-yl]-1-methyl-imidazole-2-carbonyl]amino]-2-chloro-benzoyl]piperazine-1-carboxylate | 963.8 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A30 in DMSO |
| **Interme diate D90** | 4-[4-[[5-[1-(4-benzoxy-2-fluorophenyl)-3-(trifluoromethyl)pyrazol-4-yl]-1-methyl-imidazole-2-carbonyl]amino]-2-chlorobenzoyl]piperazine-1-carboxylic acid tert-butyl ester | 782.4 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A47 in t-amyl alcohol |
| **Interme diate D91** | tert-butyl 4-[2-chloro-4-[[5-[1-[2-fluoro-4-[(2-hydroxy-2-methylpropyl)amino]phenyl]-3-(trifluoromethyl)pyrazol-4-yl]-1-methyl-imidazole-2-carbonyl]amino]benzoyl]piperazine-1-carboxylate | 763.4 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A31 in DMSO |
| **Interme diate D92** | tert-butyl 4-[2-chloro-4-[[1-methyl-5-[3-(trifluoromethyl)-1-[5-(2-trimethylsilylethoxymethyl)pyrrolo[3, 2-d]pyrimidin-2-yl]pyrazol-4-yl]imidazole-2-carbonyl]amino]benzoyl]piperazine-1-carboxylate | 829.5 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A32 in toluene |
| **Interme diate B17** | methyl 4-[[5-[1-[5-[2-[bis[2-(benzyloxycarbonylamino)ethyl]amin o]ethylamino]-2-pyridyl]-3-(trifluoromethyl)pyrazol-4-yl]-1-methyl-imidazole-2-carbonyl]amino]-2-chloro-benzoate | 917.3 [M+H]⁺ | 2-chloro-4-[[1-methyl-5-[3-(trifluoromethyl)-1H-pyrazol-4-yl]imidazole-2-carbonyl]amino]benzoi c acid methyl ester CAS 2489205-78-7) and Intermediate A33 in DMSO |
| **Interme diate D93** | tert-butyl 5-[4-[2-[[4-(4-tert-butoxycarbonylpiperazine-1-carbonyl)-3-chlorophenyl]carbamoyl]-3-methyl-imidazol-4-yl]-3-(trifluoromethyl)pyrazol-1-yl]-2-(2-ethoxy-2-oxo-ethyl)pyrrolo[3,2-b]pyridine-1-carboxylate | 884.0 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A34 in toluene |
| **Interme diate D94** | 4-[2-chloro-4-[[1-methyl-5-[1-(1H-pyrrolo[3,2-c]pyridin-6-yl)-3-(trifluoromethyl)pyrazol-4-yl]imidazole-2-carbonyl]amino]benzoyl]piperazine-1-carboxylic acid tert-butyl ester | 698.36 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 6-bromo-1H-pyrrolo[3,2-c]pyridine (CAS 1000342-71-1) in 1,4-dioxane |
| **Interme diate D95** | 5-[4-[2-[[4-(4-tert-butoxycarbonylpiperazine-1-carbonyl)-3-chlorophenyl]carbamoyl]-3-methyl-imidazol-4-yl]-3-(trifluoromethyl)pyrazol-1-yl]-1H-pyrrolo[3,2-b]pyridine-2-carboxylic acid ethyl ester | 768.3 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 5-bromo-1H-pyrrolo[3,2-b]pyridine-2-carboxylic acid ethyl ester (CAS 1255098-82-8) in 1,4-dioxane |
| **Interme diate D96** | 4-[2-chloro-4-[[5-[1-[3-(dimethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carboxylic acid tert-butyl ester | 767.4 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A46 in t-amyl alcohol |
| **Interme diate D97** | 4-[2-chloro-4-[[1-methyl-5-[1-(1H-pyrazolo[4,3-c]pyridin-6-yl)-3-(trifluoromethyl)pyrazol-4-yl]imidazole-2-carbonyl]amino]benzoyl]piperazine-1-carboxylic acid tert-butyl ester | 699.31 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A35 in t-amyl alcohol |
| **Interme diate D98** | 4-[4-[[5-[1-[1-[2-[tert-butyl(dimethyl)silyl]oxyethyl]pyrrolo[ 3,2-c]pyridin-6-yl]-3-(trifluoromethyl)pyrazol-4-yl]-1-methyl-imidazole-2-carbonyl]amino]-2-chloro-benzoyl]piperazine-1-carboxylic acid tert-butyl ester | 856.48 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A36 in t-amyl alcohol |
| **Interme diate D99** | 4-[2-chloro-4-[[1-methyl-5-[1-[3-(morpholine-4-carbonyl)-1H-pyrrolo[3,2-c]pyridin-6-yl]-3-(trifluoromethyl)pyrazol-4-yl]imidazole-2-carbonyl]amino]benzoyl]piperazine-1-carboxylic acid tert-butyl ester | 811.52 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A37 in t-amyl alcohol |
| **Interme diate D100** | 4-[2-chloro-4-[[5-[1-[3-(4,4-difluoropiperidine-1-carbonyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carboxylic acid tert-butyl ester | 845.35 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A38 in t-amyl alcohol |
| **Interme diate D101** | 4-[2-chloro-4-[[1-methyl-5-[1-(2-methylol-1H-pyrrolo[3,2-c]pyridin-6-yl)-3-(trifluoromethyl)pyrazol-4-yl]imidazole-2-carbonyl]amino]benzoyl]piperazine-1-carboxylic acid tert-butyl ester | 728.3 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A39 in t-amyl alcohol |
| **Interme diate B18** | tert-butyl 2-[(tert-butoxycarbonylamino)methyl]-5-[4-[2-[(3-chloro-4-methoxycarbonyl-phenyl)carbamoyl]-3-methyl-imidazol-4-yl]-3-(trifluoromethyl)pyrazol-1-yl]pyrrolo[3,2-b]pyridine-1-carboxylate | 773.7 [M+H]⁺ | 2-chloro-4-[[1-methyl-5-[3-(trifluoromethyl)-1H-pyrazol-4-yl]imidazole-2-carbonyl]amino]benzoi c acid methyl ester (CAS 2489205-78-7) and Intermediate A40 in toluene |
| **Interme diate B19** | tert-butyl 2-[[bis(tert-butoxycarbonyl)amino]methyl]-6-[4-[2-[(3-chloro-4-methoxycarbonyl-phenyl)carbamoyl]-3-methyl-imidazol-4-yl]-3-(trifluoromethyl)pyrazol-1-yl]pyrrolo[3,2-c]pyridine-1-carboxylate | 873.3 [M+H]⁺ | 2-chloro-4-[[1-methyl-5-[3-(trifluoromethyl)-1H-pyrazol-4-yl]imidazole-2-carbonyl]amino]benzoi c acid methyl ester (CAS 2489205-78-7) and Intermediate A41 in toluene |
| **Interme diate D102** | 5-[4-[2-[[4-(4-tert-butoxycarbonylpiperazine-1-carbonyl)-3-chlorophenyl]carbamoyl]-3-methyl-imidazol-4-yl]-3-(trifluoromethyl)pyrazol-1-yl]-1H-pyrrolo[2,3-c]pyridine-2-carboxylic acid ethyl ester | 768.4 [M-H]⁻ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A42 in t-amyl alcohol |
| **Interme diate D103** | 4-[4-[[5-[1-[6-(tert-butoxycarbonylamino)-2-pyridyl]-3-(trifluoromethyl)pyrazol-4-yl]-1-methyl-imidazole-2-carbonyl]amino]-2-chloro-benzoyl]piperazine-1-carboxylic acid tert-butyl ester | 774.90 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and tert-butyl N-(6-bromo-2-pyridyl)carbamate (CAS 344331-90-4) in 1,4-dioxane |
| **Interme diate D104** | 4-[2-chloro-4-[[5-[1-[3-(2-hydroxyethyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carboxylic acid tert-butyl ester | 740.33 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and Intermediate A43 in t-amyl alcohol |
| **Interme diate B20** | methyl 4-[[5-[1-[5-[bis[2-(tert-butoxycarbonylamino)ethyl]amino]-2-pyridyl]-3-(trifluoromethyl)pyrazol-4-yl]-1-methyl-imidazole-2-carbonyl]amino]-2-chloro-benzoate | 806.5 [M+H]⁺ | Intermediate B13 and tert-butyl N-[2-[2-(tert-butoxycarbonylamino)e thylamino]ethyl]carbam ate (CAS 117499-16-8) in DMSO |
| **Interme diate D105** | 4-[2-chloro-4-[[1-methyl-5-[1-(1H-pyrrolo[2,3-c]pyridin-5-yl)-3-(trifluoromethyl)pyrazol-4-yl]imidazole-2-carbonyl]amino]benzoyl]piperazine-1-carboxylic acid tert-butyl ester | 696.4 [M-H]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and 5-bromo-1H-pyrrolo[2,3-c]pyridine (CAS 1215387-58-8) in DMF |
| **Interme diate B21** | methyl 4-[[5-[1-[5-[(3S)-3-(tert-butoxycarbonylamino)pyrrolidin-1-yl]-2-pyridyl]-3-(trifluoromethyl)pyrazol-4-yl]-1-methyl-imidazole-2-carbonyl]amino]-2-chloro-benzoate | 689.3 [M+H]⁺ | Intermediate B13 and (S)-3-(BOC-amino)pyrrolidine (CAS 122536-76-9) in DMSO |

### Intermediate A23

### 4-[4-[[5-[1-[2-(2-amino-2-keto-ethyl)pyrazolo[4,3-c]pyridin-6-yl]-3-(trifluoromethyl)pyrazol-4-yl]-1-methyl-imidazole-2-carbonyl]amino]-2-chlorobenzoyl]piperazine-1-carboxylic acid tert-butyl ester

6-bromo-1H-pyrazolo[4,3-c]pyridine (161 mg, 0.813 mmol, 1.25 eq) was dissolved in tetrahydrofuran, extra dry (2 mL). NaH (48.78 mg, 1.22 mmol, 1.875 eq) was added at 0 °C. After stirring for 30 min, 2-bromoacetamide (242.28 mg, 1.76 mmol, 2.7 eq) and KI (53.99 mg, 325.22 µmol, 0.5 eq) were added, and the mixture was stirred at 50 °C overnight. NaHCO₃ (5 mL) and water (30 mL) were added. The mixture was extracted with ethyl acetate (3 x 30 mL). The combined organic layers were dried (Na₂SO₄), filtered, and concentrated in vacuo. The residue was purified by RP column chromatography (0-40% ACN in water) to give 2-(6-bromopyrazolo[4,3-c]pyridin-2-yl)acetamide (188 mg) and its regioisomer as a mixture. In analogy to **Intermediate B2,** the obtained mixture of isomers was subjected to an Ullmann coupling with 4-[2-chloro-4-[[1-methyl-5-[3-(trifluoromethyl)-1H-pyrazol-4-yl]imidazole-2-carbonyl]amino]benzoyl]piperazine-1-carboxylic acid tert-butyl ester in t-amyl alcohol to afford the title compound **Intermediate A23** (55 mg, 9.7%) as an off-white solid. MS [M+H]⁺ 756.24.

### Intermediate B5

### 4-[[5-[1-[6-(tert-Butoxycarbonylamino)-3-pyridyl]-3-(trifluoromethyl)pyrazol-4-yl]-1-methyl-imidazole-2-carbonyl]amino]-2-chloro-benzoic acid methyl ester

A mixture of 2-chloro-4-[[1-methyl-5-[3-(trifluoromethyl)-1H-pyrazol-4-yl]imidazole-2-carbonyl]amino]benzoic acid methyl ester (75 mg, 0.168 mmol, 1 eq) and [6-(tert-butoxycarbonylamino)-3-pyridyl]boronic acid (72.12 mg, 0.303 mmol, 1.8 eq) in N,N-dimethylformamide, extra dry (1 mL) was treated with cupric acetate (64.2 mg, 0.353 mmol, 2.1 eq) and pyridine (26.6 mg, 27.2 µL, 0.337 mmol, 2 eq), and the mixture was stirred at 50 °C for 24 h under air. Additional 6-(tert-butoxycarbonylamino)pyridin-3-ylboronic acid (40.07 mg, 0.168 mmol, 1 eq) was added, and the stirring continued at 50°C for 24 h. The reaction mixture was cooled to RT, suspended in aq. NaHCO₃, and extracted with EtOAc. The combined organics were washed with 5% aq. LiCl solution, brine, dried (Na₂SO₄), filtered, and evaporated. Purification by column chromatography (0-100% EtOAc in heptane) afforded the title compound **Intermediate B5** (13.5 mg, 10.5%) as light yellow solid. MS [M+H]⁺ 620.2.

The following intermediates were prepared in analogy of **Intermediate B5**

| **Ex#** | **Name** | **MS ESI** | **Starting Material** |
|---|---|---|---|
| **Interme diate B6** | 2-chloro-4-[[5-[1-(6-fluoro-3-pyridyl)-3-(trifluoromethyl)pyrazol-4-yl]-1-methyl-imidazole-2-carbonyl]amino]benzoic acid methyl ester | 523.3 [M+H]⁺ | 2-chloro-4-[[1-methyl-5-[3-(trifluoromethyl)-1H-pyrazol-4-yl]imidazole-2-carbonyl]amino]benzoi c acid methyl ester and (6-fluoro-3-pyridyl)boronic acid |
| **Interme diate D14** | 4-[2-chloro-4-[[5-[1-(6-fluoro-3-pyridyl)-3-(trifluoromethyl)pyrazol-4-yl]-1-methyl-imidazole-2-carbonyl]amino]benzoyl]piperazine-1-carboxylic acid tert-butyl ester | 677.2 [M+H]⁺ | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and (6-fluoro-3-pyridyl)boronic acid |
| **Interme diate D27** | 4-[2-chloro-4-[[5-[1-(6-methoxy-3-pyridyl)-3-(trifluoromethyl)pyrazol-4-yl]-1-methyl-imidazole-2-carbonyl]amino]benzoyl]piperazine-1-carboxylic acid tert-butyl ester | 733.6 [M+HCOO]- | 4-[2-chloro-4-[[1-methyl-5-[3-(trifluoromethyl)-1H-pyrazol-4-yl]imidazole-2-carbonyl]amino]benzoy l]piperazine-1-carboxylic acid tert-butyl ester and (6-methoxy-3-pyridyl)boronic acid |

### Intermediate D21

### tert-Butyl 4-[2-chloro-4-[[1-methyl-5-[3-(trifluoromethyl)-1-[1-(2-trimethylsilylethoxymethyl)indazol-3-yl]pyrazol-4-yl]imidazole-2-carbonyl]amino]benzoyl]piperazine-1-carboxylate

A mixture of 4-[2-chloro-4-[[1-methyl-5-[3-(trifluoromethyl)-1H-pyrazol-4-yl]imidazole-2-carbonyl]amino]benzoyl]piperazine-1-carboxylic acid tert-butyl ester (800.0 mg, 1.37 mmol, 1 eq), **Intermediate A2** (514.51 mg, 1.37 mmol, 1 eq), copper(II) oxide (10.93 mg, 0.140 mmol, 0.1 eq), and iron(III)-acetylacetonate (145.65 mg, 0.410 mmol, 0.300 eq) in DMF (20 mL) was treated with cesium carbonate (0.9 g, 2.75 mmol, 2 eq), and stirred at 115 °C for 48 h. The mixture was poured into water (30 mL), and extracted with EtOAc (2 x 20 mL). The combined organics were washed with brine (50 mL), and concentrated. Purification by RPHPLC afforded the title compound **Intermediate D21** (200 mg, 0.240 mmol, 18%) as yellow solid. MS [M+H]⁺ 828.4.

### Intermediate B7

### 4-[[5-[1-(5-Amino-2-pyridyl)-3-(trifluoromethyl)pyrazol-4-yl]-1-methyl-imidazole-2-carbonyl]amino]-2-chloro-benzoic acid methyl ester

A suspension of **Intermediate B1** (850 mg, 1.19 mmol, 1 eq) in ethanol (25.5 mL) / water (8.5 mL) to was treated with ammonium chloride (3.18 g, 59.52 mmol, 50 eq) and zinc (1.56 g, 23.81 mmol, 20 eq), and the mixture was stirred at RT O/N The reaction mixture was filtered over Celite, and concentrated. The residue was purified by column chromatography (0-100% DCM/MeOH 9:1 in DCM) yielded the title compound **Intermediate B7** (294 mg, 36%) as orange solid. MS [M+H]⁺ 520.1.

The following intermediates were prepared in analogy of **Intermediate B7**

| **Ex#** | **Name** | **MS ESI** | **Starting Material** |
|---|---|---|---|
| **Interme diate D31** | 4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carboxylic acid tert-butyl ester | 672.4 [M-H]- | Intermediate D1 iron powder, NH₄Cl |
| **Interme diate D34** | 4-[4-[[5-[1-(4-amino-2-fluorophenyl)-3-(trifluoromethyl)pyrazol-4-yl]-1-methyl-imidazole-2-carbonyl]amino]-2-chlorobenzoyl]piperazine-1-carboxylic acid tert-butyl ester | 735.4 [M+HCOO]- | Intermediate D3 iron powder, NH₄Cl |
| **Interme diate D51** | 4-[2-chloro-4-[[5-[1-(5,6-diamino-2-pyridyl)-3-(trifluoromethyl)pyrazol-4-yl]-1-methyl-imidazole-2-carbonyl]amino]benzoyl]piperazine-1-carboxylic acid tert-butyl ester | 733.6 [M+HCOO]- | Intermediate D17 zinc dust, NH₄Cl in DMF/H₂O |
| **Interme diate G13** | cis-tert-butyl 3-[[4-[4-[4-[[5-[1-(4-amino-2-fluoro-phenyl)-3-(difluoromethyl)pyrazol-4-yl]-1-methyl-imidazole-2-carbonyl]amino]-2-chloro-benzoyl]piperazine-1-carbonyl]-1-(2-tert-butoxy-2-oxoethyl)piperidin-1-ium-1-yl]methyl]azetidine-1-carboxylate;formate | 967.6 [M]+ | Intermediate G3 iron powder, NH₄Cl |
| **Interme diate B22** | 4-[[5-[1-(4-amino-2-fluoro-phenyl)-3-(trifluoromethyl)pyrazol-4-yl]-1-methyl-imidazole-2-carbonyl]amino]-2-chloro-benzoic acid methyl ester | 537.2 [M+H]⁺ | Intermediate B12 |
| | | | 1) zinc dust, NH₄Cl |
| | | | 2) iron powder, NH₄Cl |
| **Interme diate D106** | 4-[4-[[5-[1-(4-amino-2-fluorophenyl)-3-(trifluoromethyl)pyrazol-4-yl]-1-methyl-imidazole-2-carbonyl]amino]-2-methylbenzoyl]piperazine-1-carboxylic acid tert-butyl ester | 671.4 [M+H]⁺ | Intermediate D59 iron powder, NH₄Cl |

### Intermediate D107

### tert-Butyl 4-[2-fluoro-4-[[5-[1-(2-fluoro-4-methoxy-phenyl)-3-(trifluoromethyl)pyrazol-4-yl]-1-methyl-imidazole-2-carbonyl]amino]benzoyl]piperazine-1-carboxylate

A mixture of **Intermediate BB1** (188.21 mg, 0.58 mmol, 1.2 eq) and **Intermediate CC1** (200.0 mg, 0.49 mmol, 1.0 eq) in THF (4 mL) was cooled to -40 °C. Sodium bis(trimethylsilyl)amide (0.63 mL, 0.63 mmol, 1.3 eq) was added dropwise at -40 °C, and the mixture was stirred at that temperature for 1 h. The mixture was quenched with sat. NH₄Cl (30 mL), and then extracted with EtOAc (3 x 30 mL). The combined organic layers were washed with brine (30 mL), dried over sodium sulfate, filtered, and concentrated under vacuum. The residue was purified by RP column chromatography (13-75% acetonitrile in water, formic acid) to give the title compound **Intermediate D107** (320 mg, 0.46 mmol, 96%) as a light yellow solid. MS [M+H]⁺ 690.4.

### Intermediate D108

### tert-Butyl 4-[4-[[5-[1-(4-amino-2-fluoro-phenyl)-3-(trifluoromethyl)pyrazol-4-yl]-1-methylimidazole-2-carbonyl]amino]-2-methyl-benzoyl]piperazine-1-carboxylate

To a solution of Intermediate D58 (640.0 mg, 0.91 mmol, 1.0 eq) in MeOH (15 mL) was added palladium on charcoal (320 mg, 3.01 mmol, 3.29 eq). The reaction mixture was stirred under a H₂ atmosphere at 25 °C for 0.5 h, and filtered through a celite pad (washings with MeOH, 3 x 8 mL). The combined filtrate was concentrated under vacuum, and the residue was purified by RPHPLC (water(TFA) / ACN 0-100%) to give the title compound **Intermediate D108** (500 mg, 82%) as a white solid. MS [M+H]⁺ 671.3.

The following intermediates were prepared in analogy of **Intermediate D108**

| **Ex#** | **Name** | **MS ESI** | **Starting Material** |
|---|---|---|---|
| **Interme diate G4** | cis-tert-butyl 3-[[4-[4-[4-[[5-[1-(4-amino-2-fluoro-phenyl)-3-(trifluoromethyl)pyrazol-4-yl]-1-methyl-imidazole-2-carbonyl]amino]-2-fluoro-benzoyl]piperazine-1-carbonyl]-1-(2-tert-butoxy-2-oxoethyl)piperidin-1-ium-1-yl]methyl]azetidine-1-carboxylate | 969.5 [M]+ | Intermediate G2 |
| **Interme diate D109** | 4-[4-[[5-[1-(2-fluoro-4-hydroxyphenyl)-3-(trifluoromethyl)pyrazol-4-yl]-1-methyl-imidazole-2-carbonyl]amino]-2-methyl-benzoyl]piperazine-1-carboxylic acid tert-butyl ester | 672.5 [M]+ | Intermediate D60 |

### Intermediate B8

### 4-[[5-[1-[6-[2-(tert-Butoxycarbonylamino)ethylamino]-3-pyridyl]-3-(trifluoromethyl)pyrazol-4-yl]-1-methyl-imidazole-2-carbonyl]amino]-2-chloro-benzoic acid methyl ester

A mixture of **Intermediate B6** (66 mg, 0.120 mmol, 1 eq) in N,N-dimethylformamide (1 mL) was treated with potassium carbonate (24.86 mg, 0.180 mmol, 1.5 eq), and N-(2-aminoethyl)carbamic acid tert-butyl ester (28.82 mg, 0.180 mmol, 1.5 eq), and the reaction mixture was stirred at RT for 1 h, and subsequently at 100°C O/N. Additional N-(2-aminoethyl)carbamic acid tert-butyl ester (38.43 mg, 0.240 mmol, 2 eq) was added, and stirring was continued at 100°C for 24 h. The reaction mixture was cooled to RT, and diluted with EtOAc, and filtered. The filtrate was washed with 5% LiCl solution, brine, dried (Na₂SO₄), filtered, and evaporated. Purification by column chromatography (0-80% EtOAc in heptane) yielded the title compound **Intermediate B8** (41 mg, 43%) as off-white solid. MS [M+H]⁺ 663.2.

The following intermediates were prepared in analogy of **Intermediate B8**

| **Ex#** | **Name** | **MS ESI** | **Starting Material** |
|---|---|---|---|
| **Interme diate D48** | 4-[2-chloro-4-[[5-[1-[6-(dimethylamino)-3-pyridyl]-3-(trifluoromethyl)pyrazol-4-yl]-1-methyl-imidazole-2-carbonyl]amino]benzoyl]piperazine-1-carboxylic acid tert-butyl ester | 702.4 [M+H]⁺ | Intermediate D14 and 2 M dimethylamine in THF |
| **Interme diate D53** | 4-[2-chloro-4-[[1-methyl-5-[1-[6-(methylamino)-3-pyridyl]-3-(trifluoromethyl)pyrazol-4-yl]imidazole-2-carbonyl]amino]benzoyl]piperazine-1-carboxylic acid tert-butyl ester | 686.7 [M-H]- | Intermediate D14 and 2 M monomethylamine in THF |

### Intermediate D32

### 4-[4-[[5-[1-(5-Amino-2-pyridyl)-3-(trifluoromethyl)pyrazol-4-yl]-1-methyl-imidazole-2-carbonyl]amino]-2-chloro-benzoyl]piperazine-1-carboxylic acid 9H-fluoren-9-ylmethyl ester

A mixture of **Intermediate E1** (1 g, 1.56 mmol, 1 eq) in dichloromethane (12 mL) was treated with 9H-fluoren-9-ylmethyl chloroformate (422.75 mg, 1.63 mmol, 1.05 eq), stirred for 5 min at RT, and subsequently treated with a solution of DIPEA (804.59 mg, 1.09 mL, 6.23 mmol, 4 eq) in dichloromethane (3 mL). The mixture was stirred at RT for 1 h. The reaction mixture was transferred into half-sat. NaHCO₃ and extracted with DCM. The combined organics were dried (Na₂SO₄), filtered, and evaporated. Purification by column chromatography (0-80% EtOAc in DCM) yielded the title compound **Intermediate D32** (0.63 g, 51%) as light yellow foam. MS [M+H]⁺ 796.2.

### Intermediate B9

### 4-[[5-[1-[5-[2-[tert-Butoxycarbonyl(methyl)amino]ethylamino]-2-pyridyl]-3-(trifluoromethyl)pyrazol-4-yl]-1-methyl-imidazole-2-carbonyl]amino]-2-chloro-benzoic acid methyl ester

A mixture of **Intermediate B7** (30.15 mg, 0.058 mmol, 1 eq) in dichloromethane (0.6 mL) was treated with N-(2-ketoethyl)-N-methyl-carbamic acid tert-butyl ester (0.064 mmol, 1.1 eq) and acetic acid (3.48 mg, 3.32 µL), followed by addition of sodium triacetoxyborohydride (24.59 mg). The reaction mixtures was stirred at RT O/N. Additional aldehyde (1 eq), acetic acid (1 eq), and molecular sieves were added to the reaction mixture, and stirring was continued at RT to 40 °C for another 72 h. The reaction mixture was diluted with dichloromethane. The organic layer was washed with water, brine, dried (Na₂SO₄), filtered, and evaporated to afford the crude title compound **Intermediate B9** (57 mg, quant.) as a brown viscous oil, which was directly used in the next step without any further purification. MS [M+H]⁺ 677.4.

The following intermediates were prepared in analogy of **Intermediate B9**

| **Ex#** | **Name** | **MS ESI** | **Starting Material** |
|---|---|---|---|
| **Interme diate B10** | 4-[[[6-[4-[2-[(4-carbomethoxy-3-chloro-phenyl)carbamoyl]-3-methyl-imidazol-4-yl]-3-(trifluoromethyl)pyrazol-1-yl]-3-pyridyl]amino]methyl]piperidine-1-carboxylic acid tert-butyl ester | 717.4 [M+H]⁺ | Intermediate B7 and 4-formylpiperidine-1-carboxylic acid tert-butyl ester |
| **Interme diate B11** | 2-chloro-4-[[5-[1-[5-[(2,2-difluorocyclopropyl)methylamino]-2-pyridyl]-3-(trifluoromethyl)pyrazol-4-yl]-1-methyl-imidazole-2-carbonyl]amino]benzoic acid methyl ester | 610.1 [M+H]⁺ | Intermediate B7 and 2,2-difluorocyclopropaneca rbaldehyde |
| **Interme diate D33** | 4-[4-[[5-[1-[5-[2-(tert-butoxycarbonylamino)ethylamino]-2-pyridyl]-3-(trifluoromethyl)pyrazol-4-yl]-1-methyl-imidazole-2-carbonyl]amino]-2-chlorobenzoyl]piperazine-1-carboxylic acid 9H-fluoren-9-ylmethyl ester | 939.3 [M+H]⁺ | Intermediate D32 and N-(2-ketoethyl)carbamic acid tert-butyl ester |
| **Interme diate D36** | 4-[4-[[5-[1-[5-[3-(tert-butoxycarbonylamino)propylamino]-2-pyridyl]-3-(trifluoromethyl)pyrazol-4-yl]-1-methyl-imidazole-2-carbonyl]amino]-2-chlorobenzoyl]piperazine-1-carboxylic acid 9H-fluoren-9-ylmethyl ester | 953.3 [M+H]⁺ | Intermediate D32 and N-(3-ketopropyl)carbamic acid tert-butyl ester |
| **Interme diate D37** | 4-[2-chloro-4-[[5-[1-[5-[[(2S)-2-(9H-fluoren-9-ylmethoxycarbonylamino)propyl]amin o]-2-pyridyl]-3-(trifluoromethyl)pyrazol-4-yl]-1-methyl-imidazole-2-carbonyl]amino]benzoyl]piperazine-1-carboxylic acid tert-butyl ester | 997.4 [M+HCOO]⁻ | Intermediate D31 and 9H-fluoren-9-ylmethyl (1S)-1-methyl-2-oxoethylcarbamate |
| **Interme diate D39** | 4-[2-chloro-4-[[1-methyl-5-[1-[5-(2-pyridylmethylamino)-2-pyridyl]-3-(trifluoromethyl)pyrazol-4-yl]imidazole-2-carbonyl]amino]benzoyl]piperazine-1-carboxylic acid 9H-fluoren-9-ylmethyl ester | 888.8 [M+H]⁺ | Intermediate D32 and picolinaldehyde |
| **Interme diate D40** | 4-[4-[[5-[1-[5-[[1-(tert-butoxycarbonylamino)cyclopentyl]met hylamino]-2-pyridyl]-3-(trifluoromethyl)pyrazol-4-yl]-1-methyl-imidazole-2-carbonyl]amino]-2-chloro-benzoyl]piperazine-1-carboxylic acid 9H-fluoren-9-ylmethyl ester | 1037.8 [M+HCOO]⁻ | Intermediate D32 and N-(1-formylcyclopentyl)carb amic acid tert-butyl ester |
| **Interme diate D43** | 4-[4-[[5-[1-[5-[[(2S)-1-tert-butoxycarbonylpyrrolidin-2-yl]methylamino]-2-pyridyl]-3-(trifluoromethyl)pyrazol-4-yl]-1-methyl-imidazole-2-carbonyl]amino]-2-chloro-benzoyl]piperazine-1-carboxylic acid 9H-fluoren-9-ylmethyl ester | 1023.7 [M+HCOO]- | Intermediate D32 and (2S)-2-formylpyrrolidine-1-carboxylic acid tert-butyl ester |
| **Interme diate D56** | (3S)-3-[[[6-[4-[2-[[3-chloro-4-[4-(9H-fluoren-9-ylmethoxycarbonyl)piperazine-1-carbonyl]phenyl]carbamoyl]-3-methyl-imidazol-4-yl]-3-(trifluoromethyl)pyrazol-1-yl]-3-pyridyl]lamino]methyl]morpholine-4-carboxylic acid tert-butyl ester | 1039.7 [M+HCOO]- | Intermediate D32 and (3R)-3-formylmorpholine-4-carboxylic acid tert-butyl ester |
| **Interme diate D49** | 4-[4-[[5-[1-[5-[6-(tert-butoxycarbonylamino)hexylamino]-2-pyridyl]-3-(trifluoromethyl)pyrazol-4-yl]-1-methyl-imidazole-2-carbonyl]amino]-2-chlorobenzoyl]piperazine-1-carboxylic acid 9H-fluoren-9-ylmethyl ester | 995.9 [M+H]⁺ | Intermediate D32 and N-(6-ketohexyl)carbamic acid tert-butyl ester |
| **Interme diate D57** | 4-[4-[[5-[1-[5-[[(2S,4S)-1-tert-butoxycarbonyl-4-fluoro-pyrrolidin-2-yl]methylamino]-2-pyridyl]-3-(trifluoromethyl)pyrazol-4-yl]-1-methyl-imidazole-2-carbonyl]amino]-2-chloro-benzoyl]piperazine-1-carboxylic acid 9H-fluoren-9-ylmethyl ester | 1041.7 [M+HCOO]⁻ | Intermediate D32 and (2S,4S)-4-fluoro-2-formyl-pyrrolidine-1-carboxylic acid tert-butyl ester |
| **Interme diate D55** | 4-[2-chloro-4-[[5-[1-[5-[(dimethylamino)methyl]-2-pyridyl]-3-(trifluoromethyl)pyrazol-4-yl]-1-methyl-imidazole-2-carbonyl]amino]benzoyl]piperazine-1-carboxylic acid tert-butyl ester | 716.4 [M+H]⁺ | Intermediate D28 and 2 M dimethylamine in THF |
| **Interme diate D110** | tert-butyl 4-[4-[[5-[1-[2-fluoro-4-(methylamino)phenyl]-3-(trifluoromethyl)pyrazol-4-yl]-1-methyl-imidazole-2-carbonyl]amino]-2-methyl-benzoyl]piperazine-1-carboxylate | 685.3 [M+H]⁺ | Intermediate D108 and paraformaldehyde |
| **Interme diate D111** | tert-butyl 4-[4-[[5-[1-[2-fluoro-4-(2-methoxyethylamino)phenyl]-3-(trifluoromethyl)pyrazol-4-yl]-1-methyl-imidazole-2-carbonyl]amino]-2-methyl-benzoyl]piperazine-1-carboxylate | 729.4 [M+H]⁺ | Intermediate D108 and methoxyacetaldehyde |
| **Interme diate D112** | 4-[2-chloro-4-[[5-[1-[2-fluoro-4-(methylamino)phenyl]-3-(trifluoromethyl)pyrazol-4-yl]-1-methyl-imidazole-2-carbonyl]amino]benzoyl]piperazine-1-carboxylic acid tert-butyl ester | 749.3 [M+HCOO]⁻ | Intermediate D34 and paraformaldehyde |
| **Interme diate D113** | 4-[4-[[5-[1-[4-(2-benzoxyethylamino)-2-fluoro-phenyl]-3-(trifluoromethyl)pyrazol-4-yl]-1-methyl-imidazole-2-carbonyl]amino]-2-chloro-benzoyl]piperazine-1-carboxylic acid tert-butyl ester | 825.4 [M+H]⁺ | Intermediate D34 and 2-benzoxyacetaldehyde |
| **Interme diate D114** | 4-[2-chloro-4-[[5-[1-[2-fluoro-4-(2-methoxyethylamino)phenyl]-3-(trifluoromethyl)pyrazol-4-yl]-1-methyl-imidazole-2-carbonyl]amino]benzoyl]piperazine-1-carboxylic acid tert-butyl ester | 793.3 [M+HCOO]- | Intermediate D34 and 2-methoxyacetaldehyde in DCM |
| **Interme diate D115** | 4-[2-chloro-4-[[5-[1-[4-[3-(9H-fluoren-9-ylmethoxycarbonylamino)propylamin o]-2-fluoro-phenyl]-3-(trifluoromethyl)pyrazol-4-yl]-1-methyl-imidazole-2-carbonyl]amino]benzoyl]piperazine-1-carboxylic acid tert-butyl ester | 1014.3 [M+HCOO]- | Intermediate D34 and 9H-fluoren-9-ylmethyl N-(3-oxopropyl)carbamate |

### Intermediate C1

### 4-[[5-[1-[5-[2-(tert-Butoxycarbonylamino)ethyl]-2-pyridyl]-3-(trifluoromethyl)pyrazol-4-yl]-1-methyl-imidazole-2-carbonyl]amino]-2-chloro-benzoic acid

A mixture of **Intermediate B2** (48 mg, 0.061 mmol, 1 eq) in tetrahydrofuran (1.5 mL) / methanol (0.500 mL) / water (0.500 mL) was treated with LiOH (14.55 mg, 0.607 mmol, 10 eq), and the mixture was stirred at RT for 7 h. All volatiles were evaporated. The residue was suspended in 0.5 M HCl (30 mL), and extracted with EtOAc (3 x 12 mL). The combined organics were washed with brine (12 mL), dried (Na₂SO₄), filtered, and evaporated to afford crude title compound **Intermediate C1** (48 mg, quant.) as white solid, which was directly used in the next step without any further purification. MS [M-H]⁻ 632.5.

The following intermediates were prepared in analogy of **Intermediate C1**

| **Ex#** | **Name** | **MS ESI** | **Starting Material** |
|---|---|---|---|
| **Interme diate C2** | 4-[[5-[1-[5-[2-(tert-butoxycarbonylamino)ethoxy]-2-pyridyl]-3-(trifluoromethyl)pyrazol-4-yl]-1-methyl-imidazole-2-carbonyl]amino]-2-chloro-benzoic acid | 648.6 [M-H]- | Intermediate B3 |
| **Interme diate C3** | 4-[[5-[1-[5-(4-tert-butoxycarbonylpiperazino)-2-pyridyl]-3-(trifluoromethyl)pyrazol-4-yl]-1-methyl-imidazole-2-carbonyl]amino]-2-chloro-benzoic acid | 673.6 [M-H]- | Intermediate B4 |
| **Interme diate C5** | 4-[[5-[1-[6-[2-(tert-butoxycarbonylamino)ethylamino]-3-pyridyl]-3-(trifluoromethyl)pyrazol-4-yl]-1-methyl-imidazole-2-carbonyl]amino]-2-chloro-benzoic acid | 649.2 [M+H]⁺ | Intermediate B8 |
| **Interme diate C6** | 4-[[5-[1-[5-[2-[tert-butoxycarbonyl(methyl)amino]ethyla mino]-2-pyridyl]-3-(trifluoromethyl)pyrazol-4-yl]-1-methyl-imidazole-2-carbonyl]amino]-2-chloro-benzoic acid | 663.2 [M+H]⁺ | Intermediate B9 |
| **Interme diate C7** | 4-[[5-[1-[5-[(1-tert-butoxycarbonyl-4-piperidyl)methylamino]-2-pyridyl]-3-(trifluoromethyl)pyrazol-4-yl]-1-methyl-imidazole-2-carbonyl]amino]-2-chloro-benzoic acid | 703.2 [M+H]⁺ | Intermediate B10 |
| **Interme diate C8** | 2-chloro-4-[[5-[1-[5-[(2,2-difluorocyclopropyl)methylamino]-2-pyridyl]-3-(trifluoromethyl)pyrazol-4-yl]-1-methyl-imidazole-2-carbonyl]amino]benzoic acid | 596.1 [M+H]⁺ | Intermediate B11 |
| **Interme diate C9** | 4-[[5-[1-[6-(tert-butoxycarbonylamino)-3-pyridyl]-3-(trifluoromethyl)pyrazol-4-yl]-1-methyl-imidazole-2-carbonyl]amino]-2-chloro-benzoic acid | 606.1 [M+H]⁺ | Intermediate B5 |
| **Interme diate C10** | 4-[[5-[1-(5-amino-2-pyridyl)-3-(trifluoromethyl)pyrazol-4-yl]-1-methyl-imidazole-2-carbonyl]amino]-2-chloro-benzoic acid | 504.1 [M-H]- | Intermediate B7 |
| **Interme diate C11** | 2-chloro-4-[[1-methyl-5-[1-(2-methylol-1H-pyrrolo[3,2-c]pyridin-6-yl)-3-(trifluoromethyl)pyrazol-4-yl]imidazole-2-carbonyl]amino]benzoic acid | 560.28 [M+H]⁺ | Intermediate B15 |
| **Interme diate C12** | 4-[[5-[1-(4-amino-2-fluoro-phenyl)-3-(trifluoromethyl)pyrazol-4-yl]-1-methyl-imidazole-2-carbonyl]amino]-2-chloro-benzoic acid | 521.14 [M-H]⁻ | Intermediate B22 |
| **Interme diate D116** | 2-[5-[4-[2-[[4-(4-tert-butoxycarbonylpiperazine-1-carbonyl)-3-chlorophenyl]carbamoyl]-3-methyl-imidazol-4-yl]-3-(trifluoromethyl)pyrazol-1-yl]pyrrolo[3,2-b]pyridin-1-yl]-2-methyl-propanoic acid | 784.1 [M+H]⁺ | Intermediate D74 |
| **Interme diate C13** | 4-[[5-[1-[2-[2-(tert-butoxycarbonylamino)ethyl]-1-(dimethylsulfamoyl)pyrrolo[3,2-c]pyridin-6-yl]-3-(trifluoromethyl)pyrazol-4-yl]-1-methyl-imidazole-2-carbonyl]amino]-2-chloro-benzoic acid | 780.2 [M+H]⁺ | Intermediate B14 |
| **Interme diate C14** | 4-[[5-[1-[5-[2-[bis[2-(benzyloxycarbonylamino)ethyl]amin o]ethylamino]-2-pyridyl]-3-(trifluoromethyl)pyrazol-4-yl]-1-methyl-imidazole-2-carbonyl]amino]-2-chloro-benzoic acid;dihydrochloride | 903.1 [M+H]⁺ | Intermediate B17 |
| **Interme diate D117** | 2-[5-[4-[2-[[4-(4-tert-butoxycarbonylpiperazine-1-carbonyl)-3-chlorophenyl]carbamoyl]-3-methyl-imidazol-4-yl]-3-(trifluoromethyl)pyrazol-1-yl]-1H-pyrrolo[3,2-b]pyridin-2-yl]acetic acid | 756.3 [M+H]⁺ | Intermediate D93 |
| **Interme diate G5** | [4-[4-[4-[[5-[1-(4-amino-2-fluorophenyl)-3-(trifluoromethyl)pyrazol-4-yl]-1-methyl-imidazole-2-carbonyl]amino]-2-chlorobenzoyl]piperazin-1-yl]-4-oxo-butyl]-bis[3-(tert-butoxycarbonylamino)propyl]-(3-carboxypropyl)ammonium;2,2,2-trifluoroacetate | 1077.6 [M]⁺ | Intermediate G7 |
| **Interme diate C15** | 4-[[5-[1-[2-[(tert-butoxycarbonylamino)methyl]-1H-pyrrolo[3,2-b]pyridin-5-yl]-3-(trifluoromethyl)pyrazol-4-yl]-1-methyl-imidazole-2-carbonyl]amino]-2-chloro-benzoic acid | 659.5 [M+H]⁺ | Intermediate B18 |
| **Interme diate C16** | 4-[[5-[1-[2-[(tert-butoxycarbonylamino)methyl]-1H-pyrrolo[3,2-c]pyridin-6-yl]-3-(trifluoromethyl)pyrazol-4-yl]-1-methyl-imidazole-2-carbonyl]amino]-2-chloro-benzoic acid | 659.3 [M+H]⁺ | Intermediate B19 |
| **Interme diate C17** | 4-[[5-[1-[5-[bis[2-(tert-butoxycarbonylamino)ethyl]amino]-2-pyridyl]-3-(trifluoromethyl)pyrazol-4-yl]-1-methyl-imidazole-2-carbonyl]amino]-2-chloro-benzoic acid | 792.3 [M+H]⁺ | Intermediate B20 |
| **Interme diate C18** | 4-[[5-[1-[5-[(3S)-3-(tert-butoxycarbonylamino)pyrrolidin-1-yl]-2-pyridyl]-3-(trifluoromethyl)pyrazol-4-yl]-1-methyl-imidazole-2-carbonyl]amino]-2-chloro-benzoic acid | 675.3 [M+H]⁺ | Intermediate B21 |

### Intermediate D38

### 4-[4-[[5-[1-[5-(Benzyloxycarbonylaminomethyl)-2-pyridyl]-3-(trifluoromethyl)pyrazol-4-yl]-1-methyl-imidazole-2-carbonyl]amino]-2-chloro-benzoyl]piperazine-1-carboxylic acid tert-butyl ester

A vial was charged with **Intermediate D5** (50 mg, 0.068 mmol, 1 eq), 2-(benzyloxycarbonylamino)acetic acid(21.26 mg, 0.102 mmol, 1.5 eq, Ir[dF(CF₃)ppy]₂(dtbbpy)PF₆ (3.8 mg, 0.003 mmol, 0.05 eq), NiCl₂-glyme (4.47 mg, 0.020 mmol, 0.3 eq), 4,4'-di-tert-butyl-2,2'-bipyridyl (8.18 mg, 0.030 mmol, 0.45 eq), and Cs₂CO₃ (33.1 mg, 0.102 mmol, 1.5 eq). The vial was evacuated and backfilled with Ar (3x), and N,N-dimethylformamide, extra dry (3.3 mL) was added. The vial was sealed, and then stirred at RT under blue LED (420 nm) irradiation for 24 h. The mixture was diluted with half-sat. aq. NaHCO₃ (30 mL), extracted with EtOAc (3 x 10 mL). The combined organics were washed with brine (10 mL), dried (Na₂SO₄), filtered, and evaporated. Purification by column chromatography (0-40% DCM/MeOH 9:1 in DCM) afforded the title compound **Intermediate D38** (23 mg, 38%) as colorless amorphous. MS [M+HCOO]⁻ 866.3.

### Intermediate D41

### 4-[2-Chloro-4-[[1-methyl-5-[1-[5-(methylamino)-2-pyridyl]-3-(trifluoromethyl)pyrazol-4-yl]imidazole-2-carbonyl]amino]benzoyl]piperazine-1-carboxylic acid tert-butyl ester

A vial was charged with sodium-tert-butoxide (31.23 mg, 0.325 mmol, 2.5 eq), racemic-2,2'-bis(diphenylphosphino)-1,1'-binaphthyl (32.38 mg, 0.052 mmol, 0.400 eq), and tris(dibenzylideneacetone)dipalladium (23.81 mg, 0.026 mmol, 0.200 eq), and the atmosphere was saturated with Ar. A solution of **Intermediate D5** (95.9 mg, 0.130 mmol, 1 eq) in toluene, extra dry (1.8 mL) was added, followed by a 2 M solution of methylamine in THF (0.650 mmol, 5 eq). The vial was sealed, and placed in a heatblock shaker at 110°C for 24 hr. The vial was allowed to cool down to RT, diluted with half-sat. brine (10 mL), and extracted with EtOAc (3 x 5 mL). The combined organics were evaporated, and the residue purified by RPHPLC to afford the title compound **Intermediate D41** (33 mg, 37%) as off-white lyophilized solid.

The following intermediates were prepared in analogy of **Intermediate D41**

| **Ex#** | **Name** | **MS ESI** | **Starting Material** |
|---|---|---|---|
| **Interme diate D42** | 4-[2-chloro-4-[[1-methyl-5-[1-(5-morpholino-2-pyridyl)-3-(trifluoromethyl)pyrazol-4-yl]imidazole-2-carbonyl]amino]benzoyl]piperazine-1-carboxylic acid tert-butyl ester | 742.5 [M-H]- | Intermediate D5 and morpholine 9,9-dimethyl-4,5-bis(diphenylphosphino) xanthene |
| **Interme diate D45** | 4-[2-chloro-4-[[5-[1-[5-(isopropylamino)-2-pyridyl]-3-(trifluoromethyl)pyrazol-4-yl]-1-methyl-imidazole-2-carbonyl]amino]benzoyl]piperazine-1-carboxylic acid tert-butyl ester | 760.4 [M+HCOO]- | Intermediate D5 and isopropylamine racemic-2,2'-bis(diphenylphosphino) -1,1'-binaphthyl |
| **Interme diate D46** | 4-[2-chloro-4-[[1-methyl-5-[1-[5-(2-pyridylamino)-2-pyridyl]-3-(trifluoromethyl)pyrazol-4-yl]imidazole-2-carbonyl]amino]benzoyl]piperazine-1-carboxylic acid tert-butyl ester | 749.4 [M-H]- | Intermediate D5 and 2-aminopyridine 9,9-dimethyl-4,5-bis(diphenylphosphino) xanthene |
| **Interme diate D47** | 4-[2-chloro-4-[[5-[1-[5-(cyclopropylamino)-2-pyridyl]-3-(trifluoromethyl)pyrazol-4-yl]-1-methyl-imidazole-2-carbonyl]amino]benzoyl]piperazine-1-carboxylic acid tert-butyl ester | 712.5 [M-H]- | Intermediate D5 and cyclopropylamine racemic-2,2'-bis(diphenylphosphino) -1,1'-binaphthyl |
| **Interme diate D12** | 4-[2-chloro-4-[[5-[1-[5-(dimethylamino)-2-pyridyl]-3-(trifluoromethyl)pyrazol-4-yl]-1-methyl-imidazole-2-carbonyl]amino]benzoyl]piperazine-1-carboxylic acid tert-butyl ester | 700.4 [M-H]- | Intermediate D5 and 2 M dimethylamine in THF 9,9-dimethyl-4,5-bis(diphenylphosphino) xanthene |
| **Interme diate D50** | 4-[2-chloro-4-[[1-methyl-5-[1-[5-(4-methylpiperazino)-2-pyridyl]-3-(trifluoromethyl)pyrazol-4-yl]imidazole-2-carbonyl]amino]benzoyl]piperazine-1-carboxylic acid tert-butyl ester | 755.5 [M-H]- | Intermediate D5 and 1-methylpiperazine 9,9-dimethyl-4,5-bis(diphenylphosphino) xanthene |
| **Interme diate D54** | 4-[2-chloro-4-[[5-[1-[5-(cyclobutylamino)-2-pyridyl]-3-(trifluoromethyl)pyrazol-4-yl]-1-methyl-imidazole-2-carbonyl]amino]benzoyl]piperazine-1-carboxylic acid tert-butyl ester | 726.4 [M-H]- | Intermediate D5 and cyclobutylamine racemic-2,2'-bis(diphenylphosphino) -1,1'-binaphthyl |

### Intermediate D118

### 4-[4-[[5-[1-[2-[[tert-butyl(dimethyl)silyl]oxymethyl]-1H-pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carboxylic acid tert-butyl ester

A mixture of **Intermediate D7** (217 mg, 0.292 mmol, 1 eq) in DCM (3 mL) was treated with imidazole (29.83 mg, 0.438 mmol, 1.5 eq) and TBDMSCl (52.82 mg, 0.350 mmol, 1.2 eq), and the reaction mixture was stirred at RT for 3.5 h. Water was added, and the mixture was extracted. After drying over Na₂SO₄, filtration, and evaporation to dryness, the crude title compound **Intermediate D118** (217.3 mg, 79.5%) was obtained as an off-white solid, which was used without further purification in the next step. MS [M+H]⁺ 842.46.

### Intermediate D44

### 4-[2-Chloro-4-[[1-methyl-5-[1-(1-methylpyrrolo[3,2-b]pyridin-5-yl)-3-(trifluoromethyl)pyrazol-4-yl]imidazole-2-carbonyl]amino]benzoyl]piperazine-1-carboxylic acid tert-butyl ester

A mixture of NaH (3.96 mg, 0.099 mmol, 1.2 eq) in N,N-dimethylformamide was cooled to 0°C, and treated dropwise with a mixture of **Intermediate D2** (60 mg, 0.083 mmol, 1 eq) dissolved in DMF (0.1 mL), and the reaction mixture was stirred until the evolution of hydrogen was complete. After dropwise addition of iodomethane (11.7 mg, 5.2 µL, 0.083 mmol, 1 eq), the reaction mixture was allowed to warm to RT, and stirred for 3 h. Water was added, and the mixture was extracted with DCM. The combined organic layers were washed with 5% LiCl solution, brine, dried (Na₂SO₄), filtered, and evaporated. Purification by column chromatography (0-80% DCM/MeOH 9:1 in DCM) gave the title compound **Intermediate D44** (58.5 mg, 98%) as light yellow amorphous. MS [M+H]⁺ 712.5.

The following intermediates were prepared in analogy of **Intermediate D44**

| **Ex#** | **Name** | **MS ESI** | **Starting Material** |
|---|---|---|---|
| **Interme diate D119** | 5-[4-[2-[[4-(4-tert-butoxycarbonylpiperazine-1-carbonyl)-3-chloro-phenyl]carbamoyl]-3-methyl-imidazol-4-yl]-3-(trifluoromethyl)pyrazol-1-yl]-1-(cyanomethyl)pyrrolo[3,2-b]pyridine-2-carboxylic acid ethyl ester | 807.35 [M-H]- | Intermediate D95 and bromoacetonitrile K2CO3 |
| **Interme diate D120** | 4-[2-chloro-4-[[5-[1-[1-(2-hydroxyethyl)-2-methylol-pyrrolo[3,2-b]pyridin-5-yl]-3-(trifluoromethyl)pyrazol-4-yl]-1-methyl-imidazole-2-carbonyl]amino]benzoyl]piperazine-1-carboxylic acid tert-butyl ester | 772.3 [M+H]⁺ | Intermediate D118 and (2-bromoethoxy)-tert-butyldimethylsilane |
| **Interme diate D121** | 4-[2-chloro-4-[[5-[1-[1-(2-hydroxyethyl)pyrrolo[3,2-b]pyridin-5-yl]-3-(trifluoromethyl)pyrazol-4-yl]-1-methyl-imidazole-2-carbonyl]amino]benzoyl]piperazine-1-carboxylic acid tert-butyl ester | 742.39 [M+H]⁺ | Intermediate D2 and tert-butyl-(2-iodoethoxy)dimethylsilane (CAS 101166-65-8) |
| **Interme diate D122** | 4-[4-[[5-[1-[1-(2-amino-2-keto-ethyl)pyrrolo[3,2-b]pyridin-5-yl]-3-(trifluoromethyl)pyrazol-4-yl]-1-methyl-imidazole-2-carbonyl]amino]-2-chloro-benzoyl]piperazine-1-carboxylic acid tert-butyl ester | 755.27 [M+H]⁺ | Intermediate D2 and 2-bromoacetamide |

### Intermediate D48

### 4-[2-Chloro-4-[[5-[1-[6-(dimethylamino)-3-pyridyl]-3-(trifluoromethyl)pyrazol-4-yl]-1-methyl-imidazole-2-carbonyl]amino]benzoyl]piperazine-1-carboxylic acid tert-butyl ester

A mixture of **Intermediate D14** (50 mg, 0.052 mmol, 1 eq) and 2 M dimethylamine in THF (1.29 mL, 2.58 mmol, 50 eq) was heated in the microwave at 100°C for 45 min. All volatiles were evaporated, and the residue was purified by column chromatography (0-80% DCM/MeOH 9:1 in DCM) to yield the title compound **Intermediate D48** (31 mg, 72%) as white solid. MS [M+H]⁺ 702.4.

### Intermediate D52

### 4-[2-Chloro-4-[[5-[1-(1H-imidazo[4,5-b]pyridin-5-yl)-3-(trifluoromethyl)pyrazol-4-yl]-1-methyl-imidazole-2-carbonyl]amino]benzoyl]piperazine-1-carboxylic acid tert-butyl ester

A mixture of **Intermediate D51** (130 mg, 0.172 mmol, 1 eq) in toluene, extra dry (2 mL) was treated with trimethyl orthoformate (182.18 mg, 188 µL, 1.72 mmol, 10 eq) and p-toluenesulfonic acid monohydrate (3.3 mg, 0.017 mmol, 0.1 eq), and the mixture was stirred at 80 °C for 4 h. All volatiles were evaporated to afford the crude title compound **Intermediate D52** (120 mg, 93%) as yellow solid, which was directly used in the next step without any further purification.

### Intermediate D123

### tert-Butyl 4-[4-[[5-[1-[2-(3-amino-3-oxo-propyl)-1-(2-trimethylsilylethoxymethyl)pyrrolo[3,2-b]pyridin-5-yl]-3-(trifluoromethyl)pyrazol-4-yl]-1-methyl-imidazole-2-carbonyl]amino]-2-chloro-benzoyl]piperazine-1-carboxylate

### Step 1:

A mixture **Intermediate D63** (450.0 mg, 0.49 mmol, 1.0 eq) and lithium hydroxide monohydrate (206.49 mg, 4.92 mmol, 10.0 eq) in water (1.5 mL) / THF (1.5 mL) was stirred at 25 °C for 2 h. EtOAc (10 mL) and water (30 mL) were added, and the layers were separated. The aqueous phase was extracted with EtOAc (2 x 10 mL), and the combined extracts were washed with brine (30 mL), dried over MgSO4, filtered, and concentrated. The residue was purified by column chromatography (PE/EA 4:1) to afford 3-[5-[4-[2-[[4-(4-tert-butoxycarbonylpiperazine-1-carbonyl)-3-chloro-phenyl]carbamoyl]-3-methyl-imidazol-4-yl]-3-(trifluoromethyl)pyrazol-1-yl]-1-(2-trimethylsilylethoxymethyl)pyrrolo[3,2-b]pyridin-2-yl]propanoic acid (400.0 mg, 0.44 mmol, 58%) as yellow oil. MS [M+H]⁺ 900.5.

### Step 2:

A mixture of 3-[5-[4-[2-[[4-(4-tert-butoxycarbonylpiperazine-1-carbonyl)-3-chlorophenyl]carbamoyl]-3-methyl-imidazol-4-yl]-3-(trifluoromethyl)pyrazol-1-yl]-1-(2-trimethylsilylethoxymethyl)pyrrolo[3,2-b]pyridin-2-yl]propanoic acid (400 mg, 0.4 mmol, 1 eq) in DMF (2 mL) was treated with DIPEA (0.09 mL, 0.49 mmol, 1.1 eq), HATU (405.39 mg, 1.07 mmol, 2.4 eq), and NH₄Cl (47.52 mg, 0.89 mmol, 2.0 eq). The reaction mixture was stirred at 25 °C for 2 h, and diluted with EtOAc (10 mL) and water (20 mL). The layers were separated, and the aq. phase was extracted with EtOAc (2 x 10 mL). The combined extracts werre washed with brine (20 mL), dried (MgSO4), filtered, and evaporated. Purification by column chromatography (PE/EA 1:1) afforded the title compound **Intermediate D123** (300 mg, 75%) as a yellow oil. MS [M+H]⁺ 899.7.

### Intermediate E53

### 5-[1-(2-Fluoro-4-hydroxy-phenyl)-3-(trifluoromethyl)pyrazol-4-yl]-N-[3-fluoro-4-(piperazine-1-carbonyl)phenyl]-1-methyl-imidazole-2-carboxamide; hydrochloride

To a solution of **Intermediate D107** (290.0 mg, 0.42 mmol, 1.0 eq) in DCM (5 mL) was added boron tribromide (2.11 g, 8.41 mmol, 20.0 eq), and the mixture was stirred at 25 °C for 16 h to give a light yellow suspension. The mixture was concentrated under vacuum. MeOH (20 mL) was added dropwise to the residue, and the resulting mixture was stirred for 10 min, and then concentrated under vacuum. The residue was dissolved in DMSO (4 mL), and purified by RP column chromatography (0-80% acetonitrile in water, HCl) to give the title compound **Intermediate E53** (150 mg, 0.25 mmol, 58%) as a white solid. MS [M+H]⁺ 576.2.

### Intermediate E1

### 5-[1-(5-amino-2-pyridyl)-3-(trifluoromethyl)pyrazol-4-yl]-N-[3-chloro-4-(piperazine-1-carbonyl)phenyl]-1-methyl-imidazole-2-carboxamide .1:1 hydrogen chloride

A mixture of **Intermediate D31** (1.09 g, 1.6 mmol, 1 eq) in dichloromethane (5 mL) was treated with 4 M HCl in dioxane (4.8 g, 4 mL, 16.01 mmol, 10 eq), and the reaction mixture was stirred at RT for 1 h. The reaction mixture was concentrated to dryness, and dried under HV to give the title compound **Intermediate E1** (1.21 g, quant.) as off-white solid, which was in the next step without any further purification. MS [M+H]⁺ 574.2.

The following intermediates were prepared in analogy of **Intermediate E1**

| **Ex#** | **Name** | **MS ESI** | **Starting Material** |
|---|---|---|---|
| **Interme diate E2** | 5-[1-(4-amino-2-fluoro-phenyl)-3-(trifluoromethyl)pyrazol-4-yl]-N-[3-chloro-4-(piperazine-1-carbonyl)phenyl]-1-methyl-imidazole-2-carboxamide .1:1 hydrogen chloride | 635.3 [M+HCOO]- | Intermediate D34 |
| **Interme diate E4** | N-[3-chloro-4-(piperazine-1-carbonyl)phenyl]-1-methyl-5-[1-(1H-pyrrolo[3,2-c]pyridin-6-yl)-3-(trifluoromethyl)pyrazol-4-yl]imidazole-2-carboxamide .1:1 hydrogen chloride | 596.4 [M-H]- | Intermediate D4 |
| **Interme diate E5** | N-[3-chloro-4-(piperazine-1-carbonyl)phenyl]-1-methyl-5-[1-(1H-pyrrolo[3,2-b]pyridin-5-yl)-3-(trifluoromethyl)pyrazol-4-yl]imidazole-2-carboxamide .1:1 hydrogen chloride | 596.2 [M-H]- | Intermediate D2 |
| **Interme diate E6** | N-[(1S)-2-[[6-[4-[2-[[3-chloro-4-(piperazine-1-carbonyl)phenyl]carbamoyl]-3-methyl-imidazol-4-yl]-3-(trifluoromethyl)pyrazol-1-yl]-3-pyridyl]amino]-1-methylethyl]carbamic acid 9H-fluoren-9-ylmethyl ester .1:1 hydrogen chloride | 897.4 [M+HCOO]- | Intermediate D37 |
| **Interme diate E7** | N-[[6-[4-[2-[[3-chloro-4-(piperazine-1-carbonyl)phenyl]carbamoyl]-3-methyl-imidazol-4-yl]-3-(trifluoromethyl)pyrazol-1-yl]-3-pyridyl]methyl]carbamic acid benzyl ester .1:1 hydrogen chloride | 766.3 [M+HCOO]- | Intermediate D38 |
| **Interme diate E8** | N-[3-chloro-4-(piperazine-1-carbonyl)phenyl]-5-[1-(5-methoxy-2-pyridyl)-3-(trifluoromethyl)pyrazol-4-yl]-1-methyl-imidazole-2-carboxamide .1:1 hydrogen chloride | 587.3 [M-H]- | Intermediate D6 |
| **Interme diate E11** | N-[3-chloro-4-(piperazine-1-carbonyl)phenyl]-1-methyl-5-[1-[5-(methylamino)-2-pyridyl]-3-(trifluoromethyl)pyrazol-4-yl]imidazole-2-carboxamide .1:1 hydrogen chloride | 586.3 [M-H]- | Intermediate D41 |
| **Interme diate E12** | N-[3-chloro-4-(piperazine-1-carbonyl)phenyl]-1-methyl-5-[1-(5-morpholino-2-pyridyl)-3-(trifluoromethyl)pyrazol-4-yl]imidazole-2-carboxamide .1:1 hydrogen chloride | 642.4 [M-H]- | Intermediate D42 |
| **Interme diate E14** | N-[3-chloro-4-(piperazine-1-carbonyl)phenyl]-1-methyl-5-[1-(2-methylol-1H-pyrrolo[3,2-b]pyridin-5-yl)-3-(trifluoromethyl)pyrazol-4-yl]imidazole-2-carboxamide .1:1 hydrogen chloride | 626.5 [M-H]- | Intermediate D7 |
| **Interme diate E15** | N-[3-chloro-4-(piperazine-1-carbonyl)phenyl]-5-[1-(4-methoxyphenyl)-3-(trifluoromethyl)pyrazol-4-yl]-1-methyl-imidazole-2-carboxamide .1:1 hydrogen chloride | 586.3 [M-H]- | Intermediate D8 |
| **Interme diate E16** | N-[3-chloro-4-(piperazine-1-carbonyl)phenyl]-5-[1-[5-(isopropylamino)-2-pyridyl]-3-(trifluoromethyl)pyrazol-4-yl]-1-methyl-imidazole-2-carboxamide .1:1 hydrogen chloride | 614.4 [M-H]- | Intermediate D45 |
| **Interme diate E17** | N-[3-chloro-4-(piperazine-1-carbonyl)phenyl]-1-methyl-5-[1-[5-(2-pyridylamino)-2-pyridyl]-3-(trifluoromethyl)pyrazol-4-yl]imidazole-2-carboxamide .1:1 hydrogen chloride | 649.4 [M-H]- | Intermediate D46 |
| **Interme diate E18** | N-[3-chloro-4-(piperazine-1-carbonyl)phenyl]-1-methyl-5-[1-(1H-pyrazolo[4,3-b]pyridin-5-yl)-3-(trifluoromethyl)pyrazol-4-yl]imidazole-2-carboxamide .1:1 hydrogen chloride | 597.5 [M-H]- | Intermediate D10 |
| **Interme diate E19** | N-[3-chloro-4-(piperazine-1-carbonyl)phenyl]-1-methyl-5-[1-thiazol-2-yl-3-(trifluoromethyl)pyrazol-4-yl]imidazole-2-carboxamide .1:1 hydrogen chloride | 563.2 [M-H]- | Intermediate D11 |
| **Interme diate E20** | N-[3-chloro-4-(piperazine-1-carbonyl)phenyl]-5-[1-[5-(cyclopropylamino)-2-pyridyl]-3-(trifluoromethyl)pyrazol-4-yl]-1-methyl-imidazole-2-carboxamide .1:1 hydrogen chloride | 612.4 [M-H]- | Intermediate D47 |
| **Interme diate E21** | N-[3-chloro-4-(piperazine-1-carbonyl)phenyl]-5-[1-[5-(dimethylamino)-2-pyridyl]-3-(trifluoromethyl)pyrazol-4-yl]-1-methyl-imidazole-2-carboxamide .1:1 hydrogen chloride | 600.4 [M-H]- | Intermediate D12 |
| **Interme diate E22** | N-[3-chloro-4-(piperazine-1-carbonyl)phenyl]-5-[1-[4-(dimethylamino)phenyl]-3-(trifluoromethyl)pyrazol-4-yl]-1-methyl-imidazole-2-carboxamide .1:1 hydrogen chloride | 599.3 [M-H]- | Intermediate D13 |
| **Interme diate E24** | N-[3-chloro-4-(piperazine-1-carbonyl)phenyl]-5-[1-[6-(dimethylamino)-3-pyridyl]-3-(trifluoromethyl)pyrazol-4-yl]-1-methyl-imidazole-2-carboxamide .1:1 hydrogen chloride | 600.3 [M-H]- | Intermediate D48 |
| **Interme diate E26** | N-[3-chloro-4-(piperazine-1-carbonyl)phenyl]-1-methyl-5-[1-[5-(4-methylpiperazino)-2-pyridyl]-3-(trifluoromethyl)pyrazol-4-yl]imidazole-2-carboxamide .1:1 hydrogen chloride | 655.4 [M-H]- | Intermediate D50 |
| **Interme diate E27** | N-[3-chloro-4-(piperazine-1-carbonyl)phenyl]-5-[1-(5-hydroxy-2-pyridyl)-3-(trifluoromethyl)pyrazol-4-yl]-1-methyl-imidazole-2-carboxamide .1:1 hydrogen chloride | 575.2 [M+H] [M+H]⁺ | Intermediate D16 |
| **Interme diate E28** | N-[3-chloro-4-(piperazine-1-carbonyl)phenyl]-5-[1-(1H-imidazo[4,5-b]pyridin-5-yl)-3-(trifluoromethyl)pyrazol-4-yl]-1-methyl-imidazole-2-carboxamide .1:1 hydrogen chloride | 597.4 [M-H]⁻ | Intermediate D52 |
| **Interme diate E29** | 5-[1-(6-amino-2-pyridyl)-3-(trifluoromethyl)pyrazol-4-yl]-N-[3-chloro-4-(piperazine-1-carbonyl)phenyl]-1-methyl-imidazole-2-carboxamide .1:1 hydrogen chloride | 574.2 [M+H]⁺ | Intermediate D18 |
| **Interme diate E31** | N-[3-chloro-4-(piperazine-1-carbonyl)phenyl]-5-[1-(2,3-dihydro-1H-pyrrolo[3,2-b]pyridin-5-yl)-3-(trifluoromethyl)pyrazol-4-yl]-1-methyl-imidazole-2-carboxamide .1:1 hydrogen chloride | 598.5 [M-H]⁻ | Intermediate D20 |
| **Interme diate E33** | N-[3-chloro-4-(piperazine-1-carbonyl)phenyl]-5-[1-furo[3,2-b]pyridin-5-yl-3-(trifluoromethyl)pyrazol-4-yl]-1-methyl-imidazole-2-carboxamide .1:1 hydrogen chloride | 597.5 [M-H]⁻ | Intermediate D22 |
| **Interme diate E34** | N-[3-chloro-4-(piperazine-1-carbonyl)phenyl]-5-[1-(5,6-diamino-2-pyridyl)-3-(trifluoromethyl)pyrazol-4-yl]-1-methyl-imidazole-2-carboxamide .1:1 hydrogen chloride | 633.5 [M+HCOO]⁻ | Intermediate D51 |
| **Interme diate E35** | N-[3-chloro-4-(piperazine-1-carbonyl)phenyl]-1-methyl-5-[1-[6-(methylamino)-3-pyridyl]-3-(trifluoromethyl)pyrazol-4-yl]imidazole-2-carboxamide .1:1 hydrogen chloride | 294.7 [M+2H]²⁺ | Intermediate D53 |
| **Interme diate E36** | N-[3-chloro-4-(piperazine-1-carbonyl)phenyl]-5-[1-[5-(cyclobutylamino)-2-pyridyl]-3-(trifluoromethyl)pyrazol-4-yl]-1-methyl-imidazole-2-carboxamide .1:1 hydrogen chloride | 626.4 [M-H]⁻ | Intermediate D54 |
| **Interme diate E37** | 5-[1-(4-amino-2-pyridyl)-3-(trifluoromethyl)pyrazol-4-yl]-N-[3-chloro-4-(piperazine-1-carbonyl)phenyl]-1-methyl-imidazole-2-carboxamide .1:1 hydrogen chloride | 574.2 [M+H]⁺ | Intermediate D23 |
| **Interme diate E38** | 5-[1-(2-amino-4-pyridyl)-3-(trifluoromethyl)pyrazol-4-yl]-N-[3-chloro-4-(piperazine-1-carbonyl)phenyl]-1-methyl-imidazole-2-carboxamide .1:1 hydrogen chloride | 574.2 [M+H]⁺ | Intermediate D24 |
| **Interme diate E39** | N-[3-chloro-4-(piperazine-1-carbonyl)phenyl]-5-[1-(5-cyano-2-pyridyl)-3-(trifluoromethyl)pyrazol-4-yl]-1-methyl-imidazole-2-carboxamide .1:1 hydrogen chloride | 582.4 [M-H]⁻ | Intermediate D25 in acetonitrile |
| **Interme diate E43** | 5-[1-(1,3-benzothiazol-2-yl)-3-(trifluoromethyl)pyrazol-4-yl]-N-[3-chloro-4-(piperazine-1-carbonyl)phenyl]-1-methyl-imidazole-2-carboxamide .1:1 hydrogen chloride | 613.3 [M-H]⁻ | Intermediate D26 |
| **Interme diate E44** | N-[3-chloro-4-(piperazine-1-carbonyl)phenyl]-5-[1-(6-methoxy-3-pyridyl)-3-(trifluoromethyl)pyrazol-4-yl]-1-methyl-imidazole-2-carboxamide .1:1 hydrogen chloride | 633.5 [M+HCOO]⁻ | Intermediate D27 |
| **Interme diate E45** | N-[3-chloro-4-(piperazine-1-carbonyl)phenyl]-5-[1-[5-[(dimethylamino)methyl]-2-pyridyl]-3-(trifluoromethyl)pyrazol-4-yl]-1-methyl-imidazole-2-carboxamide .1:1 hydrogen chloride | 614.2 [M-H]⁻ | Intermediate D55 |
| **Interme diate E47** | N-[3-chloro-4-(piperazine-1-carbonyl)phenyl]-1-methyl-5-[1-[2-(methylamino)-4-pyridyl]-3-(trifluoromethyl)pyrazol-4-yl]imidazole-2-carboxamide .1:1 hydrogen chloride | 588.2 [M+H]⁺ | Intermediate D29 |
| **Interme diate E51** | N-[3-chloro-4-(piperazine-1-carbonyl)phenyl]-1-methyl-5-[1-(4-nitroisothiazol-5-yl)-3-(trifluoromethyl)pyrazol-4-yl]imidazole-2-carboxamide .1:1 hydrogen chloride | 608.3 [M-H]- | Intermediate D30 |
| **Interme diate E54** | N-[3-chloro-4-(piperazine-1-carbonyl)phenyl]-5-[1-[2-(3-hydroxypropyl)-1H-pyrrolo[3,2-b]pyridin-5-yl]-3-(trifluoromethyl)pyrazol-4-yl]-1-methyl-imidazole-2-carboxamide;hydrochloride | 656.4 [M+H]⁺ | Intermediate D62 |
| **Interme diate E55** | 5-[1-[2-(3-amino-3-oxo-propyl)-1H-pyrrolo[3,2-b]pyridin-5-yl]-3-(trifluoromethyl)pyrazol-4-yl]-N-[3-chloro-4-(piperazine-1-carbonyl)phenyl]-1-methyl-imidazole-2-carboxamide | 669.5 [M+H]⁺ | Intermediate D123 |
| | | | TFA in DCM, then NH3 in MeOH |
| **Interme diate E56** | N-[3-chloro-4-(piperazine-1-carbonyl)phenyl]-5-[1-(2-fluoro-4-morpholino-phenyl)-3-(trifluoromethyl)pyrazol-4-yl]-1-methyl-imidazole-2-carboxamide;hydrochloride | 661.0 [M+H]⁺ | Intermediate D64 |
| **Interme diate E57** | 5-[1-[2-[(1S)-1-hydroxyethyl]-1H-pyrrolo[3,2-b]pyridin-5-yl]-3-(trifluoromethyl)pyrazol-4-yl]-1-methyl-N-[3-methyl-4-(piperazine-1-carbonyl)phenyl]imidazole-2-carboxamide | 622.2 | Intermediate D65 |
| | | [M+H]⁺ | TFA in DCM, then NH3 in MeOH |
| **Interme diate E58** | N-[3-chloro-4-(piperazine-1-carbonyl)phenyl]-5-[1-[2-[2-hydroxy-1-(hydroxymethyl)ethyl]-1H-pyrrolo[3,2-b]pyridin-5-yl]-3-(trifluoromethyl)pyrazol-4-yl]-1-methyl-imidazole-2-carboxamide;hydrochloride | 672.4 [M+H]⁺ | Intermediate D66 |
| | | | HCl in dioxane, then NH3 in MeOH |
| **Interme diate E59** | N-[3-chloro-4-(piperazine-1-carbonyl)phenyl]-1-methyl-5-[1-[1-methyl-2-(morpholine-4-carbonyl)pyrrolo[3,2-b]pyridin-5-yl]-3-(trifluoromethyl)pyrazol-4-yl]imidazole-2-carboxamide;hydrochloride | 725.4 [M+H]⁺ | Intermediate D67 |
| **Interme diate E60** | 5-[4-[2-[[3-chloro-4-(piperazine-1-carbonyl)phenyl]carbamoyl]-3-methyl-imidazol-4-yl]-3-(trifluoromethyl)pyrazol-1-yl]-N-(2-hydroxyethyl)-1-methyl-pyrrolo[3,2-b]pyridine-2-carboxamide;hydrochloride | 699.3 [M+H]⁺ | Intermediate D68 |
| **Interme diate E61** | N-[3-chloro-4-(piperazine-1-carbonyl)phenyl]-5-[1-[2-(1,2-dihydroxyethyl)-1H-pyrrolo[3,2-b]pyridin-5-yl]-3-(trifluoromethyl)pyrazol-4-yl]-1-methyl-imidazole-2-carboxamide; formic acid | 658.3 [M+H]⁺ | Intermediate D69 |
| | | | HCl in dioxane, then NH3 in MeOH |
| **Interme diate E62** | 5-[1-[2-fluoro-4-(methylamino)phenyl]-3-(trifluoromethyl)pyrazol-4-yl]-1-methyl-N-[3-methyl-4-(piperazine-1-carbonyl)phenyl]imidazole-2-carboxamide;hydrochloride | 585.4 [M+H]⁺ | Intermediate D110 |
| **Interme diate E63** | 5-[1-[2-fluoro-4-(2-methoxyethylamino)phenyl]-3-(trifluoromethyl)pyrazol-4-yl]-1-methyl-N-[3-methyl-4-(piperazine-1-carbonyl)phenyl]imidazole-2-carboxamide;hydrochloride | 629.2 [M+H]⁺ | Intermediate D111 |
| **Interme diate E64** | N-[3-chloro-4-(piperazine-1-carbonyl)phenyl]-5-[1-[4-[2-(difluoromethoxy)ethylamino]-2-fluoro-phenyl]-3-(trifluoromethyl)pyrazol-4-yl]-1-methyl-imidazole-2-carboxamide;hydrochloride | 685.4 [M+H]⁺ | Intermediate D70 |
| **Interme diate E65** | 5-[1-[2-(2-azidoethyl)-1H-pyrrolo[3,2-b]pyridin-5-yl]-3-(trifluoromethyl)pyrazol-4-yl]-N-[3-chloro-4-(piperazine-1-carbonyl)phenyl]-1-methyl-imidazole-2-carboxamide; hydrochloride | 667.2 [M+H]⁺ | Intermediate D72 |
| | | | TFA in DCM, then NH3 in MeOH |
| **Interme diate E66** | N-[3-chloro-4-(piperazine-1-carbonyl)phenyl]-5-[1-[2-fluoro-4-[[(2S)-2,3-dihydroxypropyl]amino]phenyl]-3-(trifluoromethyl)pyrazol-4-yl]-1-methyl-imidazole-2-carboxamide;hydrochloride | 665.1 [M+H]⁺ | Intermediate D73 |
| **Interme diate E67** | 5-[1-[1-(2-amino-1,1-dimethyl-2-oxoethyl)pyrrolo[3,2-b]pyridin-5-yl]-3-(trifluoromethyl)pyrazol-4-yl]-N-[3-chloro-4-(piperazine-1-carbonyl)phenyl]-1-methyl-imidazole-2-carboxamide; hydrochloride | 683.4 [M+H]⁺ | Intermediate D124 |
| **Interme diate E68** | N-[3-fluoro-4-(piperazine-1-carbonyl)phenyl]-1-methyl-5-[1-(2-methylol-1H-pyrrolo[3,2-b]pyridin-5-yl)-3-(trifluoromethyl)pyrazol-4-yl]imidazole-2-carboxamide .1:1 hydrogen chloride | 610.3 [M-H]⁻ | Intermediate D81 |
| **Interme diate E69** | 5-[1-(4-amino-2-fluoro-phenyl)-3-(trifluoromethyl)pyrazol-4-yl]-1-methyl-N-[3-methyl-4-(piperazine-1-carbonyl)phenyl]imidazole-2-carboxamide .1:1 hydrogen chloride | 615.3 [M+HCOO]⁻ | Intermediate D106 |
| **Interme diate E70** | N-[3-chloro-4-(piperazine-1-carbonyl)phenyl]-5-[1-[2-[(1R)-1-hydroxyethyl]-1H-pyrrolo[3,2-b]pyridin-5-yl]-3-(trifluoromethyl)pyrazol-4-yl]-1-methyl-imidazole-2-carboxamide; formic acid | 642.2 [M+H]⁺ | Intermediate D82 |
| | | | TFA in DCM, then NH3 in MeOH |
| **Interme diate E71** | 1-methyl-5-[1-(2-methylol-1H-pyrrolo[3,2-b]pyridin-5-yl)-3-(trifluoromethyl)pyrazol-4-yl]-N-[3-methyl-4-(piperazine-1-carbonyl)phenyl]imidazole-2-carboxamide .1:1 hydrogen chloride | 606.3 [M-H]⁻ | Intermediate D83 |
| **Interme diate E72** | N-[3-chloro-4-(piperazine-1-carbonyl)phenyl]-5-[1-[2-(2-hydroxyethyl)-1H-pyrrolo[3,2-c]pyridin-6-yl]-3-(trifluoromethyl)pyrazol-4-yl]-1-methyl-imidazole-2-carboxamide; formic acid | 642.3 [M+H]⁺ | Intermediate D84 |
| | | | trifluoromethanesulfoni c acid in TFA, then K2CO3 in water |
| **Interme diate E73** | N-[3-chloro-4-(piperazine-1-carbonyl)phenyl]-5-[1-[1-(dimethylsulfamoyl)-2-(2-hydroxyethyl)pyrrolo[3,2-c]pyridin-6-yl]-3-(trifluoromethyl)pyrazol-4-yl]-1-methyl-imidazole-2-carboxamide;hydrochloride | 749.1 [M+H]⁺ | Intermediate D84 |
| **Interme diate E74** | 5-[1-[1-(3-amino-2-methyl-3-oxopropyl)pyrrolo[3,2-b]pyridin-5-yl]-3-(trifluoromethyl)pyrazol-4-yl]-N-[3-chloro-4-(piperazine-1-carbonyl)phenyl]-1-methyl-imidazole-2-carboxamide; hydrochloride | 683.3 [M+H]⁺ | Intermediate D125 |
| **Interme diate E75** | 5-[1-(4-benzoxy-2-fluoro-phenyl)-3-(trifluoromethyl)pyrazol-4-yl]-N-[3-chloro-4-(piperazine-1-carbonyl)phenyl]-1-methyl-imidazole-2-carboxamide .1:1 hydrogen chloride | 726.3 [M+HCOO]⁻ | Intermediate D90 |
| **Interme diate E76** | N-[3-chloro-4-(piperazine-1-carbonyl)phenyl]-5-[1-[2-fluoro-4-[(2-hydroxy-2-methylpropyl)amino]phenyl]-3-(trifluoromethyl)pyrazol-4-yl]-1-methyl-imidazole-2-carboxamide;hydrochloride | 663.3 [M+H]⁺ | Intermediate D91 |
| | | | HCl in MeOH |
| **Interme diate E77** | N-[3-chloro-4-(piperazine-1-carbonyl)phenyl]-1-methyl-5-[1-(5H-pyrrolo[3,2-d]pyrimidin-2-yl)-3-(trifluoromethyl)pyrazol-4-yl]imidazole-2-carboxamide;hydrochloride | 599.3 [M+H]⁺ | Intermediate D92 |
| | | | TFA in DCM, then NH3 in MeOH |
| **Interme diate E78** | 5-[1-[2-(2-amino-2-oxo-ethyl)-1H-pyrrolo[3,2-b]pyridin-5-yl]-3-(trifluoromethyl)pyrazol-4-yl]-N-[3-chloro-4-(piperazine-1-carbonyl)phenyl]-1-methyl-imidazole-2-carboxamide; hydrochloride | 655.2 [M+H]⁺ | Intermediate D126 |
| **Interme diate E79** | N-[3-chloro-4-(piperazine-1-carbonyl)phenyl]-5-[1-[2-fluoro-4-(methylamino)phenyl]-3-(trifluoromethyl)pyrazol-4-yl]-1-methyl-imidazole-2-carboxamide .1:1 hydrogen chloride | 603.2 [M-H]⁻ | Intermediate D112 |
| **Interme diate E80** | 5-[1-[4-(2-benzoxyethylamino)-2-fluoro-phenyl]-3-(trifluoromethyl)pyrazol-4-yl]-N-[3-chloro-4-(piperazine-1-carbonyl)phenyl]-1-methyl-imidazole-2-carboxamide .1:1 hydrogen chloride | 769.4 [M+HCOO]⁻ | Intermediate D113 |
| **Interme diate E81** | N-[3-chloro-4-(piperazine-1-carbonyl)phenyl]-5-[1-[2-fluoro-4-(2-methoxyethylamino)phenyl]-3-(trifluoromethyl)pyrazol-4-yl]-1-methyl-imidazole-2-carboxamide .1:1 hydrogen chloride | 693.4 [M+HCOO]⁻ | Intermediate D114 |
| **Interme diate E82** | N-[3-[4-[4-[2-[[3-chloro-4-(piperazine-1-carbonyl)phenyl]carbamoyl]-3-methyl-imidazol-4-yl]-3-(trifluoromethyl)pyrazol-1-yl]-3-fluoro-anilino]propyl]carbamic acid 9H-fluoren-9-ylmethyl ester .1:1 hydrogen chloride | 914.3 [M+HCOO]- | Intermediate D115 |
| **Interme diate E83** | N-[3-chloro-4-(piperazine-1-carbonyl)phenyl]-1-methyl-5-[1-(1H-pyrrolo[2,3-c]pyridin-5-yl)-3-(trifluoromethyl)pyrazol-4-yl]imidazole-2-carboxamide .1:1 hydrogen chloride | 596.3 [M-H]- | Intermediate D105 |

### Intermediate E3

### N-[2-[[6-[4-[2-[[3-Chloro-4-(piperazine-1-carbonyl)phenyl]carbamoyl]-3-methyl-imidazol-4-yl]-3-(trifluoromethyl)pyrazol-1-yl]-3-pyridyl]amino]ethyl]carbamic acid tert-butyl ester

A mixture of **Intermediate D33** (44 mg, 0.044 mmol, 1 eq) in acetonitrile (2.5 mL) was treated with 2 M dimethylamine in THF (110 µL, 0.220 mmol, 5 eq), and stirred at RT for 3 h. The mixture was evaporated to afford crude title compound **Intermediate E3** (32 mg, quant.) as off-white solid, which was directly used in the next step without any further purification. MS [M+HCOO]⁻ 761.6.

The following intermediates were prepared in analogy of **Intermediate E3**

| **Ex#** | **Name** | **MS ESI** | **Starting Material** |
|---|---|---|---|
| **Interme diate E9** | N-[3-chloro-4-(piperazine-1-carbonyl)phenyl]-1-methyl-5-[1-[5-(2-pyridylmethylamino)-2-pyridyl]-3-(trifluoromethyl)pyrazol-4-yl]imidazole-2-carboxamide | 665.2 [M+H]⁺ | Intermediate D39 in THF |
| **Interme diate E10** | N-[1-[[[6-[4-[2-[[3-chloro-4-(piperazine-1-carbonyl)phenyl]carbamoyl]-3-methyl-imidazol-4-yl]-3-(trifluoromethyl)pyrazol-1-yl]-3-pyridyl]amino]methyl]cyclopentyl]car bamic acid tert-butyl ester | 771.3 [M+H]⁺ | Intermediate D40 in THF |
| **Interme diate E13** | (2S)-2-[[[6-[4-[2-[[3-chloro-4-(piperazine-1-carbonyl)phenyl]carbamoyl]-3-methyl-imidazol-4-yl]-3-(trifluoromethyl)pyrazol-1-yl]-3-pyridyl]amino]methyl]pyrrolidine-1-carboxylic acid tert-butyl ester | 757.3 [M+H]⁺ | Intermediate D43 in THF |
| **Interme diate E23** | (3S)-3-[[[6-[4-[2-[[3-chloro-4-(piperazine-1-carbonyl)phenyl]carbamoyl]-3-methyl-imidazol-4-yl]-3-(trifluoromethyl)pyrazol-1-yl]-3-pyridyl]amino]methyl]morpholine-4-carboxylic acid tert-butyl ester | 773.7 [M+H]⁺ | Intermediate D56 in THF |
| **Interme diate E25** | N-[6-[[6-[4-[2-[[3-chloro-4-(piperazine-1-carbonyl)phenyl]carbamoyl]-3-methyl-imidazol-4-yl]-3-(trifluoromethyl)pyrazol-1-yl]-3-pyridyl]amino]hexyl]carbamic acid tert-butyl ester | 773.7 [M+H]⁺ | Intermediate D49 in THF |
| **Interme diate E30** | (2S,4S)-2-[[[6-[4-[2-[[3-chloro-4-(piperazine-1-carbonyl)phenyl]carbamoyl]-3-methyl-imidazol-4-yl]-3-(trifluoromethyl)pyrazol-1-yl]-3-pyridyl]amino]methyl]-4-fluoro-pyrrolidine-1-carboxylic acid tert-butyl ester | 775.7 [M+H]⁺ | Intermediate D57 in THF |

### Intermediate E84

### N-[3-chloro-4-(piperazine-1-carbonyl)phenyl]-5-[1-[2-[(2S)-2,3-dihydroxypropyl]-1H-pyrrolo[3,2-b]pyridin-5-yl]-3-(trifluoromethyl)pyrazol-4-yl]-1-methyl-imidazole-2-carboxamide;hydrochloride

### Step 1:

A mixture of **Intermediate D88** (290.0 mg, 0.29 mmol, 1.0 eq) in a solution of HCl in MeOH (6.0 mL, 24.0 mmol, 82.23 eq) was stirred at 20 °C for 1 h. The mixture was treated with MeOH (3 x 10mL), and concentrated in vacuo to afford N-[3-chloro-4-(piperazine-1-carbonyl)phenyl]-5-[1-[2-[(2S)-2,3-dihydroxypropyl]-1-(dimethylsulfamoyl)pyrrolo[3,2-b]pyridin-5-yl]-3-(trifluoromethyl)pyrazol-4-yl]-1-methyl-imidazole-2-carboxamide (240.0 mg, 0.31 mmol, quant.) as a yellow solid. MS [M+H]⁺ 779.2.

### Step 2:

A mixture of N-[3-chloro-4-(piperazine-1-carbonyl)phenyl]-5-[1-[2-[(2S)-2,3-dihydroxypropyl]-1-(dimethylsulfamoyl)pyrrolo[3,2-b]pyridin-5-yl]-3-(trifluoromethyl)pyrazol-4-yl]-1-methylimidazole-2-carboxamide (220.0 mg, 0.28 mmol, 1.0 eq) in TFA (10 mL, 129.8 mmol, 460 eq) and trifluoromethanesulfonic acid (42.4 mg, 0.28 mmol, 1.0 eq) was stirred at 20 °C for 16 h. The reaction mixture was treated with MeOH (3 x 5 mL), and concentrated in vacuo to afford N-[3-chloro-4-[4-(2,2,2-trifluoroacetyl)piperazine-1-carbonyl]phenyl]-5-[1-[2-[(2S)-2,3-dihydroxypropyl]-1H-pyrrolo[3,2-b]pyridin-5-yl]-3-(trifluoromethyl)pyrazol-4-yl]-1-methylimidazole-2-carboxamide (210.0 mg, 0.27 mmol, 17.6%) as a brown liquid. MS [M+H]⁺ 767.9.

### Step 3:

A mixture of N-[3-chloro-4-[4-(2,2,2-trifluoroacetyl)piperazine-1-carbonyl]phenyl]-5-[1-[2-[(2S)-2,3-dihydroxypropyl]-1H-pyrrolo[3,2-b]pyridin-5-yl]-3-(trifluoromethyl)pyrazol-4-yl]-1-methyl-imidazole-2-carboxamide (210.0 mg, 0.27 mmol, 1.0 eq) in methanol (5 mL) was treated with potassium carbonate (188.94 mg, 1.37 mmol, 5.0 eq), and stirred at 20 °C for 3 h. The mixture was concentrated in vacuo, and purified by RPHPLC to afford the title compound **Intermediate E84** (45.0 mg, 0.07 mmol, 24.5%) as a yellow solid. MS [M+H]⁺ 672.4.

The following intermediates were prepared in analogy of **Intermediate E84**

| **Ex#** | **Name** | **MS ESI** | **Starting Material** |
|---|---|---|---|
| **Interme diate E85** | N-[3-chloro-4-(piperazine-1-carbonyl)phenyl]-5-[1-[2-(2-hydroxyethyl)-1H-pyrrolo[3,2-b]pyridin-5-yl]-3-(trifluoromethyl)pyrazol-4-yl]-1-methyl-imidazole-2-carboxamide;hydrochloride | 642.2 [M+H]⁺ | Intermediate D89 |

### Intermediate E32

### N-[3-Chloro-4-(piperazine-1-carbonyl)phenyl]-5-[1-(1H-indazol-3-yl)-3-(trifluoromethyl)pyrazol-4-yl]-1-methyl-imidazole-2-carboxamide

A mixture of **Intermediate D21** (200.0 mg, 0.240 mmol, 1 eq) in DCM (5 mL) was treated with trifluoroacetic acid (1.0 mL, 12.98 mmol, 54 eq), and the mixture was stirred at 25 °C for 1 h . The mixture was concentrated, and the residue was treated with 7 M NH₃ in methanol (5.0 mL, 35 mmol, 145 eq). The mixture was stirred at 25 °C for 1 h, and concentrated. Purification by RPHPLC afforded the title compound **Intermediate E32** (40 mg, 28%) as white solid. MS [M+H]⁺ 598.2.

### Intermediate E40

### 5-[1-(5-Amino-2-pyridyl)-3-(trifluoromethyl)pyrazol-4-yl]-N-[3-chloro-4-[(3S)-3-methylpiperazine-1-carbonyl]phenyl]-1-methyl-imidazole-2-carboxamide .1:1 formic acid

A mixture of **Intermediate C10** (37.47 mg, 0.060 mmol, 1 eq) in DMF (1 mL) was treated with HATU (31.94 mg, 0.084 mmol, 1.4 eq) and Et₃N (30.36 mg, 41.81 µL, 0.3 mmol, 5 eq). The mixture was stirred at RT for 10 min, and then treated with tert-butyl (2S)-2-methylpiperazine-1-carboxylate (18.03 mg, 0.090 mmol, 1.5 eq). The mixture was stirred at RT O/N, and evaporated to dryness. The residue was dissolved in DCM (1 mL), treated with excess of 4 M HCl (189 mg, 180 µL, 0.720 mmol, 40 eq), and stirred at RT O/N. The mixture was evaporated, and purified by RPHPLC to afford the title product **Intermediate E40** (19.9 mg, 52%). MS [M+H]⁺ 588.2.

The following intermediates were prepared in analogy of **Intermediate E40**

| **Ex#** | **Name** | **MS ESI** | **Starting Material** |
|---|---|---|---|
| **Interme diate E41** | 5-[1-(5-amino-2-pyridyl)-3-(trifluoromethyl)pyrazol-4-yl]-N-[3-chloro-4-[(2R)-2-ethylpiperazine-1-carbonyl]phenyl]-1-methyl-imidazole-2-carboxamide .1:1 formic acid | 602.3 [M+H]⁺ | Intermediate C10 and tert-butyl (3R)-3-ethyl-1-piperazinecarboxylate |
| **Interme diate E42** | 5-[1-(5-amino-2-pyridyl)-3-(trifluoromethyl)pyrazol-4-yl]-N-[3-chloro-4-[(2S)-2-methylpiperazine-1-carbonyl]phenyl]-1-methyl-imidazole-2-carboxamide .1:1 formic acid | 588.2 [M+H]⁺ | Intermediate C10 and tert-butyl (3S)-3-methyl-1-piperazinecarboxylate |
| **Interme diate E46** | 5-[1-(5-amino-2-pyridyl)-3-(trifluoromethyl)pyrazol-4-yl]-N-[3-chloro-4-(4,7-diazaspiro[2.5]octane-7-carbonyl)phenyl]-1-methyl-imidazole-2-carboxamide .1:1 formic acid | 598.3 [M-H]⁻ | Intermediate C10 and tert-butyl 4,7-diazaspiro[2.5]octane-7-carboxylate |
| **Interme diate E48** | 5-[1-(5-amino-2-pyridyl)-3-(trifluoromethyl)pyrazol-4-yl]-N-[3-chloro-4-(3,8-diazabicyclo[3.2.1]octane-8-carbonyl)phenyl]-1-methyl-imidazole-2-carboxamide .1:1 formic acid | 600.2 [M+H]⁺ | Intermediate C10 and tert-butyl 3,8-diazabicyclo[3.2.1]octa ne-3-carboxylate |
| **Interme diate E49** | 5-[1-(5-amino-2-pyridyl)-3-(trifluoromethyl)pyrazol-4-yl]-N-[3-chloro-4-(3,9-diazabicyclo[3.3.1]nonane-3-carbonyl)phenyl]-1-methyl-imidazole-2-carboxamide .1:1 formic acid | 614.2 [M+H]⁺ | Intermediate C10 and tert-butyl 3,9-diazabicyclo[3.3.1]nona ne-9-carboxylate |
| **Interme diate E50** | 5-[1-(5-amino-2-pyridyl)-3-(trifluoromethyl)pyrazol-4-yl]-N-[3-chloro-4-(4,7-diazaspiro[2.5]octane-4-carbonyl)phenyl]-1-methyl-imidazole-2-carboxamide .1:1 formic acid | 600.2 [M+H]⁺ | Intermediate C10 and tert-butyl 4,7-diazaspiro[2.5]octane-7-carboxylate |
| **Interme diate E52** | 5-[1-(5-amino-2-pyridyl)-3-(trifluoromethyl)pyrazol-4-yl]-N-[3-chloro-4-[3-(methoxymethyl)piperazine-1-carbonyl]phenyl]-1-methyl-imidazole-2-carboxamide .1:1 formic acid | 618.3 [M+H]⁺ | Intermediate C10 and tert-butyl 2-(methoxymethyl)pipera zine-1-carboxylate |

### Intermediate F1

### 1-[4-[[5-[1-(5-Amino-2-pyridyl)-3-(trifluoromethyl)pyrazol-4-yl]-1-methyl-imidazole-2-carbonyl]amino]-2-chloro-benzoyl]isonipecotic acid

### Step 1:

A mixture of **Intermediate C10** (200 mg, 0.395 mmol, 1 eq) and isonipecotic acid methyl ester;hydrochloride (85.23 mg, 0.474 mmol, 1.2 eq) in N,N-dimethylformamide (2.5 mL) was treated with DIPEA (153.3 mg, 207.16 µL, 1.19 mmol, 3 eq), and subsequently with HATU (225.5 mg, 0.593 mmol, 1.5 eq). The reaction mixture was stirred at RT for 24 h, and all volatiles were evaporated. The residue was suspended in water (20 mL), and extracted with EtOAc (3 x 25 mL). The combined organics were washed with water (25 mL), dried (Na₂SO₄), filtered, and evaporated. Purification by column chromatography (20-70% EtOAc/EtOH 3:1 in heptane) afforded 1-[4-[[5-[1-(5-amino-2-pyridyl)-3-(trifluoromethyl)pyrazol-4-yl]-1-methyl-imidazole-2-carbonyl]amino]-2-chloro-benzoyl]isonipecotic acid methyl ester (139 mg, 48%) as light brown solid. MS [M-H]⁻ 629.3.

### Step 2:

A mixture of 1-[4-[[5-[1-(5-amino-2-pyridyl)-3-(trifluoromethyl)pyrazol-4-yl]-1-methylimidazole-2-carbonyl]amino]-2-chloro-benzoyl]isonipecotic acid methyl ester (189 mg, 0.258 mmol, 1 eq) in MeOH (0.739 mL) / THF (2.22 mL) / water (0.739 mL) was treated with 1 M LiOH (1.03 mL, 1.03 mmol, 4 eq), and the mixture was stirred at RT for 18 h. The mixture was concentratred, suspended in around 5 mL of water, and some drops of 1 M HCl were added until the pH was close to 2 or 3. The precipitate formed was filtered off, and dried to afford the title compound **Intermediate F1** (207 mg, quant.) as orange crystalline solid. MS [M+H]⁺ 617.2.

### Intermediate G1

### tert-Butyl cis-3-[[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]-1-(2-tert-butoxy-2-oxo-ethyl)piperidin-1-ium-1-yl]methyl]azetidine-1-carboxylate;2,2,2-trifluoroacetate

A mixture of **Intermediate E1** (400 mg, 0.620 mmol, 1 eq) in DMF (8 mL) was treated with **Intermediate H4** (382.62 mg, 0.930 mmol, 1.5 eq), N,N-diisopropylethylamine (0.43 mL, 2.47 mmol, 4 eq), and HATU (352.67 mg, 0.930 mmol, 1.5 eq) at 0 °C. The mixture was stirred at 0 °C for 0.5 h, and treated with additional **Intermediate H4** (178.56 mg, 0.430 mmol, 0.7 eq) and HATU (164.58 mg, 0.430 mmol, 0.700 eq). The mixture was stirred at 0 °C for another 0.5 h, and then queched with water (0.3 mL), and concentrated under vacuum. Purification by RP column chromatography gave the title compound **Intermediate G1** (400 mg, 60%) as a white solid. MS [M]⁺ 968.4.

The following intermediates were prepared in analogy of **Intermediate G1**

| **Ex#** | **Name** | **MS ESI** | **Starting Material** |
|---|---|---|---|
| **Interme diate AAAA1** | cis-tert-butyl 3-[[1-(2-tert-butoxy-2-oxo-ethyl)-4-[4-[2-fluoro-4-[[1-methyl-5-[3-(trifluoromethyl)-1H-pyrazol-4-yl]imidazole-2-carbonyl]amino]benzoyl]piperazine-1-carbonyl]piperidin-1-ium-1-yl]methyl]azetidine-1-carboxylate;formate | 860.4 [M]+ | Intermediate AAA2 and Intermediate H4 |
| **Interme diate AAAA2** | cis-tert-butyl 3-[[1-(2-tert-butoxy-2-oxo-ethyl)-4-[4-[2-chloro-4-[[5-[3-(difluoromethyl)-1H-pyrazol-4-yl]-1-methyl-imidazole-2-carbonyl]amino]benzoyl]piperazine-1-carbonyl]piperidin-1-ium-1-yl]methyl]azetidine-1-carboxylate;formate | 858.5 [M]+ | Intermediate AAA1 and Intermediate H4 |
| **Interme diate G6** | tert-butyl cis-3-[[4-[4-[4-[[5-[1-[2-(2-azidoethyl)-1H-pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(2-tert-butoxy-2-oxo-ethyl)piperidin-1-ium-1-yl]methyl]azetidine-1-carboxylate;chloride | 1061.6 [M]+ | Intermediate E65 and Intermediate H4 |
| **Interme diate D124** | tert-butyl 4-[4-[[5-[1-[1-(2-amino-1,1-dimethyl-2-oxo-ethyl)pyrrolo[3,2-b]pyridin-5-yl]-3-(trifluoromethyl)pyrazol-4-yl]-1-methyl-imidazole-2-carbonyl]amino]-2-chloro-benzoyl]piperazine-1-carboxylate - P1 | 783.1 [M+H]⁺ | Intermediate D116 and NH₄Cl additional purification performed with SFC (column:DAICEL CHIRALPAK AD; mobile phase: A1=ACN(0.05% DEA), B1=IPA; gradient elution: IPA-ACN 60). |
| **Interme diate D125** | tert-butyl 4-[4-[[5-[1-[1-(3-amino-2-methyl-3-oxo-propyl)pyrrolo[3,2-b]pyridin-5-yl]-3-(trifluoromethyl)pyrazol-4-yl]-1-methyl-imidazole-2-carbonyl]amino]-2-chloro-benzoyl]piperazine-1-carboxylate - P2 | 783.1 [M+H]⁺ | Intermediate D116 and NH₄Cl additional purification performed with SFC (column:DAICEL CHIRALPAK AD; mobile phase: A1=ACN(0.05% DEA), B1=IPA; gradient elution: IPA-ACN 60) |
| **Interme diate D126** | tert-butyl 4-[4-[[5-[1-[2-(2-amino-2-oxo-ethyl)-1H-pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carboxylate | 755.2 [M+H]⁺ | Intermediate D117 and NH₄Cl |
| **Interme diate G7** | [4-[4-[4-[[5-[1-(4-amino-2-fluorophenyl)-3-(trifluoromethyl)pyrazol-4-yl]-1-methyl-imidazole-2-carbonyl]amino]-2-chlorobenzoyl]piperazin-1-yl]-4-oxo-butyl]-bis[3-(tert-butoxycarbonylamino)propyl]-(4-methoxy-4-oxobutyl)ammonium;2,2,2-trifluoroacetate | 1091.6 [M]⁺ | Intermediate E2 and Intermediate H14 |

### Intermediate G8

### cis-6-[4-[2-[[4-[4-[1-[(1-tert-Butoxycarbonylazetidin-3-yl)methyl]-1-(carboxymethyl)piperidin-1-ium-4-carbonyl]piperazine-1-carbonyl]-3-chlorophenyl]carbamoyl]-3-methyl-imidazol-4-yl]-3-(trifluoromethyl)pyrazol-1-yl]-1H-pyrrolo[3,2-c]pyridine-2-carboxylic acid .1:1 2,2,2-trifluoroacetate

### Step 1:

A mixture of **Intermediate D71** (980 mg, 1.3 mmol, 1 eq) in DCM (6 mL) was treated with 4 M HCl (4.86 mL, 19.44 mmol, 15 eq), stirred at ambient temperature for 45 min, and concentrated in vacuo. The residue was dissolved in DMF (5 mL), and treated with **Intermediate H4** (750.68 mg, 1.43 mmol, 1.1 eq), DIPEA (837.59 mg, 1.13 mL, 6.48 mmol, 5 eq), and HATU (739.21 mg, 1.94 mmol, 1.5 eq). The mixture was stirred at ambient temperature for 45 min, and concentrated in vacuo. Purification by RP column chromatography (10-60% acetonitrile in water) afforded cis-6-[4-[2-[[4-[4-[1-[(1-tert-butoxycarbonylazetidin-3-yl)methyl]-1-(2-tert-butoxy-2-keto-ethyl)piperidin-1-ium-4-carbonyl]piperazine-1-carbonyl]-3-chlorophenyl]carbamoyl]-3-methyl-imidazol-4-yl]-3-(trifluoromethyl)pyrazol-1-yl]-1H-pyrrolo[3,2-c]pyridine-2-carboxylic acid methyl ester .1:1 2,2,2-trifluoroacetate (1.416 g, 88%) as light yellow solid. MS [M-2H]⁻ 1048.9.

### Step 2:

A mixture of cis-6-[4-[2-[[4-[4-[1-[(1-tert-butoxycarbonylazetidin-3-yl)methyl]-1-(2-tert-butoxy-2-keto-ethyl)piperidin-1-ium-4-carbonyl]piperazine-1-carbonyl]-3-chloro-phenyl]carbamoyl]-3-methyl-imidazol-4-yl]-3-(trifluoromethyl)pyrazol-1-yl]-1H-pyrrolo[3,2-c]pyridine-2-carboxylic acid methyl ester .1:1 2,2,2-trifluoroacetate (1.405 g, 1.21 mmol, 1 eq) in THF (10 mL) was treated with 1 M aq. LiOH (6.03 mL, 6.03 mmol, 5 eq), and stirred at 40 °C for 1 h.. The mixture was concentrated in vacuo. Water (300 mL) was added, and the pH was adjusted to pH=3 by adding 1 M HCl. The mixture was filtrated, and the aquaeous layer was extracted with EtOAc (3 x 200 mL). The combined organic phases were dried (Na₂SO₄), filtered, concentrated in vacuo, and combined with the filter cake to give the title compound **Intermediate G8** (1.172 g, 84%) as colorless solid. MS [M]⁺ 980.7.

### Intermediate G9

### tert-Butyl cis-3-[[4-[4-[4-[[5-[1-[2-(2-aminoethyl)-1H-pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(2-tert-butoxy-2-oxo-ethyl)piperidin-1-ium-1-yl]methyl]azetidine-1-carboxylate; chloride

To a solution of **Intermediate G6** (180 mg, 0.16 mmol, 1.0 eq) in THF (3 mL) and water (3 mL) was added triphenylphosphine (85.99 mg, 0.33 mmol, 2.0 eq) in one portion at 20 °C. Then, the solution was stirred at 20 °C for 16 h, and concentrated. The residue was purified by RP column chromatography (60-70% acetonitrile in water, 0.1% HCl) to afford the title compound **Intermediate G9** (150 mg, 0.14 mmol, 85%) as light brown solid. MS [M]⁺ 1036.5.

### Intermediate G10

### cis-3-[[1-(2-tert-Butoxy-2-keto-ethyl)-4-[4-[2-chloro-4-[[1-methyl-5-[1-(1H-pyrrolo[3,2-c]pyridin-6-yl)-3-(trifluoromethyl)pyrazol-4-yl]imidazole-2-carbonyl]amino]benzoyl]piperazine-1-carbonyl]piperidin-1-ium-1-yl]methyl]azetidine-1-carboxylic acid tert-butyl ester .1:1 2,2,2-trifluoroacetate

A mixture of **Intermediate D94** (700 mg, 1.003 mmol, 1 eq) in DCM (10 mL) was treated with 4 M HCl in dioxane (831.59 mg, 692.99 µL, 2.77 mmol, 30 eq), and the mixture was stirred at ambient temperature for 1 h. The mixture was concentrated in vacuo. The residue was dissolved in DMF (7 mL), and treated with **Intermediate H4** (580.78 mg, 1.1 mmol, 1.1 eq), DIPEA (648.02 mg, 875.7 µL, 5.01 mmol, 5 eq) and HATU (47.4 mg, 0.125 mmol, 1.5 eq). The mixture was stirred at ambient temperature for 30 min, and concentrated in vacuo. The residue was purified by RP column chromatography (10-65% ACN in water) to the title compound **Intermediate G10** (963 mg, 83.3%) as off-white lyophilized powder. MS [M]⁺ 992.47.

The following intermediates were prepared in analogy of **Intermediate G10**

| **Ex#** | **Name** | **MS ESI** | **Starting Material** |
|---|---|---|---|
| **Interme diate G11** | cis-3-[[1-(2-tert-butoxy-2-keto-ethyl)-4-[4-[2-chloro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]methyl]azetidine-1-carboxylic acid tert-butyl ester .1:1 2,2,2-trifluoroacetate | 992.54 [M]⁺ | Intermediate D2 and Intermediate H4 |

### Intermediate D127

### 4-[2-chloro-4-[[5-[1-[1-(cyanomethyl)-2-methylol-pyrrolo[3,2-b]pyridin-5-yl]-3-(trifluoromethyl)pyrazol-4-yl]-1-methyl-imidazole-2-carbonyl]amino]benzoyl]piperazine-1-carboxylic acid tert-butyl ester

A mixture of **Intermediate D119** (128 mg, 126.55 umol, 1 eq) in THF (1.5 mL) was treated with MeOH (12.16 mg, 15.36 µL, 0.3796 mmol, 3 eq), followed by portionwise addition of lithium borohydride (4.96 mg, 0.228 mmol, 1.8 eq) at 0 °C. The mixture was stirred at 0 °C to RT for 2.5 h, and evaporated. Water was added, and the product was extracted with EtOAc, dried over Na₂SO₄, filtered, and evaporated. Purification by column chromatography (0-100% DCM/MeOH 9:1 in DCM) yielded the title compound **Intermediate D127** (70.7 mg, 55%) as light brown solid. MS [M]⁺ 767.6.

### Intermediate G12

### cis-3-[[4-[4-[4-[[5-[1-[1-(2-Aminoethyl)pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(2-tert-butoxy-2-keto-ethyl)piperidin-1-ium-1-yl]methyl]azetidine-1-carboxylic acid tert-butyl ester .1:1 2,2,2-trifluoroacetate

### Step 1:

A mixture of **Intermediate D121** (55.7 mg, 0.057 mmol, 1 eq) in DCM (0.25 mL) was treated with 4 M HCl in dioxane (171.12 mg, 142.6 µL, 0.57 mmol, 10 eq), and the reaction mixture was stirred at RT for 4 h. All volatiles were evvaporated. The residue was dissolved in DCM (0.5 mL), and treated with DIPEA (36.86 mg, 49.81 µL, 0.285 mmol, 5 eq), **Intermediate H4** (33.04 mg, 0.063 mmol, 1.1 eq), and propylphosphonic acid anhydride (cyclic trimer) 50% in EtOAc (54.45 mg, 50.41 µL, 0.086 mmol, 1.5 eq), and the reaction mixture was stirred at RT for 45 min. Water was added, and the mixture was extracted with DCM. The combined organic layers were concentrated to obtain crude cis-3-[[1-(2-tert-butoxy-2-keto-ethyl)-4-[4-[2-chloro-4-[[5-[1-[1-(2-hydroxyethyl)pyrrolo[3,2-b]pyridin-5-yl]-3-(trifluoromethyl)pyrazol-4-yl]-1-methylimidazole-2-carbonyl]amino]benzoyl]piperazine-1-carbonyl]piperidin-1-ium-1-yl]methyl]azetidine-1-carboxylic acid tert-butyl ester .1:1 2,2,2-trifluoroacetate (40.1 mg, 34%) as a light yellow solid, which was directly used in the next step without any further purification. MS [M]+ 1036.50.

### Step 2:

A mixture of cis-3-[[1-(2-tert-butoxy-2-keto-ethyl)-4-[4-[2-chloro-4-[[5-[1-[1-(2-hydroxyethyl)pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]methyl]azetidine-1-carboxylic acid tert-butyl ester .1:1 2,2,2-trifluoroacetate (40.1 mg, 0.035 mmol, 1 eq) in DCM (0.8 mL) was treated with DIPEA (9.01 mg, 12.2 µL, 0.07 mmol, 2 eq), and methanesulfonic anhydride (9.11 mg, 0.052 mmol, 1.5 eq), and the reaction mixture was stirred at RT for 1 h. The reaction mixture was concentrated to dryness. The residue was dissolved in 2 M ammonia solution (2 M in iPrOH) (0.871 mL, 1.74 mmol, 50 eq), and reaction mixture was stirred at 70 °C overnight. The mixture was evaporated, and purified by RPHPLC to yield the title compound **Intermediate G12** (3.6 mg, 9%) as a white lyophilized solid. MS [M]⁺ 1035.87.

### Example 1

### 2-[4-[4-[4-[[5-[1-[5-(2-Aminoethyl)-2-pyridyl]-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetic acid .1:1 2,2,2-trifluoroacetate

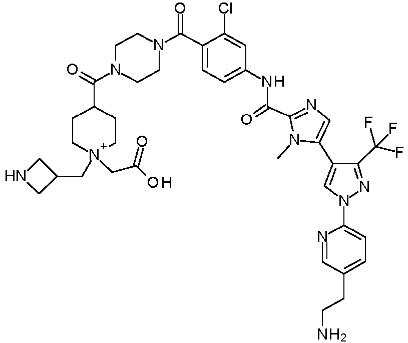

A mixture of **Intermediate C1** (48 mg, 0.076 mmol, 1 eq) and **Intermediate I1** (58.53 mg, 0.098 mmol, 1.3 eq) in dichloromethane, extra dry (2.5 mL) was treated with N,N-diisopropylethylamine (58.71 mg, 79.12 µL, 0.454 mmol, 6 eq), and subsequently with T3P in EtOAc (96.4 mg, 90.1 µL, 0.151 mmol, 2 eq). The mixture was stirred at RT for 5 h, and treated with 4 M HCl in dioxane (1.82 g, 1.51 mL, 6.06 mmol, 80 eq). Stirring was continued for another 17 h, and all volatiles were evaporated. Purification by RPHPLC afforded the title compound **Example 1** (30 mg, 37%) as white lyophilized solid. MS [M-H+HCOO]⁻ 884.8.

The following examples were prepared in analogy of **Example 1**

| **Ex#** | **Name** | **MS ESI** | **Starting Material** |
|---|---|---|---|
| **2** | 2-[4-[4-[4-[[5-[1-[5-(2-aminoethoxy)-2-pyridyl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid .1:1 2,2,2-trifluoroacetic acid .1:1 2,2,2-trifluoroacetate | 854.8 [M-2H]- | Intermediate C2 and Intermediate I1 |
| | 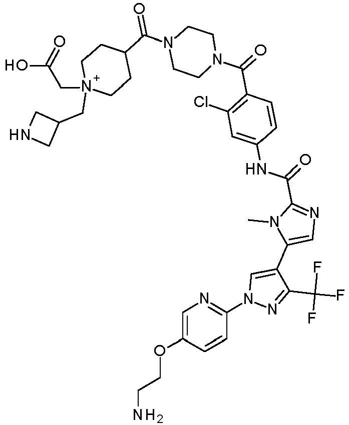 | | T3P |
| **3** | 2-[1-(azetidin-3-ylmethyl)-4-[4-[2-chloro-4-[[1-methyl-5-[1-(5-piperazino-2-pyridyl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetic acid .1:1 2,2,2-trifluoroacetate | 925.7 [M-H+HCOO]- | Intermediate C3 and Intermediate I1 |
| | | | T3P |
| | 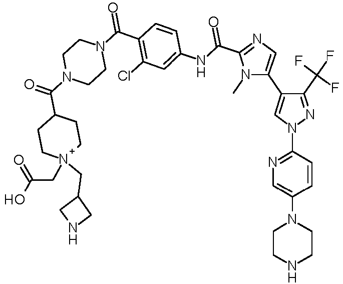 | | |
| **4** | 2-[1-(azetidin-3-ylmethyl)-4-[4-[2-chloro-4-[[1-methyl-5-[1-[5-[2-(methylamino)ethylamino]-2-pyridyl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 869.6 [M]+ | Intermediate C6 and Intermediate I1 |
| | 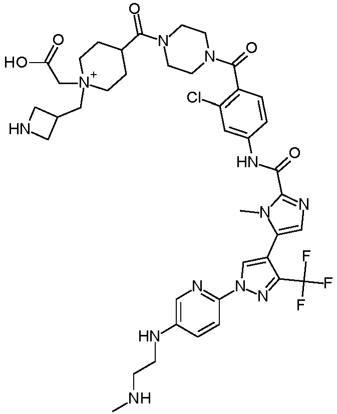 | | PyAOP |
| **5** | 2-[1-(azetidin-3-ylmethyl)-4-[4-[2-chloro-4-[[1-methyl-5-[1-[5-(4-piperidylmethylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 909.6 [M]+ | Intermediate C7 and Intermediate I1 |
| | | | PyAOP |
| | 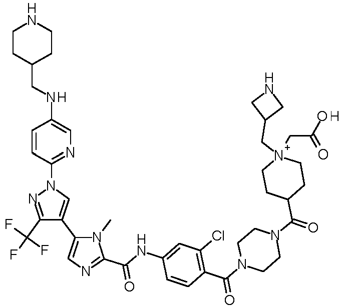 | | |
| **6** | 2-[1-(azetidin-3-ylmethyl)-4-[4-[2-chloro-4-[[5-[1-[5-[(2,2-difluorocyclopropyl)methylamino]-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 902.3 [M]+ | Intermediate C8 and Intermediate I1 |
| | 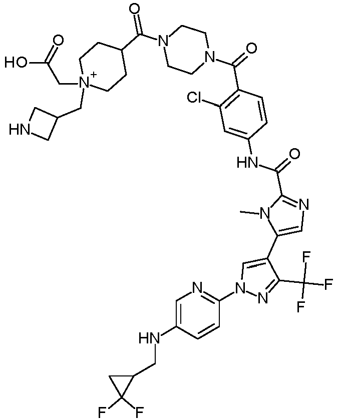 | | PyAOP |
| **7** | 2-[4-[4-[4-[[5-[1-(6-amino-3-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetate | 812.3 [M]+ | Intermediate C9 and Intermediate I1 |
| | 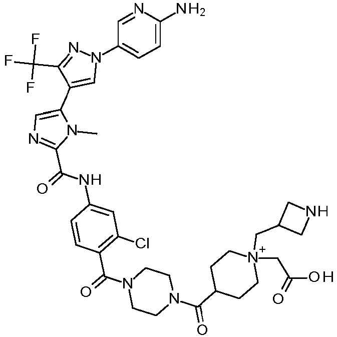 | | PyAOP |
| **8** | 2-[4-[4-[4-[[5-[1-[6-(2-aminoethylamino)-3-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetate | 855.3 [M]+ | Intermediate C5 and Intermediate I1 |
| | 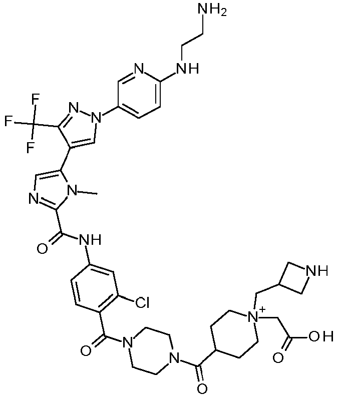 | | PyAOP |
| **11** | bis(4-aminobutyl)-[4-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl]piperazino]-4-keto-butyl]-(carboxymethyl)ammonium .1:1 2,2,2-trifluoroacetic acid .1:1 2,2,2-trifluoroacetate | 876.37 [M]+ | Intermediate E2 and Intermediate H1 |
| | 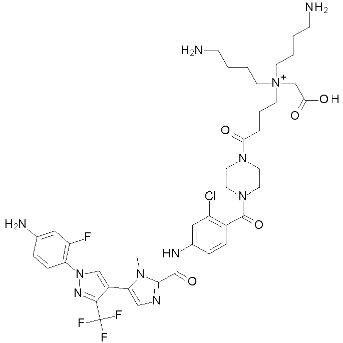 | | T3P |
| **12** | 2-[4-[4-[4-[[5-[1-[5-(2-aminoethylamino)-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetic acid .1:1 2,2,2-trifluoroacetate | 899.6 [M-H+HCOO]- | Intermediate E3 and Intermediate H2 |
| | | | PyAOP |
| | 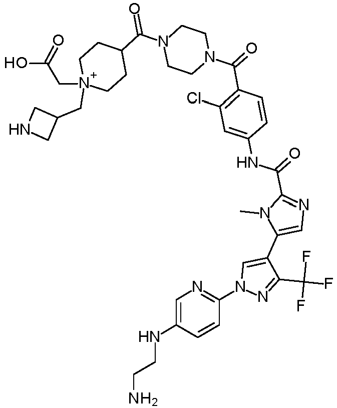 | | |
| **14** | cis-2-[1-(azetidin-3-ylmethyl)-4-[4-[2-chloro-4-[[1-methyl-5-[1-(1H-pyrrolo[3,2-c]pyridin-6-yl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 836.6 [M]+ | Intermediate E4 and Intermediate H4 |
| | 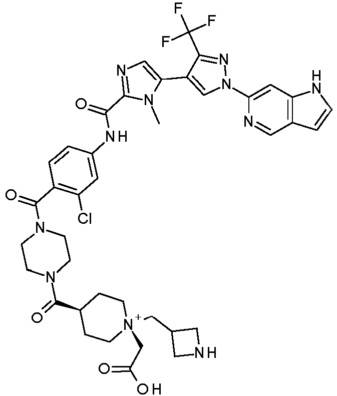 | | T3P |
| **16** | cis-2-[4-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetate | 873.5 [M-H+HCOO]- | Intermediate E2 and Intermediate H4 |
| | | | T3P |
| | 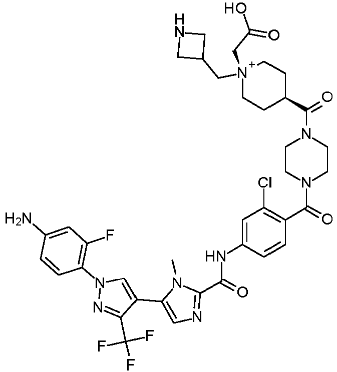 | | |
| **17** | 2-[4-[4-[4-[[5-[1-(4-amino-2-fluorophenyl)-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid .1:1 2,2,2-trifluoroacetate | 873.8 [M-H+HCOO]- | Intermediate E2 and Intermediate H2 |
| | 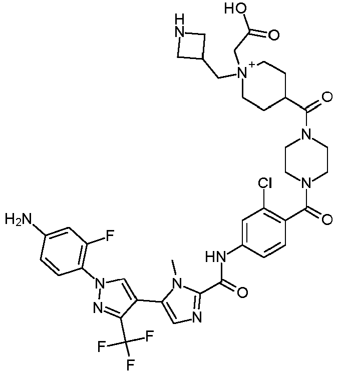 | | T3P |
| **18** | 2-[1-(azetidin-3-ylmethyl)-4-[4-[2-chloro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid .1:1 formate | 418.8 [M+H]2+ | Intermediate E5 and Intermediate H2 |
| | 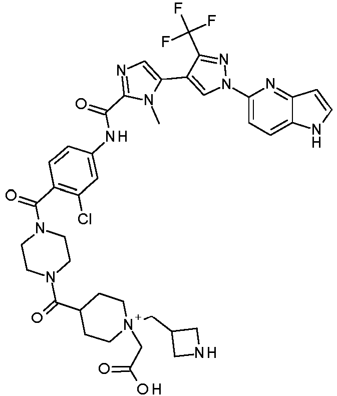 | | PyAOP |
| **22** | 2-[1-(azetidin-3-ylmethyl)-4-[4-[2-chloro-4-[[5-[1-(5-methoxy-2-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 formate | 871.5 [M-H+HCOO]- | Intermediate E8 and Intermediate H2 |
| | | | PyAOP |
| | 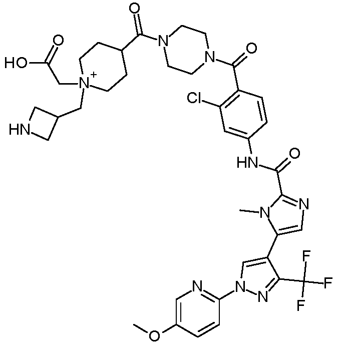 | | |
| **23** | 2-[1-(azetidin-3-ylmethyl)-4-[4-[2-chloro-4-[[1-methyl-5-[1-[5-(2-pyridylmethylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 903.3 [M]+ | Intermediate E9 and Intermediate H2 |
| | 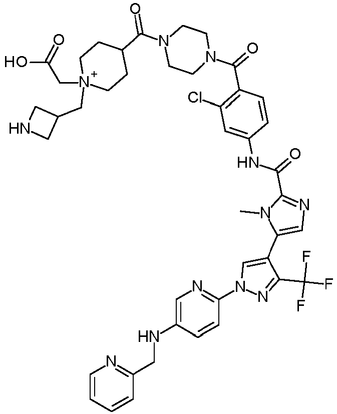 | | PyAOP |
| **24** | 2-[4-[4-[4-[[5-[1-[5-[(1-aminocyclopentyl)methylamino]-2-pyridyl]-3-(trifluoromethyl)pyrazol-4-yl]-1-methyl-imindazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetic acid .1:1 2,2,2-trifluoroacetate | 909.4 [M]+ | Intermediate E10 and Intermediate H2 |
| | | | PyAOP |
| | 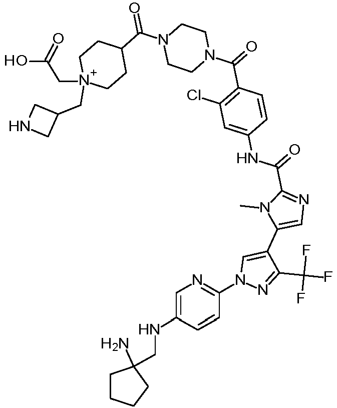 | | |
| **25** | 2-[1-(azetidin-3-ylmethyl)-4-[4-[2-chloro-4-[[1-methyl-5-[1-[5-(methylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid .1:1 formate | 870.6 [M-H+HCOO]- | Intermediate E11 and Intermediate H2 |
| | | | PyAOP |
| | 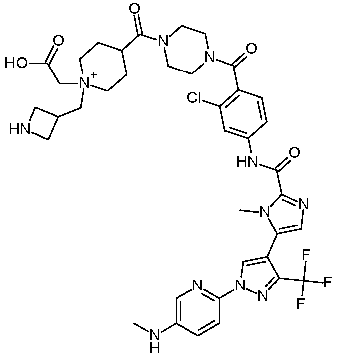 | | |
| **26** | 2-[1-(azetidin-3-ylmethyl)-4-[4-[2-chloro-4-[[1-methyl-5-[1-(5-morpholino-2-pyridyl)-3-(trifluoromethyl)pyrazol-4-yl]imidazole-2-carbonyl]amino]benzoyl]piperazine-1-carbonyl]piperidin-1-ium-1-yl]acetic acid .1:1 formate | 880.6 [M-2H]- | Intermediate E12 and Intermediate H2 |
| | 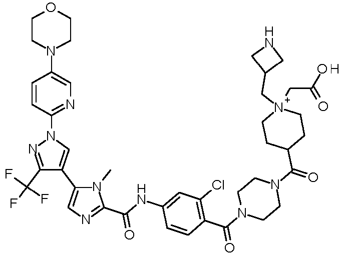 | | PyAOP |
| **28** | 2-[1-(azetidin-3-ylmethyl)-4-[4-[2-chloro-4-[[1-methyl-5-[1-[5-[[(2S)-pyrrolidin-2-yl]methylamino]-2-pyridyl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetic acid .1:1 2,2,2-trifluoroacetate | 895.4 [M]+ | Intermediate E13 and Intermediate H2 |
| | | | PyAOP |
| | 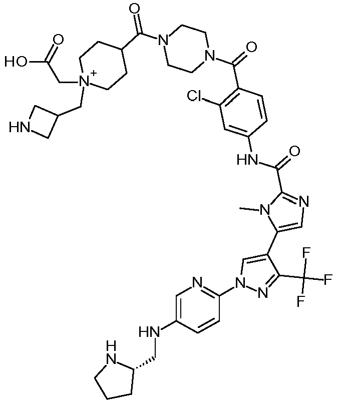 | | |
| **29** | cis-2-[1-(azetidin-3-ylmethyl)-4-[4-[2-chloro-4-[[1-methyl-5-[1-[5-(methylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 870.5, [M-H+HCOO]- | Intermediate E11 and Intermediate H4 |
| | 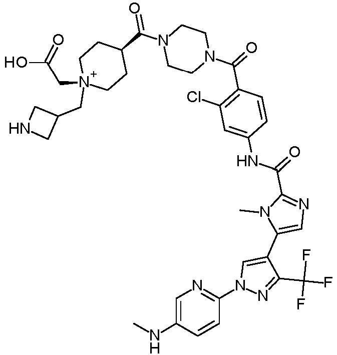 | | T3P |
| **30** | 2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid .1:1 2,2,2-trifluoroacetate | 800.4 [M]+ | Intermediate E1 and Intermediate H5 |
| | 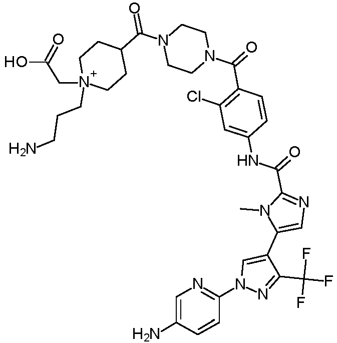 | | PyAOP |
| **33** | cis-2-[1-(azetidin-3-ylmethyl)-4-[4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 864.8 [M-2H]- | Intermediate E14 and Intermediate H4 |
| | 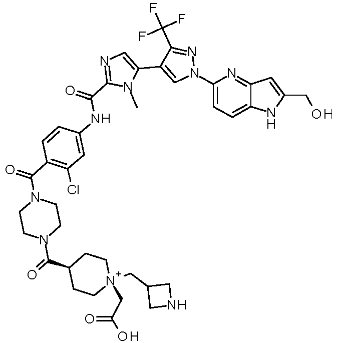 | | T3P |
| **35** | bis(3-aminopropyl)-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyl]-(carboxymethyl)ammonium .1:1 2,2,2-trifluoroacetic acid .1:1 2,2,2-trifluoroacetate | 831.5 [M]+ | Intermediate E1 and Intermediate H3 |
| | 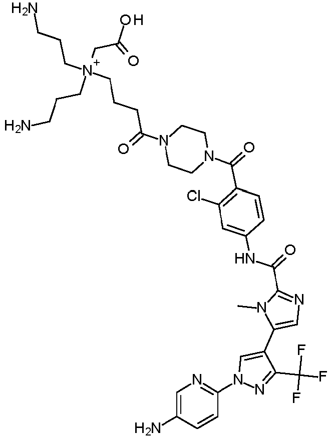 | | PyAOP |
| **37** | 2-[1-(azetidin-3-ylmethyl)-4-[4-[2-chloro-4-[[5-[1-(4-methoxyphenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 formate | 870.4 [M-H+HCOO]- | Intermediate E15 and Intermediate H2 |
| | | | PyAOP |
| | 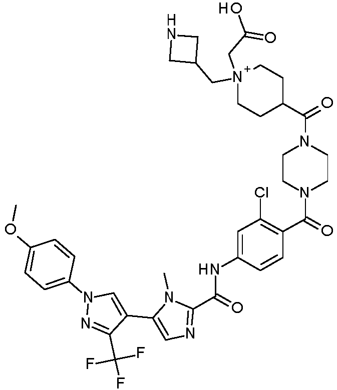 | | |
| **40** | 2-[1-(azetidin-3-ylmethyl)-4-[4-[2-chloro-4-[[5-[1-[5-(isopropylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 formate | 898.6 [M-H+HCOO]- | Intermediate E16 and Intermediate H2 |
| | 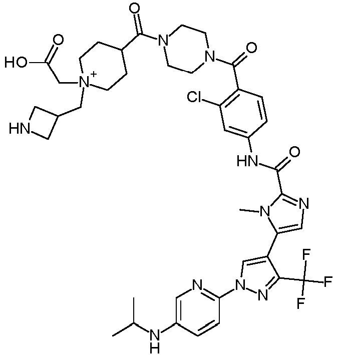 | | PyAOP |
| **41** | 2-[1-(azetidin-3-ylmethyl)-4-[4-[2-chloro-4-[[1-methyl-5-[1-[5-(2-pyridylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid .1:1 formate | 887.6 [M-2H]- | Intermediate E17 and Intermediate H2 |
| | | | PyAOP |
| | 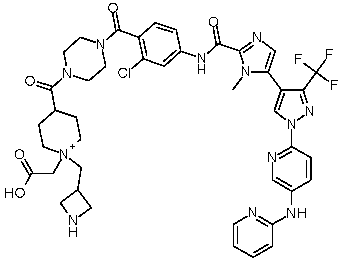 | | |
| **42** | cis-2-[1-(azetidin-3-ylmethyl)-4-[4-[2-chloro-4-[[1-methyl-5-[1-(1H-pyrazolo[4,3-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 440.6 [M-2H+HCOO]²- | Intermediate E18 and Intermediate H4 |
| | 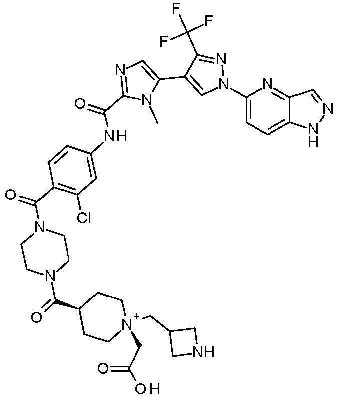 | | T3P |
| **43** | 2-[1-(azetidin-3-ylmethyl)-4-[4-[2-chloro-4-[[1-methyl-5-[1-thiazol-2-yl-3-(trifluoromethyl)pyrazol-4-yl]imidazole-2-carbonyl]amino]benzoyl]piperazine-1-carbonyl]piperidin-1-ium-1-yl]acetic acid .1:1 formate | 801.4 [M-2H]- | Intermediate E19 and Intermediate H2 |
| | | | PyAOP |
| | 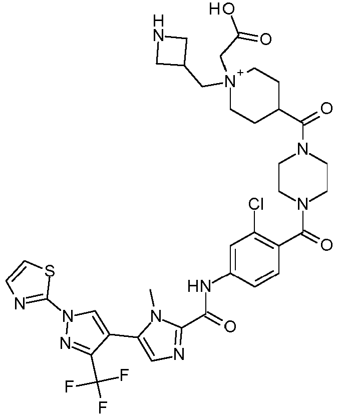 | | |
| **44** | 2-[1-(azetidin-3-ylmethyl)-4-[4-[2-chloro-4-[[5-[1-[5-(cyclopropylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 formate | 896.5 [M-H+HCOO]- | Intermediate E20 and Intermediate H2 |
| | 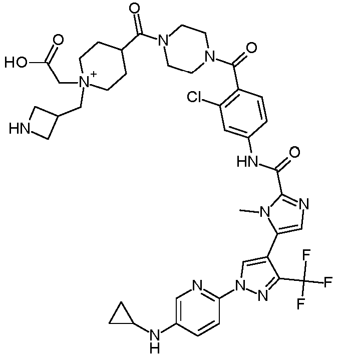 | | PyAOP |
| **45** | 2-[1-(azetidin-3-ylmethyl)-4-[4-[2-chloro-4-[[5-[1-[5-(dimethylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 formate | 838.5 [M-2H]- | Intermediate E21 and Intermediate H2 |
| | 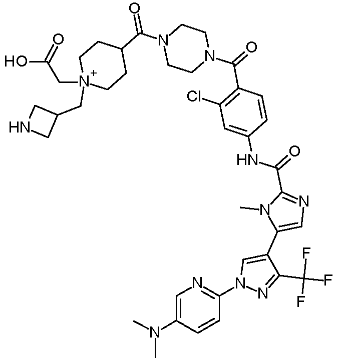 | | PyAOP |
| **46** | 2-[1-(azetidin-3-ylmethyl)-4-[4-[2-chloro-4-[[5-[1-[4-(dimethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 formate | 883.5 [M-H+HCOO]- | Intermediate E22 and Intermediate H2 |
| | | | PyAOP |
| | 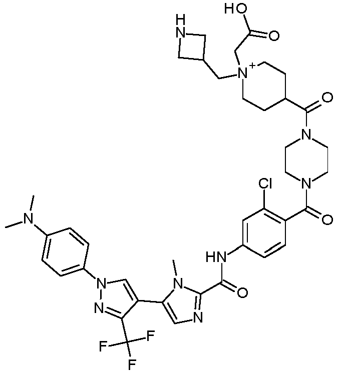 | | |
| **47** | 2-[1-(azetidin-3-ylmethyl)-4-[4-[2-chloro-4-[[1-methyl-5-[1-[5-[[(3S)-morpholin-3-yl]methylamino]-2-pyridyl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetic acid .1:1 2,2,2-trifluoroacetate | 911.7 [M]+ | Intermediate E23 and Intermediate H2 |
| | 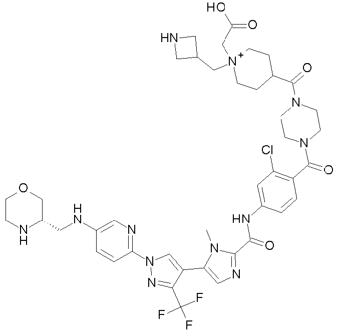 | | PyAOP |
| **49** | 2-[1-(azetidin-3-ylmethyl)-4-[4-[2-chloro-4-[[5-[1-[6-(dimethylamino)-3-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 884.5 [M-H+HCOO]- | Intermediate E24 and Intermediate H2 |
| | | | PyAOP |
| | 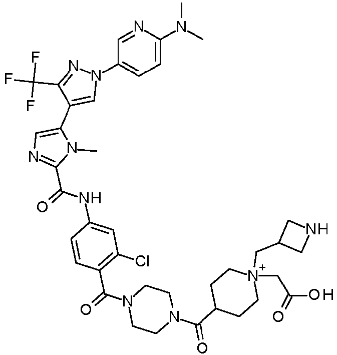 | | |
| **50** | [4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyl]-bis(azetidin-3-ylmethyl)-(carboxymethyl)ammonium .1:1 2,2,2-trifluoroacetic acid .1:1 2,2,2-trifluoroacetate | 855.5 [M]+ | Intermediate E1 and Intermediate H8 |
| | 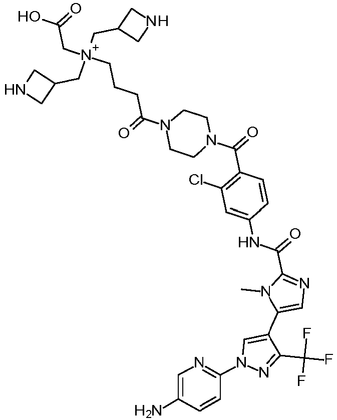 | | PyAOP |
| **51** | 2-[4-[4-[4-[[5-[1-[5-(6-aminohexylamino)-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetic acid .1:1 2,2,2-trifluoroacetate | 911.7 [M]+ | Intermediate E25 and Intermediate H2 |
| | 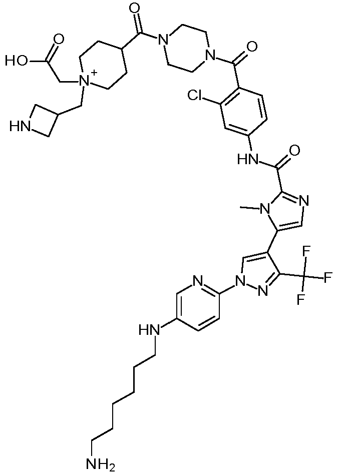 | | PyAOP |
| **53** | 2-[1-(azetidin-3-ylmethyl)-4-[4-[2-chloro-4-[[1-methyl-5-[1-[5-(4-methylpiperazino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid .1:1 formate | 893.6 [M-2H]- | Intermediate E26 and Intermediate H2 |
| | | | PyAOP |
| | 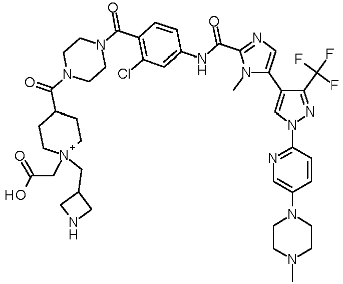 | | |
| **54** | 2-[1-(azetidin-3-ylmethyl)-4-[4-[2-chloro-4-[[5-[1-(5-hydroxy-2-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 813.6 [M]+ | Intermediate E27 and Intermediate H2 |
| | 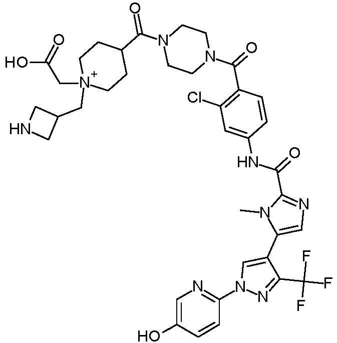 | | PyAOP |
| **55** | cis-2-[1-(azetidin-3-ylmethyl)-4-[4-[2-chloro-4-[[5-[1-(1H-imidazo[4,5-b]pyridin-5-yl)-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 440.4 [M-2H+HCOO]2 - | Intermediate E28 and Intermediate H4 |
| | | | T3P |
| | 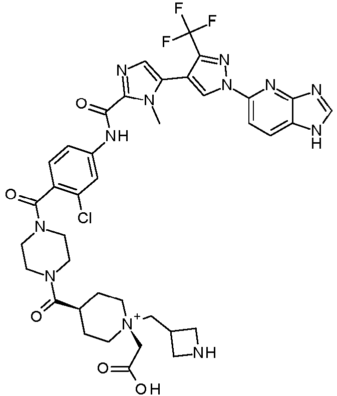 | | |
| **56** | 2-[4-[4-[4-[[5-[1-(6-amino-2-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetate | 812.7 [M]+ | Intermediate E29 and Intermediate H2 |
| | 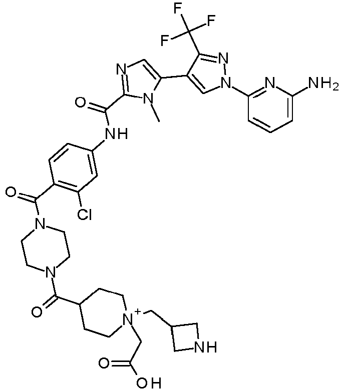 | | T3P |
| **57** | 2-[1-(azetidin-3-ylmethyl)-4-[4-[2-chloro-4-[[5-[1-[5-[[(2S,4S)-4-fluoropyrrolidin-2-yl]methylamino]-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetic acid .1:1 2,2,2-trifluoroacetate | 913.7 [M]+ | Intermediate E30 and Intermediate H2 |
| | | | PyAOP |
| | 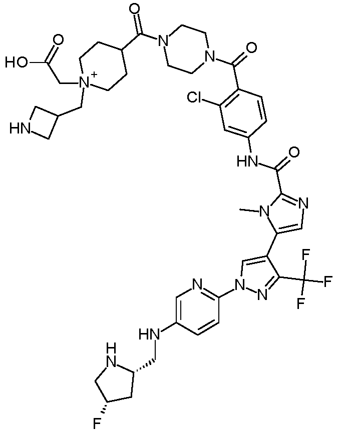 | | |
| **59** | 2-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 formate | 812.4 [M]+ | Intermediate E1 and Intermediate H2 |
| | 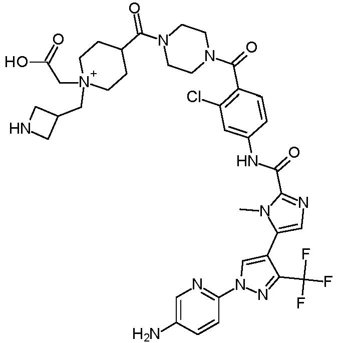 | | PyAOP |
| **60** | 4-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]butyric acid .1:1 2,2,2-trifluoroacetate | 840.6 [M]+ | Intermediate E1 and Intermediate H9 |
| | 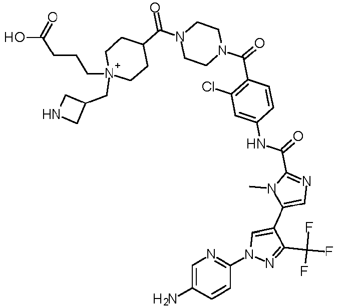 | | PyAOP |
| **61** | 2-[1-(azetidin-3-ylmethyl)-4-[4-[2-chloro-4-[[5-[1-(2,3-dihydro-1H-pyrrolo[3,2-b]pyridin-5-yl)-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 882.8 [M-H+HCOO]- | Intermediate E31 and Intermediate H2 |
| | | | T3P |
| | 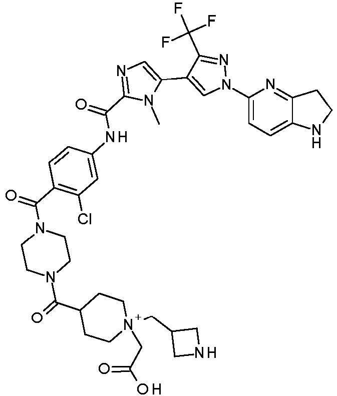 | | |
| **62** | 2-[1-(azetidin-3-ylmethyl)-4-[4-[2-chloro-4-[[5-[1-(1H-indazol-3-yl)-3-(trifluoromethyl)pyrazol-4-yl]-1-methyl-imidazole-2-carbonyl]amino]benzoyl]piperazine-1-carbonyl]piperidin-1-ium-1-yl]acetic acid;formate | 836.6 [M]+ | Intermediate E32 and Intermediate H2 |
| | 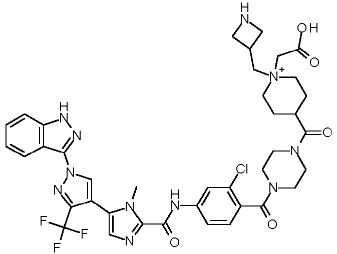 | | HATU |
| **63** | 2-[1-(azetidin-3-ylmethyl)-4-[4-[2-chloro-4-[[5-[1-furo[3,2-b]pyridin-5-yl-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 835.8 [M-2H]- | Intermediate E33 and Intermediate H2 |
| | | | T3P |
| | 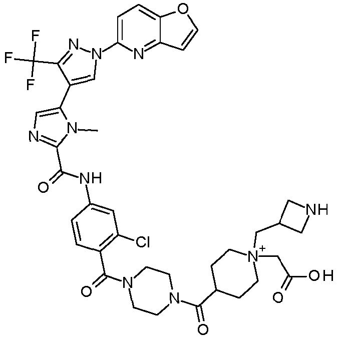 | | |
| **65** | rac-3-aminopropyl-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyl]-(carboxymethyl)-methylammonium .1:1 2,2,2-trifluoroacetate | 788.4 [M]+ | Intermediate E1 and Intermediate H11 |
| | 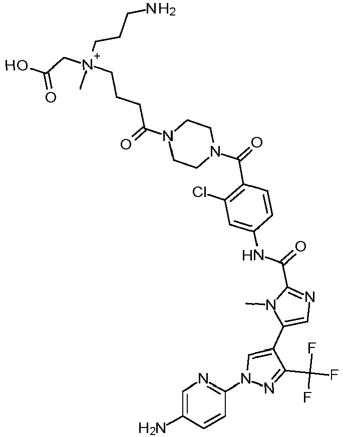 | | PyAOP |
| **66** | bis(3-aminopropyl)-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyl]-(carboxymethyl)ammonium .1:1 2,2,2-trifluoroacetate | 831.4 [M]+ | Intermediate E1 and Intermediate H3 |
| | | | PyAOP |
| | 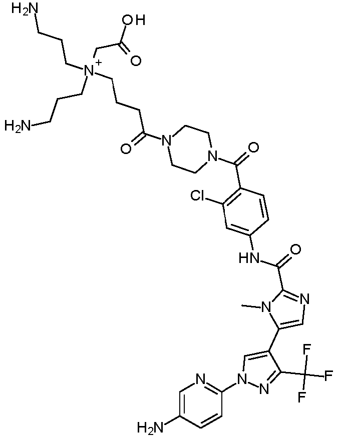 | | |
| **67** | 2-[1-(azetidin-3-ylmethyl)-4-[4-[2-chloro-4-[[5-[1-(5,6-diamino-2-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 871.8 [M-H+HCOO]- | Intermediate E34 and Intermediate H2 |
| | 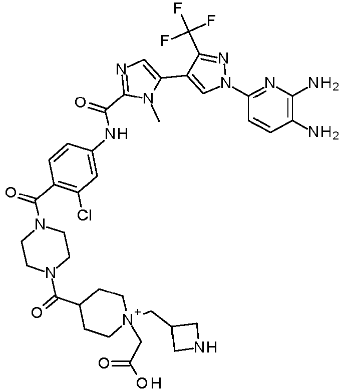 | | T3P |
| **68** | 2-[1-(azetidin-3-ylmethyl)-4-[4-[2-chloro-4-[[1-methyl-5-[1-[6-(methylamino)-3-pyridyl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 826.5 [M]+ | Intermediate E35 and Intermediate H2 |
| | 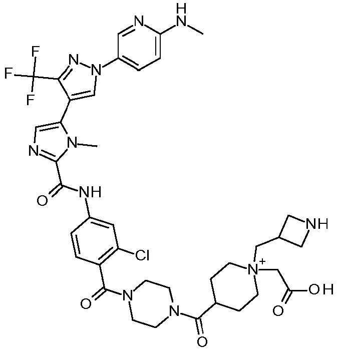 | | PyAOP |
| **69** | 2-[1-(azetidin-3-ylmethyl)-4-[4-[2-chloro-4-[[5-[1-[5-(cyclobutylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 formate | 866.5 [M]+ | Intermediate E36 and Intermediate H2 |
| | | | PyAOP |
| | 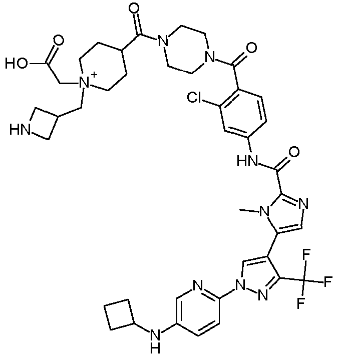 | | |
| **71** | 2-[4-[4-[4-[[5-[1-(4-amino-2-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetate | 812.5 [M]+ | Intermediate E37 and Intermediate H2 |
| | 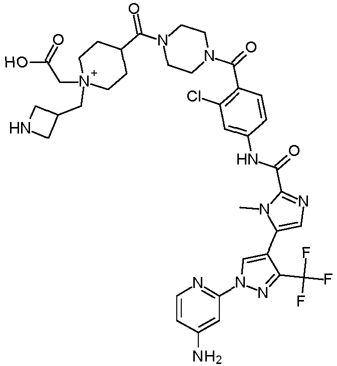 | | PyAOP |
| **72** | 2-[3-aminopropyl-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyl]amino]acetic acid .1:1 2,2,2-trifluoroacetic acid | 774.3 [M+H]⁺ | Intermediate E1 and Intermediate H12 |
| | | | PyAOP |
| | 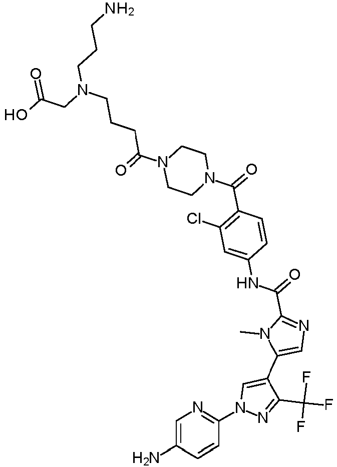 | | |
| **74** | 2-[4-[4-[4-[[5-[1-(2-amino-4-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetate | 812.6 [M+H]⁺ | Intermediate E38 and Intermediate H2 |
| | 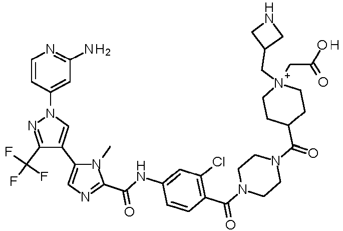 | | PyAOP |
| **75** | 2-[1-(3-aminopropyl)-4-[1-[4-[[5-[1-(5-amino-2-pyridyl)-3-(trifluoromethyl)pyrazol-4-yl]-1-methyl-imidazole-2-carbonyl]amino]-2-chloro-benzoyl]piperidine-4-carbonyl]piperazin-1-ium-1-yl] acetic acid .1:1 2,2,2-trifluoroacetate | 800.4 [M]⁺ | Intermediate F1 and Intermediate J1 |
| | | | PyAOP |
| | 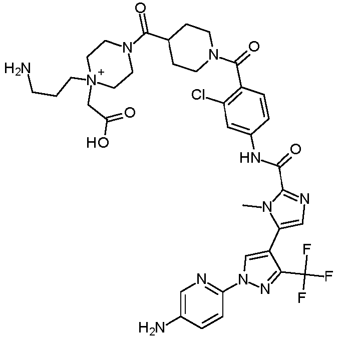 | | |
| **76** | 2-[1-(azetidin-3-ylmethyl)-4-[4-[2-chloro-4-[[5-[1-(5-cyano-2-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 formate | 820.8 [M-2H]⁻ | Intermediate E39 and Intermediate H2 |
| | 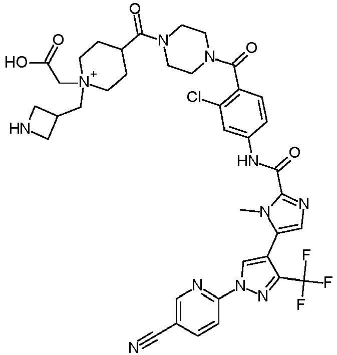 | | PyAOP, in acetonitrile |
| **77** | 2-[4-[(2S)-4-[4-[[5-[1-(5-amino-2-pyridyl)-3-(trifluoromethyl)pyrazol-4-yl]-1-methyl-imidazole-2-carbonyl]amino]-2-chloro-benzoyl]-2-methyl-piperazine-1-carbonyl]-1-(azetidin-3-ylmethyl)piperidin-1-ium-1-yl]acetic acid .1:1 formate | 870.5 [M-H+HCOO]⁻ | Intermediate E40 and Intermediate H2 |
| | | | HATU, in DMF, then HCl in DCM |
| | 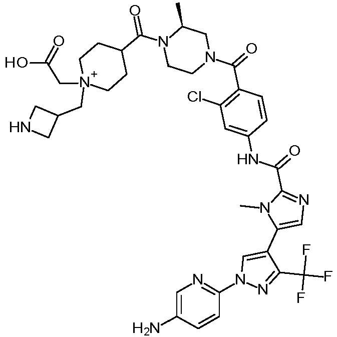 | | |
| **78** | 2-[4-[(3R)-4-[4-[[5-[1-(5-amino-2-pyridyl)-3-(trifluoromethyl)pyrazol-4-yl]-1-methyl-imidazole-2-carbonyl]amino]-2-chloro-benzoyl]-3-ethyl-piperazine-1-carbonyl]-1-(azetidin-3-ylmethyl)piperidin-1-ium-1-yl]acetic acid .1:1 formate | 884.5 [M-H+HCOO]⁻ | Intermediate E41 and Intermediate H2 |
| | 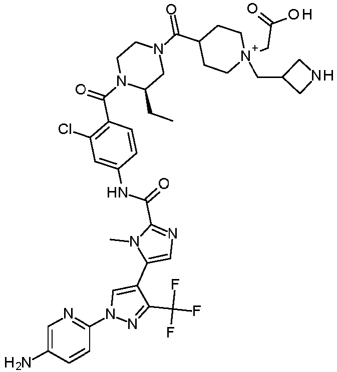 | | HATU, in DMF |
| **79** | 2-[4-[(3S)-4-[4-[[5-[1-(5-amino-2-pyridyl)-3-(trifluoromethyl)pyrazol-4-yl]-1-methyl-imidazole-2-carbonyl]amino]-2-chloro-benzoyl]-3-methyl-piperazine-1-carbonyl]-1-(azetidin-3-ylmethyl)piperidin-1-ium-1-yl]acetic acid .1:1 formate | 870.4 [M-H+HCOO]⁻ | Intermediate E42 and Intermediate H2 |
| | | | HATU, in DMF |
| | 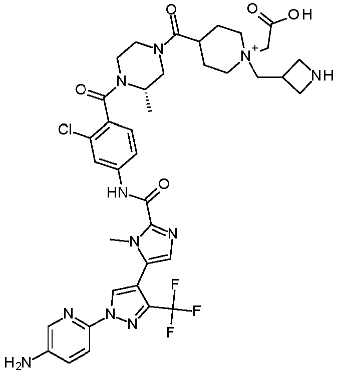 | | |
| **80** | 2-[1-(azetidin-3-ylmethyl)-4-[4-[4-[[5-[1-(1,3-benzothiazol-2-yl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid .1:1 formate | 851.6 [M-2H]⁻ | Intermediate E43 and Intermediate H2 |
| | 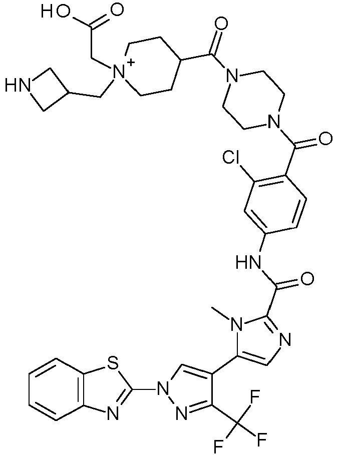 | | PyAOP |
| **82** | 2-[1-(azetidin-3-ylmethyl)-4-[4-[2-chloro-4-[[5-[1-(6-methoxy-3-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 825.8 [M-2H]⁻ | Intermediate E44and Intermediate H2 |
| | 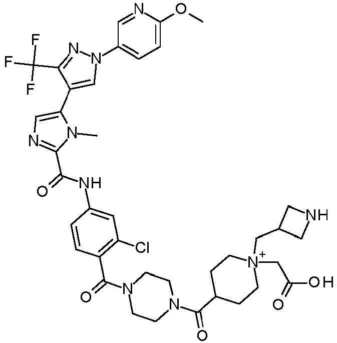 | | T3P |
| **83** | (2S)-2-amino-4-[4-[4-[[5-[1-(5-amino-2-pyridyl)-3-(trifluoromethyl)pyrazol-4-yl]-1-methyl-imidazole-2-carbonyl]amino]-2-chlorobenzoyl]piperazino]-4-keto-butyric acid .1:1 2,2,2-trifluoroacetic acid | 689.5 [M+H]⁺ | Intermediate E1 and (3 S)-4-tert-butoxy-3-(tert-butoxycarbonylamino)-4-keto-butyric acid |
| | | | PyAOP |
| | 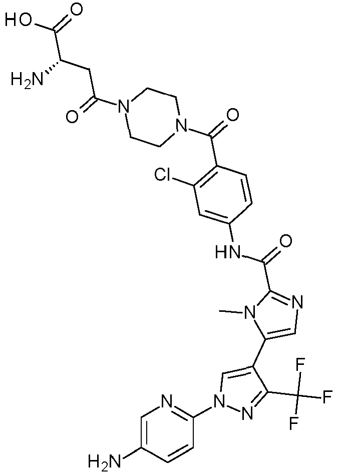 | | |
| **84** | 2-[1-(azetidin-3-ylmethyl)-4-[4-[2-chloro-4-[[5-[1-[5-[(dimethylamino)methyl]-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 formic acid .1:1 formate | 898.6 [M-H+HCOO]⁻ | Intermediate E45 and Intermediate H2 |
| | 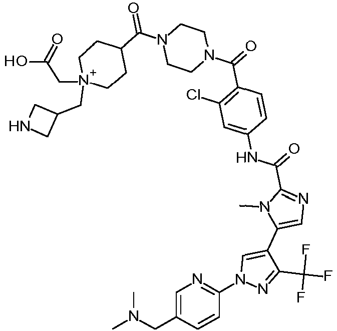 | | PyAOP |
| **85** | (3R)-3-amino-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyric acid .1:1 2,2,2-trifluoroacetic acid | 689.6 [M+H]⁺ | Intermediate E1 and (2R)-4-tert-butoxy-2-(tert-butoxycarbonylamino)-4-keto-butyric acid |
| | 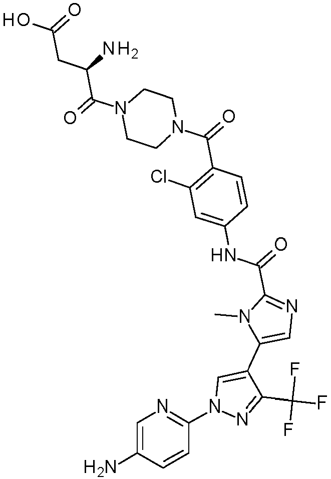 | | PyAOP |
| **86** | 2-[4-[7-[4-[[5-[1-(5-amino-2-pyridyl)-3-(trifluoromethyl)pyrazol-4-yl]-1-methyl-imidazole-2-carbonyl]amino]-2-chloro-benzoyl]-4,7- | 838.6 [M]⁺ | Intermediate E46 and Intermediate H2 |
| | diazaspiro[2.5]octane-4-carbonyl]-1-(azetidin-3-ylmethyl)piperidin-1-ium-1-yl]acetic acid .1:1 formate | | HATU, in DMF |
| | 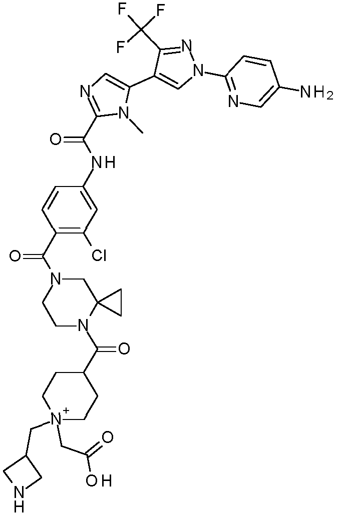 | | |
| **87** | 2-[4-[1-[4-[[5-[1-(5-amino-2-pyridyl)-3-(trifluoromethyl)pyrazol-4-yl]-1-methyl-imidazole-2-carbonyl]amino]-2-chloro-benzoyl]piperidine-4-carbonyl]-1-(azetidin-3-ylmethyl)piperazin-1-ium-1-yl]acetic acid .1:1 2,2,2-trifluoroacetate | 812.4 [M]⁺ | Intermediate F1 and Intermediate J2 |
| | 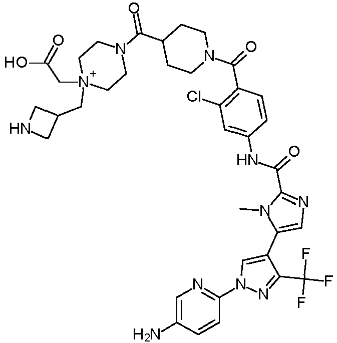 | | PyAOP |
| **88** | 2-[1-(azetidin-3-ylmethyl)-4-[4-[2-chloro-4-[[1-methyl-5-[1-[2-(methylamino)-4-pyridyl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 826.6 [M]⁺ | Intermediate E47 and Intermediate H2 |
| | | | PyAOP |
| | 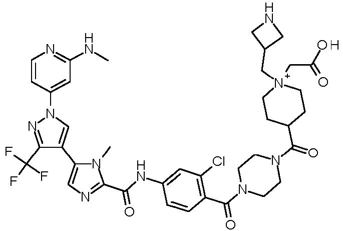 | | |
| **89** | 2-[4-[8-[4-[[5-[1-(5-amino-2-pyridyl)-3-(trifluoromethyl)pyrazol-4-yl]-1-methyl-imidazole-2-carbonyl]amino]-2-chloro-benzoyl]-3,8-diazabicyclo[3.2.1]octane-3-carbonyl]-1-(azetidin-3-ylmethyl)piperidin-1-ium-1-yl]acetic acid .1:1 formate | 838.5 [M]⁺ | Intermediate E48 and Intermediate H2 |
| | | | HATU, in DMF |
| | 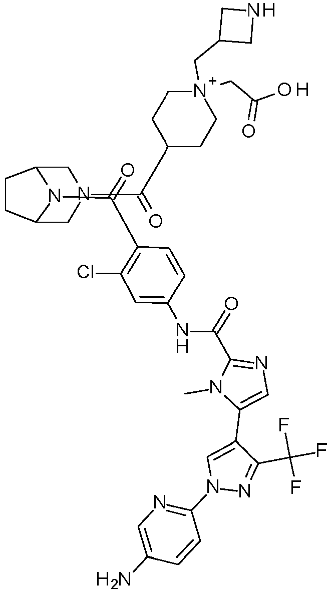 | | |
| **90** | (2S)-2-amino-5-[4-[4-[[5-[1-(5-amino-2-pyridyl)-3-(trifluoromethyl)pyrazol-4-yl]-1-methyl-imidazole-2-carbonyl]amino]-2-chlorobenzoyl]piperazino]-5-keto-valeric acid .1:1 2,2,2-trifluoroacetic acid | 703.6 [M+H]⁺ | Intermediate E1 and (4S)-5-tert-butoxy-4-(tert-butoxycarbonylamino)-5-keto-valeric acid |
| | 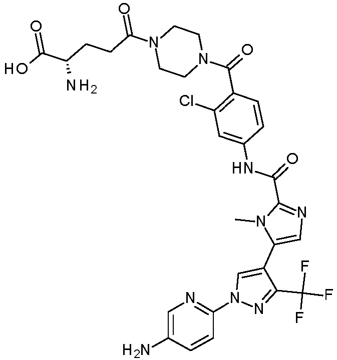 | | PyAOP |
| **91** | (3S)-3-amino-4-[4-[4-[[5-[1-(5-amino-2-pyridyl)-3-(trifluoromethyl)pyrazol-4-yl]-1-methyl-imidazole-2-carbonyl]amino]-2-chlorobenzoyl]piperazino]-4-keto-butyric acid .1:1 2,2,2-trifluoroacetic acid | 689.5 [M+H]⁺ | Intermediate E1 and (2S)-4-tert-butoxy-2-(tert-butoxycarbonylamino)-4-keto-butyric acid |
| | 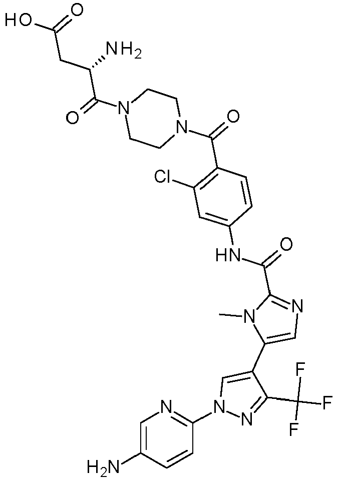 | | PyAOP |
| **92** | 2-[4-[3-[4-[[5-[1-(5-amino-2-pyridyl)-3-(trifluoromethyl)pyrazol-4-yl]-1-methyl-imidazole-2-carbonyl]amino]-2-chloro-benzoyl]-3,9-diazabicyclo[3.3.1]nonane-9-carbonyl]-1-(azetidin-3-ylmethyl)piperidin-1-ium-1-yl]acetic acid .1:1 formate | 896.6 [M-H+HCOO]⁻ | Intermediate E49 and Intermediate H2 |
| | 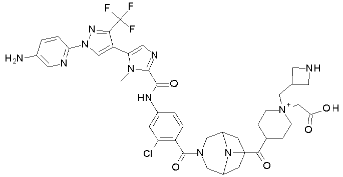 | | HATU, in DMF |
| **93** | 2-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]-4,7-diazaspiro[2.5]octane-7-carbonyl]-1-(azetidin-3-ylmethyl)piperidin-1-ium-1-yl]acetic acid .1:1 formate | 882.3 [M-H+HCOO]⁻ | Intermediate E50 and Intermediate H2 |
| | 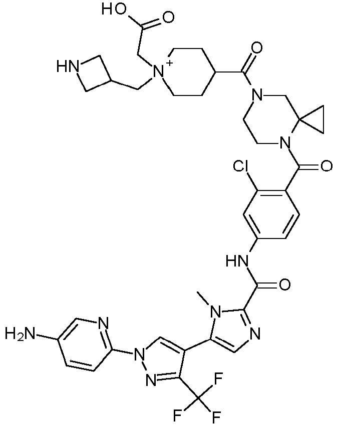 | | HATU, in DMF |
| **97** | 2-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]-2-(methoxymethyl)piperazine-1-carbonyl]-1-(azetidin-3-ylmethyl)piperidin-1-ium-1-yl]acetic acid .1:1 formate | 900.4 [M-H+HCOO]⁻ | Intermediate E52 and Intermediate H2 |
| | | | HATU, in DMF |
| | 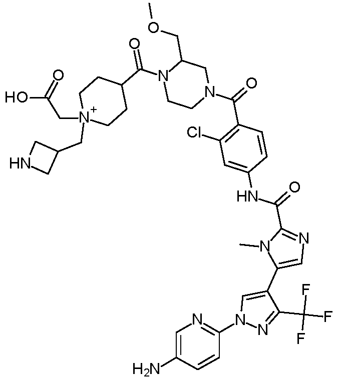 | | |
| **98** | cis-2-[1-(3-aminopropyl)-4-[4-[2-chloro-4-[[5-[1-[2-(3-hydroxypropyl)-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;chloride | 882.6 [M]⁺ | Intermediate E54 and Intermediate H6 |
| | 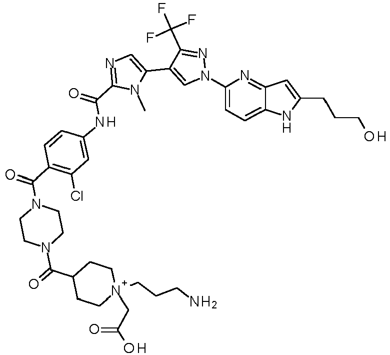 | | HATU, in DMF, then TFA in DCM |
| **99** | cis-2-[4-[4-[4-[[5-[1-[2-(3-amino-3-oxo-propyl)-1H-pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(3-aminopropyl)piperidin-1-ium-1-yl]acetic acid;2,2,2-trifluoroacetate | 895.5 [M]⁺ | Intermediate E55 and Intermediate H6 |
| | | | HATU, in DMF, then TFA in DCM |
| | 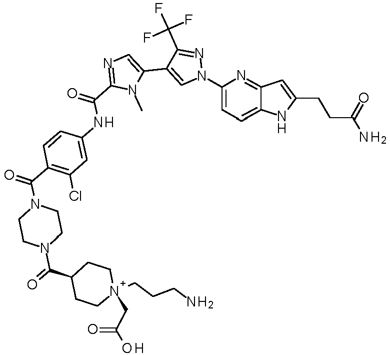 | | |
| **100** | cis-2-[1-(3-aminopropyl)-4-[4-[2-chloro-4-[[5-[1-(2-fluoro-4-morpholino-phenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;formate | 887.4 [M]⁺ | Intermediate E56 and Intermediate H6 |
| | 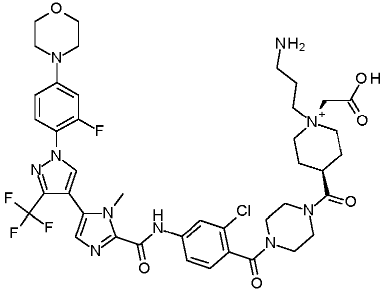 | | HATU, in DMF, then TFA in DCM |
| **101** | cis-2-[1-(3-aminopropyl)-4-[4-[4-[[5-[1-[2-[(1S)-1-hydroxyethyl]-1H-pyrrolo[3,2-b]pyridin-5-yl]-3-(trifluoromethyl)pyrazol-4-yl]-1-methyl-imidazole-2-carbonyl]amino]-2-methyl-benzoyl]piperazine-1-carbonyl]piperidin-1-ium-1-yl]acetic acid;chloride | 848.6 [M]⁺ | Intermediate E57 and Intermediate H6 |
| | | | HATU, in DMF, then TFA in DCM |
| | 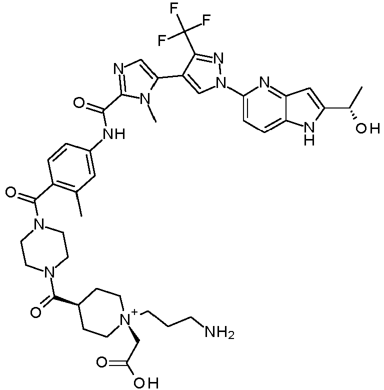 | | |
| **102** | cis-2-[1-(3-aminopropyl)-4-[4-[2-chloro-4-[[5-[1-[2-[2-hydroxy-1-(hydroxymethyl)ethyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;formate | 898.4 [M]⁺ | Intermediate E58 and Intermediate H6 |
| | 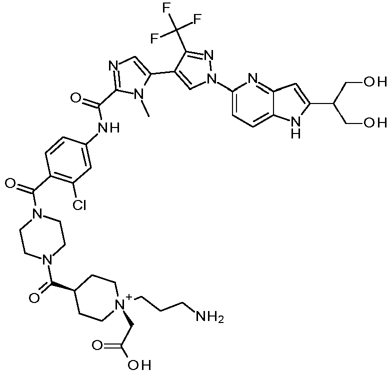 | | HATU, in DMF, then TFA in DCM |
| **103** | cis-2-[1-(3-aminopropyl)-4-[4-[2-chloro-4-[[1-methyl-5-[1-[1-methyl-2-(morpholine-4-carbonyl)pyrrolo[3,2-b]pyridin-5-yl]-3-(trifluoromethyl)pyrazol-4-yl]imidazole-2-carbonyl]amino]benzoyl]piperazine-1-carbonyl]piperidin-1-ium-1-yl]acetic acid;formate | 951.2 [M]⁺ | Intermediate E59 and Intermediate H6 |
| | 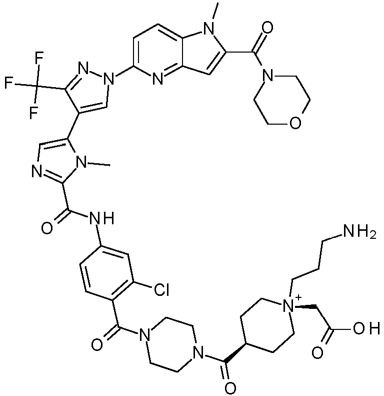 | | HATU, in DMF, then TFA in DCM |
| **104** | cis-2-[1-(3-aminopropyl)-4-[4-[2-chloro-4-[[5-[1-[2-(2-hydroxyethylcarbamoyl)-1-methyl-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;chloride | 925.2 [M]⁺ | Intermediate E60 and Intermediate H6 |
| | | | HATU, in DMF, then TFA in DCM |
| | 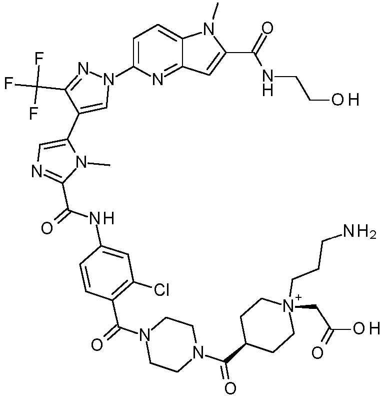 | | |
| **105** | cis-2-[1-(azetidin-3-ylmethyl)-4-[4-[2-chloro-4-[[5-[1-[2-[(1S)-1,2-dihydroxyethyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;2,2,2-trifluoroacetate | 897.2 [M]⁺ | Intermediate E61 and Intermediate H4 |
| | | | HATU, in DMF, then HCl in dioxane |
| | 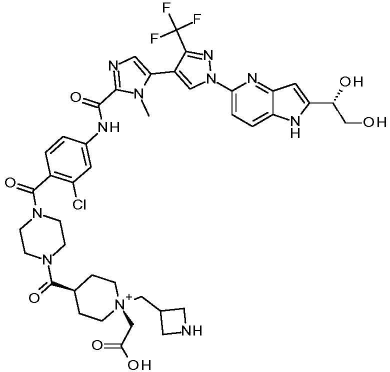 | | RPHPLC (Phenomenex Luna C18 150*25mm*10m; 20-50% ACN in water, 0.225% TFA) |
| **106** | cis-2-[1-(azetidin-3-ylmethyl)-4-[4-[2-chloro-4-[[5-[1-[2-[(1R)-1,2-dihydroxyethyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;chloride | 896.4 [M]⁺ | Intermediate E61 and Intermediate H4 |
| | 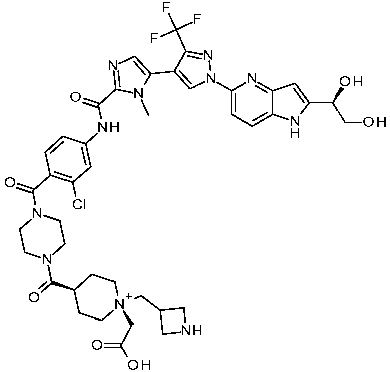 | | HATU, in DMF, then HCl in dioxane |
| | | | RPHPLC (Phenomenex Luna C18 150*25mm*10µm ; 20-50% ACN in water, 0.225% TFA), followed by a second RPHPLC (Welch Xtimate C18 150*25mm*5µm; 20-40% ACN in water, 0.225% HCl)) |
| **107** | cis-2-[1-(azetidin-3-ylmethyl)-4-[4-[4-[[5-[1-[2-fluoro-4-(methylamino)phenyl]-3-(trifluoromethyl)pyrazol-4-yl]-1-methyl-imidazole-2-carbonyl]amino]-2-methyl-benzoyl]piperazine-1-carbonyl]piperidin-1-ium-1-yl]acetic acid;2,2,2-trifluoroacetate | 823.5 [M]⁺ | Intermediate E62 and Intermediate H4 |
| | | | HATU, in DMF, then TFA in DCM |
| | 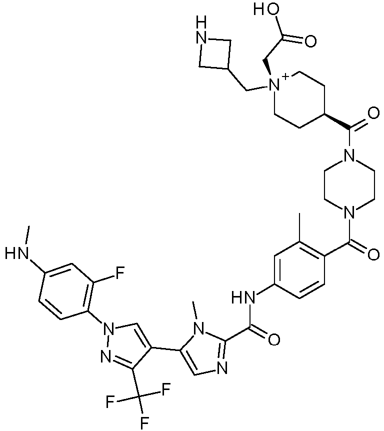 | | |
| **111** | cis-2-[1-(azetidin-3-ylmethyl)-4-[4-[4-[[5-[1-[2-fluoro-4-(2-methoxyethylamino)phenyl]-3-(trifluoromethyl)pyrazol-4-yl]-1-methyl-imidazole-2-carbonyl]amino]-2-methyl-benzoyl]piperazine-1-carbonyl]piperidin-1-ium-1-yl]acetic acid;2,2,2-trifluoroacetate | 867.4 [M]⁺ | Intermediate E63 and Intermediate H4 |
| | 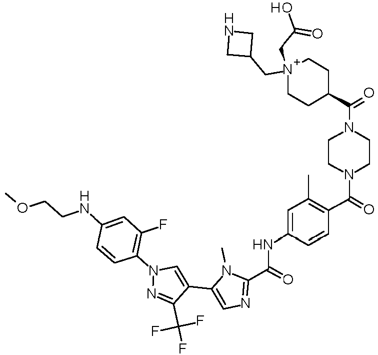 | | HATU, in DMF, then TFA in DCM |
| **112** | cis-2-[4-[4-[4-[[5-[1-[2-[(3aS,6aR)-2,3,3a,4,6,6a-hexahydro-1H-pyrrolo[3,4-c]pyrrole-5-carbonyl]-1H-pyrrolo[3,2-c]pyridin-6-yl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetate | 974.8 [M]⁺ | Intermediate G8 and (3aS,6aR)-2,3,3a,4,6,6a-hexahydro-1H-pyrrolo[3,4-c]pyrrole-5-carboxylic acid tert-butyl ester (CAS 2803135-37-5) |
| | 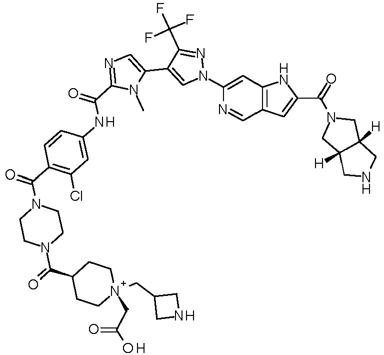 | | HATU, in DMF, then TFA in DCM |
| **113** | cis-2-[1-(azetidin-3-ylmethyl)-4-[4-[2-chloro-4-[[1-methyl-5-[1-[2-(7-oxa-2-azaspiro[3.5]nonane-2-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 989.7 [M]⁺ | Intermediate G8 and 7-oxa-2-azaspiro[3.5]nonane (CAS 194157-10-3) |
| | | | HATU, in DMF, then TFA in DCM |
| | 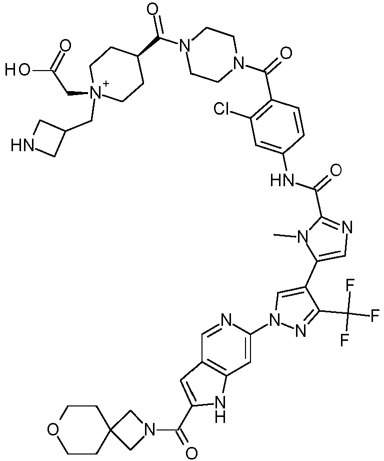 | | |
| **114** | cis-2-[1-(azetidin-3-ylmethyl)-4-[4-[2-chloro-4-[[5-[1-[2-(1-imino-1-keto-1,4-thiazinane-4-carbonyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 996.6 [M]⁺ | Intermediate G8 and 1-imino-1λ6-thiomorpholin-1-one (CAS 1621962-33-1) |
| | 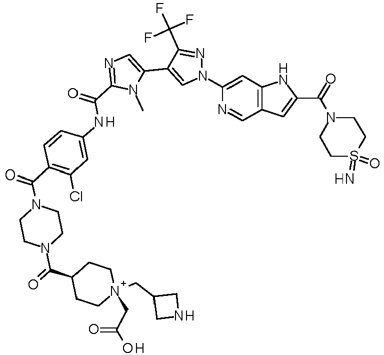 | | HATU, in DMF, then TFA in DCM |
| **115** | cis-2-[1-(azetidin-3-ylmethyl)-4-[4-[2-chloro-4-[[5-[1-[2-(2-hydroxyethylcarbamoyl)-1H- pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 923.5 [M]⁺ | Intermediate G8 and 2-(tert-butyldimethylsilyloxy)e thanamine (CAS 101711-55-1) |
| | 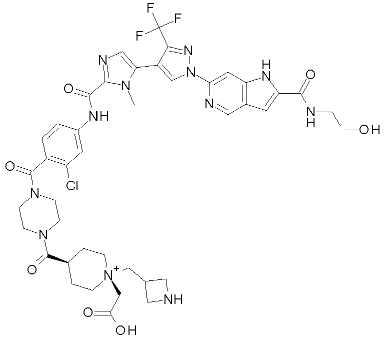 | | HATU, in DMF, then TFA in DCM |
| **116** | cis-2-[1-(azetidin-3-ylmethyl)-4-[4-[2-chloro-4-[[1-methyl-5-[1-[2-(9-oxa-3,7-diazabicyclo[3.3.1]nonane-3-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetic acid .1:1 2,2,2-trifluoroacetate | 990.5 [M]⁺ | Intermediate G8 and 9-oxa-3,7-diazabicyclo[3.3.1]nona ne-3-carboxylic acid tert-butyl ester (CAS 478647-20-0) |
| | | | HATU, in DMF, then TFA in DCM |
| | 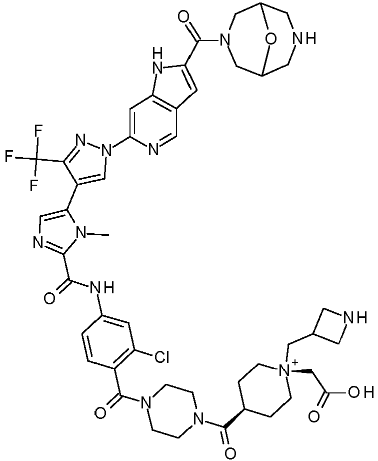 | | |
| **117** | cis-2-[1-(azetidin-3-ylmethyl)-4-[4-[2-chloro-4-[[1-methyl-5-[1-[2-[(3,3,3-trifluoro-2-hydroxypropyl)carbamoyl]-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 991.7 [M]⁺ | Intermediate G8 and 3-amino-1,1,1-trifluoropropan-2-ol (CAS 431-38-9) |
| | 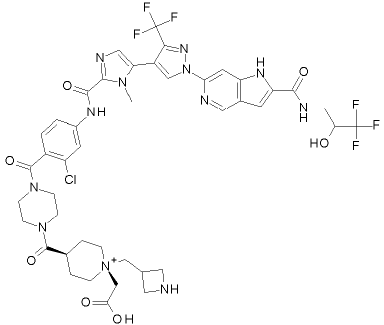 | | HATU, in DMF, then TFA in DCM |
| **118** | cis-2-[1-(azetidin-3-ylmethyl)-4-[4-[2-chloro-4-[[1-methyl-5-[1-[2-(piperazine-1-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 948.7 [M]⁺ | Intermediate G8 and tert-butyl piperazine-1-carboxylate |
| | | | HATU, in DMF, then TFA in DCM |
| | 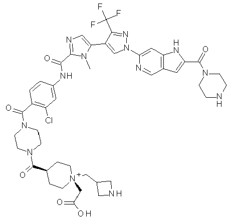 | | |
| **119** | cis-2-[1-(azetidin-3-ylmethyl)-4-[4-[2-chloro-4-[[5-[1-[2-(2,2-difluoromorpholine-4-carbonyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 985.7 [M]⁺ | Intermediate G8 and 2,2-difluoromorpholine (CAS 1263180-85-3) |
| | | | HATU, in DMF, then TFA in DCM |
| | 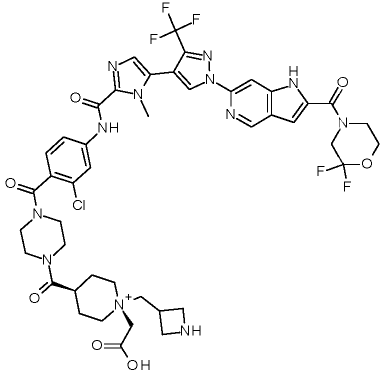 | | |
| **120** | cis-2-[1-(azetidin-3-ylmethyl)-4-[4-[2-chloro-4-[[5-[1-[2-[(2-ketopyrrolidin-3-yl)carbamoyl]-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 962.5 [M]⁺ | Intermediate G8 and 3-amino-2-pyrrolidone (CAS 2483-65-0) |
| | 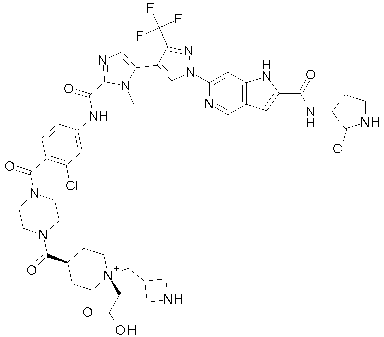 | | HATU, in DMF, then TFA in DCM |
| **121** | cis-2-[1-(azetidin-3-ylmethyl)-4-[4-[2-chloro-4-[[5-[1-[2-[(5R)-2,6-dioxa-9-azaspiro[4.5]decane-9-carbonyl]-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 1005.6 [M]⁺ | Intermediate G8 and (5R)-2,6-dioxa-9-azaspiro[4.5]decane (CAS 2382202-76-6) |
| | 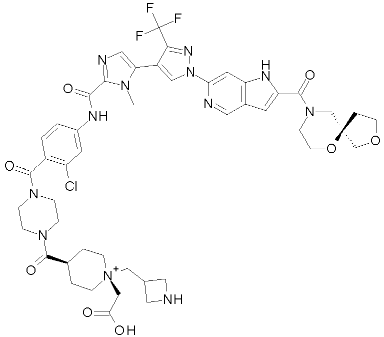 | | HATU, in DMF, then TFA in DCM |
| **122** | cis-2-[1-(azetidin-3-ylmethyl)-4-[4-[2-chloro-4-[[1-methyl-5-[1-[2-(thiomorpholine-4-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 965.6 [M]⁺ | Intermediate G8 and thiomorpholine |
| | 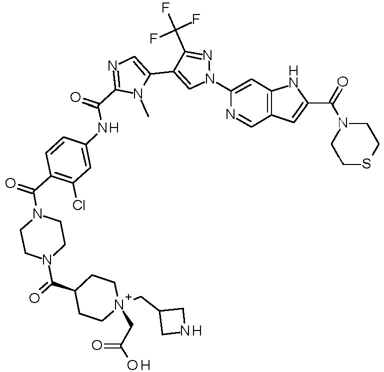 | | HATU, in DMF, then TFA in DCM |
| **123** | cis-2-[1-(azetidin-3-ylmethyl)-4-[4-[2-chloro-4-[[5-[1-[2-(dimethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 907.6 [M]⁺ | Intermediate G8 and dimethylamine, 2M in THF |
| | 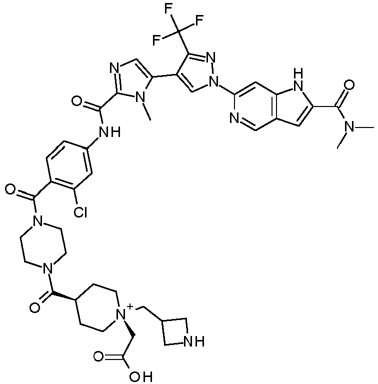 | | HATU, in DMF, then TFA in DCM |
| **125** | cis-2-[1-(azetidin-3-ylmethyl)-4-[4-[2-chloro-4-[[5-[1-[2-(hydroxycarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 895.5 [M]⁺ | Intermediate G8 and O-tetrahydropyran-2-ylhydroxylamine (CAS 6723-30-4) |
| | | | HATU, in DMF, then TFA in DCM |
| | 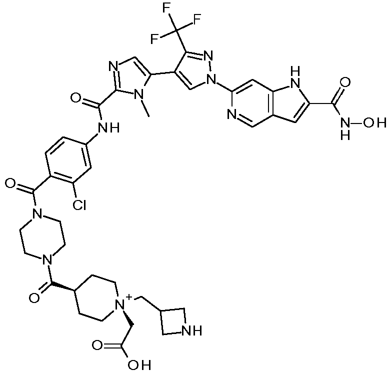 | | |
| **126** | cis-2-[1-(azetidin-3-ylmethyl)-4-[4-[2-chloro-4-[[5-[1-[4-[2-(difluoromethoxy)ethylamino]-2-fluoro-phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;formate | 923.4 [M]⁺ | Intermediate E64 and Intermediate H4 |
| | 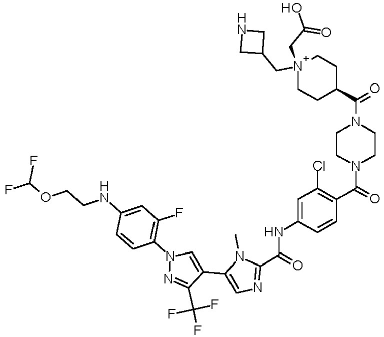 | | HATU, in DMF, then TFA in DCM |
| **131** | cis-2-[1-(3-aminopropyl)-4-[4-[2-fluoro-4-[[5-[1-(2-fluoro-4-hydroxyphenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;formate | 802.4 [M]⁺ | Intermediate E53 and Intermediate H6 |
| | | | HATU, in DMF, then TFA in DCM |
| | 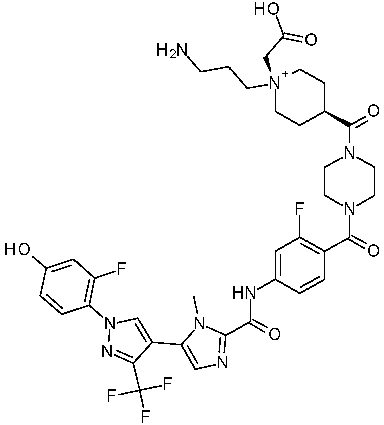 | | |
| **134** | 2-[1-(3-aminopropyl)-4-[1-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]isonipecotoyl ]piperazin-1-ium-1-yl]acetic acid .1:1 2,2,2-trifluoroacetate | 854.55 [M]⁺ | Intermediate C11 and Intermediate I4 |
| | 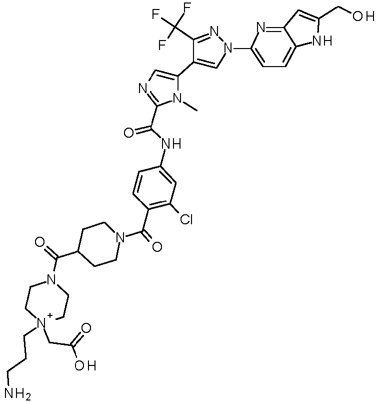 | | HATU, in DMF |
| **135** | 2-[4-[1-[4-[[5-[1-(4-amino-2-fluorophenyl)-3-(trifluoromethyl)pyrazol-4-yl]-1-methyl-imidazole-2-carbonyl]amino]-2-chlorobenzoyl]isonipecotoyl]-1-(3-aminopropyl)piperazin-1-ium-1-yl]acetic acid .1:1 2,2,2-trifluoroacetate | 817.51 [M]⁺ | Intermediate C12 and Intermediate I2 |
| | 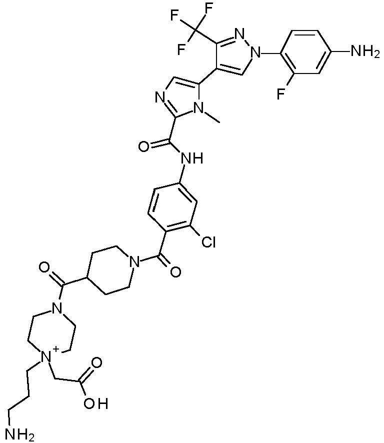 | | HATU, in DMF |
| **136** | cis-2-[1-(azetidin-3-ylmethyl)-4-[4-[2-fluoro-4-[[5-[1-(2-fluoro-4-hydroxyphenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;formate | 814.3 [M]⁺ | Intermediate E53 and Intermediate H4 |
| | | | HATU, in DMF, then TFA in DCM |
| | 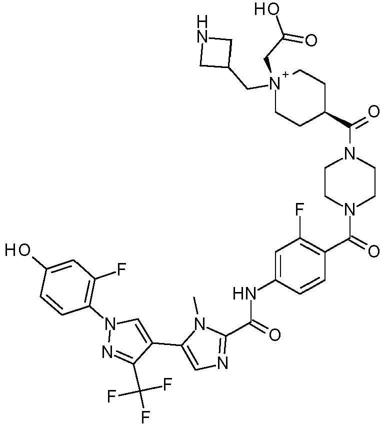 | | |
| **137** | cis-2-[1-(azetidin-3-ylmethyl)-4-[4-[2-chloro-4-[[5-[1-[4-[[(2S)-2,3-dihydroxypropyl]amino]-2-fluorophenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;formate | 903.4 [M]⁺ | Intermediate E66 and Intermediate H4 |
| | 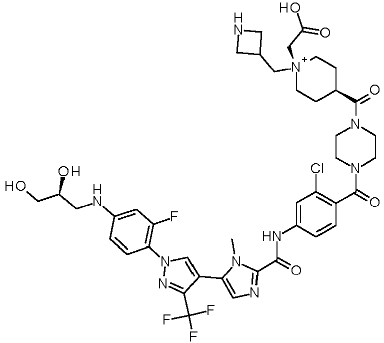 | | HATU, in DMF, then TFA in DCM |
| **138** | cis-2-[4-[4-[4-[[5-[1-[1-(2-amino-1,1-dimethyl-2-oxo-ethyl)pyrrolo[3,2-b]pyridin-5-yl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;chloride | 921.6 [M]⁺ | Intermediate E67 and Intermediate H4 |
| | | | HATU, in DMF, then TFA in DCM |
| | 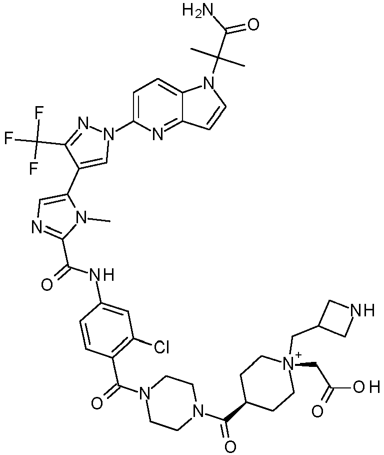 | | |
| **140** | 2-[4-[4-[4-[[5-[1-(4-amino-2-fluorophenyl)-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperazin-1-ium-1-yl]acetic acid .1:1 2,2,2-trifluoroacetate | 830.39 [M]⁺ | Intermediate C12 and Intermediate I3 |
| | | | HATU, in DMF |
| | 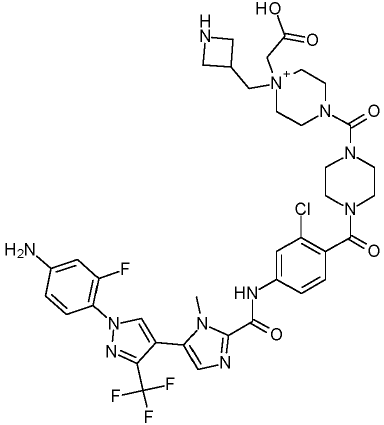 | | |
| **141** | 2-[4-[4-[4-[[5-[1-(4-amino-2-fluorophenyl)-3-(trifluoromethyl)pyrazol-4-yl]-1-methyl-imidazole-2-carbonyl]amino]-2-chlorobenzoyl]piperazine-1-carbonyl]-1-(3-aminopropyl)piperazin-1-ium-1-yl]acetic acid .1:1 2,2,2-trifluoroacetate | 409.98 [M+H]²⁺ | Intermediate C12 and Intermediate I4 |
| | 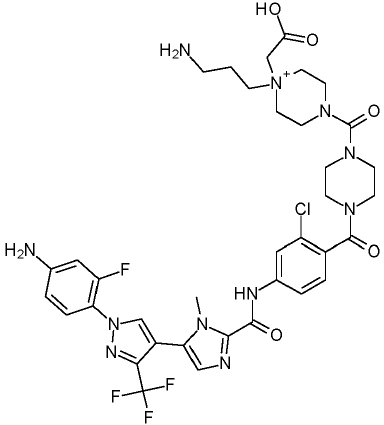 | | HATU, in DMF |
| **142** | cis-2-[4-[4-[4-[[5-[1-[2-(2-aminoethyl)-1H-pyrrolo[3,2-c]pyridin-6-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;formate | 879.6 [M]⁺ | Intermediate C13 and Intermediate I5 |
| | 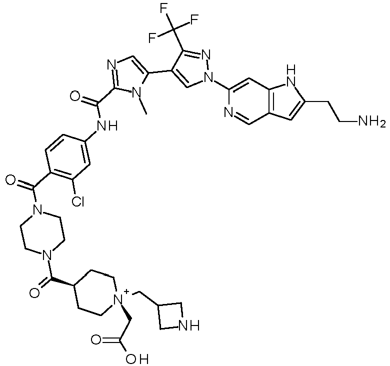 | | HATU, in DMF, then trifluoromethanesulfoni c acid in TFA |
| **144** | 2-[1-(azetidin-3-ylmethyl)-4-[4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperazin-1-ium-1-yl]acetic acid .1:1 2,2,2-trifluoroacetate | 867.39 [M]⁺ | Intermediate C11 and Intermediate I3 |
| | 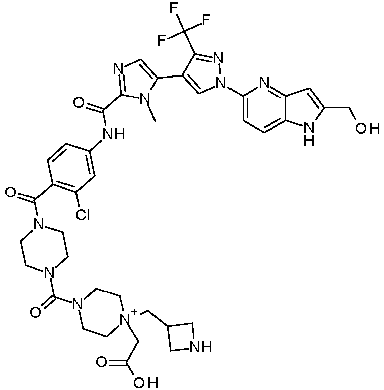 | | HATU, in DMF |
| **145** | 2-[1-(3-aminopropyl)-4-[4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperazin-1-ium-1-yl] acetic acid .1:1 2,2,2-trifluoroacetate | 855.42 [M]⁺ | Intermediate C11 and Intermediate I4 |
| | 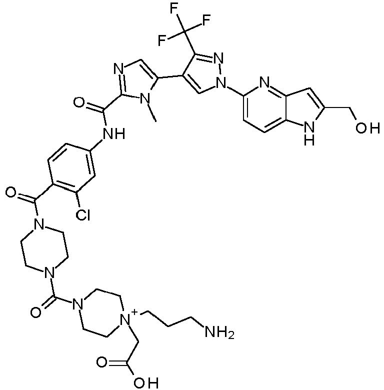 | | HATU, in DMF |
| **151** | cis-2-[1-(azetidin-3-ylmethyl)-4-[4-[2-fluoro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 850.5 [M]⁺ | Intermediate E68 and Intermediate H4 |
| | | | HATU, in DMF |
| | 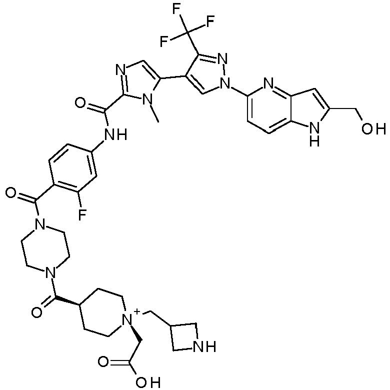 | | |
| **152** | cis-2-[4-[4-[4-[[5-[1-(4-amino-2-fluoro-phenyl)-3-(trifluoromethyl)pyrazol-4-yl]-1-methyl-imidazole-2-carbonyl]amino]-2-methyl-benzoyl]piperazine-1-carbonyl]-1-(azetidin-3-ylmethyl)piperidin-1-ium-1-yl]acetic acid .1:1 2,2,2-trifluoroacetate | 853.6 [M-H+HCOO]⁻ | Intermediate E69 and Intermediate H4 |
| | 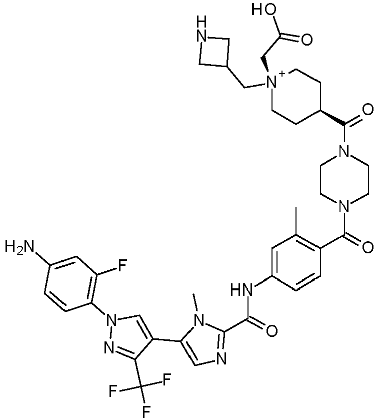 | | HATU, in DMF |
| **150** | cis-2-[1-(azetidin-3-ylmethyl)-4-[4-[2-chloro-4-[[5-[1-[2-[(1R)-1-hydroxyethyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;formate | 880.3 [M]⁺ | Intermediate E70 and Intermediate H4 |
| | 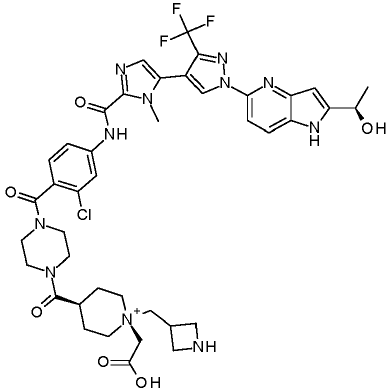 | | HATU, in DMF, then TFA in DCM |
| **153** | cis-2-[1-(azetidin-3-ylmethyl)-4-[4-[2-methyl-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 844.8 [M-2H]⁻ | Intermediate E71 and Intermediate H4 |
| | | | HATU, in DMF |
| | 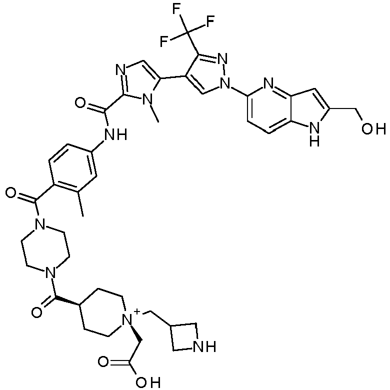 | | |
| **154** | cis-2-[1-(azetidin-3-ylmethyl)-4-[4-[2-chloro-4-[[5-[1-[2-(2-hydroxyethyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;formate | 880.3 [M]⁺ | Intermediate E72 and Intermediate H4 |
| | 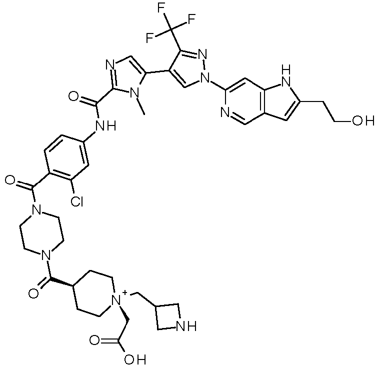 | | HATU, in DMF, then TFA in DCM |
| **158** | cis-2-[1-(azetidin-3-ylmethyl)-4-[4-[2-chloro-4-[[5-[1-[1-(dimethylsulfamoyl)-2-(2-hydroxyethyl)pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;formate | 987.5 [M]⁺ | Intermediate E73 and Intermediate H4 |
| | 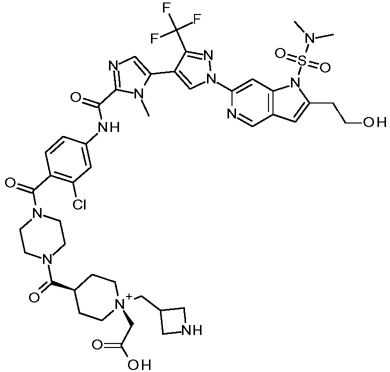 | | HATU, in DMF, then TFA in DCM, then K2CO3 in water |
| **160** | cis-2-[1-(azetidin-3-ylmethyl)-4-[4-[2-chloro-4-[[5-[1-[2-[(2S)-2,3-dihydroxypropyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;formate | 910.4 [M]⁺ | Intermediate E84 and Intermediate H4 |
| | 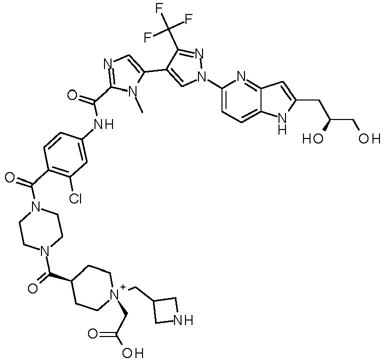 | | HATU, in DMF, then TFA in DCM |
| **161** | 2-[4-[4-[4-[[5-[1-[1-(3-amino-2-methyl-3-oxo-propyl)pyrrolo[3,2-b]pyridin-5-yl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;2,2,2-trifluoroacetate | 921.4 [M]⁺ | Intermediate E74 and Intermediate H4 |
| | 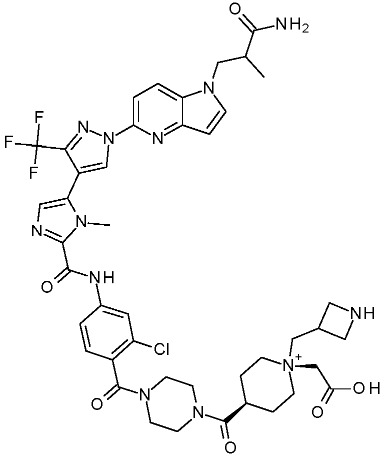 | | HATU, in DMF, then TFA in DCM |
| **162** | cis-2-[1-(azetidin-3-ylmethyl)-4-[4-[2-chloro-4-[[5-[1-[2-(2-hydroxyethyl)-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;formate | 880.4 [M]⁺ | Intermediate E85 and Intermediate H4 |
| | | | HATU, in DMF, then TFA in DCM |
| | 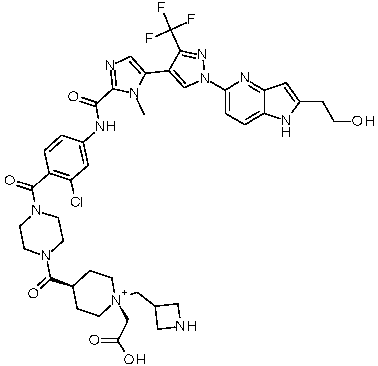 | | |
| **164** | cis-2-[1-(azetidin-3-ylmethyl)-4-[4-[2-chloro-4-[[5-[1-[2-fluoro-4-[(2-hydroxy-2-methylpropyl)amino]phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;formate | 901.3 [M]⁺ | Intermediate E76 and Intermediate H4 |
| | 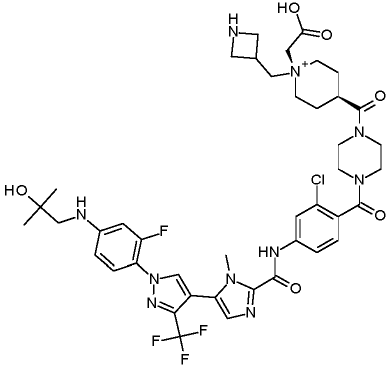 | | HATU, in DMF, then TFA in DCM |
| **165** | cis-2-[1-(azetidin-3-ylmethyl)-4-[4-[2-chloro-4-[[1-methyl-5-[1-(5H-pyrrolo[3,2-d]pyrimidin-2-yl)-3-(trifluoromethyl)pyrazol-4-yl]imidazole-2-carbonyl]amino]benzoyl]piperazine-1-carbonyl]piperidin-1-ium-1-yl]acetic acid;formate | 837.2 [M]⁺ | Intermediate E77 and Intermediate H4 |
| | | | HATU, in DMF, then TFA in DCM |
| | 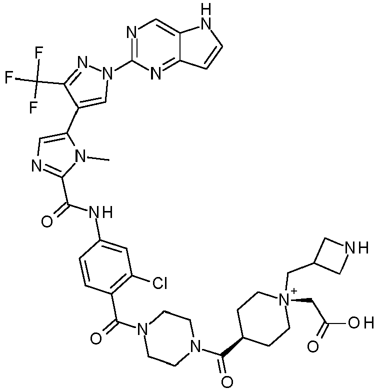 | | |
| **166** | cis-2-[1-(azetidin-3-ylmethyl)-4-[4-[4-[[5-[1-[5-[2-[bis(2-aminoethyl)amino]ethylamino]-2-pyridyl]-3-(trifluoromethyl)pyrazol-4-yl]-1-methyl-imidazole-2-carbonyl]amino]-2-chlorobenzoyl]piperazine-1-carbonyl]piperidin-1-ium-1-yl]acetic acid;formic acid;formate | 471.3 [M+H]²⁺ | Intermediate C14 and Intermediate I5 |
| | | | HATU, in DMF, then TFA |
| | 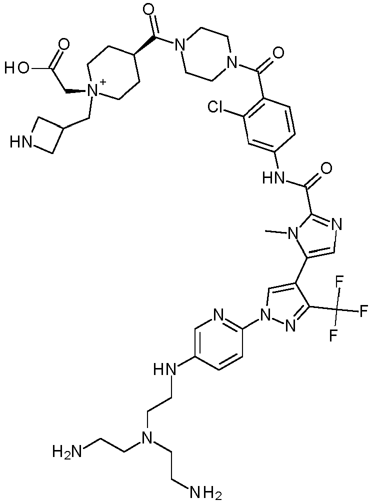 | | |
| **167** | cis-2-[4-[4-[4-[[5-[1-[2-(2-amino-2-oxo-ethyl)-1H-pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;2,2,2-trifluoroacetate | 893.4 [M]⁺ | Intermediate E78 and Intermediate H4 |
| | 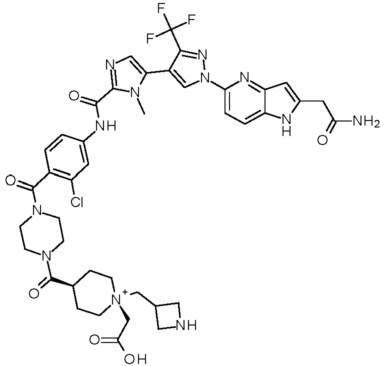 | | HATU, in DMF, then TFA in DCM |
| **171** | cis-2-[ 1-(3-aminopropyl)-4-[4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 854.64 [M]⁺ | Intermediate D7 and Intermediate H7 |
| | | | HATU, in DMF |
| | 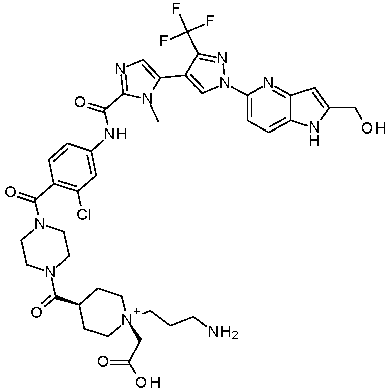 | | |
| **172** | cis-2-[1-(azetidin-3-ylmethyl)-4-[4-[2-chloro-4-[[5-[1-[3-(dimethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 907.41 [M]⁺ | Intermediate D96 and Intermediate H4 |
| | | | HATU, in DMF, then TFA in DCM |
| | 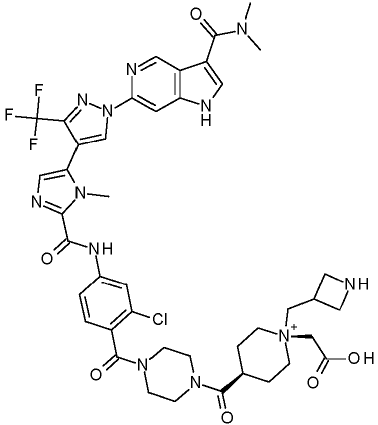 | | |
| **173** | cis-2-[1-(azetidin-3-ylmethyl)-4-[4-[2-chloro-4-[[1-methyl-5-[1-(1H-pyrazolo[4,3-c]pyridin-6-yl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 419.35 [M+H]²⁺ | Intermediate D97 and Intermediate H4 |
| | 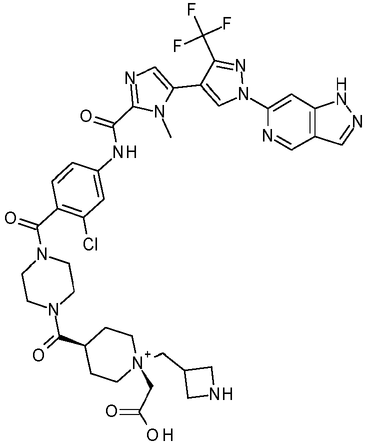 | | HATU, in DMF, then TFA in DCM |
| **176** | cis-2-[1-(azetidin-3-ylmethyl)-4-[4-[2-chloro-4-[[5-[1-[2-fluoro-4-(methylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 formate(methylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 formate | 887.4, [M-H+HCOO]⁻ | Intermediate E79 and Intermediate H4 |
| | 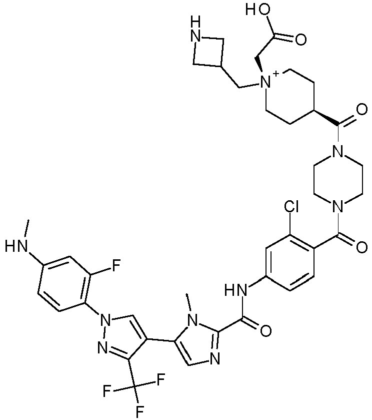 | | T3P in EtOAc, DCM |
| **181** | 4-[4-[4-[4-[[5-[1-(4-amino-2-fluorophenyl)-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]butanoic acid;formate | 857.3 [M]⁺ | Intermediate E2 and Intermediate H9 |
| | | | Bop-Cl in DMF, then TFA in DCM |
| | 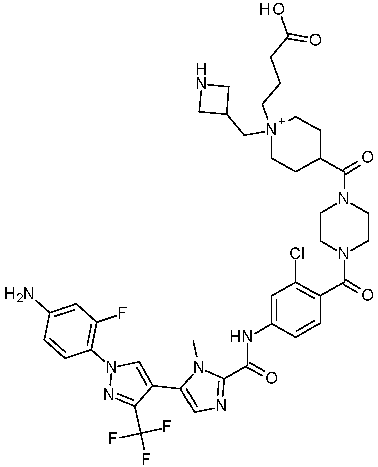 | | |
| **183** | [4-[4-[4-[[5-[1-(4-amino-2-fluorophenyl)-3-(trifluoromethyl)pyrazol-4-yl]-1-methyl-imidazole-2-carbonyl]amino]-2-chlorobenzoyl]piperazin-1-yl]-4-oxo-butyl]-bis(3-aminopropyl)-(carboxymethyl)ammonium;formate | 848.4 [M]⁺ | Intermediate E2 and Intermediate H3 |
| | 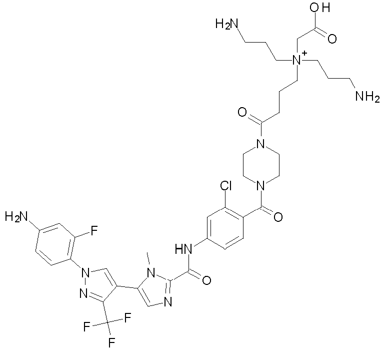 | | HATU, in DMF, then TFA in DCM |
| **184** | cis-2-[4-[4-[4-[[5-[1-[2-(aminomethyl)-1H-pyrrolo[3,2-b]pyridin-5-yl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;formate | 865.3 [M]⁺ | Intermediate C15 and Intermediate I5 |
| | | | HATU, in DMF, then TFA in DCM |
| | 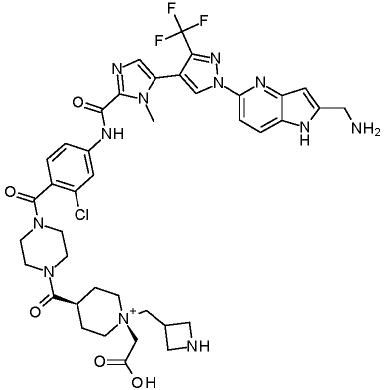 | | |
| **185** | cis-2-[1-(azetidin-3-ylmethyl)-4-[4-[2-chloro-4-[[5-[1-[2-fluoro-4-(2-methoxyethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 931.4 [M-H+HCOO]⁻ | Intermediate E81 and Intermediate H4 |
| | 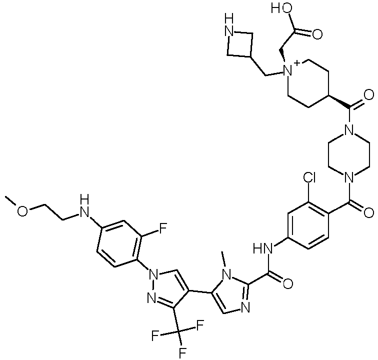 | | T3P |
| **187** | trans-2-[4-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl]piperazine-1-carbonyl]-1-[2-(dimethylamino)ethyl]piperidin-1-ium-1-yl]acetic acid;2,2,2-trifluoroacetate | 831.4 [M]⁺ | Intermediate E2 and Intermediate H15 |
| | 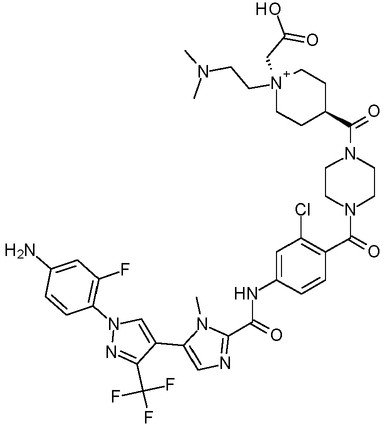 | | HATU, in DMF, then TFA in DCM |
| **188** | cis-2-[4-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl]piperazine-1-carbonyl]-1-[2-(dimethylamino)ethyl]piperidin-1-ium-1-yl]acetic acid;2,2,2-trifluoroacetate | 831.4 [M]⁺ | Intermediate E2 and Intermediate H16 |
| | | | HATU, in DMF, then TFA in DCM |
| | 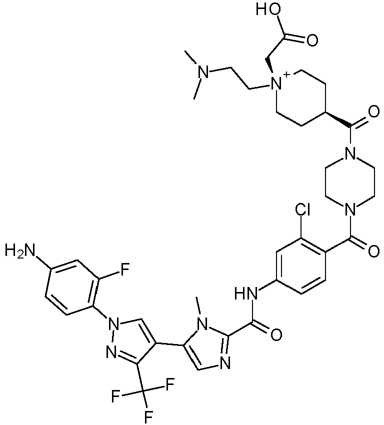 | | |
| **189** | cis-2-[4-[4-[4-[[5-[1-[2-(aminomethyl)-1H-pyrrolo[3,2-c]pyridin-6-yl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;formic acid;formate | 865.4 [M]⁺ | Intermediate C16 and Intermediate I5 |
| | 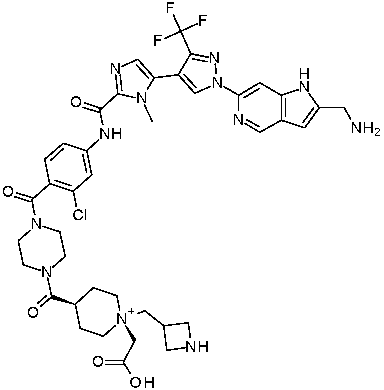 | | HATU, in DMF, then TFA in DCM |
| **193** | 2-[4-[4-[4-[[5-[1-(4-amino-2-fluorophenyl)-3-(trifluoromethyl)pyrazol-4-yl]-1-methyl-imidazole-2-carbonyl]amino]-2-chlorobenzoyl]piperazine-1-carbonyl]-1-methyl-piperidin-1-ium-1-yl]acetic acid;iodide | 774.3 [M]⁺ | Intermediate E2 and Intermediate H17 |
| | 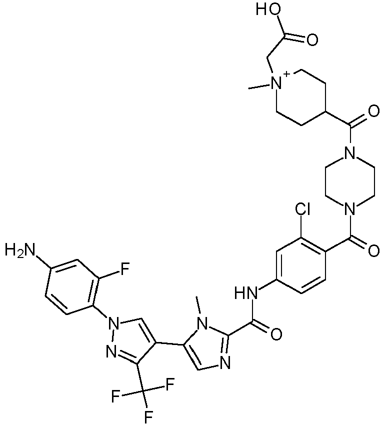 | | HATU, in DMF, then TFA in DCM |
| **195** | 2-[[4-[4-[4-[[5-[1-(4-amino-2-fluorophenyl)-3-(trifluoromethyl)pyrazol-4-yl]-1-methyl-imidazole-2-carbonyl]amino]-2-chlorobenzoyl]piperazin-1-yl]-4-oxo-butyl]-(azetidin-3-ylmethyl)-methyl-ammonio]acetate | 817.2 [M+H]⁺ | Intermediate E2 and Intermediate H18 |
| | 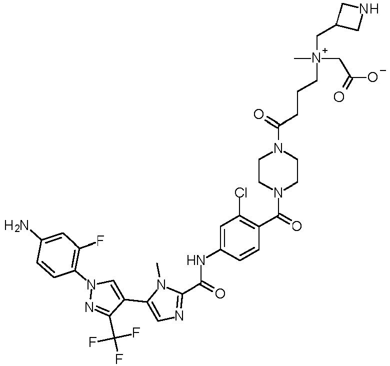 | | HATU, in DMF, then TFA in DCM |
| **196** | cis-2-[1-(azetidin-3-ylmethyl)-4-[4-[4-[[5-[1-[5-[bis(2-aminoethyl)amino]-2-pyridyl]-3-(trifluoromethyl)pyrazol-4-yl]-1-methyl-imidazole-2-carbonyl]amino]-2-chlorobenzoyl]piperazine-1-carbonyl]piperidin-1-ium-1-yl]acetic acid;formic acid;formate | 898.4 [M]⁺ | Intermediate C17 and Intermediate I5 |
| | 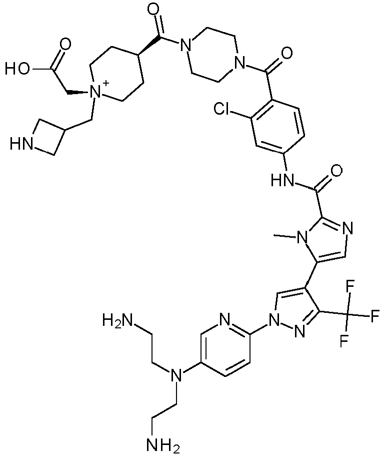 | | HATU, in DMF, then TFA in DCM |
| **200** | cis-2-[1-(azetidin-3-ylmethyl)-4-[4-[2-chloro-4-[[1-methyl-5-[1-(1H-pyrrolo[2,3-c]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 836.3 [M]⁺ | Intermediate E83 and Intermediate H4 |
| | | | T3P |
| | 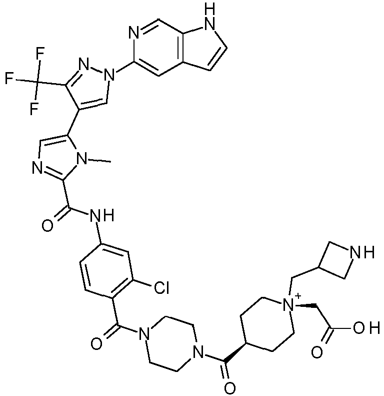 | | |
| **202** | cis-2-[4-[4-[4-[[5-[1-[5-[(3S)-3-aminopyrrolidin-1-yl]-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;formate | 881.6 [M+H]⁺ | Intermediate C18 and Intermediate I5 |
| | 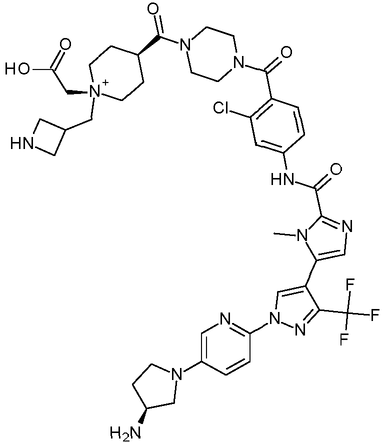 | | HATU, in DMF |

### Example 130

### 2-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]oxyethyl-bis(3-aminopropyl)-(carboxymethyl)ammonium;formate

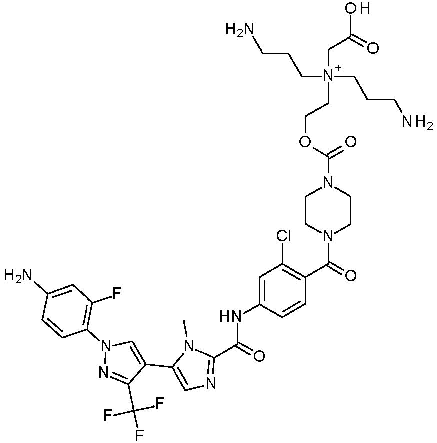

### Step 1:

A mixture of Intermediate **L1** (335.41 mg, 0.64 mmol, 4.0 eq) in THF (5 mL) was treated with CDI (103.38 mg, 0.64 mmol, 4.0 eq) at 10 °C for 1 h, and then treated with **Intermediate E2** (100.0 mg, 0.16 mmol, 1.0 eq) and triethylamine (0.13 mL, 0.96 mmol, 6.0 eq), and the mixture was stirred at 70°C for 16 h. The mixture was concentrated under reduced pressure to give a residue, which was purified by RPHPLC to give 2-[4-[4-[[5-[1-(4-amino-2-fluoro-phenyl)-3-(trifluoromethyl)pyrazol-4-yl]-1-methyl-imidazole-2-carbonyl]amino]-2-chlorobenzoyl]piperazine-1-carbonyl]oxyethyl-bis[3-(tert-butoxycarbonylamino)propyl]-(2-tert-butoxy-2-oxo-ethyl)ammonium;formate (40.0 mg, 0.03 mmol, 22%) as white solid. MS [M]⁺ 1106.7.

### Step 2:

To a stirred solution of 2-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl]piperazine-1-carbonyl]oxyethyl-bis[3-(tert-butoxycarbonylamino)propyl]-(2-tert-butoxy-2-oxo-ethyl)ammonium;formate (40.0 mg, 0.03 mmol, 1.0 eq) in DCM (3 mL) was added trifluoroacetic acid (3.0 mL, 38.94 mmol, 1122 eq), and the mixture was stirred at 25 °C for 6 h. The mixture was concentrated under vacuo, and purified by RPHPLC to afford **Example 130** (19.6 mg, 0.02 mmol, 63%) as white solid. MS [M]⁺ 850.5.

### Example 9

### Bis(4-aminobutyl)-(carboxymethyl)-[4-[4-[2-chloro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazino]-4-keto-butyl]ammonium .1:1 2,2,2-trifluoroacetic acid .1:1 2,2,2-trifluoroacetate

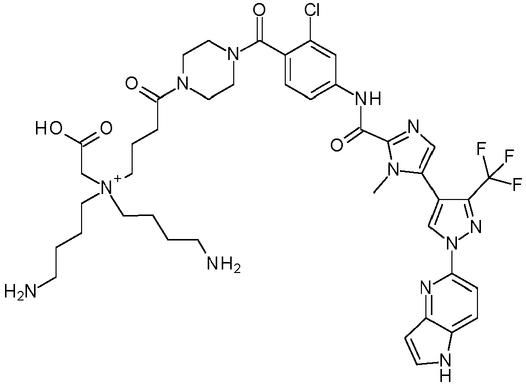

A mixture of **Intermediate D2** (60 mg, 0.086 mmol, 1 eq) in dichloromethane (0.6 mL) was treated with 4 M HCl in dioxane (257.9 mg, 214.9 µL, 0.859 mmol, 10 eq). The reaction mixture was stirred at RT for 2 h, and evaporated.

To the residue were added **Intermediate H1** (53.01 mg, 0.095 mmol, 1.1 eq), dichloromethane (0.9 mL), DIPEA (55.5 mg, 75.1 µL, 0.430 mmol, 5 eq), and PyAOP (67.22 mg, 0.129 mmol, 1.5 eq), and the resulting mixture was stirred at RT for 1.5 h until amide formation was completed.

The reaction mixture was treated with 4 M HCl in dioxane (515.7 mg, 429.7 µL, 1.72 mmol, 20 eq), and stirring was continued at RT for 72 h. All volatiles were evaporated, and the residue purified by RPHPLC to afford the title compound **Example 9** (49.9 mg, 52%) as white lyophilized solid. MS [M]⁺ 883.4.

The following examples were prepared in analogy of **Example 9**

| **Ex#** | **Name** | **MS ESI** | **Starting Material** |
|---|---|---|---|
| **13** | bis(3-aminopropyl)-(carboxymethyl)-[4-[4-[2-chloro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazino]-4-keto-butyl]ammonium .1:1 2,2,2-trifluoroacetic acid .1:1 2,2,2-trifluoroacetate | 855.3 [M]⁺ | Intermediate D2 and Intermediate H3 |
| | 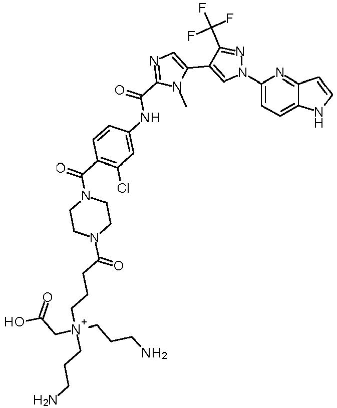 | | T3P |
| **20** | bis(4-aminobutyl)-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyl]-(carboxymethyl)ammonium .1:1 2,2,2-trifluoroacetic acid .1:1 2,2,2-trifluoroacetate | 859.4 [M]⁺ | Intermediate D31 and Intermediate H1 |
| | | | PyAOP |
| | 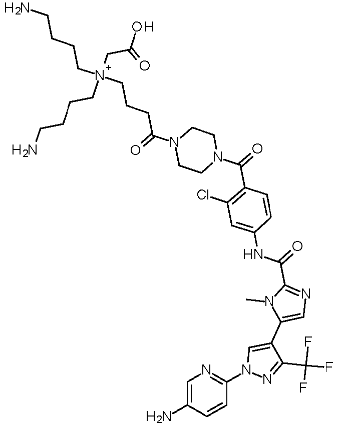 | | |
| **27** | cis-2-[1-(azetidin-3-ylmethyl)-4-[4-[2-chloro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 836.3 [M]⁺ | Intermediate D2 and Intermediate H4 |
| | 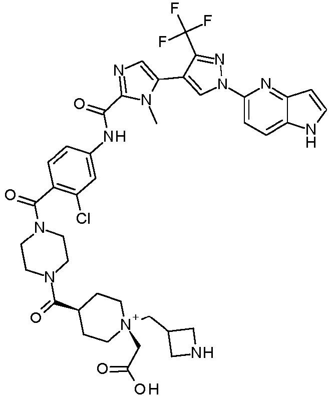 | | PyAOP |
| **32** | cis-2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid .1:1 2,2,2-trifluoroacetate | 800.6 [M]⁺ | Intermediate D31 and Intermediate H7 |
| | 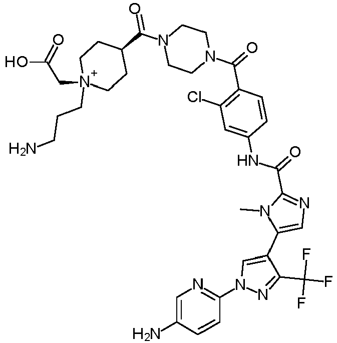 | | PyAOP |
| **34** | cis-2-[4-[4-[4-[[5-[1-(5-amino-2-pyridyl)-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid .1:1 2,2,2-trifluoroacetic acid .1:1 2,2,2-trifluoroacetate | 812.7 [M]⁺ | Intermediate D31 and Intermediate H4 |
| | 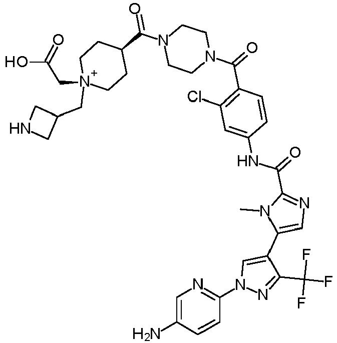 | | PyAOP |
| **39** | cis-2-[1-(azetidin-3-ylmethyl)-4-[4-[2-chloro-4-[[1-methyl-5-[1-(1-methylpyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 850.5 [M]⁺ | Intermediate D44 and Intermediate H4 |
| | 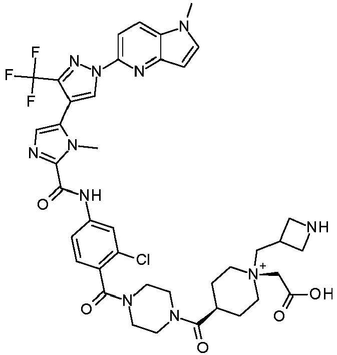 | | T3P |
| **48** | trans-2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid .1:1 2,2,2-trifluoroacetate | 800.5 [M]⁺ | Intermediate D31 and Intermediate H6 |
| | | | PyAOP |
| | 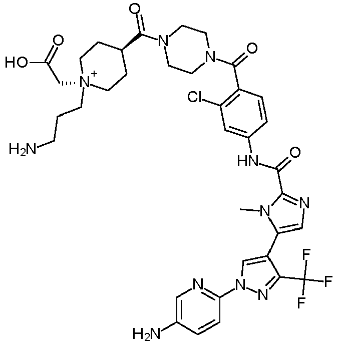 | | |
| **52** | 2-[1-(azetidin-3-ylmethyl)-4-[4-[2-chloro-4-[[1-methyl-5-[1-[4-(methylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 870.8 [M-H+HCOO]⁻ | Intermediate D15 and Intermediate H2 |
| | 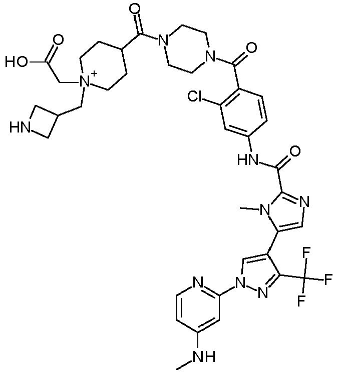 | | PyAOP |
| **64** | trans-2-[4-[4-[4-[[5-[1-(5-amino-2-pyridyl)-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid .1:1 2,2,2-trifluoroacetic acid .1:1 2,2,2-trifluoroacetate | 812.6 [M]⁺ | Intermediate D31 and Intermediate H10 |
| | | | PyAOP |
| | 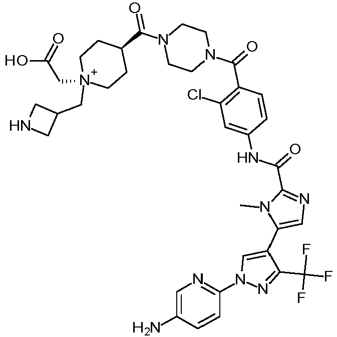 | | |
| **110** | cis-2-[1-(3-aminopropyl)-4-[4-[4-[[5-[1-(2-fluoro-4-hydroxy-phenyl)-3-(trifluoromethyl)pyrazol-4-yl]-1-methyl-imidazole-2-carbonyl]amino]-2-methyl-benzoyl]piperazine-1-carbonyl]piperidin-1-ium-1-yl]acetic acid .1:1 2,2,2-trifluoroacetate | 798.6 [M]⁺ | Intermediate D109 and Intermediate H6 |
| | 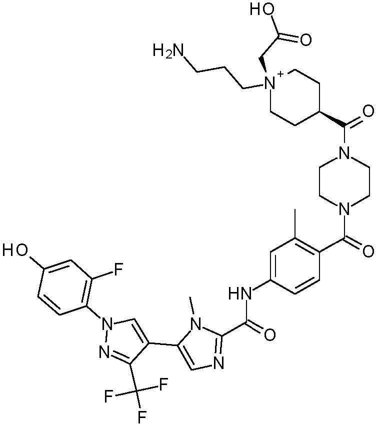 | | HATU in DMF |
| **127** | cis-2-[1-(3-aminopropyl)-4-[4-[4-[[5-[1-[2-(2-hydroxyethyl)-1H-pyrrolo[3,2-b]pyridin-5-yl]-3-(trifluoromethyl)pyrazol-4-yl]-1-methyl-imidazole-2-carbonyl]amino]-2-methyl-benzoyl]piperazine-1-carbonyl]piperidin-1-ium-1-yl]acetic acid .1:1 2,2,2-trifluoroacetate | 848.5 [M]⁺ | Intermediate D61 and Intermediate H6 |
| | 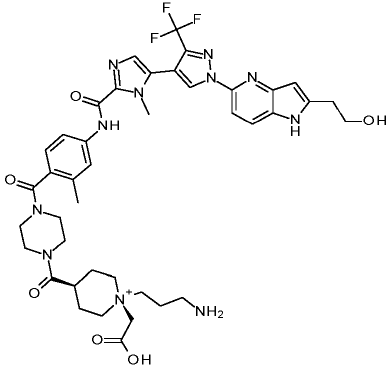 | | HATU in DMF |
| **132** | cis-2-[1-(azetidin-3-ylmethyl)-4-[4-[2-methyl-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 816.49 [M]⁺ | Intermediate D76 and Intermediate H4 |
| | | | HATU, in DMF, then TFA in DCM |
| | 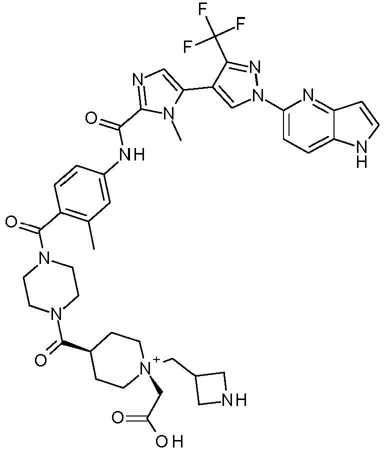 | | |
| **133** | cis-2-[1-(azetidin-3-ylmethyl)-4-[4-[2-fluoro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 820.48 [M]⁺ | Intermediate D77 and Intermediate H4 |
| | 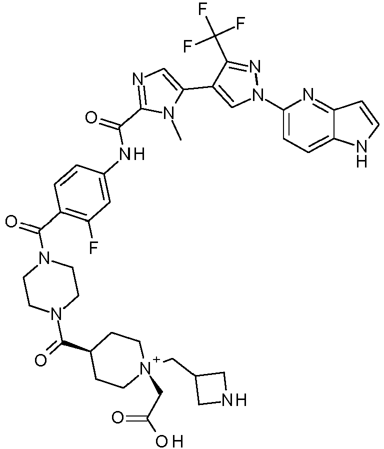 | | HATU, in DMF, then TFA in DCM |
| **139** | cis-2-[1-(azetidin-3-ylmethyl)-4-[4-[2-chloro-4-[[1-methyl-5-[1-[2-(morpholine-4-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 947.76 [M-H]⁻ | Intermediate D78 and Intermediate H4 |
| | 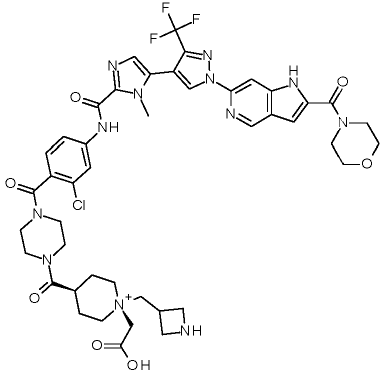 | | HATU, in DMF |
| **143** | cis-2-[1-(azetidin-3-ylmethyl)-4-[4-[2-chloro-4-[[1-methyl-5-[1-[1-(methylcarbamoyl)pyrrolo[3,2-b]pyridin-5-yl]-3-(trifluoromethyl)pyrazol-4-yl]imidazole-2-carbonyl]amino]benzoyl]piperazine-1-carbonyl]piperidin-1-ium-1-yl]acetic acid .1:1 formate | 893.4 [M]⁺ | Intermediate D79 and Intermediate H4 |
| | | | HATU, in DMF, then TFA in DCM |
| | 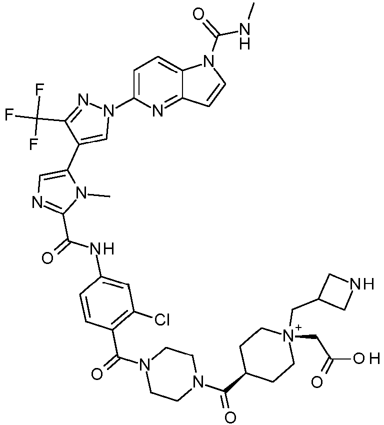 | | |
| **146** | cis-2-[1-(azetidin-3-ylmethyl)-4-[4-[2-chloro-4-[[5-[1-[3-(2-hydroxyethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 923.54 [M]⁺ | Intermediate D75 and Intermediate H4 |
| | 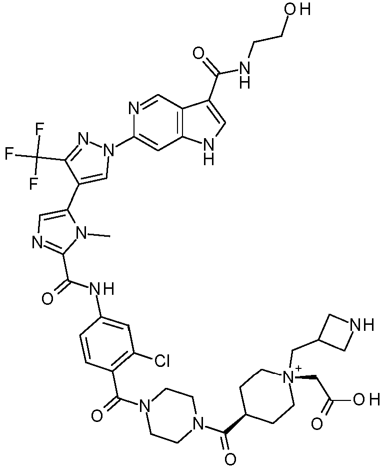 | | HATU, in DMF, then TFA in DCM |
| **148** | cis-2-[1-(azetidin-3-ylmethyl)-4-[4-[2-chloro-4-[[1-methyl-5-[1-[3-(4-methylpiperazine-1-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 960.9 [M-2H]⁻ | Intermediate D80 and Intermediate H4 |
| | 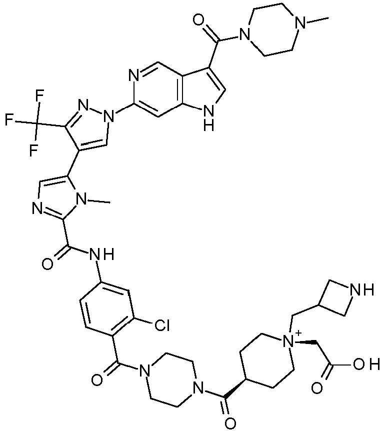 | | HATU, in DMF, then TFA in DCM |
| **149** | cis-2-[4-[4-[4-[[5-[1-[2-(2-amino-2-keto-ethyl)pyrazolo[4,3-c]pyridin-6-yl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetate | 894.35 [M]⁺ | Intermediate A23 and Intermediate H4 |
| | | | HATU, in DMF, then TFA in DCM |
| | 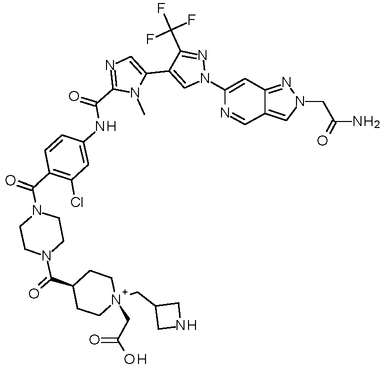 | | |
| **155** | cis-2-[1-(azetidin-3-ylmethyl)-4-[4-[2-chloro-4-[[5-[1-[3-[(3 S)-3-hydroxypyrrolidine-1-carbonyl]-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 475.46 [M+H]²⁺ | Intermediate D85 and Intermediate H4 |
| | 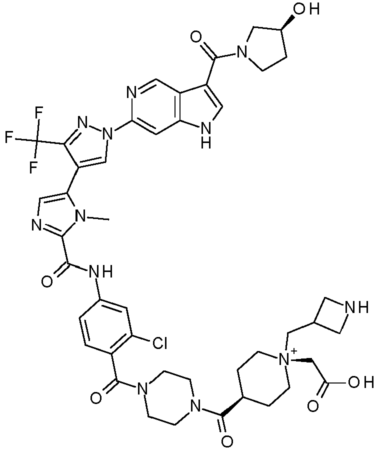 | | HATU, in DMF, then TFA in DCM |
| **156** | cis-2-[1-(azetidin-3-ylmethyl)-4-[4-[2-chloro-4-[[1-methyl-5-[1-[3-(methylcarbamoyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 893.45 [M]⁺ | Intermediate D86 and Intermediate H4 |
| | 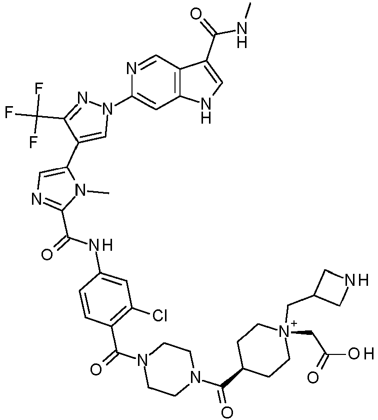 | | HATU, in DMF, then TFA in DCM |
| **157** | cis-2-[1-(azetidin-3-ylmethyl)-4-[4-[4-[[5-[1-(3-carbamoyl-1H-pyrrolo[3,2-c]pyridin-6-yl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid .1:1 2,2,2-trifluoroacetate | 879.44 [M]⁺ | Intermediate D87 and Intermediate H4 |
| | | | HATU, in DMF, then TFA in DCM |
| | 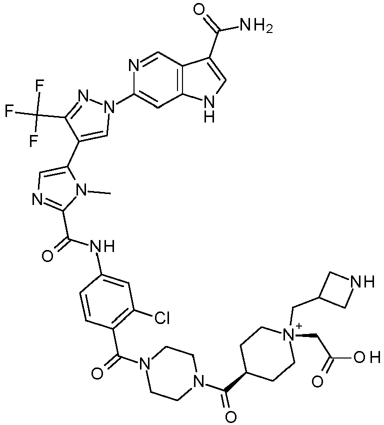 | | |
| **159** | 2-[4-[4-[4-[[5-[1-(4-amino-2-fluorophenyl)-3-(trifluoromethyl)pyrazol-4-yl]-1-methyl-imidazole-2-carbonyl]amino]-2-chlorobenzoyl]piperazine-1-carbonyl]-1-(3-hydroxypropyl)piperidin-1-ium-1-yl]acetic acid .1:1 2,2,2-trifluoroacetate | 818.52 [M]⁺ | Intermediate D34 and Intermediate H13 |
| | 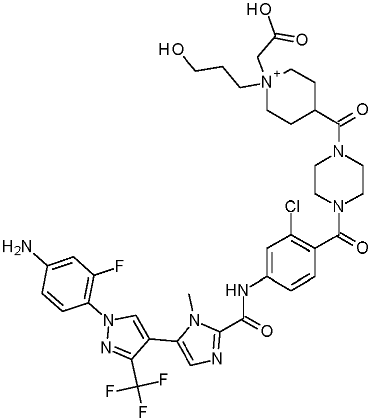 | | HATU, in DMF |
| **169** | cis-2-[1-(azetidin-3-ylmethyl)-4-[4-[2-chloro-4-[[5-[1-[1-(cyanomethyl)-2-methylol-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 905.42 [M]⁺ | Intermediate D127 and Intermediate H4 |
| | | | 1) TFA in DCM |
| | | | 2) HATU, in DMF |
| | 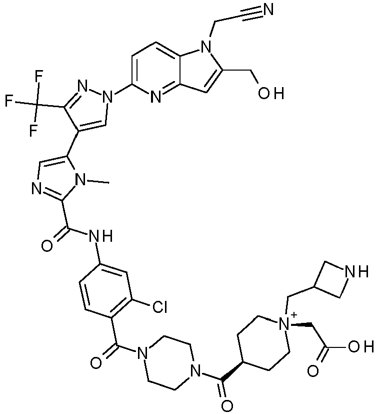 | | 3) TFA in DCM |
| **170** | cis-2-[4-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl]piperazine-1-carbonyl]-1-(3-aminopropyl)piperidin-1-ium-1-yl]acetic acid .1:1 2,2,2-trifluoroacetate | 817.4 [M]⁺ | Intermediate D34 and Intermediate H7 |
| | | | HATU, in DMF |
| | 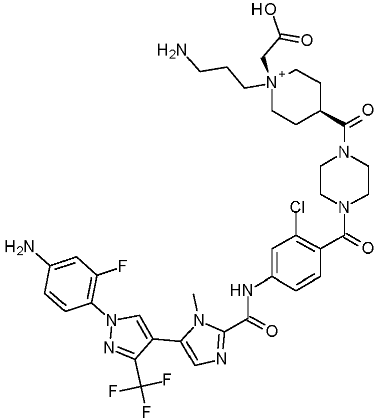 | | |
| **174** | cis-2-[1-(azetidin-3-ylmethyl)-4-[4-[2-chloro-4-[[5-[1-[1-(2-hydroxyethyl)pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 880.46 [M]⁺ | Intermediate D98 and Intermediate H4 |
| | 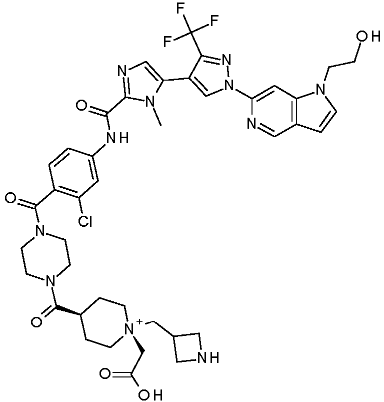 | | HATU, in DMF, then TFA in DCM |
| **175** | cis-2-[1-(azetidin-3-ylmethyl)-4-[4-[2-chloro-4-[[5-[1-[1-(2-hydroxyethyl)-2-methylol-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 910.53 [M]⁺ | Intermediate D120 and Intermediate H4 |
| | 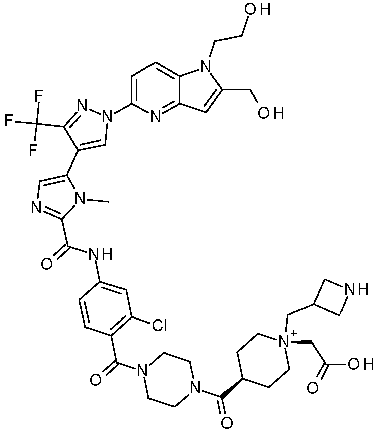 | | HATU, in DMF |
| **177** | cis-2-[1-(azetidin-3-ylmethyl)-4-[4-[2-chloro-4-[[1-methyl-5-[1-[3-(morpholine-4-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 949.47 [M]⁺ | Intermediate D99 and Intermediate H4 |
| | | | HATU, in DMF |
| | 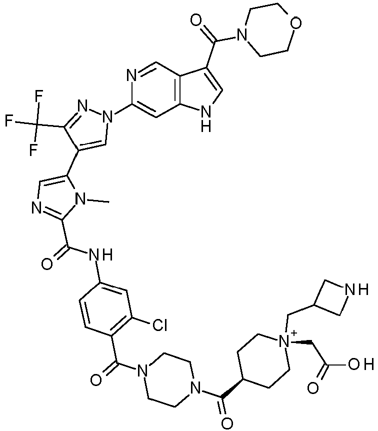 | | |
| **179** | cis-2-[1-(azetidin-3-ylmethyl)-4-[4-[2-chloro-4-[[5-[1-[3-(4,4-difluoropiperidine-1-carbonyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 983.37 [M]⁺ | Intermediate D100 and Intermediate H4 |
| | 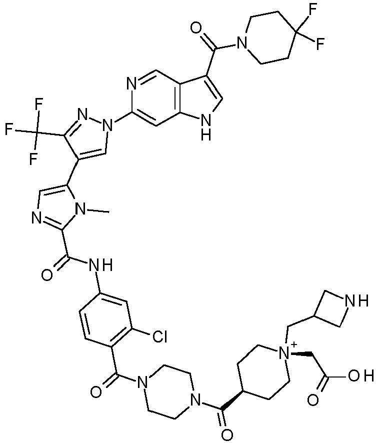 | | HATU, in DMF, then TFA infu DCM |
| **180** | cis-2-[1-(azetidin-3-ylmethyl)-4-[4-[2-chloro-4-[[1-methyl-5-[1-(2-methylol-1H-pyrrolo[3,2-c]pyridin-6-yl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 865.97 [M]⁺ | Intermediate D101 and Intermediate H4 |
| | 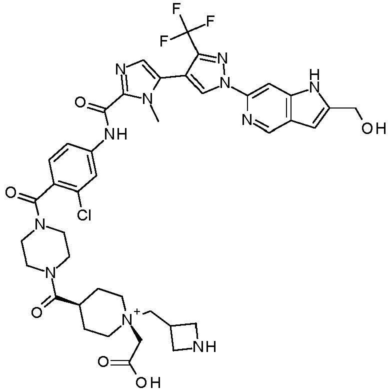 | | HATU, in DMF, then TFA in DCM |
| **191** | cis-2-[1-(azetidin-3-ylmethyl)-4-[4-[2-chloro-4-[[1-methyl-5-[1-(2-methylol-1H-pyrrolo[2,3-c]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetic acid | 866.28 [M]⁺ | Intermediate D102 and Intermediate H4 |
| | | | In addition, as a first step, the starting material was treated with LiBH4 in THF, MeOH, then: |
| | | | HATU, in DMF, then TFA in DCM |
| | 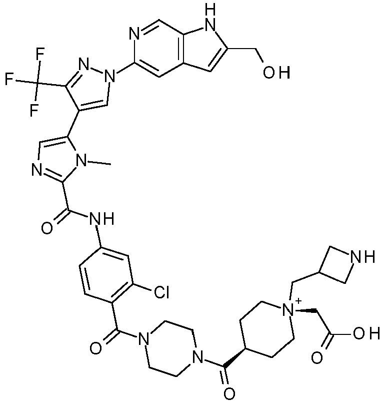 | | |
| **192** | cis-2-[4-[4-[4-[[5-[1-(6-amino-2-pyridyl)-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid .1:1 2,2,2-trifluoroacetate | 812.57 [M]⁺ | Intermediate D103 and Intermediate H4 |
| | 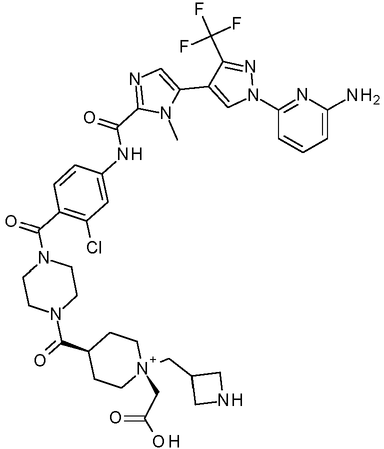 | | T3P in EtOAc, HATU, in DMF |
| **194** | cis-2-[1-(azetidin-3-ylmethyl)-4-[4-[2-chloro-4-[[5-[1-[3-(2-hydroxyethyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 880.4 [M]⁺ | Intermediate D104 and Intermediate H4 |
| | 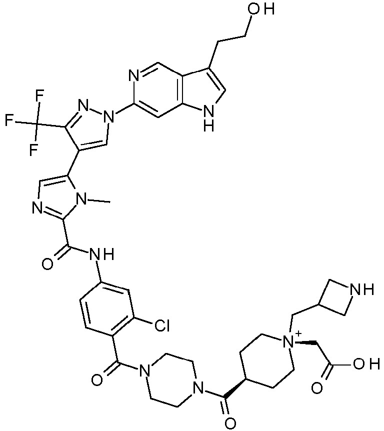 | | HATU, in DMF |
| **199** | cis-2-[4-[4-[4-[[5-[1-[1-(2-amino-2-keto-ethyl)pyrrolo[3,2-b]pyridin-5-yl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetate | 893.66 [M]⁺ | Intermediate D122 and Intermediate H4 |
| | | | T3P in EtOAc, DCM |
| | 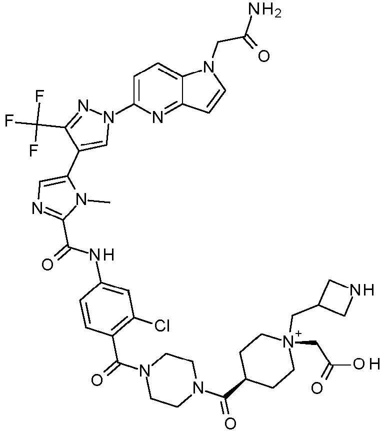 | | |

### Example 58

### cis-2-[4-[4-[4-[[5-[1-(5-Amino-3-fluoro-2-pyridyl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetate

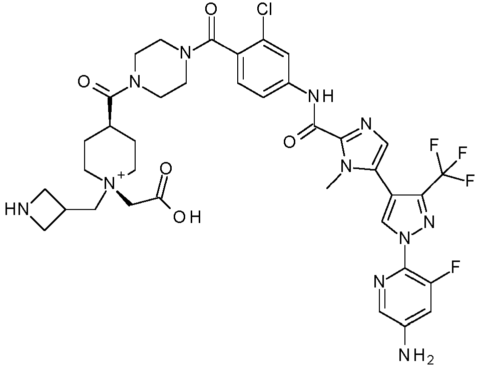

### Step 1:

A mixture of **Intermediate D19** (75 mg, 0.104 mmol, 1 eq) in dichloromethane (300 µL) was treated with 4 M HCl in dioxane (311.61 mg, 259.68 µL, 1.04 mmol, 10 eq), and the reaction mixture was stirred at RT for 28 h, and evaporated. The residue was dissolved in N,N-dimethylformamide (750 µL), and DIPEA (67.13 mg, 90.71 µL, 0.519 mmol, 5 eq), **Intermediate H4** (60.16 mg, 0.114 mmol, 1.1 eq), and T3P 50% in EtOAc (99.15 mg, 91.8 µL, 0.156 mmol, 1.5 eq) were then added, and the resulting mixture was stirred at RT for 0.5 h. Water was added, and the mixture was extracted with EtOAc. The combined organic layers were washed with 5% LiCl solution, brine, dried over sodium sulfate, filtered, and evaporated to afford crude cis-3-[[1-(2-tert-butoxy-2-keto-ethyl)-4-[4-[2-chloro-4-[[5-[1-(3-fluoro-5-nitro-2-pyridyl)-3-(trifluoromethyl)pyrazol-4-yl]-1-methyl-imidazole-2-carbonyl]amino]benzoyl]piperazine-1-carbonyl]piperidin-1-ium-1-yl]methyl]azetidine-1-carboxylic acid tert-butyl ester .1:1 2,2,2-trifluoroacetate (82.4 mg, 60%) as orange solid, which was directly used in the next step. MS [M]⁺ 1016.6.

### Step 2:

A mixture of cis-3-[[1-(2-tert-butoxy-2-keto-ethyl)-4-[4-[2-chloro-4-[[5-[1-(3-fluoro-5-nitro-2-pyridyl)-3-(trifluoromethyl)pyrazol-4-yl]-1-methyl-imidazole-2-carbonyl]amino]benzoyl]piperazine-1-carbonyl]piperidin-1-ium-1-yl]methyl]azetidine-1-carboxylic acid tert-butyl ester;2,2,2-trifluoroacetate (81.4 mg, 0.061 mmol, 1 eq) in ethanol (2.4 mL) and water (0.800 mL) was treated with ammonium chloride (163.7 mg, 3.06 mmol, 50 eq) and zinc (80.07 mg, 1.22 mmol, 20 eq), and the reaction mixture was stirred at RT for 48 h. The reaction mixture was filtered over celite, and concentrated to dryness. The residue was dissolved in dichloromethane (0.500 mL), and 4 M HCl in dioxane (183.6 mg, 153.0 µL, 0.612 mmol, 10 eq) was added, and the obtained mixture was stirred at RT O/N. Additional HCl in dioxane (5 eq) were added, and stirring was continued at RT for 5 h. Evaporation, and purification by RPHPLC yielded the title compound **Example 58** (29.4 mg, 50%) as white lyophilized solid. MS [M]⁺ 830.5.

### Example 10

### cis-2-[4-[4-[4-[[5-[1-[5-(2-Aminoethylamino)-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetic acid .1:1 2,2,2-trifluoroacetate

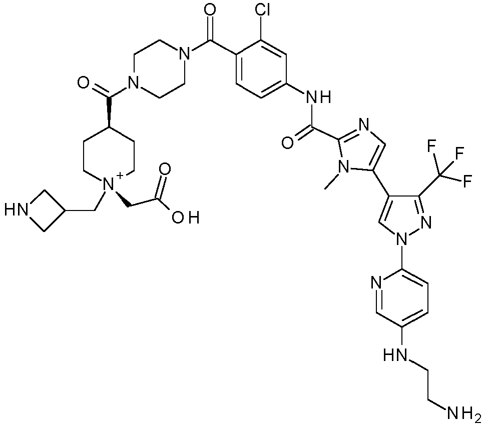

**Intermediate D33** (60 mg, 0.064 mmol, 1 eq) was treated with 2 M dimethylamine in THF (95.81 µL, 0.192 mmol, 3 eq), and the reaction mixture was stirred at RT for 3.5 h. All volatiles were evaporated, and the residue was co-evaporation with THF (2x).

The residue was dissolved in dichloromethane (0.8 mL), and treated with **Intermediate H4** (37.07 mg, 0.070 mmol, 1.1 eq), DIPEA (41.3 mg, 55.8 µL, 0.319 mmol, 5 eq), and PyAOP (39.96 mg, 0.077 mmol, 1.2 eq), and the mixture was stirred at RT for 30min.

The reaction mixture was treated with 4 M HCl in dioxane (191.6 mg, 159.7 µL, 0.639 mmol, 10 eq), and stirring was continued at RT O/N. The reaction mixture was evaporated to dryness, and purification of the residue by RPHPLC yielded the title compound **Example 10** (12.2 mg, 16%) as white lyophilized solid. MS [M]⁺ 855.3.

The following examples were prepared in analogy of **Example 10**

| **Ex#** | **Name** | **MS ESI** | **Starting Material** | |
|---|---|---|---|---|
| **15** | cis-2-[4-[4-[4-[[5-[1-[5-(3-aminopropylamino)-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 2,2,2-trifluoroacetic acid .1:1 2,2,2-trifluoroacetate | 869.4 [M]⁺ | Intermediate D36 and Intermediate H4 | |
| | | | PyAOP | |
| | 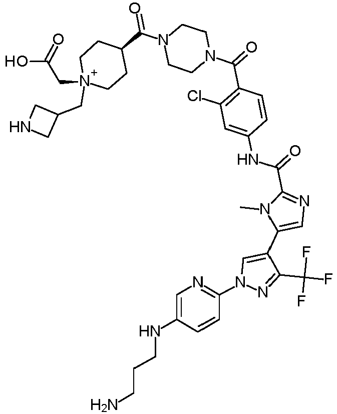 | | | |
| **36** | bis(4-aminobutyl)-[4-[4-[4-[[5-[1-[5-(2-aminoethylamino)-2-pyridyl]-3-(trifluoromethyl)pyrazol-4-yl]-1-methyl-imidazole-2-carbonyl]amino]-2-chloro-benzoyl]piperazino]-4-keto-butyl]-(carboxymethyl)ammonium .1:2 2,2,2-trifluoroacetic acid .1:1 2,2,2-trifluoroacetate | 902.6 [M]⁺ | Intermediate D33 and Intermediate H1 | |
| | | | PyAOP | |
| | 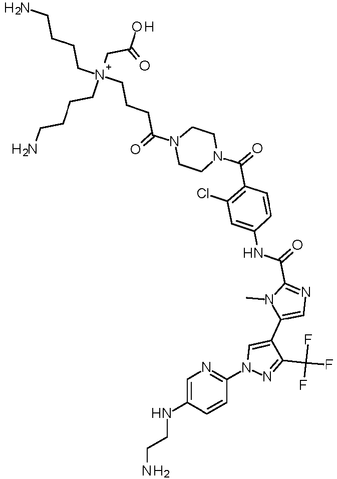 | | | |
| **147** | cis-2-[1-(azetidin-3-ylmethyl)-4-[4-[2-chloro-4-[[1-methyl-5-[1-[3-(piperazine-1-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid .1:1 2,2,2-trifluoroacetate | 474.88 [M+H]²⁺ | Intermediate B16 and Intermediate I5 | |
| | 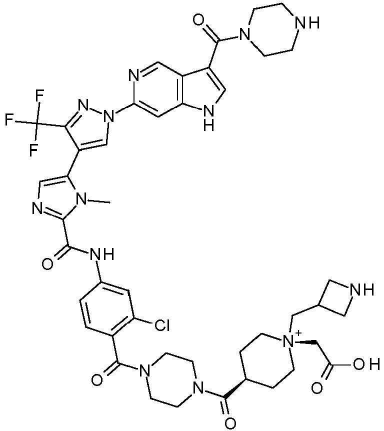 | | | 1) LiOH, THF |
| | | | | 2) HATU, in DMF |
| | | | | 3) TFA in DCM |

### Example 19

### 2-[4-[4-[4-[[5-[1-[5-[[(2S)-2-Aminopropyl]amino]-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 formic acid .1:1 formate

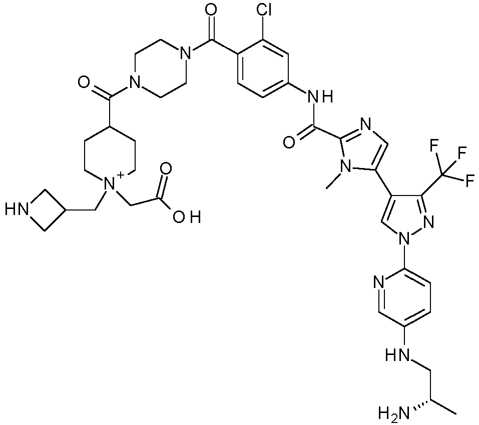

### Step 1:

A mixture of Intermediate **E6** (70 mg, 0.079 mmol, 1 eq) and **Intermediate H2** (52.98 mg, 0.118 mmol, 1.5 eq) in N,N-dimethylformamide, extra dry (2.5 mL) was treated with DIPEA (61.01 mg, 82.22 µL, 0.472 mmol, 6 eq), and subsequently with PyAOP (53.32 mg, 0.102 mmol, 1.3 eq). The mixture was stirred at RT for 4 h (until amide bond formation was complete), and then treated with 4-methylpiperidine (93.6 mg, 111.7 µL, 0.944 mmol, 12 eq). The mixture was stirred for another 2 h, and evaporated to afford crude 3-[[4-[4-[4-[[5-[1-[5-[[(2S)-2-aminopropyl]amino]-2-pyridyl]-3-(trifluoromethyl)pyrazol-4-yl]-1-methyl-imidazole-2-carbonyl]amino]-2-chloro-benzoyl]piperazine-1-carbonyl]-1-(2-tert-butoxy-2-keto-ethyl)piperidin-1-ium-1-yl]methyl]azetidine-1-carboxylic acid tert-butyl ester .1:1 chloride (81 mg, 59%) as yellow solid, which was directly used in the next step without any further purification. MS [M]⁺ 1025.7.

### Step 2:

A mixture of 3-[[4-[4-[4-[[5-[1-[5-[[(2S)-2-aminopropyl]amino]-2-pyridyl]-3-(trifluoromethyl)pyrazol-4-yl]-1-methyl-imidazole-2-carbonyl]amino]-2-chlorobenzoyl]piperazine-1-carbonyl]-1-(2-tert-butoxy-2-keto-ethyl)piperidin-1-ium-1-yl]methyl]azetidine-1-carboxylic acid tert-butyl ester (81 mg, 0.079 mmol, 1 eq) in dichloromethane, extra dry (2.5 mL) and 4 M HCl in dioxane (1.8 g, 1.5 mL, 6 mmol, 76.04 eq) was stirred at RT for 20 h, and evaporated. Purification by RPHPLC afforded the title compound **Example 19** (50 mg, 62%) as white lyophilized solid. MS [M+H]²⁺ 435.4.

The following examples were prepared in analogy of **Example 19**

| **Ex#** | **Name** | **MS ESI** | **Starting Material** |
|---|---|---|---|
| | 2-[[(5S)-5-amino-6-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-6-keto-hexyl]amino]acetic acid .1:1 2,2,2-trifluoroacetic acid | | Intermediate E1 and (2S)-6-[tert-butoxycarbonyl-(2-tert-butoxy-2-keto-ethyl)amino]-2-(9H-fluoren-9-ylmethoxycarbonylami no)hexanoic acid HATU |
| **81** | 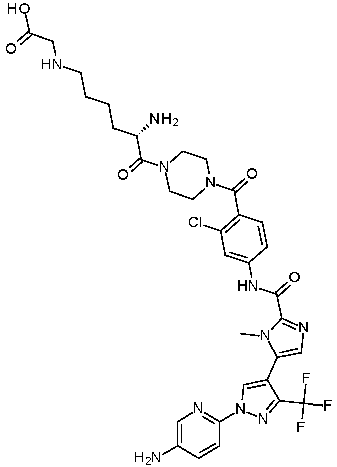 | 760.6 [M+H]⁺ | |

### Example 21

### 2-[4-[4-[4-[[5-[1-[5-(Aminomethyl)-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 formic acid .1:1 formate

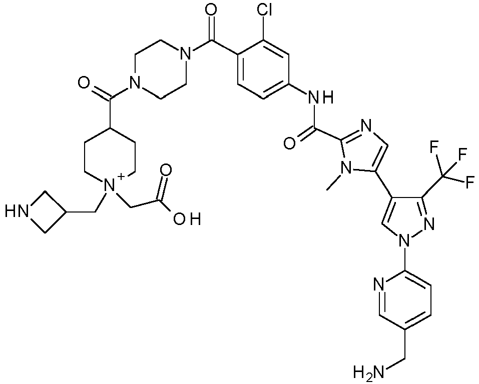

### Step 1:

A mixture of **Intermediate E7** (20 mg, 0.026 mmol, 1 eq) and **Intermediate H2** (23.7 mg, 0.053 mmol, 2 eq) in dichloromethane, extra dry (2.5 mL) was treated with DIPEA (20.45 mg, 27.6 µL, 0.158 mmol, 6 eq), and subsequently with PyAOP (20.62 mg, 0.040 mmol, 1.5 eq), and the mixture was stirred at RT for 3.5 h, until amide formation was complete. All volatiles were evaporated to afford crude 3-[[4-[4-[4-[[5-[1-[5-(benzyloxycarbonylaminomethyl)-2-pyridyl]-3-(trifluoromethyl)pyrazol-4-yl]-1-methyl-imidazole-2-carbonyl]amino]-2-chlorobenzoyl]piperazine-1-carbonyl]-1-(2-tert-butoxy-2-keto-ethyl)piperidin-1-ium-1-yl]methyl]azetidine-1-carboxylic acid tert-butyl ester .1:1 chloride (30 mg, 94%) as yellow solid, which was directly used in the next step without any further purification. [M-H+HCOO]⁻ 1160.4.

### Step 2:

A mixture of 3-[[4-[4-[4-[[5-[1-[5-(benzyloxycarbonylaminomethyl)-2-pyridyl]-3-(trifluoromethyl)pyrazol-4-yl]-1-methyl-imidazole-2-carbonyl]amino]-2-chlorobenzoyl]piperazine-1-carbonyl]-1-(2-tert-butoxy-2-keto-ethyl)piperidin-1-ium-1-yl]methyl]azetidine-1-carboxylic acid tert-butyl ester .1:1 chloride (30 mg, 0.025 mmol, 1 eq) in dichloromethane (2.5 mL) was cooled to -10 °C, and treated dropwise with 1 M BBr₃ (1.31 g, 494.3 µL, 0.494 mmol, 20 eq), and stirred at that temperature for 15 min, after which the cooling bath was removed, and stirring was continued at RT for another 40 min. The mixture was transferred into half-sat. NH₄Cl (10 mL), and diluted with DCM (5 mL), and evaporated. Purification by RPHPLC afforded the title compound **Example 21** (10 mg, 44%) as white lyophilized solid. MS [M-H+HCOO]⁻ 870.4.

The following examples were prepared in analogy of **Example 21**

| **Ex#** | **Name** | **MS ESI** | **Starting Material** |
|---|---|---|---|
| **163** | cis-2-[1-(azetidin-3-ylmethyl)-4-[4-[2-chloro-4-[[5-[1-(2-fluoro-4-hydroxy-phenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 830.3 [M]⁺ | Intermediate E75 and Intermediate H4 |
| | 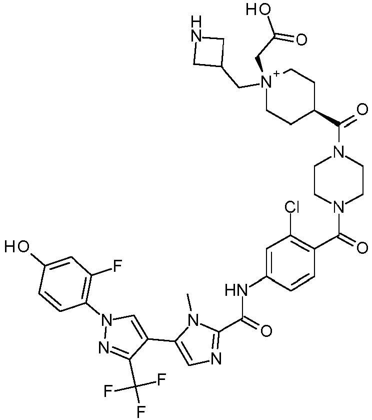 | | T3P in EtOAc, DCM |
| **178** | cis-2-[1-(azetidin-3-ylmethyl)-4-[4-[2-chloro-4-[[5-[1-[2-fluoro-4-(2-hydroxyethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 formate | 873.3 [M]⁺ | Intermediate E80 and Intermediate H4 |
| | | | T3P in EtOAc, DCM |
| | 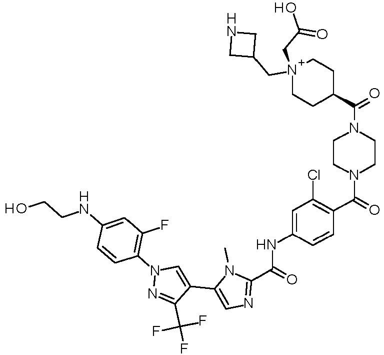 | | |

### Example 31

### cis-2-[1-(Azetidin-3-ylmethyl)-4-[4-[2-chloro-4-[[5-[1-[5-(2-methoxyethylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;2,2,2-trifluoroacetate

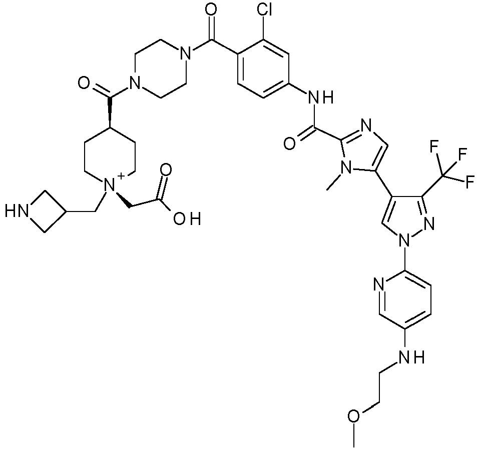

### Step 1:

A mixture of Intermediate G1 in MeOH (1 mL) was treated with methoxyacetaldehyde (1.12 mL, 0.740 mmol, 8 eq), 4Å molecular sieve (100 mg), and acetic acid (5.55 mg, 0.090 mmol, 1 eq), and the mixture was stirred at 25 °C for 1 h. Sodium cyanoborohydride (23 mg, 0.37 mmol, 4 eq) was added into the mixture, and stirring was continued at 25 °C for another 16 h to give a white suspension. The mixture was filtered (washings with MeOH, 3 x 1 mL), and the filtrate was concentrated under vacuum. Purification by RPHPLC yielded tert-butyl cis-3-[[1-(2-tert-butoxy-2-oxo-ethyl)-4-[4-[2-chloro-4-[[5-[1-[5-(2-methoxyethylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]methyl]azetidine-1-carboxylate;formate (60 mg, 61%) as a white solid. MS [M]⁺ 1026.4.

### Step 2:

To a solution of tert-butyl cis-3-[[1-(2-tert-butoxy-2-oxo-ethyl)-4-[4-[2-chloro-4-[[5-[1-[5-(2-methoxyethylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]methyl]azetidine-1-carboxylate;formate (50.0 mg, 0.050 mmol, 1 eq) in DCM (1 mL) was added trifluoroacetic acid (1.0 mL, 12.98 mmol, 278 eq), and the mixture was stirred at 25 °C for 16 h. The mixture was concentrated under vacuum, and the residue was purified by RPHPLC to give the title compound **Example 31** (43 mg, 92%) as a white solid. MS [M]⁺ 870.3.

The following examples were prepared in analogy of **Example 31**

| **Ex#** | **Name** | **MS ESI** | **Starting Material** |
|---|---|---|---|
| **38** | cis-2-[1-(azetidin-3-ylmethyl)-4-[4-[2-chloro-4-[[5-[1-[5-(2-hydroxyethylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;formate 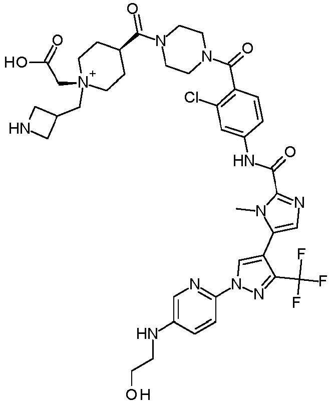 | 856.2 [M]⁺ | Intermediate G1 and (tert-butyldimethylsilyloxy)a cetaldehyde |
| | | | in addition, as a last step the residue was treated with K₂CO₃ in in MeOH at 25 °C for 16 h |
| **73** | cis-2-[4-[4-[4-[[5-[1-[5-(2-aminoethylamino)-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;formic acid;formate | 855.3 [M]⁺ | Intermediate G1 and (2-oxoethyl)carbamic acid tert-butyl ester |
| | 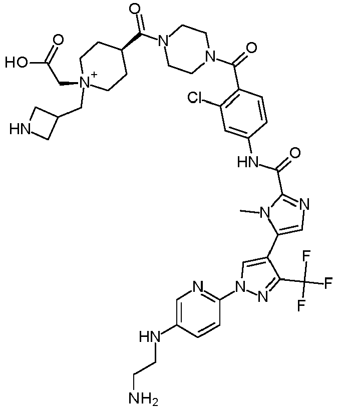 | | |
| **108** | cis-2-[1-(azetidin-3-ylmethyl)-4-[4-[2-fluoro-4-[[5-[1-[2-fluoro-4-(methylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;2,2,2-trifluoroacetate | 827.4 [M]⁺ | Intermediate G4 and paraformaldehyde |
| | 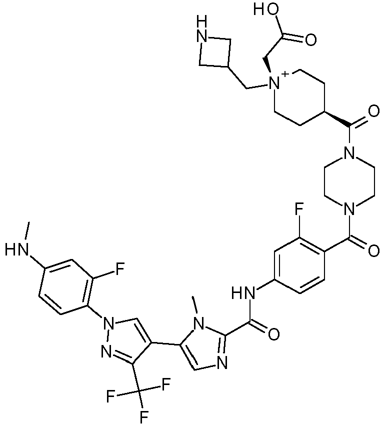 | | |
| **109** | cis-2-[1-(azetidin-3-ylmethyl)-4-[4-[4-[[5-[1-[4-(dimethylamino)-2-fluoro-phenyl]-3-(trifluoromethyl)pyrazol-4-yl]-1-methyl-imidazole-2-carbonyl]amino]-2-fluoro-benzoyl]piperazine-1-carbonyl]piperidin-1-ium-1-yl]acetic acid;2,2,2-trifluoroacetate 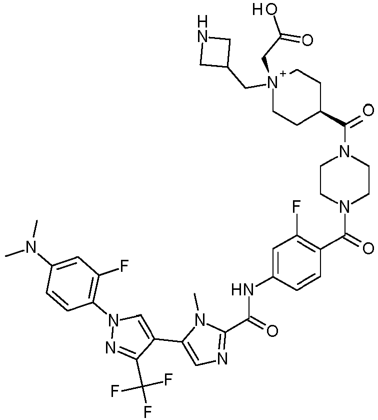 | 841.4 [M]⁺ | Intermediate G4 and paraformaldehyde |
| **124** | cis-2-[1-(azetidin-3-ylmethyl)-4-[4-[2-fluoro-4-[[5-[1-[2-fluoro-4-(2-methoxyethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;formate | 871.4 [M]⁺ | Intermediate G4 and methoxyacetaldehyde in DCM |
| | 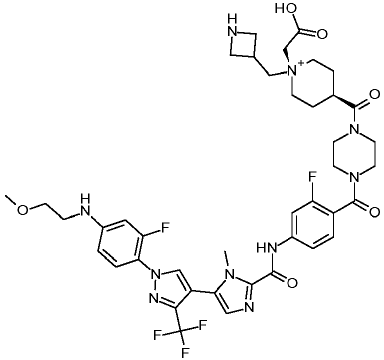 | | |

### Example 70

### 2-[2-(3-Aminopropyl)-4-[3-[[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]amino]propyl]pyridin-1-ium-1-yl]acetic acid .1:1 2,2,2-trifluoroacetic acid .1:1 2,2,2-trifluoroacetate

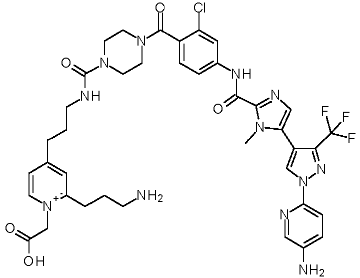

### Step 1:

A mixture of **Intermediate E1** (80 mg, 0.125 mmol, 1 eq) in N,N-dimethylformamide (0.8 mL) was treated with triethylamine (62.99 mg, 86.29 µL, 0.623 mmol, 5 eq) and CDI (50.47 mg, 0.311 mmol, 2.5 eq), and the reaction mixture was stirred at RT for 20 min. Subsequently, the mixture was treated with **Intermediate K1** (123.21 mg, 0.374 mmol, 3 eq), and stirring was continued at RT for 1 h. The reaction mixture was diluted with water, and extracted with EtOAc. The combined organic layers were washed with 5% LiCl solution, brine, dried (Na₂SO₄), filtered, evaporated, and dried under HV. Since some of the carbonyl imidazole intermediate was still present in the obtained crude residue, the residue was again dissolved in N,N-dimethylformamide (0.5 mL), and treated with triethylamine (62.99 mg, 86.29 µL, 0.623 mmol, 5 eq) and N-[3-[4-(3-aminopropyl)-2-pyridyl]propyl]carbamic acid tert-butyl ester;hydrochloride (82.14 mg, 0.249 mmol, 2 eq). The reaction mixture was stirred at RT O/N, and treated with additional N-[3-[4-(3-aminopropyl)-2-pyridyl]propyl]carbamic acid tert-butyl ester;hydrochloride (2 eq), and stirring was continued at 45°C for 48 h. The mixture was directly purified by RP column chromatography (0-80% acetonitrile in water), and the obtained fraction was further purified by RPHPLC to yield N-[3-[4-[3-[[4-[4-[[5-[1-(5-amino-2-pyridyl)-3-(trifluoromethyl)pyrazol-4-yl]-1-methyl-imidazole-2-carbonyl]amino]-2-chlorobenzoyl]piperazine-1-carbonyl]amino]propyl]-2-pyridyl]propyl]carbamic acid tert-butyl ester (20.7 mg, 18%) as white lyophilized solid. MS [M]⁺ 893.7.

### Step 2:

A mixture of N-[3-[4-[3-[[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]amino]propyl]-2-pyridyl]propyl]carbamic acid tert-butyl ester (20 mg, 0.022 mmol, 1 eq) in acetonitrile (0.6 mL) was treated with N-ethyldiisopropylamine (17.36 mg, 23.46 µL, 0.134 mmol, 6 eq), and tert-butyl bromoacetate (13.1 mg, 9.92 µL, 0.067 mmol, 3 eq), and the reaction mixture was stirred at 50 °C for 7 h, and subsequently at RT O/N. The reaction mixture was evaporated to dryness to yield crude 2-[4-[3-[[4-[4-[[5-[1-(5-amino-2-pyridyl)-3-(trifluoromethyl)pyrazol-4-yl]-1-methylimidazole-2-carbonyl]amino]-2-chloro-benzoyl]piperazine-1-carbonyl]amino]propyl]-2-[3-(tert-butoxycarbonylamino)propyl]pyridin-1-ium-1-yl]acetic acid tert-butyl ester .1:1 bromide (30 mg, 65%) as off-white amorphous which was directly used in the next step without any further purification. MS [M]⁺ 1007.8.

### Step 3:

A mixture of 2-[4-[3-[[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]amino]propyl]-2-[3-(tert-butoxycarbonylamino)propyl]pyridin-1-ium-1-yl]acetic acid tert-butyl ester .1:1 bromide (30 mg, 0.028 mmol, 1 eq) in dichloromethane (600 µL) was treated with 4 M HCl in dioxane (41.34 mg, 34.45 µL, 0.138 mmol, 5 eq), and the reaction mixture was stirred at RT O/N. Evaporation, and purification by RPHPLC yielded the title compound **Example 70** (11.7 mg, 39%) as white lyophilized solid. MS [M]⁺ 851.6.

The following examples were prepared in analogy of **Example 70**

| **Ex#** | **Name** | **MS ESI** | **Starting Material** |
|---|---|---|---|
| **96** | 2-[2-(3-aminopropyl)-4-[3-[[1-[4-[[5-[1-(5-amino-2-pyridyl)-3-(trifluoromethyl)pyrazol-4-yl]-1-methyl-imidazole-2-carbonyl]amino]-2-chloro-benzoyl]piperidine-4-carbonyl]amino]propyl]pyridin-1-ium-1-yl]acetic acid .1:1 2,2,2-trifluoroacetic acid .1:1 2,2,2-trifluoroacetate | 850.6 [M]⁺ | Intermediate F1 and Intermediate K1 and tert-butyl bromoacetate |
| | | | HATU in DMF instead of CDI |
| | 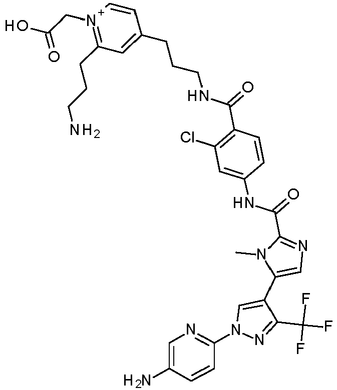 | | |

### Example 94

### 2-[4-[4-[4-[[5-[1-(4-Aminoisothiazol-5-yl)-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 formate

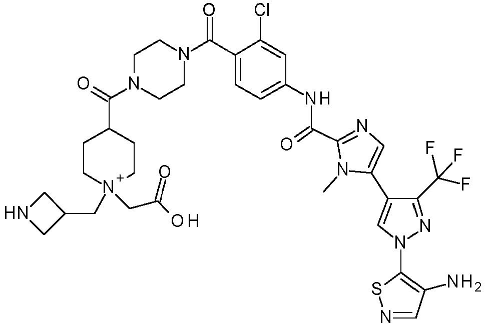

A mixture of crude **Intermediate E51** (30 mg, 0.046 mmol, 1 eq) and **Intermediate H2** (38.3 mg, 0.093 mmol, 2 eq) in dichloromethane, extra dry (2.5 mL) was treated with ethyldiisopropylamine (35.99 mg, 48.5 µL, 0.278 mmol, 6 eq), and subsequently with PyAOP (31.46 mg, 0.060 mmol, 1.3 eq), and the mixture was stirred at RT for 3 h, until amide formation was complete. Subsequently, the reaction mixture was treated with 4 M HCl in dioxane (1.04 mL, 4.18 mmol, 90 eq), and stirring was continued at RT for 16 h. All volatiles were evaporated. A mixture of the residue in ethanol (3 mL) and water (0.750 mL) was treated with iron (64.8 mg, 1.16 mmol, 25 eq) and ammonium chloride (74.47 mg, 1.39 mmol, 30 eq), and the mixture was stirred at 70 °C for 2.5 h. The mixture was cooled to RT, and filtered through a pad of Decalite (washed with MeOH), and concentrated. Purification by RPHPLC afforded the title compound **94** (4 mg, 9.5%) as off-white solid. MS [M-2H]⁻ 816.5.

### Example 95

### 2-[1-(Azetidin-3-ylmethyl)-4-[4-[4-[[5-[1-(benzothiophen-2-yl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid;formic acid;formate

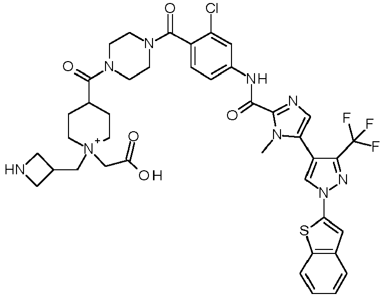

### Step 1:

A mixture of N-[3-chloro-4-(piperazine-1-carbonyl)phenyl]-1-methyl-5-[3-(trifluoromethyl)-1H-pyrazol-4-yl]imidazole-2-carboxamide;dihydrochloride (CAS 2489198-91-4; 1.0 g, 1.8 mmol, 1 eq), **Intermediate H2** (0.89 g, 1.8 mmol, 1 eq) and N,N-diisopropylethylamine (1.26 mL, 7.21 mmol, 4 eq) in DCM (10 mL) was treated with HATU (0.82 g, 2.16 mmol, 1.2 eq), and the mixture was stirred at 20 °C for 16 h. The mixture was concentrated, and the residue was purified by RPHPLC to afford tert-butyl 3-[[1-(2-tert-butoxy-2-oxo-ethyl)-4-[4-[2-chloro-4-[[1-methyl-5-[3-(trifluoromethyl)-1H-pyrazol-4-yl]imidazole-2-carbonyl]amino]benzoyl]piperazine-1-carbonyl]piperidin-1-ium-1-yl]methyl]azetidine-1-carboxylate;bromide (750 mg, 47.4%) as light yellow solid. MS [M]⁺ 876.2.

### Step 2:

A mixture of tert-butyl 3-[[1-(2-tert-butoxy-2-oxo-ethyl)-4-[4-[2-chloro-4-[[1-methyl-5-[3-(trifluoromethyl)-1H-pyrazol-4-yl]imidazole-2-carbonyl]amino]benzoyl]piperazine-1-carbonyl]piperidin-1-ium-1-yl]methyl]azetidine-1-carboxylate;bromide (121.46 mg, 0.130 mmol, 1.1 eq) in ACN (5 mL) was treated with 2-iodobenzothiophene (30.0 mg, 0.120 mmol, 1 eq), cesium carbonate (75.16 mg, 0.230 mmol, 2 eq), and copper (1.47 mg, 0.020 mmol, 0.200 eq), and the mixture was stirred at 85 °C for 16 h. The mixture was filtered, and purified by RPHPLC to afford tert-butyl 3-[[4-[4-[4-[[5-[1-(benzothiophen-2-yl)-3-(trifluoromethyl)pyrazol-4-yl]-1-methyl-imidazole-2-carbonyl]amino]-2-chloro-benzoyl]piperazine-1-carbonyl]-1-(2-tert-butoxy-2-oxo-ethyl)piperidin-1-ium-1-yl]methyl]azetidine-1-carboxylate;bromide (40 mg, 31.8%) as light yellow solid. MS [M]⁺ 1008.3.

### Step 3:

To a mixture of tert-butyl 3-[[4-[4-[4-[[5-[1-(benzothiophen-2-yl)-3-(trifluoromethyl)pyrazol-4-yl]-1-methyl-imidazole-2-carbonyl]amino]-2-chloro-benzoyl]piperazine-1-carbonyl]-1-(2-tert-butoxy-2-oxo-ethyl)piperidin-1-ium-1-yl]methyl]azetidine-1-carboxylate;bromide (40.0 mg, 0.040 mmol, 1 eq) in DCM (2 mL) was added trifluoroacetic acid (2.0 mL, 25.96 mmol, 707.1 eq), and the mixture was stirred at 20 °C for 16. The mixture was concentrated, and the residue purified by RPHPLC to afford the title compound **95** (4.2 mg, 11.6%) as white solid. MS [M]⁺ 852.3.

### Example 128

### cis-2-[4-[4-[4-[[5-[1-(4-Amino-2-fluoro-phenyl)-3-(difluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;2,2,2-trifluoroacetate

A solution of **Intermediate G13** (100 mg, 0.1 mmol, 1 eq) in DCM (1 mL) was treated with trifluoroacetic acid (0.01 mL, 0.1 mmol, 1.0 eq). Then the mixture was stirred at 25 °C for 5 h, and concentrated in vacuo. The residue was purified RPHPLC to afford the title compound **Example 128** (14.3 mg, 0.02 mmol, 14%) as a white solid. MS [M]⁺ 811.7.

The following examples were prepared in analogy of **Example 128**

| **Ex#** | **Name** | **MS ESI** | **Starting Material** |
|---|---|---|---|
| **129** | cis-2-[4-[4-[4-[[5-[1-[2-(2-aminoethyl)-1H-pyrrolo[3,2-b]pyridin-5-yl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;chloride | 880.5 [M]⁺ | Intermediate G9 |
| | 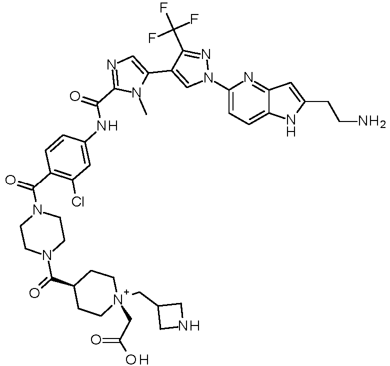 | | |
| **182** | [4-[4-[4-[[5-[1-(4-amino-2-fluorophenyl)-3-(trifluoromethyl)pyrazol-4-yl]-1-methyl-imidazole-2-carbonyl]amino]-2-chlorobenzoyl]piperazin-1-yl]-4-oxo-butyl]-bis(3-aminopropyl)-(3-carboxypropyl)ammonium;2,2,2-trifluoroacetate | 876.3 [M]⁺ | Intermediate G5 |
| | 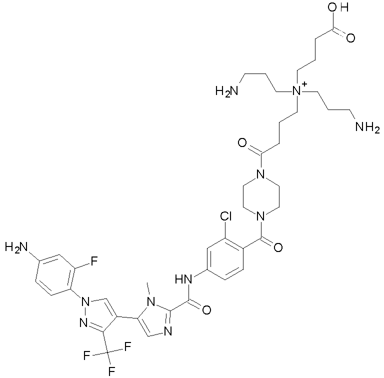 | | |
| **190** | cis-2-[4-[4-[4-[[5-[1-[1-(2-aminoethyl)pyrrolo[3,2-b]pyridin-5-yl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid . 1:1 hydrogen chloride .1:1 chloride | 879.56 [M]⁺ | Intermediate G12 |
| | 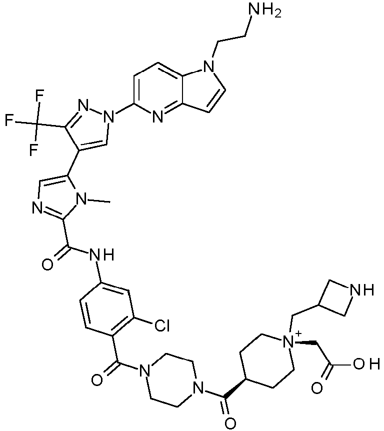 | | HCl in dioxane, DCM |

### Example 168

### cis-2-[4-[4-[4-[[5-[1-[1-(2-amino-2-keto-ethyl)pyrrolo[3,2-c]pyridin-6-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid .1:1 2,2,2-trifluoroacetate

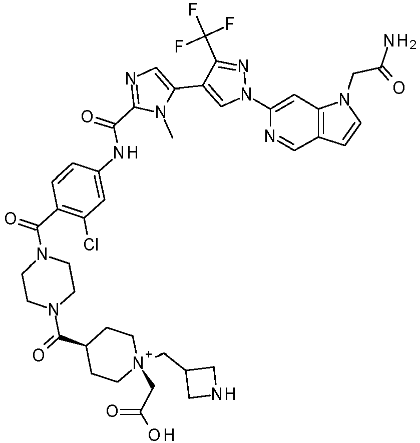

A mixture of **Intermediate G10** (33.78 mg, 0.034 mmol, 1 eq) in acetonitrile, extra dry (0.4 mL) was treated with 2-bromoacetamide (9.85 mg, 0.071 mmol, 2.1 eq), KI (5.64 mg, 0.034 mmol, 1 eq) and cesium carbonate (24.37 mg, 0.075 mmol, 2.2 eq), and the mixtures was stirred at ambient temperature for 4 h. If incomplete conversion, additional halide was added, and the mixture was stirred at 70 °C until full conversion. The reaction mixture was filtered over Celite and concentrated in vacuo. The residue was dissolved in DCM (0.3 mL), and treated with TFA (193.83 mg, 130.97 µL, 1.7 mmol, 50 eq), and the mixture was stirred at ambient temperature overnight. The mixture was concentrated, and the residue was purified by RPHPLC to afford the title compound **Example 168** (9.1 mg, 25%) as a colorless lyophilized powder. MS [M+H]²⁺ 447.9.

The following examples were prepared in analogy of **Example 168**

| **Ex#** | **Name** | **MS ESI** | **Starting Material** |
|---|---|---|---|
| **197** | cis-2-[1-(azetidin-3-ylmethyl)-4-[4-[2-chloro-4-[[5-[1-[1-(cyanomethyl)pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 875.43 [M]⁺ | Intermediate G11 and bromoacetonitrile |
| | | | NaH in DMF |
| | 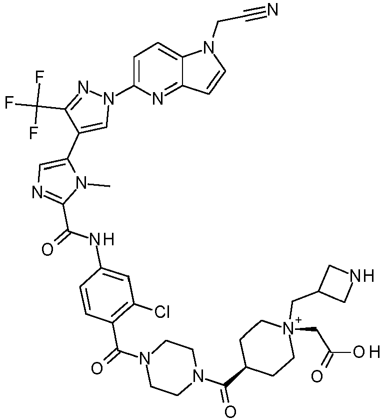 | | |
| **198** | cis-2-[1-(azetidin-3-ylmethyl)-4-[4-[2-chloro-4-[[5-[1-[1-(2-hydroxyethyl)pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 2,2,2-trifluoroacetate | 880.43 [M]⁺ | Intermediate G11 and (2-bromoethoxy)-tert-butyldimethylsilane (CAS 86864-60-0) |
| | 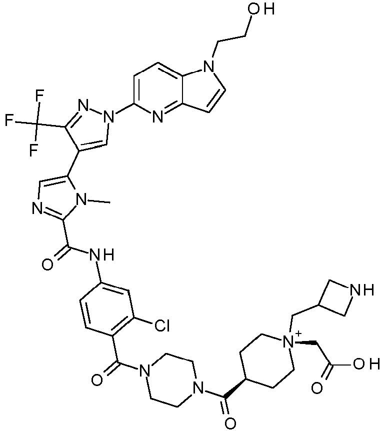 | | NaH in DMF, then HCl in dioxane, DCM |

### Example 186

### cis-2-[4-[4-[4-[[5-[1-[4-(3-aminopropylamino)-2-fluoro-phenyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid .1:1 formate

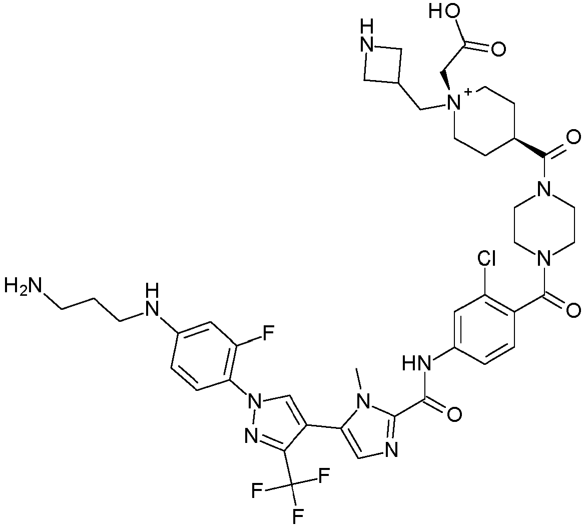

### Step 1:

A mixture of **Intermediate E82** (91 mg, 0.074 mmol, 1 eq) in DCM (3 mL) was treated with DIPEA (57.59 mg, 77.82 µL, 0.446 mmol, 6 eq) and **Intermediate H4** (46.92 mg, 0.089 mmol, 1.2 eq), and subsequently n-propylphosphonic acid anhydride, cyclic trimer (94.52 mg, 87.52 µL, 0.149 mmol, 2 eq) before stirring at RT for 2 h. The reaction mixture was subsequently treated with 4 M HCl (1.2 g, 1 mL, 4 mmol, 53.9 eq) and stirred for 16 h. The reaction mixture was then concentrated in vacuo to afford crude cis-2-[1-(azetidin-3-ylmethyl)-4-[4-[2-chloro-4-[[5-[1-[4-[3-(9H-fluoren-9-ylmethoxycarbonylamino)propylamino]-2-fluoro-phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 chloride (120 mg, 97.4%), which was used in subsequent steps without purification. MS [M]⁻ 1108.6.

### Step 2:

A mixture of cis-2-[1-(azetidin-3-ylmethyl)-4-[4-[2-chloro-4-[[5-[1-[4-[3-(9H-fluoren-9-ylmethoxycarbonylamino)propylamino]-2-fluoro-phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid .1:1 chloride (120 mg, 0.072 mmol, 1 eq) in DCM (2.5 mL) was treated with 2 M dimethylamine in THF (890 mg, 1 mL, 2 mmol, 27.7 eq) and stirred at RT for 3 d. The reaction mixture was then concentrated in vacuo, and purified by RPHPLC to afford the title compound **Example 186** (22 mg, 31.98%) as white lyophilized powder. MS [M-H+HCOO]⁻ 930.5.

### Example 201

### 2-[4-[4-[4-[[5-[1-[5-(2-aminoethylamino)-2-pyridyl]-3-(trifluoromethyl)pyrazol-4-yl]-1-methyl-imidazole-2-carbonyl]amino]-2-chloro-benzoyl]piperazine-1-carbonyl]-1-methyl-piperidin-1-ium-1-yl]acetate

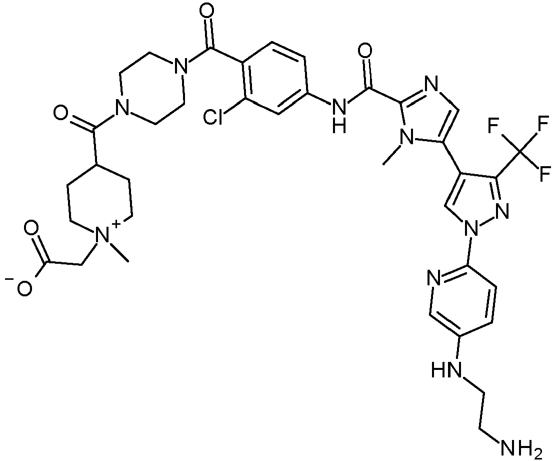

### Step 1:

A mixture of **Intermediate E1** (200 mg, 0.328 mmol, 1 eq) and 1-methylisonipecotic acid (56.29 mg, 0.393 mmol, 1.2 eq) in N,N-dimethylformamide, extra dry (2.5 mL) was treated with DIPEA (254.07 mg, 343.33 µL, 1.97 mmol, 6 eq), and subsequently with HATU (161.95 mg, 0.426 mmol, 1.3 eq). The reaction mixture was stirred at RT for 5 h. The reaction mixture was then diluted with half-sat. NaHCO₃ (30 mL), and extracted with EtOAc (3 x 20 mL). The combined organics were washed with brine, dried (Na₂SO₄), filtered, and evaporated. The residue was purified by column chromatography (0-50% DCM/MeOH 1:1 in DCM) to afford 5-[1-(5-amino-2-pyridyl)-3-(trifluoromethyl)pyrazol-4-yl]-N-[3-chloro-4-[4-(1-methylisonipecotoyl)piperazine-1-carbonyl]phenyl]-1-methyl-imidazole-2-carboxamide (38 mg, 16.6%) as a white oil. MS [M+HCOO]⁻ 743.4.

### Step 2:

A mixture of 5-[1-(5-amino-2-pyridyl)-3-(trifluoromethyl)pyrazol-4-yl]-N-[3-chloro-4-[4-(1-methylisonipecotoyl)piperazine-1-carbonyl]phenyl]-1-methyl-imidazole-2-carboxamide (38 mg, 0.054 mmol, 1 eq) in dichloromethane, extra dry (2.5 mL) was treated with acetic acid (16.32 mg, 15.56 µL, 0.272 mmol, 5 eq) and N-(2-ketoethyl)carbamic acid tert-butyl ester (17.3 mg, 0.109 mmol, 2 eq). The reaction mixture was stirred at 0 °C, and sodium triacetoxyborohydride (28.8 mg, 0.136 mmol, 2.5 eq) was added. The reaction mixture was stirred for a further 10 min, before the temperature was increased to RT, and the mixture was stirred for 18 h. The reaction mixure was diluted with aq. NaHCO₃ (30 mL), and extracted with DCM (3 x 15 mL). The combined organics were washed with brine, dried (Na₂SO₄), filtered, and evaporated. The crude was purified by column chromatography (DCM/MeOH 1:1 in DCM) to afford N-[2-[[6-[4-[2-[[3-chloro-4-[4-(1-methylisonipecotoyl)piperazine-1-carbonyl]phenyl]carbamoyl]-3-methyl-imidazol-4-yl]-3-(trifluoromethyl)pyrazol-1-yl]-3-pyridyl]amino]ethyl]carbamic acid tert-butyl ester (30 mg, 62.9%) as a colorless oil. MS [M+HCOO]⁻ 886.5.

### Step 3:

N-[2-[[6-[4-[2-[[3-chloro-4-[4-(1-methylisonipecotoyl)piperazine-1-carbonyl]phenyl]carbamoyl]-3-methyl-imidazol-4-yl]-3-(trifluoromethyl)pyrazol-1-yl]-3-pyridyl]amino]ethyl]carbamic acid tert-butyl ester (30 mg, 0.034 mmol, 1 eq) in N,N-dimethylformamide, extra dry (2.5 mL) was treated with K₂CO₃ (9.45 mg, 0.068 mmol, 2 eq) and tert-butyl bromoacetate (10. mg, 7.58 µL, 0.051 mmol, 1.5 eq), and stirred at 50 °C for 2.5 h. The reaction mixture was then concentrated in vacuo, and redissolved in dichloromethane, extra dry (2.5 mL), and subsequently treated with 4 M HCl in dioxane (0.2 mL, 0.8 mmol, 23.4 eq). The reaction mixture was stirred at RT for 2 h, evaporated, and purified by RPHPLC to yield the title compound **Example 201** (5 mg, 16.6%) as a white lyophilized solid. MS [M+HCOO]⁻ 845.4.

### Assay procedures

### Antimicrobial susceptibility testing: 90% Growth Inhibitory Concentration (IC90) determination

The in vitro antimicrobial activity of the compounds was determined according to the following procedure:
The assay used a 10-points Iso-Sensitest broth medium to measure quantitatively the *in vitro* activity of the compounds against *Acinetobacter baumannii* ATCC17961.

Stock compounds in DMSO were serially twofold diluted (e.g. range from 10 to 0.0195 µM final concentration) in 384 wells microtiter plates and inoculated with 49 µL the bacterial suspension in Iso-Sensitest medium to have a final cell concentration of ~ 5x10⁽⁵⁾ CFU/mL in a final volume/well of 50 µL/well. Microtiter plates were incubated at 35 ± 2 °C.

Bacterial cell growth was determined with the measurement of optical density at λ=600 nm each 20 min over a time course of 16 h. Growth inhibition was calculated during the logarithmic growth of the bacterial cells with determination of the concentration inhibiting 50% (IC50) and 90% (IC90) of the growth.

Table 1 provides the 90% growth inhibitory concentrations (IC90) in micromoles per liter of the compounds of present invention obtained against the strain *Acinetobacter baumannii* ATCC17961.

Particular compounds of the present invention exhibit an IC90 (*Acinetobacter baumannii* ATCC17961) ≤ 25 µmol/l.

More particular compounds of the present invention exhibit an IC90 (*Acinetobacter baumannii* ATCC17961) ≤ 5 µmol/l.

Most particular compounds of the present invention exhibit an IC90 (*Acinetobacter baumannii* ATCC17961) ≤ 1 µmol/l.

**Table 1**

| **Ex.** | **ATCC 17961 IC90 [µM]** |
|---|---|
| 1 | 0.33 |
| 2 | 0.33 |
| 3 | 0.39 |
| 4 | 0.39 |
| 5 | 0.59 |
| 6 | 0.69 |
| 7 | 1.48 |
| 8 | 1.55 |
| 9 | 0.10 |
| 10 | 0.17 |
| 11 | 0.17 |
| 12 | 0.18 |
| 13 | 0.20 |
| 14 | 0.20 |
| 15 | 0.21 |
| 16 | 0.21 |
| 17 | 0.24 |
| 18 | 0.26 |
| 19 | 0.26 |
| 20 | 0.30 |
| 21 | 0.32 |
| 22 | 0.33 |
| 23 | 0.34 |
| 24 | 0.35 |
| 25 | 0.35 |
| 26 | 0.36 |
| 27 | 0.36 |
| 28 | 0.36 |
| 29 | 0.36 |
| 30 | 0.37 |
| 31 | 0.37 |
| 32 | 0.37 |
| 33 | 0.38 |
| 34 | 0.40 |
| 35 | 0.40 |
| 36 | 0.42 |
| 37 | 0.42 |
| 38 | 0.43 |
| 39 | 0.43 |
| 40 | 0.43 |
| 41 | 0.44 |
| 42 | 0.44 |
| 43 | 0.46 |
| 44 | 0.49 |
| 45 | 0.50 |
| 46 | 0.50 |
| 47 | 0.50 |
| 48 | 0.51 |
| 49 | 0.52 |
| 50 | 0.52 |
| 51 | 0.52 |
| 52 | 0.53 |
| 53 | 0.55 |
| 54 | 0.55 |
| 55 | 0.55 |
| 56 | 0.56 |
| 57 | 0.61 |
| 58 | 0.63 |
| 59 | 0.65 |
| 60 | 0.66 |
| 61 | 0.67 |
| 62 | 0.68 |
| 63 | 0.68 |
| 64 | 0.68 |
| 65 | 0.72 |
| 66 | 0.72 |
| 67 | 0.72 |
| 68 | 0.76 |
| 69 | 0.79 |
| 70 | 0.85 |
| 71 | 0.86 |
| 72 | 0.87 |
| 73 | 0.87 |
| 74 | 0.94 |
| 75 | 0.95 |
| 76 | 1.03 |
| 77 | 1.10 |
| 78 | 1.11 |
| 79 | 1.12 |
| 80 | 1.20 |
| 81 | 1.21 |
| 82 | 1.22 |
| 83 | 1.24 |
| 84 | 1.28 |
| 85 | 1.36 |
| 86 | 1.37 |
| 87 | 1.41 |
| 88 | 1.41 |
| 89 | 1.49 |
| 90 | 1.52 |
| 91 | 1.53 |
| 92 | 1.62 |
| 93 | 1.66 |
| 94 | 1.73 |
| 95 | 1.76 |
| 96 | 1.82 |
| 97 | 1.84 |
| 98 | 0.387 |
| 99 | 0.361 |
| 100 | 0.195 |
| 101 | 0.338 |
| 102 | 0.445 |
| 103 | 0.604 |
| 104 | 0.783 |
| 105 | 0.525 |
| 106 | 0.611 |
| 107 | 0.175 |
| 108 | 0.0587 |
| 109 | 0.213 |
| 110 | 0.207 |
| 111 | 0.185 |
| 112 | 0.356 |
| 113 | 0.707 |
| 114 | 0.637 |
| 115 | 0.295 |
| 116 | 0.213 |
| 117 | 0.215 |
| 118 | 0.213 |
| 119 | 0.236 |
| 120 | 0.527 |
| 121 | 0.375 |
| 122 | 0.235 |
| 123 | 0.216 |
| 124 | 0.103 |
| 125 | 0.361 |
| 126 | 0.176 |
| 127 | 0.364 |
| 128 | 0.358 |
| 129 | 0.355 |
| 130 | 0.252 |
| 131 | 0.103 |
| 132 | 0.139 |
| 133 | 0.146 |
| 134 | 0.327 |
| 135 | 0.393 |
| 136 | 0.173 |
| 137 | 0.334 |
| 138 | 0.408 |
| 139 | 0.172 |
| 140 | 0.334 |
| 141 | 0.242 |
| 142 | 0.0695 |
| 143 | 0.682 |
| 144 | 0.269 |
| 145 | 0.205 |
| 146 | 0.311 |
| 147 | 0.181 |
| 148 | 0.349 |
| 149 | 0.744 |
| 150 | 0.217 |
| 151 | 0.403 |
| 152 | 0.421 |
| 153 | 0.384 |
| 154 | 0.172 |
| 155 | 0.329 |
| 156 | 0.156 |
| 157 | 0.368 |
| 158 | 0.783 |
| 159 | 0.647 |
| 160 | 0.474 |
| 161 | 0.390 |
| 162 | 0.203 |
| 163 | 0.190 |
| 164 | 0.163 |
| 165 | 0.649 |
| 166 | 0.152 |
| 167 | 0.715 |
| 168 | 0.555 |
| 169 | 0.466 |
| 170 | 0.199 |
| 171 | 0.281 |
| 172 | 0.194 |
| 173 | 0.352 |
| 174 | 0.430 |
| 175 | 0.617 |
| 176 | 0.0881 |
| 177 | 0.201 |
| 178 | 0.135 |
| 179 | 0.0958 |
| 180 | 0.135 |
| 181 | 0.244 |
| 182 | 0.201 |
| 183 | 0.108 |
| 184 | 0.0642 |
| 185 | 0.196 |
| 186 | 0.160 |
| 187 | 0.745 |
| 188 | 0.540 |
| 189 | 0.0532 |
| 190 | 0.199 |
| 191 | 0.315 |
| 192 | 0.265 |
| 193 | 0.371 |
| 194 | 0.168 |
| 195 | 0.330 |
| 196 | 0.185 |
| 197 | 0.171 |
| 198 | 0.370 |
| 199 | 0.308 |
| 200 | 0.400 |
| 201 | 0.682 |
| 202 | 0.231 |

### Example 203

A compound of formula (I) can be used in a manner known per se as the active ingredient for the production of tablets of the following composition:

### Per tablet

| | |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Example 204

A compound of formula (I) can be used in a manner known per se as the active ingredient for the production of capsules of the following composition:

### Per capsule

| | |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

### Example 205

A compound of formula (I) can be used in a manner known per se as the active ingredient for the production of an infusion solution of the following composition:

| | |
|---|---|
| Active ingredient | 100 mg |
| Lactic acid 90% | 100 mg |
| NaOH q.s. or HCl q.s. for adjustment to pH 4.0 | |
| Sodium chloride q.s. or glucose q.s. for adjustment of the osmolality to 290 mOsm/kg | |
| Water for injection (WFI) | ad 100 ml |

### Example 206

A compound of formula (I) can be used in a manner known per se as the active ingredient for the production of an infusion solution of the following composition:

| | |
|---|---|
| Active ingredient | 100 mg |
| Hydroxypropyl-beta-cyclodextrin | 10 g |
| NaOH q.s. or HCl q.s. for adjustment to pH 7.4 | |
| Sodium chloride q.s. or glucose q.s. for adjustment of the osmolality to 290 mOsm/kg | |
| Water for injection (WFI) | ad 100 ml |

## Claims

1. A compound of formula (I) 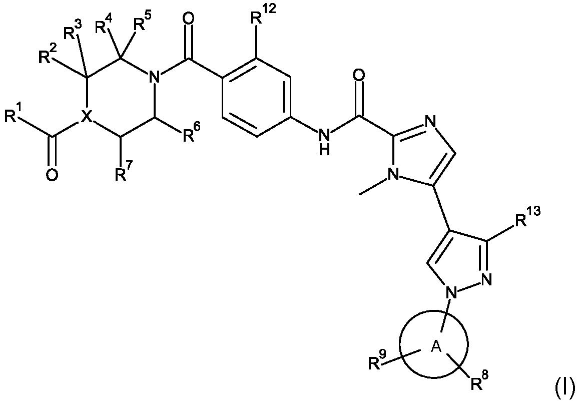 or a pharmaceutically acceptable salt thereof, wherein:
X and Y are each independently selected from N and CH;
n is selected from 0 to 6;
A is selected from 5- to 14-membered heteroaryl and C₆-C₁₄-aryl;
B is selected from 3- to 14-membered heterocyclyl, 5- to 14-membered heteroaryl and C₃-C₁₀-cycloalkyl;
L is selected from a covalent bond, carbonyl, NHC(O), C(O)NH, CH₂NH, NHCH₂ and NH;
L¹ is -O- or a covalent bond;
R¹ is selected from
(i) C₁-C₆-alkyl substituted with one amino and one carboxy substituent;
(ii) C₁-C₆-alkyl substituted with one amino and one carboxy-C₁-C₆-alkyl-NH-substituent;
(iii) C₁-C₆-alkyl-N(R^{1a}R^{1b});
(iv) -L¹-C₁-C₆-alkyl-N⁺(R^{1c}R^{1d}R^{1e});
(v) a group 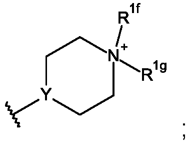 and
(vi) a group 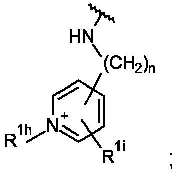
R^{1a} is amino-C₁-C₆-alkyl;
R^{1b} is carboxy-C₁-C₆-alkyl;
R^{1c} is selected from amino-C₁-C₆-alkyl and (3- to 14-membered heterocyclyl)-C₁-C₆-alkyl-;
R^{1d} is carboxy-C₁-C₆-alkyl;
R^{1e} is selected from C₁-C₆-alkyl, amino-C₁-C₆-alkyl and (3- to 14-membered heterocyclyl)-C₁-C₆-alkyl-;
R^{1f} is selected from C₁-C₆-alkyl, hydroxy-C₁-C₆-alkyl, amino-C₁-C₆-alkyl, C₁-C₆-alkyl-NH-C₁-C₆-alkyl, (C₁-C₆-alkyl)₂N-C₁-C₆-alkyl, and (3- to 14-membered heterocyclyl)-C₁-C₆-alkyl-,
R^{1g} is carboxy-C₁-C₆-alkyl;
R^{1h} is carboxy-C₁-C₆-alkyl;
R¹ⁱ is amino-C₁-C₆-alkyl;
R² is selected from hydrogen, C₁-C₆-alkyl and C₁-C₆-alkoxy; and R³ and R⁷ are both hydrogen; or
R² and R⁷, taken together with the atoms to which they are attached, form a 4- to 14-membered heterocycle or a C₄-C₁₀-cycloalkyl; and R³ is hydrogen; or
R² and R³, taken together with the carbon atom to which they are attached, form a C₃-C₁₀-cycloalkyl; and R⁷ is hydrogen;
R⁴ is selected from hydrogen and C₁-C₆-alkyl; and R⁵ and R⁶ are both hydrogen; or
R⁴ and R⁶, taken together with the atoms to which they are attached, form a 4- to 14-membered heterocycle; and R⁵ is hydrogen; or
R⁴ and R⁵, taken together with the carbon atom to which they are attached, form a C₃-C₁₀-cycloalkyl; and R⁶ is hydrogen;
R⁸ is selected from hydrogen, cyano, hydroxy, amino, C₁-C₆-alkyl-NH-, (C₁-C₆-alkyl)₂N-, (C₁-C₆-alkyl)₂N-SO₂-, (C₁-C₆-alkyl)₂N-C₁-C₆-alkyl-, amino-C₁-C₆-alkyl, amino-C₁-C₆-alkoxy, amino-C₁-C₆-alkyl-NH-, (amino-C₁-C₆-alkyl)₂N-, (amino-C₁-C₆-alkyl)₂N-C₁-C₆-alkyl-NH-, C₁-C₆-alkyl-NH-C₁-C₆-alkyl-NH-, C₁-C₆-alkoxy-C₁-C₆-alkyl-NH-, halo-C₁-C₆-alkoxy-C₁-C₆-alkyl-NH-, C₃-C₁₀-cycloalkyl-NH-, C₁-C₆-alkoxy, C₁-C₆-alkyl, cyano-C₁-C₆-alkyl-, hydroxy-C₁-C₆-alkyl-, hydroxy-C₁-C₆-alkyl-NH-, hydroxy-C₁-C₆-alkyl-NHC(O)-, halo-hydroxy-C₁-C₆-alkyl-NHC(O)-, carbamoyl, hydroxycarbamoyl, C₁-C₆-alkyl-NHC(O)-, (C₁-C₆-alkyl)₂NC(O)-, carbamoyl-C₁-C₆-alkyl-, and a group 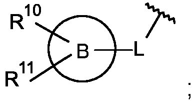
R⁹ is selected from hydrogen, halogen, amino, C₁-C₆-alkyl, and hydroxy-C₁-C₆-alkyl-;
R¹⁰ is selected from hydrogen, C₁-C₆-alkyl, halogen, hydroxy, oxo, imino, and amino;
R¹¹ is selected from hydrogen and halogen;
R¹² is selected from halogen and C₁-C₆-alkyl;
R¹³ is halo-C₁-C₆-alkyl.

2. The compound of formula (I) according to claim 1, or a pharmaceutically acceptable salt thereof, wherein L is selected from a covalent bond, carbonyl, NHC(O), CH₂NH and NH.

3. The compound of formula (I) according to any one of claims 1 or 2, or a pharmaceutically acceptable salt thereof, wherein:
R^{1f} is selected from C₁-C₆-alkyl, hydroxy-C₁-C₆-alkyl, amino-C₁-C₆-alkyl, (C₁-C₆-alkyl)₂N-C₁-C₆-alkyl, and (3- to 14-membered heterocyclyl)-C₁-C₆-alkyl-.

4. The compound of formula (I) according to any one of claims 1 or 2, or a pharmaceutically acceptable salt thereof, wherein:
R¹ is selected from
(i) C₁-C₆-alkyl-N⁺(R^{1c}R^{1d}R^{1e}); and
(ii) a group 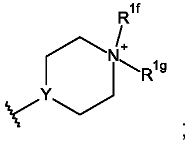 wherein
Y is CH;
R^{1c} and R^{1e} are both amino-C₁-C₆-alkyl;
R¹⁴ and R^{1g} are both carboxy-C₁-C₆-alkyl; and
R^{1f} is selected from amino-C₁-C₆-alkyl and (3- to 14-membered heterocyclyl)-C₁-C₆-alkyl-.

5. The compound of formula (I) according to claim 3, or a pharmaceutically acceptable salt thereof, wherein:
R¹ is selected from
(i) -(CH₂)₃-N⁺(R^{1c}R^{1d}R^{1e}); and
(ii) a group 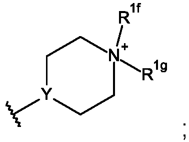 wherein
Y is CH;
R^{1c} and R^{1e} are both 4-aminobutyl;
R^{1d} and R^{1g} are both carboxymethyl; and
R^{1f} is selected from 3-aminopropyl and azetidin-3-ylmethyl.

6. The compound of formula (I) according to any one of claims 1 to 5, or a pharmaceutically acceptable salt thereof, wherein R², R³, R⁴, R⁵, R⁶ and R⁷ are all hydrogen.

7. The compound of formula (I) according to any one of claims 1 to 6, or a pharmaceutically acceptable salt thereof, wherein:
A is selected from thiazolyl; isothiazolyl; pyridyl; 1H-pyrrolo[3,2-b]pyridine; 1H-imidazo[4,5-b]pyridine; 1,3-benzothiazole; benzothiophene; 1H-pyrazolo[4,3-b]pyridine; 1H-pyrrolo[3,2-c]pyridine; 1H-pyrrolo[2,3-b]pyridine, 1H-pyrrolo[2,3-c]pyridine, 2H-pyrazolo[4,3-c]pyridine, 1H-pyrrolo[3,2-d]pyrimidine, 1H-indazolyl; furo[3,2-b]pyridine; 2,3-dihydro-1H-pyrrolo[3,2-b]pyridine; and phenyl; and
B is selected from morpholine, thiomorpholine, piperazine, pyrrolidinyl, piperidyl, pyridyl, cyclopropyl, cyclopentyl, 1,2,3,3a,4,5,6,6a-octahydropyrrolo[3,4-c]pyrrole, 9-oxa-3,7-diazabicyclo[3.3.1]nonane, 7-oxa-2-azaspiro[3.5]nonane, and 2,6-dioxa-9-azaspiro[4.5]decane.

8. The compound of formula (I) according to any one of claims 1 to 7, or a pharmaceutically acceptable salt thereof, wherein X is N.

9. The compound of formula (I) according to any one of claims 1 to 8, or a pharmaceutically acceptable salt thereof, wherein:
R¹² is selected from chloro and methyl; and
R¹³ is selected from CHF₂ and CF₃.

10. The compound of formula (I) according to claim 1, or a pharmaceutically acceptable salt thereof, wherein:
X and Y are each independently selected from N and CH;
n is selected from 0 to 6;
A is selected from 5- to 14-membered heteroaryl and C₆-C₁₄-aryl;
B is selected from 3- to 14-membered heterocyclyl, 5- to 14-membered heteroaryl and C₃-C₁₀-cycloalkyl;
L is selected from a covalent bond, carbonyl, NHC(O), CH₂NH and NH;
L¹ is -O- or a covalent bond;
R¹ is selected from
(i) C₁-C₆-alkyl substituted with one amino and one carboxy substituent;
(ii) C₁-C₆-alkyl substituted with one amino and one carboxy-C₁-C₆-alkyl-NH-substituent;
(iii) C₁-C₆-alkyl-N(R^{1a}R^{1b});
(iv) -L¹-C₁-C₆-alkyl-N⁺(R^{1c}R^{1d}R^{1e});
(v) a group 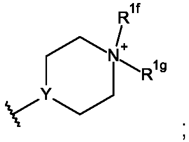 and
(vi) a group 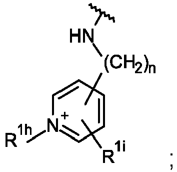
R^{1a} is amino-C₁-C₆-alkyl;
R^{1b} is carboxy-C₁-C₆-alkyl;
R^{1c} is selected from amino-C₁-C₆-alkyl and (3- to 14-membered heterocyclyl)-C₁-C₆-alkyl-;
R^{1d} is carboxy-C₁-C₆-alkyl;
R^{1e} is selected from C₁-C₆-alkyl, amino-C₁-C₆-alkyl and (3- to 14-membered heterocyclyl)-C₁-C₆-alkyl-;
R^{1f} is selected from C₁-C₆-alkyl, hydroxy-C₁-C₆-alkyl, amino-C₁-C₆-alkyl, (C₁-C₆-alkyl)₂N-C₁-C₆-alkyl, and (3- to 14-membered heterocyclyl)-C₁-C₆-alkyl-;
R^{1g} is carboxy-C₁-C₆-alkyl;
R^{1h} is carboxy-C₁-C₆-alkyl;
R¹ⁱ is amino-C₁-C₆-alkyl;
R² is selected from hydrogen, C₁-C₆-alkyl and C₁-C₆-alkoxy; and R³ and R⁷ are both hydrogen; or
R² and R⁷, taken together with the atoms to which they are attached, form a 4- to 14-membered heterocycle or a C₄-C₁₀-cycloalkyl; and R³ is hydrogen; or
R² and R³, taken together with the carbon atom to which they are attached, form a C₃-C₁₀-cycloalkyl; and R⁷ is hydrogen;
R⁴ is selected from hydrogen and C₁-C₆-alkyl; and R⁵ and R³ are both hydrogen; or
R⁴ and R⁶, taken together with the atoms to which they are attached, form a 4- to 14-membered heterocycle; and R⁵ is hydrogen; or
R⁴ and R⁵, taken together with the carbon atom to which they are attached, form a C₃-C₁₀-cycloalkyl; and R⁶ is hydrogen;
R⁸ is selected from hydrogen, cyano, amino, hydroxy, C₁-C₆-alkyl-NH-, (C₁-C₆-alkyl)₂N-, (C₁-C₆-alkyl)₂N-SO₂-, (C₁-C₆-alkyl)₂N-C₁-C₆-alkyl-, amino-C₁-C₆-alkyl, amino-C₁-C₆-alkoxy, amino-C₁-C₆-alkyl-NH-, (amino-C₁-C₆-alkyl)₂N-, (amino-C₁-C₆-alkyl)₂N-C₁-C₆-alkyl-NH-, C₁-C₆-alkyl-NH-C₁-C₆-alkyl-NH-, C₁-C₆-alkoxy-C₁-C₆-alkyl-NH-, halo-C₁-C₆-alkoxy-C₁-C₆-alkyl-NH-, C₃-C₁₀-cycloalkyl-NH-, C₁-C₆-alkoxy, C₁-C₆-alkyl, cyano-C₁-C₆-alkyl-, hydroxy-C₁-C₆-alkyl-, hydroxy-C₁-C₆-alkyl-NH-, hydroxy-C₁-C₆-alkyl-NHC(O)-, halo-hydroxy-C₁-C₆-alkyl-NHC(O)-, carbamoyl, hydroxycarbamoyl, C₁-C₆-alkyl-NHC(O)-, (C₁-C₆-alkyl)₂NC(O)-, carbamoyl-C₁-C₆-alkyl-, and a group 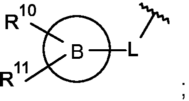
R⁹ is selected from hydrogen, halogen amino, C₁-C₆-alkyl, and hydroxy-C₁-C₆-alkyl-;
R¹⁰ is selected from hydrogen, C₁-C₆-alkyl, halogen, hydroxy, oxo, imino, and amino;
R¹¹ is selected from hydrogen and halogen;
R¹² is selected from halogen and C₁-C₆-alkyl;
R¹³ is halo-C₁-C₆-alkyl.

11. The compound of formula (I) according to claim 10, or a pharmaceutically acceptable salt thereof, wherein:
A is selected from 5- to 14-membered heteroaryl and C₆-C₁₄-aryl;
B is 3- to 14-membered heterocyclyl;
L is a covalent bond;
X is N;
Y is CH;
R¹ is selected from
(i) C₁-C₆-alkyl-N⁺(R^{1c}R^{1d}R^{1e}); and
(ii) a group 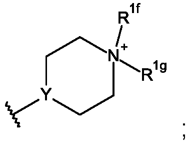
R^{1c} and R^{1e} are both amino-C₁-C₆-alkyl;
R^{1d} and R^{1g} are both carboxy-C₁-C₆-alkyl;
R^{1f} is selected from amino-C₁-C₆-alkyl and (3- to 14-membered heterocyclyl)-C₁-C₆-alkyl-;
R², R³, R⁴, R⁵, R⁶ and R⁷ are all hydrogen;
R⁸ is selected from hydrogen, amino, hydroxy, C₁-C₆-alkyl-NH-, amino-C₁-C₆-alkyl-NH-, hydroxy-C₁-C₆-alkyl-, hydroxy-C₁-C₆-alkyl-NH-, C₁-C₆-alkoxy-C₁-C₆-alkyl-NH-, carbamoyl-C₁-C₆-alkyl-, (C₁-C₆-alkyl)₂NC(O)-, and a group 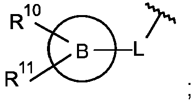
R⁹ is selected from hydrogen and halogen;
R¹⁰ is amino;
R¹¹ is hydrogen;
R¹² is selected from halogen and C₁-C₆-alkyl; and
R¹³ is halo-C₁-C₆-alkyl.

12. The compound of formula (I) according to claim 1, or a pharmaceutically acceptable salt thereof, wherein the compound of formula (I) is selected from:
bis(4-aminobutyl)-(carboxymethyl)-[4-[4-[2-chloro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazino]-4-keto-butyl]ammonium⁻,
cis-2-[4-[4-[4-[[5-[1-[5-(2-aminoethylamino)-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
bis(4-aminobutyl)-[4-[4-[4-[[5-[1-(4-amino-2-fluoro-phenyl)-3-(trifluoromethyl)pyrazol-4-yl]-1-methyl-imidazole-2-carbonyl]amino]-2-chlorobenzoyl]piperazino]-4-keto-butyl]-(carboxymethyl)ammonium;
2-[4-[4-[4-[[5-[1-[5-(2-aminoethylamino)-2-pyridyl]-3-(trifluoromethyl)pyrazol-4-yl]-1-methyl-imidazole-2-carbonyl]amino]-2-chloro-benzoyl] piperazine-1-carbonyl]-1-(azetidin-3-ylmethyl)piperidin-1-ium-1-yl]acetic acid;
bis(3-aminopropyl)-(carboxymethyl)-[4-[4-[2-chloro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazino]-4-keto-butyl]ammonium⁻,
cis-2-[1-(azetidin-3-ylmethyl)-4-[4-[2-chloro-4-[[1-methyl-5-[1-(1H-pyrrolo[3,2-c]pyridin-6-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[4-[4-[4-[[5-[1-[5-(3-aminopropylamino)-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
cis-2-[4-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl] piperazine-1-carbonyl]-1-(azetidin-3-ylmethyl)piperidin-1-ium-1-yl]acetic acid;
2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[1-(azetidin-3-ylmethyl)-4-[4-[2-chloro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
2-[4-[4-[4-[[5-[1-[5-[[(2S)-2-aminopropyl]amino]-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
bis(4-aminobutyl)-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-ketobutyl]-(carboxymethyl)ammonium;
2-[4-[4-[4-[[5-[1-[5-(aminomethyl)-2-pyridyl]-3-(trifluoromethyl)pyrazol-4-yl]-1-methyl-imidazole-2-carbonyl]amino]-2-chloro-benzoyl] piperazine-1-carbonyl]-1-(azetidin-3-ylmethyl)piperidin-1-ium-1-yl]acetic acid;
2-[1-(azetidin-3-ylmethyl)-4-[4-[2-chloro-4-[[5-[1-(5-methoxy-2-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[4-[4-[4-[[5-[1-[5-(2-aminoethyl)-2-pyridyl]-3-(trifluoromethyl)pyrazol-4-yl]-1-methyl-imidazole-2-carbonyl]amino]-2-chloro-benzoyl] piperazine-1-carbonyl]-1-(azetidin-3-ylmethyl)piperidin-1-ium-1-yl]acetic acid;
2-[4-[4-[4-[[5-[1-[5-(2-aminoethoxy)-2-pyridyl]-3-(trifluoromethyl)pyrazol-4-yl]-1-methyl-imidazole-2-carbonyl]amino]-2-chloro-benzoyl] piperazine-1-carbonyl]-1-(azetidin-3-ylmethyl)piperidin-1-ium-1-yl]acetic acid;
2-[1-(azetidin-3-ylmethyl)-4-[4-[2-chloro-4-[[1-methyl-5-[1-[5-(2-pyridylmethylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[4-[4-[4-[[5-[1-[5-[(1-aminocyclopentyl)methylamino]-2-pyridyl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
2-[1-(azetidin-3-ylmethyl)-4-[4-[2-chloro-4-[[1-methyl-5-[1-[5-(methylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-(5-morpholino-2-pyridyl)-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-[5-[[(2S)-pyrrolidin-2-yl]methylamino]-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[5-(methylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid;
cis-2-[1-(azetidin-3-ylmethyl)-4-[4-[2-chloro-4-[[5-[1-[5-(2-methoxyethylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
trans-2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chlorobenzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-(5-piperazino-2-pyridyl)-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-[5-[2-(methylamino)ethylamino]-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
cis-2-[4-[4-[4-[[5-[1-(5-amino-2-pyridyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
bis(3-aminopropyl)-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-ketobutyl]-(carboxymethyl)ammonium;
bis(4-aminobutyl)-[4-[4-[4-[[5-[1-[5-(2-aminoethylamino)-2-pyridyl]-3-(trifluoromethyl)pyrazol-4-yl]-1-methyl-imidazole-2-carbonyl]amino]-2-chlorobenzoyl]piperazino]-4-keto-butyl]-(carboxymethyl)ammonium;
2-[1-(azetidin-3-ylmethyl)-4-[4-[2-chloro-4-[[5-[1-(4-methoxyphenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[5-(2-hydroxyethylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(1-methylpyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-[5-(isopropylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-[5-(2-pyridylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(1H-pyrazolo[4,3-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-thiazol-2-yl-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-[5-(cyclopropylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-[5-(dimethylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-[4-(dimethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-[5-[[(3S)-morpholin-3-yl]methylamino]-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chlorobenzoyl]piperazine-1-carbonyl]piperidin-1-ium-1-yl]acetic acid;
2-[1-(azetidin-3-ylmethyl)-4-[4-[2-chloro-4-[[5-[1-[6-(dimethylamino)-3-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
[4-[4-[4-[[5-[1-(5-aminopyridin-2-yl)-3-(trifluoromethyl)pyrazol-4-yl]-1-methylimidazole-2-carbonyl]amino]-2-chlorobenzoyl]piperazin-1-yl]-4-oxobutyl]-bis(azetidin-3-ylmethyl)-(carboxymethyl)azanium;
2-[4-[4-[4-[[5-[1-[5-(6-aminohexylamino)-2-pyridyl]-3-(trifluoromethyl)pyrazol-4-yl]-1-methyl-imidazole-2-carbonyl]amino]-2-chloro-benzoyl] piperazine-1-carbonyl]-1-(azetidin-3-ylmethyl)piperidin-1-ium-1-yl]acetic acid;
2-[1-(azetidin-3-ylmethyl)-4-[4-[2-chloro-4-[[1-methyl-5-[1-[4-(methylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-[5-(4-methylpiperazino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-(5-hydroxy-2-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-(1H-imidazo[4,5-b]pyridin-5-yl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[4-[4-[4-[[5-[1-(6-amino-2-pyridyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[1-(3-aminopropyl)-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid;
2-[1-(azetidin-3-ylmethyl)-4-[4-[2-chloro-4-[[1-methyl-5-[1-[5-(4-piperidylmethylamino)-2-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-[5-[[(2S,4S)-4-fluoropyrrolidin-2-yl]methylamino]-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[4-[4-[4-[[5-[1-(5-amino-3-fluoro-2-pyridyl)-3-(trifluoromethyl)pyrazol-4-yl]-1-methyl-imidazole-2-carbonyl]amino]-2-chloro-benzoyl] piperazine-1-carbonyl]-1-(azetidin-3-ylmethyl)piperidin-1-ium-1-yl]acetic acid;
2-[4-[4-[4-[[5-[1-(5-amino-2-pyridyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
4-[4-[4-[4-[[5-[1-(5-amino-2-pyridyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]butyric acid;
2-[1-(azetidin-3-ylmethyl)-4-[4-[2-chloro-4-[[5-[1-(2,3-dihydro-1H-pyrrolo[3,2-b]pyridin-5-yl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-(1H-indazol-3-yl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-furo[3,2-b]pyridin-5-yl-3-(trifluoromethyl)pyrazol-4-yl]-1-methyl-imidazole-2-carbonyl]amino]benzoyl]piperazine-1-carbonyl]piperidin-1-ium-1-yl]acetic acid;
trans-2-[4-[4-[4-[[5-[1-(5-amino-2-pyridyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[1-(azetidin-3-ylmethyl)-4-[4-[2-chloro-4-[[5-[1-[5-[(2,2-difluorocyclopropyl)methylamino]-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
3-aminopropyl-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyl]-(carboxymethyl)-methyl-ammonium;
bis(3-aminopropyl)-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-ketobutyl]-(carboxymethyl)ammonium;
2-[1-(azetidin-3-ylmethyl)-4-[4-[2-chloro-4-[[5-[1-(5,6-diamino-2-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-[6-(methylamino)-3-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[5-[1-[5-(cyclobutylamino)-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[2-(3-aminopropyl)-4-[3-[[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazine-1-carbonyl]amino]propyl]pyridin-1-ium-1-yl]acetic acid;
2-[4-[4-[4-[[5-[1-(4-amino-2-pyridyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[3-aminopropyl-[4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-ketobutyl]amino]acetic acid;
cis-2-[4-[4-[4-[[5-[1-[5-(2-aminoethylamino)-2-pyridyl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
2-[4-[4-[4-[[5-[1-(2-amino-4-pyridyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[1-(3-aminopropyl)-4-[1-[4-[[5-[1-(5-amino-2-pyridyl)-3-(trifluoromethyl)pyrazol-4-yl]-1-methyl-imidazole-2-carbonyl]amino]-2-chloro-benzoyl]piperidine-4-carbonyl]piperazin-1-ium-1-yl]acetic acid;
2-[1-(azetidin-3-ylmethyl)-4-[4-[2-chloro-4-[[5-[1-(5-cyano-2-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[4-[(2S)-4-[4-[[5-[1-(5-amino-2-pyridyl)-3-(trifluoromethyl)pyrazol-4-yl]-1-methylimidazole-2-carbonyl]amino]-2-chloro-benzoyl]-2-methyl-piperazine-1-carbonyl]-1-(azetidin-3-ylmethyl)piperidin-1-ium-1-yl]acetic acid;
2-[4-[(3R)-4-[4-[[5-[1-(5-amino-2-pyridyl)-3-(trifluoromethyl)pyrazol-4-yl]-1-methylimidazole-2-carbonyl]amino]-2-chloro-benzoyl]-3-ethyl-piperazine-1-carbonyl]-1-(azetidin-3-ylmethyl)piperidin-1-ium-1-yl]acetic acid;
2-[4-[(3S)-4-[4-[[5-[1-(5-amino-2-pyridyl)-3-(trifluoromethyl)pyrazol-4-yl]-1-methylimidazole-2-carbonyl]amino]-2-chloro-benzoyl]-3-methyl-piperazine-1-carbonyl]-1-(azetidin-3-ylmethyl)piperidin-1-ium-1-yl]acetic acid;
2-[1-(azetidin-3-ylmethyl)-4-[4-[4-[[5-[1-(1,3-benzothiazol-2-yl)-3-(trifluoromethyl)pyrazol-4-yl]-1-methyl-imidazole-2-carbonyl]amino]-2-chlorobenzoyl]piperazine-1-carbonyl]piperidin-1-ium-1-yl]acetic acid;
2-[[(55)-5-amino-6-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-6-ketohexyl]amino]acetic acid;
2-[1-(azetidin-3-ylmethyl)-4-[4-[2-chloro-4-[[5-[1-(6-methoxy-3-pyridyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
(2S)-2-amino-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyric acid;
2-[1-(azetidin-3-ylmethyl)-4-[4-[2-chloro-4-[[5-[1-[5-[(dimethylamino)methyl]-2-pyridyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
(3R)-3-amino-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyric acid;
2-[4-[7-[4-[[5-[1-(5-amino-2-pyridyl)-3-(trifluoromethyl)pyrazol-4-yl]-1-methylimidazole-2-carbonyl]amino]-2-chloro-benzoyl]-4,7-diazaspiro[2.5]octane-4-carbonyl]-1-(azetidin-3-ylmethyl)piperidin-1-ium-1-yl]acetic acid;
2-[4-[1-[4-[[5-[1-(5-amino-2-pyridyl)-3-(trifluoromethyl)pyrazol-4-yl]-1-methylimidazole-2-carbonyl]amino]-2-chloro-benzoyl]piperidine-4-carbonyl]-1-(azetidin-3-ylmethyl)piperazin-1-ium-1-yl]acetic acid;
2-[1-(azetidin-3-ylmethyl)-4-[4-[2-chloro-4-[[1-methyl-5-[1-[2-(methylamino)-4-pyridyl]-3-(trifluoromethyl)pyrazol-4-yl]imidazole-2-carbonyl]amino]benzoyl]piperazine-1-carbonyl]piperidin-1-ium-1-yl]acetic acid;
2-[4-[4-[4-[[5-[1-(6-amino-3-pyridyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[4-[8-[4-[[5-[1-(5-amino-2-pyridyl)-3-(trifluoromethyl)pyrazol-4-yl]-1-methylimidazole-2-carbonyl]amino]-2-chloro-benzoyl]-3,8-diazabicyclo[3.2.1]octane-3-carbonyl]-1-(azetidin-3-ylmethyl)piperidin-1-ium-1-yl]acetic acid;
(2S)-2-amino-5-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-5-keto-valeric acid;
(3S)-3-amino-4-[4-[4-[[5-[1-(5-amino-2-pyridyl)-3-(trifluoromethyl)pyrazol-4-yl]-1-methyl-imidazole-2-carbonyl]amino]-2-chloro-benzoyl]piperazino]-4-keto-butyric acid;
2-[4-[4-[4-[[5-[1-[6-(2-aminoethylamino)-3-pyridyl]-3-(trifluoromethyl)pyrazol-4-yl]-1-methyl-imidazole-2-carbonyl]amino]-2-chloro-benzoyl] piperazine-1-carbonyl]-1-(azetidin-3-ylmethyl)piperidin-1-ium-1-yl]acetic acid;
2-[4-[3-[4-[[5-[1-(5-amino-2-pyridyl)-3-(trifluoromethyl)pyrazol-4-yl]-1-methylimidazole-2-carbonyl]amino]-2-chloro-benzoyl]-3,9-diazabicyclo[3.3.1]nonane-9-carbonyl]-1-(azetidin-3-ylmethyl)piperidin-1-ium-1-yl]acetic acid;
2-[4-[4-[4-[[5-[1-(5-amino-2-pyridyl)-3-(trifluoromethyl)pyrazol-4-yl]-1-methylimidazole-2-carbonyl]amino]-2-chloro-benzoyl]-4,7-diazaspiro[2.5]octane-7-carbonyl]-1-(azetidin-3-ylmethyl)piperidin-1-ium-1-yl]acetic acid;
2-[4-[4-[4-[[5-[1-(4-aminoisothiazol-5-yl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[1-(azetidin-3-ylmethyl)-4-[4-[4-[[5-[1-(benzothiophen-2-yl)-3-(trifluoromethyl)pyrazol-4-yl]-1-methyl-imidazole-2-carbonyl]amino]-2-chlorobenzoyl]piperazine-1-carbonyl]piperidin-1-ium-1-yl]acetic acid;
2-[2-(3-aminopropyl)-4-[3-[[1-[4-[[5-[1-(5-amino-2-pyridyl)-3-(trifluoromethyl)pyrazol-4-yl]-1-methyl-imidazole-2-carbonyl]amino]-2-chloro-benzoyl]piperidine-4-carbonyl]amino]propyl]pyridin-1-ium-1-yl]acetic acid;
2-[4-[4-[4-[[5-[1-(5-amino-2-pyridyl)-3-(trifluoromethyl)pyrazol-4-yl]-1-methylimidazole-2-carbonyl]amino]-2-chloro-benzoyl]-2-(methoxymethyl)piperazine-1-carbonyl]-1-(azetidin-3-ylmethyl)piperidin-1-ium-1-yl]acetic acid;
cis-2-[1-(3-aminopropyl)-4-[4-[2-chloro-4-[[5-[1-[2-(3-hydroxypropyl)-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[4-[4-[4-[[5-[1-[2-(3-amino-3-oxo-propyl)-1H-pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(3-aminopropyl)piperidin-1-ium-1-yl]acetic acid;
cis-2-[1-(3-aminopropyl)-4-[4-[2-chloro-4-[[5-[1-(2-fluoro-4-morpholino-phenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(3-aminopropyl)-4-[4-[4-[[5-[1-[2-[(1S)-1-hydroxyethyl]-1H-pyrrolo[3,2-b]pyridin-5-yl]-3-(trifluoromethyl)pyrazol-4-yl]-1-methyl-imidazole-2-carbonyl]amino]-2-methyl-benzoyl]piperazine-1-carbonyl]piperidin-1-ium-1-yl]acetic acid;
cis-2-[1-(3-aminopropyl)-4-[4-[2-chloro-4-[[5-[1-[2-[2-hydroxy-1-(hydroxymethyl)ethyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(3-aminopropyl)-4-[4-[2-chloro-4-[[1-methyl-5-[1-[1-methyl-2-(morpholine-4-carbonyl)pyrrolo[3,2-b]pyridin-5-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(3-aminopropyl)-4-[4-[2-chloro-4-[[5-[1-[2-(2-hydroxyethylcarbamoyl)-1-methyl-pyrrolo[3,2-b]pyridin-5-yl]-3-(trifluoromethyl)pyrazol-4-yl]-1-methylimidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-[(1S)-1,2-dihydroxyethyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-[(1R)-1,2-dihydroxyethyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[4-[[5-[1-[2-fluoro-4-(methylamino)phenyl]-3-(trifluoromethyl)pyrazol-4-yl]-1-methyl-imidazole-2-carbonyl]amino]-2-methylbenzoyl]piperazine-1-carbonyl]piperidin-1-ium-1-yl]acetic acid;
cis-2-[1-(azetidin-3-ylmethyl)-4-[4-[2-fluoro-4-[[5-[1-[2-fluoro-4-(methylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[4-[[5-[1-[4-(dimethylamino)-2-fluoro-phenyl]-3-(trifluoromethyl)pyrazol-4-yl]-1-methyl-imidazole-2-carbonyl]amino]-2-fluorobenzoyl]piperazine-1-carbonyl]piperidin-1-ium-1-yl]acetic acid;
cis-2-[1-(3-aminopropyl)-4-[4-[4-[[5-[1-(2-fluoro-4-hydroxy-phenyl)-3-(trifluoromethyl)pyrazol-4-yl]-1-methyl-imidazole-2-carbonyl]amino]-2-methylbenzoyl]piperazine-1-carbonyl]piperidin-1-ium-1-yl]acetic acid;
cis-2-[1-(azetidin-3-ylmethyl)-4-[4-[4-[[5-[1-[2-fluoro-4-(2-methoxyethylamino)phenyl]-3-(trifluoromethyl)pyrazol-4-yl]-1-methyl-imidazole-2-carbonyl]amino]-2-methyl-benzoyl]piperazine-1-carbonyl]piperidin-1-ium-1-yl]acetic acid;
cis-2-[4-[4-[4-[[5-[1-[2-[(3aS,6aR)-2,3,3a,4,6,6a-hexahydro-1H-pyrrolo[3,4-c]pyrrole-5-carbonyl]-1H-pyrrolo[3,2-c]pyridin-6-yl]-3-(trifluoromethyl)pyrazol-4-yl]-1-methyl-imidazole-2-carbonyl]amino]-2-chloro-benzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[1-methyl-5-[1-[2-(7-oxa-2-azaspiro[3.5]nonane-2-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-(1-imino-1-keto-1,4-thiazinane-4-carbonyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-(2-hydroxyethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[2-(9-oxa-3, 7-diazabicyclo[3.3.1]nonane-3-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[2-[(3,3,3-trifluoro-2-hydroxy-propyl)carbamoyl]-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[2-(piperazine-1-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-(2,2-difluoromorpholine-4-carbonyl)-1H-pyrrolo[3,2-c]pyridin-6-yl]-3-(trifluoromethyl)pyrazol-4-yl]-1-methylimidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-[(2-ketopyrrolidin-3-yl)carbamoyl]-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-[(5R)-2,6-dioxa-9-azaspiro[4.5]decane-9-carbonyl]-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[2-(thiomorpholine-4-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-(dimethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-fluoro-4-[[5-[1-[2-fluoro-4-(2-methoxyethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-(hydroxycarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[4-[2-(difluoromethoxy)ethylamino]-2-fluoro-phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(3-aminopropyl)-4-[4-[4-[[5-[1-[2-(2-hydroxyethyl)-1H-pyrrolo[3,2-b]pyridin-5-yl]-3-(trifluoromethyl)pyrazol-4-yl]-1-methyl-imidazole-2-carbonyl]amino]-2-methyl-benzoyl]piperazine-1-carbonyl]piperidin-1-ium-1-yl]acetic acid;
cis-2-[4-[4-[4-[[5-[1-(4-Amino-2-fluoro-phenyl)-3-(difluoromethyl)pyrazol-4-yl]-1-methyl-imidazole-2-carbonyl]amino]-2-chloro-benzoyl] piperazine-1-carbonyl]-1-(azetidin-3-ylmethyl)piperidin-1-ium-1-yl]acetic acid;
cis-2-[4-[4-[4-[[5-[1-[2-(2-aminoethyl)-1H-pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
2-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]oxyethylbis(3-aminopropyl)-(carboxymethyl)ammonium;
cis-2-[1-(3-aminopropyl)-4-[4-[2-fluoro-4-[[5-[1-(2-fluoro-4-hydroxy-phenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-methyl-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-fluoro-4-[[1-methyl-5-[1-(1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
2-[1-(3-aminopropyl)-4-[1-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]isonipecotoyl]piperazin-1-ium-1-yl]acetic acid;
2-[4-[1-[4-[[5-[1-(4-amino-2-fluoro-phenyl)-3-(trifluoromethyl)pyrazol-4-yl]-1-methylimidazole-2-carbonyl]amino]-2-chloro-benzoyl]isonipecotoyl]-1-(3-aminopropyl)piperazin-1-ium-1-yl]acetic acid;
cis-2-[1-(azetidin-3-ylmethyl)-4-[4-[2-fluoro-4-[[5-[1-(2-fluoro-4-hydroxy-phenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[4-[[(2S)-2, 3-dihydroxypropyl]amino]-2-fluoro-phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[4-[4-[4-[[5-[1-[1-(2-amino-1,1-dimethyl-2-oxo-ethyl)pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[1-methyl-5-[1-[2-(morpholine-4-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperazin-1-ium-1-yl]acetic acid;
2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-(3-aminopropyl)piperazin-1-ium-1-yl]acetic acid;
cis-2-[4-[4-[4-[[5-[1-[2-(2-aminoethyl)-1H-pyrrolo[3,2-c]pyridin-6-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[1-methyl-5-[1-[1-(methylcarbamoyl)pyrrolo[3,2-b]pyridin-5-yl]-3-(trifluoromethyl)pyrazol-4-yl]imidazole-2-carbonyl]amino]benzoyl]piperazine-1-carbonyl]piperidin-1-ium-1-yl]acetic acid;
2-[1-(azetidin-3-ylmethyl)-4-[4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperazin-1-ium-1-yl]acetic acid;
2-[1-(3-aminopropyl)-4-[4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperazin-1-ium-1-yl]acetic acid;
cis-2-[1-(azetidin-3-ylmethyl)-4-[4-[2-chloro-4-[[5-[1-[3-(2-hydroxyethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[3-(piperazine-1-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[3-(4-methylpiperazine-1-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[4-[4-[4-[[5-[1-[2-(2-amino-2-keto-ethyl)pyrazolo[4,3-c]pyridin-6-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[5-[1-[2-[(1R)-1-hydroxyethyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-fluoro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[4-[4-[4-[[5-[1-(4-amino-2-fluoro-phenyl)-3-(trifluoromethyl)pyrazol-4-yl]-1-methyl-imidazole-2-carbonyl]amino]-2-methyl-benzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-methyl-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-(2-hydroxyethyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[3-[(3S)-3-hydroxypyrrolidine-1-carbonyl]-1H-pyrrolo[3,2-c]pyridin-6-yl]-3-(trifluoromethyl)pyrazol-4-yl]-1-methylimidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[3-(methylcarbamoyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[4-[[5-[1-(3-carbamoyl-1H-pyrrolo[3,2-c]pyridin-6-yl)-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid;
cis-2-[1-(azetidin-3-ylmethyl)-4-[4-[2-chloro-4-[[5-[1-[1-(dimethylsulfamoyl)-2-(2-hydroxyethyl)pyrrolo[3,2-c]pyridin-6-yl]-3-(trifluoromethyl)pyrazol-4-yl]-1-methylimidazole-2-carbonyl]amino]benzoyl]piperazine-1-carbonyl]piperidin-1-ium-1-yl]acetic acid;
2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-(3-hydroxypropyl)piperidin-1-ium-1-yl]acetic acid;
cis-2-[1-(azetidin-3-ylmethyl)-4-[4-[2-chloro-4-[[5-[1-[2-[(2S)-2, 3-dihydroxypropyl]-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[4-[4-[4-[[5-[1-[1-(3-amino-2-methyl-3-oxo-propyl)pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[5-[1-[2-(2-hydroxyethyl)-1H-pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-(2-fluoro-4-hydroxy-phenyl)-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-fluoro-4-[(2-hydroxy-2-methyl-propyl)amino]phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(5H-pyrrolo[3,2-d]pyrimidin-2-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[4-[[5-[1-[5-[2-[bis(2-aminoethyl)amino]ethylamino]-2-pyridyl]-3-(trifluoromethyl)pyrazol-4-yl]-1-methyl-imidazole-2-carbonyl]amino]-2-chloro-benzoyl]piperazine-1-carbonyl]piperidin-1-ium-1-yl]acetic acid;
cis-2-[4-[4-[4-[[5-[1-[2-(2-amino-2-oxo-ethyl)-1H-pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[4-[4-[4-[[5-[1-[1-(2-amino-2-keto-ethyl)pyrrolo[3,2-c]pyridin-6-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[5-[1-[1-(cyanomethyl)-2-methylolpyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[4-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl] piperazine-1-carbonyl]-1-(3-aminopropyl)piperidin-1-ium-1-yl]acetic acid;
cis-2-[1-(3-aminopropyl)-4-[4-[2-chloro-4-[[1-methyl-5-[1-(2-methylol-1H-pyrrolo[3,2-b]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[3-(dimethylcarbamoyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(1H-pyrazolo[4,3-c]pyridin-6-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[1-(2-hydroxyethyl)pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[1-(2-hydroxyethyl)-2-methylolpyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-fluoro-4-(methylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-[3-(morpholine-4-carbonyl)-1H-pyrrolo[3,2-c]pyridin-6-yl]-3-(trifluoromethyl)pyrazol-4-yl]imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[2-fluoro-4-(2-hydroxyethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[3-(4,4-difluoropiperidine-1-carbonyl)-1H-pyrrolo[3,2-c]pyridin-6-yl]-3-(trifluoromethyl)pyrazol-4-yl]-1-methylimidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[1-methyl-5-[1-(2-methylol-1H-pyrrolo[3,2-c]pyridin-6-yl)-3-(trifluoromethyl)pyrazol-4-yl]imidazole-2-carbonyl]amino]benzoyl]piperazine-1-carbonyl]piperidin-1-ium-1-yl]acetic acid;
4-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]butanoic acid;
[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazin-1-yl]-4-oxo-butyl]-bis(3-aminopropyl)-(3-carboxypropyl)ammonium;
[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazin-1-yl]-4-oxo-butyl]-bis(3-aminopropyl)-(carboxymethyl)ammonium;
cis-2-[4-[4-[4-[[5-[1-[2-(aminomethyl)-1H-pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[5-[1-[2-fluoro-4-(2-methoxyethylamino)phenyl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[4-[4-[4-[[5-[1-[4-(3-aminopropylamino)-2-fluoro-phenyl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
trans-2-[4-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl] piperazine-1-carbonyl]-1-[2-(dimethylamino)ethyl]piperidin-1-ium-1-yl]acetic acid;
cis-2-[4-[4-[4-[[5-[1-(4-amino-2-fluoro-phenyl)-3-(trifluoromethyl)pyrazol-4-yl]-1-methyl-imidazole-2-carbonyl]amino]-2-chloro-benzoyl] piperazine-1-carbonyl]-1-[2-(dimethylamino)ethyl]piperidin-1-ium-1-yl]acetic acid;
cis-2-[4-[4-[4-[[5-[1-[2-(aminomethyl)-1H-pyrrolo[3,2-c]pyridin-6-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[4-[4-[4-[[5-[1-[1-(2-aminoethyl)pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[1-methyl-5-[1-(2-methylol-1H-pyrrolo[2,3-c]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
cis-2-[4-[4-[4-[[5-[1-(6-amino-2-pyridyl)-3-(trifluoromethyl)pyrazol-4-yl]-1-methylimidazole-2-carbonyl]amino]-2-chloro-benzoyl]piperazine-1-carbonyl]-1-(azetidin-3-ylmethyl)piperidin-1-ium-1-yl]acetic acid;
2-[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazine-1-carbonyl]-1-methylpiperidin-1-ium-1-yl]acetic acid;
cis-2-[1-(azetidin-3-ylmethyl)-4-[4-[2-chloro-4-[[5-[1-[3-(2-hydroxyethyl)-1H-pyrrolo[3,2-c]pyridin-6-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
2-[[4-[4-[4-[[5-[1-(4-amino-2-fluoro-phenyl)-3-(trifluoromethyl)pyrazol-4-yl]-1-methylimidazole-2-carbonyl]amino]-2-chloro-benzoyl]piperazin-1-yl]-4-oxo-butyl]-(azetidin-3-ylmethyl)-methyl-ammonio]acetate;
cis-2-[1-(azetidin-3-ylmethyl)-4-[4-[4-[[5-[1-[5-[bis(2-aminoethyl)amino]-2-pyridyl]-3-(trifluoromethyl)pyrazol-4-yl]-1-methyl-imidazole-2-carbonyl]amino]-2-chlorobenzoyl]piperazine-1-carbonyl]piperidin-1-ium-1-yl]acetic acid;
cis-2-[1-(azetidin-3-ylmethyl)-4-[4-[2-chloro-4-[[5-[1-[1-(cyanomethyl)pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[1-(azetidin-3-ylmethyl)-4-[4-[2-chloro-4-[[5-[1-[1-(2-hydroxyethyl)pyrrolo[3,2-b]pyridin-5-yl]-3-(trifluoromethyl)pyrazol-4-yl]-1-methyl-imidazole-2-carbonyl]amino]benzoyl]piperazine-1-carbonyl]piperidin-1-ium-1-yl]acetic acid;
cis-2-[4-[4-[4-[[5-[1-[1-(2-amino-2-keto-ethyl)pyrrolo[3,2-b]pyridin-5-yl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid;
cis-2-[1-(azetidin-3-ylmethyl)-4-[4-[2-chloro-4-[[1-methyl-5-[1-(1H-pyrrolo[2,3-c]pyridin-5-yl)-3-(trifluoromethyl)pyrazol-4-yl]imidazole-2-carbonyl]amino]benzoyl]piperazine-1-carbonyl]piperidin-1-ium-1-yl]acetic acid;
2-[4-[4-[4-[[5-[1-[5-(2-aminoethylamino)-2-pyridyl]-3-(trifluoromethyl)pyrazol-4-yl]-1-methyl-imidazole-2-carbonyl]amino]-2-chloro-benzoyl]piperazine-1-carbonyl]-1-methyl-piperidin-1-ium-1-yl]acetate; and
cis-2-[4-[4-[4-[[5-[1-[5-[(3S)-3-aminopyrrolidin-1-yl]-2-pyridyl]-3-(trifluoromethyl)pyrazol-4-yl]-1-methyl-imidazole-2-carbonyl]amino]-2-chlorobenzoyl]piperazine-1-carbonyl]-1-(azetidin-3-ylmethyl)piperidin-1-ium-1-yl]acetic acid.

13. A process of manufacturing a compound of formula (I) according to any one of claims 1 to 12, or a pharmaceutically acceptable salt thereof, wherein said process is as described in scheme 4 disclosed herein.

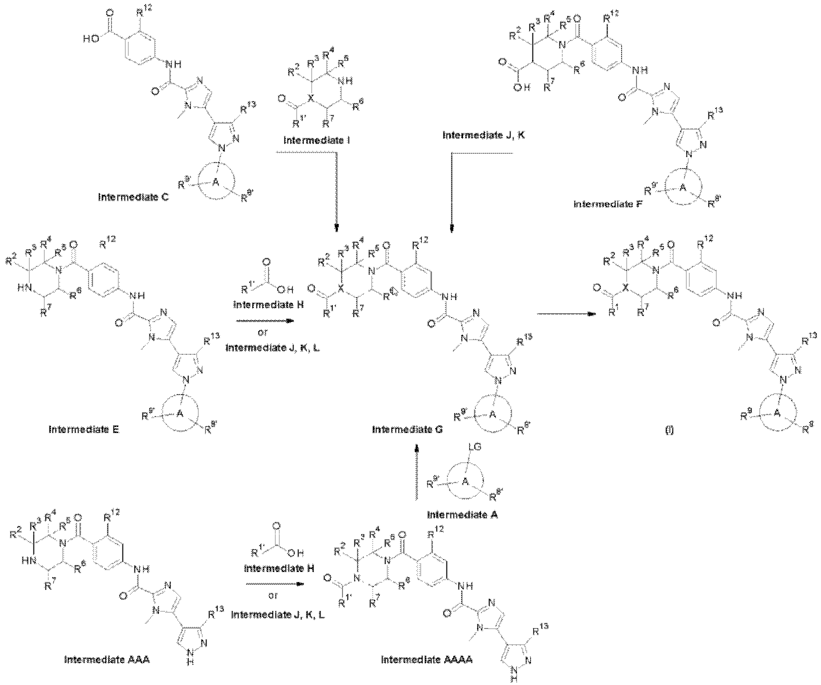

14. A compound of formula (I) according to any of claims 1 to 12, or a pharmaceutically acceptable salt thereof, for use in the treatment or prevention of infections and resulting diseases caused by Gram-negative bacteria.

15. A compound of formula (I) according to any of claims 1 to 12, or a pharmaceutically acceptable salt thereof, for use in the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or *E. coli,* or a combination thereof.

## Patentansprüche

1. Verbindung der Formel (I) 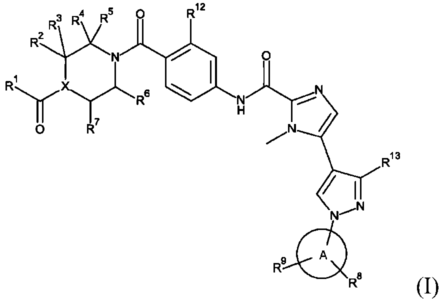 oder ein pharmazeutisch annehmbares Salz davon, wobei:
X und Y jeweils unabhängig voneinander aus N und CH ausgewählt sind;
n ausgewählt ist aus 0 bis 6;
A ausgewählt ist aus 5- bis 14-gliedrigem Heteroaryl und C₆-C₁₄-Aryl;
B ausgewählt ist aus 3- bis 14-gliedrigem Heterocyclyl, 5- bis 14-gliedrigem Heteroaryl und C₃-C₁₀-Cycloalkyl;
L ausgewählt ist aus einer kovalenten Bindung, Carbonyl, NHC(O), C(O)NH, CH₂NH, NHCH₂ und NH;
L¹ -O- oder eine kovalente Bindung ist;
R¹ ausgewählt ist aus
(i) C₁-C₆-Alkyl, substituiert mit einem Amino- und einem Carboxysubstituenten;
(ii) C₁-C₆-Alkyl, substituiert mit einem Amino- und einem Carboxy-C₁-C₆-alkyl-NH-Substituenten;
(iii) C₁-C₆-Alkyl-N(R^{1a}R^{1b});
(iv) -L¹-C₁-C₆-Alkyl-N⁺(R^{1c}R^{1d}R^{1e});
(v) einer Gruppe 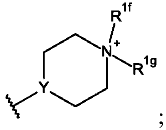 und
(vi) einer Gruppe 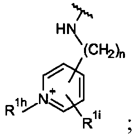
R^{1a} Amino-C₁-C₆-alkyl ist;
R^{1b} Carboxy-C₁-C₆-alkyl ist;
R^{1c} ausgewählt ist aus Amino-C₁-C₆-alkyl und (3- bis 14-gliedrigem Heterocyclyl)-C₁-C₆-alkyl-;
R^{1d} Carboxy-C₁-C₆-alkyl ist;
R^{1e} ausgewählt ist aus C₁-C₆-Alkyl, Amino-C₁-C₆-alkyl und (3- bis 14-gliedrigem Heterocyclyl)-C₁-C₆-alkyl-;
R^{1f} ausgewählt ist aus C₁-C₆-Alkyl, Hydroxy-C₁-C₆-alkyl, Amino-C₁-C₆-alkyl, C₁-C₆-Alkyl-NH-C₁-C₆-alkyl, (C₁-C₆-Alkyl)zN-C₁-C₆-alkyl und (3- bis 14-gliedrigem Heterocyclyl)-C₁-C₆-alkyl-;
R^{1g} Carboxy-C₁-C₆-alkyl ist;
R^{1h} Carboxy-C₁-C₆-alkyl ist;
R¹ⁱ Amino-C₁-C₆-alkyl ist;
R² ausgewählt ist aus Wasserstoff, C₁-C₆-Alkyl und C₁-C₆-Alkoxy; und R³ und R⁷ beide Wasserstoff sind; oder
R² und R⁷ zusammen mit den Atomen, an die sie gebunden sind, einen 4- bis 14-gliedrigen Heterocyclus oder ein C₄-C₁₀-Cycloalkyl bilden; und R³ Wasserstoff ist; oder
R² und R³ zusammen mit dem Kohlenstoffatom, an das sie gebunden sind, ein C₃₋C₁₀-Cycloalkyl bilden; und R⁷ Wasserstoff ist;
R⁴ ausgewählt ist aus Wasserstoff und C₁-C₆-Alkyl; und R⁵ und R⁶ beide Wasserstoff sind; oder
R⁴ und R⁶ zusammen mit den Atomen, an die sie gebunden sind, einen 4- bis 14-gliedrigen Heterocyclus bilden; und R⁵ Wasserstoff ist; oder
R⁴ und R⁵ zusammen mit dem Kohlenstoffatom, an das sie gebunden sind, ein C₃₋C₁₀-Cycloalkyl bilden; und R⁶ Wasserstoff ist;
R⁸ ausgewählt ist aus Wasserstoff, Cyano, Hydroxy, Amino, C₁-C₆-Alkyl-NH-, (C₁-C₆-Alkyl)₂N-, (C₁-C₆-Alkyl)₂N-SO₂-, (C₁-C₆-Alkyl)₂N-C₁-C₆-alkyl-, Amino-C₁-C₆-alkyl, Amino-C₁-C₆-alkoxy, Amino-C₁-C₆-alkyl-NH-, (Amino-C₁-C₆-alkyl)₂N-, (Amino-C₁-C₆-alkyl)₂N-C₁-C₆-alkyl-NH-, C₁-C₆-Alkyl-NH-C₁-C₆-alkyl-NH-, C₁-C₆-Alkoxy-C₁-C₆-alkyl-NH-, Halogen-C₁-C₆-alkoxy-C₁-C₆-alkyl-NH-, C₃₋C₁₀-Cycloalkyl-NH-, C₁-C₆-Alkoxy, C₁-C₆-Alkyl, Cyano-C₁-C₆-alkyl-, Hydroxy-C₁-C₆-alkyl-, Hydroxy-C₁-C₆-alkyl-NH-, Hydroxy-C₁-C₆-alkyl-NHC(O)-, Halogen-hydroxy-C₁-C₆-alkyl-NHC(O)-, Carbamoyl, Hydroxycarbamoyl, C₁-C₆-Alkyl-NHC(O)-, (C₁-C₆-Alkyl)₂NC(O)-, Carbamoyl-C₁-C₆-alkyl- und einer Gruppe 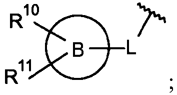
R⁹ ausgewählt ist aus Wasserstoff, Halogen, Amino, C₁-C₆-Alkyl und Hydroxy-C₁-C₆-alkyl-;
R¹⁰ ausgewählt ist aus Wasserstoff, C₁-C₆-Alkyl, Halogen, Hydroxy, Oxo, Imino und Amino;
R¹¹ ausgewählt ist aus Wasserstoff und Halogen;
R¹² ausgewählt ist aus Halogen und C₁-C₆-Alkyl;
R¹³ Halogen-C₁-C₆-alkyl ist.

2. Verbindung der Formel (I) nach Anspruch 1 oder ein pharmazeutisch annehmbares Salz davon, wobei L ausgewählt ist aus einer kovalenten Bindung, Carbonyl, NHC(O), CH₂NH und NH.

3. Verbindung der Formel (I) nach einem der Ansprüche 1 oder 2 oder ein pharmazeutisch annehmbares Salz davon, wobei:
R^{1f} ausgewählt ist aus C₁-C₆-Alkyl, Hydroxy-C₁-C₆-alkyl, Amino-C₁-C₆-alkyl, (C₁-C₆-Alkyl)₂N-C₁-C₆-alkyl und (3- bis 14-gliedrigem Heterocyclyl)-C₁-C₆-alkyl-.

4. Verbindung der Formel (I) nach einem der Ansprüche 1 oder 2 oder ein pharmazeutisch annehmbares Salz davon, wobei:
R¹ ausgewählt ist aus
(i) C₁-C₆-Alkyl-N⁺(R^{1c}R^{1d}R^{1c}); und
(ii) einer Gruppe 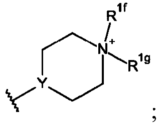 wobei
Y CH ist;
R^{1c} und R^{1c} beide Amino-C₁-C₆-alkyl sind;
R^{1d} und R^{1g} beide Carboxy-C₁-C₆-alkyl sind; und
R^{1f} ausgewählt ist aus Amino-C₁-C₆-alkyl und (3- bis 14-gliedrigem Heterocyclyl)-C₁-C₆-alkyl-.

5. Verbindung der Formel (I) nach Anspruch 3 oder ein pharmazeutisch annehmbares Salz davon, wobei:
R¹ ausgewählt ist aus
(i) -(CH₂)₃-N⁺(R^{1c}R^{1d}R^{1e}); und
(ii) einer Gruppe 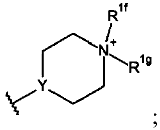 wobei
Y CH ist;
R^{1c} und R^{1c} beide 4-Aminobutyl sind;
R^{1d} und R^{1g} beide Carboxymethyl sind; und
R^{1f} ausgewählt ist aus 3-Aminopropyl und Azetidin-3-ylmethyl.

6. Verbindung der Formel (I) nach einem der Ansprüche 1 bis 5 oder ein pharmazeutisch annehmbares Salz davon, wobei R², R³, R⁴, R⁵, R⁶ und R⁷ alle Wasserstoff sind.

7. Verbindung der Formel (I) nach einem der Ansprüche 1 bis 6 oder ein pharmazeutisch annehmbares Salz davon, wobei:
A ausgewählt ist aus Thiazolyl; Isothiazolyl; Pyridyl; 1H-Pyrrolo[3,2-b]pyridin; 1H-Imidazo[4,5-b]pyridin; 1,3-Benzothiazol; Benzothiophen; 1H-Pyrazolo[4,3-b]pyridin; 1H-Pyrrolo[3,2-c]pyridin; 1H-Pyrrolo[2,3-b]pyridin, 1H-Pyrrolo[2,3-c]pyridin, 2H-Pyrazolo[4,3-c]pyridin, 1H-Pyrrolo[3,2-d]pyrimidin, 1H-Indazolyl; Furo[3,2-b]pyridin; 2,3-Dihydro-1H-pyrrolo[3,2-b]pyridin; und Phenyl; und
B ausgewählt ist aus Morpholin, Thiomorpholin, Piperazin, Pyrrolidinyl, Piperidyl, Pyridyl, Cyclopropyl, Cyclopentyl, 1,2,3,3a,4,5,6,6a-Octahydropyrrolo-[3,4-c]pyrrol, 9-Oxa-3,7-diazabicyclo[3.3.1]nonan, 7-Oxa-2-azaspiro[3.5]nonan und 2,6-Dioxa-9-azaspiro[4.5]decan.

8. Verbindung der Formel (I) nach einem der Ansprüche 1 bis 7 oder ein pharmazeutisch annehmbares Salz davon, wobei X N ist.

9. Verbindung der Formel (I) nach einem der Ansprüche 1 bis 8 oder ein pharmazeutisch annehmbares Salz davon, wobei:
R¹² ausgewählt ist aus Chlor und Methyl; und
R¹³ ausgewählt ist aus CHF₂ und CF₃.

10. Verbindung der Formel (I) nach Anspruch 1 oder ein pharmazeutisch annehmbares Salz davon, wobei:
X und Y jeweils unabhängig voneinander aus N und CH ausgewählt sind;
n ausgewählt ist aus 0 bis 6;
A ausgewählt ist aus 5- bis 14-gliedrigem Heteroaryl und C₆-C₁₄-Aryl;
B ausgewählt ist aus 3- bis 14-gliedrigem Heterocyclyl, 5- bis 14-gliedrigem Heteroaryl und C₃₋C₁₀-Cycloalkyl;
L ausgewählt ist aus einer kovalenten Bindung, Carbonyl, NHC(O), CH₂NH und NH;
L¹ -O- oder eine kovalente Bindung ist;
R¹ ausgewählt ist aus
(i) C₁-C₆-Alkyl, substituiert mit einem Amino- und einem Carboxysubstituenten;
(ii) C₁-C₆-Alkyl, substituiert mit einem Amino- und einem Carboxy-C₁-C₆-alkyl-NH-Substituenten;
(iii) C₁-C₆-Alkyl-N(R^{1a}R^{1b});
(iv) -L¹⁻C₁-C₆-Alkyl-N⁺(R^{1c}R^{1d}R^{1e});
(v) einer Gruppe 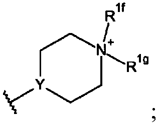 und
(vi) einer Gruppe 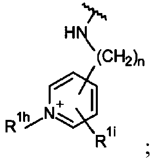
R^{1a} Amino-C₁-C₆-alkyl ist;
R^{1b} Carboxy-C₁-C₆-alkyl ist;
R^{1c} ausgewählt ist aus Amino-C₁-C₆-alkyl und (3- bis 14-gliedrigem Heterocyclyl)-C₁-C₆-alkyl-;
R^{1d} Carboxy-C₁-C₆-alkyl ist;
R^{1e} ausgewählt ist aus C₁-C₆-Alkyl, Amino-C₁-C₆-alkyl und (3- bis 14-gliedrigem Heterocyclyl)-C₁-C₆-alkyl-;
R^{1f} ausgewählt ist aus C₁-C₆-Alkyl, Hydroxy-C₁-C₆-alkyl, Amino-C₁-C₆-alkyl, (C₁-C₆-Alkyl)₂N-C₁-C₆-alkyl und (3- bis 14-gliedrigem Heterocyclyl)-C₁-C₆-alkyl-;
R^{1g} Carboxy-C₁-C₆-alkyl ist;
R^{1h} Carboxy-C₁-C₆-alkyl ist;
R¹ⁱ Amino-C₁-C₆-alkyl ist;
R² ausgewählt ist aus Wasserstoff, C₁-C₆-Alkyl und C₁-C₆-Alkoxy; und R³ und R⁷ beide Wasserstoff sind; oder
R² **und** R⁷ zusammen mit den Atomen, an die sie gebunden sind, einen 4- bis 14-gliedrigen Heterocyclus oder ein C₄₋C₁₀-Cycloalkyl bilden; und R³ Wasserstoff ist; oder
R² und R³ zusammen mit dem Kohlenstoffatom, an das sie gebunden sind, ein C₃₋C₁₀-Cycloalkyl bilden; und R⁷ Wasserstoff ist;
R⁴ ausgewählt ist aus Wasserstoff und C₁-C₆-Alkyl; und R⁵ und R⁶ beide Wasserstoff sind; oder
R⁴ und R⁶ zusammen mit den Atomen, an die sie gebunden sind, einen 4- bis 14-gliedrigen Heterocyclus bilden; und R⁵ Wasserstoff ist; oder
R⁴ und R⁵ zusammen mit dem Kohlenstoffatom, an das sie gebunden sind, ein C₃₋C₁₀-Cycloalkyl bilden; und R⁶ Wasserstoff ist;
R⁸ ausgewählt ist aus Wasserstoff, Cyano, Amino, Hydroxy, C₁-C₆-Alkyl-NH-, (C₁-C₆-Alkyl)₂N-, (C₁-C₆-Alkyl)₂N-SO₂-, (C₁-C₆-Alkyl)₂N-C₁-C₆-alkyl-, Amino-C₁-C₆-alkyl, Amino-C₁-C₆-alkoxy, Amino-C₁-C₆-alkyl-NH-, (Amino-C₁-C₆-alkyl)₂N-, (Amino-C₁-C₆-alkyl)₂N-C₁-C₆-alkyl-NH-, C₁-C₆-Alkyl-NH-C₁-C₆-alkyl-NH-, C₁-C₆-Alkoxy-C₁-C₆-alkyl-NH-, Halogen-C₁-C₆-alkoxy-C₁-C₆-alkyl-NH-, C₃-C₁₀-Cycloalkyl-NH-, C₁-C₆-Alkoxy, C₁-C₆-Alkyl, Cyano-C₁-C₆-alkyl-, Hydroxy-C₁-C₆-alkyl-, Hydroxy-C₁-C₆-alkyl-NH-, Hydroxy-C₁-C₆-alkyl-NHC(O)-, Halogenhydroxy-C₁-C₆-alkyl-NHC(O)-, Carbamoyl, Hydroxycarbamoyl, C₁-C₆-Alkyl-NHC(O)-, (C₁-C₆-Alkyl)₂NC(O)-, Carbamoyl-C₁-C₆-alkyl- und einer Gruppe 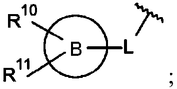
R⁹ ausgewählt ist aus Wasserstoff, Halogen, Amino, C₁-C₆-Alkyl und Hydroxy-C₁-C₆-alkyl-;
R¹⁰ ausgewählt ist aus Wasserstoff, C₁-C₆-Alkyl, Halogen, Hydroxy, Oxo, Imino und Amino;
R¹¹ ausgewählt ist aus Wasserstoff und Halogen;
R¹² ausgewählt ist aus Halogen und C₁-C₆-Alkyl;
R¹³ Halogen-C₁-C₆-alkyl ist.

11. Verbindung der Formel (I) nach Anspruch 10 oder ein pharmazeutisch annehmbares Salz davon, wobei:
A ausgewählt ist aus 5- bis 14-gliedrigem Heteroaryl und C₆-C₁₄-Aryl;
B 3- bis 14-gliedriges Heterocyclyl ist;
L eine kovalente Bindung ist;
X N ist;
Y CH ist;
R¹ ausgewählt ist aus
(i) C₁-C₆-Alkyl-N⁺(R^{1c}R^{1d}R^{1e}); und
(ii) einer Gruppe 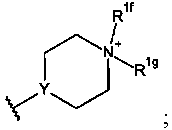
R^{1c} und R^{1c} beide Amino-C₁-C₆-alkyl sind;
R^{1d} und R^{1g} beide Carboxy-C₁-C₆-alkyl sind;
R^{1f} ausgewählt ist aus Amino-C₁-C₆-alkyl und (3- bis 14-gliedrigem Heterocyclyl)-C₁-C₆-alkyl-;
R², R³, R⁴, R⁵, R⁶ und R⁷ alle Wasserstoff sind;
R⁸ ausgewählt ist aus Wasserstoff, Amino, Hydroxy, C₁-C₆-Alkyl-NH-, Amino-C₁-C₆-alkyl-NH-, Hydroxy-C₁-C₆-alkyl-, Hydroxy-C₁-C₆-alkyl-NH-, C₁-C₆-Alkoxy-C₁-C₆-alkyl-NH-, Carbamoyl-C₁-C₆-alkyl-, (C₁-C₆-Alkyl)₂NC(O)- und einer Gruppe 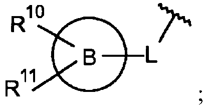
R⁹ ausgewählt ist aus Wasserstoff und Halogen;
R¹⁰ Amino ist;
R¹¹ Wasserstoff ist;
R¹² ausgewählt ist aus Halogen und C₁-C₆-Alkyl; und
R¹³ Halogen-C₁-C₆-alkyl ist.

12. Verbindung der Formel (I) nach Anspruch 1 oder ein pharmazeutisch annehmbares Salz davon, wobei die Verbindung der Formel (I) ausgewählt ist aus:
Bis(4-aminobutyl)-(carboxymethyl)-[4-[4-[2-chlor-4-[[1-methyl-5-[1-(1H-pyrrolo-[3,2-b]pyridin-5-yl)-3-(trifluormethyl)pyrazol-4-yl]imidazol-2-carbonyl]amino]benzoyl]piperazino]-4-ketobutyl]ammonium;
cis-2-[4-[4-[4-[[5-[1-[5-(2-Aminoethylamino)-2-pyridyl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
Bis(4-aminobutyl)-[4-[4-[4-[[5-[1-(4-amino-2-fluorphenyl)-3-(trifluormethyl)pyrazol-4-yl]-1-methylimidazol-2-carbonyl]amino]-2-chlorbenzoyl]piperazino]-4-ketobutyl]-(carboxymethyl)ammonium;
2-[4-[4-[4-[[5-[1-[5-(2-Aminoethylamino)-2-pyridyl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
Bis(3-aminopropyl)-(carboxymethyl)-[4-[4-[2-chlor-4-[[1-methyl-5-[1-(1H-pyrrolo-[3,2-b]pyridin-5-yl)-3-(trifluormethyl)pyrazol-4-yl]imidazol-2-carbonyl]amino]benzoyl]piperazino]-4-ketobutyl]ammonium;
cis-2-[1-(Azetidin-3-ylmethyl)-4-[4-[2-chlor-4-[[1-methyl-5-[1-(1H-pyrrolo-[3,2-c]pyridin-6-yl)-3-(trifluormethyl)pyrazol-4-yl]imidazol-2-carbonyl]amino]benzoyl]piperazin-1-carbonyl]piperidin-1-ium-1-yl]essigsäure;
cis-2-[4-[4-[4-[[5-[1-[5-(3-Aminopropylamino)-2-pyridyl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
cis-2-[4-[4-[4-[[5-[1-(4-Amino-2-fluorphenyl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[4-[4-[4-[[5-[1-(4-Amino-2-fluorphenyl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[1-(Azetidin-3-ylmethyl)-4-[4-[2-chlor-4-[[1-methyl-5-[1-(1H-pyrrolo[3,2-b]pyridin-5-yl)-3-(trifluormethyl)pyrazol-4-yl]imidazol-2-carbonyl]amino]benzoyl]piperazin-1-carbonyl]piperidin-1-ium-1-yl]essigsäure;
2-[4-[4-[4-[[5-[1-[5-[[(2S)-2-Aminopropyl]amino]-2-pyridyl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
Bis(4-aminobutyl)-[4-[4-[4-[[5-[1-(5-amino-2-pyridyl)-3-(trifluormethyl)pyrazol-4-yl]-1 -methylimidazol-2-carbonyl]amino]-2-chlorbenzoyl]piperazino]-4-ketobutyl]-(carboxymethyl)ammonium;
2-[4-[4-[4-[[5-[1-[5-(Aminomethyl)-2-pyridyl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[1-(Azetidin-3-ylmethyl)-4-[4-[2-chlor-4-[[5-[1-(5-methoxy-2-pyridyl)-3-(trifluormethyl)pyrazol-4-yl]-1-methylimidazol-2-carbonyl]amino]benzoyl]piperazin-1-carbonyl]piperidin-1-ium-1-yl]essigsäure;
2-[4-[4-[4-[[5-[1-[5-(2-Aminoethyl)-2-pyridyl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[4-[4-[4-[[5-[1-[5-(2-Aminoethoxy)-2-pyridyl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[1-(Azetidin-3-ylmethyl)-4-[4-[2-chlor-4-[[1-methyl-5-[1-[5-(2-pyridylmethylamino)-2-pyridyl]-3-(trifluormethyl)pyrazol-4-yl]imidazol-2-carbonyl]amino]benzoyl]piperazin-1-carbonyl]piperidin-1-ium-1-yl]essigsäure;
2-[4-[4-[4-[[5-[1-[5-[(1-Aminocyclopentyl)methylamino]-2-pyridyl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[1-(Azetidin-3-ylmethyl)-4-[4-[2-chlor-4-[[1-methyl-5-[1-[5-(methylamino)-2-pyridyl]-3-(trifluormethyl)pyrazol-4-yl]imidazol-2-carbonyl]amino]benzoyl]-piperazin-1-carbonyl]piperidin-1-ium-1-yl]essigsäure;
2-[1-(Azetidin-3-ylmethyl)-4-[4-[2-chlor-4-[[1-methyl-5-[1-(5-morpholino-2-pyridyl)-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-(1H-pyrrolo-[3,2-b]pyridin-5-yl)-3-(trifluormethyl)pyrazol-4-yl]imidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[1-methyl-5-[1-[5-[[(2S)-pyrrolidin-2-yl]methylamino]-2-pyridyl]-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-[5-(methylamino)-2-pyridyl]-3-(trifluormethyl)pyrazol-4-yl]imidazol-2-carbonyl]amino]benzoyl]piperazin-1-carbonyl]piperidin-1-ium-1-yl]essigsäure;
2-[1-(3-Aminopropyl)-4-[4-[4-[[5-[1-(5-amino-2-pyridyl)-3-(trifluormethyl)pyrazol-4-yl]-1-methylimidazol-2-carbonyl]amino]-2-chlorbenzoyl]piperazin-1-carbonyl]piperidin-1-ium-1-yl]essigsäure;
cis-2-[1-(Azetidin-3-ylmethyl)-4-[4-[2-chlor-4-[[5-[1-[5-(2-methoxyethylamino)-2-pyridyl]-3-(trifluormethyl)pyrazol-4-yl]-1-methylimidazol-2-carbonyl]amino]benzoyl]piperazin-1-carbonyl]piperidin-1-ium-1-yl]essigsäure;
trans-2-[1-(3-Aminopropyl)-4-[4-[4-[[5-[1-(5-amino-2-pyridyl)-3-(trifluormethyl)pyrazol-4-yl]-1-methylimidazol-2-carbonyl]amino]-2-chlorbenzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-(2-methylol-1H-pyrrolo[3,2-b]pyridin-5-yl)-3-(trifluormethyl)pyrazol-4-yl]imidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[1-methyl-5-[1-(5-piperazino-2-pyridyl)-3-(trifluormethyl)pyrazol-4-yl]imidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[1-methyl-5-[1-[5-[2-(methylamino)ethylamino]-2-pyridyl]-3-(trifluormethyl)pyrazol-4-yl]imidazol-2-carbonyl]amino]benzoyl]piperazin-1-carbonyl]piperidin-1-ium-1-yl]essigsäure;
cis-2-[4-[4-[4-[[5-[1-(5-Amino-2-pyridyl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
Bis(3-aminopropyl)-[4-[4-[4-[[5-[1-(5-amino-2-pyridyl)-3-(trifluormethyl)pyrazol-4-yl]-1-methylimidazol-2-carbonyl]amino]-2-chlorbenzoyl]piperazino]-4-ketobutyl]-(carboxymethyl)ammonium;
Bis(4-aminobutyl)-[4-[4-[4-[[5-[1-[5-(2-aminoethylamino)-2-pyridyl]-3-(trifluormethyl)pyrazol-4-yl]-1-methylimidazol-2-carbonyl]amino]-2-chlorbenzoyl]piperazino]-4-ketobutyl]-(carboxymethyl)ammonium;
2-[1-(Azetidin-3-ylmethyl)-4-[4-[2-chlor-4-[[5-[1-(4-methoxyphenyl)-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[5-(2-hydroxyethylamino)-2-pyridyl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-(1-methylpyrrolo-[3,2-b]pyridin-5-yl)-3-(trifluormethyl)pyrazol-4-yl]imidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[5-[1-[5-(isopropylamino)-2-pyridyl]-3-(trifluormethyl)pyrazol-4-yl]-1-methylimidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[1-methyl-5-[1-[5-(2-pyridylamino)-2-pyridyl]-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-(1H-pyrazolo[4,3-b]-pyridin-5-yl)-3-(trifluormethyl)pyrazol-4-yl]imidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[1-methyl-5-[1-thiazol-2-yl-3-(trifluormethyl)pyrazol-4-yl]imidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[5-[1-[5-(cyclopropylamino)-2-pyridyl]-3-(trifluormethyl)pyrazol-4-yl]-1-methylimidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[5-[1-[5-(dimethylamino)-2-pyridyl]-3-(trifluormethyl)pyrazol-4-yl]-1-methylimidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[5-[1-[4-(dimethylamino)phenyl]-3-(trifluormethyl)pyrazol-4-yl]-1-methylimidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[1-methyl-5-[1-[5-[[(3S)-morpholin-3-yl]methylamino]-2-pyridyl]-3-(trifluormethyl)pyrazol-4-yl]imidazol-2-carbonyl]amino]benzoyl]piperazin-1-carbonyl]piperidin-1-ium-1-yl]essigsäure;
cis-2-[1-(3-Aminopropyl)-4-[4-[4-[[5-[1-(5-amino-2-pyridyl)-3-(trifluormethyl)pyrazol-4-yl]-1-methylimidazol-2-carbonyl]amino]-2-chlorbenzoyl]piperazin-1-carbonyl]piperidin-1-ium-1-yl]essigsäure;
2-[1-(Azetidin-3-ylmethyl)-4-[4-[2-chlor-4-[[5-[1-[6-(dimethylamino)-3-pyridyl]-3-(trifluormethyl)pyrazol-4-yl]-1-methylimidazol-2-carbonyl]amino]benzoyl]piperazin-1-carbonyl]piperidin-1-ium-1-yl]essigsäure;
[4-[4-[4-[[5-[1-(5-Aminopyridin-2-yl)-3-(trifluormethyl)pyrazol-4-yl]-1-methylimidazol-2-carbonyl]amino]-2-chlorbenzoyl]piperazin-1-yl]-4-oxobutyl]-bis(azetidin-3-ylmethyl)-(carboxymethyl)azanium;
2-[4-[4-[4-[[5-[1-[5-(6-Aminohexylamino)-2-pyridyl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[1-(Azetidin-3-ylmethyl)-4-[4-[2-chlor-4-[[1-methyl-5-[1-[4-(methylamino)-2-pyridyl]-3-(trifluormethyl)pyrazol-4-yl]imidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[1-methyl-5-[1-[5-(4-methylpiperazino)-2-pyridyl]-3-(trifluormethyl)pyrazol-4-yl]imidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[5-[1-(5-hydroxy-2-pyridyl)-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-(1H-imidazo[4,5-b]pyridin-5-yl)-3-(trifluormethyl)pyrazol-4-yl]-1-methylimidazol-2-carbonyl]amino]benzoyl]piperazin-1-carbonyl]piperidin-1-ium-1-yl]essigsäure;
2-[4-[4-[4-[[5-[1-(6-Amino-2-pyridyl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[1-(3-Aminopropyl)-4-[4-[4-[[5-[1-(5-amino-2-pyridyl)-3-(trifluormethyl)pyrazol-4-yl]-1-methylimidazol-2-carbonyl]amino]-2-chlorbenzoyl]piperazin-1-carbonyl]piperidin-1-ium-1-yl]essigsäure;
2-[1-(Azetidin-3-ylmethyl)-4-[4-[2-chlor-4-[[1-methyl-5-[1-[5-(4-piperidylmethylamino)-2-pyridyl]-3-(trifluormethyl)pyrazol-4-yl]imidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[5-[1-[5-[[(2S,4S)-4-fluorpyrrolidin-2-yl]methylamino]-2-pyridyl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[4-[4-[4-[[5-[1-(5-Amino-3-fluor-2-pyridyl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[4-[4-[4-[[5-[1-(5-Amino-2-pyridyl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
4-[4-[4-[4-[[5-[1-(5-Amino-2-pyridyl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]buttersäure;
2-[1-(Azetidin-3-ylmethyl)-4-[4-[2-chlor-4-[[5-[1-(2,3-dihydro-1H-pyrrolo[3,2-b]-pyridin-5-yl)-3-(trifluormethyl)pyrazol-4-yl]-1-methylimidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[5-[1-(1H-indazol-3-yl)-3-(trifluormethyl)pyrazol-4-yl]-1-methylimidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[5-[1-furo[3,2-b]pyridin-5-yl-3-(trifluormethyl)pyrazol-4-yl]-1-methylimidazol-2-carbonyl]amino]benzoyl]piperazin-1-carbonyl]piperidin-1-ium-1-yl]essigsäure;
trans-2-[4-[4-[4-[[5-[1-(5-Amino-2-pyridyl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[1-(Azetidin-3-ylmethyl)-4-[4-[2-chlor-4-[[5-[1-[5-[(2,2-difluorcyclopropyl)methylamino]-2-pyridyl]-3-(trifluormethyl)pyrazol-4-yl]-1-methylimidazol-2-carbonyl]amino]benzoyl]piperazin-1-carbonyl]piperidin-1-ium-1-yl]essigsäure;
3-Aminopropyl-[4-[4-[4-[[5-[1-(5-amino-2-pyridyl)-3-(trifluormethyl)pyrazol-4-yl]-1-methylimidazol-2-carbonyl]amino]-2-chlorbenzoyl]piperazino]-4-ketobutyl]-(carboxymethyl)methylammonium;
Bis(3-aminopropyl)-[4-[4-[4-[[5-[1-(5-amino-2-pyridyl)-3-(trifluormethyl)pyrazol-4-yl]-1-methylimidazol-2-carbonyl]amino]-2-chlorbenzoyl]piperazino]-4-ketobutyl]-(carboxymethyl)ammonium;
2-[1-(Azetidin-3-ylmethyl)-4-[4-[2-chlor-4-[[5-[1-(5,6-diamino-2-pyridyl)-3-(trifluormethyl)pyrazol-4-yl]-1-methylimidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[1-methyl-5-[1-[6-(methylamino)-3-pyridyl]-3-(trifluormethyl)pyrazol-4-yl]imidazol-2-carbonyl]amino]benzoyl]piperazin-1-carbonyl]piperidin-1-ium-1-yl]essigsäure;
2-[1-(Azetidin-3-ylmethyl)-4-[4-[2-chlor-4-[[5-[1-[5-(cyclobutylamino)-2-pyridyl]-3-(trifluormethyl)pyrazol-4-yl]-1-methylimidazol-2-carbonyl]amino]benzoyl]piperazin-1-carbonyl]piperidin-1-ium-1-yl]essigsäure;
2-[2-(3-Aminopropyl)-4-[3-[[4-[4-[[5-[1-(5-amino-2-pyridyl)-3-(trifluormethyl)pyrazol-4-yl]-1-methylimidazol-2-carbonyl]amino]-2-chlorbenzoyl]piperazin-1-carbonyl]amino]propyl]pyridin-1-ium-1-yl]essigsäure;
2-[4-[4-[4-[[5-[1-(4-Amino-2-pyridyl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[3-Aminopropyl-[4-[4-[4-[[5-[1-(5-amino-2-pyridyl)-3-(trifluormethyl)pyrazol-4-yl]-1-methylimidazol-2-carbonyl]amino]-2-chlorbenzoyl]piperazino]-4-ketobutyl]amino]essigsäure;
cis-2-[4-[4-[4-[[5-[1-[5-(2-Aminoethylamino)-2-pyridyl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[4-[4-[4-[[5-[1-(2-Amino-4-pyridyl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[1-(3-Aminopropyl)-4-[1-[4-[[5-[1-(5-amino-2-pyridyl)-3-(trifluormethyl)pyrazol-4-yl]-1-methylimidazol-2-carbonyl]amino]-2-chlorbenzoyl]piperidin-4-carbonyl]piperazin-1-ium-1-yl]essigsäure;
2-[1-(Azetidin-3-ylmethyl)-4-[4-[2-chlor-4-[[5-[1-(5-cyano-2-pyridyl)-3-(trifluormethyl)pyrazol-4-yl]-1-methylimidazol-2-carbonyl]amino]benzoyl]piperazin-1-carbonyl]piperidin-1-ium-1-yl]essigsäure;
2-[4-[(2S)-4-[4-[[5-[1-(5-Amino-2-pyridyl)-3-(trifluormethyl)pyrazol-4-yl]-1-methylimidazol-2-carbonyl]amino]-2-chlorbenzoyl]-2-methylpiperazin-1-carbonyl]-1-(azetidin-3-ylmethyl)piperidin-1-ium-1-yl]essigsäure;
2-[4-[(3R)-4-[4-[[5-[1-(5-Amino-2-pyridyl)-3-(trifluormethyl)pyrazol-4-yl]-1-methylimidazol-2-carbonyl]amino]-2-chlorbenzoyl]-3-ethylpiperazin-1-carbonyl]-1-(azetidin-3-ylmethyl)piperidin-1-ium-1-yl]essigsäure;
2-[4-[(3S)-4-[4-[[5-[1-(5-Amino-2-pyridyl)-3-(trifluormethyl)pyrazol-4-yl]-1-methylimidazol-2-carbonyl]amino]-2-chlorbenzoyl]-3-methylpiperazin-1-carbonyl]-1-(azetidin-3-ylmethyl)piperidin-1-ium-1-yl]essigsäure;
2-[1-(Azetidin-3-ylmethyl)-4-[4-[4-[[5-[1-(1,3-benzothiazol-2-yl)-3-(trifluormethyl)pyrazol-4-yl]-1-methylimidazol-2-carbonyl]amino]-2-chlorbenzoyl]piperazin-1-carbonyl]piperidin-1-ium-1-yl]essigsäure;
2-[[(5S)-5-Amino-6-[4-[4-[[5-[1-(5-amino-2-pyridyl)-3-(trifluormethyl)pyrazol-4-yl]-1-methylimidazol-2-carbonyl]amino]-2-chlorbenzoyl]piperazino]-6-ketohexyl]amino]essigsäure;
2-[1-(Azetidin-3-ylmethyl)-4-[4-[2-chlor-4-[[5-[1-(6-methoxy-3-pyridyl)-3-(trifluormethyl)pyrazol-4-yl]-1-methylimidazol-2-carbonyl]amino]benzoyl]piperazin-1-carbonyl]piperidin-1-ium-1-yl]essigsäure;
(2S)-2-Amino-4-[4-[4-[[5-[1-(5-amino-2-pyridyl)-3-(trifluormethyl)pyrazol-4-yl]-1-methylimidazol-2-carbonyl]amino]-2-chlorbenzoyl]piperazino]-4-ketobuttersäure;
2-[1-(Azetidin-3-ylmethyl)-4-[4-[2-chlor-4-[[5-[1-[5-[(dimethylamino)methyl]-2-pyridyl]-3-(trifluormethyl)pyrazol-4-yl]-1-methylimidazol-2-carbonyl]amino]benzoyl]piperazin-1-carbonyl]piperidin-1-ium-1-yl]essigsäure;
(3R)-3-Amino-4-[4-[4-[[5-[1-(5-amino-2-pyridyl)-3-(trifluormethyl)pyrazol-4-yl]-1-methylimidazol-2-carbonyl]amino]-2-chlorbenzoyl]piperazino]-4-ketobuttersäure;
2-[4-[7-[4-[[5-[1-(5-Amino-2-pyridyl)-3-(trifluormethyl)pyrazol-4-yl]-1-methylimidazol-2-carbonyl]amino]-2-chlorbenzoyl]-4,7-diazaspiro[2.5]octan-4-carbonyl]-1-(azetidin-3-ylmethyl)piperidin-1-ium-1-yl]essigsäure;
2-[4-[1-[4-[[5-[1-(5-Amino-2-pyridyl)-3-(trifluormethyl)pyrazol-4-yl]-1-methylimidazol-2-carbonyl]amino]-2-chlorbenzoyl]piperidin-4-carbonyl]-1-(azetidin-3-ylmethyl)piperazin-1-ium-1-yl]essigsäure;
2-[1-(Azetidin-3-ylmethyl)-4-[4-[2-chlor-4-[[1-methyl-5-[1-[2-(methylamino)-4-pyridyl]-3-(trifluormethyl)pyrazol-4-yl]imidazol-2-carbonyl]amino]benzoyl]piperazin-1-carbonyl]piperidin-1-ium-1-yl]essigsäure;
2-[4-[4-[4-[[5-[1-(6-Amino-3-pyridyl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[4-[8-[4-[[5-[1-(5-Amino-2-pyridyl)-3-(trifluormethyl)pyrazol-4-yl]-1-methylimidazol-2-carbonyl]amino]-2-chlorbenzoyl]-3,8-diazabicyclo[3.2.1]octan-3-carbonyl]-1-(azetidin-3-ylmethyl)piperidin-1-ium-1-yl]essigsäure;
(2S)-2-Amino-5-[4-[4-[[5-[1-(5-amino-2-pyridyl)-3-(trifluormethyl)pyrazol-4-yl]-1-methylimidazol-2-carbonyl]amino]-2-chlorbenzoyl]piperazino]-5-ketovaleriansäure;
(3S)-3-Amino-4-[4-[4-[[5-[1-(5-amino-2-pyridyl)-3-(trifluormethyl)pyrazol-4-yl]-1-methylimidazol-2-carbonyl]amino]-2-chlorbenzoyl]piperazino]-4-ketobuttersäure;
2-[4-[4-[4-[[5-[1-[6-(2-Aminoethylamino)-3-pyridyl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[4-[3-[4-[[5-[1-(5-Amino-2-pyridyl)-3-(trifluormethyl)pyrazol-4-yl]-1-methylimidazol-2-carbonyl]amino]-2-chlorbenzoyl]-3,9-diazabicyclo[3.3.1]nonan-9-carbonyl]-1-(azetidin-3-ylmethyl)piperidin-1-ium-1-yl]essigsäure;
2-[4-[4-[4-[[5-[1-(5-Amino-2-pyridyl)-3-(trifluormethyl)pyrazol-4-yl]-1-methylimidazol-2-carbonyl]amino]-2-chlorbenzoyl]-4,7-diazaspiro[2.5]octan-7-carbonyl]-1-(azetidin-3-ylmethyl)piperidin-1-ium-1-yl]essigsäure;
2-[4-[4-[4-[[5-[1-(4-Aminoisothiazol-5-yl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[1-(Azetidin-3-ylmethyl)-4-[4-[4-[[5-[1-(benzothiophen-2-yl)-3-(trifluormethyl)pyrazol-4-yl]-1-methylimidazol-2-carbonyl]amino]-2-chlorbenzoyl]piperazin-1-carbonyl]piperidin-1-ium-1-yl]essigsäure;
2-[2-(3-Aminopropyl)-4-[3-[[1-[4-[[5-[1-(5-amino-2-pyridyl)-3-(trifluormethyl)pyrazol-4-yl]-1-methylimidazol-2-carbonyl]amino]-2-chlorbenzoyl]piperidin-4-carbonyl]amino]propyl]pyridin-1-ium-1-yl]essigsäure;
2-[4-[4-[4-[[5-[1-(5-Amino-2-pyridyl)-3-(trifluormethyl)pyrazol-4-yl]-1-methylimidazol-2-carbonyl]amino]-2-chlorbenzoyl]-2-(methoxymethyl)piperazin-1-carbonyl]-1-(azetidin-3-ylmethyl)piperidin-1-ium-1-yl]essigsäure;
cis-2-[1-(3-Aminopropyl)-4-[4-[2-chlor-4-[[5-[1-[2-(3-hydroxypropyl)-1H-pyrrolo-[3,2-b]pyridin-5-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[4-[4-[4-[[5-[1-[2-(3-Amino-3-oxopropyl)-1H-pyrrolo[3,2-b]pyridin-5-yl]-3-(trifluormethyl)pyrazol-4-yl]-1-methylimidazol-2-carbonyl]amino]-2-chlorbenzoyl]piperazin-1-carbonyl]-1-(3-aminopropyl)piperidin-1-ium-1-yl]essigsäure;
cis-2-[1-(3-Aminopropyl)-4-[4-[2-chlor-4-[[5-[1-(2-fluor-4-morpholinophenyl)-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(3-Aminopropyl)-4-[4-[4-[[5-[1-[2-[(1S)-1-hydroxyethyl]-1H-pyrrolo-[3,2-b]pyridin-5-yl]-3-(trifluormethyl)pyrazol-4-yl]-1-methylimidazol-2-carbonyl]amino]-2-methylbenzoyl]piperazin-1-carbonyl]piperidin-1-ium-1-yl]essigsäure;
cis-2-[1-(3-Aminopropyl)-4-[4-[2-chlor-4-[[5-[1-[2-[2-hydroxy-1-(hydroxymethyl)ethyl]-1H-pyrrolo[3,2-b]pyridin-5-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(3-Aminopropyl)-4-[4-[2-chlor-4-[[1-methyl-5-[1-[1-methyl-2-(morpholin-4-carbonyl)pyrrolo[3,2-b]pyridin-5-yl]-3-(trifluormethyl)pyrazol-4-yl]imidazol-2-carbonyl]amino]benzoyl]piperazin-1-carbonyl]piperidin-1-ium-1-yl]essigsäure;
cis-2-[1-(3-Aminopropyl)-4-[4-[2-chlor-4-[[5-[1-[2-(2-hydroxyethylcarbamoyl)-1-methylpyrrolo[3,2-b]pyridin-5-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[2-[(1S)-1,2-dihydroxyethyl]-1H-pyrrolo[3,2-b]pyridin-5-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[2-[(1R)-1,2-dihydroxyethyl]-1H-pyrrolo[3,2-b]pyridin-5-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[4-[[5-[1-[2-fluor-4-(methylamino)phenyl]-3-(trifluormethyl)pyrazol-4-yl]-1-methylimidazol-2-carbonyl]amino]-2-methylbenzoyl]piperazin-1-carbonyl]piperidin-1-ium-1-yl]essigsäure;
cis-2-[1-(Azetidin-3-ylmethyl)-4-[4-[2-fluor-4-[[5-[1-[2-fluor-4-(methylamino)phenyl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[4-[[5-[1-[4-(dimethylamino)-2-fluorphenyl]-3-(trifluormethyl)pyrazol-4-yl]-1-methylimidazol-2-carbonyl]amino]-2-fluorbenzoyl]piperazin-1-carbonyl]piperidin-1-ium-1-yl]essigsäure;
cis-2-[1-(3-Aminopropyl)-4-[4-[4-[[5-[1-(2-fluor-4-hydroxyphenyl)-3-(trifluormethyl)pyrazol-4-yl]-1-methylimidazol-2-carbonyl]amino]-2-methylbenzoyl]piperazin-1-carbonyl]piperidin-1-ium-1-yl]essigsäure;
cis-2-[1-(Azetidin-3-ylmethyl)-4-[4-[4-[[5-[1-[2-fluor-4-(2-methoxyethylamino)phenyl]-3-(trifluormethyl)pyrazol-4-yl]-1-methylimidazol-2-carbonyl]amino]-2-methylbenzoyl]piperazin-1-carbonyl]piperidin-1-ium-1-yl]-essigsäure;
cis-2-[4-[4-[4-[[5-[1-[2-[(3aS,6aR)-2,3,3a,4,6,6a-Hexahydro-1H-pyrrolo[3,4-c]pyrrol-5-carbonyl]-1H-pyrrolo[3,2-c]pyridin-6-yl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
cis-2-[1-(Azetidin-3-ylmethyl)-4-[4-[2-chlor-4-[[1-methyl-5-[1-[2-(7-oxa-2-azaspiro[3.5]nonan-2-carbonyl)-1H-pyrrolo[3,2-c]pyridin-6-yl]-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[2-(1-imino-1-keto-1,4-thiazinan-4-carbonyl)-1H-pyrrolo[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[2-(2-hydroxyethylcarbamoyl)-1H-pyrrolo[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-[2-(9-oxa-3,7-diazabicyclo[3.3.1]nonan-3-carbonyl)-1H-pyrrolo[3,2-c]pyridin-6-yl]-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-[2-[(3,3,3-trifluor-2-hydroxypropyl)carbamoyl]-1H-pyrrolo[3,2-c]pyridin-6-yl]-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-[2-(piperazin-1-carbonyl)-1H-pyrrolo[3,2-c]pyridin-6-yl]-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[2-(2,2-difluormorpholin-4-carbonyl)-1H-pyrrolo[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[2-[(2-ketopyrrolidin-3-yl)carbamoyl]-1H-pyrrolo[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[2-[(5R)-2,6-dioxa-9-azaspiro[4.5]decan-9-carbonyl]-1H-pyrrolo[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-[2-(thiomorpholin-4-carbonyl)-1H-pyrrolo[3,2-c]pyridin-6-yl]-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[2-(dimethylcarbamoyl)-1H-pyrrolo[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-fluor-4-[[5-[1-[2-fluor-4-(2-methoxyethylamino)phenyl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[2-(hydroxycarbamoyl)-1H-pyrrolo[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[4-[2-(difluormethoxy)ethylamino]-2-fluorphenyl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(3-Aminopropyl)-4-[4-[4-[[5-[1-[2-(2-hydroxyethyl)-1H-pyrrolo[3,2-b]pyridin-5-yl]-3-(trifluormethyl)pyrazol-4-yl]-1-methylimidazol-2-carbonyl]amino]-2-methylbenzoyl]piperazin-1-carbonyl]piperidin-1-ium-1-yl]essigsäure;
cis-2-[4-[4-[4-[[5-[1-(4-Amino-2-fluorphenyl)-3-(difluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
cis-2-[4-[4-[4-[[5-[1-[2-(2-Aminoethyl)-1H-pyrrolo[3,2-b]pyridin-5-yl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]-essigsäure;
2-[4-[4-[[5-[1-(4-Amino-2-fluorphenyl)-3-(trifluormethyl)pyrazol-4-yl]-1-methylimidazol-2-carbonyl]amino]-2-chlorbenzoyl]piperazin-1-carbonyl]oxyethyl-bis(3-aminopropyl)-(carboxymethyl)ammonium;
cis-2-[1-(3-Aminopropyl)-4-[4-[2-fluor-4-[[5-[1-(2-fluor-4-hydroxyphenyl)-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-methyl-4-[[1-methyl-5-[1-(1H-pyrrolo[3,2-b]-pyridin-5-yl)-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[2-fluor-4-[[1-methyl-5-[1-(1H-pyrrolo[3,2-b]-pyridin-5-yl)-3-(trifluormethyl)pyrazol-4-yl]imidazol-2-carbonyl]amino]benzoyl]piperazin-1-carbonyl]piperidin-1-ium-1-yl]essigsäure;
2-[1-(3-Aminopropyl)-4-[1-[2-chlor-4-[[1-methyl-5-[1-(2-methyl-1H-pyrrolo-[3,2-b]-pyridin-5-yl)-3-(trifluormethyl)pyrazol-4-yl]imidazol-2-carbonyl]amino]benzoyl]isonipecotoyl]piperazin-1-ium-1-yl]essigsäure;
2-[4-[1-[4-[[5-[1-(4-Amino-2-fluorphenyl)-3-(trifluormethyl)pyrazol-4-yl]-1-methylimidazol-2-carbonyl]amino]-2-chlorbenzoyl]isonipecotoyl]-1-(3-aminopropyl)piperazin-1-ium-1-yl]essigsäure;
cis-2-[1-(Azetidin-3-ylmethyl)-4-[4-[2-fluor-4-[[5-[1-(2-fluor-4-hydroxyphenyl)-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[4-[[(2S)-2,3-dihydroxypropyl]amino]-2-fluorphenyl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[4-[4-[4-[[5-[1-[1-(2-Amino-1,1-dimethyl-2-oxoethyl)pyrrolo[3,2-b]pyridin-5-yl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]-essigsäure;
cis-2-[1-(Azetidin-3-ylmethyl)-4-[4-[2-chlor-4-[[1-methyl-5-[1-[2-(morpholin-4-carbonyl)-1H-pyrrolo[3,2-c]pyridin-6-yl]-3-(trifluormethyl)pyrazol-4-yl]imidazol-2-carbonyl]amino]benzoyl]piperazin-1-carbonyl]piperidin-1-ium-1-yl]essigsäure;
2-[4-[4-[4-[[5-[1-(4-Amino-2-fluorphenyl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperazin-1-ium-1-yl]essigsäure;
2-[4-[4-[4-[[5-[1-(4-Amino-2-fluorphenyl)-3-(trifluormethyl)pyrazol-4-yl]-1-methylimidazol-2-carbonyl]amino]-2-chlorbenzoyl]piperazin-1-carbonyl]-1-(3-aminopropyl)piperazin-1-ium-1-yl]essigsäure;
cis-2-[4-[4-[4-[[5-[1-[2-(2-Aminoethyl)-1H-pyrrolo[3,2-c]pyridin-6-yl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]-essigsäure;
cis-2-[1-(Azetidin-3-ylmethyl)-4-[4-[2-chlor-4-[[1-methyl-5-[1-[1-(methylcarbamoyl)pyrrolo[3,2-b]pyridin-5-yl]-3-(trifluormethyl)pyrazol-4-yl]-imidazol-2-carbonyl]amino]benzoyl]piperazin-1-carbonyl]piperidin-1-ium-1-yl]-essigsäure;
2-[1-(Azetidin-3-ylmethyl)-4-[4-[2-chlor-4-[[1-methyl-5-[1-(2-methyl-1H-pyrrolo-[3,2-b]pyridin-5-yl)-3-(trifluormethyl)pyrazol-4-yl]imidazol-2-carbonyl]amino]benzoyl]piperazin-1-carbonyl]piperazin-1-ium-1-yl]essigsäure;
2-[1-(3-Aminopropyl)-4-[4-[2-chlor-4-[[1-methyl-5-[1-(2-methylol-1H-pyrrolo-[3,2-b]pyridin-5-yl)-3-(trifluormethyl)pyrazol-4-yl]imidazol-2-carbonyl]amino]benzoyl]piperazin-1-carbonyl]piperazin-1-ium-1-yl]essigsäure;
cis-2-[1-(Azetidin-3-ylmethyl)-4-[4-[2-chlor-4-[[5-[1-[3-(2-hydroxyethylcarbamoyl)-1H-pyrrolo[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-[3-(piperazin-1-carbonyl)-1H-pyrrolo[3,2-c]pyridin-6-yl]-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-[3-(4-methylpiperazin-1-carbonyl)-1H-pyrrolo[3,2-c]pyridin-6-yl]-3-(trifluormethyl)pyrazol-4-yl]imidazol-2-carbonyl]amino]benzoyl]piperazin-1-carbonyl]piperidin-1-ium-1-yl]essigsäure;
cis-2-[4-[4-[4-[[5-[1-[2-(2-Amino-2-ketoethyl)pyrazolo[4,3-c]pyridin-6-yl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
cis-2-[1-(Azetidin-3-ylmethyl)-4-[4-[2-chlor-4-[[5-[1-[2-[(1R)-1-hydroxyethyl]-1H-pyrrolo[3,2-b]pyridin-5-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-fluor-4-[[1-methyl-5-[1-(2-methylol-1H-pyrrolo[3,2-b]pyridin-5-yl)-3-(trifluormethyl)pyrazol-4-yl]imidazol-2-carbonyl]amino]benzoyl]piperazin-1-carbonyl]piperidin-1-ium-1-yl]essigsäure;
cis-2-[4-[4-[4-[[5-[1-(4-Amino-2-fluorphenyl)-3-(trifluormethyl)pyrazol-4-yl]-1-methylimidazol-2-carbonyl]amino]-2-methylbenzoyl]piperazin-1-carbonyl]-1-(azetidin-3-ylmethyl)piperidin-1-ium-1-yl]essigsäure;
cis-2-[1-(Azetidin-3-ylmethyl)-4-[4-[2-methyl-4-[[1-methyl-5-[1-(2-methylol-1H-pyrrolo[3,2-b]pyridin-5-yl)-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[2-(2-hydroxyethyl)-1H-pyrrolo[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[3-[(3S)-3-hydroxypyrrolidin-1-carbonyl]-1H-pyrrolo[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-[3-(methylcarbamoyl)-1H-pyrrolo[3,2-c]pyridin-6-yl]-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[4-[[5-[1-(3-carbamoyl-1H-pyrrolo[3,2-c]pyridin-6-yl)-3-(trifluormethyl)pyrazol-4-yl]-1-methylimidazol-2-carbonyl]amino]-2-chlorbenzoyl]piperazin-1-carbonyl]piperidin-1-ium-1-yl]essigsäure;
cis-2-[1-(Azetidin-3-ylmethyl)-4-[4-[2-chlor-4-[[5-[1-[1-(dimethylsulfamoyl)-2-(2-hydroxyethyl)pyrrolo[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
2-[4-[4-[4-[[5-[1-(4-Amino-2-fluorphenyl)-3-(trifluormethyl)pyrazol-4-yl]-1-methylimidazol-2-carbonyl]amino]-2-chlorbenzoyl]piperazin-1-carbonyl]-1-(3-hydroxypropyl)piperidin-1-ium-1-yl]essigsäure;
cis-2-[1-(Azetidin-3-ylmethyl)-4-[4-[2-chlor-4-[[5-[1-[2-[(2S)-2,3-dihydroxypropyl]-1H-pyrrolo[3,2-b]pyridin-5-yl]-3-(trifluormethyl)pyrazol-4-yl]-1-methylimidazol-2-carbonyl]amino]benzoyl]piperazin-1-carbonyl]piperidin-1-ium-1-yl]essigsäure;
2-[4-[4-[4-[[5-[1-[1-(3-Amino-2-methyl-3-oxopropyl)pyrrolo[3,2-b]pyridin-5-yl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
cis-2-[1-(Azetidin-3-ylmethyl)-4-[4-[2-chlor-4-[[5-[1-[2-(2-hydroxyethyl)-1H-pyrrolo[3,2-b]pyridin-5-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-(2-fluor-4-hydroxyphenyl)-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[2-fluor-4-[(2-hydroxy-2-methylpropyl)amino]phenyl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-(5H-pyrrolo[3,2-d]-pyrimidin-2-yl)-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[4-[[5-[1-[5-[2-[bis(2-aminoethyl)amino]ethylamino]-2-pyridyl]-3-(trifluormethyl)pyrazol-4-yl]-1-methylimidazol-2-carbonyl]amino]-2-chlorbenzoyl]piperazin-1-carbonyl]piperidin-1-ium-1-yl]essigsäure;
cis-2-[4-[4-[4-[[5-[1-[2-(2-Amino-2-oxoethyl)-1H-pyrrolo[3,2-b]pyridin-5-yl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
cis-2-[4-[4-[4-[[5-[1-[1-(2-Amino-2-ketoethyl)pyrrolo[3,2-c]pyridin-6-yl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
cis-2-[1-(Azetidin-3-ylmethyl)-4-[4-[2-chlor-4-[[5-[1-[1-(cyanomethyl)-2-methylolpyrrolo[3,2-b]pyridin-5-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[4-[4-[4-[[5-[1-(4-Amino-2-fluorphenyl)-3-(trifluormethyl)pyrazol-4-yl]-1-methylimidazol-2-carbonyl]amino]-2-chlorbenzoyl]piperazin-1-carbonyl]-1-(3-aminopropyl)piperidin-1-ium-1-yl]essigsäure;
cis-2-[1-(3-Aminopropyl)-4-[4-[2-chlor-4-[[1-methyl-5-[1-(2-methylol-1H-pyrrolo-[3,2-b]pyridin-5-yl)-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[3-(dimethylcarbamoyl)-1H-pyrrolo[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-(1H-pyrazolo[4,3-c]-pyridin-6-yl)-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[1-(2-hydroxyethyl)pyrrolo-[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[1-(2-hydroxyethyl)-2-methylolpyrrolo[3,2-b]pyridin-5-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[2-fluor-4-(methylamino)phenyl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-[3-(morpholin-4-carbonyl)-1H-pyrrolo[3,2-c]pyridin-6-yl]-3-(trifluormethyl)pyrazol-4-yl]imidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[2-fluor-4-(2-hydroxyethylamino)phenyl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[3-(4,4-difluorpiperidin-1-carbonyl)-1H-pyrrolo[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[1-methyl-5-[1-(2-methylol-1H-pyrrolo[3,2-c]pyridin-6-yl)-3-(trifluormethyl)pyrazol-4-yl]imidazol-2-carbonyl]amino]benzoyl]piperazin-1-carbonyl]piperidin-1-ium-1-yl]essigsäure;
4-[4-[4-[4-[[5-[1-(4-Amino-2-fluorphenyl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]butansäure;
[4-[4-[4-[[5-[1-(4-Amino-2-fluorphenyl)-3-(trifluormethyl)pyrazol-4-yl]-1-methylimidazol-2-carbonyl]amino]-2-chlorbenzoyl]piperazin-1-yl]-4-oxobutyl]-bis(3-aminopropyl)-(3-carboxypropyl)ammonium;
[4-[4-[4-[[5-[1-(4-Amino-2-fluorphenyl)-3-(trifluormethyl)pyrazol-4-yl]-1-methylimidazol-2-carbonyl]amino]-2-chlorbenzoyl]piperazin-1-yl]-4-oxobutyl]-bis(3-aminopropyl)-(carboxymethyl)ammonium;
cis-2-[4-[4-[4-[[5-[1-[2-(Aminomethyl)-1H-pyrrolo[3,2-b]pyridin-5-yl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
cis-2-[1-(Azetidin-3-ylmethyl)-4-[4-[2-chlor-4-[[5-[1-[2-fluor-4-(2-methoxyethylamino)phenyl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[4-[4-[4-[[5-[1-[4-(3-Aminopropylamino)-2-fluorphenyl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
trans-2-[4-[4-[4-[[5-[1-(4-Amino-2-fluorphenyl)-3-(trifluormethyl)pyrazol-4-yl]-1-methylimidazol-2-carbonyl]amino]-2-chlorbenzoyl]piperazin-1-carbonyl]-1-[2-(dimethylamino)ethyl]piperidin-1-ium-1-yl]essigsäure;
cis-2-[4-[4-[4-[[5-[1-(4-Amino-2-fluorphenyl)-3-(trifluormethyl)pyrazol-4-yl]-1-methylimidazol-2-carbonyl]amino]-2-chlorbenzoyl]piperazin-1-carbonyl]-1-[2-(dimethylamino)ethyl]piperidin-1-ium-1-yl]essigsäure;
cis-2-[4-[4-[4-[[5-[1-[2-(Aminomethyl)-1H-pyrrolo[3,2-c]pyridin-6-yl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
cis-2-[4-[4-[4-[[5-[1-[1-(2-Aminoethyl)pyrrolo[3,2-b]pyridin-5-yl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
cis-2-[1-(Azetidin-3-ylmethyl)-4-[4-[2-chlor-4-[[1-methyl-5-[1-(2-methyl-1H-pyrrolo[2,3-c]pyridin-5-yl)-3-(trifluormethyl)pyrazol-4-yl]imidazol-2-carbonyl]amino]benzoyl]piperazin-1-carbonyl]piperidin-1-ium-1-yl]essigsäure;
cis-2-[4-[4-[4-[[5-[1-(6-Amino-2-pyridyl)-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
2-[4-[4-[4-[[5-[1-(4-Amino-2-fluorphenyl)-3-(trifluormethyl)pyrazol-4-yl]-1-methylimidazol-2-carbonyl]amino]-2-chlorbenzoyl]piperazin-1-carbonyl]-1-methylpiperidin-1-ium-1-yl]essigsäure;
cis-2-[1-(Azetidin-3-ylmethyl)-4-[4-[2-chlor-4-[[5-[1-[3-(2-hydroxyethyl)-1H-pyrrolo[3,2-c]pyridin-6-yl]-3-(trifluormethyl)pyrazol-4-yl]-1-methylimidazol-2-carbonyl]amino]benzoyl]piperazin-1-carbonyl]piperidin-1-ium-1-yl]essigsäure;
2-[[4-[4-[4-[[5-[1-(4-Amino-2-fluorphenyl)-3-(trifluormethyl)pyrazol-4-yl]-1-methylimidazol-2-carbonyl]amino]-2-chlorbenzoyl]piperazin-1-yl]-4-oxobutyl]-(azetidin-3-ylmethyl)methylammonio]acetat;
cis-2-[1-(Azetidin-3-ylmethyl)-4-[4-[4-[[5-[1-[5-[bis(2-aminoethyl)amino]-2-pyridyl]-3-(trifluormethyl)pyrazol-4-yl]-1-methylimidazol-2-carbonyl]amino]-2-chlorbenzoyl]piperazin-1-carbonyl]piperidin-1-ium-1-yl]essigsäure;
cis-2-[1-(Azetidin-3-ylmethyl)-4-[4-[2-chlor-4-[[5-[1-[1-(cyanomethyl)pyrrolo-[3,2-b]pyridin-5-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[1-(Azetidin-3-ylmethyl)-4-[4-[2-chlor-4-[[5-[1-[1-(2-hydroxyethyl)pyrrolo-[3,2-b]pyridin-5-yl]-3-(trifluormethyl)pyrazol-4-yl]-1-methylimidazol-2-carbonyl]amino]benzoyl]piperazin-1-carbonyl]piperidin-1-ium-1-yl]essigsäure;
cis-2-[4-[4-[4-[[5-[1-[1-(2-Amino-2-ketoethyl)pyrrolo[3,2-b]pyridin-5-yl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure;
cis-2-[1-(Azetidin-3-ylmethyl)-4-[4-[2-chlor-4-[[1-methyl-5-[1-(1H-pyrrolo[2,3-c]-pyridin-5-yl)-3-(trifluormethyl)pyrazol-4-yl]imidazol-2-carbonyl]amino]benzoyl]piperazin-1-carbonyl]piperidin-1-ium-1-yl]essigsäure;
2-[4-[4-[4-[[5-[1-[5-(2-Aminoethylamino)-2-pyridyl]-3-(trifluormethyl)pyrazol-4-yl]-1-methylimidazol-2-carbonyl]amino]-2-chlorbenzoyl]piperazin-1-carbonyl]-1-methylpiperidin-1-ium-1-yl]acetat; und
cis-2-[4-[4-[4-[[5-[1-[5-[(3S)-3-Aminopyrrolidin-1-yl]-2-pyridyl]-3-(trifluormethyl)pyrazol-4-yl]-1-methylimidazol-2-carbonyl]amino]-2-chlorbenzoyl]piperazin-1-carbonyl]-1-(azetidin-3-ylmethyl)piperidin-1-ium-1-yl]essigsäure.

13. Verfahren zur Herstellung einer Verbindung der Formel (I) nach einem der Ansprüche 1 bis 12 oder eines pharmazeutisch annehmbaren Salzes davon, wobei das Verfahren wie in dem hierin offenbarten Schema 4 beschrieben ist.

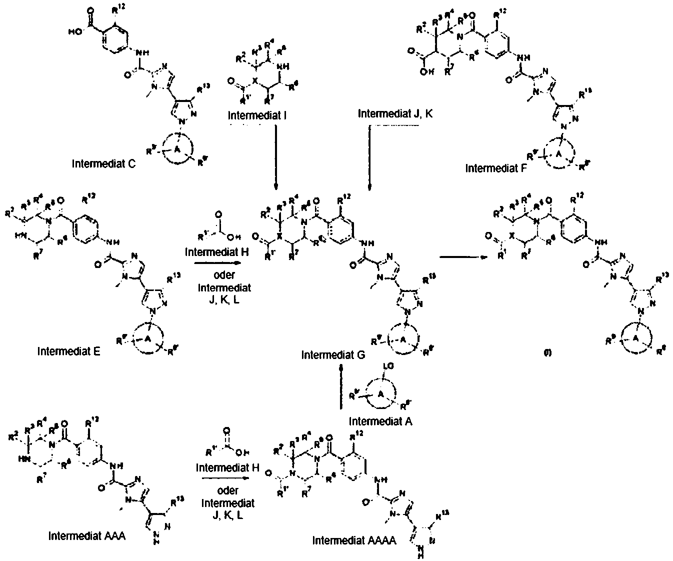

14. Verbindung der Formel (I) nach einem der Ansprüche 1 bis 12 oder ein pharmazeutisch annehmbares Salz davon zur Verwendung bei der Behandlung oder Prävention von Infektionen und daraus resultierenden Krankheiten, verursacht durch gramnegative Bakterien.

15. Verbindung der Formel (I) nach einem der Ansprüche 1 bis 12 oder ein pharmazeutisch annehmbares Salz davon zur Verwendung bei der Behandlung oder Prävention von Infektionen und daraus resultierenden Krankheiten, verursacht durch *Enterococcus faecium, Staphylococcus aureus, Klebsiella pneumoniae, Acinetobacter baumannii, Pseudomonas aeruginosa, Enterobacter species* oder *E. coli* oder eine Kombination davon.

## Revendications

1. Composé de formule (I) 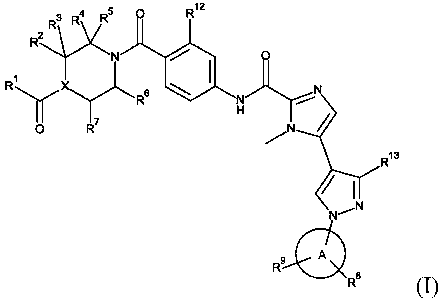 ou un sel pharmaceutiquement acceptable de celui-ci, dans lequel :
X et Y sont chacun indépendamment choisis parmi N et CH ;
n est choisi parmi 0 à 6 ;
A est choisi parmi un hétéroaryle à 5 à 14 chaînons et un aryle en C₆-C₁₄ ;
B est choisi parmi un hétérocyclyle à 3 à 14 chaînons, un hétéroaryle à 5 à 14 chaînons et un cycloalkyle en C₃-C₁₀ ;
L est choisi parmi une liaison covalente, un carbonyle, NHC(O), C(O)NH, CH₂NH, NHCH₂ et NH ;
L¹ représente -O- ou une liaison covalente ;
R¹ est choisi parmi
(i) un alkyle en C₁-C₆ substitué par un amino et un substituant carboxy ;
(ii) un alkyle en C₁-C₆ substitué par un amino et un substituant carboxyalkyle en C₁-C₆-NH ;
(iii) un alkyle en C₁-C₆-N(R^{1a}R^{1b}) ;
(iv) un -L¹-alkyle en C₁-C₆-N⁺(R^{1c}R^{1d}R^{1e}) ;
(v) un groupe 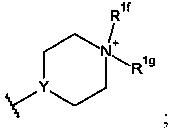 et
(vi) un groupe 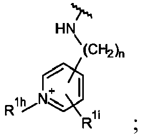
R^{1a} représente un aminoalkyle en C₁-C₆ ;
R^{1b} représente un carboxyalkyle en C₁-C₆ ;
R^{1c} est choisi parmi un aminoalkyle en C₁-C₆ et un (hétérocyclyle à 3 à 14 chaînons)-alkyle en C₁-C₆- ;
R^{1d} représente un carboxyalkyle en C₁-C₆ ;
R^{1e} est choisi parmi un alkyle en C₁-C₆, un aminoalkyle en C₁-C₆ et un (hétérocyclyle à 3 à 14 chaînons)-alkyle en C₁-C₆- ;
R^{1f} est choisi parmi un alkyle en C₁-C₆, un hydroxyalkyle en C₁-C₆, un aminoalkyle en C₁-C₆, un alkyle en C₁-C₆-NH-alkyle en C₁-C₆, un (alkyle en C₁-C₆)₂N-alkyle en C₁-C₆ et un (hétérocyclyle à 3 à 14 chaînons)-alkyle en C₁-C₆- ;
R^{1g} représente un carboxyalkyle en C₁-C₆ ;
R^{1h} représente un carboxyalkyle en C₁-C₆ ;
R¹ⁱ représente un aminoalkyle en C₁-C₆ ;
R² est choisi parmi un hydrogène, un alkyle en C₁-C₆ et un alcoxy en C₁-C₆ ; et R³ et R⁷ représentent tous deux un hydrogène ; ou
R² et R⁷, pris conjointement avec les atomes auxquels ils sont liés, forment un hétérocycle à 4 à 14 chaînons ou un cycloalkyle en C₄-C₁₀ ; et R³ représente un hydrogène ; ou
R² et R³, pris conjointement avec l'atome de carbone auquel ils sont liés, forment un cycloalkyle en C₃-C₁₀ ; et R⁷ représente un hydrogène ;
R⁴ est choisi parmi un hydrogène et un alkyle en C₁-C₆ ; et R⁵ et R⁶ représentent tous deux un hydrogène ; ou
R⁴ et R⁶, pris conjointement avec les atomes auxquels ils sont liés, forment un hétérocycle à 4 à 14 chaînons ; et R⁵ représente un hydrogène ; ou
R⁴ et R⁵, pris conjointement avec l'atome de carbone auquel ils sont liés, forment un cycloalkyle en C₃-C₁₀ ; et R⁶ représente un hydrogène ;
R⁸ est choisi parmi un hydrogène, un cyano, un hydroxy, un amino, un alkyle en C₁-C₆-NH-, un (alkyle en C₁-C₆)₂N-, un (alkyle en C₁-C₆)₂N-SO₂-, un (alkyle en C₁-C₆)₂N-alkyle en C₁-C₆-, un aminoalkyle en C₁-C₆, un aminoalcoxy en C₁-C₆, un aminoalkyle en C₁-C₆-NH-, un (aminoalkyle en C₁-C₆)₂N-, un (aminoalkyle en C₁-C₆)₂N-alkyle en C₁-C₆-NH-, un alkyle en C₁-C₆-NH-alkyle en C₁-C₆-NH-, un alcoxy en C₁-C₆-alkyle en C₁-C₆-NH-, un halogénoalcoxy en C₁-C₆-alkyle en C₁-C₆-NH-, un cycloalkyle en C₃-C₁₀-NH-, un alcoxy en C₁-C₆, un alkyle en C₁-C₆, un cyanoalkyle en C₁-C₆-, un hydroxyalkyle en C₁-C₆-, un hydroxyalkyle en C₁-C₆-NH-, un hydroxyalkyle en C₁-C₆-NHC(O)-, un halogénohydroxyalkyle en C₁-C₆-NHC(O)-, un carbamoyle, un hydroxycarbamoyle, un alkyle en C₁-C₆-NHC(O)-, un (alkyle en C₁-C₆)₂NC(O)-, un carbamoylalkyle en C₁-C₆- et un groupe 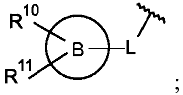
R⁹ est choisi parmi un hydrogène, un halogène, un amino, un alkyle en C₁-C₆ et un hydroxyalkyle en C₁-C₆- ;
R¹⁰ est choisi parmi un hydrogène, un alkyle en C₁-C₆, un halogène, un hydroxy, un oxo, un imino et un amino ;
R¹¹ est choisi parmi un hydrogène et un halogène ;
R¹² est choisi parmi un halogène et un alkyle en C₁-C₆ ;
R¹³ représente un halogénoalkyle en C₁-C₆.

2. Composé de formule (I) selon la revendication 1, ou un sel pharmaceutiquement acceptable de celui-ci, dans lequel L est choisi parmi une liaison covalente, un carbonyle, NHC(O), CH₂NH et NH.

3. Composé de formule (I) selon l'une quelconque des revendications 1 ou 2, ou un sel pharmaceutiquement acceptable de celui-ci, dans lequel :
R^{1f} est choisi parmi un alkyle en C₁-C₆, un hydroxyalkyle en C₁-C₆, un aminoalkyle en C₁-C₆, un (alkyle en C₁-C₆)₂N-alkyle en C₁-C₆ et un (hétérocyclyle à 3 à 14 chaînons)-alkyle en C₁-C₆-.

4. Composé de formule (I) selon l'une quelconque des revendications 1 ou 2, ou un sel pharmaceutiquement acceptable de celui-ci, dans lequel :
R¹ est choisi parmi
(i) un alkyle en C₁-C₆-N⁺(R^{1c}R^{1d}R^{1e}) ; et
(ii) un groupe 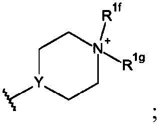 dans lequel
Y représente CH ;
R^{1c} et R^{1e} représentent tous deux un aminoalkyle en C₁-C₆ ;
R^{1d} et R^{1g} représentent tous deux un carboxyalkyle en C₁-C₆ ; et
R^{1f} est choisi parmi un aminoalkyle en C₁-C₆ et un (hétérocyclyle à 3 à 14 chaînons)-alkyle en C₁-C₆-.

5. Composé de formule (I) selon la revendication 3, ou un sel pharmaceutiquement acceptable de celui-ci, dans lequel :
R¹ est choisi parmi
(i) -(CH₂)₃-N⁺(R^{1c}R^{1d}R^{1e}) ; et
(ii) un groupe 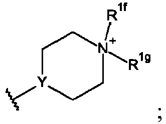 dans lequel
Y représente CH ;
R^{1c} et R^{1e} représentent tous deux un 4-aminobutyle ;
R^{1d} et R^{1g} représentent tous deux un carboxyméthyle ; et
R^{1f} est choisi parmi un 3-aminopropyle et un azétidin-3-ylméthyle.

6. Composé de formule (I) selon l'une quelconque des revendications 1 à 5, ou un sel pharmaceutiquement acceptable de celui-ci, dans lequel R², R³, R⁴, R⁵, R⁶ et R⁷ représentent tous un hydrogène.

7. Composé de formule (I) selon l'une quelconque des revendications 1 à 6, ou un sel pharmaceutiquement acceptable de celui-ci, dans lequel :
A est choisi parmi un thiazolyle ; un isothiazolyle ; un pyridyle ; une 1H-pyrrolo[3,2-b]pyridine ; une 1H-imidazo[4,5-b]pyridine ; un 1,3-benzothiazole ; un benzothiophène ; une 1H-pyrazolo[4,3-b]pyridine ; une 1H-pyrrolo[3,2-c]pyridine ; une 1H-pyrrolo[2,3-b]pyridine, une 1H-pyrrolo[2,3-c]pyridine, une 2H-pyrazolo[4,3-c]pyridine, une 1H-pyrrolo[3,2-d]pyrimidine, un 1H-indazolyle ; une furo[3,2-b]pyridine ; une 2,3-dihydro-1H-pyrrolo[3,2-b]pyridine ; et un phényle ; et
B est choisi parmi une morpholine, une thiomorpholine, une pipérazine, un pyrrolidinyle, un pipéridyle, un pyridyle, un cyclopropyle, un cyclopentyle, un 1,2,3,3a,4,5,6,6a-octahydropyrrolo[3,4-c]pyrrole, un 9-oxa-3,7- diazabicyclo[3.3.1]nonane, un 7-oxa-2-azaspiro[3.5]nonane et un 2,6-dioxa-9-azaspiro[4.5]décane.

8. Composé de formule (I) selon l'une quelconque des revendications 1 à 7, ou un sel pharmaceutiquement acceptable de celui-ci, dans lequel X représente N.

9. Composé de formule (I) selon l'une quelconque des revendications 1 à 8, ou un sel pharmaceutiquement acceptable de celui-ci, dans lequel :
R¹² est choisi parmi un chlore et un méthyle ; et
R¹³ est choisi parmi CHF₂ et CF₃.

10. Composé de formule (I) selon la revendication 1, ou un sel pharmaceutiquement acceptable de celui-ci, dans lequel :
X et Y sont chacun indépendamment choisis parmi N et CH ;
n est choisi parmi 0 à 6 ;
A est choisi parmi un hétéroaryle à 5 à 14 chaînons et un aryle en C₆-C₁₄ ;
B est choisi parmi un hétérocyclyle à 3 à 14 chaînons, un hétéroaryle à 5 à 14 chaînons et un cycloalkyle en C₃-C₁₀ ;
L est choisi parmi une liaison covalente, un carbonyle, NHC(O),CH₂NH et NH ;
L¹ représente -O- ou une liaison covalente ;
R¹ est choisi parmi
(i) un alkyle en C₁-C₆ substitué par un amino et un substituant carboxy ;
(ii) un alkyle en C₁-C₆ substitué par un amino et un substituant carboxyalkyle en C₁-C₆-NH ;
(iii) un alkyle en C₁-C₆-N(R^{1a}R^{1b}) ;
(iv) un -L¹-alkyle en C₁-C₆-N⁺(R^{1c}R^{1d}R^{1e}) ;
(v) un groupe 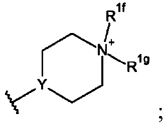 et
(vi) un groupe 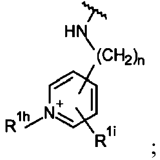
R^{1a} représente un aminoalkyle en C₁-C₆ ;
R^{1b} représente un carboxyalkyle en C₁-C₆ ;
R^{1c} est choisi parmi un aminoalkyle en C₁-C₆ et un (hétérocyclyle à 3 à 14 chaînons)-alkyle en C₁-C₆- ;
R^{1d} représente un carboxyalkyle en C₁-C₆ ;
R^{1e} est choisi parmi un alkyle en C₁-C₆, un aminoalkyle en C₁-C₆ et un (hétérocyclyle à 3 à 14 chaînons)-alkyle en C₁-C₆- ;
R^{1f} est choisi parmi un alkyle en C₁-C₆, un hydroxyalkyle en C₁-C₆, un aminoalkyle en C₁-C₆, un (alkyle en C₁-C₆)₂N-alkyle en C₁-C₆ et un (hétérocyclyle à 3 à 14 chaînons)-alkyle en C₁-C₆- ;
R^{1g} représente un carboxyalkyle en C₁-C₆ ;
R^{1h} représente un carboxyalkyle en C₁-C₆ ;
R¹ⁱ représente un aminoalkyle en C₁-C₆ ;
R² est choisi parmi un hydrogène, un alkyle en C₁-C₆ et un alcoxy en C₁-C₆ ; et R³ et R⁷ représentent tous deux un hydrogène ; ou
R² et R⁷, pris conjointement avec les atomes auxquels ils sont liés, forment un hétérocycle à 4 à 14 chaînons ou un cycloalkyle en C₄-C₁₀ ; et R³ représente un hydrogène ; ou
R² et R³, pris conjointement avec l'atome de carbone auquel ils sont liés, forment un cycloalkyle en C₃-C₁₀ ; et R⁷ représente un hydrogène ;
R⁴ est choisi parmi un hydrogène et un alkyle en C₁-C₆ ; et R⁵ et R⁶ représentent tous deux un hydrogène ; ou
R⁴ et R⁶, pris conjointement avec les atomes auxquels ils sont liés, forment un hétérocycle à 4 à 14 chaînons ; et R⁵ représente un hydrogène ; ou
R⁴ et R⁵, pris conjointement avec l'atome de carbone auquel ils sont liés, forment un cycloalkyle en C₃-C₁₀ ; et R⁶ représente un hydrogène ;
R⁸ est choisi parmi un hydrogène, un cyano, un amino, un hydroxy, un alkyle en C₁-C₆-NH-, un (alkyle en C₁-C₆)₂N-, un (alkyle en C₁-C₆)₂N-SO₂-, un (alkyle en C₁-C₆)₂N-alkyle en C₁-C₆-, un aminoalkyle en C₁-C₆, un aminoalcoxy en C₁-C₆, un aminoalkyle en C₁-C₆-NH-, un (aminoalkyle en C₁-C₆)₂N-, un (aminoalkyle en C₁-C₆)₂N-alkyle en C₁-C₆-NH-, un alkyle en C₁-C₆-NH-alkyle en C₁-C₆-NH-, un alcoxy en C₁-C₆-alkyle en C₁-C₆-NH-, un halogénoalcoxy en C₁-C₆-alkyle en C₁-C₆-NH-, un cycloalkyle en C₃-C₁₀-NH-, un alcoxy en C₁-C₆, un alkyle en C₁-C₆, un cyanoalkyle en C₁-C₆-, un hydroxyalkyle en C₁-C₆-, un hydroxyalkyle en C₁-C₆-NH-, un hydroxyalkyle en C₁-C₆-NHC(O)-, un halogénohydroxyalkyle en C₁-C₆-NHC(O)-, un carbamoyle, un hydroxycarbamoyle, un alkyle en C₁-C₆-NHC(O)-, un (alkyle en C₁-C₆)₂NC(O)-, un carbamoylalkyle en C₁-C₆- et un groupe 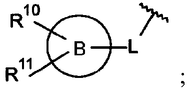
R⁹ est choisi parmi un hydrogène, un halogène, un amino, un alkyle en C₁-C₆ et un hydroxyalkyle en C₁-C₆- ;
R¹⁰ est choisi parmi un hydrogène, un alkyle en C₁-C₆, un halogène, un hydroxy, un oxo, un imino et un amino ;
R¹¹ est choisi parmi un hydrogène et un halogène ;
R¹² est choisi parmi un halogène et un alkyle en C₁-C₆ ;
R¹³ représente un halogénoalkyle en C₁-C₆.

11. Composé de formule (I) selon la revendication 10, ou un sel pharmaceutiquement acceptable de celui-ci, dans lequel :
A est choisi parmi un hétéroaryle à 5 à 14 chaînons et un aryle en C₆-C₁₄ ;
B représente un hétérocyclyle à 3 à 14 chaînons ;
L représente une liaison covalente ;
X représente N ;
Y représente CH ;
R¹ est choisi parmi
(i) un alkyle en C₁-C₆-N⁺(R^{1c}R^{1d}R^{1e}) ; et
(ii) un groupe 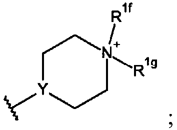
R^{1c} et R^{1e} représentent tous deux un aminoalkyle en C₁-C₆ ;
R^{1d} et R^{1g} représentent tous deux un carboxyalkyle en C₁-C₆ ;
R^{1f} est choisi parmi un aminoalkyle en C₁-C₆ et un (hétérocyclyle à 3 à 14 chaînons)-alkyle en C₁-C₆- ;
R², R³, R⁴, R⁵, R⁶ et R⁷ représentent tous un hydrogène ;
R⁸ est choisi parmi un hydrogène, un amino, un hydroxy, un alkyle en C₁-C₆-NH-, un aminoalkyle en C₁-C₆-NH-, un hydroxyalkyle en C₁-C₆-, un hydroxyalkyle en C₁-C₆-NH-, un alcoxy en C₁-C₆-alkyle en C₁-C₆-NH-, un carbamoylalkyle en C₁-C₆-, un (alkyle en C₁-C₆)₂NC(O)- et un groupe 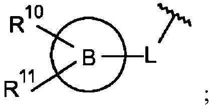
R⁹ est choisi parmi un hydrogène et un halogène ;
R¹⁰ représente un amino ;
R¹¹ représente un hydrogène ;
R¹² est choisi parmi un halogène et un alkyle en C₁-C₆ ; et
R¹³ représente un halogénoalkyle en C₁-C₆.

12. Composé de formule (I) selon la revendication 1, ou un sel pharmaceutiquement acceptable de celui-ci, dans lequel le composé de formule (I) est choisi parmi :
le bis(4-aminobutyl)-(carboxyméthyl)-[4-[4-[2-chloro-4-[[1-méthyl-5-[1-(1H-pyrrolo[3,2-b]pyridin-5-yl)-3-(trifluorométhyl)pyrazol-4-yl]imidazole-2-carbonyl]amino]benzoyl]pipérazino]-4-céto-butyl]ammonium ;
l'acide cis-2-[4-[4-[4-[[5-[1-[5-(2-aminoéthylamino)-2-pyridyl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
le bis(4-aminobutyl)-[4-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazino]-4-céto-butyl]-(carboxyméthyl)ammonium ;
l'acide 2-[4-[4-[4-[[5-[1-[5-(2-aminoéthylamino)-2-pyridyl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
le bis(3-aminopropyl)-(carboxyméthyl)-[4-[4-[2-chloro-4-[[1-méthyl-5-[1-(1H-pyrrolo[3,2-b]pyridin-5-yl)-3-(trifluorométhyl)pyrazol-4-yl]imidazole-2-carbonyl]amino]benzoyl]pipérazino]-4-céto-butyl]ammonium ;
l'acide cis-2-[1-(azétidin-3-ylméthyl)-4-[4-[2-chloro-4-[[1-méthyl-5-[1-(1H-pyrrolo[3,2-c]pyridin-6-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[4-[4-[4-[[5-[1-[5-(3-aminopropylamino)-2-pyridyl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide cis-2-[4-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[4-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[1-(azétidin-3-ylméthyl)-4-[4-[2-chloro-4-[[1-méthyl-5-[1-(1H-pyrrolo[3,2-b]pyridin-5-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[4-[4-[4-[[5-[1-[5-[[(2S)-2-aminopropyl]amino]-2-pyridyl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
le bis(4-aminobutyl)-[4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazino]-4-céto-butyl]-(carboxyméthyl)ammonium ;
l'acide 2-[4-[4-[4-[[5-[1-[5-(aminométhyl)-2-pyridyl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[1-(azétidin-3-ylméthyl)-4-[4-[2-chloro-4-[[5-[1-(5-méthoxy-2-pyridyl)-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide 2-[4-[4-[4-[[5-[1-[5-(2-aminoéthyl)-2-pyridyl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[4-[4-[4-[[5-[1-[5-(2-aminoéthoxy)-2-pyridyl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[1-(azétidin-3-ylméthyl)-4-[4-[2-chloro-4-[[1-méthyl-5-[1-[5-(2-pyridylméthylamino)-2-pyridyl]-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[4-[4-[4-[[5-[1-[5-[(1-aminocyclopentyl)méthylamino]-2-pyridyl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[1-(azétidin-3-ylméthyl)-4-[4-[2-chloro-4-[[1-méthyl-5-[1-[5-(méthylamino)-2-pyridyl]-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[1-méthyl-5-[1-(5-morpholino-2-pyridyl)-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-(1H-pyrrolo[3,2-b]pyridin-5-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[1-méthyl-5-[1-[5-[[(2S)-pyrrolidin-2-yl]méthylamino]-2-pyridyl]-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-[5-(méthylamino)-2-pyridyl]-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[1-(3-aminopropyl)-4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[5-(2-méthoxyéthylamino)-2-pyridyl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide trans-2-[1-(3-aminopropyl)-4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-(2-méthylol-1H-pyrrolo[3,2-b]pyridin-5-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[1-méthyl-5-[1-(5-pipérazino-2-pyridyl)-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[1-méthyl-5-[1-[5-[2-(méthylamino)éthylamino]-2-pyridyl]-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
le bis(3-aminopropyl)-[4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazino]-4-céto-butyl]-(carboxyméthyl)ammonium ;
le bis(4-aminobutyl)-[4-[4-[4-[[5-[1-[5-(2-aminoéthylamino)-2-pyridyl]-3-(trifluorométhyl)pyrazol-4-yl]-1-méthyl-imidazole-2-carbonyl]amino]-2-chloro-benzoyl]pipérazino]-4-céto-butyl]-(carboxyméthyl)ammonium ;
l'acide 2-[1-(azétidin-3-ylméthyl)-4-[4-[2-chloro-4-[[5-[1-(4-méthoxyphényl)-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[5-(2-hydroxyéthylamino)-2-pyridyl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-(1-méthylpyrrolo[3,2-b]pyridin-5-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[5-[1-[5-(isopropylamino)-2-pyridyl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[1-méthyl-5-[1-[5-(2-pyridylamino)-2-pyridyl]-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-(1H-pyrazolo[4,3-b]pyridin-5-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[1-méthyl-5-[1-thiazol-2-yl-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[5-[1-[5-(cyclopropylamino)-2-pyridyl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[5-[1-[5-(diméthylamino)-2-pyridyl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[5-[1-[4-(diméthylamino)phényl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[1-méthyl-5-[1-[5-[[(3S)-morpholin-3-yl]méthylamino]-2-pyridyl]-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(3-aminopropyl)-4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazine-1-carbonyl]pipéridin-1-ium-1-yl]acétique ;
l'acide 2-[1-(azétidin-3-ylméthyl)-4-[4-[2-chloro-4-[[5-[1-[6-(diméthylamino)-3-pyridyl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
le [4-[4-[4-[[5-[1-(5-aminopyridin-2-yl)-3-(trifluorométhyl)pyrazol-4-yl]-1-méthylimidazole-2-carbonyl]amino]-2-chlorobenzoyl]pipérazin-1-yl]-4-oxobutyl]-bis(azétidin-3-ylméthyl)-(carboxyméthyl)azanium ;
l'acide 2-[4-[4-[4-[[5-[1-[5-(6-aminohexylamino)-2-pyridyl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[1-(azétidin-3-ylméthyl)-4-[4-[2-chloro-4-[[1-méthyl-5-[1-[4-(méthylamino)-2-pyridyl]-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[1-méthyl-5-[1-[5-(4-méthylpipérazino)-2-pyridyl]-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[5-[1-(5-hydroxy-2-pyridyl)-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-(1H-imidazo[4,5-b]pyridin-5-yl)-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide 2-[4-[4-[4-[[5-[1-(6-amino-2-pyridyl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[1-(3-aminopropyl)-4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazine-1-carbonyl]pipéridin-1-ium-1-yl]acétique ;
l'acide 2-[1-(azétidin-3-ylméthyl)-4-[4-[2-chloro-4-[[1-méthyl-5-[1-[5-(4-pipéridylméthylamino)-2-pyridyl]-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[5-[1-[5-[[(2S,4S)-4-fluoropyrrolidin-2-yl]méthylamino]-2-pyridyl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[4-[4-[4-[[5-[1-(5-amino-3-fluoro-2-pyridyl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 4-[4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]butyrique ;
l'acide 2-[1-(azétidin-3-ylméthyl)-4-[4-[2-chloro-4-[[5-[1-(2,3-dihydro-1H-pyrrolo[3,2-b]pyridin-5-yl)-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[5-[1-(1H-indazol-3-yl)-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[5-[1-furo[3,2-b]pyridin-5-yl-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide trans-2-[4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[1-(azétidin-3-ylméthyl)-4-[4-[2-chloro-4-[[5-[1-[5-[(2,2-difluorocyclopropyl)méthylamino]-2-pyridyl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
le 3-aminopropyl-[4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazino]-4-céto-butyl]-(carboxyméthyl)-méthyl-ammonium ;
le bis(3-aminopropyl)-[4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazino]-4-céto-butyl]-(carboxyméthyl)ammonium ;
l'acide 2-[1-(azétidin-3-ylméthyl)-4-[4-[2-chloro-4-[[5-[1-(5,6-diamino-2-pyridyl)-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[1-méthyl-5-[1-[6-(méthylamino)-3-pyridyl]-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[5-[1-[5-(cyclobutylamino)-2-pyridyl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide 2-[2-(3-aminopropyl)-4-[3-[[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazine-1-carbonyl]amino]propyl]pyridin-1-ium-1-yl]acétique ;
l'acide 2-[4-[4-[4-[[5-[1-(4-amino-2-pyridyl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[3-aminopropyl-[4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazino-4-céto-butyl]amino]acétique ;
l'acide cis-2-[4-[4-[4-[[5-[1-[5-(2-aminoéthylamino)-2-pyridyl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[4-[4-[4-[[5-[1-(2-amino-4-pyridyl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[1-(3-aminopropyl)-4-[1-[4-[[5-[1-(5-amino-2-pyridyl)-3-(trifluorométhyl)pyrazol-4-yl]-1-méthyl-imidazole-2-carbonyl]amino]-2-chloro-benzoyl]pipéridine-4-carbonyl]pipérazin-1-ium-1-yl]acétique ;
l'acide 2-[1-(azétidin-3-ylméthyl)-4-[4-[2-chloro-4-[[5-[1-(5-cyano-2-pyridyl)-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide 2-[4-[(2S)-4-[4-[[5-[1-(5-amino-2-pyridyl)-3-(trifluorométhyl)pyrazol-4-yl]-1-méthyl-imidazole-2-carbonyl]amino]-2-chloro-benzoyl]-2-méthyl-pipérazine-1-carbonyl]-1-(azétidin-3-ylméthyl)pipéridin-1-ium-1-yl]acétique ;
l'acide 2-[4-[(3R)-4-[4-[[5-[1-(5-amino-2-pyridyl)-3-(trifluorométhyl)pyrazol-4-yl]-1-méthyl-imidazole-2-carbonyl]amino]-2-chloro-benzoyl]-3-éthyl-pipérazine-1-carbonyl]-1-(azétidin-3-ylméthyl)pipéridin-1-ium-1-yl]acétique ;
l'acide 2-[4-[(3S)-4-[4-[[5-[1-(5-amino-2-pyridyl)-3-(trifluorométhyl)pyrazol-4-yl]-1-méthyl-imidazole-2-carbonyl]amino]-2-chloro-benzoyl]-3-méthyl-pipérazine-1-carbonyl]-1-(azétidin-3-ylméthyl)pipéridin-1-ium-1-yl]acétique ;
l'acide 2-[1-(azétidin-3-ylméthyl)-4-[4-[4-[[5-[1-(1,3-benzothiazol-2-yl)-3-(tnfluorométhyl)pyrazol-4-yl]-1-méthyl-imidazole-2-carbonyl]amino]-2-chloro-benzoyl]pipérazine-1-carbonyl]pipéridin-1-ium-1-yl]acétique ;
l'acide 2-[[(5S)-5-amino-6-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazino]-6-céto-hexyl]amino]acétique ;
l'acide 2-[1-(azétidin-3-ylméthyl)-4-[4-[2-chloro-4-[[5-[1-(6-méthoxy-3-pyridyl)-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide (2S)-2-amino-4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazino]-4-céto-butyrique ;
l'acide 2-[1-(azétidin-3-ylméthyl)-4-[4-[2-chloro-4-[[5-[1-[5-[(diméthylamino)méthyl]-2-pyridyl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide (3R)-3-amino-4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazino]-4-céto-butyrique ;
l'acide 2-[4-[7-[4-[[5-[1-(5-amino-2-pyridyl)-3-(trifluorométhyl)pyrazol-4-yl]-1-méthyl-imidazole-2-carbonyl]amino]-2-chloro-benzoyl]-4,7-diazaspiro[2.5]octane-4-carbonyl]-1-(azétidin-3-ylméthyl)pipéridin-1-ium-1-yl]acétique ;
l'acide 2-[4-[1-[4-[[5-[1-(5-amino-2-pyridyl)-3-(trifluorométhyl)pyrazol-4-yl]-1-méthyl-imidazole-2-carbonyl]amino]-2-chloro-benzoyl]pipéridine-4-carbonyl]-1-(azétidin-3-ylméthyl)pipérazin-1-ium-1-yl]acétique ;
l'acide 2-[1-(azétidin-3-ylméthyl)-4-[4-[2-chloro-4-[[1-méthyl-5-[1-[2-(méthylamino)-4-pyridyl]-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[4-[4-[4-[[5-[1-(6-amino-3-pyridyl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[4-[8-[4-[[5-[1-(5-amino-2-pyridyl)-3-(trifluorométhyl)pyrazol-4-yl]-1-méthyl-imidazole-2-carbonyl]amino]-2-chloro-benzoyl]-3,8-diazabicyclo[3.2.1]octane-3-carbonyl]-1-(azétidin-3-ylméthyl)pipéridin-1-ium-1-yl]acétique ;
l'acide (2S)-2-amino-5-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazino]-5-céto-valerique ;
l'acide (3S)-3-amino-4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]pipérazino]-4-céto-butyrique ;
l'acide 2-[4-[4-[4-[[5-[1-[6-(2-aminoéthylamino)-3-pyridyl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[4-[3-[4-[[5-[1-(5-amino-2-pyridyl)-3-(trifluorométhyl)pyrazol-4-yl]-1-méthyl-imidazole-2-carbonyl]amino]-2-chloro-benzoyl]-3,9-diazabicyclo[3.3.1]nonane-9-carbonyl]-1-(azétidin-3-ylméthyl)pipéridin-1-ium-1-yl]acétique ;
l'acide 2-[4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]-4,7-diazaspiro[2.5]octane-7-carbonyl]-1-(azétidin-3-ylméthyl)pipéridin-1-ium-1-yl]acétique ;
l'acide 2-[4-[4-[4-[[5-[1-(4-aminoisothiazol-5-yl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[1-(azétidin-3-ylméthyl)-4-[4-[4-[[5-[1-(benzothiophén-2-yl)-3-(trifluorométhyl)pyrazol-4-yl]-1-méthyl-imidazole-2-carbonyl]amino]-2-chloro-benzoyl]pipérazine-1-carbonyl]pipéridin-1-ium-1-yl]acétique ;
l'acide 2-[2-(3-aminopropyl)-4-[3-[[1-[4-[[5-[1-(5-amino-2-pyridyl)-3-(trifluorométhyl)pyrazol-4-yl]-1-méthyl-imidazole-2-carbonyl]amino]-2-chloro-benzoyl]pipéridine-4-carbonyl]amino]propyl]pyridin-1-ium-1-yl]acétique ;
l'acide 2-[4-[4-[4-[[5-[1-(5-amino-2-pyridyl)-3-(trifluorométhyl)pyrazol-4-yl]-1-méthyl-imidazole-2-carbonyl]amino]-2-chloro-benzoyl]-2-(méthoxyméthyl)pipérazine-1-carbonyl]-1-(azétidin-3-ylméthyl)pipéridin-1-ium-1-yl]acétique ;
l'acide cis-2-[1-(3-aminopropyl)-4-[4-[2-chloro-4-[[5-[1-[2-(3-hydroxypropyl)-1H-pyrrolo[3,2-b]pyridin-5-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[4-[4-[4-[[5-[1-[2-(3-amino-3-oxo-propyl)-1H-pyrrolo[3,2-b]pyridin-5-yl]-3-(trifluorométhyl)pyrazol-4-yl]-1-méthyl-imidazole-2-carbonyl]amino]-2-chloro-benzoyl]pipérazine-1-carbonyl]-1-(3-aminopropyl)pipéridin-1-ium-1-yl]acétique ;
l'acide cis-2-[1-(3-aminopropyl)-4-[4-[2-chloro-4-[[5-[1-(2-fluoro-4-morpholino-phényl)-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(3-aminopropyl)-4-[4-[4-[[5-[1-[2-[(1S)-1-hydroxyéthyl]-1H-pyrrolo[3,2-b]pyridin-5-yl]-3-(trifluorométhyl)pyrazol-4-yl]-1-méthyl-imidazole-2-carbonyl]amino]-2-méthyl-benzoyl]pipérazine-1-carbonyl]pipéridin-1-ium-1-yl]acétique ;
l'acide cis-2-[1-(3-aminopropyl)-4-[4-[2-chloro-4-[[5-[1-[2-[2-hydroxy-1-(hydroxyméthyl)éthyl]-1H-pyrrolo[3,2-b]pyridin-5-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(3-aminopropyl)-4-[4-[2-chloro-4-[[1-méthyl-5-[1-[1-méthyl-2-(morpholine-4-carbonyl)pyrrolo[3,2-b]pyridin-5-yl]-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(3-aminopropyl)-4-[4-[2-chloro-4-[[5-[1-[2-(2-hydroxyéthylcarbamoyl)-1-méthyl-pyrrolo[3,2-b]pyridin-5-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[2-[(1S)-1,2-dihydroxyéthyl]-1H-pyrrolo[3,2-b]pyridin-5-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[2-[(1R)-1,2-dihydroxyéthyl]-1H-pyrrolo[3,2-b]pyridin-5-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[4-[[5-[1-[2-fluoro-4-(méthylamino)phényl]-3-(trifluorométhyl)pyrazol-4-yl]-1-méthyl-imidazole-2-carbonyl]amino]-2-méthyl-benzoyl]pipérazine-1-carbonyl]pipéridin-1-ium-1-yl]acétique ;
l'acide cis-2-[1-(azétidin-3-ylméthyl)-4-[4-[2-fluoro-4-[[5-[1-[2-fluoro-4-(méthylamino)phényl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[4-[[5-[1-[4-(diméthylamino)-2-fluoro-phényl]-3-(trifluorométhyl)pyrazol-4-yl]-1-méthyl-imidazole-2-carbonyl]amino]-2-fluoro-benzoyl]pipérazine-1-carbonyl]pipéridin-1-ium-1-yl]acétique ;
l'acide cis-2-[1-(3-aminopropyl)-4-[4-[4-[[5-[1-(2-fluoro-4-hydroxy-phényl)-3-(trifluorométhyl)pyrazol-4-yl]-1-méthyl-imidazole-2-carbonyl]amino]-2-méthyl-benzoyl]pipérazine-1-carbonyl]pipéridin-1-ium-1-yl]acétique ;
l'acide cis-2-[1-(azétidin-3-ylméthyl)-4-[4-[4-[[5-[1-[2-fluoro-4-(2-méthoxyéthylamino)phényl]-3-(trifluorométhyl)pyrazol-4-yl]-1-méthyl-imidazole-2-carbonyl]amino]-2-méthyl-benzoyl]pipérazine-1-carbonyl]pipéridin-1-ium-1-yl]acétique ;
l'acide cis-2-[4-[4-[4-[[5-[1-[2-[(3aS,6aR)-2,3,3a,4,6,6a-hexahydro-1H-pyrrolo[3,4-c]pyrrole-5-carbonyl]-1H-pyrrolo[3,2-c]pyridin-6-yl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide cis-2-[1-(azétidin-3-ylméthyl)-4-[4-[2-chloro-4-[[1-méthyl-5-[1-[2-(7-oxa-2-azaspiro[3.5]nonane-2-carbonyl)-1H-pyrrolo[3,2-c]pyridin-6-yl]-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[2-(1-imino-1-céto-1,4-thiazinane-4-carbonyl)-1H-pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[2-(2-hydroxyéthylcarbamoyl)-1H-pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-[2-(9-oxa-3,7-diazabicyclo[3.3.1]nonane-3-carbonyl)-1H-pyrrolo[3,2-c]pyridin-6-yl]-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-[2-[(3,3,3-trifluoro-2-hydroxy-propyl)carbamoyl]-1 H-pyrrolo[3,2-c]pyridin-6-yl]-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-[2-(pipérazine-1-carbonyl)-1H-pyrrolo[3,2-c]pyridin-6-yl]-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[2-(2,2-difluoromorpholine-4-carbonyl)-1H-pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[2-[(2-cétopyrrolidin-3-yl)carbamoyl]-1H-pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[2-[(5R)-2,6-dioxa-9-azaspiro[4.5]décane-9-carbonyl]-1H-pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-[2-(thiomorpholine-4-carbonyl)-1H-pyrrolo[3,2-c]pyridin-6-yl]-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[2-(diméthylcarbamoyl)-1H-pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-fluoro-4-[[5-[1-[2-fluoro-4-(2-méthoxyéthylamino)phényl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[2-(hydroxycarbamoyl)-1H-pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[4-[2-(difluorométhoxy)éthylamino]-2-fluoro-phényl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(3-aminopropyl)-4-[4-[4-[[5-[1-[2-(2-hydroxyéthyl)-1H-pyrrolo[3,2-b]pyridin-5-yl]-3-(trifluorométhyl)pyrazol-4-yl]-1-méthyl-imidazole-2-carbonyl]amino]-2-méthyl-benzoyl]pipérazine-1-carbonyl]pipéridin-1-ium-1-yl]acétique ;
l'acide cis-2-[4-[4-[4-[[5-[1-(4-amino-2-fluoro-phényl)-3-(difluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide cis-2-[4-[4-[4-[[5-[1-[2-(2-aminoéthyl)-1H-pyrrolo[3,2-b]pyridin-5-yl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
le 2-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazine-1-carbonyl]oxyéthyl-bis(3-aminopropyl)-(carboxyméthyl)ammonium ;
l'acide cis-2-[1-(3-aminopropyl)-4-[4-[2-fluoro-4-[[5-[1-(2-fluoro-4-hydroxy-phényl)-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-méthyl-4-[[1-méthyl-5-[1-(1H-pyrrolo[3,2-b]pyridin-5-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-fluoro-4-[[1-méthyl-5-[1-(1H-pyrrolo[3,2-b]pyridin-5-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[1-(3-aminopropyl)-4-[1-[2-chloro-4-[[1-méthyl-5-[1-(2-méthylol-1H-pyrrolo[3,2-b]pyridin-5-yl)-3-(trifluorométhyl)pyrazol-4-yl]imidazole-2-carbonyl]amino]benzoyl]isonipecotoyl]pipérazin-1-ium-1-yl]acétique ;
l'acide 2-[4-[1-[4-[[5-[1-(4-amino-2-fluoro-phényl)-3-(trifluorométhyl)pyrazol-4-yl]-1-méthyl-imidazole-2-carbonyl]amino]-2-chloro-benzoyl]isonipécotoyl]-1-(3-aminopropyl)pipérazin-1-ium-1-yl]acétique ;
l'acide cis-2-[1-(azétidin-3-ylméthyl)-4-[4-[2-fluoro-4-[[5-[1-(2-fluoro-4-hydroxy-phényl)-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[4-[[(2S)-2,3-dihydroxypropyl]amino]-2-fluoro-phényl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[4-[4-[4-[[5-[1-[1-(2-amino-1,1-diméthyl-2-oxo-éthyl)pyrrolo[3,2-b]pyridin-5-yl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide cis-2-[1-(azétidin-3-ylméthyl)-4-[4-[2-chloro-4-[[1-méthyl-5-[1-[2-(morpholine-4-carbonyl)-1H-pyrrolo[3,2-c]pyridin-6-yl]-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[4-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipérazin-1-ium-1-yl]acétique ;
l'acide 2-[4-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazine-1-carbonyl]-1-(3-aminopropyl)pipérazin-1-ium-1-yl]acétique ;
l'acide cis-2-[4-[4-[4-[[5-[1-[2-(2-aminoéthyl)-1H-pyrrolo[3,2-c]pyridin-6-yl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide cis-2-[1-(azétidin-3-ylméthyl)-4-[4-[2-chloro-4-[[1-méthyl-5-[1-[1-(méthylcarbamoyl)pyrrolo[3,2-b]pyridin-5-yl]-3-(trifluorométhyl)pyrazol-4-yl]imidazole-2-carbonyl]amino]benzoyl]pipérazine-1-carbonyl]pipéridin-1-ium-1-yl]acétique ;
l'acide 2-[1-(azétidin-3-ylméthyl)-4-[4-[2-chloro-4-[[1-méthyl-5-[1-(2-méthylol-1H-pyrrolo[3,2-b]pyridin-5-yl)-3-(trifluorométhyl)pyrazol-4-yl]imidazole-2-carbonyl]amino]benzoyl]pipérazine-1-carbonyl]pipérazin-1-ium-1-yl]acétique ;
l'acide 2-[1-(3-aminopropyl)-4-[4-[2-chloro-4-[[1-méthyl-5-[1-(2-méthylol-1H-pyrrolo[3,2-b]pyridin-5-yl)-3-(trifluorométhyl)pyrazol-4-yl]imidazole-2-carbonyl]amino]benzoyl]pipérazine-1-carbonyl]pipérazin-1-ium-1-yl]acétique ;
l'acide cis-2-[1-(azétidin-3-ylméthyl)-4-[4-[2-chloro-4-[[5-[1-[3-(2-hydroxyéthylcarbamoyl)-1H-pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-[3-(pipérazine-1-carbonyl)-1H-pyrrolo[3,2-c]pyridin-6-yl]-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-[3-(4-méthylpipérazine-1-carbonyl)-1H-pyrrolo[3,2-c]pyridin-6-yl]-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[4-[4-[4-[[5-[1-[2-(2-amino-2-céto-éthyl)pyrazolo[4,3-c]pyridin-6-yl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide cis-2-[1-(azétidin-3-ylméthyl)-4-[4-[2-chloro-4-[[5-[1-[2-[(1R)-1-hydroxyéthyl]-1H-pyrrolo[3,2-b]pyridin-5-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-fluoro-4-[[1-méthyl-5-[1-(2-méthylol-1H-pyrrolo[3,2-b]pyridin-5-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[4-[4-[4-[[5-[1-(4-amino-2-fluoro-phényl)-3-(trifluorométhyl)pyrazol-4-yl]-1-méthyl-imidazole-2-carbonyl]amino]-2-méthyl-benzoyl]pipérazine-1-carbonyl]-1-(azétidin-3-ylméthyl)pipéridin-1-ium-1-yl]acétique ;
l'acide cis-2-[1-(azétidin-3-ylméthyl)-4-[4-[2-méthyl-4-[[1-méthyl-5-[1-(2-méthylol-1H-pyrrolo[3,2-b]pyridin-5-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[2-(2-hydroxyéthyl)-1H-pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[3-[(3S)-3-hydroxypyrrolidine-1-carbonyl]-1H-pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-[3-(méthylcarbamoyl)-1H-pyrrolo[3,2-c]pyridin-6-yl]-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[4-[[5-[1-(3-carbamoyl-1H-pyrrolo[3,2-c]pyridin-6-yl)-3-(trifluorométhyl)pyrazol-4-yl]-1-méthyl-imidazole-2-carbonyl]amino]-2-chloro-benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[1-(diméthylsulfamoyl)-2-(2-hydroxyéthyl)pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide 2-[4-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazine-1-carbonyl]-1-(3-hydroxypropyl)pipéridin-1-ium-1-yl]acétique ;
l'acide cis-2-[1-(azétidin-3-ylméthyl)-4-[4-[2-chloro-4-[[5-[1-[2-[(2S)-2,3-dihydroxypropyl]-1H-pyrrolo[3,2-b]pyridin-5-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide 2-[4-[4-[4-[[5-[1-[1-(3-amino-2-méthyl-3-oxo-propyl)pyrrolo[3,2-b]pyridin-5-yl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide cis-2-[1-(azétidin-3-ylméthyl)-4-[4-[2-chloro-4-[[5-[1-[2-(2-hydroxyéthyl)-1H-pyrrolo[3,2-b]pyridin-5-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-(2-fluoro-4-hydroxy-phényl)-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[2-fluoro-4-[(2-hydroxy-2-méthyl-propyl)amino]phényl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-(5H-pyrrolo[3,2-d]pyrimidin-2-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[4-[[5-[1-[5-[2-[bis(2-aminoéthyl)amino]éthylamino]-2-pyridyl]-3-(trifluorométhyl)pyrazol-4-yl]-1-méthyl-imidazole-2-carbonyl]amino]-2-chloro-benzoyl]pipérazine-1-carbonyl]pipéridin-1-ium-1-yl]acétique ;
l'acide cis-2-[4-[4-[4-[[5-[1-[2-(2-amino-2-oxo-éthyl)-1H-pyrrolo[3,2-b]pyridin-5-yl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide cis-2-[4-[4-[4-[[5-[1-[1-(2-amino-2-céto-éthyl)pyrrolo[3,2-c]pyridin-6-yl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide cis-2-[1-(azétidin-3-ylméthyl)-4-[4-[2-chloro-4-[[5-[1-[1-(cyanométhyl)-2-méthylol-pyrrolo[3,2-b]pyridin-5-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[4-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazine-1-carbonyl]-1-(3-aminopropyl)pipéridin-1-ium-1-yl]acétique ;
l'acide cis-2-[1-(3-aminopropyl)-4-[4-[2-chloro-4-[[1-méthyl-5-[1-(2-méthylol-1H-pyrrolo[3,2-b]pyridin-5-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[3-(diméthylcarbamoyl)-1H-pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-(1H-pyrazolo[4,3-c]pyridin-6-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[1-(2-hydroxyéthyl)pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[1-(2-hydroxyéthyl)-2-méthylol-pyrrolo[3,2-b]pyridin-5-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[2-fluoro-4-(méthylamino)phényl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-[3-(morpholine-4-carbonyl)-1H-pyrrolo[3,2-c]pyridin-6-yl]-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[2-fluoro-4-(2-hydroxyéthylamino)phényl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[3-(4,4-difluoropipéridine-1-carbonyl)-1H-pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[1-méthyl-5-[1-(2-méthylol-1H-pyrrolo[3,2-c]pyridin-6-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide 4-[4-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]butanoïque ;
le [4-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazin-1-yl]-4-oxo-butyl]-bis(3-aminopropyl)-(3-carboxypropyl)ammonium ;
le [4-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazin-1-yl]-4-oxo-butyl]-bis(3-aminopropyl)-(carboxyméthyl)ammonium ;
l'acide cis-2-[4-[4-[4-[[5-[1-[2-(aminométhyl)-1H-pyrrolo[3,2-b]pyridin-5-yl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide cis-2-[1-(azétidin-3-ylméthyl)-4-[4-[2-chloro-4-[[5-[1-[2-fluoro-4-(2-méthoxyéthylamino)phényl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[4-[4-[4-[[5-[1-[4-(3-aminopropylamino)-2-fluoro-phényl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide trans-2-[4-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazine-1-carbonyl]-1-[2-(diméthylamino)éthyl]pipéridin-1-ium-1-yl]acétique ;
l'acide cis-2-[4-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazine-1-carbonyl]-1-[2-(diméthylamino)éthyl]pipéridin-1-ium-1-yl]acétique ;
l'acide cis-2-[4-[4-[4-[[5-[1-[2-(aminométhyl)-1H-pyrrolo[3,2-c]pyridin-6-yl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide cis-2-[4-[4-[4-[[5-[1-[1-(2-aminoéthyl)pyrrolo[3,2-b]pyridin-5-yl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide cis-2-[1-(azétidin-3-ylméthyl)-4-[4-[2-chloro-4-[[1-méthyl-5-[1-(2-méthylol-1H-pyrrolo[2,3-c]pyridin-5-yl)-3-(trifluorométhyl)pyrazol-4-yl]imidazole-2-carbonyl]amino]benzoyl]pipérazine-1-carbonyl]pipéridin-1-ium-1-yl]acétique ;
l'acide cis-2-[4-[4-[4-[[5-[1-(6-amino-2-pyridyl)-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide 2-[4-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazine-1-carbonyl]-1-méthyl-pipéridin-1-ium-1-yl]acétique ;
l'acide cis-2-[1-(azétidin-3-ylméthyl)-4-[4-[2-chloro-4-[[5-[1-[3-(2-hydroxyéthyl)-1H-pyrrolo[3,2-c]pyridin-6-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
le 2-[[4-[4-[4-[[5-[1-(4-amino-2-fluoro-phényl)-3-(trifluorométhyl)pyrazol-4-yl]-1-méthyl-imidazole-2-carbonyl]amino]-2-chloro-benzoyl]pipérazin-1-yl]-4-oxo-butyl]-(azétidin-3-ylméthyl)-méthyl-ammonio]acétate ;
l'acide cis-2-[1-(azétidin-3-ylméthyl)-4-[4-[4-[[5-[1-[5-[bis(2-aminoéthyl)amino]-2-pyridyl]-3-(trifluorométhyl)pyrazol-4-yl]-1-méthyl-imidazole-2-carbonyl]amino]-2-chloro-benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[1-(cyanométhyl)pyrrolo[3,2-b]pyridin-5-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[1-(azétidin-3-ylméthyl)-4-[4-[2-chloro-4-[[5-[1-[1-(2-hydroxyéthyl)pyrrolo[3,2-b]pyridin-5-yl]-3-(trifluorométhyl)pyrazol-4-yl]-1-méthyl-imidazole-2-carbonyl]amino]benzoyl]pipérazine-1-carbonyl]pipéridin-1-ium-1-yl]acétique ;
l'acide cis-2-[4-[4-[4-[[5-[1-[1-(2-amino-2-céto-éthyl)pyrrolo[3,2-b]pyridin-5-yl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique ;
l'acide cis-2-[1-(azétidin-3-ylméthyl)-4-[4-[2-chloro-4-[[1-méthyl-5-[1-(1H-pyrrolo[2,3-c]pyridin-5-yl)-3-(trifluorométhyl)pyrazol-4-yl]imidazole-2-carbonyl]amino]benzoyl]pipérazine-1-carbonyl]pipéridin-1-ium-1-yl]acétique ;
le 2-[4-[4-[4-[[5-[1-[5-(2-aminoéthylamino)-2-pyridyl]-3-(trifluorométhyl)pyrazol-4-yl]-1-méthyl-imidazole-2-carbonyl]amino]-2-chloro-benzoyl]pipérazine-1-carbonyl]-1-méthyl-pipéridin-1-ium-1-yl]acétate ; et
l'acide cis-2-[4-[4-[4-[[5-[1-[5-[(3S)-3-aminopyrrolidin-1-yl]-2-pyridyl]-3-(trifluorométhyl)pyrazol-4-yl]-1-méthyl-imidazole-2-carbonyl]amino]-2-chloro-benzoyl]pipérazine-1-carbonyl]-1-(azétidin-3-ylméthyl)pipéridin-1-ium-1-yl]acétique.

13. Procédé de fabrication d'un composé de formule (I) selon l'une quelconque des revendications 1 à 12, ou d'un sel pharmaceutiquement acceptable de celui-ci, dans lequel ledit procédé est tel que décrit dans le schéma 4 décrit ici.

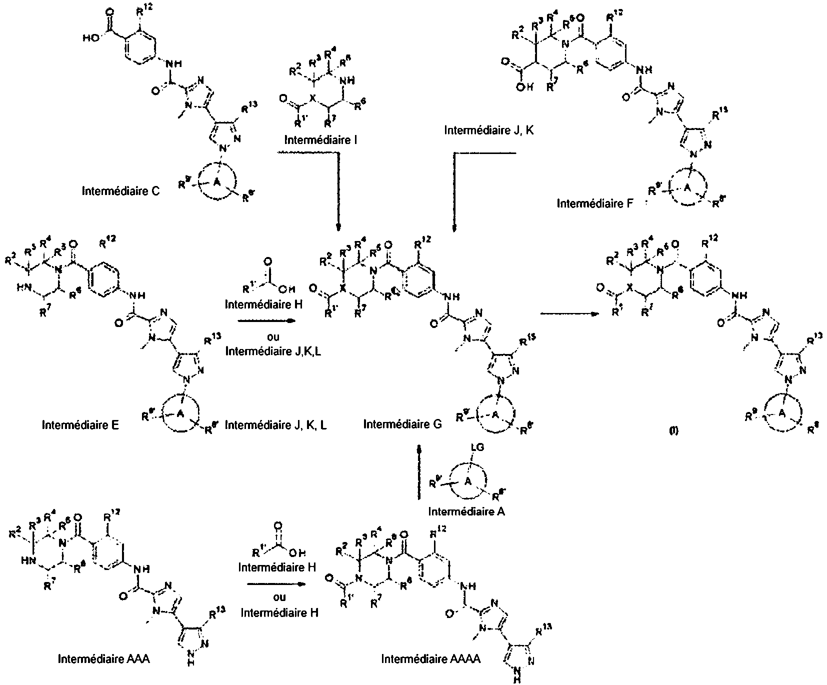

14. Composé de formule (I) selon l'une quelconque des revendications 1 à 12, ou un sel pharmaceutiquement acceptable de celui-ci, pour une utilisation dans le traitement ou la prévention d'infections et de maladies résultantes provoquées par les bactéries à Gram négatif.

15. Composé de formule (I) selon l'une quelconque des revendications 1 à 12, ou un sel pharmaceutiquement acceptable de celui-ci, pour une utilisation dans le traitement ou la prévention d'infections et de maladies résultantes provoquées par ***E**nterococcus faecium, Staphylococcus aureus, **K**lebsiella pneumoniae, Acinetobacter baumannii, Pseudomonas aeruginosa, Enterobacter species* ou *E. coli,* ou une combinaison de celles-ci.
